# EUROPEAN PATENT SPECIFICATION

(11) **EP 3 138 903 B1**
(45) Date of publication and mention of the grant of the patent: **09.02.2022**
(21) Application number: 15786546.0
(22) Date of filing: 01.05.2015
(51) Int. Cl.: C12N 5/00, C07K 16/18, C07K 16/22, C12N 5/10, C12N 15/00, C12N 15/09, C40B 40/02

(54) **CELLS FOR PRODUCING HUMAN ANTIBODY**
ZELLEN ZUR HERSTELLUNG EINES HUMANEN ANTIKÖRPERS
CELLULES POUR LA PRODUCTION D'ANTICORPS HUMAIN

(30) Priority: 02.05.2014 JP 2014095236
(43) Date of publication of application: 08.03.2017
(60) Divisional application: 21151830.3
(73) Proprietor: Chiome Bioscience Inc., Tokyo 151-0071 (JP)
(72) Inventor: HASHIMOTO, Shuichi, Tokyo 151-0071 (JP); UCHIKI, Tomoaki, Tokyo 151-0071 (JP); KAWATA, Shigehisa, Tokyo 151-0071 (JP); ASAGOSHI, Kenjiro, Tokyo 151-0071 (JP); YABUKI, Takashi, Tokyo 151-0071 (JP); SANO, Hitomi, Tokyo 151-0071 (JP); MIYAI, Shunsuke, Tokyo 151-0071 (JP); TAKAHASHI, Naoki, Tokyo 151-0071 (JP); TAKESUE, Aki, Tokyo 151-0071 (JP); SAWADA, Atsushi, Tokyo 151-0071 (JP)
(74) Representative: Vossius & Partner Patentanwälte Rechtsanwälte mbB
(86) International application number: PCT/JP2015/063090
(87) International publication number: WO 2015/167011

(56) References cited:
- WO-A1-2008/047480
- WO-A1-2012/067188
- WO-A1-2013/138518
- JP-A- 2008 099 602
- JP-A- 2011 103 876
- JP-A- 2013 501 525
- US-A1- 2010 310 552
- US-A1- 2010 333 219
- US-A1- 2013 167 256
- ZHU LEI ET AL: "Production of human monoclonal antibody in eggs of chimeric chickens", NATURE BIOTECHNOLOGY, GALE GROUP INC, US, vol. 23, no. 9, 28 August 2005 (2005-08-28), pages 1159-1169, XP002385238, ISSN: 1087-0156, DOI: 10.1038/NBT1132
- BENJAMIN SCHUSSER ET AL: "Harnessing Gene Conversion in Chicken B Cells to Create a Human Antibody Sequence Repertoire", PLOS ONE, vol. 8, no. 11, 21 November 2013 (2013-11-21), page e80108, XP055096053, DOI: 10.1371/journal.pone.0080108
- PHILIP A. LEIGHTON ET AL: "A Diverse Repertoire of Human Immunoglobulin Variable Genes in a Chicken B Cell Line is Generated by Both Gene Conversion and Somatic Hypermutation", FRONTIERS IN IMMUNOLOGY, vol. 6, 20 March 2015 (2015-03-20), XP055340845, DOI: 10.3389/fimmu.2015.00126
- 'Riken Hatsu Bio Venture Chiome Bioscience Inc. no Kigyo' DAIGAKU HATSU VENTURE SOSHUTSU NI MUKETA SYMPOSIUM pages 1 - 19, XP008185310
- 'Harnessing Gene Conversion in Chicken B Cells to Create a Human Antibody Sequence Repertoire' PLOS ONE vol. 8, no. 11, 2013, page E80108, XP055096053
- Kohei Kurosawa ET AL: "Genetic Diversification by Somatic Gene Conversion", Genes, vol. 2, no. 1, 10 January 2011 (2011-01-10), pages 48-58, XP055510213, DOI: 10.3390/genes2010048

## Description

### Technical Field

Disclosed is a cell that produces a human antibody. More specifically, disclosed is a chicken B cell that produces a human antibody.

### Background Art

An antibody is useful for performing identification of biological substances, the function analysis thereof, and the like, and it also plays an important role in the treatment of diseases. Such an antibody binds to a specific antigen in a living body and provokes various *in vivo* protective immune reactions, such as antibody-dependent cell-mediated cytotoxicity (ADCC) or complement-dependent cytotoxicity (CDC), so that the antibody recognizes a "foreign substance" to a living body and removes it. Paying attention on the ability of such an antibody, in recent years, antibody drugs have been vigorously developed. In order to treat a wide variety of diseases, it is necessary to promptly and simply supply antibodies that recognize various target antigens.

To date, as a means for preparing an antibody group comprising desired physical and physiological characteristics, a technique of producing a monoclonal antibody has been used. In a case where such a monoclonal antibody is produced, a hybridoma method comprising fusing B cells generated as a result *of in vivo* immunity with myelomas has been generally applied. However, this method has many problems in that, for example, immunological tolerance is produced due to the use of *in vivo* immunity, and it takes time and effort to finally obtain a desired antibody.

As a technique of overcoming the problem regarding immunological tolerance, a method that does not utilize *in vivo* immunity has been developed. This method comprises embedding a single chain variable fragment (scFv) gene consisting of the variable regions of various antibodies into a phage particle, then allowing the phage to present a single chain antibody gene product on the surface thereof, and then obtaining a clone having an affinity for an antigen of interest from a single chain antibody library displayed by this phage. This method is referred to as a phage display method. This technique is excellent in terms of avoidance of immunological tolerance. However, since the clone obtained from the phage library is a single chain clone, it is necessary to prepare a complete antibody consisting of two chains according to a recombinant DNA technique or the like. Moreover, a change may be generated in affinity during the process of converting a single chain antibody to a complete antibody, and thus, there may be a considerable number of cases where a variable region sequence needs to be adjusted. Accordingly, in terms of time and effort, it cannot be said that the phage display method has been significantly improved in comparison to the method involving *in vivo* immunity.

As methods for overcoming the problem regarding the aforementioned existing method for producing a monoclonal antibody, an ADLib system (Patent Literature 1 and Non Patent Literature 1) and a method of further modifying such an ADLib system (Patent Literature 2) have been reported. The ADLib system is a technique capable of simply preparing a variety of antibodies with desired binding properties to all types of antigens. This method is a technique of selectively obtaining a desired antibody from an antibody library constructed from a chicken B cell-derived cell line DT40, in which antibody genes have become diversified autonomously. The ADLib system is superior to prior arts, in that it enables the avoidance of immunological tolerance, which is an advantage of an *in vitro* system antibody production technique, and in that a complete IgM antibody can be promptly obtained.

In general, in a case where an antibody is administered as a pharmaceutical product to an animal or a human, the antibody is required to be compatible with the type of the animal species, while considering minimization of immunogenicity *in vivo.* Hence, utilizing a DNA recombination technique, a chimeric antibody has been produced by replacing the constant region of a monoclonal antibody produced in mice or the like with a humanized constant region, and a humanized antibody has also been produced by transplanting the complementarity determining region (CDR) of the antibody into a human antibody. However, there may be a case where a change would occur in affinity or function in the process of such chimerization or humanization therefore it cannot be said that the probability of producing a humanized antibody that retains the original functions is high. Moreover, such a chimeric antibody or a humanized antibody only has a small region derived from other animal species therefore its complementarity determining region has immunogenicity, although its immunogenicity is lower than that of the original monoclonal antibody. Hence, such a chimeric antibody or a humanized antibody has been problematic in terms of the appearance of anti-antibody.

Thus, it has been attempted to produce an animal generating a human antibody by introducing a human antibody gene into a mouse. In Patent Literature 3, a microcell of a chromosomal fragment comprising a human antibody gene has been transplanted into mouse ES cells, and then, a chimeric mouse has been produced from the ES cells, thereby producing a chimeric mouse that expresses a human antibody. Moreover, in Non Patent Literature 2, genomic DNA in a mouse antibody gene locus has been replaced with the corresponding genomic DNA in a human antibody gene locus, so as to produce a chimeric mouse that expresses a human antibody. By both of the methods, it is possible to obtain an antibody having complementarity determining regions that are completely derived from human antibody genes. However, since *in vivo* immunity is utilized in both of the methods, the problems regarding time and effort required until a desired antibody is finally obtained have still remained.

Under the aforementioned circumstances, even in the case of using the ADLib system, a method of producing an antibody having an amino acid sequence derived from a human from chicken B cells is necessary to prepare an antibody drug. In addition, in order to treat various diseases, a technique of simply producing an antibody group having various antigen-recognizing properties is necessary.

Nevertheless, differing from a case where a human antibody gene is introduced into a mouse to "humanize" it, in a case where chicken B cells are "humanized," a difference in the system of diversifying an antibody becomes problematic. In the case of animals such as a chicken, a rabbit, a bovine or sheep, there is a region in which "pseudogenes" are gathered, upstream of an antibody variable region. The sequence of the respective pseudogenes are similar to that of antibody variable region and are not translated by themselves. All or a part of variable region sequences are rewritten by pseudogene sequences by a phenomenon referred to as gene conversion, so that a variety of sequences are generated. Diversification of antibodies occurs as a result of V(D)J recombination in animals such as a human or a mouse. In contrast, in animals such as a chicken, a rabbit, a bovine or sheep, such diversification of antibodies occurs by a mechanism referred to as gene conversion that is completely different from the recombination. Moreover, since the pseudogene region has a structure comprising an extremely large number of repeating sequences, it is significantly difficult to analyze the sequence of this region. Thus, even at the time point of March 2015, the pseudogene region of a chicken antibody heavy chain has not yet been analyzed. Due to such a difference in mechanisms or a problem regarding the structure of an antibody gene locus, it has been considered extremely difficult to construct a system of producing a desired human antibody from chicken B cells.

Under such circumstances, it has been reported that a cell line was produced by replacing a variable region and a pseudogene region present in the antibody gene locus of DT40 cells with human-derived sequences (Non Patent Literature 3). In this publication, a chicken antibody variable region has been replaced with a gene sequence comprising fluorescent proteins, namely, a green fluorescent protein (GFP) and a cyan fluorescent protein (CFP), and an attP sequence as a recombinase-recognizing site, and a DT40 cell, into which a human antibody variable region and the after-mentioned pseudogene sequence had been inserted utilizing the recombinase, has been then produced, and thereafter, the occurrence of gene conversion has been confirmed (Non Patent Literatures 3 and 4). However, since a chicken-derived constant region sequence has been used, the expressed antibody molecule is a human-chicken chimeric antibody, and thus, the immunogenicity of the antibody is extremely high. Furthermore, since the light chain is a fusion protein of a human-derived κ chain and a chicken-derived λ chain, the antibody molecule is largely different from a native human antibody. Hence, in order to produce an antibody used for the treatment of humans, it is necessary to further carry out a chimerization operation, and it is still likely that affinity or functions will be changed in the process of chimerization or humanization. Further, with regard to the sequence of the pseudogene region used herein, in the case of a light chain, a minimalist library that is a cluster of artificial sequences, in which one amino acid in each of the CDR sequences of the inserted human antibody variable region has been substituted with Tyr or Trp, and the sequences in which mutations have been introduced into the framework region of a variant of a naturally existing human antibody variable region, have been utilized. On the other hand, a heavy chain thereof is composed of an artificial sequence in which each of the CDR sequences of the inserted human antibody variable region has been substituted with Tyr or Trp, and mutant sequences thereof. These sequences have been designed for the purpose of allowing a small number of pseudogenes to correspond to a variety of antigens. A method of obtaining an antigen-specific antibody in the cells has not been reported.

### Citation List

### Patent Literature

Patent Literature 1: Japanese Patent No.4214234
Patent Literature 2: WO 2008/047480
Patent Literature 3: WO 97/07671

### Non Patent Literature

Non Patent Literature 1: Seo et al., Nature Biotech, Volume 23, pp. 731-735, 2005
Non Patent Literature 2: Recombinant Antibodies for Immunotherapy, pp. 100-108, 2009, Cambridge Press
Non Patent Literature 3: Schusser et al., PLOS ONE, Volume 8 issue 11 e80108, 2013
Non Patent Literature 4: Leighton et al., Frontiers in Immunology, Volume 6 Article 126, 2015

### Summary

### Technical Problem

Under the aforementioned circumstances, it is an object to provide chicken B cells, each of which produces each of various human antibodies.

It is another object to provide a method for producing antibodies from the chicken B cells.

### Solution to Problem

The present inventors have conducted intensive studies regarding production of cells that produce a variety of human antibodies. As a result, the inventors have inserted or replaced the genes in the variable region and constant region of the light chain and heavy chain of a human antibody into chicken B cell antibody light chain and heavy chain gene loci, so that they have succeeded in obtaining transformed cells that each express a human antibody. Moreover, the present inventors have further produced transformed cells, in which a plurality of human-derived antibody variable region sequences have been inserted as pseudogenes into the light chain and heavy chain, and thereafter, the inventors have treated these cells with an HDAC inhibitor, so that they have confirmed that gene conversion has occurred in the transformed cells, as in the case of the original chicken B cells. Furthermore, the present inventors have also succeeded in producing an antigen-specific human antibody from the transformed cells.

That is to say, the present inventors have confirmed that the aforementioned cells are prepared, and from each of the prepared cell, antibody in which the variable region has been modified in various ways can be obtained, and they have then succeeded in producing a variety of human antibodies.
The invention relates to the embodiments as defined in the claims. In particular the invention relates to a chicken B cell, in which the chicken antibody light chain gene locus is replaced by the DNA sequences of a human antibody light chain variable region and by the DNA sequences of a human antibody light chain constant region,
and in which in the chicken antibody heavy chain gene locus the DNA sequences of a human antibody heavy chain variable region and of a human antibody heavy chain constant region are inserted, and in which the chicken antibody light chain pseudogene locus is replaced by 25 or more DNA sequences of human antibody light chain variable regions, and in which in the chicken antibody heavy chain pseudogene locus 30 or more DNA sequences of human antibody heavy chain variable regions are inserted, wherein the chicken B cell has the ability to express a human antibody on the cell surface and also to secrete the human antibody into the culture solution.
In addition, the invention relates to an antibody-producing cell library consisting of chicken B cells of the invention.
Furthermore, the invention relates to a method for producing a human antibody from the chicken B cell of the invention, comprising culturing said chicken B cell under culturing conditions suitable for the cell to produce a human antibody and obtaining said antibody.

### Advantageous Effects

It becomes possible to simply and promptly obtain human antibodies that recognize a variety of antigens from the cells disclosed herein.

### Brief Description of Drawings

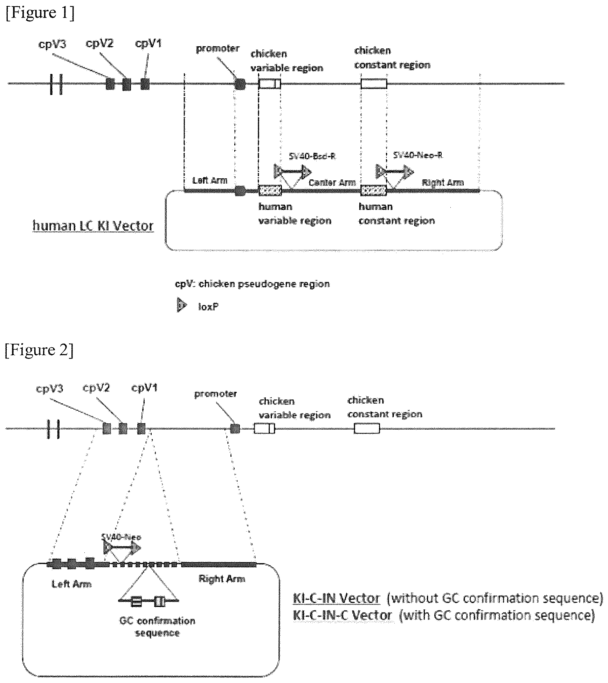

[Figure 1] Figure 1 is a view schematically showing the structure of a targeting vector for replacing the antibody light chain constant region and antibody light chain variable region of a DT40 cell with the light chain constant region and light chain variable region of a human-derived antibody (IgG). cpV1 to cpV3: chicken pseudogenes.
[Figure 2] Figure 2 is a view schematically showing the structure of a targeting vector for inserting a sequence for confirming gene conversion (GC confirmation sequence; a human pseudogene consisting of a sequence derived from a human antibody variable region, the same applied below) downstream of the antibody light chain pseudogene region of a DT40 cell. cpV1 to cpV3: chicken pseudogenes.

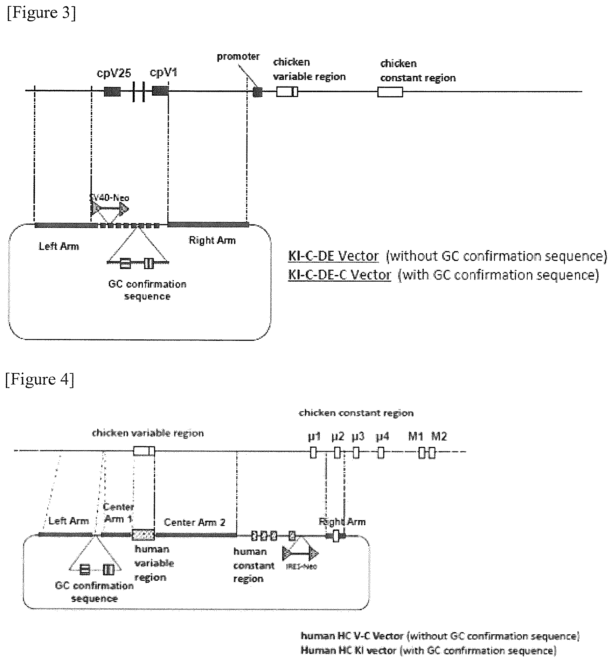

[Figure 3] Figure 3 is a view schematically showing the structure of a targeting vector for deleting the antibody light chain pseudogene region of a DT40 cell, and replacing the antibody light chain pseudogene region of the DT40 cell with a GC confirmation sequence. cpV25, cpV1: chicken pseudogenes.
[Figure 4] Figure 4 is a view schematically showing the structure of a targeting vector for replacing the antibody heavy chain constant region and antibody heavy chain variable region of a DT40 cell with the heavy chain constant region and heavy chain variable region of a human-derived antibody (IgG) and also for inserting a GC confirmation sequence therein.

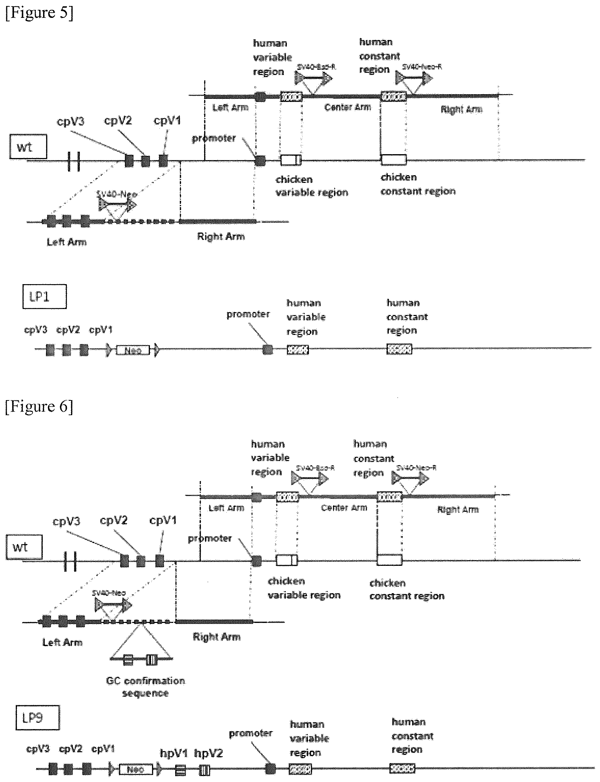

[Figure 5] Figure 5 is a view schematically showing the procedures for replacing the antibody light chain gene regions of a DT40 cell with the light chain variable region and light chain constant region of a human-derived antibody, and the thus obtained antibody light chain gene region of the DT40 cell. cpV1 to cpV3: chicken pseudogenes.
[Figure 6] Figure 6 is a view schematically showing the procedures for replacing the antibody light chain gene regions of a DT40 cell with human-derived antibody light chain variable region and light chain constant region genes, and further inserting a GC confirmation sequence downstream of the antibody light chain pseudogene region of the DT40 cell, and the obtained DT40 cell antibody light chain gene region. cpV1 to cpV3: chicken pseudogenes; and hpV1 and hpV2: human pseudogenes.

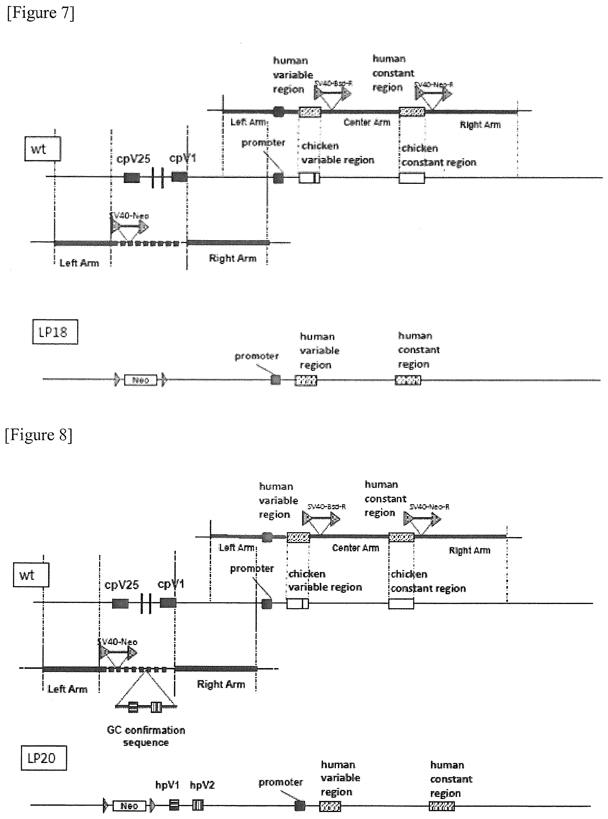

[Figure 7] Figure 7 is a view schematically showing the procedures for replacing the antibody light chain gene regions of a DT40 cell with human-derived antibody light chain variable region and light chain constant region genes, and further deleting the antibody light chain pseudogene region of the DT40 cell, and the thus obtained antibody light chain gene region of the DT40 cell. cpV25 and cpV1: chicken pseudogenes.
[Figure 8] Figure 8 is a view schematically showing the procedures for replacing the antibody light chain gene regions of a DT40 cell with human-derived antibody light chain variable region and light chain constant region genes, and further replacing the pseudogene region of the DT40 cell with a GC confirmation sequence, and the obtained DT40 cell antibody light chain gene region. cpV25 and cpV1: chicken pseudogenes; and hpV1 and hpV2: human pseudogenes.

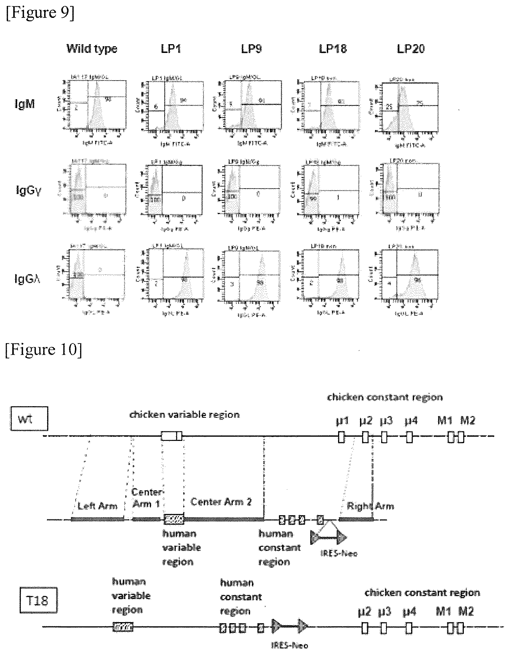

[Figure 9] Figure 9 shows the results obtained by analyzing by flow cytometry an antibody produced by a cell line in which a human-derived antibody light chain gene region has been inserted into the antibody light chain gene region of a DT40 cell, or a cell line in which the antibody light chain gene region of a DT40 cell has been replaced with a human antibody light chain gene.
[Figure 10] Figure 10 is a view schematically showing the procedures for converting the antibody heavy chain gene regions of a DT40 cell to the heavy chain variable region and heavy chain constant region of a human-derived antibody, and the thus obtained antibody heavy chain gene region of the DT40 cell.

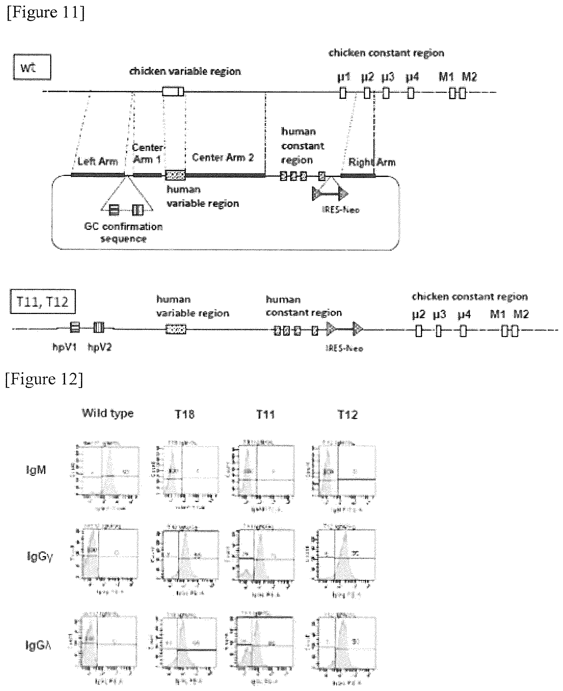

[Figure 11] Figure 11 is a view schematically showing the procedures for converting the antibody heavy chain gene regions of a DT40 cell to the heavy chain variable region and heavy chain constant region gene of a human-derived antibody, and further inserting a GC confirmation sequence downstream of the heavy chain pseudogene region of the DT40 cell, the thus obtained antibody light chain gene region of the DT40 cell, and the structure of the used targeting vector. hpV1 and hpV2: human pseudogenes.
[Figure 12] Figure 12 shows the results obtained by analyzing by flow cytometry an antibody produced by a cell line, in which the antibody heavy chain gene regions of a DT40 cell have been replaced with human-derived antibody heavy chain variable region and heavy chain constant region genes, and further, a GC confirmation sequence has been inserted downstream of the heavy chain pseudogene region of the DT40 cell.

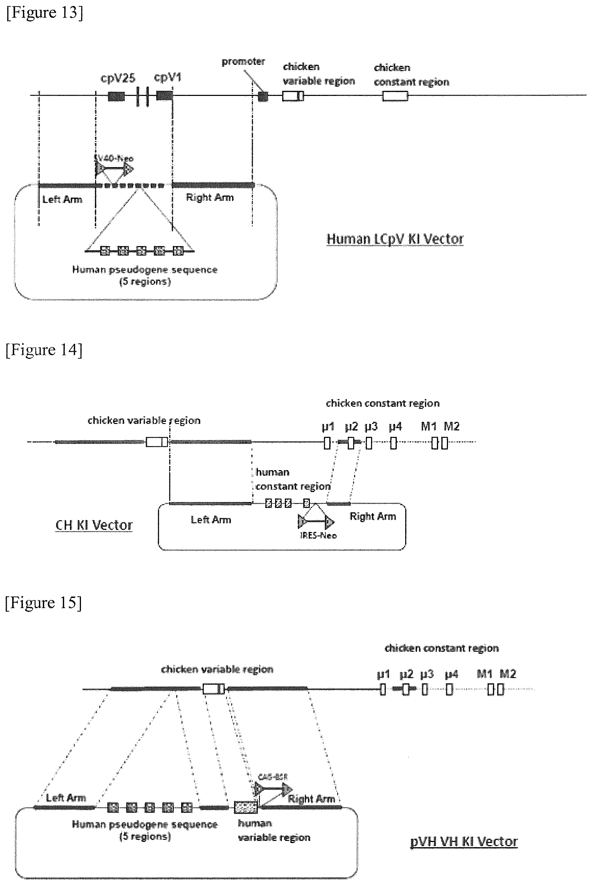

[Figure 13] Figure 13 is a view schematically showing the procedures for replacing the antibody light chain pseudogene region of a DT40 cell with a pseudogene consisting of a sequence derived from a human Igγ variable region (human light chain pseudogene), the thus obtained antibody light chain region of the DT40 cell, and the structure of the used targeting vector.
[Figure 14] Figure 14 is a view schematically showing the structure of a targeting vector for replacing the antibody heavy chain constant region of a DT40 cell with the heavy chain gene region of human IgG₁.
[Figure 15] Figure 15 is a view schematically showing the structure of a targeting vector for introducing the heavy chain variable region of a human-derived antibody and five pseudogenes consisting of human Igγ variable region-derived sequences (human heavy chain pseudogenes) into the antibody heavy chain variable region of a DT40 cell.

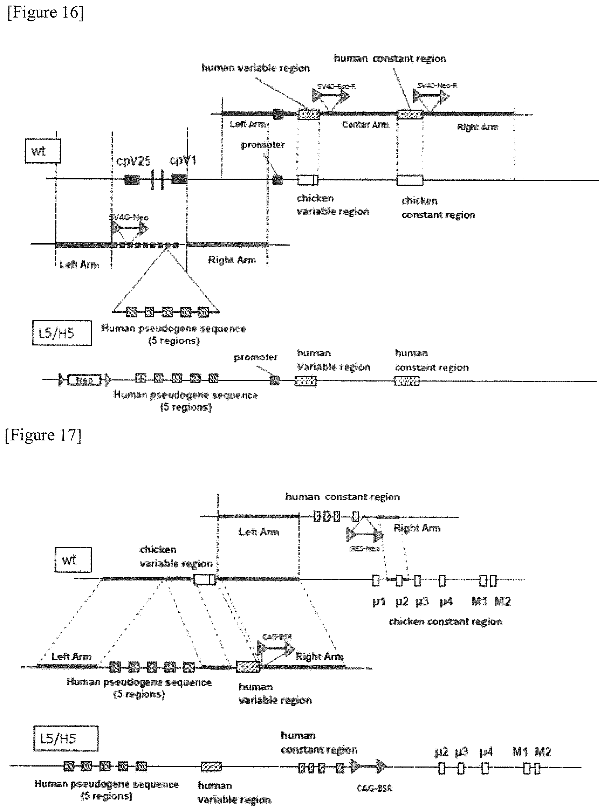

[Figure 16] Figure 16 is a view schematically showing the procedures for replacing the antibody light chain gene regions of a DT40 cell with the light chain variable region and light chain constant region of a human-derived antibody, and then inserting human light chain pseudogene sequences (five sequences) therein, and the thus obtained antibody light chain gene region of the DT40 cell. cpV25 and cpV1: chicken pseudogenes.
[Figure 17] Figure 17 is a view schematically showing the procedures for introducing the heavy chain variable region and heavy chain constant region of a human-derived antibody into the antibody heavy chain gene regions of a DT40 cell, and then inserting human heavy chain pseudogene sequences (five sequences) therein, and the thus obtained antibody heavy chain gene region of the DT40 cell.

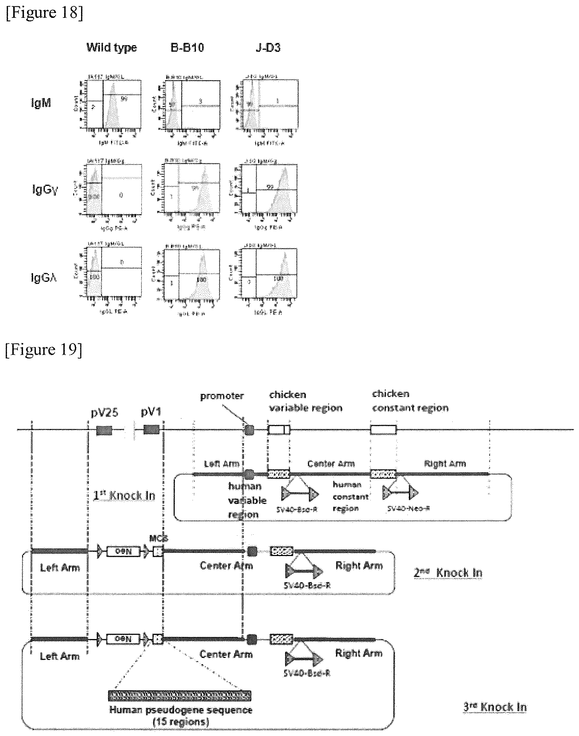

[Figure 18] Figure 18 shows the results obtained by analyzing by flow cytometry an antibody produced by a cell line, in which the antibody light chain gene region and antibody heavy chain gene region of a DT40 cell have been replaced with human-derived antibody genes, and human light chain pseudogene sequences (five sequences) have been then inserted therein.
[Figure 19] Figure 19 is a view schematically showing the procedures for replacing the antibody light chain gene regions of a DT40 cell with the light chain variable region and light chain constant region of a human-derived antibody, and then inserting human heavy chain pseudogene sequences (15 sequences) therein, and the structure of the used targeting vector. pV25 and pV1: chicken pseudogenes.

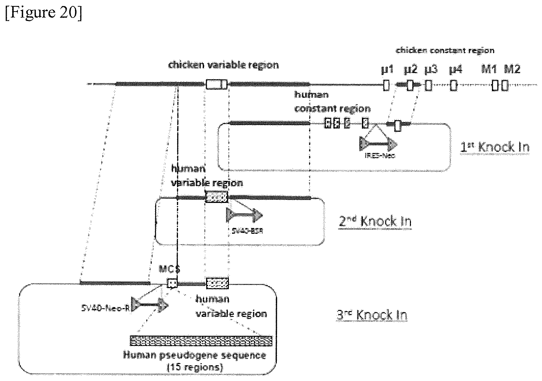

[Figure 20] Figure 20 is a view schematically showing the procedures for introducing the heavy chain variable region and heavy chain constant region of a human-derived antibody into the antibody heavy chain gene regions of a DT40 cell, and then inserting human heavy chain pseudogene sequences (15 sequences) therein, and the structure of the used targeting vector.

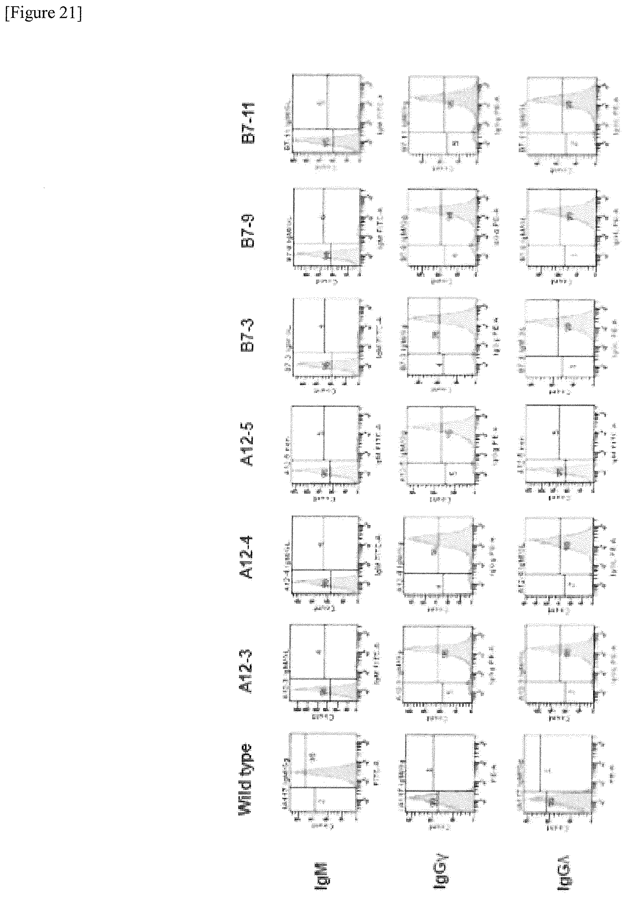

[Figure 21] Figure 21 shows the results obtained by analyzing by flow cytometry an antibody produced by an antibody produced by an L15/H15 cell line, in which the antibody light chain gene region and antibody heavy chain gene region of a DT40 cell have been replaced with the gene regions of a human-derived antibody, and human light chain pseudogene sequences (15 sequences) have been then inserted therein.

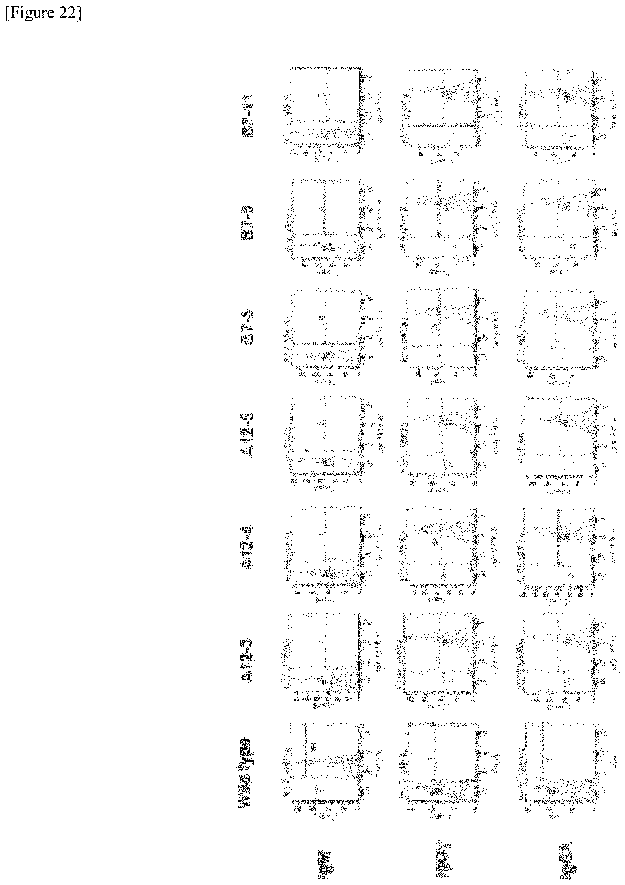

[Figure 22] Figure 22 shows the results shown in Figure 21, in which incorrect data have been corrected based on the data before the priority date.

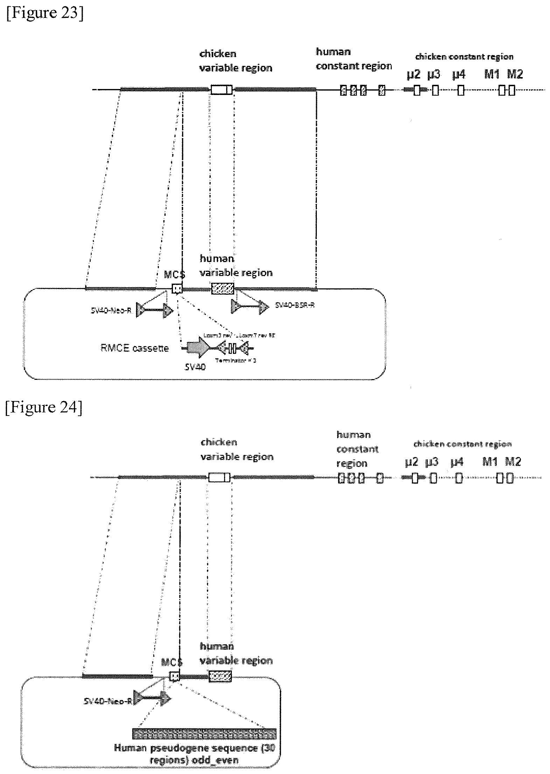

[Figure 23] Figure 23 is a view schematically showing the procedures for replacing the antibody heavy chain variable region of a DT40 cell with the heavy chain variable region of a human-derived antibody, and at the same time, inserting a cassette sequence for RMCE into the antibody heavy chain variable region of the DT40 cell, and the structure of the used targeting vector.
[Figure 24] Figure 24 is a view schematically showing the procedures for inserting 30 human heavy chain pseudogene sequences into the antibody heavy chain gene region of a DT40 cell, and the structure of the used targeting vector.

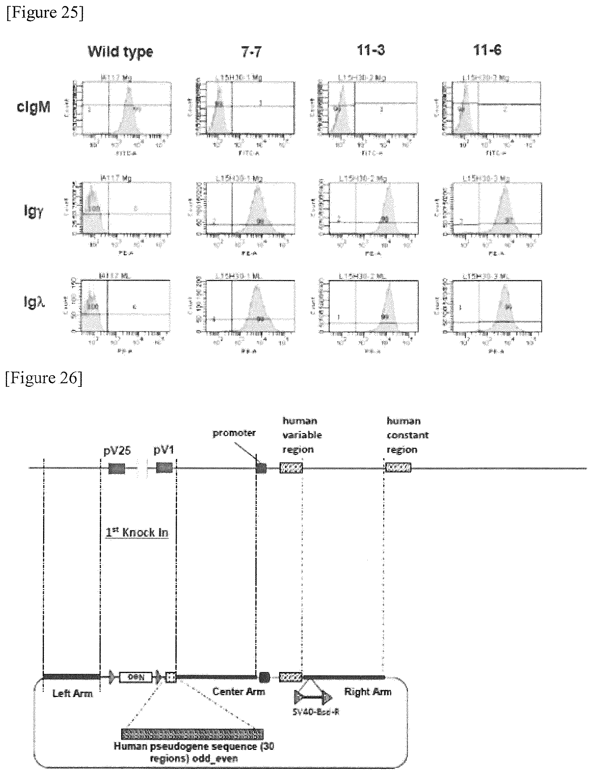

[Figure 25] Figure 25 shows the results obtained by analyzing by flow cytometry an antibody produced by an L15/H30 cell line, in which the antibody light chain gene region and antibody heavy chain gene region of a DT40 cell have been replaced with the gene regions of a human-derived antibody, and thereafter, 15 human light chain pseudogene sequences have been inserted into the light chain and 30 human heavy chain pseudogene sequences have been inserted into the heavy chain.
[Figure 26] Figure 26 is a view schematically showing the procedures for inserting 30 human light chain pseudogene sequences into the antibody light chain gene region of a DT40 cell, and the structure of the used targeting vector.

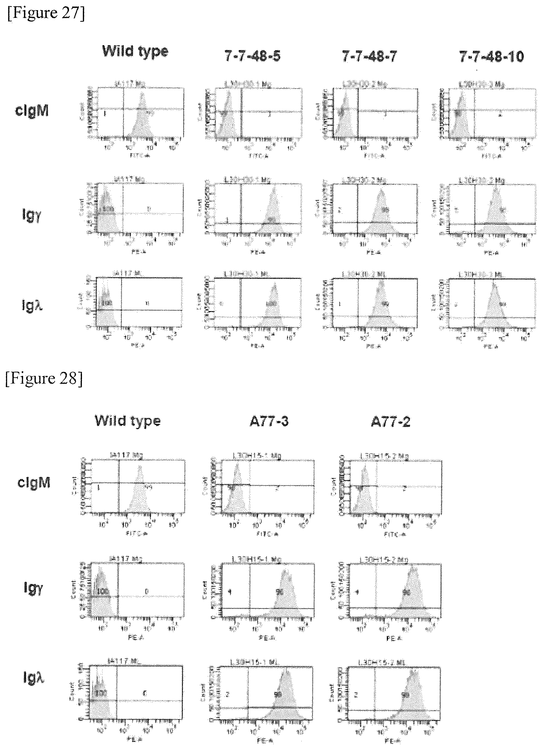

[Figure 27] Figure 27 shows the results obtained by analyzing by flow cytometry an antibody produced by an L30/H30 cell line, in which the antibody light chain gene region and antibody heavy chain gene region of a DT40 cell have been replaced with the gene regions of a human-derived antibody, and thereafter, 30 human light chain pseudogene sequences have been inserted into the light chain and 30 human heavy chain pseudogene sequences have been inserted into the heavy chain.
[Figure 28] Figure 28 shows the results obtained by analyzing by flow cytometry an antibody produced by an L30/H15 cell line, in which the antibody light chain gene region and antibody heavy chain gene region of a DT40 cell have been replaced with the gene regions of a human-derived antibody, and thereafter, 30 human light chain pseudogene sequences have been inserted into the light chain and 15 human heavy chain pseudogene sequences have been inserted into the heavy chain.

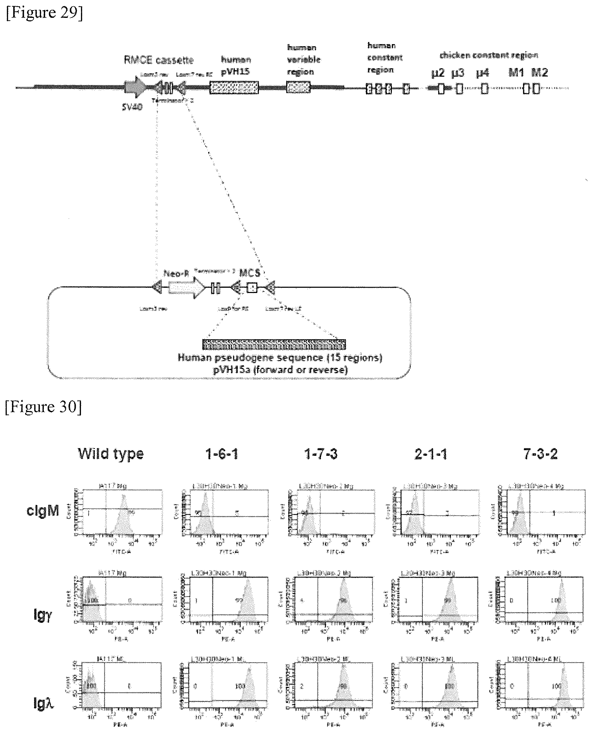

[Figure 29] Figure 29 is a view schematically showing the procedures for additionally inserting 15 human heavy chain pseudogene sequences into the antibody heavy chain gene region of the L30/H15 cell line in the forward or reverse direction according to an RMCE method, so as to have 30 human pseudogene sequences in total, and the structure of the used targeting vector. In the figure, the symbols "for" and "rev" indicate the forward direction and the reverse direction, respectively. The same is applied to other figures below.
[Figure 30] Figure 30 shows the results obtained by analyzing by flow cytometry an antibody produced by a cell line, in which 15 human heavy chain pseudogene sequences have been additionally inserted into the L30/H15 cell line in the forward or reverse direction according to the RMCE method (which is an L30/H15f15f cell line in the case of the forward direction, and an L30/H15r15f cell line in the case of the reverse direction).

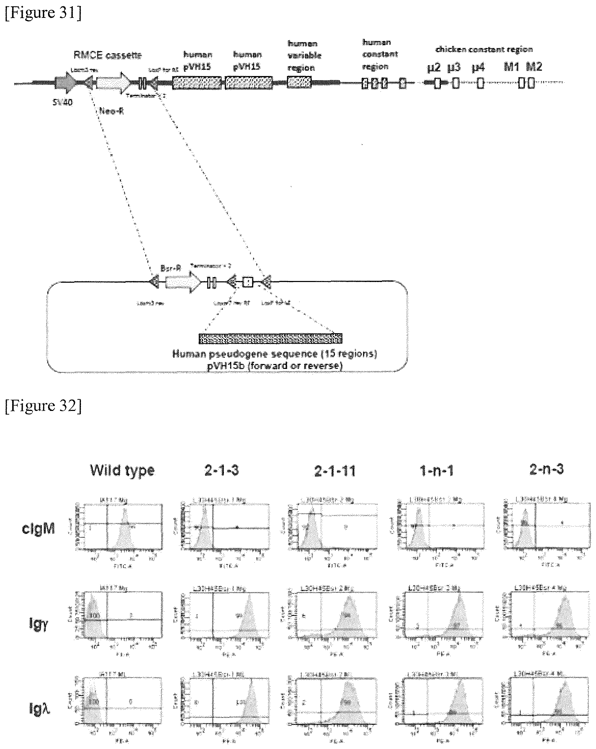

[Figure 31] Figure 31 is a view schematically showing the procedures for additionally inserting 15 human heavy chain pseudogene sequences to each of the antibody heavy chain gene regions of the L30/H15f15f cell line and the L30/H15r15f cell line in the forward or reverse direction, so as to have 45 human pseudogene sequences in total, and the structure of the used targeting vector.
[Figure 32] Figure 32 shows the results obtained by analyzing by flow cytometry an antibody produced by a cell line (L30/H15f15f15f cell line), in which 15 human heavy chain pseudogene sequences have been additionally inserted into the antibody heavy chain gene region of the L30/H15f15f cell line in the forward direction.

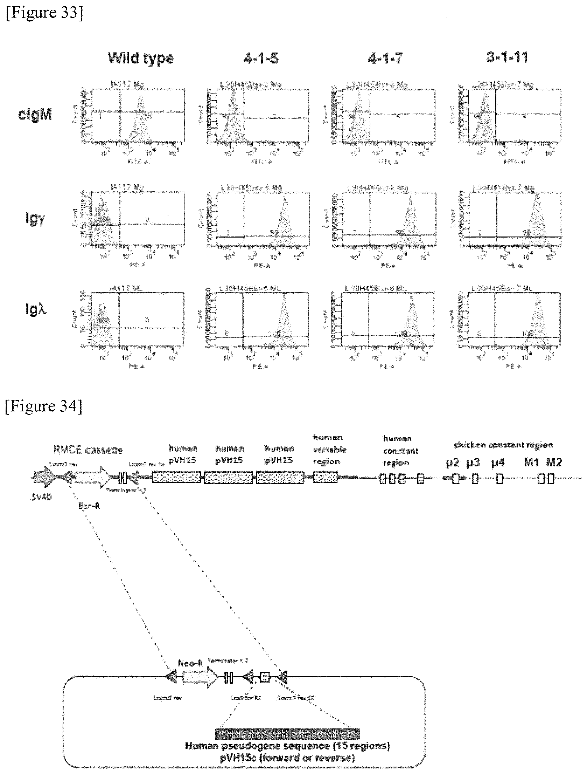

[Figure 33] Figure 33 shows the results obtained by analyzing by flow cytometry an antibody produced by a cell line (L30/H15r15r15f cell line), in which 15 human heavy chain pseudogene sequences have been additionally inserted into the antibody heavy chain gene region of the L30/H15r15f cell line in the reverse direction.
[Figure 34] Figure 34 is a view schematically showing the procedures for additionally inserting 15 human heavy chain pseudogene sequences into each of the antibody heavy chain gene regions of the L30/H15f15f15f cell line and the L30/H15r15r15f cell line in the forward or reverse direction according to the RMCE method, so as to have 60 human pseudogene sequences in total, and the structure of the used targeting vector.

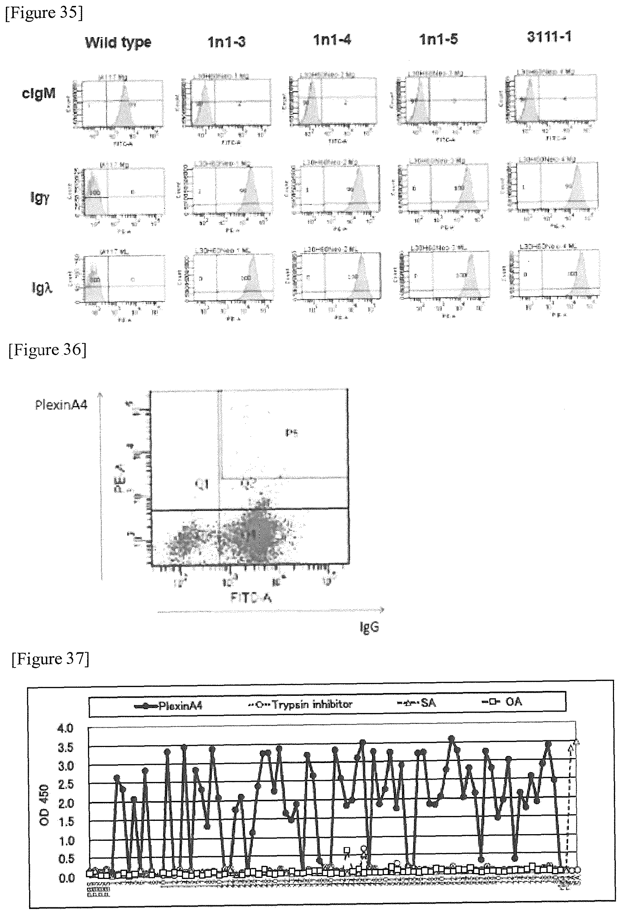

[Figure 35] Figure 35 shows the results obtained by analyzing by flow cytometry an antibody produced by a cell line, in which 30 human light chain pseudogene sequences have been inserted into the antibody light chain gene region of a DT40 cell, and 60 human heavy chain pseudogene sequences have been inserted into the antibody heavy chain gene region thereof.
[Figure 36] Figure 36 shows the results obtained by separating a cell population binding to a Plexin A4 protein from a TSA-treated L15/H15 cell line using an ADLib system, and then analyzing the cell population by flow cytometry.
[Figure 37] Figure 37 shows the results obtained by performing single cell sorting on a cell population in the P5 area of Figure 36, plating the obtained cells on a 96-well plate to culture them, and carrying out ELISA analysis on the cell supernatant in each well. The horizontal axis indicates the number of each well fraction of the 96-well plate, from which the cell supernatant used as a measurement sample is derived. Trypsin inhibitor: trypsin inhibitor; SA: streptavidin; OA: ovalbumin.

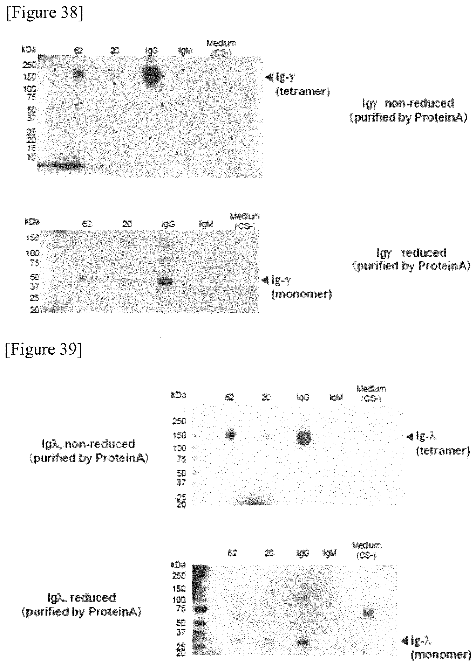

[Figure 38] Figure 38 shows the results obtained by analyzing antibodies secreted from positive clones #62 and #20 according to Western blotting. The samples were treated under reduction and non-reduction conditions, and Western blotting was then carried out on the samples, using a goat anti-human IgGy antibody.
[Figure 39] Figure 39 shows the results obtained by analyzing antibodies secreted from positive clones #62 and #20 according to Western blotting. The samples were treated under reduction and non-reduction conditions, and Western blotting was then carried out on the samples, using a goat anti-human IgGy antibody.

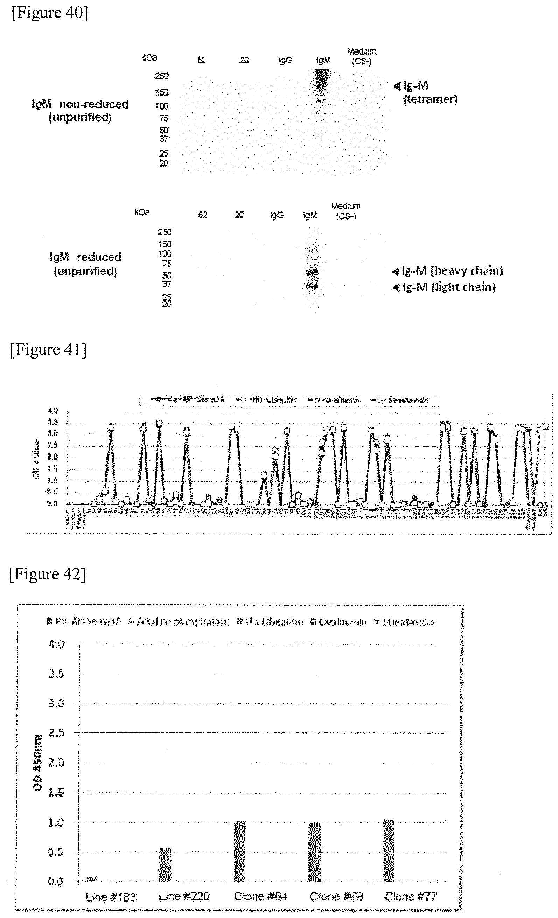

[Figure 40] Figure 40 shows the results obtained by analyzing antibodies secreted from positive clones #62 and #20 according to Western blotting. The samples were treated under reduction and non-reduction conditions, and Western blotting was then carried out on the samples, using a goat anti-chicken IgM antibody.
[Figure 41] Figure 41 shows the results of a primary screening of a cell population binding to His-AP-hSema3A from the TSA-treated L15/H15 cell line, which has been carried out using an ADLib system.
[Figure 42] Figure 42 shows the results of a secondary screening, which has been carried out after the monocloning of the cell population binding to His-AP-hSema3A.

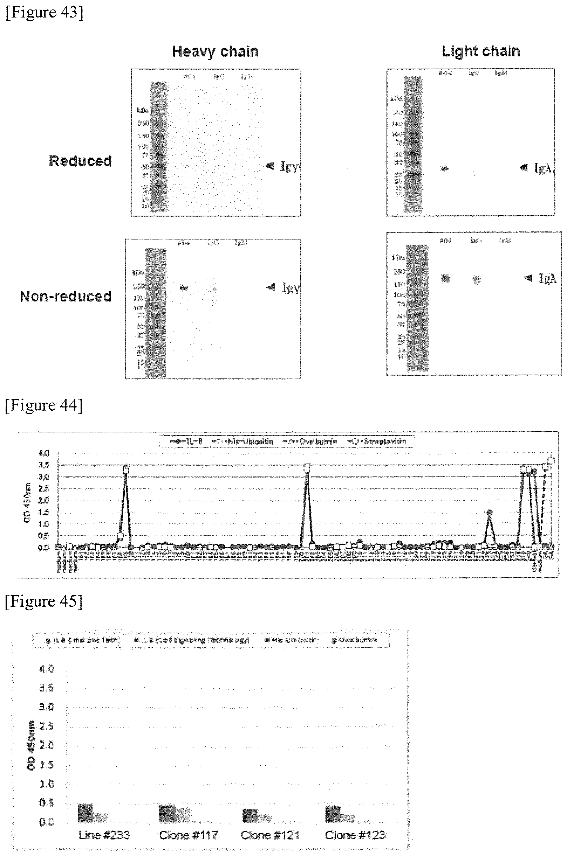

[Figure 43] Figure 43 shows the results obtained by analyzing by Western blotting a His-AP-hSema3A antibody secreted from the positive clones after completion of the secondary screening. The samples were treated under reduction and non-reduction conditions, and Western blotting was then carried out on the samples, using a goat anti-human IgGy antibody or a goat anti-human IgGλ antibody.
[Figure 44] Figure 44 shows the results of a primary screening of a cell population binding to IL-8 from a TSA-treated L30/H45 cell line, which has been carried out using an ADLib system.
[Figure 45] Figure 45 shows the results of a secondary screening, which has been carried out after the monocloning of the cell population binding to IL-8.

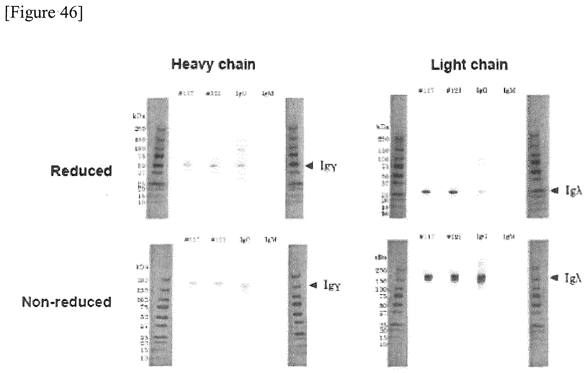

[Figure 46] Figure 46 shows the results obtained by analyzing by Western blotting an IL-8 antibody secreted from the positive clones after completion of the secondary screening. The samples were treated under reduction and non-reduction conditions, and Western blotting was then carried out on the samples, using a goat anti-human IgGy antibody or a goat anti-human IgGλ antibody.

### Description

Disclosed is a chicken B cell, in which, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions.

The B cell may be an antibody-producing B cell derived from a chicken, and an example of the B cell can be a DT 40 cell from a chicken. Other than such DT40 cells, B cells from a bovine, sheep, a rabbit and the like, which have a system for diversifying antibody sequences according to gene conversion, can also be used.

Moreover, in addition to the aforementioned configuration such that, in an antibody light chain gene locus thereof, all or a part of a DNA sequence derived from a human antibody light chain variable region and a human antibody light chain constant region are inserted, or the antibody light chain gene locus is replaced with all or a part of a DNA sequence derived from a human antibody light chain variable region and a human antibody light chain constant region, and in an antibody heavy chain gene locus thereof, all or a part of a DNA sequence derived from a human antibody heavy chain variable region and a human antibody heavy chain constant region are inserted, or the antibody heavy chain gene locus is replaced with all or a part of a DNA sequence derived from a human antibody heavy chain variable region and a human antibody heavy chain constant region, and in an antibody light chain pseudogene locus thereof, two or more DNA sequences derived from human antibody light chain variable regions are inserted, or the antibody light chain pseudogene locus is replaced with two or more DNA sequences derived from human antibody light chain variable regions, and/or in an antibody heavy chain pseudogene locus thereof, two or more DNA sequences derived from human antibody heavy chain variable regions are inserted, or the antibody heavy chain pseudogene locus is replaced with two or more DNA sequences derived from human antibody heavy chain variable regions, the chicken B cell described herein may also comprise mutations or modifications in other gene regions and the like.

The term "antibody light chain gene locus" is used herein to mean a gene locus in which a gene encoding the light chain variable region and light chain constant region of an antibody is present, and the term "antibody heavy chain gene locus" is a gene locus in which a gene encoding the heavy chain variable region and heavy chain constant region of an antibody is present.

In addition, the term "pseudogene" is used herein to mean a DNA sequence that is similar to a functional gene but does not function as an expressing gene. The chicken antibody light chain pseudogene locus and the chicken heavy chain pseudogene locus are each located upstream of the antibody light chain gene locus and heavy chain gene locus, in which the functional gene is present, and contribute to generate the diversity as a result of gene conversion. A pseudogene is not present in an antibody gene locus in human genome, but in the present description, a DNA sequence having a sequence similar to a human antibody variable region, which has been introduced into a chicken antibody gene locus for the purpose of causing gene conversion with the inserted human antibody variable region, is collectively referred to as a "human pseudogene."

A DNA sequence derived from a human antibody light chain variable region and a human antibody light chain constant region, which have been inserted into a chicken antibody light chain gene locus or have been replaced with a sequence in the chicken antibody light chain gene locus, may be all of the sequence of the human antibody light chain variable region and the human antibody light chain constant region, or may also be a part of the sequence. Moreover, the position of the DNA sequence derived from the human antibody light chain variable region inserted into the chicken antibody light chain gene locus is desirably upstream of a position, into which the DNA sequence derived from the human antibody light chain constant region has been inserted.

Likewise, a DNA sequence derived from a human antibody heavy chain variable region and a human antibody heavy chain constant region, which have been inserted into a chicken antibody heavy chain gene locus or have been replaced with a sequence in the chicken antibody heavy chain gene locus, may be all of the sequence of the human heavy chain variable region and the human heavy chain constant region, or may also be a part of the sequence. Moreover, the position of the DNA sequence derived from the human antibody heavy chain variable region inserted into the chicken antibody heavy chain gene locus is desirably upstream of a position, into which the DNA sequence derived from the human antibody heavy chain constant region has been inserted.

Furthermore, the positions of the DNA sequences derived from the human antibody heavy chain variable region and the human antibody constant region inserted into the chicken antibody light chain gene locus are desirably upstream of the positions of DNA sequence derived from a chicken antibody light chain variable region and a chicken antibody light chain constant region. Likewise, the positions of the DNA sequences derived from the human antibody heavy chain variable region and the human antibody heavy chain constant region inserted into the chicken antibody heavy chain gene locus are desirably upstream of the positions of the chicken antibody heavy chain variable region and the chicken antibody heavy chain constant region.

The DNA sequences derived from the human antibody light chain variable region, which have been inserted into the chicken antibody light chain pseudogene locus or have been replaced with the DNA sequences in the chicken antibody light chain pseudogene locus (which are also referred to as "human light chain pseudogene sequences" in the present description and drawings) can be the sequences of known human antibody light chain variable regions. Such sequence information can also be obtained from websites such as V Base (http://www2.mrc-lmb.cam.ac.uk/vbase/list2.php). As such human light chain pseudogene sequences, DNA sequences comprising all or a part of the CDR1, CDR2 and CDR3 of the human antibody light chain variable region can be used. It is desirable that each CDR be not identical to the DNA sequence of the human antibody light chain variable region that has been inserted into the chicken antibody light chain gene locus or has been replaced with the sequence in the chicken antibody light chain gene locus, or to the CDRs of other human light chain pseudogene sequences. Moreover, it is desirable that the framework (FW) region of the human light chain pseudogene sequence be identical to that of the human antibody light chain variable region that has been inserted into the chicken antibody light chain gene locus or has been replaced with the sequence in the chicken antibody light chain gene locus. For instance, when the DNA sequence of SEQ ID NO: 5 is selected as an antibody light chain variable region, the sequences shown in SEQ ID NO: 69 to SEQ ID NO: 88 and SEQ ID NO: 127 to SEQ ID NO: 141 can be used in combination as a light chain pseudogene.

Likewise, the DNA sequences derived from the human antibody heavy chain variable regions, which have been inserted into the chicken antibody heavy chain pseudogene locus or have been replaced with the DNA sequences in the chicken antibody heavy chain pseudogene locus (which are also referred to as "human heavy chain pseudogene sequences" in the present description and drawings) can be the sequences of known human antibody heavy chain variable regions. Such sequence information can also be obtained from websites such as V Base (http://www2.mrc-lmb.cam.ac.uk/vbase/list2.php). As such human heavy chain pseudogene sequences, DNA sequences comprising all or a part of the CDR1, CDR2 and CDR3 of the human antibody heavy chain variable regions can be used. It is desirable that each CDR be not identical to the DNA sequence of the human antibody heavy chain variable region that has been inserted into the chicken antibody heavy chain gene locus or has been replaced with the sequence in the chicken antibody heavy chain gene locus, or to the CDRs of other human heavy chain pseudogene sequences. Moreover, it is desirable that the framework (FW) regions of the human heavy chain pseudogene sequences be identical to that of the human antibody heavy chain variable region that has been inserted into the chicken antibody heavy chain gene locus or has been replaced with the sequence in the chicken antibody heavy chain gene locus. For instance, when the DNA sequence of SEQ ID NO: 30 is selected as an antibody heavy chain variable region, the sequences shown in SEQ ID NO: 89 to SEQ ID NO: 108, SEQ ID NO: 127 to SEQ ID NO: 141, SEQ ID NO: 147 to SEQ ID NO: 161, and SEQ ID NO: 165 to SEQ ID NO: 179 can be used in combination as a heavy chain pseudogene.

The position of the DNA sequences derived from the human antibody light chain variable regions inserted into the chicken antibody light chain pseudogene locus is desirably upstream of the position of the human antibody light chain variable region inserted into the chicken antibody light chain gene locus. In addition, when a chicken antibody light chain pseudogene sequence remains, the DNA sequences derived from the human antibody light chain variable regions is desirably located downstream of the chicken antibody light chain pseudogene region. Likewise, the position of the DNA sequences derived from the human antibody heavy chain variable regions inserted into the chicken antibody heavy chain pseudogene locus is desirably upstream of the position of the human antibody heavy chain variable region inserted into the chicken antibody heavy chain gene locus. In addition, when a chicken antibody heavy chain pseudogene sequence remains, the DNA sequence derived from the human antibody heavy chain variable regions is desirably located downstream of the chicken antibody heavy chain pseudogene region.

Moreover, the DNA sequences derived from the human antibody variable regions, which are inserted or substituted as a pseudogene upstream of the chicken antibody variable region, may be used either in the same direction as, or in the direction opposite to, the direction of the human antibody variable region sequence inserted or substituted into the chicken antibody variable region.

The number of the DNA sequences derived from the human antibody light chain variable regions, which are inserted into the chicken antibody light chain pseudogene locus, or are substituted (replaced) with pseudogenes in the chicken antibody light chain pseudogene locus, is preferably 2 or more, more preferably 5 or more, 7 or more, 9 or more, 11 or more, 13 or more, further preferably 15 or more, 20 or more, or 25 or more. Likewise, the number of DNA sequences derived from the human antibody heavy chain variable regions, which are inserted into the chicken antibody heavy chain pseudogene locus, or are substituted (replaced) with pseudogenes in the chicken antibody heavy chain pseudogene locus, is preferably 2 or more, more preferably 5 or more, 7 or more, 9 or more, 11 or more, 13 or more, further preferably 15 or more, 20 or more, 25 or more, and most preferably 30 or more.

It is to be noted that the DNA sequence derived from the human antibody light chain variable region or the DNA sequence derived from the human antibody heavy chain variable region, which is used herein, is considered to be one DNA sequence, when it is a series of continuous variable region homologous sequences contained in a region over a variable region, and comprises any one of FW1, CDR1, FW2, CDR2, FW3, CDR3 and FW4.

The type of a human antibody heavy chain, which is inserted into the chicken antibody heavy chain gene locus, may be any one of a γ chain, an s chain, an α chain, a δ chain and a ε chain. It is preferably a γ chain. On the other hand, the type of a human antibody light chain, which is inserted into the chicken antibody light chain gene locus and the chicken antibody light chain pseudogene locus may be either a λ chain or a κ chain.

In order to use the transgenic chicken B cell for preparing an antibody library, the chicken B cell preferably does not only express a human antibody on the cell membrane thereof, but it also has an ability to secrete the human antibody into a culture solution. Therefore, when the introduced human antibody gene is transcribed, alternative splicing similar to an *in vivo* expression control mechanism needs to appropriately take place. Thus, with regard to introduction of the constant region, it is ideal to use a genomic structure that is completely identical to the constant region of a chicken antibody heavy chain constant region. However, since the nucleotide sequence of the genomic region of a chicken has not yet been analyzed at the time point of March 2015, this cannot be used. An intron sequence in the genomic sequence of a mouse antibody heavy chain has been used, and as a result, a human antibody on the surface of a cell membrane and a secretory human antibody have been successfully expressed at the same time. Accordingly, it is also possible to use, as a substitute, an intron sequence derived from known mammalian animals such as a mouse or a human.

A knocking-in method involving homologous recombination is used as a method of inserting a human antibody gene into a chicken antibody gene locus and/or replacing the gene in the chicken antibody gene locus with a human antibody gene. Moreover, as methods of inserting human antibody variable region-derived sequences into the pseudogene region in the chicken antibody chain gene locus and/or replacing the gene in the chicken antibody chain gene locus with a human antibody gene, a knocking-in method involving homologous recombination and a recombinase mediated cassette exchange (RMCE) method involving site-specific recombination can be preferably used.

As described above, a chicken B cell produced as described herein means a chicken B cell, into which DNA sequences derived from a human antibody gene locus have been inserted, or which have been replaced with the DNA sequences derived from the human antibody gene locus; and a chicken B cell, in which a wide variety of mutations have been further introduced into the human-derived antibody light chain or heavy chain variable region of the aforementioned cell. Diversification of antibodies can be achieved by reorganization of the variable region that will cause generation of various variable region sequences. Therefore, a chicken B cell produced as described herein also means a chicken B cell on which a treatment of introducing various mutations necessary for the reorganization of the variable region has been performed. Herein, examples of the method of introducing a mutation necessary for the reorganization of the variable region, which can be used herein, include methods known in the present technical field, such as a method of using B cells in which XRCC2 and XRCC3 have been deleted (e.g., Cumber et al., Nature Biotech. 204: 1129-1134 2002, JP Patent Publication (Kokai) No. 2003-503750 A, etc.), a method of controlling the expression of an AID gene (e.g., Kanayama et al., Nucleic Acids Res. 34: e10., 2006, JP Patent Publication (Kokai) No. 2004-298072 A, etc.), and a method of relaxing chromatin to promote gene conversion (e.g., Patent Literature 1, Patent Literature 2, Non Patent Literature 1, etc.). Among others, a method of relaxing chromatin to promote gene conversion is particularly preferable.

An example of the method of relaxing chromatin in a chicken B cell can be a method, which comprises inhibiting histone deacetylase (HDAC) activity existing in the chicken B cell by a certain method, and thereby significantly promoting gene conversion in the cell (for details, see the above-mentioned Patent Literature 1, Patent Literature 2, Non Patent Literature 1, etc.). As methods of inhibiting the activity of HDAC existing in cells, a method of treating the B cell as described herein with an HDAC inhibitor (see Patent Literature 1 or Non Patent Literature 1), a method of reducing or deleting the function of an HDAC gene in a chicken B cell (see Patent Literature 2), and the like can be used. The HDAC inhibitor is not particularly limited, as long as it is able to inhibit the activity of HDAC. Examples of the HDAC inhibitor that can be used herein include trichostatin A (TSA), butyric acid, valproic acid, suberoylanilide hydroxamic acid (SAHA), CBHA (m-carboxycinnamic acid bis-hydroxamide), PTACH, Apicidin, Scriptaid, M344, APHA compound 8, BML-210, Oxamflatin, and MS-275. Other than these substances, an inactive-type protein of the HDAC as a target of activity inhibition (a dominant negative) may also be used as an inhibitory substance, if possible.

When the function of an HDAC gene is reduced or deleted, isoforms of the HDAC as a target whose gene function is to be reduced or deleted vary depending on an antibody-producing B cell used. The isoform is preferably an HDAC2 gene (for details, see Patent Literature 2).

Also disclosed herein is an antibody-producing cell library consisting of the chicken B cells described herein. The present cell library means a population of the chicken B cells as described herein that produce antibodies reacting with various antigens. The chicken B cell as described herein is a cell, in which variation is generated in the variable region sequence of an antibody produced by the cell, by insertion of DNA sequences derived from a human-derived antibody variable regions into a chicken antibody pseudogene locus, etc., and the sequence of the antibody variable region is further diversified by a treatment of relaxing chromatin, so that the cell can have an ability to produce antibodies reacting with various antigens.

Therefore, a population comprising a plurality of the chicken B cells as described herein can be a cell population producing antibodies reacting with various antigens.

Also disclosed is a method for producing an antibody from the herein described chicken B cell of t, and a produced antibody.

The antibody produced from the herein described chicken B cell is a humanized antibody, and more preferably a human antibody. The humanized antibody means an antibody, in which a part of a sequence derived from a chicken gene is comprised in the amino acid sequence of the heavy chain or light chain of a produced antibody, and the human antibody means an antibody, in which the entire amino acid sequence of the heavy chain or light chain of a produced antibody is a sequence derived from a human gene.

When the chicken B cell as described herein is cultured under culture conditions suitable for the cell, it produces a humanized antibody or a human antibody. It has been known that, in a cell line derived from chicken B cells such as DT40 cells, gene conversion occurs in an antibody gene locus thereof, although it does not occur with high efficiency. Thus, such gene conversion occurs also in the chicken B cell described herein, and primary variation in the produced antibodies is achieved by gene conversion occurring in an antibody gene locus, into which a human-derived antibody gene has been inserted. Moreover, by relaxing chromatin in the herein described chicken B cell , the gene conversion efficiency in the antibody gene locus is further increased, so that a population of chicken B cells capable of producing more various antibodies can be prepared. In order to select chicken B cells producing antibodies exhibiting desired antigen specificity by utilizing the relaxing of chromatin, an ADLib system can be used. The above-mentioned antibody-producing cell library can also be prepared by utilizing the ADLib system (see Patent Literature 1 or Non Patent Literature 1).

Furthermore, in particular, as a method of obtaining a desired antibody reacting with a membrane-bound protein, an ADLib axCELL (antigen expressing cell) method can be utilized (WO2010/064454).

Also disclosed is a kit for producing the chicken B cell as described herein or producing an antibody.

The kit for producing the chicken B cell comprises: a medium, supplements and the like that are necessary for the culture of the chicken B cell; a vector used for inserting a DNA sequence derived from a human antibody gene into the antibody gene locus of the chicken B cell, or replacing the chicken B cell antibody gene locus with the DNA sequence derived from the human antibody gene; and reagents. In addition, the kit for producing the chicken B cell may also comprise: elements necessary for preparing an antibody-producing cell library, such as an HDAC inhibitor; and reagents necessary for reducing or deleting the function of the HDAC genes.

On the other hand, the kit for producing an antibody may comprise, as reagents for selecting an antibody having desired antigen specificity, all types of items that are considered necessary for production of the antibody. Examples of such reagents include various antigens, magnetic beads, a reagent for preparing such magnetic beads, a labeled antibody used to select an antibody, and a plate necessary for examinations such as ELISA, which are.

### Examples

### Confirmation of gene conversion

### 1. Preparation of vectors

### 1-1. Replacement of constant region and variable region of chicken antibody light chain (Igλ) (human LC KI vector)

For replacement of the constant region and variable region of chicken Igλ, a targeting vector as shown in Figure 1 was prepared. Methods for preparing individual parts will be described below.

### (1) Variable region and constant region of human Igλ

cDNA prepared from a human Burkitt's lymphoma cell line Ramos was used as a template, and PCR was carried out using a sense primer
(TCCACCATGGCCTGGGCTCTG) (SEQ ID NO: 1) and an antisense primer (GTTGAGAACCTATGAACATTCTGTAGGGGCCAC) (SEQ ID NO: 2), so that the variable region and constant region of a human antibody light chain (Igλ) were amplified. The amplified regions were cloned into pGEM-T-Easy (Promega) to obtain pGEM-hIgG1-LC. This plasmid pGEM-hIgG1-LC was used as a template, and PCR was carried out using a sense primer
(ATTGGCGCGCCTCTCCAGGTTCCCTGGTGCAGGCACAGTCTGCCCTGACTC AGC) (SEQ ID NO: 3) and an antisense primer
(TTCCATATGAGCGACTCACCTAGGACGGTCAGCTTGGTCC) (SEQ ID NO: 4), so as to obtain the variable region (SEQ ID NO: 5) of human Igλ.

Moreover, the pGEM-hIgG1-LC was used as a template, and PCR was carried out using a sense primer
(ATTGGCGCGCCTCTGCCTCTCTCTTGCAGGTCAGCCCAAGGCTGCCCCCTC) (SEQ ID NO: 6) and an antisense primer
(GGAATTCCATATGGAGTGGGACTACTATGAACATTCTGTAGGGG) (SEQ ID NO: 7), so as to obtain the constant region (SEQ ID NO: 8) of human Igλ.

### (2) Left Arm

The genomic DNA of a chicken B cell line DT40 was used as a template, and PCR was carried out using a sense primer (GAGATCTCCTCCTCCCATCC) (SEQ ID NO: 9) and an antisense primer (CAAAGGACACGACAGAGCAA) (SEQ ID NO: 10), so that a region ranging from upstream of the variable region of chicken Igλ to downstream of the constant region thereof was amplified. A functional allele was separated from a non-functional allele by electrophoresis. The functional allele with a size of approximately 8.0 kbp was cut out, and it was then cloned into pGEM-T-Easy to obtain pGEM-cIgM-LCgenome. Using the pGEM-cIgM-LCgenome as a template, PCR was carried out with a sense primer
(TGTCTCGAGTGAAGGTCACCAAGGATGG) (SEQ ID NO: 11) and an antisense primer (TTAAGCTTGGAGAGGAGAGAGGGGAGAA) (SEQ ID NO: 12), so as to obtain Left Arm shown in SEQ ID NO: 13.

### (3) Center Arm

Based on the sequence analysis of the pGEM-cIgM-LCgenome, the sequence of an intron region located between the variable region and the constant region of chicken Igλ was determined, and Center Arm shown in SEQ ID NO: 14 (wherein a blasticidin resistance gene having loxP sequences at both ends was inserted into the midstream thereof) was then synthesized.

### (4) Right Arm

The sequence of a region downstream of the constant region of chicken Igλ was determined using the pGEM-cIgM-LCgenome, and Right Arm shown in SEQ ID NO: 15 (wherein a neomycin resistance gene having lox2272 sequences at both ends was inserted into the midstream thereof) was then synthesized.

### (5) Construction of targeting vector

The above-described Left Arm, human Igλ variable region, Center Arm, human Igλ, constant region, and Right Arm were incorporated into pBluescript to have the structure shown in Figure 1, so as to obtain a human LC KI vector. The gene sequence of this vector is shown in SEQ ID NO: 16.

### 1-2. Knocking-in into downstream of chicken antibody light chain (Igλ) pseudogene region (KI-C-IN vector)

In order to insert a sequence for confirming gene conversion (a GC confirmation sequence; a human pseudogene consisting of a sequence derived from the variable region of human Igλ) (SEQ ID NO: 17) downstream of the pseudogene region of chicken Igλ, a targeting vector as shown in Figure 2 was first produced. Methods for preparing individual parts will be described below.

### (1) Left Arm

The genomic DNA of DT40 was used as a template, and a 4.3-kb region (pseudogenes (pV) 1 to 3) located upstream of the promoter in the variable region was amplified by PCR using a sense primer (TTCTGTGAGCTGAGAAAAGGAGTGTA) (SEQ ID NO: 18) and an antisense primer (CCTGCATTGTGGCACAGCGGGGTT) (SEQ ID NO: 19), and it was then cloned into pCR4blunt-TOPO (Life Technologies) to obtain pCR4-cIgM-LC pV1 genome. Based on this sequence, a region comprising pV1-3 (SEQ ID NO: 20) was cloned, so as to obtain Left Arm.

### (2) Right Arm

Based on the sequence of the pCR4-cIgM-LCpV1 genome obtained in the above section, a portion that is a region between pV1 and the promoter (SEQ ID NO: 21) was cloned, so as to obtain Right Arm.

### (3) Construction of targeting vector

The above-described Left Arm, neomycin resistance gene, and Right Arm were incorporated into the pCR4blunt-TOPO to have the structure shown in Figure 2, so as to obtain a KI-C-IN vector. The gene sequence of this vector is shown in SEQ ID NO: 22.

### 1-3. Insertion of GC confirmation sequence downstream of pseudogene region of chicken antibody light chain (Igλ) (KI-C-IN-C vector)

In order to insert a GC confirmation sequence downstream of the pseudogene region of chicken Igλ, a targeting vector as shown in Figure 2 was produced. Methods for preparing individual parts will be described below.

### (1) Sequence for confirmation of GC

Based on the variable region of human Igλ cloned in 1-1 above, a mutant sequence, in which 3 nucleotides were inserted into each CDR, and a mutant sequence, in which 3 nucleotides were deleted from each CDR, were designed, and these mutant sequences were then ligated to each other using the untranslated region sequence of a chicken Igλ pseudogene region to synthesize a sequence for confirmation of GC (SEQ ID NO: 17).

### (2) Construction of targeting vector

The sequence for confirmation of GC was incorporated into the KI-C-IN vector prepared in 1-2 above to have the structure shown in Figure 2, so as to obtain a KI-C-IN-C vector. The gene sequence of this vector is shown in SEQ ID NO: 23.

### 1-4. Deletion of pseudogene region of chicken antibody light chain (Igλ) (KI-C-DE vector)

In order to delete the pseudogene region of chicken Igλ, a targeting vector as shown in Figure 3 was produced. Methods for preparing individual parts will be described below.

### (1) Left Arm

The genomic DNA of DT40 was used as a template, and an approximately 3.0-kb region located upstream of pV25 was amplified by PCR using a sense primer (CGCTTTGTACGAACGTTGTCACGT) (SEQ ID NO: 24) and an antisense primer (TACCTGAAGGTCTCTTTGTGTTTTG) (SEQ ID NO: 25), and it was then cloned into the pCR4blunt-TOPO to obtain Left Arm shown in SEQ ID NO: 26.

### (2) Right Arm

The same Right Arm as that prepared in 1-2 above was used.

### (3) Construction of targeting vector

The above-described Left Arm, neomycin resistance gene, and Right Arm were incorporated into the pCR4blunt-TOPO to have the structure shown in Figure 3, so as to obtain a KI-C-DE vector. The gene sequence of this vector is shown in SEQ ID NO: 27.

### 1-5. Replacement of pseudogene region of chicken antibody light chain (Igλ) with sequence for confirmation of GC (KI-C-DE-C vector)

In order to replace the pseudogene region of chicken Igλ, with a sequence for confirmation of GC, a targeting vector as shown in Figure 3 was produced. Methods for preparing individual parts will be described below.

### (1) GC confirmation sequence

The GC confirmation sequence prepared in 1-2 above was used.

### (2) Construction of targeting vector

The above-described GC confirmation sequence was incorporated into the KI-C-DE vector produced in 1-3 above to have the structure shown in Figure 3, so as to obtain a KI-C-DE-C vector. The gene sequence of this vector is shown in SEQ ID NO: 28.

### 1-6. Knocking-in into constant region and variable region of chicken antibody heavy chain (IgM)

In order to substitute a region ranging from the variable region of chicken IgM (IgM) to the constant region µ1 thereof with a human antibody heavy chain (IgG₁), a targeting vector as shown in Figure 4 was produced. Methods for preparing individual parts will be described below.

### (1) Human IgG₁ constant region

Among sequences obtained by subjecting human genome chromosome 14 32.33 comprising human IgG₁ to shotgun sequencing, a sequence corresponding to IgG₁ was obtained (Accession No.: NW_001838121.1). The sequences of CHI corresponding to the "C region" of "IGH1," a hinge, CH2, CH3 and a transmembrane domain were extracted from a sequence between the nucleotide 41520 and the nucleotide 43117, and a sequence was then designed by replacing the intron with a mouse IgG₂ₐ-derived sequence. Thereafter, a human IgG₁ constant region sequence (SEQ ID NO: 29) was obtained by synthesis.

### (2) Human IgG₁ variable region

Based on the sequence of a human germ cell-derived IgG₁ variable region gene, a sequence was designed by ligating VH3-23, D5-12, and JH1 sequences to one another, and a chicken-derived secretory signal sequence or splicing signal sequence was then added to the sequence to prepare a human IgG₁ variable region (SEQ ID NO: 30).

### (3) Left Arm

The genomic DNA of DT40 was used as a template, and a region ranging from a region upstream of a chicken IgM variable region to the variable region was amplified by PCR using a sense primer (TTCCCGAAGCGAAAGCCGCGT) (SEQ ID NO: 31) and an antisense primer (ACTCACCGGAGGAGACGATGA) (SEQ ID NO: 32), and an approximately 4.1-kbp fragment was then cloned, so as to obtain pCR4 Blunt-TOPO-cIgM-HC pV1 genome. Based on this sequence, Left Arm (SEQ ID NO: 33), to the 3' terminal side of which a Vlox sequence was added, was designed and synthesized.

### (4) Center Arm 1

Based on the approximately 4.1-kbp sequence ranging from the region upstream of the chicken IgM variable region to the variable region, which had been cloned upon the above-described cloning of the Left Arm, Center Arm 1 corresponding to an approximately 1.4-kbp region upstream of the chicken IgM variable region was synthesized (SEQ ID NO: 34).

### (5) Center Arm 2

The genomic DNA of DT40 was used as a template, and an approximately 4.3-kbp region located downstream of the chicken IgM variable region was amplified by PCR using a sense primer (GGGGATCCTGGGTCAGTCGAAGGGGGCG) (SEQ ID NO: 35) and an antisense primer
(GTGCGGCCGCCAAAAGCGGTAAAATCCACCC) (SEQ ID NO: 36), and it was then cloned, so as to obtain Center Arm 2 shown in SEQ ID NO: 37.

### (6) Right Arm

The genomic DNA of DT40 was used as a template, and an approximately 0.9-kbp region comprising a chicken IgM constant region µ2 was amplified by PCR using a sense primer (CCAAACCACCTCCTGGTGTCC) (SEQ ID NO: 38) and an antisense primer (CAAACCAAAACTACGGATTCTCTGACC) (SEQ ID NO: 39), and it was then cloned, so as to obtain Right Arm shown in SEQ ID NO: 40. (7) Construction of targeting vector

The above-described Left Arm, multi-cloning site, Center Arm1, human IgG₁ variable region, blasticidin resistance gene, Center Arm 2, human IgG₁ constant region, neomycin resistance gene, and Right Arm were incorporated into pBluescript to have the structure shown in Figure 4, so as to obtain a human HC V-C vector. The gene sequence of this vector is shown in SEQ ID NO: 41.

### 1-7. Knocking-in into constant region, variable region, and pseudogene region of chicken antibody heavy chain (IgM) (human HC KI vector)

In order to insert a sequence for confirming gene conversion (a GC confirmation sequence; a human pseudogene consisting of a human Igγ variable region-derived sequence), a human IgG₁ variable region, and a human IgG₁ constant region into a region ranging from a region downstream of the pseudogene region of chicken IgM to a constant region µ1 thereof, a targeting vector as shown in Figure 4 was produced. Methods for preparing individual parts will be described below.
(1) Human IgG₁ constant region
   The human IgG₁ constant region sequence described in 1-6 above was used.
(2) Human IgG₁ variable region
   The human IgG₁ variable region described in 1-6 above was used.
(3) Sequence for confirming gene conversion
   Based on the variable region of human Igγ designed in 1-6 above, a mutant sequence was designed by deleting one nucleotide from CDR1, another mutant sequence was designed by deleting two nucleotides from CDR2, and another mutant sequence was designed by inserting one nucleotide into CDR3. These mutant sequences were ligated to one another using the untranslated region sequence of a chicken Igγ pseudogene region to synthesize a GC confirmation sequence (SEQ ID NO: 42).
(4) Left Arm
   The Left Arm described in 1-6 above was used.
(5) Center Arm 1
   The Center Arm 1 described in 1-6 above was used.
(6) Center Arm 2
   The Center Arm 2 described in 1-6 above was used.
(7) Right Arm
   The Right Arm described in 1-6 above was used.
(8) Construction of targeting vector
   The above-described Left Arm, GC confirmation sequence, Center Arm 1, human IgG₁ variable region, blasticidin resistance gene, Center Arm 2, human IgG₁ constant region, neomycin resistance gene, and Right Arm were incorporated into pBluescript to have the structure shown in Figure 4, so as to obtain a human HC KI vector. The gene sequence of this vector is shown in SEQ ID NO: 43.

### 1-8. Construction of vector for expressing Cre recombinase

Since a drug resistance gene has been incorporated into the above-produced vector in such a form that 1oxP sequences have been added to both ends of the gene, Cre recombinase is allowed to act on the vector after completion of the gene transfer, so that this gene can be removed. Hence, a Cre recombinase gene shown in SEQ ID NO: 44 was incorporated into pEGFP-C1 (BD Bioscience, #6084-1) to produce Cre_EGFP-C1 shown in SEQ ID NO: 45.

### 2. Production of cell lines into light chain of which human sequence has been inserted or substituted

As a host, DT40, a cell line derived from chicken B cells, was used. The DT40 was cultured by the following method.

Using a CO₂ thermostatic chamber, the cells were cultured at 39.5°C in the presence of 5 % CO₂. IMDM (Life Technologies) was used as a medium, and 9 % FBS, 1 % chicken serum, 100 units/mL penicillin, 100 µg/mL streptomycin, and 50 µM monothioglycerol were added to the medium, and the thus obtained medium was then used. Hereinafter, unless otherwise specified, the "medium" indicates a medium having the aforementioned composition.

In order to evaluate gene conversion in a light chain, five types of cell lines shown in the following Table 1 were prepared.

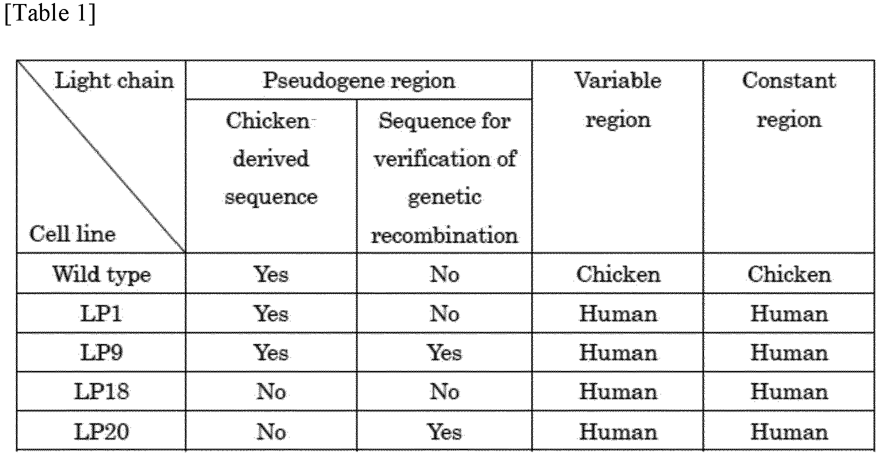

Methods for producing these cell lines will be described below.

### 2-1. Production of cell lines in which variable region and constant region have been replaced with human-type regions

The human LC KI vector produced in 1-1 above was linearized with the restriction enzyme NotI, and it was then introduced into DT40 cells by electroporation. The resulting cells were cultured in a medium, to which 2 mg/mL G418 and 10 µg/mL blasticidin had been added, for 7 to 10 days. Thereafter, the growing cells were subjected to genotyping by PCR, and a cell line in which gene substitution had occurred in a gene locus of interest was selected. The Cre_pEGFP-C1 produced in 1-8 above was introduced into the selected cell line, and the resulting cell line was then subjected to single cell sorting by flow cytometry, so as to select GFP-positive cells. Thereafter, the GFP-positive cells were subjected to genotyping by PCR, so as to obtain a cell line 37-3, in which the removal of the drug resistance genes could be confirmed. Thereafter, the KI-C-IN vector produced in 1-2 above was linearized with the restriction enzyme NotI, and it was then introduced into the cell line 37-3. The cells were cultured in a medium, to which 2 mg/mL G418 had been added, for 7 to 10 days. Thereafter, the growing cells were subjected to genotyping by PCR, so as to obtain a cell line LP1, in which gene insertion had occurred in a gene locus of interest (Figure 5).

### 2-2. Production of cell line in which variable region and constant region have been replaced with human-type regions, and further, in which sequence for confirming gene conversion (GC sequence) has been inserted downstream of chicken pseudogene region

The KI-C-IN-C vector produced in 1-3 above was linearized with the restriction enzyme NotI, and it was then introduced into the cell line 37-3 obtained in the above section. The resulting cells were cultured in a medium, to which 2 mg/mL G418 had been added, for 7 to 10 days. Thereafter, the growing cells were subjected to genotyping by PCR, so as to obtain a cell line LP9, in which gene insertion had occurred in a gene locus of interest (Figure 6).

### 2-3. Production of cell line in which variable region and constant region have been replaced with human-type regions, and further, in which chicken pseudogene region has been deleted

The KI-C-DE vector produced in 1-4 above was linearized with the restriction enzyme NotI, and it was then introduced into the cell line 37-3 obtained in 2-1 above. The resulting cells were cultured in a medium, to which 2 mg/mL G418 had been added, for 7 to 10 days. Thereafter, the growing cells were subjected to genotyping by PCR, so as to obtain a cell line LP18, in which gene insertion had occurred in a gene locus of interest (Figure 7).

### 2-4. Production of cell line in which variable region and constant region have been replaced with human-type regions, and further, in which chicken pseudogene region has been replaced with sequence for confirming gene conversion (GC sequence)

The KI-C-DE-C vector produced in 1-5 above was linearized with the restriction enzyme NotI, and it was then introduced into the cell line 37-3 obtained in 2-1 above. The resulting cells were cultured in a medium, to which 2 mg/mL G418 had been added, for 7 to 10 days. Thereafter, the growing cells were subjected to genotyping by PCR, so as to obtain a cell line LP20, in which gene substitution had occurred in a gene locus of interest (Figure 8).

### 3. Confirmation of antibody expression (flow cytometry and ELISA)

### 3-1. Analysis by flow cytometry

An antibody expressing on the surface of cells of each of the cell lines produced in 2 above was analyzed. Each cell line was cultured, and the cultured cells were then dispensed on a 96-well plate (Nunc, 249662) in an amount of 5 × 10⁵ cells/well. The cells were washed with 150 µL of FACS buffer (PBS containing 0.3 % bovine serum albumin (BSA)) twice, and 50 µL of a primary antibody solution, which had been prepared by diluting Goat anti-Chicken IgM-FITC conjugate (Bethyl, A30-102F-20), Goat Anti-Human IgG (Gamma chain specific) R-PE conjugate (Southern biotech, A2040-09) or Goat Anti-human lambda PE conjugate (Southern biotech, 2070-09) with FACS buffer, 1000 times, 500 times, and 500 times, respectively, was added to the resulting cells. The mixture was incubated on ice in a dark place for 30 minutes, and it was then washed with 150 µL of FACS buffer twice. Thereafter, 200 µL of a PI solution, which had been 1000 times diluted with FACS buffer, was added to the reaction mixture, and was then suspended therein. This suspension was subjected to a flow cytometric analysis, and the expression of chicken IgM and human Igγ (heavy chain) or human Igλ (light chain) was analyzed. As a result, it was confirmed that, as shown in Figure 9, only chicken IgM was expressed in wild-type DT40, whereas chicken IgM and human Igλ, were expressed in the transformed cell lines LP1, LP9, LP18, and LP20. In all of the cell lines, the expression of Igγ that had not undergone gene transfer was not observed.

### 3-2. Analysis by ELISA

The concentrations of chicken IgM and human IgG₁, which were secreted from the cells produced in 2 above into culture supernatants, were measured. Individual cell lines were suspended in a medium containing no chicken serum to a concentration of 4 × 10⁵ cells/mL, and 2 mL each of the suspension was then plated on each well of a 24-well plate, followed by performing a culture for 3 days. Thereafter, the culture solution was recovered, was then filtrated through a 0.22-µm filter, and was then used in the measurement.

The method of measuring IgG is as follows. 20 µL of 1.0 µg/mL Goat anti-Human IgG-Fc affinity purified (Bethyl, A80-104A) was dispensed into an immuno 384-well plate Maxisorp (Nunc, 464718) , and it was then reacted at room temperature for 1 hour or more, so that it was immobilized on the plate. Thereafter, the resultant on the plate was washed with a washing solution (PBS containing 0.05% Tween 20) five times, 50 µL of a blocking solution (PBS containing 1% BSA) was then added thereto, and the mixture was then reacted at room temperature for 30 minutes. Thereafter, the reaction mixture was washed with a washing solution five times, 20 µL of a measurement sample or Human IgG1 Lambda-UNLB (Southern Biotech, 0151L-01) serving as a standard substance was then added thereto, and the obtained mixture was then reacted at room temperature for 1 hour. Thereafter, the reaction mixture was washed with a washing solution five times, 20 µL of Goat anti-Human IgG-Fc HRP conjugated (Bethyl, A80-104P), which had been 1000 times diluted with PBS containing 1 % BSA and 0.05% Tween 20, was then added thereto, and the obtained mixture was then reacted at room temperature for 1 hour. Thereafter, the reaction mixture was washed with a washing solution five times, 20 µL of TMB+ (Dako, S159985) was then added thereto, and the obtained mixture was subjected to a coloring reaction at room temperature for 3 minutes. Subsequently, 20 µL of 1 N sulfuric acid was added to the reaction mixture to termination the reaction. Using Infinite M1000 (TECAN), the absorbance at 450 nm was measured.

For the measurement of IgM, the system of AlphaLISA Immunoassay (Perkin Elmer) was used. Goat anti-chicken IgM antibody (Bethyl, A30-102A) was labeled with biotin, employing Chromalink^{™} Biotin Labeling Reagent (SoluLink Inc., #B1001-105), so that a biotinylated antibody was prepared. Subsequently, Goat anti-chicken IgM antibody (Bethyl, A30-102A) was allowed to bind to Unconjugated AlphaLISA Acceptor Beads (Perkin Elmer), so that antibody-bound Alpha Screen beads were prepared. These biotin-labeled antibody and antibody-bound Alpha Screen beads were each diluted with an assay buffer (PBS containing 1% BSA) to concentrations of 4.5 nM and 50 µg/mL, respectively. Thereafter, they were mixed with each other in equal amounts, so as to prepare a chicken IgM concentration measurement solution. 20 µL of the chicken IgM concentration measurement solution was mixed with 5 µL of a measurement sample or the chicken serum (GIBCO, 16110) serving as a standard substance on a 96-well plate (Nunc), and the mixed solution was then dispensed into Alpha Plate-384 (Perkin Elmer) in an amount of 12.5 µL each. The mixed solution was reacted at room temperature for 60 minutes, and thereafter, 12.5 µL of AlphaScreen Streptavidin Donor Beads (Perkin Elmer), which had been adjusted to a concentration of 80 µg/mL with an assay buffer, was added to the reaction solution, and the obtained mixture was then reacted at room temperature under light shielding conditions for 30 minutes. Thereafter, using EnSpire (Perkin Elmer), an analysis was carried out.

The measurement results are shown in Table 2. It was confirmed that, as with the wild-type DT40, chicken IgM was secreted also in the transformed cell lines LP1, LP9, LP18 and LP20.

**[Table 2]**

| Cell line | IgG (µg/mL) IgM (µg/mL) | |
|---|---|---|
| Wild type | <0.01 | 2.30 |
| LP1 | <0.01 | 0.09 |
| LP9 | <0.01 | 0.10 |
| LP18 | <0.01 | 0.09 |
| LP20 | <0.01 | 0.03 |

### 4. Confirmation of gene conversion

The cell lines, in which the expression of human Igλ (light chain) had been confirmed in 3 above, were cultured in a medium containing 2.5 ng/mL TSA for 20 days, and genomic DNA was then extracted from 1 × 10⁶ cells. This genomic DNA was used as a template, and a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47). Thereafter, the resultant was purified by agarose gel extraction, and was then concentrated using QIAGEN PCR Purification Kit (QIAGEN). Using the Next-Generation Sequencing Analysis System of Roche Diagnostics, the sequence of this sample was analyzed, and the presence or absence of gene conversion was evaluated based on whether or not a gene sequence identical to the inserted human pseudogene sequence would be present in the variable region. The results are shown in Table 3. It was confirmed that gene conversion had not occurred in LP1 and LP18 that did not comprise the human pseudogene sequence, whereas such gene conversion had occurred in LP9 and LP20 having the human pseudogene sequence.

**[Table 3]**

| Cell line | Number of sequences | Number of sequences with genetic recombination | Frequency |
|---|---|---|---|
| LP1 | 785 | 0 | 0.0% |
| LP9 | 344 | 18 | 5.2% |
| LP18 | 378 | 0 | 0.0% |
| LP20 | 634 | 31 | 4.9% |

### 5. Production of cell line, in which human sequence has been inserted and/or substituted into heavy chain

In order to evaluate gene conversion in a heavy chain, the three types of cell lines shown in Table 4 were prepared.

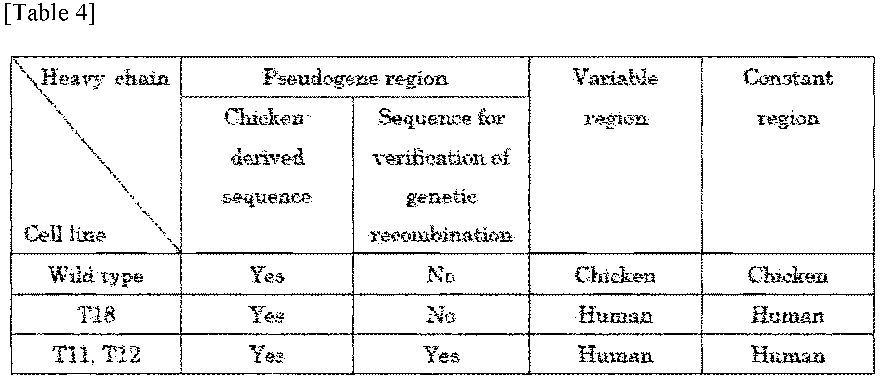

Methods for producing these cell lines will be described below.

### 5-1. Production of cell line in which variable region and constant region of heavy chain have been replaced with human-type regions

The human HC V-C vector produced in 1-6 above was linearized with the restriction enzyme SalI, and it was then introduced into the cell line 37-3 obtained in 2-2 above. The resulting cells were cultured in a medium, to which 2 mg/mL G418 and 10 µg/mL blasticidin had been added, for 7 to 10 days. Thereafter, the growing cells were subjected to genotyping by PCR, and a cell line T18 in which gene substitution had occurred in a gene locus of interest was obtained (Figure 10).

### 5-2. Production of cell line in which variable region and constant region of heavy chain have been replaced with human-type regions, and sequence for confirming gene conversion (GC confirmation sequence) has been inserted downstream of pseudogene region

The human HC KI vector produced in 1-7 above was linearized with the restriction enzyme SalI, and it was then introduced into the cell line 37-3 obtained in 2-2 above. The resulting cells were cultured in a medium, to which 2 mg/mL G418 and 10 µg/mL blasticidin had been added, for 7 to 10 days. Thereafter, the growing cells were subjected to genotyping by PCR, and cell lines T11 and T12, in which gene substitution had occurred in a gene locus of interest was obtained (Figure 11).

### 6. Confirmation of antibody expression (flow cytometry and ELISA)

### 6-1. Analysis by flow cytometry

Based on the method described in 3-1 above, the expression of an antibody in the cells produced in 5 above was confirmed. The results are shown in Figure 12. It was confirmed that only chicken IgM was expressed in wild-type DT40, whereas the expression of chicken IgM disappeared and instead, human Igγ and Igλ were expressed in the transformed cell lines T18, T11 and T12.

### 6-2. Analysis by ELISA

Based on the method described in 3-2 above, the antibodies secreted from the cells produced in 5 above into a culture supernatant were measured. The results are shown in Table 5. As with 6-1 above, it was confirmed that only chicken IgM was secreted from wild-type DT40, whereas human IgG₁ was secreted from T18, T11 and T12, into which human IgG₁ had been introduced.

**[Table 5]**

| Cell line | IgG (µg/mL) | IgM (µg/mL) |
|---|---|---|
| Wild type | <0.01 | 2.30 |
| T18 | 1.00 | 0.03 |
| T11 | 0.40 | <0.01 |
| T12 | 0.69 | <0.01 |

### 7. Confirmation of gene conversion

Using the cell lines in which the expression of an antibody had been confirmed in 6 above, gene conversion was confirmed. The genomic DNA extracted by the method described in 4 above was used as a template, and a heavy chain variable region was amplified by PCR using a sense primer
(CTATGCGCCTTGCCAGCCCGCTCAGTCCGTCAGCGCTCTCT) (SEQ ID NO: 48) and an antisense primer to which an identification tag had been added
(CGTATCGCCTCCCTCGCGCC ATCAGNNNNNNNNNNCGGTGACCAGGGTGCCCTG) (SEQ ID NO: 49).

Thereafter, a sequence analysis was carried out based on the method described in 4 above. The results are shown in Table 6. It was confirmed that gene conversion had not occurred at all in T18 containing no human pseudogenes, whereas such gene conversion had occurred in T11 and T12 having such a human pseudogene sequence.

**[Table 6]**

| Cell line | Number of sequences | Number of sequences with genetic recombination | Frequency |
|---|---|---|---|
| T18 | 1125 | 0 | 0.0% |
| T11 | 3038 | 415 | 13.4% |
| T12 | 4108 | 305 | 7.4% |

### Production of L5/H5 cell line, in which pseudogene region consisting of five human variable region-derived sequences has been inserted into each of light chain and heavy chain

### 8. Production of vectors

### 8-1. Knocking-in into chicken antibody (Igλ) pseudogene region

In order to substitute the pseudogene region of chicken Igλ with a pseudogene consisting of a sequence derived from the variable region of human Igλ (a human light chain pseudogene), a targeting vector as shown in Figure 13 was produced. The production method will be described below.

### (1) Designing and production of human antibody light chain pseudogene sequence (pVL5)

Based on the gene sequences of immunoglobulin λ light chain variable regions, which had not caused V(D)J recombination in the human genome in the database, five sequences were selected from each of CDR1, CDR2 and CDR3, and the framework sequence of the Igλ variable region cloned in 1-1 above was then combined therewith, so as to design five sequences, namely, pVL001 (SEQ ID NO: 69), pVL002 (SEQ ID NO: 70), pVL003 (SEQ ID NO: 71), pVL004 (SEQ ID NO: 72) and pVL005 (SEQ ID NO: 73). Thereafter, a human light chain pseudogene sequence pVL5 (SEQ ID NO: 50) comprising these sequences was designed, and was then subjected to gene synthesis.

### (2) Production of targeting vector

On the basis of the KI-C-DE vector produced in 1-4 above, the GC confirmation sequence was replaced with the human antibody light chain pseudogene sequence pVL5 described in the above section to produce a human LCpV KI vector. The gene sequence of this vector is shown in SEQ ID NO: 51.

### 8-2. Substitution of chicken antibody heavy chain (IgM) constant region

In order to substitute the heavy chain constant region of chicken IgM with human IgG₁, a targeting vector as shown in Figure 14 was produced. The production method will be described below.

### (1) Human IgG₁ constant region

The human IgG₁ constant region produced in 1-6 above was used.

### (2) Left Arm

The Center Arm 2 produced in 1-6 above was used.

### (3) Right Arm

The Right Arm produced in 1-6 above was used.

### (4) Production of targeting vector

The above-described Left Arm, human IgG₁ constant region sequence, neomycin resistance gene, and Right Arm were incorporated into pBluescript to produce a human CH KI vector. The gene sequence of this vector is shown in SEQ ID NO: 52.

### 8-3. Insertion into chicken IgM variable and pseudogene regions

### (1) Human IgG₁ variable region

The human IgG₁ variable region produced in 1-6 above was used.

### (2) Designing and production of human antibody heavy chain pseudogene sequence (pVH5)

Based on the sequences of human immunoglobulin heavy chain variable regions in the database, five sequences were selected from each of CDR1, CDR2 and CDR3, and the framework sequence of the variable region cloned in 1-6 above was then combined therewith, so as to design five sequences, namely, pVH001 (SEQ ID NO: 89), pVH002 (SEQ ID NO: 90), pVH003 (SEQ ID NO: 91), pVH004 (SEQ ID NO: 92) and pVH005 (SEQ ID NO: 93). Thereafter, a human antibody heavy chain pseudogene sequence pVH5 (SEQ ID NO: 53) comprising these sequences was designed, and was then subjected to gene synthesis.

### (3) Left Arm

The Left Arm produced in 1-6 above was used.

### (4) Center Arm

The Center Arm 1 produced in 1-6 above was used.

### (5) Right arm

The Center Arm 2 produced in 1-6 above was used. The confirmed sequence of the Right Arm is shown in SEQ ID NO: 54.

### (6) Production of targeting vector

The above-described Left Arm, human antibody heavy chain pseudogene sequence pVH5, Center Arm, human IgG₁ variable region sequence, blasticidin resistance gene, and Right Arm were incorporated into pBluescript to obtain a human pVH VH KI vector. The gene sequence of this vector is shown in SEQ ID NO: 55.

### 9. Production of L5/H5 cell line, in which five human pseudogenes have been introduced into each of gene loci of chicken antibody light chain and heavy chain

The human CH KI vector produced in 8-2 above, the human pVH VH KI vector produced in 8-3 above, and the human LC pV KI vector produced in 8-1 above were introduced into the cell line 37-3 obtained in 2-2 above, so as to produce a L5/H5 cell line (Figure 15).

The human CH KI vector, which had been linearized with the restriction enzyme SalI, was introduced into the cell line 37-3, and the cells were then cultured in a medium containing 2 mg/mL G418. The growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the human pVH VH KI vector, which had been linearized with the restriction enzyme SalI, was introduced into the cell line 37-3, and the cells were then cultured in a medium containing 10 µg/mL blasticidin. The growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Finally, the human LC pV KI vector, which had been linearized with the restriction enzyme NotI, was introduced into the cell line 37-3, and the cells were then cultured in a medium containing 2 mg/mL G418. The growing cells were subjected to genotyping by PCR, so as to obtain cell lines B-B10 and J-D3, in each of which substitution had occurred in a gene locus of interest (Figure 16 and Figure 17).

### 10. Confirmation of antibody expression (flow cytometry and ELISA)

### 10-1. Analysis by flow cytometry

Based on the method described in 3-1 above, the expression of an antibody in the cells produced in 9 above was confirmed. The results are shown in Figure 18. It was confirmed that only chicken IgM was expressed in wild-type DT40, whereas the expression of chicken IgM disappeared and instead, human Igγ and Igλ were expressed in the transformed cell lines B-B10 and J-D3.

### 10-2. Analysis of ELISA

Based on the method described in 3-2 above, the antibodies secreted from the cells produced in 9 above were measured. The results are shown in Table 7. As with 10-1 above, it was confirmed that only chicken IgM was secreted from wild-type DT40, whereas human IgG₁ was secreted from the transformed cell lines B-B10 and J-D3.

**[Table 7]**

| Cell line | IgG (µg/mL) | IgM (µg/mL) |
|---|---|---|
| Wild type | <0.01 | 2.55 |
| B-B10 | 2.19 | 0.01 |
| J-D3 | 3.45 | <0.01 |

### 11. Confirmation of gene conversion

Using the cell lines in which the expression of an antibody had been confirmed in 10 above, gene conversion was confirmed. The genomic DNA extracted from the cells, which had been cultured in the presence of TSA for 42 days, according to the method described in 4 above was used as a template, and a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added
(CGTATCGCCTCCCTCGCGCCATCAGNNNNNNNNNNTGGGGGGGGTTCATA TGAAG) (SEQ ID NO: 57). Thereafter, sequence analyses were carried out according to the method described in 4 above. The results obtained by analyzing the sequences of the light chain and heavy chain variable regions in the cell line B-B10 are shown in Table 8 and Table 9, respectively. A clone comprising the sequence derived from the inserted human pseudogene was confirmed in both of the light chain and heavy chain variable regions, and thus, it was demonstrated that a variety of sequences could be generated as a result of gene conversion. Moreover, it was also confirmed that a variety of sequences could be generated as a result of gene conversion even in the cell line J-D3.

**[Table 8]**

| # | % | CDR1 | CDR2 | CDR3 |
|---|---|---|---|---|
| 1 | 0.183 | VL | pVL003 | VL |
| 2 | 0.046 | Not matched | VL | pVL003 |
| 3 | 0.046 | VL | VL | pVL004 |

| | | | | |
|---|---|---|---|---|
| VL: sequence inserted into variable region pVL001-005: sequences inserted into pseudogene region Not matched: sequence that is not completely matched with either VL or pVL (which is probably caused by partial genetic recombination or mutation) [' Not matched: sequence that is not completely matched with either VH or pVH (which is probably caused by partial genetic recombination or mutation) | | | | |

### Production of L15/H15 cell line, in which 15 human variable region-derived sequences have been inserted into each of chicken antibody light chain and heavy chain gene loci

### 12. Production of vectors

### 12-1. Knocking-in into chicken antibody light chain (Igλ) variable and constant regions

Based on the method described in 1-1 above, a targeting vector, Lambda-VL-CL KI vector 2.0, shown in Figure 19 was produced.

### (1) Variable region and constant region of human Igλ

The variable region and the constant region produced in 1-1 above were used.

### (2) Left Arm

The Left Arm produced in 1-1 above was used.

### (3) Center Arm

The Center Arm produced in 1-1 above was used.

### (4) Right Arm

Based on the sequence of a region downstream of the constant region of a chicken antibody light chain, which was determined in 1-1 above, Right Arm was synthesized. (5) Construction of targeting vector

The above-described Left Arm, human Igλ variable region, Center Arm, human Igλ constant region, and Right Arm were incorporated into pBluescript to have the structure shown in Figure 19, so as to obtain Lambda-VL-CL KI vector 2.0 (SEQ ID NO: 58).

### 12-2. Knock-out of chicken antibody light chain (Igλ) pseudogene

### (1) Human Igλ variable region

The variable region sequence described in 1-1 above was used.

### (2) Left Arm

The Left Arm described in 1-2 above was used.

### (3) Center Arm

The Left Arm described in 1-1 above was used as Center Arm.

### (4) Right Arm

The Center Arm described in 1-1 above was used as Right Arm.

### (5) Multi-cloning site

For insertion of a human antibody light chain pseudogene sequence pVL15, a multi-cloning site shown in SEQ ID NO: 59 was designed. (6) Construction of targeting vector

The above-described Left Arm, neomycin resistance gene, multi-cloning site, Center Arm, human Igλ variable region, blasticidin resistance gene, and Right Arm were incorporated into pUC19, so as to construct a targeting vector pVL KI_pUC19_step5_BrRev (SEQ ID NO: 60), as shown in Figure 19.

### 12-3. Knocking-in into chicken antibody light chain (Igλ) pseudogene and variable regions

### (1) Human antibody light chain pseudogene sequence (pVL15)

Based on the sequences of human immunoglobulin λ variable regions in the database, 15 CDR1, CDR2 and CDR3 sequences were selected, and the framework sequence of the human Igλ variable region cloned in 1-1 above were then combined therewith, so as to design 15 sequences, namely, pVL101 (SEQ ID NO: 74), pVL102 (SEQ ID NO: 75), pVL103 (SEQ ID NO: 76), pVL104 (SEQ ID NO: 77), pVL105 (SEQ ID NO: 78), pVL106 (SEQ ID NO: 79), pVL107 (SEQ ID NO: 80), pVL108 (SEQ ID NO: 81), pVL109 (SEQ ID NO: 82), pVL110 (SEQ ID NO: 83), pVL111 (SEQ ID NO: 84), pVL112 (SEQ ID NO: 85), pVL113 (SEQ ID NO: 86), pVL114 (SEQ ID NO: 87), and pVL115 (SEQ ID NO: 88). A human antibody light chain pseudogene sequence pVL15 (SEQ ID NO: 61) comprising these sequences was designed, and was then subjected to gene synthesis.

### (2) Construction of targeting vector

The above-described human antibody light chain pseudogene sequence pVL15 was incorporated into the multi-cloning site of the pVL

### KI_pUC19_step6 _BrRev produced in 12-2 above, so as to construct a targeting vector pVL KI_pUC19_step6 _BrRev_pVL#odd (SEQ ID NO: 62) as shown in Figure 19.

### 12-4. Knocking-in into chicken antibody heavy chain (IgM) constant region

The human CH KI vector produced in 8-2 above was used.

### 12-5. Knocking-in into chicken antibody heavy chain (IgM) variable region

### (1) Human IgG₁ variable region

The variable region sequence described in 1-6 above was used.

### (2) Left Arm

The Center Arm described in 1-6 above was used.

### (3) Right Arm

The Right Arm described in 1-6 above was used.

### (4) Construction of targeting vector

The above-described Left Arm, human IgG₁ variable region, blasticidin resistance gene, and Right Arm were incorporated into pUC19, so as to produce a targeting vector H(X)0 • hV(B)_074-009/No4 (SEQ ID NO: 63) as shown in Figure 20.

### 12-6. Knocking-in into chicken antibody heavy chain (IgM) pseudogene region

### (1) Human antibody heavy chain pseudogene sequence (pVH15)

Based on the sequences of human immunoglobulin heavy chain variable regions in the database, 15 CDR1, CDR2 and CDR3 sequences were selected, and the framework sequence of the human IgG₁ variable region cloned in 1-6 above was then combined therewith, so as to design 15 sequences, namely, pVH101 (SEQ ID NO: 94), pVH102 (SEQ ID NO: 95), pVH103 (SEQ ID NO: 96), pVH104 (SEQ ID NO: 97), pVH105 (SEQ ID NO: 98), pVH106 (SEQ ID NO: 99), pVH107 (SEQ ID NO: 100), pVH108 (SEQ ID NO: 101), pVH109 (SEQ ID NO: 102), pVH110 (SEQ ID NO: 103), pVH111 (SEQ ID NO: 104), pVH112 (SEQ ID NO: 105), pVH113 (SEQ ID NO: 106), pVH114 (SEQ ID NO: 107), and pVH115 (SEQ ID NO: 108). Thereafter, a human antibody heavy chain pseudogene sequence pVH15 (SEQ ID NO: 64) was designed by ligating these sequences to one another, and was then subjected to gene synthesis.

### (2) Left Arm

The Left Arm described in 1-6 above was used.

### (3) Right Arm

The Center Arm described in 1-6 above was used.

### (4) Multi-cloning site

For insertion of the human antibody heavy chain pseudogene sequence, a multi-cloning site shown in SEQ ID NO: 65 was designed.

### (5) Site-specific recombination using Cre recombinase (RMCE method)

It has been known that if a pseudogene sequence region is prolonged, the transduction efficiency of homologous recombination is reduced. Thus, in order to easily insert a pseudogene sequence using Cre recombinase, an RMCE method was applied. First, a cassette sequence for RMCE comprising a loxm3 rev sequence and a loxm7 rev RE sequence was designed. (6) Construction of targeting vector

The Left Arm, neomycin resistance gene, cassette sequence for RMCE, multi-cloning site, Right Arm, and human IgG₁ variable region were incorporated into pUC19, and thereafter, the human antibody heavy chain pseudogene region pVH15 was incorporated into the multi-cloning site, so as to produce a targeting vector pVH KI_pUC19_step3_pVH#odd_15pVH_RMCE (SEQ ID NO: 66) as shown in Figure 20.

### 13. Production of cell lines

The Lambda-VL-CL KI vector 2.0 prepared in 12-1 above, the pVL KI_pUC19_step5_BrRev prepared in 12-2 above, the pVL
KI_pUC19_step6 _BrRev_pVL#odd prepared in 12-3 above, the human CH KI vector prepared in 12-4 above, the H(X)0 • hV(B)_074-009/No4 prepared in 12-5 above, and the pVH KI_pUC19_step3_pVH#odd_15pVH_RMCE prepared in 12-6 above were each introduced into DT40 cells, so as to produce cell lines.

First, the human CH KI vector, which had been linearized with the restriction enzyme SalI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the Lambda-VL-CL KI vector 2.0, which had been linearized with the restriction enzyme NotI was introduced into DT40, and the obtained mixture was then culture in a medium containing 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Thereafter, the pVL KI_pUC19_step5_BrRev, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Thereafter, the pVL KI_pUC19_step6_BrRev_pVL#odd, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Finally, the H(X)0 • hV(B)_074-009/No4 linearized with the restriction enzyme ScaI and pVH KI_pUC19_step3_pVH#odd_15pVH_RMCE linearized with the restriction enzyme NotI were introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines A12-3, A12-4, A12-5, B7-3, B7-9 and B7-11, from each of which the drug resistance genes had been removed.

### 14. Confirmation of antibody expression (flow cytometry and ELISA)

### 14-1. Analysis by flow cytometry

Based on the method described in 3 above, the expression of an antibody in the cells produced in 13 above was confirmed. The results are shown in Figure 21 and Figure 22. It was confirmed that only chicken IgM was expressed in wild-type DT40, whereas the expression of chicken IgM disappeared and instead, human Igγ and Igλ, were expressed in the transformed cell lines A12-3, A12-4, A12-5, B7-3, B7-9 and B7-11 (Figures 21 and 22). It is to be noted that since the histogram of Igλ in A12-5 was incorrect in Figure 21, Figure 22 including correct data obtained before the priority date was attached herewith.

### 14-2. Analysis by ELISA

Based on the method described in 3-2 above, the antibodies secreted from the cells produced in 13 above were measured. The results are shown in Table 10. As with 10-1 above, it was confirmed that only chicken IgM was secreted from wild-type DT40, whereas human IgG₁ was secreted from the transformed cell lines A12-3, A12-4, A12-5, B7-3, B7-9 and B7-11.

**[Table 10]**

| Cell line | IgG (µg/mL) | IgM (µg/mL) |
|---|---|---|
| Wild type | <0.01 | 2.54 |
| A12-3 | 0.52 | <0.01 |
| A12 4 | 0.51 | <0.01 |
| A12 5 | 0.65 | 0.01 |
| B7 3 | 0.62 | 0.01 |
| B7-9 | 0.76 | 0.02 |
| B7-11 | 0.77 | 0.02 |

### 15. Confirmation of gene conversion

Using the cell lines in which the expression of an antibody had been confirmed in 10 above, gene conversion was confirmed. Using, as a template, the genomic DNA extracted from the cells, which had been cultured for 21 days or 42 days in the presence of TSA, by the method described in 4 above, a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added
(CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line B7-11 cultured in the presence of TSA for 21 days are shown in Table 11 and Table 12, respectively. The results (the top 10) of the sequence analysis performed on the variable regions of the light chain and heavy chain in the cell line B7-11 cultured in the presence of TSA for 42 days are shown in Table 13 and Table 14, respectively. A clone comprising a sequence derived from a human pseudogene was found in the variable regions of both of the light chain and the heavy chain, and it was demonstrated that a variety of sequences were generated as a result of gene conversion. In addition, it was also confirmed that a variety of sequences were generated as a result of gene conversion even in other cell lines.

**[Table 11]**

| # | % | CDR1 | CDR2 | CDR3 |
|---|---|---|---|---|
| 1 | 0.327 | VL | pVL103 | VL |
| 2 | 0.327 | VL | pVL102 | VL |
| 3 | 0.196 | VL | VL | pVL114 |
| 4 | 0.196 | VL | pVL105 | VL |
| 5 | 0.131 | VL | pVL104 | VL |
| 6 | 0.131 | VL | VL | pVL112 |
| 7 | 0.131 | pVL105 | VL | VL |
| 8 | 0.065 | VL | Not matched | pVL106 |
| 9 | 0.065 | VL | pVL109 | VL |
| 10 | 0.065 | VL | VL | pVL110 |
| 11 | 0.065 | VL | pVL107 | VL |

| | | | | |
|---|---|---|---|---|
| VL: sequence inserted into variable region pVL101-115: sequences inserted into pseudogene region Not matched: sequence that is not completely matched with either VL or pVL (which is probably caused by partial genetic recombination or mutation) | | | | |

**[Table 12]**

| # | % | CDR1 | CDR2 | CDR3 |
|---|---|---|---|---|
| 1 | 0.46 | pVH105 | VH | VH |
| 2 | 0.345 | pVH108 | VH | VH |
| 3 | 0.345 | VH | pVH107 | Not matched |
| 4 | 0.345 | pVH107 | VH | VH |
| 5 | 0.345 | pVH111 | pVH111 | VH |
| 6 | 0.46 | pVH112 | Not matched | VH |
| 7 | 0.23 | pVH109 | VH | VH |
| 8 | 0.23 | VH | pVH112 | VH |
| 9 | 0.23 | pVH105 | Not matched | VH |
| 10 | 0.115 | pVH108 | pVH108 | VH |
| 11 | 0.115 | VH | VH | pVH113 |
| 12 | 0.115 | VH | VH | pVH109 |
| 13 | 0.115 | pVH107 | VH | Not matched |
| 14 | 0.115 | pVH103 | Not matched | VH |
| 15 | 0.23 | pVH111 | Not matched | VH |
| 16 | 0.23 | pVH111 | Not matched | Not matched |
| 17 | 0.115 | pVH111 | VH | VH |

| | | | | |
|---|---|---|---|---|
| VH: sequence inserted into variable region pVH101-115: sequences inserted into pseudogene region Not matched: sequence that is not completely matched with either VH or pVH (which is probably caused by partial genetic recombination or mutation) | | | | |

**[Table 13]**

| # | % | CDR1 | CDR2 | CDR3 |
|---|---|---|---|---|
| 1 | 0.175 | VL | pVL109 | VL |
| 2 | 0.175 | pVL105 | VL | VL |
| 3 | 0.175 | pVL105 | pVL105 | VL |
| 4 | 0.116^{*} | VL | pVL105 | VL |
| 5 | 0.116^{*} | VL | pVL105 | VL |
| 6 | 0.116 | Not matched | pVL105 | VL |
| 7 | 0.058 | VL | pVL109 | Not matched |
| 8 | 0.058 | VL | VL | pVL114 |
| 9 | 0.058 | VL | pVL102 | VL |
| 10 | 0.058 | VL | pVL108 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}: Sequences of portions other than CDRs are different, respectively. | | | | |

**[Table 14]**

| # | % | CDR1 | CDR2 | CDR3 |
|---|---|---|---|---|
| 1 | 1.789 | pVH108 | VH | VH |
| 2 | 1.022 | pVH112 | VH | VH |
| 3 | 0.681 | pVH109 | VH | VH |
| 4 | 0.596 | VH | pVH 1 12 | VH |
| 5 | 0.596 | pVH111 | Not matched | VH |
| 6 | 0.511 | pVH107 | VH | VH |
| 7 | 0.426 | pVH111 | pVH111 | VH |
| 8 | 0.341 | pVH111 | VH | VH |
| 9 | 0.256 | pVH108 | pVH108 | VH |
| 10 | 0.256 | VH | pVH107 | Not matched |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) | | | | |

### Production of L15/H30 cell line, in which 15 pseudogene regions each consisting of human variable region-derived sequence have been inserted into light chain and 30 such pseudogene regions have been inserted into heavy chain

### 16. Production of vectors

### 16-1. Knocking-in into chicken antibody light chain (Igλ) variable and constant regions

The Lambda-VL-CL KI vector 2.0 produced in 12-1 above was used.

### 16-2. Knock-out of chicken antibody light chain (Igλ) pseudogene

The pVL KI_pUC19_step5 _BrRev produced in 12-2 above was used.

### 16-3. Knocking-in into chicken antibody light chain (Igλ) pseudogene and variable regions

The pVL KI_pUC19_step6 _BrRev_pVL#odd produced in 12-3 above was used.

### 16-4. Knocking-in into chicken antibody heavy chain (IgM) constant region

The human CH KI vector produced in 8-2 above was used.

### 16-5. Knocking-in into chicken antibody heavy chain (IgM) variable region

### (1) Human IgG₁ variable region

The variable region sequence described in 1-6 above was used.

### (2) Left Arm

The Left Arm described in 1-6 above was used.

### (3) Center Arm

The Center Arm described in 1-6 above was used.

### (4) Right Arm

The Right Arm described in 1-6 above was used.

### (5) Cassette sequence for RMCE

The cassette sequence for RMCE designed in 12-6 above was used.

### (6) Construction of targeting vector

The above-described Left Arm, neomycin resistance gene, cassette sequence for RMCE, Center Arm, human IgG₁ variable region, and Right Arm were incorporated into pUC19, so as to produce a targeting vector pVH KI_pUC19_step5_BsrRev_RMCE_cassette (SEQ ID NO: 109) as shown in Figure 23.

### 16-6. Knocking-in into chicken antibody heavy chain (IgM) pseudogene region

### (1) Human antibody heavy chain pseudogene sequence (pVH30)

Based on the sequences of human immunoglobulin heavy chain variable regions in the database, 30 CDR1, CDR2 and CDR3 sequences were selected, and the framework sequence of the human IgG₁ variable region cloned in 1-6 above was then combined therewith, so as to design 30 sequences, namely, pVH101 (SEQ ID NO: 94), pVH102 (SEQ ID NO: 95), pVH103 (SEQ ID NO: 96), pVH104 (SEQ ID NO: 97), pVH105 (SEQ ID NO: 98), pVH106 (SEQ ID NO: 99), pVH107 (SEQ ID NO: 100), pVH108 (SEQ ID NO: 101), pVH109 (SEQ ID NO: 102), pVH110 (SEQ ID NO: 103), pVH111 (SEQ ID NO: 104), pVH112 (SEQ ID NO: 105), pVH113 (SEQ ID NO: 106), pVH114 (SEQ ID NO: 107), pVH115 (SEQ ID NO: 108), pVH116 (SEQ ID NO: 110), pVH117 (SEQ ID NO: 111), pVH118 (SEQ ID NO: 112), pVH119 (SEQ ID NO: 113), pVH120 (SEQ ID NO: 114), pVH121 (SEQ ID NO: 115), pVH122 (SEQ ID NO: 116), pVH123 (SEQ ID NO: 117), pVH124 (SEQ ID NO: 118), pVH125 (SEQ ID NO: 119), pVH126 (SEQ ID NO: 120), pVH127 (SEQ ID NO: 121), pVH128 (SEQ ID NO: 122), pVH129 (SEQ ID NO: 123), and pVH130 (SEQ ID NO: 124). Thereafter, a human antibody heavy chain pseudogene sequence pVH30 (SEQ ID NO: 125) was designed by ligating these sequences to one another, and was then subjected to gene synthesis.

### (2) Left Arm

The Left Arm described in 1-6 above was used.

### (3) Right Arm

The Right Arm described in 1-6 above was used.

### (4) Multi-cloning site

For insertion of the human antibody heavy chain pseudogene sequence, a multi-cloning site shown in SEQ ID NO: 65 was designed. (5) Construction of targeting vector

The Left Arm, neomycin resistance gene, multi-cloning site, Right Arm, human IgG₁ variable region were incorporated into pUC19, and thereafter, the human antibody heavy chain pseudogene region pVH30 was incorporated into the multi-cloning site, so as to produce a targeting vector pVH
KI_pUC19_step3_pVH#odd_even (SEQ ID NO: 126) as shown in Figure 24.

### 17. Production of cell lines

The Lambda-VL-CL KI vector 2.0 produced in 12-1 above, the pVL KI_pUC19_step5BrRev produced in 12-2 above, the pVL
KI_pUC19_step6_BrRev_pVL#odd produced in 12-3 above, the human CH KI vector produced in 12-4 above, the pVH KI_pUC19_step5_BsrRev_RMCE_cassette produced in 16-5 above, and the pVH KI_pUC19_step3_pVH#odd_even produced in 16-6 above were each introduced into DT40 cells, so as to produce cell lines.

First, the human CH KI vector, which had been linearized with the restriction enzyme SalI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Crep_EGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Subsequently, the Lambda-VL-CL KI vector 2.0, which had been linearized with the restriction enzyme NotI, was introduced into DT40 cells, and the obtained mixture was then cultured in a medium containing 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance gene had been removed.

Thereafter, the pVL KI_pUC19_step5_BrRev, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Thereafter, the pVL KI_pUC19_step6_BrRev_pVL#odd, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Further, the pVH KI_pUC19_step5_BsrRev_RMCE_ cassette, which had been linearized with the restriction enzyme NotI, was introduced into the cells, and the obtained mixture was then cultured in a medium containing 2 mg/mL G418 and 10 µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to confirm that the drug resistance genes had been removed.

Finally, the pVH KI_pUC19_step3_pVH#odd_even, which had been linearized with the restriction enzyme NotI, was introduced into the cells, and the obtained mixture was then cultured in a medium containing 2 mg/mL G418. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines #7-7, #11-3 and #11-6, from each of which the drug resistance gene had been removed.

### 18. Confirmation of antibody expression (flow cytometry and ELISA)

### 18-1. Analysis by flow cytometry

Based on the method described in 3 above, the expression of an antibody in the cells produced in 16 above was confirmed. The results are shown in Figure 25. It was confirmed that only chicken IgM was expressed in wild-type DT40, whereas the expression of chicken IgM disappeared and instead, human Igγ and Igλ were expressed in the transformed cell lines #7-7, #11-3 and #11-6 (Figure 25).

### 18-2. Analysis by ELISA

The antibodies secreted from the cells produced in 17 above were measured. The measurement of human IgG was carried out based on the method described in 3-2 above. The method for measuring chicken IgM is as follows.

That is, 20 µL of 1.0 µg/mL Goat anti-chicken IgM (Bethyl, A30-102A) was dispensed on an immuno 384-well plate Maxisorp (Nunc, 464718), and it was then reacted at room temperature for 1 hour or more, so that it was immobilized on the plate. Thereafter, the resultant was washed with a washing solution (PBS containing 0.05% Tween 20) five times, and 50 µL of a blocking solution (PBS containing 1% BSA) was then added thereto. The obtained mixture was reacted at room temperature for 30 minutes. Thereafter, the resultant was washed with a washing solution five times, and 20 µL of a measurement sample or chicken serum (GIBCO, 16110) serving as a standard substance was then added thereto. The obtained mixture was reacted at room temperature for 1 hour. The resultant was washed with a washing solution five times, and 20 µL of Goat anti-chicken IgM HRP conjugated (Bethyl, A30-102P), which had been 5000 times diluted with PBS containing 1 % BSA and 0.05% Tween 20, was then added thereto. The obtained mixture was reacted at room temperature for 1 hour. Thereafter, the reaction mixture was washed with a washing solution five times, 20 µL of TMB+ (Dako, S159985) was then added thereto, and the obtained mixture was subjected to a coloring reaction at room temperature for 3 minutes. Subsequently, 20 µL of 1 N sulfuric acid was added to the reaction mixture to termination the reaction. Using Infinite M1000 (TECAN), the absorbance at 450 nm was measured.

The results are shown in Table 15. It was confirmed that, as with 13-1 above, only chicken IgM was secreted from wild-type DT40, whereas human IgG₁ was secreted from the transformed cell lines #7-7, #11-3 and #11-6.

**[Table 15]**

| | Human IgG1 (µg/mL) | Chicken IgM (µg/mL) |
|---|---|---|
| Wild type | <0.01 | 2.33 |
| #7-7 | 0.13 | <0.01 |
| #11-3 | 0.24 | <0.01 |
| #11-6 | 0.16 | <0.01 |

### 19. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56) and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line #11-6 (the top 10) are shown in Table 16 and Table 17, respectively. A clone comprising a the additionally inserted human pseudogene-derived sequence was found in the variable regions of both of the light chain and the heavy chain, and it was demonstrated that more various sequences than in the case of L15/H15 cell line were generated as a result of gene conversion. In addition, it was also confirmed that a variety of sequences were generated as a result of gene conversion even in other cell lines.

**[Table 16]**

| # | % | CDR1 | CDR2 | CDR3 |
|---|---|---|---|---|
| 1 | 0.166 | VL | pVL105 | VL |
| 2 | 0.166 | pVL105 | VL | VL |
| 3 | 0.125 | pVL113 | pVL113 | pVL113 |
| 4 | 0.125 | VL | pVL109 | VL |
| 5 | 0.083 | Not matched | pVL105 | VL |
| 6 | 0.083 | VL | pVL104 | pVL104 |
| 7 | 0.083 | VL | pVL113 | VL |
| 8 | 0.083 | pVL114 | pVL114 | pVL114 |
| 9 | 0.042 | pVL109 | pVL109 | pVL109 |
| 10 | 0.042 | pVL115 | pVL115 | pVL115 |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) | | | | |

**[Table 17]**

| # | % | CDR1 | CDR2 | CDR3 |
|---|---|---|---|---|
| 1 | 1.077 | pVH123 | VH | VH |
| 2 | 0.824 | VH | pVH129 | VH |
| 3 | 0.760 | pVH104 | Not matched | VH |
| 4 | 0.507 | pVH123 | Not matched | VH |
| 5 | 0.444 | pVH117 | Not matched | VH |
| 6 | 0.380 | pVH129 | pVH129 | VH |
| 7 | 0.380 | pVH126 | pVH112 | VH |
| 8 | 0.380 | VH | pVH111 | Not matched |
| 9 | 0.317 | pVH102 | VH | Not matched |
| 10 | 0.253 | pVH112 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) | | | | |

### Production of L30/H30 cell line, in which 30 pseudogene regions each consisting of human variable region-derived sequence have been inserted into light chain, and 30 such pseudogene regions have been inserted into heavy chain

### 20. Knocking-in into chicken antibody light chain (Igλ) pseudogene and variable regions

### (1) Human antibody light chain pseudogene sequence (pVL30)

Based on the sequences of human immunoglobulin λ variable regions in the database, 30 CDR1, CDR2 and CDR3 sequences were selected, and the framework sequence of the human Igλ variable region cloned in 1-1 above was then combined therewith, so as to design 30 sequences, namely, pVL101 (SEQ ID NO: 74), pVL102 (SEQ ID NO: 75), pVL103 (SEQ ID NO: 76), pVL104 (SEQ ID NO: 77), pVL105 (SEQ ID NO: 78), pVL106 (SEQ ID NO: 79), pVL107 (SEQ ID NO: 80), pVL108 (SEQ ID NO: 81), pVL109 (SEQ ID NO: 82), pVL110 (SEQ ID NO: 83), pVL111 (SEQ ID NO: 84), pVL112 (SEQ ID NO: 85), pVL113 (SEQ ID NO: 86), pVL114 (SEQ ID NO: 87), pVL115 (SEQ ID NO: 88), pVL116 (SEQ ID NO: 127), pVL117 (SEQ ID NO: 128), pVL118 (SEQ ID NO: 129), pVL119 (SEQ ID NO: 130), pVL120 (SEQ ID NO: 131), pVL121 (SEQ ID NO: 132), pVL122 (SEQ ID NO: 133), pVL123 (SEQ ID NO: 134), pVL124 (SEQ ID NO: 135), pVL125 (SEQ ID NO: 136), pVL126 (SEQ ID NO: 137), pVL127 (SEQ ID NO: 138), pVL128 (SEQ ID NO: 139), pVL129 (SEQ ID NO: 140), and pVL130 (SEQ ID NO: 141). Thereafter, a human antibody light chain pseudogene sequence pVL30 (SEQ ID NO: 142) comprising these sequences was designed, and was then subjected to gene synthesis. (2) Construction of targeting vector

The human pseudogene sequence pVL30 synthesized in the above section was incorporated into the multi-cloning site of the pVL KI_pUC19_step6_BrRev in 16-2 above, so as to construct a targeting vector pVL KI_pUC 19_step6 _BrRev_#odd+even (SEQ ID NO: 143) as shown in Figure 26.

### 21. Production of cell lines

The pVL KI_pUC19_step6 _BrRev_#odd+even prepared in 20 above was introduced into the L15/H30 cell line produced in 17 above to produce cell lines.

The pVL KI_pUC19_step6_BrRev_#odd+even, which had been linearized with the restriction enzyme NotI, was introduced into the aforementioned cell line, and the obtained mixture was then cultured in a medium containing 2 mg/mL G418 and 10µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines #7-7-48-5, #7-7-48-7 and #7-7-48-10, from each of which the drug resistance genes had been removed.

### 22. Confirmation of antibody expression (flow cytometry and ELISA)

### 22-1. Analysis by flow cytometry

Based on the method described in 3 above, the expression of an antibody in the cells produced in 21 above was confirmed. The results are shown in Figure 27. It was confirmed that only chicken IgM was expressed in wild-type DT40, whereas the expression of chicken IgM disappeared and instead, human Igγ and Igλ were expressed in the transformed cell lines #7-7-48-5, #7-7-48-7 and #7-7-48-10 (Figure 27).

### 22-2. Analysis by ELISA

Based on the method described in 18-2 above, the antibodies secreted from the cells produced in 21 above were measured. The results are shown in Table 18. As with 13-1 above, it was confirmed that only chicken IgM was secreted from wild-type DT40, whereas human IgG₁ was secreted from the transformed cell lines #7-7-48-5, #7-7-48-7 and #7-7-48-10.

**[Table 18]**

| | **Human IgG1 (µg/mL)** | **Chicken IgM (µg/mL)** |
|---|---|---|
| **Wild type** | <0.01 | 2.33 |
| #7-7-48-5 | 0.26 | <0.01 |
| #7-7-48-7 | 0.16 | <0.01 |
| #7-7-48-10 | 0.13 | <0.01 |

### 23. Confirmation of gene conversion

Using the cell lines in which the expression of an antibody had been confirmed in 22 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line #7-7-48-10 (the top 10) are shown in Table 19 and Table 20, respectively. A clone comprising the additionally inserted human pseudogene-derived sequence was found in the variable regions of both of the light chain and the heavy chain, and it was demonstrated that more various sequences were generated by increasing the number of pseudogenes. In addition, it was also confirmed that a variety of sequences were generated as a result of gene conversion even in other cell lines.

**[Table 19]**

| # | % | CDR1 | CDR2 | CDR3 |
|---|---|---|---|---|
| 1 | 0.347* | VL | pVL118 | VL |
| 2 | 0.270 | pVL119 | VL | VL |
| 3 | 0.154 | VL | pVL123 | pVL118 |
| 4 | 0.116 | pVL130 | pVL130 | pVL130 |
| 5 | 0.116 | VL | VL | pVL127 |
| 6 | 0.077 | pVL116 | pVL116 | pVL116 |
| 7 | 0.077 | pVL119 | pVL119 | VL |
| 8 | 0.077 | VL | pVL129 | pVL129 |
| 9 | 0.077 | VL | pVL105 | VL |
| 10 | 0.077* | VL | pVL118 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}: Sequences of portions other than CDRs are different, respectively. | | | | |

**[Table 20]**

| # | % | CDR1 | CDR2 | CDR3 |
|---|---|---|---|---|
| 1 | 33.191* | pVH104 | Not matched | VH |
| 2 | 8.085 | pVH129 | Not matched | VH |
| 3 | 6.241 | pVH108 | Not matched | VH |
| 4 | 5.106 | pVH111 | Not matched | VH |
| 5 | 3.688** | pVH126 | Not matched | VH |
| 6 | 1.418 | pVH126 | pVH126 | VH |
| 7 | 1.418 | pVH101 | Not matched | VH |
| 8 | 1.277* | pVH104 | Not matched | VH |
| 9 | 1.277** | pVH126 | Not matched | VH |
| 10 | 0.993* | pVH104 | Not matched | VH |

| | | | | |
|---|---|---|---|---|
| **VH: sequence inserted into variable region** **pVH: sequence inserted into pseudogene region** **Not matched: sequence that is not completely matched with either VH or pVH (which** is **probably caused by partial genetic recombination or mutation)** ^{∗}: **Sequences of CDR2 are different, respectively.** ^{∗∗}: **Sequences of CDR2 are different, respectively.** | | | | |

### Production of L30/H15 cell line, in which 30 pseudogene regions each consisting of human variable region-derived sequence have been inserted into light chain, and 15 such pseudogene regions have been inserted into heavy chain

### 24. Production of cell lines

The pVL KI_pUC19_step6 _BrRev_#odd+even prepared in 20 above was introduced into the L15/H15 cell line A12-4 produced in 17 above to produce cell lines.

The pVL KI_pUC19_step6_BrRev_pVL#odd+even, which had been linearized with the restriction enzyme NotI, was introduced into the aforementioned cell line, and the obtained mixture was then cultured in a medium containing 2 mg/mL G418 and 10µg/mL blasticidin. Thereafter, the growing cells were subjected to genotyping by PCR, so as to confirm that substitution had occurred in a gene locus of interest. Thereafter, Cre_pEGFP-C1 was introduced into the cells, and the resulting cells were then subjected to cell sorting to select GFP-positive cells. Thereafter, the selected cells were subjected to genotyping by PCR, so as to obtain cell lines A77-3 and A77-2, from each of which the drug resistance genes had been removed.

### 25. Confirmation of antibody expression (flow cytometry and ELISA)

### 25-1. Analysis by flow cytometry

Based on the method described in 3 above, the expression of an antibody in the cells produced in 24 above was confirmed. The results are shown in Figure 28. It was confirmed that only chicken IgM was expressed in wild-type DT40, whereas the expression of chicken IgM disappeared and instead, human Igγ and Igλ were expressed in the transformed cell lines A77-3 and A77-2 (Figure 28).

### 25-2. Analysis by ELISA

Based on the method described in 3-2 above, the antibodies secreted from the cells produced in 24 above were measured. The results are shown in Table 21. As with 13-1 above, it was confirmed that only chicken IgM was secreted from wild-type DT40, whereas human IgG₁ was secreted from the transformed cell lines A77-3 and A77-2.

**[Table 21]**

| | Human IgG1 (µg/mL) | Chicken IgM (µg/mL) |
|---|---|---|
| Wild type | <0.01 | 2.33 |
| A77-3 | 0.43 | <0.01 |
| A77-2 | 0.48 | <0.01 |

### Production of L30/H15f15f and L30/H15r15f cell lines, in which 30 pseudogene regions each consisting of human variable region-derived sequence have been inserted into light chain and 30 such pseudogene regions have been inserted into heavy chain according to RMCE method

### 26. Production of vectors

### 26-1. Further insertion of 15 pseudogenes constructed in forward direction into chicken antibody heavy chain (IgM) pseudogene region

### (1) Human antibody heavy chain pseudogene sequence (pVH15a)

The pVH116, pVH117, pVH118, pVH119, pVH120, pVH121, pVH122, pVH123, pVH124, pVH125, pVH126, pVH127, pVH128, pVH129 and pVH130, which had been designed in 16 above, were ligated to one another in the forward direction, so as to produce pVH15a (SEQ ID NO: 144). (2) Construction of vector

Loxm3 rev sequence, Neomycin resistance gene, SV40 early poly A terminator, SV40 polyadenylation region, loxP for RE sequence, pVH15a (forward direction), and loxm7 rev LE sequence were inserted into a pUC57-Amp vector, so as to produce a vector RMCE_pVH1stKl_pVH15evenFor (SEQ ID NO: 145) as shown in Figure 29.

### 26-2. Further insertion of 15 pseudogenes constructed in reverse direction into chicken antibody heavy chain (IgM) pseudogene region

Loxm3 rev sequence, Neomycin resistance gene, SV40 early poly A terminator, SV40 polyadenylation region, loxP for RE sequence, pVH15a (reverse direction), and loxm7 rev LE sequence were inserted into a pUC57-Amp vector, so as to produce a vector RMCE_pVH1stKI_pVH15evenRev (SEQ ID NO: 146) as shown in Figure 29.

### 27. Production of cell lines

An L30/H15f15f cell line was produced by introducing the RMCE_pVH1stKI_pVH15evenFor produced in 26-1 above into the L30/H15 cell line produced in 24 above.

The RMCE_pVH1stKI_pVH15evenFor, together with Cre_pEGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 1-6-1 and 1-7-3 were obtained, in which substitution was confirmed to have occurred in a gene locus of interest.

An L30/H15r15f cell line was produced by introducing the RMCE_pVH1stKI_pVH15evenRev produced in 26-2 into the L30/H15 cell line produced in 24 above.

The RMCE_pVH1stKI_pVH15evenRev, together with CrepEGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 2-1-1 and 7-3-2 were obtained, in which substitution was confirmed to have occurred in a gene locus of interest.

### 28. Confirmation of antibody expression (flow cytometry and ELISA)

### 28-1. Analysis by flow cytometry

Based on the method described in 3 above, the expression of an antibody in the cells produced in 27 above was confirmed. The results are shown in Figure 30. It was confirmed that only chicken IgM was expressed in wild-type DT40, whereas the expression of chicken IgM disappeared and instead, human Igγ and Igλ were expressed in the transformed cell lines 1-6-1, 1-7-3, 2-1-1 and 7-3-2 (Figure 30).

### 28-2. Analysis by ELISA

Based on the method described in 3-2 above, the antibodies secreted from the cells produced in 27 above were measured. The results are shown in Table 22. As with 13-1 above, it was confirmed that only chicken IgM was secreted from wild-type DT40, whereas human IgG₁ was secreted from the transformed cell lines 1-6-1, 1-7-3, 2-1-1 and 7-3-2.

**[Table 22]**

| | Human IgG1 (µg/mL) | Chicken IgM (µg/mL) |
|---|---|---|
| Wild type | <0.01 | 2.33 |
| 1-6-1 | 0.18 | <0.01 |
| 1-7-3 | 0.07 | <0.01 |
| 2-1-1 | 0.12 | <0.01 |
| 7-3-2 | 0.18 | <0.01 |

### 29. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 1-6-1 (the top 10) are shown in Table 23 and Table 24, respectively, and the results of the sequence analysis performed on the variable regions of the light chain and heavy chain in the cell line 7-3-2 (the top 10) are shown in Table 25 and Table 26, respectively. A clone comprising the additionally inserted human pseudogene-derived sequence was found in the variable regions of both of the light chain and the heavy chain, and it was demonstrated that a variety of sequences were generated as in the case of the L30/H30 cell line. In addition, it was also confirmed that a variety of sequences were generated as a result of gene conversion even in other cell lines.

**[Table 23]**

| # | % | CDR1 | CDR2 | CDR3 |
|---|---|---|---|---|
| 1 | 0.935 | VL | pVL102 | VL |
| 2 | 0.468 | VL | pVL118 | VL |
| 3 | 0.271 | Not matched | pVL102 | VL |
| 4 | 0.271* | pVL119 | VL | VL |
| 5 | 0.222 | VL | VL | pVL129 |
| 6 | 0.172* | pVL119 | VL | VL |
| 7 | 0.172 | VL | pVL105 | VL |
| 8 | 0.123 | VL | pVL130 | VL |
| 9 | 0.098 | Not matched | pVL122 | VL |
| 10 | 0.098 | VL | pVL109 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}: Sequences of portions other than CDRs are different, respectively. | | | | |

**[Table 24]**

| # | % | CDR1 | CDR2 | CDR3 |
|---|---|---|---|---|
| 1 | 6.393* | pVH112 | Not matched | VH |
| 2 | 0.586 | pVH109 | Not matched | VH |
| 3 | 0.479 | pVH108 | Not matched | VH |
| 4 | 0.479 | pVH117 | Not matched | VH |
| 5 | 0.426 | pVH107 | Not matched | VH |
| 6 | 0.320 | pVH124 | Not matched | VH |
| 7 | 0.266* | pVH112 | Not matched | VH |
| 8 | 0.266 | VH | Not matched | pVH109 |
| 9 | 0.160 | Not matched | pVH125 | VH |
| 10 | 0.160* | pVH112 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) ^{∗}: Sequences of CDR2 are different, respectively. | | | | |

**[Table 25]**

| # | % | CDR1 | CDR2 | CDR3 |
|---|---|---|---|---|
| 1 | 0.293 | VL | pVL109 | VL |
| 2 | 0.267 | pVL119 | VL | VL |
| 3 | 0.187* | pVL105 | VL | VL |
| 4 | 0.160 | VL | VL | pVL129 |
| 5 | 0.133 | VL | pVL101 | VL |
| 6 | 0.133 | VL | pVL118 | VL |
| 7 | 0.133 | VL | VL | pVL127 |
| 8 | 0.107 | pVL119 | pVL119 | VL |
| 9 | 0.080 | VL | VL | pVL123 |
| 10 | 0.080* | pVL105 | VL | VL |

| | | | | |
|---|---|---|---|---|
| VL: sequence inserted into variable region pVL: sequence inserted into pseudogene region Not matched, sequence that is not completely matched with either VL or pVL (which is probably caused by partial genetic recombination or mutation) ^{∗}: Sequences of portions other than CDRs are different, respectively. | | | | |

**[Table 26]**

| # | % | CDR1 | CDR2 | CDR3 |
|---|---|---|---|---|
| 1 | 0.867* | VH | Not matched | pVH109 |
| 2 | 0.819* | VH | Not matched | pVH109 |
| 3 | 0.819 | VH | Not matched | pVH116 |
| 4 | 0.337 | pVH109 | Not matched | VH |
| 5 | 0.241* | VH | Not matched | pVH109 |
| 6 | 0.193 | pVH105 | Not matched | VH |
| 7 | 0.193 | pVH117 | Not matched | VH |
| 8 | 0.145 | pVH112 | Not matched | VH |
| 9 | 0.145 | pVH126 | Not matched | pVH116 |
| 10 | 0.096 | pVH107 | Not matched | pVH109 |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) ^{∗}: Sequences of CDR2 are different, respectively. | | | | |

### Production of L30/H15f15f15f and L30/H15r15r15f cell lines, in which 30 pseudogene regions each consisting of human variable region-derived sequence have been inserted into light chain and 45 such pseudogene regions have been inserted into heavy chain according to RMCE method

### 30. Production of vector

### 30-1. Further insertion of 15 pseudogenes constructed in forward direction into chicken antibody heavy chain (IgM) pseudogene region

### (1) Human antibody heavy chain pseudogene sequence (pVH15b)

Based on the sequences of a human immunoglobulin heavy chain variable regions in the database, novel 15 CDR1, CDR2 and CDR 3 sequences were selected, and pVH131 (SEQ ID NO: 147), pVH132 (SEQ ID NO: 148), pVH133 (SEQ ID NO: 149), pVH134 (SEQ ID NO: 150), pVH135 (SEQ ID NO: 151), pVH136 (SEQ ID NO: 152), pVH137 (SEQ ID NO: 153), pVH138 (SEQ ID NO: 154), pVH139 (SEQ ID NO: 155), pVH140 (SEQ ID NO: 156), pVH141 (SEQ ID NO: 157), pVH142 (SEQ ID NO: 158), pVH143 (SEQ ID NO: 159), pVH144 (SEQ ID NO: 160), and pVH145 (SEQ ID NO: 161) were ligated to one another in the forward direction to produce pVH15b (SEQ ID NO: 162).

### (2) Construction of vector

Loxm3 rev sequence, blasticidin resistance gene, SV40 early poly A terminator, SV40 polyadenylation region, loxm7 rev RE sequence, pVH15b (forward direction), and loxP for LE sequence were inserted into a pUC57-Amp vector, so as to produce a vector pVH15B-ver3_RMCE2nd_For (SEQ ID NO: 163) as shown in Figure 31.

### 30-2. Further insertion of 15 pseudogenes constructed in reverse direction into chicken antibody heavy chain (IgM) pseudogene region

Loxm3 rev sequence, blasticidin resistance gene, SV40 early poly A terminator, SV40 polyadenylation region, loxm7 rev RE sequence, pVH15b (reverse direction), and loxP for LE sequence were inserted into a pUC57-Amp vector, so as to produce a vector pVH15B-ver3_RMCE2nd_Rev (SEQ ID NO: 164) as shown in Figure 31.

### 31. Production of cell lines

An L30/H15f15f15f cell line was produced by introducing the pVH15B-ver3_RMCE2nd_For produced in 30-1 above into the L30/H15f15f cell line produced in 2 above.

The pVH15B-ver3_RMCE2nd_For, together with Cre_pEGFP-C1, was introduced into the aforementioned cell line to induce site-specific recombination, and the obtained mixture was then cultured in a medium containing 10 µg/mL blasticidin. Thereafter, the growing cells were subjected to genotyping by PCR, and cell lines 2-1-3, 2-1-11, 1-n-1 and 2-n-3 were obtained, in which substitution was confirmed to have occurred in a gene locus of interest.

An L30/H15r15r15f cell line was produced by introducing the pVH15B-ver3_RMCE2nd_Rev produced in 27-2 above into the L30/H15r15f cell line produced in 27 above.

The pVH15B-ver3RMCE2nd_Rev, together with Cre_pEGFP-C1, was introduced into the aforementioned cell line to induce site-specific recombination, and the obtained mixture was then cultured in a medium containing 10 µg/mL blasticidin. Thereafter, the growing cells were subjected to genotyping by PCR, and cell lines 4-1-5, 4-1-7 and 3-1-11 were obtained, in which substitution was confirmed to have occurred in a gene locus of interest.

### 32. Confirmation of antibody expression (flow cytometry and ELISA)

### 32-1. Analysis by flow cytometry

Based on the method described in 3 above, the expression of an antibody in the cells produced in 31 above was confirmed. The results are shown in Figure 32 and Figure 33. It was confirmed that only chicken IgM was expressed in wild-type DT40, whereas the expression of chicken IgM disappeared and instead, human Igγ and Igλ were expressed in the transformed cell lines 2-1-3, 2-1-11, 1-n-1 and 2-n-3 (which are shown in Figure 32), and in the transformed cell lines 4-1-5, 4-1-7 and 3-1-11 (which are shown in Figure 33).

### 32-2. Analysis by ELISA

Based on the method described in 3-2 above, the antibodies secreted from the cells produced in 31 above were measured. The results are shown in Table 27. As with 13-1 above, it was confirmed that only chicken IgM was secreted from wild-type DT40, whereas human IgG₁ was secreted from the transformed cell lines 2-1-3, 2-1-11, 1-n-1, 2-n-3, 4-1-5, 4-1-7 and 3-1-11.

**[Table 27]**

| | Human IgG1 ((.ig/mL) | Chicken IgM (µg/mL) |
|---|---|---|
| Wild type | <0.01 | 2.33 |
| 2-1-3 | 0.36 | <0.01 |
| 2-1-11 | 0.16 | <0.01 |
| 1-n-1 | 0.18 | <0.01 |
| 2-n-3 | 0.16 | <0.01 |
| 4-1-5 | 0.37 | <0.01 |
| 4-1-7 | 0.94 | <0.01 |
| 3-1-11 | 1.31 | <0.01 |

### 33. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added
(CGTATCGCCTCCCTCGCGCCATCAGNNNNNNNNNNCAGGTTCCCTGGTGC AGGC) (SEQ ID NO: 46), d, and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 2-1-3 (the top 10) are shown in Table 28 and Table 29, and the results of the sequence analysis performed on the variable regions of the light chain and heavy chain in the cell line 3-1-11 (the top 10) are shown in Table 30 and Table 31, respectively. A clone comprising the additionally inserted human pseudogene-derived sequence was found in the variable regions of both of the light chain and the heavy chain, and it was demonstrated that a variety of sequences were generated as a result of gene conversion. In addition, it was also confirmed that a variety of sequences were generated as a result of gene conversion even in other cell lines.

**[Table 28]**

| # | % | CDR1 | CDR2 | CDR3 |
|---|---|---|---|---|
| 1 | 0.882* | pVL119 | VL | VL |
| 2 | 0.428** | VL | pVL118 | VL |
| 3 | 0.241 | pVL119 | pVL119 | VL |
| 4 | 0.187** | VL | pVL118 | VL |
| 5 | 0.187** | VL | pVL118 | VL |
| 6 | 0.134 | VL | pVL119 | VL |
| 7 | 0.134 | VL | pVL109 | VL |
| 8 | 0.134 | pVL105 | VL | VL |
| 9 | 0.107* | pVL119 | VL | VL |
| 10 | 0.080 | VL | pVL105 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}, ^{∗∗}: Sequences of portions other than CDRs are different, respectively. | | | | |

**[Table 29]**

| # | % | CDR1 | CDR2 | CDR3 |
|---|---|---|---|---|
| 1 | 2.226 | pVH112 | Not matched | VH |
| 2 | 1.272 | pVH109 | Not matched | VH |
| 3 | 0.636 | VH | pVH112 | VH |
| 4 | 0.477 | pVH144 | Not **matched** | VH |
| 5 | 0.477 | pVH105 | Not matched | VH |
| 6 | 0.318 | pVH145 | Not matched | VH |
| 7 | 0.318 | pVH137 | Not matched | VH |
| 8 | 0.318* | pVH108 | Not matched | VH |
| 9 | 0.318* | pVH108 | Not matched | VH |
| 10 | 0.318 | VH | Not matched | pVH116 |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) ^{∗}: Sequences of CDR2 are different, respectively. | | | | |

**[Table 30]**

| # | % | CDR1 | CDR2 | CDR3 |
|---|---|---|---|---|
| 1 | 1.000 | pVL105 | VL | VL |
| 2 | 0.788 | pVL119 | VL | VL |
| 3 | 0.515* | VL | pVL118 | VL |
| 4 | 0.455* | VL | pVL118 | VL |
| 5 | 0.182 | VL | pVL105 | VL |
| 6 | 0.152** | pVL119 | pVL119 | VL |
| 7 | 0.152 | VL | pVL109 | VL |
| 8 | 0.121 | VL | pVL104 | VL |
| 9 | 0.091** | pVL119 | pVL119 | VL |
| 10 | 0.091 | VL | pVL107 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}, ^{∗∗}: Sequences of portions other than CDRs are different, respectively. | | | | |

**[Table 31]**

| # | % | CDR1 | CDR2 | CDR3 |
|---|---|---|---|---|
| 1 | 1.524 | pVH109 | Not matched | VH |
| 2 | 1.220 | pVH112 | Not matched | VH |
| 3 | 0.915 | pVH117 | Not matched | VH |
| 4 | 0.915 | pVH142 | Not matched | VH |
| 5 | 0.610 | pVH108 | Not matched | VH |
| 6 | 0.610 | pVH138 | Not matched | VH |
| 7 | 0.610 | pVH107 | Not matched | VH |
| 8 | 0.457 | pVH139 | Not matched | VH |
| 9 | 0.457 | pVH102 | Not matched | VH |
| 10 | 0.457 | pVH142 | Not matched | Not matched |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) | | | | |

### Production of L30/H15f15f15f15f and L30/H15r15r15r15f cell lines, in which 30 pseudogene regions each consisting of human variable region-derived sequence have been inserted into light chain and 60 such pseudogene regions have been inserted into heavy chain according to RMCE method

### 34. Production of vector

### 34-1. Further insertion of 15 pseudogenes constructed in forward direction into chicken antibody heavy chain (IgM) pseudogene region

### (1) Human antibody heavy chain pseudogene sequence (pVH15c)

Based on the sequences of human immunoglobulin heavy chain variable regions in the database, novel 15 CDR1, CDR2 and CDR 3 sequences were selected, and pVH146 (SEQ ID NO: 165), pVH147 (SEQ ID NO: 166), pVH148 (SEQ ID NO: 167), pVH149 (SEQ ID NO: 168), pVH150 (SEQ ID NO: 169), pVH151 (SEQ ID NO: 170), pVH152 (SEQ ID NO: 171), pVH153 (SEQ ID NO: 172), pVH154 (SEQ ID NO: 173), pVH155 (SEQ ID NO: 174), pVH156 (SEQ ID NO: 175), pVH157 (SEQ ID NO: 176), pVH158 (SEQ ID NO: 177), pVH159 (SEQ ID NO: 178), and pVH160 (SEQ ID NO: 179) were ligated to one another in the forward direction to produce pVH15c (SEQ ID NO: 180).

### (2) Construction of vector

Loxm3 rev sequence, neomycin resistance gene, SV40 early poly A terminator, SV40 polyadenylation region, loxP for RE sequence, pVH15c (forward direction), and loxm7 rev LE sequence were inserted into a pUC57-Amp vector, so as to produce a vector pVH15ARMCE1st_Fw (SEQ ID NO: 181) as shown in Figure 34.

### 34-2. Further insertion of 15 pseudogenes constructed in reverse direction into chicken antibody heavy chain (IgM) pseudogene region

Loxm3 rev sequence, neomycin resistance gene, SV40 early poly A terminator, SV40 polyadenylation region, loxP for RE sequence, pVH15c (reverse direction), and loxm7 rev LE sequence were inserted into a pUC57-Amp vector, so as to produce a vector pVH15A_RMCE1st_Rv (SEQ ID NO: 182) as shown in Figure 34.

### 35. Production of cell lines

An L30/H15f15f15f15f cell line was produced by introducing the pVH15A _RMCE1st_Fw produced in 34-1 above into the L30/H15f15f15f cell line produced in 31 above.

The pVH15A_RMCE1st_Fw, together with Cre_PEGFP-C1, was introduced into the aforementioned cell line to induce site-specific recombination, and the obtained mixture was then cultured in a medium containing 2 mg/mL G418. Thereafter, the growing cells were subjected to genotyping by PCR, and cell lines 1n1-3, 1n1-4, and 1n1-5 were obtained, in which substitution was confirmed to have occurred in a gene locus of interest.

An L30/H15r15r15r15f cell line was produced by introducing the pVH15A _RMCE1st_Rv produced in 34-2 above into the L30/H15r15r15f cell line produced in 31 above.

The pVH15ARMCElst Rv, together with Cre_pEGFP-C1, was introduced into the aforementioned cell line to induce site-specific recombination, and the obtained mixture was then cultured in a medium containing 2 mg/mL G418. Thereafter, the growing cells were subjected to genotyping by PCR, and a cell line 3111-1 was obtained, in which substitution was confirmed to have occurred in a gene locus of interest.

### 36. Confirmation of antibody expression (flow cytometry and ELISA)

### 36-1. Analysis by flow cytometry

Based on the method described in 3 above, the expression of an antibody in the cells produced in 35 above was confirmed. The results are shown in Figure 35. It was confirmed that only chicken IgM was expressed in wild-type DT40, whereas the expression of chicken IgM disappeared and instead, human Igγ and Igλ were expressed in the transformed cell lines 1n1-3, 1n1-4, 1n1-5 and 3111-1 (Figure 35).

### 36-2. Analysis by ELISA

Based on the method described in 3-2 above, the antibodies secreted from the cells produced in 35 above were measured. The results are shown in Table 32. As with 13-1 above, it was confirmed that only chicken IgM was secreted from wild-type DT40, whereas human IgG₁ was secreted from the transformed cell lines 1n1-3, 1n1-4, 1n1-5 and 3111-1.

**[Table 32]**

| | Human IgG1 (µg/mL) | Chicken IgM (µg/mL) |
|---|---|---|
| Wild type | <0.01 | 2.33 |
| 1n1-3 | 0.32 | <0.01 |
| 1n1-4 | 0.34 | <0.01 |
| 1n1-5 | 0.22 | <0.01 |
| 3111-1 | 0.49 | <0.01 |

### 37. Confirmation of gene conversion

Using the cell lines in which the expression of an antibody had been confirmed in 10 above, gene conversion was confirmed. Using, as a template, the genomic DNA extracted from the cells, which had been cultured for 42 days in the presence of TSA, by the method described in 4 above, a light chain variable region was amplified by PCR using a sense primer to which a recognition sequence had been added
(GTATCGCCTCCCTCGCGCCATCAGNNNNNNNNNNCAGGTTCCCTGGTGCA GGC) (SEQ ID NO: 46), and an antisense primer
(CTATGCGCCTTGCCAGCCCGCTCAGGCTTGGTCCCTCCGCCGAA) (SEQ ID NO: 47), and a heavy chain variable region was amplified by PCR using a sense primer (CTATGCGCCTTGCCAGCCCGCTCAGTCCGTCAGCGCTCTCT) (SEQ ID NO: 56), and an antisense primer to which an identification tag had been added (CGTATCGCCTCCCTCGCGCCATCAGNNNNNNNNNNTGGGGGGGGTTCATA TGAAG) (SEQ ID NO: 57). Thereafter, sequence analyses were carried out based on the method described in 4 above. The results of the sequence analysis performed on the variable regions of the light chain and heavy chain in the cell line 1n1-3 (the top 10) are shown in Table 33 and Table 34, and the results of the sequence analysis performed on the variable regions of the light chain and heavy chain in the cell line 3111-1 (the top 10) are shown in Table 35 and Table 36, respectively. A clone comprising the additionally inserted human pseudogene-derived sequence was found in the variable regions of both of the light chain and the heavy chain, and it was demonstrated that more various sequences were generated as a result of gene conversion. In addition, it was also confirmed that a variety of sequences were generated as a result of gene conversion even in other cell lines.

**[Table 33]**

| # | % | CDR1 | CDR2 | CDR3 |
|---|---|---|---|---|
| 1 | 1.217 | Not matched | pVL105 | VL |
| 2 | 0.466* | Not matched | pVL118 | VL |
| 3 | 0.304 | pVL119 | VL | VL |
| 4 | 0.223** | pVL119 | pVL119 | VL |
| 5 | 0.223* | Not matched | pVL118 | VL |
| 6 | 0.162 | Not matched | pVL109 | VL |
| 7 | 0.162 | pVL105 | VL | VL |
| 8 | 0.142 | Not matched | pVL119 | VL |
| 9 | 0.081 | Not matched | VL | pVL129 |
| 10 | 0.061** | pVL119 | pVL119 | VL |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}: Sequences of CDR2 are different, respectively ^{∗∗}: Sequences of portions other than CDRs are different, respectively. | | | | |

**[Table 34]**

| # | % | CDR1 | CDR2 | CDR3 |
|---|---|---|---|---|
| 1 | 63.115* | pVH105 | Not matched | VH |
| 2 | 2.186 | pVH138 | pVH138 | VH |
| 3 | 0.929 | pVH124 | Not matched | VH |
| 4 | 0.820 | pVH112 | Not matched | VH |
| 5 | 0.765* | pVH105 | Not matched | VH |
| 6 | 0.656* | pVH105 | Not matched | VH |
| 7 | 0.601 | pVH155 | Not matched | VH |
| 8 | 0.601* | pVH105 | Not matched | VH |
| 9 | 0.546* | pVH105 | Not matched | VH |
| 10 | 0.492 | pVH156 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) ^{∗}: Sequences of CDR2 are different, respectively. | | | | |

**[Table 35]**

| # | % | CDR1 | CDR2 | CDR3 |
|---|---|---|---|---|
| 1 | 0.142 | VL | VL | pVL129 |
| 2 | 0.094 | VL | VL | pVL128 |
| 3 | 0.071* | pVL119 | VL | VL |
| 4 | 0.047* | pVL119 | VL | VL |
| 5 | 0.024 | Not matched | pVL107 | pVL107 |
| 6 | 0.024 | pVL108 | pVL123 | pVL123 |
| 7 | 0.024 | pVL108 | pVL108 | pVL123 |
| 8 | 0.024* | pVL119 | VL | VL |
| 9 | 0.024 | VL | VL | pVL125 |
| 10 | 0.024 | VL | VL | pVL130 |

| | | | | |
|---|---|---|---|---|
| VL: sequence inserted into variable region pVL: sequence inserted into pseudogene region Not matched: sequence that is not completely matched with either VL or pVL (which is probably caused by partial genetic recombination or mutation) ^{∗}: Sequences of portions other than CDRs are different, respectively. | | | | |

**[Table 36]**

| # | % | CDR1 | CDR2 | CDR3 |
|---|---|---|---|---|
| 1 | 51.762* | pVH126 | Not matched | VH |
| 2 | 23.429 | pVH108 | Not matched | VH |
| 3 | 1.053 | pVH129 | Not matched | VH |
| 4 | 0.545* | pVH126 | Not matched | VH |
| 5 | 0.436* | pVH126 | Not matched | VH |
| 6 | 0.291 | pVH104 | Not matched | VH |
| 7 | 0.291* | pVH126 | Not matched | VH |
| 8 | 0.291** | pVH126 | Not matched | Not matched |
| 9 | 0.254** | pVH126 | Not matched | Not matched |
| 10 | 0.254* | pVH126 | Not matched | VH |

| | | | | |
|---|---|---|---|---|
| VH: sequence inserted into variable region pVH: sequence inserted into pseudogene region Not matched: sequence that is not completely matched with either VH or pVH (which is probably caused by partial genetic recombination or mutation) ^{∗}: Sequences of CDR2 are different, respectively. ^{∗}: Sequences of CDR2 and CDR3 are different, respectively. | | | | |

### Antibody selection 1

### 38. Preparation of antigen

Plexin A4 was selected as an antigen protein. Plexin A4 is a molecule belonging to Plexin A type, and has been known as a factor for controlling axon extension in the sensory nerve or the sympathetic nerve.

As an antigen, Plexin A4, in which FLAG tags were added to the C-termini of a Sema domain and a PSI (Plexin-Semaphorin-Integrin) domain, was used (the amino acid sequence is shown in SEQ ID NO: 68). Based on Accession No. NM_020911 and Accession No. EAW83796, the nucleotide sequence shown in SEQ ID NO: 67 was synthesized, and it was then inserted into an expression vector in which the EBNA-1 gene and Hygromycin resistance gene of pCEP4 (Life Technologies, V044-50) had been deleted, thereby constructing a Plexin A4 expression vector.

The obtained Plexin A4 expression vector was introduced into FreeStyle 293 F cells (Life Technologies, R790-07), using PEI (polyethylenimine), so that it was allowed to express therein. At that time, an expression vector comprising an EBNA-1 gene was also introduced into the cells, separately. The expression was carried out at 3 L-scale, and the culture was carried out for 7 days.

Utilizing FLAG tags, a protein of interest in the culture supernatant was purified. An XK16/20 column (GE Healthcare, 28-9889-37) was filled with 2.5 mL of anti-M2-agarose resin (Sigma, A2220), and it was then equilibrated with D-PBS(-) (Wako Pure Chemical Industries, Ltd., 045-29795). Thereafter, the culture supernatant was loaded thereon, it was washed with D-PBS(-), and it was then eluted with D-PBS(-) containing 0.1 mg/mL FLAG peptide (Sigma, F3290). The resultant was monitored with the absorbance at 280 nm, and a fraction corresponding to the peak was then recovered. The molecular weight of the recovered protein was measured by SDS-PAGE and CBB staining, and it was then confirmed that the obtained molecular weight corresponded to the molecular weight of the protein of interest.

Using an ultrafiltration membrane (Amicon, UFC0801008), approximately 24 mL of the recovered fraction was concentrated to a volume of 2 mL, and then, using Hi Load 16/600 Superdex 200 pg (GE Healthcare, 28-9893-35), the fraction was purified by gel filtration chromatography. The column was equilibrated with D-PBS(-), and the recovered fraction was then loaded thereon, followed by elution with D-PBS(-). The resultant was monitored with the absorbance at 280 nm, and a fraction corresponding to the peak was then recovered. The molecular weight of the recovered protein was measured by SDS-PAGE and CBB staining.

### 39. Selection of antibody

### 39-1. Production of antigen magnetic beads

Using Dynabeads M-270 Carboxylic Acid (Life Technologies, 14305D) and Dynabeads M-270 Epoxy (Life Technologies, 14301) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 10 µL of beads were washed with 20 µL of 25 mM MES (pH 5.0) three times, and 10 µL of 50 mg/mL NHS solution and 10 µL of 50 mg/mL EDC solution were added to the resulting solution. The obtained mixture was reacted at room temperature for 30 minutes, and the reaction solution was then washed with 20 µL of 25 mM MES (pH 5.0) twice. A supernatant was removed from this magnetic beads suspension, and 10 µL of Plexin A4 protein solution, which had been adjusted to 0.6 mg/mL with 25 mM MES (pH 5.0), was then added to the suspension. The obtained mixture was reacted at 4°C overnight, while being subjected to rotary stirring. Thereafter, a supernatant was removed from the reaction solution, and 20 µL of quenching buffer (50 mM Tris-HCl) was then added thereto, followed by the reaction of the mixture at room temperature for 15 minutes while being subjected to rotary stirring. Regarding Dynabeads M-270 Epoxy, 10 µL of beads were washed with 20 µL of 100 mM Na-phosphate buffer three times, and thereafter, 13.3 µL of Plexin A4 protein solution adjusted to 0.45 mg/ml with 100 mM Na-phosphate buffer and 6.7 µL of 100 mM Na-phosphate buffer containing 3 M ammonium sulfate were added and suspended therein. The obtained mixture was reacted at 37°C overnight, while being subjected to rotary stirring. After the removal of a supernatant, the two types of beads were collectively suspended in 1 mL of selection buffer (PBS containing 1% BSA), and the obtained suspension was then washed with the same buffer as mentioned above three times. Thereafter, the resultant was suspended in 200 µL of selection buffer, and was then subjected to selection.

### 39-2. Selection by antigen magnetic beads

An L15/H15 cell line, B7-3, was cultured in a medium containing 2.5 ng/mL TSA for 3 weeks. Thereafter, approximately 1 × 10⁸ cells were washed with 10 mL of selection buffer, and a supernatant was then removed from the reaction solution. The remaining solution was washed with 1 mL of selection buffer, and it was then suspended in 950 µL of selection buffer. To this cell suspension, 50 µL of the antigen magnetic beads prepared in 17-1 above was added, and the obtained mixture was then reacted at 4°C for 30 minutes, while being stirred by rotation. Thereafter, using KingFisher mL (Thermo, 5400050), the reaction solution was washed with 0.5 mL of selection buffer three times. Thereafter, the recovered cells were suspended in 20 mL of medium, the suspension was then plated on a 9-cm dish, and it was then cultured in a CO₂ incubator for 7 days.

### 39-3. Screening by flow cytometry

The cell culture solution obtained in 39-2 above was recovered, and 3 × 10⁶ cells were then fractionated into a 1.5-mL tube. The cells were centrifuged to remove a supernatant, and the resulting cells were then washed with 1.5 mL of PBS to remove a supernatant. Using EZ-Link (registered trademark) NHS-PEG4-Biotinylation Kit (PIERCE, 21455), a biotin-labeled Plexin A4 protein was prepared, and 300 µL of primary staining solution (2.5 µg/mL biotin-labeled Plexin A4 protein solution was then added to the protein. The obtained mixture was left at rest at 4°C under light-shielded conditions for 60 minutes. Thereafter, a supernatant was removed by centrifugation, and the resultant was then washed with 500 µL of PBS twice. After that, 300 µL of secondary staining solution, in which Goat Anti-Human IgG gamma chain specific R-FITC conjugated (Southern biotech, A2040-02) and Streptavidin PE conjugated (eBioscience, 12-4317-87) had been each diluted with buffer 1000 times and 500 times, respectively, was added to the resultant, and the obtained mixture was then left at rest at 4°C under light-shielded conditions for 60 minutes. Thereafter, a supernatant was removed by centrifugation, and the resultant was then washed with 500 µL of PBS twice. The resultant was suspended in 500 µL of FACS buffer (containing PI solution (Invitrogen, P3566), which had been 1000 times diluted with PBS). This sample was subjected to a flow cytometric analysis. The results are shown in Figure 36. A cell population, in which the signals of both Plexin A4 protein and IgGγ were high, was discovered in the P5 area.

### 39-4. Screening by antigen-immobilized ELISA method

The cell culture supernatant obtained in 39-3 above was recovered, and a clone reacting with a Plexin A4 antigen was screened by an antigen-immobilized ELISA method using such a Plexin A4 antigen.

First, 20 µL of solution comprising a 2.5 µg/mL Plexin A4 antigen protein and a negative control that was a trypsin inhibitor (Sigma, T6522), streptavidin (Nacalai Tesque Inc., 32243) or ovalbumin (Sigma, A5503) was dispensed in an immuno 384-well plate Maxisorp (Nunc, 464718), and it was then reacted at 4°C overnight, so that it was immobilized on the plate. Thereafter, the reaction mixture was washed with a washing solution (PBS containing 0.05% Tween 20) three times, and 45 µL of blocking solution (PBS containing 1% BSA) was then added thereto. The obtained mixture was reacted at room temperature for 1 hour. The resultant was washed with a washing solution three times, 25 µL of a measurement sample was then added thereto, and the obtained mixture was then reacted at room temperature for 1 hour. The resultant was washed with a washing solution three times, 25 µL of Goat anti-Human IgG-Fc HRP-conjugated (Bethyl, A80-104P), which had been 2000 times diluted with PBS buffer, was then added thereto, and the obtained mixture was then reacted for 1 hour. The resultant was washed with a washing solution three times, 25 µL of TMB+ (Dako, S159985) was then added thereto, and the obtained mixture was then subjected to a coloring reaction for 5 minutes. Thereafter, 25 µL of 1 M sulfuric acid was added to the reaction mixture, so as to terminate the reaction. Using Infinite M1000 (TECAN), the absorbance at 450 nm was measured. The results are shown in Figure 37. It was demonstrated that an antibody specifically reacting with Plexin A4 was generated in almost all of the clones.

According to screening in which the flow cytometry described in 39-3 above was combined with the antigen-immobilized ELISA method described in 39-4 above, a positive clone specifically binding to Plexin A4 could be identified. In addition to the case of B7-3, even in selections in which the L15/H30 cell line #11-6 and the L30/H30 cell line 7-7-48-10 were used, a positive clone specifically binding to Plexin A4 could be identified.

### 40. Confirmation of expression of anti-Plexin A4 antibody

### 40-1. Analysis by ELISA

Among the positive clones identified in 39-5above, regarding the positive clones #62 and #20, the concentrations of chicken IgM and human IgG₁ secreted into the culture supernatant were measured. Each cell line was suspended in a medium containing no chicken serum to result in 4 × 10⁵ cells/mL, and 1 mL each of the suspension was plated on a 24-well plate. The cells were cultured for 3 days, and the culture solution was recovered and was then filtrated through a 0.22-µm filter. Thereafter, the resulting cells were used in the measurement.

The method for measuring IgG is as follows. 100 µL of Goat anti-Human IgG-Fc affinity purified (Bethyl, A80-104A), which had been adjusted to 1.0 µg/mL by dilution with PBS, was dispensed in F96 Maxisorp Nunc Immunoplate (Nunc, 464718), and it was then reacted at 4°C for 1 hour, so that it was immobilized on the plate. Thereafter, the reaction mixture was washed with a washing solution (PBS containing 0.05% Tween 20) five times, and 200 µL of blocking solution (PBS containing 1% BSA) was then added thereto. The obtained mixture was reacted at room temperature for 95 minutes. The resultant was washed with a washing solution five times, and 100 µL of a measurement sample or Human IgG1 Lambda-UNLB (Southern Biotech, 0151L-01) serving as a standard substance was then added thereto. The obtained mixture was reacted at room temperature for 1hour. The resultant was washed with a washing solution five times, and 100 µL of Goat anti-Human IgG-Fc HRP conjugated (Bethyl, A80-104P), which had been 1000 times diluted with PBS, was then added thereto. The obtained mixture was reacted at room temperature for 1 hour. The resultant was washed with a washing solution three times, and 100 µL of TMB+ (Dako, S159985) was then added thereto. The obtained mixture was subjected to a coloring reaction at room temperature for 3 minutes, and 20 µL of 1 M sulfuric acid was then added thereto, so as to terminate the reaction. Using Infinite M1000 (TECAN), the absorbance at 450 nm was measured.

The method for measuring IgM is as follows. 100 µL of Goat anti-chicken IgM (Bethyl, A30-102A), which had been diluted with PBS to 1.0 µg/mL, was dispensed in F96 Maxisorp Nunc Immunoplate (Nunc, 439454), and it was then reacted at 4°C overnight, so that it was immobilized on the plate. Thereafter, the reaction mixture was washed with a washing solution (PBS containing 0.05% Tween 20) five times, and 200 µL of blocking solution (PBS containing 1% BSA) was then added thereto. The obtained mixture was reacted at room temperature for 95 minutes. The resultant was washed with a washing solution five times, and 100 µL of chicken serum (GIBCO, 16110) serving as a measurement sample or a standard substance was added thereto. The obtained mixture was reacted at room temperature for 1 hour. The resultant was washed with a washing solution five times, and 100 µL of Goat anti-chicken IgM HRP conjugated (Bethyl, A30-102P), which had been 5000 times diluted with PBS containing 1% BSA and 0.05% Tween 20, was then added thereto. The obtained mixture was reacted at room temperature for 1 hour. The resultant was washed with a washing solution five times, and 100 µL of TMB+ (Dako, S159985) was then added thereto. The obtained mixture was subjected to a coloring reaction at room temperature for 3 minutes, and 100 µL of 1 N sulfuric acid was then added thereto, so as to terminate the reaction. Using Infinite M1000 (TECAN), the absorbance at 450 nm was measured.

The measurement results are shown in Table 37. It was confirmed that human IgG₁ was secreted from the clone #20 and the clone #62, and that the secretion level of chicken IgM was extremely small.

**[Table 37]**

| Clone name | IgG (µg/mL) | **IgM** (µg/mL) |
|---|---|---|
| #62 | 5.79 | 0.186 |
| #20 | 1.54 | 0.231 |

### 40-2. Analysis by Western blotting

Antibodies secreted from the positive clones #62 and #20, which produce the antigen-reactive antibody obtained in 17-4 above, were analyzed according to Western blotting.

Each cell line was suspended in a medium containing no chicken serum to result in 4 × 10⁵ cells/mL, and 1 mL each of the suspension was plated on a 24-well plate. The cells were cultured for 3 days, and the culture solution containing the cell line was recovered and was then filtrated through a 0.22-µm filter (Millipore, SLGV J13 SL). For detection of Igγ and Igλ, a sample purified with a Protein A column was used, and for detection of IgM, a sample before purification was used. To a sample used in detection under reduction conditions (reduction sample), Tris-SDS-β-mercaptoethanol sample treatment solution (COSMO BIO Co., Ltd., 423437) was added in an equal amount of the aforementioned sample. On the other hand, to a sample used in detection under non-reduction conditions (non-reduction sample), Tris-SDS sample treatment solution (COSMO BIO Co., Ltd., 423420) was added in an equal amount of the aforementioned sample. Thereafter, the reduction sample was reacted at 98°C for 3 minutes, and the non-reduction sample was reacted at 37°C for 30 minutes. Thereafter, the sample was electrophoresed on a 4% to 20% polyacrylamide gel (COSMO BIO Co., Ltd., 414879), and it was then transcribed on a nylon membrane. After that, it was blocked with a blocking buffer (PBS containing 1% BSA), and was then reacted with a primary antibody (Goat Anti-Human IgG-Fc Fragment Ab-HRP (Bethyl, A80-104P), Goat Anti-Human IgG Lambda-HRP (Southern Biotech, 2070-05), and Goat Anti-chicken IgM-HRP (Bethyl, A30-102P). The resultant was washed with PBS containing 0.1 % Tween 20 three times, and then, chicken IgM, human Igγ (heavy chain), and human Igλ (light chain) were detected by chemiluminescence using ECL plus (GE Healthcare, RPN2132). The results are shown in Figure 38 to Figure 40. According to Western blotting using an anti-Igy antibody, a band of 55 kDa was detected under reduction conditions, and a band of approximately 160 kDa was detected under non-reduction conditions (Figure 38). According to Western blotting using an anti-Igλ antibody, a band of 25kDa was detected under reduction conditions, and a band of approximately 160 kDa was detected under non-reduction conditions (Figure 39). Moreover, according to Western blotting using an anti-IgM antibody, no signals were detected (Figure 40).

From these results, it was confirmed that the antibody produced by the obtained positive clone is a full-length human antibody.

### Antibody selection 2

### 41. Preparation of antigen

Semaphorin 3A (Sema3A) was selected as an antigen protein. Sema3A is a molecule belonging to the Sema3 family, and has been known as a factor for controlling axon extension in the sensory nerve or the sympathetic nerve.

As an antigen, His-AP-hSema3A (SEQ ID NO: 183), in which a His tag and human alkaline phosphatase (AP) were added to the N-terminus of Sema3A, was used. A cell line HEK293 stably expressing this protein was provided by Department of Molecular Pharmacology & Neurobiology, Yokohama City University, Graduate School of Medicine.

The expression was carried out at 4 L-scale, and the culture was carried out for 4 days. Utilizing His tags, a protein of interest in the culture supernatant was purified. 5 mL of HisTrap EXCEL (GE Healthcare, 17-3712-06) was equilibrated with Solution A (20 mM Na-phosphate, 150 mM NaCl, 20 mM Imidazole (pH 7.5)), a culture supernatant was then loaded thereon, and it was then washed with Solution A. Thereafter, elution was carried out by gradient elution in which Solution A was linearly replaced with Solution B (20 mM Na-phosphate, 150 mM NaCl, 500 mM Imidazole (pH 7.5)) at 25 column volumes. The resultant was monitored with the absorbance at 280 nm, and a fraction corresponding to the peak was then recovered. The molecular weight of the recovered protein was measured by SDS-PAGE and CBB staining, and it was then confirmed that the obtained molecular weight corresponded to the molecular weight of the protein of interest.

Using an ultrafiltration membrane (Amicon, UFC0801008), approximately 80 mL of the recovered fraction was concentrated to a volume of 1.5 mL, and then, using Hi Load 16/600 Superdex 200 pg (GE Healthcare, 28-9893-35), the fraction was purified by gel filtration chromatography. The column was equilibrated with an elution buffer (50 mM Na-phosphate, 300 mM NaCl, 400 mM Arginine, pH 7.0), and the recovered fraction was then loaded thereon, followed by elution with the elution buffer. The resultant was monitored with the absorbance at 280 nm, and a fraction corresponding to the peak was then recovered. The molecular weight of the recovered protein was measured by SDS-PAGE and CBB staining. Subsequently, in order to remove arginine from the eluant, the second gel filtration chromatography was carried out with D-PBS(-). Similarly, the resultant was monitored with the absorbance at 280 nm, and a fraction corresponding to the peak was then recovered. The molecular weight of the recovered protein was measured by SDS-PAGE and CBB staining.

### 42. Selection of antibody

### 42-1. Production of antigen magnetic beads

Using Dynabeads M-270 Carboxylic Acid (Life Technologies, 14305D), Dynabeads M-270 Epoxy (Life Technologies, 14301), and Dynabeads His-Tag Isolation & Pulldown (Life Technologies, 10103D) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 40 µL of beads were washed with 80 µL of 25 mM MES (pH 5.0) three times, and 40 µL of 50 mg/mL NHS solution and 40 µL of 50 mg/mL EDC solution were added to the resulting beads. The obtained mixture was reacted at room temperature for 30 minutes, and the reaction solution was then washed with 80 µL of 25 mM MES (pH 5.0) twice. A supernatant was removed from this magnetic beads suspension. For positive selection, 50.6 µL of His-AP-hSema3A protein solution, which had been adjusted to 0.475 mg/ml with 25 mM MES (pH 5.0), was added to the above-obtained solution to make a suspension. For negative selection, PBS was used instead of the antigen solution, and 50.6 µL of the solution diluted with 25 mM MES (pH 5.0) was added to the above-obtained solution to make a suspension. The obtained suspension was reacted at 4°C overnight, while being subjected to rotary stirring. Thereafter, a supernatant was removed from the reaction solution, and 80 µL of quenching buffer (50 mM Tris-HCl) was then added thereto, followed by the reaction of the mixture at room temperature for 15 minutes while being subjected to rotary stirring. Regarding Dynabeads M-270 Epoxy, 40 µL of beads were washed with 80 µL of 100 mM Na-phosphate buffer three times. Thereafter, for positive selection, 53.3 µL of His-AP-hSema3A protein solution adjusted to 0.45 mg/ml with a 100 mM Na-phosphate buffer and 26.7 µL of 100 mM Na-phosphate buffer containing 3 M ammonium sulfate were added to the above-obtained beads to make a suspension, and for negative selection, PBS was used instead of the antigen solution, and 53.3 µL of the solution diluted with 25 mM MES (pH 5.0) and 26.7 µL of 100 mM Na-phosphate buffer containing 3 M ammonium sulfate were added to the above-obtained beads to make a suspension. The obtained suspension was reacted at 37°C overnight, while being subjected to rotary stirring. Regarding Dynabeads His-Tag Isolation & Pulldown, 8 µL of beads were washed with 80 µL of PBS three times. Thereafter, for positive selection, 101.9 µL of His-AP-hSema3A protein solution having an antigen concentration of 4.49 µM was added to the above-obtained beads to make a suspension, and for negative selection, 101.9 µL of PBS was used instead of the antigen solution, and it was then added to the above-obtained beads to make a suspension. The obtained suspension was reacted at 4°C for 10 minutes, while being subjected to rotary stirring. After the removal of a supernatant, the three types of beads were collectively suspended in 1 mL of selection buffer (PBS containing 1% BSA), and the obtained suspension was then washed with the same buffer as mentioned above three times. Thereafter, the resultant was suspended in 200 µL of selection buffer, and was then subjected to selection.

### 42-2. Selection by antigen magnetic beads

An L15/H15 cell line, B7-3, was cultured in a medium containing 2.5 ng/mL TSA for 86 days. Thereafter, approximately 1.5 × 10⁷ cells were washed with 10 mL of selection buffer, and a supernatant was then removed from the reaction solution. The remaining solution was washed with 1 mL of selection buffer, and it was then suspended in 950 µL of selection buffer. To this cell suspension, 50 µL of the antigen magnetic beads for negative selection prepared in 39-1 above was added, and the obtained mixture was then reacted at 4°C for 30 minutes, while being stirred by rotation. Thereafter, using KingFisher mL (Thermo, 5400050), the magnetic beads were removed. Subsequently, to this cell suspension, 50 µL of the antigen magnetic beads for positive selection was added, and the obtained mixture was then reacted at 4°C for 30 minutes, while being stirred by rotation. Thereafter, using KingFisher mL (Thermo, 5400050), the reaction solution was washed with 1.7 mL of selection buffer three times. Thereafter, the recovered cells were suspended in 20 mL of medium, the suspension was then plated on a 96-well plate, and it was then cultured in a CO₂ incubator.

### 42-3. Primary screening

The cell culture solution obtained in 42-2 above was recovered, and a clone specifically reacting with a target antigen was screened based on the antigen reactivity of secretory IgG in antigen-immobilized ELISA. 20 µL of antigen protein and negative control antigen solution (His-Ubiquitin, Ovalbumin, Streptavidin), which had been adjusted to 2.5 µg/mL with PBS, was dispensed on an immuno 384-well plate Maxisorp (Nunc, 464718), and it was then reacted at 4°C overnight, so that it was immobilized thereon. The resultant was washed with a washing solution (PBS containing 0.05% Tween 20) five times, and 45 µL of a blocking solution (PBS containing 1% BSA) was then added thereto. The obtained mixture was reacted at room temperature for 115 minutes. The reaction mixture was washed with a washing solution five times, and 25 µL of culture supernatant was then added thereto. The obtained mixture was reacted at room temperature 130 minutes. Thereafter, the resultant was washed with a washing solution five times, and 25 µL of Goat anti-Human IgG-Fc HRP-conjugated, which had been 2000 times diluted with a blocking solution, was added thereto. The obtained mixture was reacted at room temperature 47 minutes. The resultant was washed with a washing solution five times, 25 µL of TMB+ (Dako, S159985) was then added thereto, and the obtained mixture was then reacted for 5 minutes. Thereafter, 25 µL of 1 N sulfuric acid was added to the reaction mixture, so as to terminate the reaction, and then, using a microplate reader, the absorbance at 450 nm was measured (Figure 41). The cell having an absorbance of 0.1 or more and an S/N ratio of 5 or more was determined to be a positive cell, and it was then subjected to secondary screening.

### 42-4. Secondary screening

The cell culture supernatant obtained in 42-3 above was recovered, and it was then subjected to the secondary screening according to antigen-immobilized ELISA based on the method described in 42-3 above. The cell having an absorbance of 0.5 or more and an S/N ratio of 5 or more was determined to be a positive cell, and it was then subjected to monocloning

### 42-5. Primary screening after monocloning

The cell culture supernatant obtained in 42-4 above was recovered, and it was then subjected to screening according to antigen-immobilized ELISA based on the method described in 42-3 above. The cell having an absorbance of 0.2 or more and an S/N ratio of 5 or more was determined to be a positive cell, and it was then subjected to secondary screening.

### 42-6. Secondary screening after monocloning

The cell culture supernatant obtained in 42-5 above was recovered, and it was then subjected to screening according to antigen-immobilized ELISA based on the method described in 42-3 above. Positive clones #64, #69 and #77, which had an absorbance of 0.5 or more and an S/N ratio of 5 or more, were selected (Figure 42).

### 43. Confirmation of expression of anti-His-AP-hSema3A antibody

### 43-1. Analysis by ELISA

Among the positive clones selected in 42-6 above, regarding the positive clone #64, the concentrations of chicken IgM and human IgG₁ secreted into the culture supernatant were measured. The measurement method was as described in 40-1 above.

The measurement results are shown in Table 38. It was confirmed that, as with wild-type DT40, human IgG was secreted from the positive clone #64, and that secretion of chicken IgM was not observed.

**[Table 38]**

| Clone name | **IgG** (µg/mL) | Chicken IgM (µg/mL) |
|---|---|---|
| #64 | 2.66 | <0.0005 |

### 43-2. Analysis by Western blotting

According to Western blotting, an antibody secreted from the positive clone #64 producing an antigen-reactive antibody obtained in 42-6 above was analyzed.

Each cell line was suspended in a medium containing no chicken serum to result in 4 × 10⁵ cells/mL, and 20 mL of the suspension was plated on a 9-cm dish. The cells were cultured for 4 days, and the culture solution containing the cell line was recovered and was then filtrated through a 0.22-µm filter (Millipore, SLGV J13 SL). For detection of Igγ and Igλ, a sample purified with a Protein A column was used, and for detection of IgM, a sample before purification was used. To a sample used in detection under reduction conditions (reduction sample), Tris-SDS-β-mercaptoethanol sample treatment solution (COSMO BIO Co., Ltd., 423437) was added in an equal amount of the aforementioned sample. On the other hand, to a sample used in detection under non-reduction conditions (non-reduction sample), Tris-SDS sample treatment solution (COSMO BIO Co., Ltd., 423420) was added in an equal amount of the aforementioned sample. Thereafter, the reduction sample was reacted at 98°C for 3 minutes, and the non-reduction sample was reacted at 37°C for 30 minutes. Thereafter, the sample was electrophoresed on XV PANTERA 5-20% T-HCL 10W (DRC, NXV-275HP), and it was then transcribed on a PVDF membrane. After that, it was blocked with a blocking buffer (TBS containing 5 % skimmed milk and 0.1 % Tween 20), and was then reacted with a primary antibody (Goat Anti-Human IgG-Fc Fragment Ab-HRP (Bethyl, A80-104P) and Goat Anti-Human IgG Lambda-HRP (Southern Biotech, 2070-05)). The resultant was washed with TBS containing 0.1 % Tween 20 three times, and then, chicken IgM, human Igγ (heavy chain), and human Igλ (light chain) were detected by chemiluminescence using Luminata Forte Western HRP substrate (Millipore, WBLUF0100). The results are shown in Figure 43. According to Western blotting using an anti-Igy antibody, a band of 55 kDa was detected under reduction conditions (Figure 43A), and a band of approximately 160 kDa was detected under non-reduction conditions (Figure 43B). According to Western blotting using an anti-Igλ antibody, a band of 25kDa was detected under reduction conditions (Figure 43C), and a band of approximately 160 kDa was detected under non-reduction conditions (Figure 43D).

From these results, it was confirmed that the antibody produced by the obtained positive clone is a full-length human antibody.

### Antibody selection 3

### 44. Preparation of antigen

IL-8 was selected as an antigen protein. IL-8 is one type of chemokine. IL-8 exhibits chemotaxis to neutrophils and T lymphocytes, and has an activity of promoting adhesion of leucocytes to vascular endothelial cells or the functions of neutrophils. Hence, it is considered that IL-8 is associated with inflammatory disease, rheumatoid arthritis and other diseases, which are attended with infiltration of neutrophils.

As antigens, human IL-8 (Immune TECH, IT-401-003P) was used in selection, and two types of human IL-8 (Immune TECH, IT-401-003P, and CELL Signaling Technology, 8921LF) were used in screening.

### 45. Selection of antibody

### 45-1. Production of antigen magnetic beads

Using Dynabeads M-270 Carboxylic Acid (Life Technologies, 14305D), Dynabeads M-270 Epoxy (Life Technologies, 14301) and Dynabeads His-Tag Isolation & Pulldown (Life Technologies, 10103D) as magnetic beads, these beads were allowed to bind to the antigen in accordance with the instruction manual. At this time, MPC-S (Life Technologies, DBA13346) was used as a magnetic stand. Regarding Dynabeads M-270 Carboxylic Acid, 35 µL of beads were washed with 70 µL of 25 mM MES (pH 5.0) three times, and 35 µL of 50 mg/mL NHS solution and 35 µL of 50 mg/mL EDC solution were added to the resulting beads. The obtained mixture was reacted at room temperature for 30 minutes, and the reaction solution was then washed with 70 µL of 25 mM MES (pH 5.0) twice. A supernatant was removed from this magnetic beads suspension. For positive selection, 35 µL of IL-8 (Immune TECH, IT-401-003P) protein solution adjusted to 0.6 mg/ml with 25 mM MES (pH 5.0) was added to the above-obtained solution to make a suspension. For negative selection, PBS was used instead of the antigen solution, and 35 µL of the solution diluted with 25 mM MES (pH 5.0) was added to the above-obtained solution to make a suspension. The obtained suspension was reacted at 4°C overnight, while being subjected to rotary stirring. Thereafter, a supernatant was removed from the reaction solution, and 70 µL of quenching buffer (50 mM Tris-HCl) was then added thereto, followed by the reaction of the mixture at room temperature for 15 minutes while being subjected to rotary stirring. Regarding Dynabeads M-270 Epoxy, 35 µL of beads were washed with 70 µL of 100 mM Na-phosphate buffer three times. Thereafter, for positive selection, 46.7 µL of IL-8 protein solution adjusted to 0.45 mg/ml with a 100 mM Na-phosphate buffer and 23.3 µL of 100 mM Na-phosphate buffer containing 3 M ammonium sulfate were added to the above-obtained solution to make a suspension, and for negative selection, PBS was used instead of the antigen solution, and 46.7 µL of the solution diluted with 100 mM Na-phosphate buffer and 23.3 µL of 100 mM Na-phosphate buffer containing 3 M ammonium sulfate were added to the above-obtained solution to make a suspension. The obtained suspension was reacted at 37°C overnight, while being subjected to rotary stirring. Regarding Dynabeads His-Tag Isolation & Pulldown, 7 µL of beads were washed with 70 µL of PBS three times. Thereafter, for positive selection, 70 µL of IL-8 protein solution having an antigen concentration of 5.75 µM was added to the above-obtained solution to make a suspension, and for negative selection, 70 µL of PBS was used instead of the antigen solution, and it was added to the above-obtained solution to make a suspension. The obtained suspension was reacted at 4°C for 210 minutes, while being subjected to rotary stirring. After the removal of a supernatant, the three types of beads were collectively suspended in 1 mL of a selection buffer (PBS containing 1% BSA), and the obtained suspension was then washed with the same buffer as mentioned above three times. Thereafter, the resultant was suspended in 700 µL of selection buffer, and was then subjected to selection.

### 45-2. Selection by antigen magnetic beads

An L30/H15f15f15f cell line, 2-1-3, was cultured in a medium containing 2.5 ng/mL TSA for 45 days. Thereafter, approximately 1.5 × 10⁷ cells were washed with 10 mL of selection buffer, and a supernatant was then removed from the reaction solution. The remaining cells were washed with 10 mL of selection buffer, and it was then suspended in 950 µL of selection buffer. To this cell suspension, 50 µL of the antigen magnetic beads for negative selection prepared in 39-1 above was added, and the obtained mixture was then reacted at 4°C for 30 minutes, while being stirred by rotation. Thereafter, using KingFisher mL (Thermo, 5400050), the magnetic beads were removed. Subsequently, to this cell suspension, 50 µL of the antigen magnetic beads for positive selection was added, and the obtained mixture was then reacted at 4°C for 30 minutes, while being stirred by rotation. Thereafter, using KingFisher mL (Thermo, 5400050), the reaction solution was washed with 1.7 mL of selection buffer three times. Thereafter, the recovered cells were suspended in 20 mL of medium, the suspension was then plated on a 96-well plate, and it was then cultured in a CO₂ incubator.

### 45-3. Screening and identification of positive clones

In the same matter as described in 42-3 to 42-6 above, two times of screening, monocloning, and two times of screening were carried out to obtain positive clone #117, #121 and #123. The results of the primary screening after completion of the selection are shown in Figure 44, and the results of the secondary screening after completion of the monocloning are shown in Figure 45.

### 46. Confirmation of expression of anti-IL-8 antibody

### 46-1. Analysis by ELISA

Regarding the positive clones #117 and #121 generating antigen-reactive antibodies obtained in 45-3 above, the concentrations of chicken IgM and human IgG₁ secreted into the culture supernatant were measured. The measurement method was as described in 40-1 above.

The measurement results are shown in Table 39. It was confirmed that, as with wild-type DT40, human IgG was secreted from the positive clones #117 and #121, and that secretion of chicken IgM was not observed.

**[Table 39]**

| **Clone name** | **IgG (µg/mL)** | **IgM (µg/mL)** |
|---|---|---|
| #117 | 3.65 | <0.041 |
| #121 | 3.7 | <0.041 |

### 46-2. Analysis by Western blotting

According to Western blotting, antibodies secreted from the positive clones #117 and #121 were analyzed. The method was as described in 40-2 above.

The results are shown in Figure 46. According to Western blotting using an anti-Igy antibody, a band of 55 kDa was detected under reduction conditions (Figure 46A), and a band of approximately 160 kDa was detected under non-reduction conditions (Figure 46B). According to Western blotting using an anti-Igλ antibody, a band of 25 kDa was detected under reduction conditions (Figure 46C), and a band of approximately 160 kDa was detected under non-reduction conditions (Figure 46D).

From these results, it was confirmed that the antibody produced by the obtained positive clone is a full-length human antibody.

### Industrial Applicability

A method is described for promptly producing a variety of human antibodies. Considering the importance of antibody drugs, it is anticipated that the technique provided herein will play an extremely important role in the development of biotechnology-based pharmaceutical products exhibiting desired drug effects, in particular, antibody drugs, in the field of future drug discovery and medical services.

### Sequence Listing

### TPC0130CBS-sequenching_ST25.txt

## Claims

1. A chicken B cell, in which the chicken antibody light chain gene locus is replaced by the DNA sequences of a human antibody light chain variable region and by the DNA sequences of a human antibody light chain constant region,
and in which in the chicken antibody heavy chain gene locus the DNA sequences of a human antibody heavy chain variable region and of a human antibody heavy chain constant region are inserted,
and in which the chicken antibody light chain pseudogene locus is replaced by 25 or more DNA sequences of human antibody light chain variable regions,
and in which in the chicken antibody heavy chain pseudogene locus 30 or more DNA sequences of human antibody heavy chain variable regions are inserted,
wherein the chicken B cell has the ability to express a human antibody on the cell surface and also to secrete the human antibody into the culture solution.

2. The chicken B cell according to claim 1, wherein the position of the DNA sequence of the human antibody light chain variable region inserted into the antibody light chain gene locus is upstream of the position of the DNA sequence of the human antibody light chain constant region, and the position of the DNA sequence of the human antibody heavy chain variable region inserted into the antibody heavy chain gene locus is upstream of the position of the DNA sequence of the human antibody heavy chain constant region.

3. The chicken B cell according to claim 1 or 2, wherein the human antibody heavy chain is a γ chain.

4. The chicken B cell according to any one of claims 1 to 3, wherein the human antibody light chain is a λ chain or a κ chain.

5. The chicken B cell according to any one of claims 1 to 4, which is a DT40 cell.

6. The chicken B cell according to any one of claims 1 to 5, which has undergone a treatment for relaxing chromatin.

7. The chicken B cell according to claim 6, wherein the treatment for relaxing chromatin is reduction or deletion of the function of histone deacetylase in the chicken B cell.

8. The chicken B cell according to claim 7, wherein the method for reducing or deleting the function of histone deacetylase is reduction or deletion of the expression of a histone deacetylase gene in the chicken B cell.

9. The chicken B cell according to claim 8, wherein the histone deacetylase is HDAC2.

10. The chicken B cell according to claim 7, wherein the method for reducing or deleting the function of histone deacetylase is a treatment with an HDAC inhibitor.

11. An antibody-producing cell library consisting of chicken B cells according to any one of claims 6 to 10.

12. A method for producing a human antibody from the chicken B cell according to any one of claims 1 to 10, comprising culturing said chicken B cell under culturing conditions suitable for the cell to produce a human antibody and obtaining said antibody.

## Patentansprüche

1. Huhn-B-Zelle, in der der Gen-Locus der leichten Kette des Huhn-Antikörpers durch die DNA-Sequenzen einer variablen Region der leichten Kette eines humanen Antikörpers und durch die DNA-Sequenzen einer konstanten Region der leichten Kette eines humanen Antikörpers ersetzt ist,
und in der im Gen-Locus der schweren Kette des Huhn-Antikörpers die DNA-Sequenzen einer variablen Region der schweren Kette des humanen Antikörpers und einer konstanten Region der schweren Kette des humanen Antikörpers inseriert sind,
und in der der Pseudogen-Locus der leichten Kette des Huhn-Antikörpers durch 25 oder mehr DNA-Sequenzen der variablen Regionen der leichten Kette des humanen Antikörpers ersetzt ist,
und in der in dem Pseudogen-Locus der schweren Kette des Huhn-Antikörpers 30 oder mehr DNA-Sequenzen der variablen Regionen der schweren Kette des humanen Antikörpers inseriert sind,
wobei die Huhn-B-Zelle die Fähigkeit aufweist, einen humanen Antikörper auf der Zelloberfläche zu exprimieren und auch den humanen Antikörper in die Kulturlösung zu sekretieren.

2. Huhn-B-Zelle nach Anspruch 1, wobei die Position der DNA-Sequenz der variablen Region der leichten Kette des humanen Antikörpers, die in den Gen-Locus der leichten Kette des Antikörpers inseriert ist, stromaufwärts der Position der DNA-Sequenz der konstanten Region der leichten Kette des humanen Antikörpers gelegen ist, und die Position der DNA-Sequenz der variablen Region der schweren Kette des humanen Antikörpers, die in den Gen-Locus der schweren Kette des Antikörpers inseriert ist, stromaufwärts der Position der DNA-Sequenz der konstanten Region der schweren Kette des humanen Antikörpers gelegen ist.

3. Huhn-B-Zelle nach Anspruch 1 oder 2, wobei die schwere Kette des humanen Antikörpers eine γ-Kette ist.

4. Huhn-B-Zelle nach einem der Ansprüche 1 bis 3, wobei die leichte Kette des humanen Antikörpers eine λ-Kette oder eine κ-Kette ist.

5. Huhn-B-Zelle nach einem der Ansprüche 1 bis 4, die eine DT40-Zelle ist.

6. Huhn-B-Zelle nach einem der Ansprüche 1 bis 5, die einer Behandlung zur Chromatin-Relaxation unterzogen wurde.

7. Huhn-B-Zelle nach Anspruch 6, wobei die Behandlung zur Chromatin-Relaxation die Verringerung oder Deletion der Funktion von Histon-Deacetylase in der Huhn-B-Zelle ist.

8. Huhn-B-Zelle nach Anspruch 7, wobei das Verfahren zur Verringerung oder Deletion der Funktion von Histon-Deacetylase die Verringerung oder Deletion der Expression eines Histon-Deacetylase-Gens in der Huhn-B-Zelle ist.

9. Huhn-B-Zelle nach Anspruch 8, wobei die Histon-Deacetylase HDAC2 ist.

10. Huhn-B-Zelle nach Anspruch 7, wobei das Verfahren zur Verringerung oder Deletion der Funktion von Histon-Deacetylase eine Behandlung mit einem HDAC-Inhibitor ist.

11. Antikörper-produzierende Zellbibliothek, die aus Huhn-B-Zellen nach einem der Ansprüche 6 bis 10 besteht.

12. Verfahren zur Herstellung eines humanen Antikörpers aus der Huhn-B-Zelle nach einem der Ansprüche 1 bis 10, umfassend das Züchten der Huhn-B-Zelle unter Kulturbedingungen, die für die Zelle geeignet sind, um einen humanen Antikörper herzustellen, und Erhalten des Antikörpers.

## Revendications

1. Cellule B de poulet, dans laquelle le locus de gène de chaîne légère d'anticorps de poulet est remplacé par les séquences d'ADN d'une région variable de chaîne légère d'anticorps humain et par les séquences d'ADN d'une région constante de chaîne légère d'anticorps humain,
et dans laquelle les séquences d'ADN d'une région variable de chaîne lourde d'anticorps humain et d'une région constante de chaîne lourde d'anticorps humain sont insérées dans le locus de gène de chaîne légère d'anticorps de poulet,
et dans laquelle le locus de pseudo-gène de chaîne légère d'anticorps de poulet est remplacé par 25 ou plus de 25 séquences d'ADN de régions variables de chaîne légère d'anticorps humain,
et dans laquelle 30 ou plus de 30 séquences d'ADN de régions variables de chaîne lourde d'anticorps humain sont insérées dans le locus de pseudo-gène de chaîne lourde d'anticorps de poulet,
dans laquelle la cellule B de poulet a la capacité d'exprimer un anticorps humain sur la surface cellulaire et aussi de sécréter l'anticorps humain dans la solution de culture.

2. Cellule B de poulet selon la revendication 1, dans laquelle la position de la séquence d'ADN de la région variable de chaîne légère d'anticorps humain insérée dans le locus de gène de chaîne légère d'anticorps est en amont de la position de la séquence d'ADN de la région constante de chaîne légère d'anticorps humain, et la position de la séquence d'ADN de la région variable de chaîne lourde d'anticorps humain insérée dans le locus de chaîne lourde d'anticorps est en amont de la position de la séquence d'ADN de la région constante de chaîne lourde d'anticorps humain.

3. Cellule B de poulet selon la revendication 1 ou 2, dans laquelle la chaîne lourde d'anticorps humain est une chaîne γ.

4. Cellule B de poulet selon l'une quelconque des revendications 1 à 3, dans laquelle la chaîne légère d'anticorps humain est une chaîne λ ou une chaîne κ.

5. Cellule B de poulet selon l'une quelconque des revendications 1 à 4, qui est une cellule DT40.

6. Cellule B de poulet selon l'une quelconque des revendications 1 à 5, qui a subi un traitement pour relâcher la chromatine.

7. Cellule B de poulet selon la revendication 6, dans laquelle le traitement pour relâcher la chromatine est une réduction ou une délétion de la fonction d'histone désacétylase dans la cellule B de poulet.

8. Cellule B de poulet selon la revendication 7, dans laquelle la méthode pour réduire ou supprimer la fonction d'histone désacétylase est une réduction ou une délétion de l'expression d'un gène d'histone désacétylase dans la cellule B de poulet.

9. Cellule B de poulet selon la revendication 8, dans laquelle l'histone désacétylase est HDAC2.

10. Cellule B de poulet selon la revendication 7, dans laquelle la méthode pour réduire ou supprimer la fonction d'histone désacétylase est un traitement avec un inhibiteur de HDAC.

11. Bibliothèque de cellules productrices d'anticorps consistant en des cellules B de poulet selon l'une quelconque des revendications 6 à 10.

12. Méthode pour produire un anticorps humain à partir de la cellule B de poulet selon l'une quelconque des revendications 1 à 10, comprenant la culture de ladite cellule B de poulet dans des conditions de culture convenant pour que la cellule produise un anticorps humain, et l'obtention dudit anticorps.


### SEQUENCE LISTING

<110> Chiome Bioscience Inc.
<120> Cells producing human antibodies
<130> TPC0130CBS
<150> JP2014- 95236
   <151> 2014-05-02
<160> 183
<170> PatentIn version 3.5
<210> 1
   <211> 21
   <212> DNA
   <213> Artificial Sequence
<220>
   <223> Synthetic Primer
<400> 1
   tccaccatgg cctgggctct g 21
<210> 2
   <211> 33
   <212> DNA
   <213> Artificial Sequence
<220>
   <223> Synthetic Primer
<400> 2
   gttgagaacc tatgaacatt ctgtaggggc cac 33
<210> 3
   <211> 54
   <212> DNA
   <213> Artificial Sequence
<220>
   <223> Synthetic Primer
<400> 3
   attggcgcgc ctctccaggt tccctggtgc aggcacagtc tgccctgact cagc 54
<210> 4
   <211> 40
   <212> DNA
   <213> Artificial Sequence
<220>
   <223> Synthetic Primer
<400> 4
   ttccatatga gcgactcacc taggacggtc agcttggtcc 40
<210> 5
   <211> 334
   <212> DNA
   <213> Homo sapiens
<400> 5
   cagtctgccc tgactcagcc tgcctccgtg tctgggtctc ctggacagtc gatcaccatc 60
   tcctgcactg gaaccagcag tgacgttggt ggttataact atgtctcctg gtaccaacaa 120
   aacccaggca aagcccccaa actcatgatt tatgatgtca gtaatcggcc ctcagggatt 180
   tctaatcgct tctctggctc caagtctggc aacacggcct ccctgaccat ctctgggctc 240
   caggctgacg acgaggctga ttattactgc acctcatata caaacgacag caattctcag 300
   gtattcggcg gagggaccaa gctgaccgtc ctag 334
<210> 6
   <211> 51
   <212> DNA
   <213> Artificial Sequence
<220>
   <223> Synthetic Primer
<400> 6
   attggcgcgc ctctgcctct ctcttgcagg tcagcccaag gctgccccct c 51
<210> 7
   <211> 44
   <212> DNA
   <213> Artificial Sequence
<220>
   <223> Synthetic Primer
<400> 7
   ggaattccat atggagtggg actactatga acattctgta gggg 44
<210> 8
   <211> 320
   <212> DNA
   <213> Homo sapiens
<400> 8
   gtcagcccaa ggctgccccc tcggtcactc tgttcccgcc ctcctctgag gagcttcaag       60
   ccaacaaggc cacactggtg tgtctcataa gtgacttcta cccgggagcc gtgacagtgg      120
   cctggaaggc agatagcagc cccgtcaagg cgggagtgga gaccaccaca ccctccaaac      180
   aaagcaacaa caagtacgcg gccagcagct acctgagcct gacgcctgag cagtggaagt      240
   cccacagaag ctacagctgc caggtcacgc atgaagggag caccgtggag aagacagtgg      300
   cccctacaga atgttcatag                                                  320
<210> 9
   <211> 20
   <212> DNA
   <213> Artificial Sequence
<220>
   <223> Synthetic Primer
<400> 9
   gagatctcct cctcccatcc 20
<210> 10
   <211> 20
   <212> DNA
   <213> Artificial Sequence
<220>
   <223> Synthetic Primer
<400> 10
   caaaggacac gacagagcaa 20
<210> 11
   <211> 28
   <212> DNA
   <213> Artificial Sequence
<220>
   <223> Synthetic Primer
<400> 11
   tgtctcgagt gaaggtcacc aaggatgg 28
<210> 12
   <211> 28
   <212> DNA
   <213> Artificial Sequence
<220>
   <223> Synthetic Primer
<400> 12
   ttaagcttgg agaggagaga ggggagaa 28
<210> 13
   <211> 1491
   <212> DNA
   <213> Gallus gallus
<400> 13
   tcgagtgaag gtcaccaagg atggagactt cagagctatc tgggacacgt gttttaacgt      60
   ttgatcagcc ttgcagcaaa acagtgtttt gctctattga acagtgttca acaaaaacga     120
   tcagtggtct ggaacacatc tcctacgaag aaaggttgcg agagctgggt ctggagaaga     180
   ggaggctctg gggcaacctc cctgtggcct gtcagtgctt aaaggaggct caagagaaag     240
   atggagagag actttataca cggagctgta gcaatatgac aaggagaaat ggtttaaaat     300
   ggaaagaggg tagatttagg ttagctatca agaagaaatt cttcactcag aggtgatgaa     360
   gcactgtagc aggttgccca gagaagtatt ggatgcttcc ttcctggcag ggcccaaggc     420
   cagactggat gggttctggg cagcctgttc tggtgggaga tgtccctgcc cgtggcacgg     480
   cttggaaatg ggtagccttt gaggtccctc tcagcccaaa tcattctatg ttcttgtggt     540
   tccctgcatt tcagcctttg cctgttgact ctggtgccac tgttggacaa ttctactcag     600
   ctgtgtttca tccctgcccc aaagcagtaa tcccatggat aagcaatgct ctgaggcctt     660
   caccaccatc agggctcctg ctgaccctag cggcctccct tgcagcatga gccctttgtt     720
   gtccgcactg ctgctgctga gggtcctgcc agccccccag ggccttttct gcccaggcac     780
   tgccacctgc taaaacccag cctgcccatg gcagggcatc gcatgtctga gggatggaac     840
   ggggcaaggg cagtgctgca ggcagtggtt caagaaacgt ttagatgttg tactgaggga     900
   tgtggtttag tgggaaatac tggtgatagg tggatggttg gactggagga tcttagaggt     960
   cttttccaac cttggtgatt atgtgattct atgaagggag ccatagcctg ctcccatcat    1020
   gccccgccag tccggctctc ccgagcactt tggttccgca gcatccaggc ccgctctggg    1080
   gccggtgtgg gcctgggtgc tgccacgtgt ccccgctgca aggcctggcc aaccccgctg    1140
   tgccacaatg caggggctgg gctaggagtg ggcagggagg tgtggggaga ggtgtaggac    1200
   gtggctggga gcgcagggag ttatttgcat aggggggcgt gctgcggaag gacgcgggta     1260
   taaaagggca tcgaggtccc cggcacagcc ccatcggcgt ggggacacac agctgctggg    1320
   attccgccat ggcctgggct cctctcctcc tggcggtgct cgcccacacc tcaggtactc    1380
   gttgcgcccg gtcggggact gtgggcacgg ggctctgtcc cactgctgcg cgggcagggc    1440
   tgtgcgtgcg gggccgtcac tgattgccgt tttctcccct ctctcctctc c             1491
<210> 14
   <211> 1712
   <212> DNA
   <213> Gallus gallus
<400> 14
   tctagagacc tcgtctcggt ctttcttccc ccatcgtgaa attgtgacat tttgtcgatt      60
   tttggtgatt tgggggtttt tcttggactt ggcggcaggc tggggtctgc caccggcgca     120
   gggccgggca ctcagcgcga cagcctgggc tgagtcttgt ccccaccgag ccggagggct     180
   ccggtgtgcg ccatggagga cttagggtta ttttgtcaat ggaaagttct taaaatttga     240
   ccagaaaatg tgcccgaggt ctgtctctgc cacacgattt cagaaattgt gtctaggtcg     300
   atgagaagac agtttttgtc tttgtcagga aattagttgt gagttgttag tccttccctc     360
   ttagtcctaa ggactaagac ctttgtcccc ggtctggtct ctcactgggg actcttggct     420
   ccagtgccat ggggagccca agtgtcactg acacggtgtc cttgggggtg aaattcagtt     480
   tttcagctgt ccaggtaagt gctgtcaaaa aatggaggtt attctgctga aaaagctgag      540
   aggaatattt tgtcattttt ttcggaaata tatatatata tatatatata aatatataaa      600
   ttatttatat attttatata tatatatata taaaatatat gtatttctct ctttcttttt      660
   atatatatat ataaatatat atatatttct ccctctttct ttatatatat ttatagggag      720
   agaatctata tttttggcca atttggccaa tttctctctc tctctcttta tatatatata      780
   tatatgtata tgtaatttat atatatatat ataaatttat atttatttat ttatttattt      840
   atatagatta tttttatata catatatata tataaatttg gccaatttag aagaaattgg      900
   ccaaaaaaac ccaatctgaa ccaaattact tagggcataa gtccaaaaaa aaagtgcagt      960
   tcaagaattc ctcgctggaa agtttttaat gggggcagtt cttccccatt ttctggtgta     1020
   gatatgagtg aagatataga tatgtatgta gatatagatt tggctatagt ttgtttgatg     1080
   aacagtctga ggaagagtaa atcttgcctt cctcatggca atttttaatt ctacttcttc     1140
   tgagacaagt ctgaacatgc cttgaggtag aaagagcccg aaaattgagt gctttttctt     1200
   tgaaatttta attaaaatta aaaaatagcc ttacagcagg aaagaaaggg ggtgatggca     1260
   gctaatcgga gttccaaagc tgaaccacgt tcacccaaac acgtggttga aattttgtgt     1320
   gtgtatatat ataaatatat atatgtatac aaaagaaaat gcctatatat aaaaaaattg     1380
   ttatgtatat tatatagatt tattatagat atgtacataa gatataacac gtatacatat     1440
   atatgtagtc tgctgtatgc tttgtataaa tttttataga tatgatgtat attatatgca     1500
   tgggttatat atctataata tattttatct gtagaaggaa ctacagtata aaatgcatat     1560
   atgcgtatat atacacatat ttatatatat gtgtatatat atgtatacgc acacgtataa     1620
   catccatgaa aatagatatg gatgggtgga tgtgtttgtt ttacagaggt gcatgtgtgt     1680
   ctgtacatac acagccatac atacgcgtgt gg                                    1712
<210> 15
   <211> 1992
   <212> DNA
   <213> Gallus gallus
<400> 15
   acgacactga actagtgctg actctgcatc catgtctctg tgtccttttg cgtgctgtct      60
   gcatctcaca caggggggtc ggccccagta tggggaaggg ctggggggcg catacacaca      120
   tattggtaat gttgggggcg gggggggggg gtgggggggt caacagatca gcactggaga      180
   cactggtgta taccctggca ccaccaacat ctaaggcagg gtgctttggg gcaattttgg      240
   ggcagtttaa ggtctgtgct ggcactgagc acgtggctgt ggccgtgctg tcctcatctc      300
   ccacccacta cggtctgtgc gccaggtccc tagcagagat ttgctttatg ctgggaacag      360
   ggggagttct ggctctgttc ccttgcattc agacaccctg gtgccccctg ggtgggatgt      420
   cagtgtgaat actcctttgt gccctgtgcc tgcagcagcc tgaccctcca cacaccacac 480
   gccttgtgtg caccccaccc ctgtcactat ccctctcccc gctccccagg gagattttgc 540
   agtggcccct gtagggcagc ttttagcaca gcccccagca gcaagcaagc agaaagcact 600
   gctgtgcaca gcttgtcagc tgtgtgtgtt tgctgaggag gatctgtctt ttgctgaggc 660
   catcagtctt gtcctgctca acctccatcg atgctgccca cctcaacaca tctacccatc 720
   tattccatct acaccaacat ctccattcat cccacccacc caaacatgtc catccatcac 780
   aacacctcca tccaacccgc acactccagc acctccaatc attccatcta caccaccatg 840
   ctgatctgct acagccactc caacgcaacc gtccattcca tctacaccaa tgtccatcca 900
   tcccagccac tccagcacct ccagccatcc cacccaccct atgtctccat ccagccactg 960
   gtggggtgca ggacatgggg ccagctctac tgtcaggact ggggtttttg catggcccca 1020
   taccacttct gcagaagaga cgcactgaaa gtttggctga ccattttctc cacggtagag 1080
   ttgtggcagt tctgtaattt agggtctttt atccagtttg gagatgggct gggatctccc 1140
   agctccatgg caggcattca tgacactggg tttagtatct gatgggtggg atgtggctga 1200
   acttcatttt ctttccccag tgacaaagtt tttgcagttg aatatgaatt cctgctttct 1260
   gctctatgag ttgttttttt cccaggacgt acacagggaa tcagcagtct tcattctccc 1320
   tctgccatgt gtagactctc tgccacacag gactgtgctg tcctcatgcc cctgcgccca 1380
   aattgctgcc ctctgcccat gcctgccaag ctgagccctg caggctgcca tgctggattg 1440
   acatgagccc tgagattggt acagaaatgg tgattttggg gttttctttg cactcaggaa 1500
   gctgaaggct caatgctcag tgatggattt accaaactgt gccctgaggc agctgctcat 1560
   gctggataaa gtcactggag cacagggaac caggcgctgg gcagggattt ctcatgggcc 1620
   ccacttggaa agctgcaggc tgcaaacctg gctctgcctt cacgcctcac cctcatgagg 1680
   acaacctcac taattattga ttaaaagatt ttgctaaacc atctccagaa gcaacaaccc 1740
   actgaggagc atgtgctgaa ttatacatca cagctccacg gccctgccct catagcaggg 1800
   ctgcatggca cccacagtgg cactcagagg gaccacaggg ctgagacagc cgggtctggt 1860
   ggtggggaca cagctgagca taggatgagc cccccgggca gtgctgggct ttgctaatga 1920
   gcagaagtat ggatagaaag caaccccagg gctccgtacc cagctgcagc tcttgctctg 1980
   tcgtgtcctt tg 1992
<210> 16
   <211> 11734
   <212> DNA
   <213> Artificial Sequence
<220>
   <223> Targeting vector
<400> 16
<210> 17
   <211> 335
   <212> DNA
   <213> Artificial Sequence
<220>
   <223> Synthetic DNA
<400> 17
   cagtctgccc tgactcagcc tgcctccgtg tctgggtctc ctggacagtc gatcaccatc 60
   tcctgcactg gaaccagcag tgacgttggt tataactatg tctcctggta ccaacaaaac 120
   ccaggcaaag cccccaaact catgatttat gatggtaatc ggccctcagg gatttctaat 180
   cgcttctctg gctccaagtc tggcaacacg gcctccctga ccatctctgg gctccaggct 240
   gacgacgagg ctgattatta ctgcacctca tatacaaacg acagctctca ggtattcggc 300
   ggagggacca agctgaccgt cctaggtggc gcgcc 335
<210> 18
   <211> 26
   <212> DNA
   <213> Artificial Sequence
<220>
   <223> Synthetic Primer
<400> 18
   ttctgtgagc tgagaaaagg agtgta 26
<210> 19
   <211> 24
   <212> DNA
   <213> Artificial Sequence
<220>
   <223> Synthetic Primer
<400> 19
   cctgcattgt ggcacagcgg ggtt 24
<210> 20
   <211> 2387
   <212> DNA
   <213> Gallus gallus
<400> 20
   tagatgggag tttaagagtg aattgtggtc catggcatat gcctgtcagc atagggatgc      60
   tgtaggaatt gcaccttggc ttgagcccct ctcctcctga tgatgcttgc ccacagctct     120
   gatgtcctgg gaaggctcac tgcaccttgt tgtcagtgaa actgcaataa actatcaaga     180
   tttgatataa ttctgcccct ctgttctact tgtgagacca cacctggagt actgcatcca     240
   gttctggtac cactgcatcc agttctggta ccaccaacac aaaaaggaca tggagctgct     300
   ggagcagggc cagaggaggg ccatgaagat gaacagaggg ctgaagcacc tcccctatgg     360
   ggacaggctg agagacatgg ggcttttgag actggagaag agaaggctcc atgggacctt     420
   tcagtgtcct tccaatactt gaaagggtcc ttgaggaaag ctggggaggg acttcctata     480
   aggacatata gcaacaggac aaaaggaaag ggttttaaac tggaagaaag tagatttagt     540
   ctagatagta acaagaaatt ctttacagta aggatcacga gacactgaaa caggttgccc     600
   aaagcagctg tggatgcccc ctccctggaa gtgttccagg ccaggctgga tggggctttg     660
   agcaacctgt tctagtgaga ggtgaccctg cctacagcag gggagttgaa cctagatggt     720
   cttaaatgtc ccttccaacc caaatcattc tatggcaatt ctatagagaa cacctatgct     780
   gagagtagct acaagtatgg ctggttcctg gcagtgcccc tgtctctgtg atctatagca     840
   gcaccaacag accctcaaac atcccttcac gattctccgg ttccaaatcc ggctccacac     900
   acacattaac catcactggg gtccaagccg atgacaaggc tgtctattac tgtgggagca     960
   cagacagcag ctatgttggt atcacagtga catggagcaa ttaaaaatga tgcataacct    1020
   tttgcacatc tgtaatgcta taaggtgaga gaaagtaagc aataatagca gagcagcaca    1080
   atcctaggct acagcaaatg agaacaagcc cagatgcagc cacggaaata aagaaaaaaa    1140
   aactgcttac ctttagaaag cctcatatag acagggatct ccagcaagat acccacacct    1200
   ccactgccaa cccttaaatg agggctggga agggctggat cctggctcca cccctttcaa    1260
   tcactcaggt acattgcaca cacctgagct cccctggctg gccctgcctt cccaccaggt    1320
   gctcaatcac tgctttgggc tgtgactcag catctccact acagcatcac agggagcggt    1380
   tgtcggtctc tgctgttcct atgtgggtgc cccgggacac ctgaaggcat gagtggcagt    1440
   gagggctccc agctctatcc atggtgaaca gacattatgt ttatgcttta cagcagcatg    1500
   aatctggcag tggtgttatt gctgtgatgc tgctgtctat gctcccacag taatagatag    1560
   cctcgtcctc ggcttggacc ccagtgatgg ttaatgtgtt tgtggagccg gatccagaac    1620
   cggagaatcg tgaagggatg tccgagggtc tgttattgcc attatagatc acagtgacag    1680
   gggcactgcc aggcatagtc gctgtaaccc ccggagcagg tgatctcgac ggttcctccc    1740
   aggtttgctg acaccgaggc cggctgagtc agcgctgcct gcaccaggga acctggagag      1800
   ggagaggaga gaggggagaa aatggatctg agcaattgcc tgacacacac agccctcccc      1860
   agactgtggg atgaggccct gtgcccgcag tcacatgtgg aatatcaaga cacacacatc      1920
   tatgacaatc acaatctgat tatcaaccac tatggctggt accagcagaa ggcacctagc      1980
   agtgcccctg tcactctgat ctactatgat gatgagagac cctcgaacat cccttcacga      2040
   ttctccggtt ccaaatccgg ctccacacac acattaacca tcactggggt ccaagccgac      2100
   gacgaggctg tctattactg tgggaatgaa gacagcagcg gtactggtgc cccagtgaca      2160
   cagagcaatg gagatatgat acacagacct gacatctcag ggagcagctg gtcattatcc      2220
   tggtgacaca agtcccaggc atggcccagc actgtgctgc aggtggctat gccgggtgcc      2280
   ctgctctgcc ctcctgtaga gcctcaggac tgtgcccatc actcagggag gagatggtcc      2340
   cagcagccct gtccctgccc tgcactgcac ttagctcctg gacccca                    2387
<210> 21
   <211> 1957
   <212> DNA
   <213> Gallus gallus
<400> 21
   atattgcctc cctgtgttgc tgttgcaggg ttgcttctgc ctcatactgg tttctccctt      60
   ctggaggtgg ccaaaagccg ggccctgtgc aaccctggtg cataaatacc ttatggcccc     120
   taagtagggc aggtgtggga cacgctctgg cacctggggt gtgtgcaagt gctcaggaag     180
   acctgcaggc acaggtggca gtgggggtct ctggctgtgc tcgagcagca gctgcctggg     240
   gtaagggtag tactctgtgc atgaacaatg ctgcagggct cagctctgct cagaccacga     300
   ccctggcacc aacagagacc tgcctggctc tgtggtcatg taaaccttta caggagctca     360
   agacaaggct gtttattact gctctggcag gaaagaagca ctggccatgg tcatagagag     420
   ttccagcaac aggaaagtga gagcccaagc tgctgaggta ccagggctcc tcaggtgcct     480
   gctgcagcag cttggacaca gtcgaggaac agcaattgta cctgtgtggt ggatcaggct     540
   gtgctgcctg tgaacctatt ctagcacatc tgtcacctct gtgccactca cagggatacc     600
   acccctgaga cccctacccc atcagcctct gtgtgggata tggtgttggg cccaagggct     660
   ctgttgcaca gggagataga ggcctgggga ggagggaaag cattgaggtg gtgttgatac     720
   cagggatgtg agcccaagca agagatcagc agagcaagga ggaagaattg caggtgttgg     780
   ggctggggaa agccccagat ggctggagct ggtggggcca ctggagatct cctcctccca     840
   tcctgctcca tgctggggca gctgctgcag gctgaccagg gcctgcccgg gcacgttgtg     900
   aaggtcacca aggatggaga cttcagagct atctgggaca cgtgttttaa cgtttgatca     960
   gccttgcagc aaaacagtgt tttgctctat tgaacagtgt tcaacaaaaa cgatcagtgg    1020
   cctggaacac atctcctacg aagaaaggtt gcgagagctg ggtctggaga agagaaggct 1080
   ctggggcaac ctccctgtgg cctgtcagtg cttaaaggag gctcaagaga aagatggaga 1140
   gagactttat acacggagct gtagcaatat gacaaggaga aatggtttaa aatggaaaga 1200
   gggtagattt aggttagcta tcaagaagaa attcttcact cagaggtgat gaagcactgt 1260
   agcaggttgc ccagagaagt attggatgct tccttcctgg cagggcccaa ggccagactg 1320
   gatgggttct gggcagcctg ttctggtggg agatgtccct gcctgtggca tggcttggaa 1380
   atgggtagcc tttgaggtcc ctctcagccc aaatcattct atgttcttgt ggttccctgc 1440
   atttcagcct ttgcctgttg actctggtgc cactgttgga caattctact cagctgcgtt 1500
   tcatccctgc cccaaagcag taatcccatg gataagcaat gctctgaggc cttcaccacc 1560
   ctcagggctc ctgctgaccc tagcggcctc cctggcagca tgagcccttt gttgtccgca 1620
   ctgctgctgc cgagggtcct gccagccccc cagggccttt tctgcccagg cactgccacc 1680
   tgctaaaacc cagcctgccc atggcagggc atcgcatgtc tgagggatgg aacggggcaa 1740
   gggcagtgct gcaggcagtg gttcaagaaa cgtttagatg ttgtactgag ggatgtggtt 1800
   tagtgggaaa tactggtgat aggtggatgg ttggactgga ggatcttaga ggtcttttcc 1860
   aaccttggtg attatgtgat tctatgaagg gagccatagc ctgctcccat catgccccgc 1920
   cagtccggct ctcccgagca ctttggttcc gcagcat 1957
<210> 22
   <211> 9897
   <212> DNA
   <213> Artificial Sequence
<220>
   <223> Targeting vector
<400> 22
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagctca 240
   gaattaaccc tcactaaagg gactagtcct gcaggtttaa acgaattcgc ccttgtcgac 300
   tagatgggag tttaagagtg aattgtggtc catggcatat gcctgtcagc atagggatgc 360
   tgtaggaatt gcaccttggc ttgagcccct ctcctcctga tgatgcttgc ccacagctct 420
   gatgtcctgg gaaggctcac tgcaccttgt tgtcagtgaa actgcaataa actatcaaga 480
   tttgatataa ttctgcccct ctgttctact tgtgagacca cacctggagt actgcatcca 540
   gttctggtac cactgcatcc agttctggta ccaccaacac aaaaaggaca tggagctgct 600
   ggagcagggc cagaggaggg ccatgaagat gaacagaggg ctgaagcacc tcccctatgg 660
   ggacaggctg agagacatgg ggcttttgag actggagaag agaaggctcc atgggacctt 720
   tcagtgtcct tccaatactt gaaagggtcc ttgaggaaag ctggggaggg acttcctata 780
   aggacatata gcaacaggac aaaaggaaag ggttttaaac tggaagaaag tagatttagt 840
   ctagatagta acaagaaatt ctttacagta aggatcacga gacactgaaa caggttgccc 900
   aaagcagctg tggatgcccc ctccctggaa gtgttccagg ccaggctgga tggggctttg 960
   agcaacctgt tctagtgaga ggtgaccctg cctacagcag gggagttgaa cctagatggt 1020
   cttaaatgtc ccttccaacc caaatcattc tatggcaatt ctatagagaa cacctatgct 1080
   gagagtagct acaagtatgg ctggttcctg gcagtgcccc tgtctctgtg atctatagca 1140
   gcaccaacag accctcaaac atcccttcac gattctccgg ttccaaatcc ggctccacac 1200
   acacattaac catcactggg gtccaagccg atgacaaggc tgtctattac tgtgggagca 1260
   cagacagcag ctatgttggt atcacagtga catggagcaa ttaaaaatga tgcataacct 1320
   tttgcacatc tgtaatgcta taaggtgaga gaaagtaagc aataatagca gagcagcaca 1380
   atcctaggct acagcaaatg agaacaagcc cagatgcagc cacggaaata aagaaaaaaa 1440
   aactgcttac ctttagaaag cctcatatag acagggatct ccagcaagat acccacacct 1500
   ccactgccaa cccttaaatg agggctggga agggctggat cctggctcca cccctttcaa 1560
   tcactcaggt acattgcaca cacctgagct cccctggctg gccctgcctt cccaccaggt 1620
   gctcaatcac tgctttgggc tgtgactcag catctccact acagcatcac agggagcggt 1680
   tgtcggtctc tgctgttcct atgtgggtgc cccgggacac ctgaaggcat gagtggcagt 1740
   gagggctccc agctctatcc atggtgaaca gacattatgt ttatgcttta cagcagcatg 1800
   aatctggcag tggtgttatt gctgtgatgc tgctgtctat gctcccacag taatagatag 1860
   cctcgtcctc ggcttggacc ccagtgatgg ttaatgtgtt tgtggagccg gatccagaac 1920
   cggagaatcg tgaagggatg tccgagggtc tgttattgcc attatagatc acagtgacag 1980
   gggcactgcc aggcatagtc gctgtaaccc ccggagcagg tgatctcgac ggttcctccc 2040
   aggtttgctg acaccgaggc cggctgagtc agcgctgcct gcaccaggga acctggagag 2100
   ggagaggaga gaggggagaa aatggatctg agcaattgcc tgacacacac agccctcccc 2160
   agactgtggg atgaggccct gtgcccgcag tcacatgtgg aatatcaaga cacacacatc 2220
   tatgacaatc acaatctgat tatcaaccac tatggctggt accagcagaa ggcacctagc 2280
   agtgcccctg tcactctgat ctactatgat gatgagagac cctcgaacat cccttcacga 2340
   ttctccggtt ccaaatccgg ctccacacac acattaacca tcactggggt ccaagccgac 2400
   gacgaggctg tctattactg tgggaatgaa gacagcagcg gtactggtgc cccagtgaca 2460
   cagagcaatg gagatatgat acacagacct gacatctcag ggagcagctg gtcattatcc 2520
   tggtgacaca agtcccaggc atggcccagc actgtgctgc aggtggctat gccgggtgcc 2580
   ctgctctgcc ctcctgtaga gcctcaggac tgtgcccatc actcagggag gagatggtcc 2640
   cagcagccct gtccctgccc tgcactgcac ttagctcctg gaccccatca atttctgaga 2700
   actgtcattc tcggaatacc tcgatcgata acttcgtata atgtatacta tacgaagtta 2760
   ttttatttgt gaaatttgtg atgctattgc tttatttgta accattataa gctgcaataa 2820
   acaagttggg gtgggcgaag aactccagca tgagatcccc gcgctggagg atcatccagc 2880
   cggcgtcccg gaaaacgatt ccgaagccca acctttcata gaaggcggcg gtggaatcga 2940
   aatctcgtga tggcaggttg ggcgtcgctt ggtcggtcat ttcgaacccc agagtcccgc 3000
   tcagaagaac tcgtcaagaa ggcgatagaa ggcgatgcgc tgcgaatcgg gagcggcgat 3060
   accgtaaagc acgaggaagc ggtcagccca ttcgccgcca agctcttcag caatatcacg 3120
   ggtagccaac gctatgtcct gatagcggtc cgccacaccc agccggccac agtcgatgaa 3180
   tccagaaaag cggccatttt ccaccatgat attcggcaag caggcatcgc catgggtcac 3240
   gacgagatcc tcgccgtcgg gcatgcgcgc cttgagcctg gcgaacagtt cggctggcgc 3300
   gagcccctga tgctcttcgt ccagatcatc ctgatcgaca agaccggctt ccatccgagt 3360
   acgtgctcgc tcgatgcgat gtttcgcttg gtggtcgaat gggcaggtag ccggatcaag 3420
   cgtatgcagc cgccgcattg catcagccat gatggatact ttctcggcag gagcaaggtg 3480
   agatgacagg agatcctgcc ccggcacttc gcccaatagc agccagtccc ttcccgcttc 3540
   agtgacaacg tcgagcacag ctgcgcaagg aacgcccgtc gtggccagcc acgatagccg 3600
   cgctgcctcg tcctgcagtt cattcagggc accggacagg tcggtcttga caaaaagaac 3660
   cgggcgcccc tgcgctgaca gccggaacac ggcggcatca gagcagccga ttgtctgttg 3720
   tgcccagtca tagccgaata gcctctccac ccaagcggcc ggagaacctg cgtgcaatcc 3780
   atcttgttca atcatgcgaa acgatcctca tcctgtctct tgatcagatc cgaaaatgga 3840
   tatacaagct cccgggagct ttttgcaaaa gcctaggcct ccaaaaaagc ctcctcacta 3900
   cttctggaat agctcagagg cagaggcggc ctcggcctct gcataaataa aaaaaattag 3960
   tcagccatgg ggcggagaat gggcggaact gggcggagtt aggggcggga tgggcggagt 4020
   taggggcggg actatggttg ctgactaatt gagatgcatg ctttgcatac ttctgcctgc 4080
   tggggagcct ggggactttc cacacctggt tgctgactaa ttgagatgca tgctttgcat 4140
   acttctgcct gctggggagc ctggggactt tccacaccct aaataacttc gtataatgta 4200
   tactatacga agttatctcg tgtccgataa ctgtaattat cggaccgtca atcgattcgc 4260
   gaggcgcgcc atattgcctc cctgtgttgc tgttgcaggg ttgcttctgc ctcatactgg 4320
   tttctccctt ctggaggtgg ccaaaagccg ggccctgtgc aaccctggtg cataaatacc 4380
   ttatggcccc taagtagggc aggtgtggga cacgctctgg cacctggggt gtgtgcaagt 4440
   gctcaggaag acctgcaggc acaggtggca gtgggggtct ctggctgtgc tcgagcagca 4500
   gctgcctggg gtaagggtag tactctgtgc atgaacaatg ctgcagggct cagctctgct 4560
   cagaccacga ccctggcacc aacagagacc tgcctggctc tgtggtcatg taaaccttta 4620
   caggagctca agacaaggct gtttattact gctctggcag gaaagaagca ctggccatgg 4680
   tcatagagag ttccagcaac aggaaagtga gagcccaagc tgctgaggta ccagggctcc 4740
   tcaggtgcct gctgcagcag cttggacaca gtcgaggaac agcaattgta cctgtgtggt 4800
   ggatcaggct gtgctgcctg tgaacctatt ctagcacatc tgtcacctct gtgccactca 4860
   cagggatacc acccctgaga cccctacccc atcagcctct gtgtgggata tggtgttggg 4920
   cccaagggct ctgttgcaca gggagataga ggcctgggga ggagggaaag cattgaggtg 4980
   gtgttgatac cagggatgtg agcccaagca agagatcagc agagcaagga ggaagaattg 5040
   caggtgttgg ggctggggaa agccccagat ggctggagct ggtggggcca ctggagatct 5100
   cctcctccca tcctgctcca tgctggggca gctgctgcag gctgaccagg gcctgcccgg 5160
   gcacgttgtg aaggtcacca aggatggaga cttcagagct atctgggaca cgtgttttaa 5220
   cgtttgatca gccttgcagc aaaacagtgt tttgctctat tgaacagtgt tcaacaaaaa 5280
   cgatcagtgg cctggaacac atctcctacg aagaaaggtt gcgagagctg ggtctggaga 5340
   agagaaggct ctggggcaac ctccctgtgg cctgtcagtg cttaaaggag gctcaagaga 5400
   aagatggaga gagactttat acacggagct gtagcaatat gacaaggaga aatggtttaa 5460
   aatggaaaga gggtagattt aggttagcta tcaagaagaa attcttcact cagaggtgat 5520
   gaagcactgt agcaggttgc ccagagaagt attggatgct tccttcctgg cagggcccaa 5580
   ggccagactg gatgggttct gggcagcctg ttctggtggg agatgtccct gcctgtggca 5640
   tggcttggaa atgggtagcc tttgaggtcc ctctcagccc aaatcattct atgttcttgt 5700
   ggttccctgc atttcagcct ttgcctgttg actctggtgc cactgttgga caattctact 5760
   cagctgcgtt tcatccctgc cccaaagcag taatcccatg gataagcaat gctctgaggc 5820
   cttcaccacc ctcagggctc ctgctgaccc tagcggcctc cctggcagca tgagcccttt 5880
   gttgtccgca ctgctgctgc cgagggtcct gccagccccc cagggccttt tctgcccagg 5940
   cactgccacc tgctaaaacc cagcctgccc atggcagggc atcgcatgtc tgagggatgg 6000
   aacggggcaa gggcagtgct gcaggcagtg gttcaagaaa cgtttagatg ttgtactgag 6060
   ggatgtggtt tagtgggaaa tactggtgat aggtggatgg ttggactgga ggatcttaga 6120
   ggtcttttcc aaccttggtg attatgtgat tctatgaagg gagccatagc ctgctcccat 6180
   catgccccgc cagtccggct ctcccgagca ctttggttcc gcagcatgcg gccgcaaggg 6240
   cgaattcgcg gccgctaaat tcaattcgcc ctatagtgag tcgtattaca attcactggc 6300
   cgtcgtttta caacgtcgtg actgggaaaa ccctggcgtt acccaactta atcgccttgc 6360
   agcacatccc cctttcgcca gctggcgtaa tagcgaagag gcccgcaccg atcgcccttc 6420
   ccaacagttg cgcagcctat acgtacggca gtttaaggtt tacacctata aaagagagag 6480
   ccgttatcgt ctgtttgtgg atgtacagag tgatattatt gacacgccgg ggcgacggat 6540
   ggtgatcccc ctggccagtg cacgtctgct gtcagataaa gtctcccgtg aactttaccc 6600
   ggtggtgcat atcggggatg aaagctggcg catgatgacc accgatatgg ccagtgtgcc 6660
   ggtctccgtt atcggggaag aagtggctga tctcagccac cgcgaaaatg acatcaaaaa 6720
   cgccattaac ctgatgttct ggggaatata aatgtcaggc atgagattat caaaaaggat 6780
   cttcacctag atccttttca cgtagaaagc cagtccgcag aaacggtgct gaccccggat 6840
   gaatgtcagc tactgggcta tctggacaag ggaaaacgca agcgcaaaga gaaagcaggt 6900
   agcttgcagt gggcttacat ggcgatagct agactgggcg gttttatgga cagcaagcga 6960
   accggaattg ccagctgggg cgccctctgg taaggttggg aagccctgca aagtaaactg 7020
   gatggctttc ttgccgccaa ggatctgatg gcgcagggga tcaagctctg atcaagagac 7080
   aggatgagga tcgtttcgca tgattgaaca agatggattg cacgcaggtt ctccggccgc 7140
   ttgggtggag aggctattcg gctatgactg ggcacaacag acaatcggct gctctgatgc 7200
   cgccgtgttc cggctgtcag cgcaggggcg cccggttctt tttgtcaaga ccgacctgtc 7260
   cggtgccctg aatgaactgc aagacgaggc agcgcggcta tcgtggctgg ccacgacggg 7320
   cgttccttgc gcagctgtgc tcgacgttgt cactgaagcg ggaagggact ggctgctatt 7380
   gggcgaagtg ccggggcagg atctcctgtc atctcacctt gctcctgccg agaaagtatc 7440
   catcatggct gatgcaatgc ggcggctgca tacgcttgat ccggctacct gcccattcga 7500
   ccaccaagcg aaacatcgca tcgagcgagc acgtactcgg atggaagccg gtcttgtcga 7560
   tcaggatgat ctggacgaag agcatcaggg gctcgcgcca gccgaactgt tcgccaggct 7620
   caaggcgagc atgcccgacg gcgaggatct cgtcgtgacc catggcgatg cctgcttgcc 7680
   gaatatcatg gtggaaaatg gccgcttttc tggattcatc gactgtggcc ggctgggtgt 7740
   ggcggaccgc tatcaggaca tagcgttggc tacccgtgat attgctgaag agcttggcgg 7800
   cgaatgggct gaccgcttcc tcgtgcttta cggtatcgcc gctcccgatt cgcagcgcat 7860
   cgccttctat cgccttcttg acgagttctt ctgaattatt aacgcttaca atttcctgat 7920
   gcggtatttt ctccttacgc atctgtgcgg tatttcacac cgcatcaggt ggcacttttc 7980
   ggggaaatgt gcgcggaacc cctatttgtt tatttttcta aatacattca aatatgtatc 8040
   cgctcatgag attatcaaaa aggatcttca cctagatcct tttaaattaa aaatgaagtt 8100
   ttaaatcaat ctaaagtata tatgagtaaa cttggtctga cagttaccaa tgcttaatca 8160
   gtgaggcacc tatctcagcg atctgtctat ttcgttcatc catagttgcc tgactccccg 8220
   tcgtgtagat aactacgata cgggagggct taccatctgg ccccagtgct gcaatgatac      8280
   cgcgagaccc acgctcaccg gctccagatt tatcagcaat aaaccagcca gccggaaggg      8340
   ccgagcgcag aagtggtcct gcaactttat ccgcctccat ccagtctatt aattgttgcc      8400
   gggaagctag agtaagtagt tcgccagtta atagtttgcg caacgttgtt gccattgcta      8460
   caggcatcgt ggtgtcacgc tcgtcgtttg gtatggcttc attcagctcc ggttcccaac      8520
   gatcaaggcg agttacatga tcccccatgt tgtgcaaaaa agcggttagc tccttcggtc      8580
   ctccgatcgt tgtcagaagt aagttggccg cagtgttatc actcatggtt atggcagcac      8640
   tgcataattc tcttactgtc atgccatccg taagatgctt ttctgtgact ggtgagtact      8700
   caaccaagtc attctgagaa tagtgtatgc ggcgaccgag ttgctcttgc ccggcgtcaa      8760
   tacgggataa taccgcgcca catagcagaa ctttaaaagt gctcatcatt ggaaaacgtt      8820
   cttcggggcg aaaactctca aggatcttac cgctgttgag atccagttcg atgtaaccca      8880
   ctcgtgcacc caactgatct tcagcatctt ttactttcac cagcgtttct gggtgagcaa      8940
   aaacaggaag gcaaaatgcc gcaaaaaagg gaataagggc gacacggaaa tgttgaatac      9000
   tcatactctt cctttttcaa tattattgaa gcatttatca gggttattgt ctcatgacca      9060
   aaatccctta acgtgagttt tcgttccact gagcgtcaga ccccgtagaa aagatcaaag      9120
   gatcttcttg agatcctttt tttctgcgcg taatctgctg cttgcaaaca aaaaaaccac      9180
   cgctaccagc ggtggtttgt ttgccggatc aagagctacc aactcttttt ccgaaggtaa      9240
   ctggcttcag cagagcgcag ataccaaata ctgttcttct agtgtagccg tagttaggcc      9300
   accacttcaa gaactctgta gcaccgccta catacctcgc tctgctaatc ctgttaccag      9360
   tggctgctgc cagtggcgat aagtcgtgtc ttaccgggtt ggactcaaga cgatagttac      9420
   cggataaggc gcagcggtcg ggctgaacgg ggggttcgtg cacacagccc agcttggagc      9480
   gaacgaccta caccgaactg agatacctac agcgtgagct atgagaaagc gccacgcttc      9540
   ccgaagggag aaaggcggac aggtatccgg taagcggcag ggtcggaaca ggagagcgca      9600
   cgagggagct tccaggggga aacgcctggt atctttatag tcctgtcggg tttcgccacc      9660
   tctgacttga gcgtcgattt ttgtgatgct cgtcaggggg gcggagccta tggaaaaacg      9720
   ccagcaacgc ggccttttta cggttcctgg ccttttgctg gccttttgct cacatgttct      9780
   ttcctgcgtt atcccctgat tctgtggata accgtattac cgcctttgag tgagctgata      9840
   ccgctcgccg cagccgaacg accgagcgca gcgagtcagt gagcgaggaa gcggaag         9897
<210> 23
   <211> 10664
   <212> DNA
   <213> Artificial Sequence
<220>
   <223> Targeting vector
<400> 23
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gccaagctca 240
   gaattaaccc tcactaaagg gactagtcct gcaggtttaa acgaattcgc ccttgtcgac 300
   tagatgggag tttaagagtg aattgtggtc catggcatat gcctgtcagc atagggatgc 360
   tgtaggaatt gcaccttggc ttgagcccct ctcctcctga tgatgcttgc ccacagctct 420
   gatgtcctgg gaaggctcac tgcaccttgt tgtcagtgaa actgcaataa actatcaaga 480
   tttgatataa ttctgcccct ctgttctact tgtgagacca cacctggagt actgcatcca 540
   gttctggtac cactgcatcc agttctggta ccaccaacac aaaaaggaca tggagctgct 600
   ggagcagggc cagaggaggg ccatgaagat gaacagaggg ctgaagcacc tcccctatgg 660
   ggacaggctg agagacatgg ggcttttgag actggagaag agaaggctcc atgggacctt 720
   tcagtgtcct tccaatactt gaaagggtcc ttgaggaaag ctggggaggg acttcctata 780
   aggacatata gcaacaggac aaaaggaaag ggttttaaac tggaagaaag tagatttagt 840
   ctagatagta acaagaaatt ctttacagta aggatcacga gacactgaaa caggttgccc 900
   aaagcagctg tggatgcccc ctccctggaa gtgttccagg ccaggctgga tggggctttg 960
   agcaacctgt tctagtgaga ggtgaccctg cctacagcag gggagttgaa cctagatggt 1020
   cttaaatgtc ccttccaacc caaatcattc tatggcaatt ctatagagaa cacctatgct 1080
   gagagtagct acaagtatgg ctggttcctg gcagtgcccc tgtctctgtg atctatagca 1140
   gcaccaacag accctcaaac atcccttcac gattctccgg ttccaaatcc ggctccacac 1200
   acacattaac catcactggg gtccaagccg atgacaaggc tgtctattac tgtgggagca 1260
   cagacagcag ctatgttggt atcacagtga catggagcaa ttaaaaatga tgcataacct 1320
   tttgcacatc tgtaatgcta taaggtgaga gaaagtaagc aataatagca gagcagcaca 1380
   atcctaggct acagcaaatg agaacaagcc cagatgcagc cacggaaata aagaaaaaaa 1440
   aactgcttac ctttagaaag cctcatatag acagggatct ccagcaagat acccacacct 1500
   ccactgccaa cccttaaatg agggctggga agggctggat cctggctcca cccctttcaa 1560
   tcactcaggt acattgcaca cacctgagct cccctggctg gccctgcctt cccaccaggt 1620
   gctcaatcac tgctttgggc tgtgactcag catctccact acagcatcac agggagcggt 1680
   tgtcggtctc tgctgttcct atgtgggtgc cccgggacac ctgaaggcat gagtggcagt 1740
   gagggctccc agctctatcc atggtgaaca gacattatgt ttatgcttta cagcagcatg 1800
   aatctggcag tggtgttatt gctgtgatgc tgctgtctat gctcccacag taatagatag 1860
   cctcgtcctc ggcttggacc ccagtgatgg ttaatgtgtt tgtggagccg gatccagaac 1920
   cggagaatcg tgaagggatg tccgagggtc tgttattgcc attatagatc acagtgacag 1980
   gggcactgcc aggcatagtc gctgtaaccc ccggagcagg tgatctcgac ggttcctccc 2040
   aggtttgctg acaccgaggc cggctgagtc agcgctgcct gcaccaggga acctggagag 2100
   ggagaggaga gaggggagaa aatggatctg agcaattgcc tgacacacac agccctcccc 2160
   agactgtggg atgaggccct gtgcccgcag tcacatgtgg aatatcaaga cacacacatc 2220
   tatgacaatc acaatctgat tatcaaccac tatggctggt accagcagaa ggcacctagc 2280
   agtgcccctg tcactctgat ctactatgat gatgagagac cctcgaacat cccttcacga 2340
   ttctccggtt ccaaatccgg ctccacacac acattaacca tcactggggt ccaagccgac 2400
   gacgaggctg tctattactg tgggaatgaa gacagcagcg gtactggtgc cccagtgaca 2460
   cagagcaatg gagatatgat acacagacct gacatctcag ggagcagctg gtcattatcc 2520
   tggtgacaca agtcccaggc atggcccagc actgtgctgc aggtggctat gccgggtgcc 2580
   ctgctctgcc ctcctgtaga gcctcaggac tgtgcccatc actcagggag gagatggtcc 2640
   cagcagccct gtccctgccc tgcactgcac ttagctcctg gaccccatca atttctgaga 2700
   actgtcattc tcggaatacc tcgatcgata acttcgtata atgtatacta tacgaagtta 2760
   ttttatttgt gaaatttgtg atgctattgc tttatttgta accattataa gctgcaataa 2820
   acaagttggg gtgggcgaag aactccagca tgagatcccc gcgctggagg atcatccagc 2880
   cggcgtcccg gaaaacgatt ccgaagccca acctttcata gaaggcggcg gtggaatcga 2940
   aatctcgtga tggcaggttg ggcgtcgctt ggtcggtcat ttcgaacccc agagtcccgc 3000
   tcagaagaac tcgtcaagaa ggcgatagaa ggcgatgcgc tgcgaatcgg gagcggcgat 3060
   accgtaaagc acgaggaagc ggtcagccca ttcgccgcca agctcttcag caatatcacg 3120
   ggtagccaac gctatgtcct gatagcggtc cgccacaccc agccggccac agtcgatgaa 3180
   tccagaaaag cggccatttt ccaccatgat attcggcaag caggcatcgc catgggtcac 3240
   gacgagatcc tcgccgtcgg gcatgcgcgc cttgagcctg gcgaacagtt cggctggcgc 3300
   gagcccctga tgctcttcgt ccagatcatc ctgatcgaca agaccggctt ccatccgagt 3360
   acgtgctcgc tcgatgcgat gtttcgcttg gtggtcgaat gggcaggtag ccggatcaag 3420
   cgtatgcagc cgccgcattg catcagccat gatggatact ttctcggcag gagcaaggtg 3480
   agatgacagg agatcctgcc ccggcacttc gcccaatagc agccagtccc ttcccgcttc 3540
   agtgacaacg tcgagcacag ctgcgcaagg aacgcccgtc gtggccagcc acgatagccg 3600
   cgctgcctcg tcctgcagtt cattcagggc accggacagg tcggtcttga caaaaagaac 3660
   cgggcgcccc tgcgctgaca gccggaacac ggcggcatca gagcagccga ttgtctgttg 3720
   tgcccagtca tagccgaata gcctctccac ccaagcggcc ggagaacctg cgtgcaatcc 3780
   atcttgttca atcatgcgaa acgatcctca tcctgtctct tgatcagatc cgaaaatgga 3840
   tatacaagct cccgggagct ttttgcaaaa gcctaggcct ccaaaaaagc ctcctcacta 3900
   cttctggaat agctcagagg cagaggcggc ctcggcctct gcataaataa aaaaaattag 3960
   tcagccatgg ggcggagaat gggcggaact gggcggagtt aggggcggga tgggcggagt 4020
   taggggcggg actatggttg ctgactaatt gagatgcatg ctttgcatac ttctgcctgc 4080
   tggggagcct ggggactttc cacacctggt tgctgactaa ttgagatgca tgctttgcat 4140
   acttctgcct gctggggagc ctggggactt tccacaccct aaataacttc gtataatgta 4200
   tactatacga agttatctcg tgtccgataa ctgtaattat cggaccgtca atcgattcgc 4260
   gacagtctgc cctgactcag cctgcctccg tgtctgggtc tcctggacag tcgatcacca 4320
   tctcctgcac tggaaccagc tcaagtgacg ttggtggtta taactatgtc tcctggtacc 4380
   aacaaaaccc aggcaaagcc cccaaactca tgatttatga tgtcagttca aatcggccct 4440
   cagggatttc taatcgcttc tctggctcca agtctggcaa cacggcctcc ctgaccatct 4500
   ctgggctcca ggctgacgac gaggctgatt attactgcac ctcatataca aacgacggta 4560
   gcaattctca ggtattcggc ggagggacca agctgaccgt cctaggtgcc tgacacacac 4620
   agccctcccc agactgtggg atgaggccct gtgcccgcag tcacatgtgg aatatcaaga 4680
   cacacacatc tatgacaatc accagtctgc cctgactcag cctgcctccg tgtctgggtc 4740
   tcctggacag tcgatcacca tctcctgcac tggaaccagc agtgacgttg gttataacta 4800
   tgtctcctgg taccaacaaa acccaggcaa agcccccaaa ctcatgattt atgatggtaa 4860
   tcggccctca gggatttcta atcgcttctc tggctccaag tctggcaaca cggcctccct 4920
   gaccatctct gggctccagg ctgacgacga ggctgattat tactgcacct catatacaaa 4980
   cgacagctct caggtattcg gcggagggac caagctgacc gtcctaggtg gcgcgccata 5040
   ttgcctccct gtgttgctgt tgcagggttg cttctgcctc atactggttt ctcccttctg 5100
   gaggtggcca aaagccgggc cctgtgcaac cctggtgcat aaatacctta tggcccctaa 5160
   gtagggcagg tgtgggacac gctctggcac ctggggtgtg tgcaagtgct caggaagacc 5220
   tgcaggcaca ggtggcagtg gggtctctgg gctgtgctcg agcagcagct gcctggggta 5280
   agggtagtac tctgtgcatg aacaatgctg cagggctcag ctctgctcag accacgaccc 5340
   tggcaccaac agagacctgc ctggctctgt ggtcatgtaa acctttacag gagctcaaga 5400
   caaggctgtt tattactgct ctggcaggaa agaagcactg gccatggtca tagagagttc 5460
   cagcaacagg aaagtgagag cccaagctgc tgaggtacca gggctcctca ggtgcctgct 5520
   gcagcagctt ggacacagtc gaggaacagc aattgtacct gtgtggtgga tcaggctgtg 5580
   ctgcctgtga acctattcta gcacatctgt cacctctgtg ccactcacag ggataccacc 5640
   cctgagaccc ctaccccatc agcctctgtg tgggatatgg tgttgggccc aagggctctg 5700
   ttgcacaggg agatagaggc ctggggagga gggaaagcat tgaggtggtg ttgataccag 5760
   ggatgtgagc ccaagcaaga gatcagcaga gcaaggagga agaattgcag gtgttggggc 5820
   tggggaaagc cccagatggc tggagctggt ggggccactg gagatctcct cctcccatcc 5880
   tgctccatgc tggggcagct gctgcaggct gaccagggcc tgcccgggca cgttgtgaag 5940
   gtcaccaagg atggagactt cagagctatc tgggacacgt gttttaacgt ttgatcagcc 6000
   ttgcagcaaa acagtgtttt gctctattga acagtgttca acaaaaacga tcagtggcct 6060
   ggaacacatc tcctacgaag aaaggttgcg agagctgggt ctggagaaga gaaggctctg 6120
   gggcaacctc cctgtggcct gtcagtgctt aaaggaggct caagagaaag atggagagag 6180
   actttataca cggagctgta gcaatatgac aaggagaaat ggtttaaaat ggaaagaggg 6240
   tagatttagg ttagctatca agaagaaatt cttcactcag aggtgatgaa gcactgtagc 6300
   aggttgccca gagaagtatt ggatgcttcc ttcctggcag ggcccaaggc cagactggat 6360
   gggttctggg cagcctgttc tggtgggaga tgtccctgcc tgtggcatgg cttggaaatg 6420
   ggtagccttt gaggtccctc tcagcccaaa tcattctatg ttcttgtggt tccctgcatt 6480
   tcagcctttg cctgttgact ctggtgccac tgttggacaa ttctactcag ctgcgtttca 6540
   tccctgcccc aaagcagtaa tcccatggat aagcaatgct ctgaggcctt caccaccctc 6600
   agggctcctg ctgaccctag cggcctccct ggcagcatga gccctttgtt gtccgcactg 6660
   ctgctgccga gggtcctgcc agccccccag ggccttttct gcccaggcac tgccacctgc 6720
   taaaacccag cctgcccatg gcagggcatc gcatgtctga gggatggaac ggggcaaggg 6780
   cagtgctgca ggcagtggtt caagaaacgt ttagatgttg tactgaggga tgtggtttag 6840
   tgggaaatac tggtgatagg tggatggttg gactggagga tcttagaggt cttttccaac 6900
   cttggtgatt atgtgattct atgaagggag ccatagcctg ctcccatcat gccccgccag 6960
   tccggctctc ccgagcactt tggttccgca gcatgcggcc gcaagggcga attcgcggcc 7020
   gctaaattca attcgcccta tagtgagtcg tattacaatt cactggccgt cgttttacaa 7080
   cgtcgtgact gggaaaaccc tggcgttacc caacttaatc gccttgcagc acatccccct 7140
   ttcgccagct ggcgtaatag cgaagaggcc cgcaccgatc gcccttccca acagttgcgc 7200
   agcctatacg tacggcagtt taaggtttac acctataaaa gagagagccg ttatcgtctg 7260
   tttgtggatg tacagagtga tattattgac acgccggggc gacggatggt gatccccctg 7320
   gccagtgcac gtctgctgtc agataaagtc tcccgtgaac tttacccggt ggtgcatatc 7380
   ggggatgaaa gctggcgcat gatgaccacc gatatggcca gtgtgccggt ctccgttatc 7440
   ggggaagaag tggctgatct cagccaccgc gaaaatgaca tcaaaaacgc cattaacctg 7500
   atgttctggg gaatataaat gtcaggcatg agattatcaa aaaggatctt cacctagatc 7560
   cttttcacgt agaaagccag tccgcagaaa cggtgctgac cccggatgaa tgtcagctac 7620
   tgggctatct ggacaaggga aaacgcaagc gcaaagagaa agcaggtagc ttgcagtggg 7680
   cttacatggc gatagctaga ctgggcggtt ttatggacag caagcgaacc ggaattgcca 7740
   gctggggcgc cctctggtaa ggttgggaag ccctgcaaag taaactggat ggctttcttg 7800
   ccgccaagga tctgatggcg caggggatca agctctgatc aagagacagg atgaggatcg 7860
   tttcgcatga ttgaacaaga tggattgcac gcaggttctc cggccgcttg ggtggagagg 7920
   ctattcggct atgactgggc acaacagaca atcggctgct ctgatgccgc cgtgttccgg 7980
   ctgtcagcgc aggggcgccc ggttcttttt gtcaagaccg acctgtccgg tgccctgaat 8040
   gaactgcaag acgaggcagc gcggctatcg tggctggcca cgacgggcgt tccttgcgca 8100
   gctgtgctcg acgttgtcac tgaagcggga agggactggc tgctattggg cgaagtgccg 8160
   gggcaggatc tcctgtcatc tcaccttgct cctgccgaga aagtatccat catggctgat 8220
   gcaatgcggc ggctgcatac gcttgatccg gctacctgcc cattcgacca ccaagcgaaa 8280
   catcgcatcg agcgagcacg tactcggatg gaagccggtc ttgtcgatca ggatgatctg 8340
   gacgaagagc atcaggggct cgcgccagcc gaactgttcg ccaggctcaa ggcgagcatg 8400
   cccgacggcg aggatctcgt cgtgacccat ggcgatgcct gcttgccgaa tatcatggtg 8460
   gaaaatggcc gcttttctgg attcatcgac tgtggccggc tgggtgtggc ggaccgctat 8520
   caggacatag cgttggctac ccgtgatatt gctgaagagc ttggcggcga atgggctgac 8580
   cgcttcctcg tgctttacgg tatcgccgct cccgattcgc agcgcatcgc cttctatcgc 8640
   cttcttgacg agttcttctg aattattaac gcttacaatt tcctgatgcg gtattttctc 8700
   cttacgcatc tgtgcggtat ttcacaccgc atcaggtggc acttttcggg gaaatgtgcg 8760
   cggaacccct atttgtttat ttttctaaat acattcaaat atgtatccgc tcatgagatt 8820
   atcaaaaagg atcttcacct agatcctttt aaattaaaaa tgaagtttta aatcaatcta 8880
   aagtatatat gagtaaactt ggtctgacag ttaccaatgc ttaatcagtg aggcacctat 8940
   ctcagcgatc tgtctatttc gttcatccat agttgcctga ctccccgtcg tgtagataac 9000
   tacgatacgg gagggcttac catctggccc cagtgctgca atgataccgc gagacccacg 9060
   ctcaccggct ccagatttat cagcaataaa ccagccagcc ggaagggccg agcgcagaag 9120
   tggtcctgca actttatccg cctccatcca gtctattaat tgttgccggg aagctagagt 9180
   aagtagttcg ccagttaata gtttgcgcaa cgttgttgcc attgctacag gcatcgtggt 9240
   gtcacgctcg tcgtttggta tggcttcatt cagctccggt tcccaacgat caaggcgagt 9300
   tacatgatcc cccatgttgt gcaaaaaagc ggttagctcc ttcggtcctc cgatcgttgt 9360
   cagaagtaag ttggccgcag tgttatcact catggttatg gcagcactgc ataattctct 9420
   tactgtcatg ccatccgtaa gatgcttttc tgtgactggt gagtactcaa ccaagtcatt 9480
   ctgagaatag tgtatgcggc gaccgagttg ctcttgcccg gcgtcaatac gggataatac 9540
   cgcgccacat agcagaactt taaaagtgct catcattgga aaacgttctt cggggcgaaa 9600
   actctcaagg atcttaccgc tgttgagatc cagttcgatg taacccactc gtgcacccaa 9660
   ctgatcttca gcatctttta ctttcaccag cgtttctggg tgagcaaaaa caggaaggca 9720
   aaatgccgca aaaaagggaa taagggcgac acggaaatgt tgaatactca tactcttcct 9780
   ttttcaatat tattgaagca tttatcaggg ttattgtctc atgaccaaaa tcccttaacg 9840
   tgagttttcg ttccactgag cgtcagaccc cgtagaaaag atcaaaggat cttcttgaga 9900
   tccttttttt ctgcgcgtaa tctgctgctt gcaaacaaaa aaaccaccgc taccagcggt 9960
   ggtttgtttg ccggatcaag agctaccaac tctttttccg aaggtaactg gcttcagcag 10020
   agcgcagata ccaaatactg ttcttctagt gtagccgtag ttaggccacc acttcaagaa 10080
   ctctgtagca ccgcctacat acctcgctct gctaatcctg ttaccagtgg ctgctgccag 10140
   tggcgataag tcgtgtctta ccgggttgga ctcaagacga tagttaccgg ataaggcgca 10200
   gcggtcgggc tgaacggggg gttcgtgcac acagcccagc ttggagcgaa cgacctacac 10260
   cgaactgaga tacctacagc gtgagctatg agaaagcgcc acgcttcccg aagggagaaa 10320
   ggcggacagg tatccggtaa gcggcagggt cggaacagga gagcgcacga gggagcttcc 10380
   agggggaaac gcctggtatc tttatagtcc tgtcgggttt cgccacctct gacttgagcg 10440
   tcgatttttg tgatgctcgt caggggggcg gagcctatgg aaaaacgcca gcaacgcggc 10500
   ctttttacgg ttcctggcct tttgctggcc ttttgctcac atgttctttc ctgcgttatc 10560
   ccctgattct gtggataacc gtattaccgc ctttgagtga gctgataccg ctcgccgcag 10620
   ccgaacgacc gagcgcagcg agtcagtgag cgaggaagcg gaag 10664
<210> 24
   <211> 24
   <212> DNA
   <213> Artificial Sequence
<220>
   <223> Synthetic Primer
<400> 24
   cgctttgtac gaacgttgtc acgt 24
<210> 25
   <211> 25
   <212> DNA
   <213> Artificial Sequence
<220>
   <223> Synthetic Primer
<400> 25
   tacctgaagg tctctttgtg ttttg 25
<210> 26
   <211> 2794
   <212> DNA
   <213> Gallus gallus
<400> 26
   gggtatcacg tggggcaaat cgagagctac cattggctat ggggaggaga aaatgaggcg      60
   tgctacttcc tcgttcctgc agtgcgtgtc gaggtgccgt gttggtcgga tctcagtggc     120
   agagcaggat ggtgagcggt ggccttgggg ccgctccctc actctgctgg gctggtgcgg     180
   tggctccggg accggacttt cagtggtgtt tggtgtggtg ctcactgtcc tttggtgtca     240
   ctgtgtggtg aattccctca gctgggcggt gagagcccat gctgtgtcct ggtaggagta     300
   gtctgggctg cagtgcgggc tctttgtgcg tgttgggggc ggttccctgg aagctaattc     360
   tggaagaaat cccggctgcc tctgtaacag ttgttggcga tgtcaaacac cctggagctg     420
   ctgactggct ctgtaccatt gagtgatgca gtttaaacct ctctctttcc tcctgctgtg     480
   tgtgaagctg gtgttagtgc taggagacct gcacatccct caccgatgca gtggtttacc     540
   agcaaagttc aagagcctcc tggttccagg gaagattcag catattttgt gtacaggaaa     600
   cctctgcacc aaggagagct atgactacct caggactttg gctggagatg ttcacgttgt     660
   taagggggac tctgaggtaa tgttcttgct gctgtgaatt cctcagtcct ccatcttaca     720
   agccgtagtt gtcccactga aagctgggaa acccttcggt gtttgcaatt cctgagcgct     780
   aatgtgtggg ggaaggatgt aggaacaaat aaggctcaag agacaacaac ttcctaggct     840
   cctcttattg cccttgtggt aagggaggac ttggtgtgta gtggtaatac atgcactcaa     900
   cttattaagt tctcatagct gctgtgaagc tgctgcagtc cctcagatcc ctgcaggaag     960
   aggggactga ctgactgttg aatagccttg aagctcctgc tctgcatgtg gttccggtca    1020
   ggctcaagat cccagttctt agctcttcag ttgctcttct gggggtatag tactagcatc    1080
   tgggtcactg cagatggaca gagactgtgc ttgggtagca aaaggcagac ttacgttgtg    1140
   aaaccttcat tgtgttggtg aggttctttg atttctacct tgtgtatctc tcttcctcag    1200
   agtctgaatt atcctgaaca gaaggttgta actgttgggc agttcagaat tgggttgatt    1260
   catggccatc aagtcattcc ctggggtgat gtggcaagct tggcattgct gcagaggcag    1320
   ctggatgtag acattcttat ctcaggacat acacacagat ttgaagcatt tgagcatgaa    1380
   aataagttct acatcaaccc aggatcagct acaggagcct acactggttt acaaacgtga    1440
   gtacaatgag ccttgtgttt gtctttaaga cagatgtctg tccttaagag tgcagcccat    1500
   gtgttgaaga gcactggtga gatgcagtgc catgcaggcc atctctccct ttcccagaga 1560
   acacggccta cctctctctg ttcataaggt cttggttaac ttatgagttg gccagcacag 1620
   cctggcactg atgtgtcatg atcataaccc tgagcccacg ctctgctatg ctgttgcttc 1680
   ttagagttgc attgccttga ggtgtattcc agaagctgtg tgctgagctc ttgttgggaa 1740
   agaggccctc agggcactgc caacatctga ggcttgcagg agctggaaca ttcagccctg 1800
   agggctgctc tggccaactg cagctctgag tctgcagtca gtgcctctga gctgtgatgc 1860
   ttctcagata actgcagccc tttgcaatgt aatgttttat aatatatttc cataggacat 1920
   cttggtaaga ggtgtaagct aatcagggtg attgacaaag tatgttttct ttccaggaac 1980
   gtcattcctt catttgtact gatggatatc caggcttcca cagttgtgac ttatgtatac 2040
   cagctaattg aggacgatgt gaaagtagaa agaattgagt tcaagaaata ctgacacgtg 2100
   tttgaatctg cctgctgact tctcactgcc tctcattctt tctctcctag ttaaagctga 2160
   atccggccac gtgggggtgc tgctgcagcg gcgtgtaact gtgagcttgg gaagttctca 2220
   actagggctt agagcaggag aaactgatta acagaaattg ccttattaaa tgtatatatt 2280
   tcctgtgtct aagaacgagg tgctgttgtt cctgacttgc ggagctgcag ctagtggtag 2340
   cttggaattt aacctctcct gtaaaatgag catagaataa aacagtattt tcacatttct 2400
   ggatcctgtt aattgtttgt taattaagag gacaggaatt cagtgtcagg gggctgctct 2460
   ttgcttccca atgtgtacca ctgaagcctc tctggattta tcagagatac tcaatggact 2520
   cttaacagca ctgtggcacc tgacctcctg ctgccttcag cctcccttcc tggctggtgt 2580
   gcttatgttg aggagctgtg ggaggctggt gcagttctgt gggatgaggt aaggccagct 2640
   gaatagcctg ggggctggtc agggggctta gtgagaggag cagggctggg gaatgttgtg 2700
   aacagcaggt ggtgccactg ctattctgag gaaggaggca gcttctgcaa atgctttgca 2760
   atgcaggatg acaaagtgct tctgctagcc ctag 2794
<210> 27
   <211> 10304
   <212> DNA
   <213> Artificial Sequence
<220>
   <223> Targeting vector
<400> 27
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccaagctca     240
   gaattaaccc tcactaaagg gactagtcct gcaggtttaa acgaattcgc ccttgtcgac     300
   gggtatcacg tggggcaaat cgagagctac cattggctat ggggaggaga aaatgaggcg      360
   tgctacttcc tcgttcctgc agtgcgtgtc gaggtgccgt gttggtcgga tctcagtggc      420
   agagcaggat ggtgagcggt ggccttgggg ccgctccctc actctgctgg gctggtgcgg      480
   tggctccggg accggacttt cagtggtgtt tggtgtggtg ctcactgtcc tttggtgtca      540
   ctgtgtggtg aattccctca gctgggcggt gagagcccat gctgtgtcct ggtaggagta      600
   gtctgggctg cagtgcgggc tctttgtgcg tgttgggggc ggttccctgg aagctaattc      660
   tggaagaaat cccggctgcc tctgtaacag ttgttggcga tgtcaaacac cctggagctg      720
   ctgactggct ctgtaccatt gagtgatgca gtttaaacct ctctctttcc tcctgctgtg      780
   tgtgaagctg gtgttagtgc taggagacct gcacatccct caccgatgca gtggtttacc      840
   agcaaagttc aagagcctcc tggttccagg gaagattcag catattttgt gtacaggaaa      900
   cctctgcacc aaggagagct atgactacct caggactttg gctggagatg ttcacgttgt      960
   taagggggac tctgaggtaa tgttcttgct gctgtgaatt cctcagtcct ccatcttaca     1020
   agccgtagtt gtcccactga aagctgggaa acccttcggt gtttgcaatt cctgagcgct     1080
   aatgtgtggg ggaaggatgt aggaacaaat aaggctcaag agacaacaac ttcctaggct     1140
   cctcttattg cccttgtggt aagggaggac ttggtgtgta gtggtaatac atgcactcaa     1200
   cttattaagt tctcatagct gctgtgaagc tgctgcagtc cctcagatcc ctgcaggaag     1260
   aggggactga ctgactgttg aatagccttg aagctcctgc tctgcatgtg gttccggtca     1320
   ggctcaagat cccagttctt agctcttcag ttgctcttct gggggtatag tactagcatc     1380
   tgggtcactg cagatggaca gagactgtgc ttgggtagca aaaggcagac ttacgttgtg     1440
   aaaccttcat tgtgttggtg aggttctttg atttctacct tgtgtatctc tcttcctcag     1500
   agtctgaatt atcctgaaca gaaggttgta actgttgggc agttcagaat tgggttgatt     1560
   catggccatc aagtcattcc ctggggtgat gtggcaagct tggcattgct gcagaggcag     1620
   ctggatgtag acattcttat ctcaggacat acacacagat ttgaagcatt tgagcatgaa     1680
   aataagttct acatcaaccc aggatcagct acaggagcct acactggttt acaaacgtga     1740
   gtacaatgag ccttgtgttt gtctttaaga cagatgtctg tccttaagag tgcagcccat     1800
   gtgttgaaga gcactggtga gatgcagtgc catgcaggcc atctctccct ttcccagaga     1860
   acacggccta cctctctctg ttcataaggt cttggttaac ttatgagttg gccagcacag     1920
   cctggcactg atgtgtcatg atcataaccc tgagcccacg ctctgctatg ctgttgcttc     1980
   ttagagttgc attgccttga ggtgtattcc agaagctgtg tgctgagctc ttgttgggaa     2040
   agaggccctc agggcactgc caacatctga ggcttgcagg agctggaaca ttcagccctg     2100
   agggctgctc tggccaactg cagctctgag tctgcagtca gtgcctctga gctgtgatgc     2160
   ttctcagata actgcagccc tttgcaatgt aatgttttat aatatatttc cataggacat 2220
   cttggtaaga ggtgtaagct aatcagggtg attgacaaag tatgttttct ttccaggaac 2280
   gtcattcctt catttgtact gatggatatc caggcttcca cagttgtgac ttatgtatac 2340
   cagctaattg aggacgatgt gaaagtagaa agaattgagt tcaagaaata ctgacacgtg 2400
   tttgaatctg cctgctgact tctcactgcc tctcattctt tctctcctag ttaaagctga 2460
   atccggccac gtgggggtgc tgctgcagcg gcgtgtaact gtgagcttgg gaagttctca 2520
   actagggctt agagcaggag aaactgatta acagaaattg ccttattaaa tgtatatatt 2580
   tcctgtgtct aagaacgagg tgctgttgtt cctgacttgc ggagctgcag ctagtggtag 2640
   cttggaattt aacctctcct gtaaaatgag catagaataa aacagtattt tcacatttct 2700
   ggatcctgtt aattgtttgt taattaagag gacaggaatt cagtgtcagg gggctgctct 2760
   ttgcttccca atgtgtacca ctgaagcctc tctggattta tcagagatac tcaatggact 2820
   cttaacagca ctgtggcacc tgacctcctg ctgccttcag cctcccttcc tggctggtgt 2880
   gcttatgttg aggagctgtg ggaggctggt gcagttctgt gggatgaggt aaggccagct 2940
   gaatagcctg ggggctggtc agggggctta gtgagaggag cagggctggg gaatgttgtg 3000
   aacagcaggt ggtgccactg ctattctgag gaaggaggca gcttctgcaa atgctttgca 3060
   atgcaggatg acaaagtgct tctgctagcc ctagtcaatt tctgagaact gtcattctcg 3120
   gaatacctcg atcgataact tcgtataatg tatactatac gaagttattt tatttgtgaa 3180
   atttgtgatg ctattgcttt atttgtaacc attataagct gcaataaaca agttggggtg 3240
   ggcgaagaac tccagcatga gatccccgcg ctggaggatc atccagccgg cgtcccggaa 3300
   aacgattccg aagcccaacc tttcatagaa ggcggcggtg gaatcgaaat ctcgtgatgg 3360
   caggttgggc gtcgcttggt cggtcatttc gaaccccaga gtcccgctca gaagaactcg 3420
   tcaagaaggc gatagaaggc gatgcgctgc gaatcgggag cggcgatacc gtaaagcacg 3480
   aggaagcggt cagcccattc gccgccaagc tcttcagcaa tatcacgggt agccaacgct 3540
   atgtcctgat agcggtccgc cacacccagc cggccacagt cgatgaatcc agaaaagcgg 3600
   ccattttcca ccatgatatt cggcaagcag gcatcgccat gggtcacgac gagatcctcg 3660
   ccgtcgggca tgcgcgcctt gagcctggcg aacagttcgg ctggcgcgag cccctgatgc 3720
   tcttcgtcca gatcatcctg atcgacaaga ccggcttcca tccgagtacg tgctcgctcg 3780
   atgcgatgtt tcgcttggtg gtcgaatggg caggtagccg gatcaagcgt atgcagccgc 3840
   cgcattgcat cagccatgat ggatactttc tcggcaggag caaggtgaga tgacaggaga 3900
   tcctgccccg gcacttcgcc caatagcagc cagtcccttc ccgcttcagt gacaacgtcg 3960
   agcacagctg cgcaaggaac gcccgtcgtg gccagccacg atagccgcgc tgcctcgtcc 4020
   tgcagttcat tcagggcacc ggacaggtcg gtcttgacaa aaagaaccgg gcgcccctgc 4080
   gctgacagcc ggaacacggc ggcatcagag cagccgattg tctgttgtgc ccagtcatag 4140
   ccgaatagcc tctccaccca agcggccgga gaacctgcgt gcaatccatc ttgttcaatc 4200
   atgcgaaacg atcctcatcc tgtctcttga tcagatccga aaatggatat acaagctccc 4260
   gggagctttt tgcaaaagcc taggcctcca aaaaagcctc ctcactactt ctggaatagc 4320
   tcagaggcag aggcggcctc ggcctctgca taaataaaaa aaattagtca gccatggggc 4380
   ggagaatggg cggaactggg cggagttagg ggcgggatgg gcggagttag gggcgggact 4440
   atggttgctg actaattgag atgcatgctt tgcatacttc tgcctgctgg ggagcctggg 4500
   gactttccac acctggttgc tgactaattg agatgcatgc tttgcatact tctgcctgct 4560
   ggggagcctg gggactttcc acaccctaaa taacttcgta taatgtatac tatacgaagt 4620
   tatctcgtgt ccgataactg taattatcgg accgtcaatc gattcgcgag gcgcgccata 4680
   ttgcctccct gtgttgctgt tgcagggttg cttctgcctc atactggttt ctcccttctg    4740
   gaggtggcca aaagccgggc cctgtgcaac cctggtgcat aaatacctta tggcccctaa    4800
   gtagggcagg tgtgggacac gctctggcac ctggggtgtg tgcaagtgct caggaagacc 4860
   tgcaggcaca ggtggcagtg gggtctctgg gctgtgctcg agcagcagct gcctggggta 4920
   agggtagtac tctgtgcatg aacaatgctg cagggctcag ctctgctcag accacgaccc 4980
   tggcaccaac agagacctgc ctggctctgt ggtcatgtaa acctttacag gagctcaaga 5040
   caaggctgtt tattactgct ctggcaggaa agaagcactg gccatggtca tagagagttc 5100
   cagcaacagg aaagtgagag cccaagctgc tgaggtacca gggctcctca ggtgcctgct 5160
   gcagcagctt ggacacagtc gaggaacagc aattgtacct gtgtggtgga tcaggctgtg 5220
   ctgcctgtga acctattcta gcacatctgt cacctctgtg ccactcacag ggataccacc 5280
   cctgagaccc ctaccccatc agcctctgtg tgggatatgg tgttgggccc aagggctctg 5340
   ttgcacaggg agatagaggc ctggggagga gggaaagcat tgaggtggtg ttgataccag 5400
   ggatgtgagc ccaagcaaga gatcagcaga gcaaggagga agaattgcag gtgttggggc 5460
   tggggaaagc cccagatggc tggagctggt ggggccactg gagatctcct cctcccatcc 5520
   tgctccatgc tggggcagct gctgcaggct gaccagggcc tgcccgggca cgttgtgaag 5580
   gtcaccaagg atggagactt cagagctatc tgggacacgt gttttaacgt ttgatcagcc 5640
   ttgcagcaaa acagtgtttt gctctattga acagtgttca acaaaaacga tcagtggcct 5700
   ggaacacatc tcctacgaag aaaggttgcg agagctgggt ctggagaaga gaaggctctg 5760
   gggcaacctc cctgtggcct gtcagtgctt aaaggaggct caagagaaag atggagagag 5820
   actttataca cggagctgta gcaatatgac aaggagaaat ggtttaaaat ggaaagaggg 5880
   tagatttagg ttagctatca agaagaaatt cttcactcag aggtgatgaa gcactgtagc 5940
   aggttgccca gagaagtatt ggatgcttcc ttcctggcag ggcccaaggc cagactggat 6000
   gggttctggg cagcctgttc tggtgggaga tgtccctgcc tgtggcatgg cttggaaatg 6060
   ggtagccttt gaggtccctc tcagcccaaa tcattctatg ttcttgtggt tccctgcatt 6120
   tcagcctttg cctgttgact ctggtgccac tgttggacaa ttctactcag ctgcgtttca 6180
   tccctgcccc aaagcagtaa tcccatggat aagcaatgct ctgaggcctt caccaccctc 6240
   agggctcctg ctgaccctag cggcctccct ggcagcatga gccctttgtt gtccgcactg 6300
   ctgctgccga gggtcctgcc agccccccag ggccttttct gcccaggcac tgccacctgc 6360
   taaaacccag cctgcccatg gcagggcatc gcatgtctga gggatggaac ggggcaaggg 6420
   cagtgctgca ggcagtggtt caagaaacgt ttagatgttg tactgaggga tgtggtttag 6480
   tgggaaatac tggtgatagg tggatggttg gactggagga tcttagaggt cttttccaac 6540
   cttggtgatt atgtgattct atgaagggag ccatagcctg ctcccatcat gccccgccag 6600
   tccggctctc ccgagcactt tggttccgca gcatgcggcc gcaagggcga attcgcggcc 6660
   gctaaattca attcgcccta tagtgagtcg tattacaatt cactggccgt cgttttacaa 6720
   cgtcgtgact gggaaaaccc tggcgttacc caacttaatc gccttgcagc acatccccct 6780
   ttcgccagct ggcgtaatag cgaagaggcc cgcaccgatc gcccttccca acagttgcgc 6840
   agcctatacg tacggcagtt taaggtttac acctataaaa gagagagccg ttatcgtctg 6900
   tttgtggatg tacagagtga tattattgac acgccggggc gacggatggt gatccccctg 6960
   gccagtgcac gtctgctgtc agataaagtc tcccgtgaac tttacccggt ggtgcatatc 7020
   ggggatgaaa gctggcgcat gatgaccacc gatatggcca gtgtgccggt ctccgttatc 7080
   ggggaagaag tggctgatct cagccaccgc gaaaatgaca tcaaaaacgc cattaacctg 7140
   atgttctggg gaatataaat gtcaggcatg agattatcaa aaaggatctt cacctagatc 7200
   cttttcacgt agaaagccag tccgcagaaa cggtgctgac cccggatgaa tgtcagctac 7260
   tgggctatct ggacaaggga aaacgcaagc gcaaagagaa agcaggtagc ttgcagtggg 7320
   cttacatggc gatagctaga ctgggcggtt ttatggacag caagcgaacc ggaattgcca 7380
   gctggggcgc cctctggtaa ggttgggaag ccctgcaaag taaactggat ggctttcttg 7440
   ccgccaagga tctgatggcg caggggatca agctctgatc aagagacagg atgaggatcg 7500
   tttcgcatga ttgaacaaga tggattgcac gcaggttctc cggccgcttg ggtggagagg 7560
   ctattcggct atgactgggc acaacagaca atcggctgct ctgatgccgc cgtgttccgg 7620
   ctgtcagcgc aggggcgccc ggttcttttt gtcaagaccg acctgtccgg tgccctgaat 7680
   gaactgcaag acgaggcagc gcggctatcg tggctggcca cgacgggcgt tccttgcgca 7740
   gctgtgctcg acgttgtcac tgaagcggga agggactggc tgctattggg cgaagtgccg 7800
   gggcaggatc tcctgtcatc tcaccttgct cctgccgaga aagtatccat catggctgat 7860
   gcaatgcggc ggctgcatac gcttgatccg gctacctgcc cattcgacca ccaagcgaaa 7920
   catcgcatcg agcgagcacg tactcggatg gaagccggtc ttgtcgatca ggatgatctg 7980
   gacgaagagc atcaggggct cgcgccagcc gaactgttcg ccaggctcaa ggcgagcatg 8040
   cccgacggcg aggatctcgt cgtgacccat ggcgatgcct gcttgccgaa tatcatggtg 8100
   gaaaatggcc gcttttctgg attcatcgac tgtggccggc tgggtgtggc ggaccgctat 8160
   caggacatag cgttggctac ccgtgatatt gctgaagagc ttggcggcga atgggctgac 8220
   cgcttcctcg tgctttacgg tatcgccgct cccgattcgc agcgcatcgc cttctatcgc 8280
   cttcttgacg agttcttctg aattattaac gcttacaatt tcctgatgcg gtattttctc 8340
   cttacgcatc tgtgcggtat ttcacaccgc atcaggtggc acttttcggg gaaatgtgcg 8400
   cggaacccct atttgtttat ttttctaaat acattcaaat atgtatccgc tcatgagatt 8460
   atcaaaaagg atcttcacct agatcctttt aaattaaaaa tgaagtttta aatcaatcta 8520
   aagtatatat gagtaaactt ggtctgacag ttaccaatgc ttaatcagtg aggcacctat 8580
   ctcagcgatc tgtctatttc gttcatccat agttgcctga ctccccgtcg tgtagataac 8640
   tacgatacgg gagggcttac catctggccc cagtgctgca atgataccgc gagacccacg 8700
   ctcaccggct ccagatttat cagcaataaa ccagccagcc ggaagggccg agcgcagaag 8760
   tggtcctgca actttatccg cctccatcca gtctattaat tgttgccggg aagctagagt 8820
   aagtagttcg ccagttaata gtttgcgcaa cgttgttgcc attgctacag gcatcgtggt 8880
   gtcacgctcg tcgtttggta tggcttcatt cagctccggt tcccaacgat caaggcgagt 8940
   tacatgatcc cccatgttgt gcaaaaaagc ggttagctcc ttcggtcctc cgatcgttgt 9000
   cagaagtaag ttggccgcag tgttatcact catggttatg gcagcactgc ataattctct 9060
   tactgtcatg ccatccgtaa gatgcttttc tgtgactggt gagtactcaa ccaagtcatt 9120
   ctgagaatag tgtatgcggc gaccgagttg ctcttgcccg gcgtcaatac gggataatac 9180
   cgcgccacat agcagaactt taaaagtgct catcattgga aaacgttctt cggggcgaaa 9240
   actctcaagg atcttaccgc tgttgagatc cagttcgatg taacccactc gtgcacccaa 9300
   ctgatcttca gcatctttta ctttcaccag cgtttctggg tgagcaaaaa caggaaggca 9360
   aaatgccgca aaaaagggaa taagggcgac acggaaatgt tgaatactca tactcttcct 9420
   ttttcaatat tattgaagca tttatcaggg ttattgtctc atgaccaaaa tcccttaacg 9480
   tgagttttcg ttccactgag cgtcagaccc cgtagaaaag atcaaaggat cttcttgaga 9540
   tccttttttt ctgcgcgtaa tctgctgctt gcaaacaaaa aaaccaccgc taccagcggt 9600
   ggtttgtttg ccggatcaag agctaccaac tctttttccg aaggtaactg gcttcagcag 9660
   agcgcagata ccaaatactg ttcttctagt gtagccgtag ttaggccacc acttcaagaa 9720
   ctctgtagca ccgcctacat acctcgctct gctaatcctg ttaccagtgg ctgctgccag 9780
   tggcgataag tcgtgtctta ccgggttgga ctcaagacga tagttaccgg ataaggcgca 9840
   gcggtcgggc tgaacggggg gttcgtgcac acagcccagc ttggagcgaa cgacctacac 9900
   cgaactgaga tacctacagc gtgagctatg agaaagcgcc acgcttcccg aagggagaaa 9960
   ggcggacagg tatccggtaa gcggcagggt cggaacagga gagcgcacga gggagcttcc 10020
   agggggaaac gcctggtatc tttatagtcc tgtcgggttt cgccacctct gacttgagcg 10080
   tcgatttttg tgatgctcgt caggggggcg gagcctatgg aaaaacgcca gcaacgcggc 10140
   ctttttacgg ttcctggcct tttgctggcc ttttgctcac atgttctttc ctgcgttatc 10200
   ccctgattct gtggataacc gtattaccgc ctttgagtga gctgataccg ctcgccgcag 10260
   ccgaacgacc gagcgcagcg agtcagtgag cgaggaagcg gaag 10304
<210> 28
   <211> 11071
   <212> DNA
   <213> Artificial Sequence
<220>
   <223> Targeting vector
<400> 28
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccaagctca     240
   gaattaaccc tcactaaagg gactagtcct gcaggtttaa acgaattcgc ccttgtcgac     300
   gggtatcacg tggggcaaat cgagagctac cattggctat ggggaggaga aaatgaggcg     360
   tgctacttcc tcgttcctgc agtgcgtgtc gaggtgccgt gttggtcgga tctcagtggc     420
   agagcaggat ggtgagcggt ggccttgggg ccgctccctc actctgctgg gctggtgcgg     480
   tggctccggg accggacttt cagtggtgtt tggtgtggtg ctcactgtcc tttggtgtca     540
   ctgtgtggtg aattccctca gctgggcggt gagagcccat gctgtgtcct ggtaggagta     600
   gtctgggctg cagtgcgggc tctttgtgcg tgttgggggc ggttccctgg aagctaattc     660
   tggaagaaat cccggctgcc tctgtaacag ttgttggcga tgtcaaacac cctggagctg     720
   ctgactggct ctgtaccatt gagtgatgca gtttaaacct ctctctttcc tcctgctgtg     780
   tgtgaagctg gtgttagtgc taggagacct gcacatccct caccgatgca gtggtttacc     840
   agcaaagttc aagagcctcc tggttccagg gaagattcag catattttgt gtacaggaaa     900
   cctctgcacc aaggagagct atgactacct caggactttg gctggagatg ttcacgttgt     960
   taagggggac tctgaggtaa tgttcttgct gctgtgaatt cctcagtcct ccatcttaca    1020
   agccgtagtt gtcccactga aagctgggaa acccttcggt gtttgcaatt cctgagcgct 1080
   aatgtgtggg ggaaggatgt aggaacaaat aaggctcaag agacaacaac ttcctaggct 1140
   cctcttattg cccttgtggt aagggaggac ttggtgtgta gtggtaatac atgcactcaa 1200
   cttattaagt tctcatagct gctgtgaagc tgctgcagtc cctcagatcc ctgcaggaag 1260
   aggggactga ctgactgttg aatagccttg aagctcctgc tctgcatgtg gttccggtca 1320
   ggctcaagat cccagttctt agctcttcag ttgctcttct gggggtatag tactagcatc 1380
   tgggtcactg cagatggaca gagactgtgc ttgggtagca aaaggcagac ttacgttgtg 1440
   aaaccttcat tgtgttggtg aggttctttg atttctacct tgtgtatctc tcttcctcag 1500
   agtctgaatt atcctgaaca gaaggttgta actgttgggc agttcagaat tgggttgatt 1560
   catggccatc aagtcattcc ctggggtgat gtggcaagct tggcattgct gcagaggcag 1620
   ctggatgtag acattcttat ctcaggacat acacacagat ttgaagcatt tgagcatgaa 1680
   aataagttct acatcaaccc aggatcagct acaggagcct acactggttt acaaacgtga 1740
   gtacaatgag ccttgtgttt gtctttaaga cagatgtctg tccttaagag tgcagcccat 1800
   gtgttgaaga gcactggtga gatgcagtgc catgcaggcc atctctccct ttcccagaga 1860
   acacggccta cctctctctg ttcataaggt cttggttaac ttatgagttg gccagcacag 1920
   cctggcactg atgtgtcatg atcataaccc tgagcccacg ctctgctatg ctgttgcttc 1980
   ttagagttgc attgccttga ggtgtattcc agaagctgtg tgctgagctc ttgttgggaa 2040
   agaggccctc agggcactgc caacatctga ggcttgcagg agctggaaca ttcagccctg 2100
   agggctgctc tggccaactg cagctctgag tctgcagtca gtgcctctga gctgtgatgc 2160
   ttctcagata actgcagccc tttgcaatgt aatgttttat aatatatttc cataggacat 2220
   cttggtaaga ggtgtaagct aatcagggtg attgacaaag tatgttttct ttccaggaac 2280
   gtcattcctt catttgtact gatggatatc caggcttcca cagttgtgac ttatgtatac 2340
   cagctaattg aggacgatgt gaaagtagaa agaattgagt tcaagaaata ctgacacgtg 2400
   tttgaatctg cctgctgact tctcactgcc tctcattctt tctctcctag ttaaagctga 2460
   atccggccac gtgggggtgc tgctgcagcg gcgtgtaact gtgagcttgg gaagttctca 2520
   actagggctt agagcaggag aaactgatta acagaaattg ccttattaaa tgtatatatt 2580
   tcctgtgtct aagaacgagg tgctgttgtt cctgacttgc ggagctgcag ctagtggtag 2640
   cttggaattt aacctctcct gtaaaatgag catagaataa aacagtattt tcacatttct 2700
   ggatcctgtt aattgtttgt taattaagag gacaggaatt cagtgtcagg gggctgctct 2760
   ttgcttccca atgtgtacca ctgaagcctc tctggattta tcagagatac tcaatggact 2820
   cttaacagca ctgtggcacc tgacctcctg ctgccttcag cctcccttcc tggctggtgt 2880
   gcttatgttg aggagctgtg ggaggctggt gcagttctgt gggatgaggt aaggccagct 2940
   gaatagcctg ggggctggtc agggggctta gtgagaggag cagggctggg gaatgttgtg 3000
   aacagcaggt ggtgccactg ctattctgag gaaggaggca gcttctgcaa atgctttgca 3060
   atgcaggatg acaaagtgct tctgctagcc ctagtcaatt tctgagaact gtcattctcg 3120
   gaatacctcg atcgataact tcgtataatg tatactatac gaagttattt tatttgtgaa 3180
   atttgtgatg ctattgcttt atttgtaacc attataagct gcaataaaca agttggggtg 3240
   ggcgaagaac tccagcatga gatccccgcg ctggaggatc atccagccgg cgtcccggaa 3300
   aacgattccg aagcccaacc tttcatagaa ggcggcggtg gaatcgaaat ctcgtgatgg 3360
   caggttgggc gtcgcttggt cggtcatttc gaaccccaga gtcccgctca gaagaactcg 3420
   tcaagaaggc gatagaaggc gatgcgctgc gaatcgggag cggcgatacc gtaaagcacg 3480
   aggaagcggt cagcccattc gccgccaagc tcttcagcaa tatcacgggt agccaacgct 3540
   atgtcctgat agcggtccgc cacacccagc cggccacagt cgatgaatcc agaaaagcgg 3600
   ccattttcca ccatgatatt cggcaagcag gcatcgccat gggtcacgac gagatcctcg 3660
   ccgtcgggca tgcgcgcctt gagcctggcg aacagttcgg ctggcgcgag cccctgatgc 3720
   tcttcgtcca gatcatcctg atcgacaaga ccggcttcca tccgagtacg tgctcgctcg 3780
   atgcgatgtt tcgcttggtg gtcgaatggg caggtagccg gatcaagcgt atgcagccgc 3840
   cgcattgcat cagccatgat ggatactttc tcggcaggag caaggtgaga tgacaggaga 3900
   tcctgccccg gcacttcgcc caatagcagc cagtcccttc ccgcttcagt gacaacgtcg 3960
   agcacagctg cgcaaggaac gcccgtcgtg gccagccacg atagccgcgc tgcctcgtcc 4020
   tgcagttcat tcagggcacc ggacaggtcg gtcttgacaa aaagaaccgg gcgcccctgc 4080
   gctgacagcc ggaacacggc ggcatcagag cagccgattg tctgttgtgc ccagtcatag 4140
   ccgaatagcc tctccaccca agcggccgga gaacctgcgt gcaatccatc ttgttcaatc 4200
   atgcgaaacg atcctcatcc tgtctcttga tcagatccga aaatggatat acaagctccc 4260
   gggagctttt tgcaaaagcc taggcctcca aaaaagcctc ctcactactt ctggaatagc 4320
   tcagaggcag aggcggcctc ggcctctgca taaataaaaa aaattagtca gccatggggc 4380
   ggagaatggg cggaactggg cggagttagg ggcgggatgg gcggagttag gggcgggact 4440
   atggttgctg actaattgag atgcatgctt tgcatacttc tgcctgctgg ggagcctggg 4500
   gactttccac acctggttgc tgactaattg agatgcatgc tttgcatact tctgcctgct 4560
   ggggagcctg gggactttcc acaccctaaa taacttcgta taatgtatac tatacgaagt 4620
   tatctcgtgt ccgataactg taattatcgg accgtcaatc gattcgcgac agtctgccct 4680
   gactcagcct gcctccgtgt ctgggtctcc tggacagtcg atcaccatct cctgcactgg 4740
   aaccagctca agtgacgttg gtggttataa ctatgtctcc tggtaccaac aaaacccagg 4800
   caaagccccc aaactcatga tttatgatgt cagttcaaat cggccctcag ggatttctaa 4860
   tcgcttctct ggctccaagt ctggcaacac ggcctccctg accatctctg ggctccaggc 4920
   tgacgacgag gctgattatt actgcacctc atatacaaac gacggtagca attctcaggt 4980
   attcggcgga gggaccaagc tgaccgtcct aggtgcctga cacacacagc cctccccaga 5040
   ctgtgggatg aggccctgtg cccgcagtca catgtggaat atcaagacac acacatctat 5100
   gacaatcacc agtctgccct gactcagcct gcctccgtgt ctgggtctcc tggacagtcg 5160
   atcaccatct cctgcactgg aaccagcagt gacgttggtt ataactatgt ctcctggtac 5220
   caacaaaacc caggcaaagc ccccaaactc atgatttatg atggtaatcg gccctcaggg 5280
   atttctaatc gcttctctgg ctccaagtct ggcaacacgg cctccctgac catctctggg 5340
   ctccaggctg acgacgaggc tgattattac tgcacctcat atacaaacga cagctctcag 5400
   gtattcggcg gagggaccaa gctgaccgtc ctaggtggcg cgccatattg cctccctgtg 5460
   ttgctgttgc agggttgctt ctgcctcata ctggtttctc ccttctggag gtggccaaaa 5520
   gccgggccct gtgcaaccct ggtgcataaa taccttatgg cccctaagta gggcaggtgt 5580
   gggacacgct ctggcacctg gggtgtgtgc aagtgctcag gaagacctgc aggcacaggt 5640
   ggcagtgggg gtctctggct gtgctcgagc agcagctgcc tggggtaagg gtagtactct 5700
   gtgcatgaac aatgctgcag ggctcagctc tgctcagacc acgaccctgg caccaacaga 5760
   gacctgcctg gctctgtggt catgtaaacc tttacaggag ctcaagacaa ggctgtttat 5820
   tactgctctg gcaggaaaga agcactggcc atggtcatag agagttccag caacaggaaa 5880
   gtgagagccc aagctgctga ggtaccaggg ctcctcaggt gcctgctgca gcagcttgga 5940
   cacagtcgag gaacagcaat tgtacctgtg tggtggatca ggctgtgctg cctgtgaacc 6000
   tattctagca catctgtcac ctctgtgcca ctcacaggga taccacccct gagaccccta 6060
   ccccatcagc ctctgtgtgg gatatggtgt tgggcccaag ggctctgttg cacagggaga 6120
   tagaggcctg gggaggaggg aaagcattga ggtggtgttg ataccaggga tgtgagccca 6180
   agcaagagat cagcagagca aggaggaaga attgcaggtg ttggggctgg ggaaagcccc 6240
   agatggctgg agctggtggg gccactggag atctcctcct cccatcctgc tccatgctgg 6300
   ggcagctgct gcaggctgac cagggcctgc ccgggcacgt tgtgaaggtc accaaggatg 6360
   gagacttcag agctatctgg gacacgtgtt ttaacgtttg atcagccttg cagcaaaaca 6420
   gtgttttgct ctattgaaca gtgttcaaca aaaacgatca gtggcctgga acacatctcc 6480
   tacgaagaaa ggttgcgaga gctgggtctg gagaagagaa ggctctgggg caacctccct 6540
   gtggcctgtc agtgcttaaa ggaggctcaa gagaaagatg gagagagact ttatacacgg 6600
   agctgtagca atatgacaag gagaaatggt ttaaaatgga aagagggtag atttaggtta 6660
   gctatcaaga agaaattctt cactcagagg tgatgaagca ctgtagcagg ttgcccagag 6720
   aagtattgga tgcttccttc ctggcagggc ccaaggccag actggatggg ttctgggcag 6780
   cctgttctgg tgggagatgt ccctgcctgt ggcatggctt ggaaatgggt agcctttgag 6840
   gtccctctca gcccaaatca ttctatgttc ttgtggttcc ctgcatttca gcctttgcct 6900
   gttgactctg gtgccactgt tggacaattc tactcagctg cgtttcatcc ctgccccaaa 6960
   gcagtaatcc catggataag caatgctctg aggccttcac caccctcagg gctcctgctg 7020
   accctagcgg cctccctggc agcatgagcc ctttgttgtc cgcactgctg ctgccgaggg 7080
   tcctgccagc cccccagggc cttttctgcc caggcactgc cacctgctaa aacccagcct 7140
   gcccatggca gggcatcgca tgtctgaggg atggaacggg gcaagggcag tgctgcaggc 7200
   agtggttcaa gaaacgttta gatgttgtac tgagggatgt ggtttagtgg gaaatactgg 7260
   tgataggtgg atggttggac tggaggatct tagaggtctt ttccaacctt ggtgattatg 7320
   tgattctatg aagggagcca tagcctgctc ccatcatgcc ccgccagtcc ggctctcccg 7380
   agcactttgg ttccgcagca tgcggccgca agggcgaatt cgcggccgct aaattcaatt 7440
   cgccctatag tgagtcgtat tacaattcac tggccgtcgt tttacaacgt cgtgactggg 7500
   aaaaccctgg cgttacccaa cttaatcgcc ttgcagcaca tccccctttc gccagctggc 7560
   gtaatagcga agaggcccgc accgatcgcc cttcccaaca gttgcgcagc ctatacgtac 7620
   ggcagtttaa ggtttacacc tataaaagag agagccgtta tcgtctgttt gtggatgtac 7680
   agagtgatat tattgacacg ccggggcgac ggatggtgat ccccctggcc agtgcacgtc 7740
   tgctgtcaga taaagtctcc cgtgaacttt acccggtggt gcatatcggg gatgaaagct 7800
   ggcgcatgat gaccaccgat atggccagtg tgccggtctc cgttatcggg gaagaagtgg 7860
   ctgatctcag ccaccgcgaa aatgacatca aaaacgccat taacctgatg ttctggggaa 7920
   tataaatgtc aggcatgaga ttatcaaaaa ggatcttcac ctagatcctt ttcacgtaga 7980
   aagccagtcc gcagaaacgg tgctgacccc ggatgaatgt cagctactgg gctatctgga 8040
   caagggaaaa cgcaagcgca aagagaaagc aggtagcttg cagtgggctt acatggcgat 8100
   agctagactg ggcggtttta tggacagcaa gcgaaccgga attgccagct ggggcgccct 8160
   ctggtaaggt tgggaagccc tgcaaagtaa actggatggc tttcttgccg ccaaggatct 8220
   gatggcgcag gggatcaagc tctgatcaag agacaggatg aggatcgttt cgcatgattg 8280
   aacaagatgg attgcacgca ggttctccgg ccgcttgggt ggagaggcta ttcggctatg 8340
   actgggcaca acagacaatc ggctgctctg atgccgccgt gttccggctg tcagcgcagg 8400
   ggcgcccggt tctttttgtc aagaccgacc tgtccggtgc cctgaatgaa ctgcaagacg 8460
   aggcagcgcg gctatcgtgg ctggccacga cgggcgttcc ttgcgcagct gtgctcgacg 8520
   ttgtcactga agcgggaagg gactggctgc tattgggcga agtgccgggg caggatctcc 8580
   tgtcatctca ccttgctcct gccgagaaag tatccatcat ggctgatgca atgcggcggc 8640
   tgcatacgct tgatccggct acctgcccat tcgaccacca agcgaaacat cgcatcgagc 8700
   gagcacgtac tcggatggaa gccggtcttg tcgatcagga tgatctggac gaagagcatc 8760
   aggggctcgc gccagccgaa ctgttcgcca ggctcaaggc gagcatgccc gacggcgagg 8820
   atctcgtcgt gacccatggc gatgcctgct tgccgaatat catggtggaa aatggccgct 8880
   tttctggatt catcgactgt ggccggctgg gtgtggcgga ccgctatcag gacatagcgt 8940
   tggctacccg tgatattgct gaagagcttg gcggcgaatg ggctgaccgc ttcctcgtgc 9000
   tttacggtat cgccgctccc gattcgcagc gcatcgcctt ctatcgcctt cttgacgagt 9060
   tcttctgaat tattaacgct tacaatttcc tgatgcggta ttttctcctt acgcatctgt 9120
   gcggtatttc acaccgcatc aggtggcact tttcggggaa atgtgcgcgg aacccctatt 9180
   tgtttatttt tctaaataca ttcaaatatg tatccgctca tgagattatc aaaaaggatc 9240
   ttcacctaga tccttttaaa ttaaaaatga agttttaaat caatctaaag tatatatgag 9300
   taaacttggt ctgacagtta ccaatgctta atcagtgagg cacctatctc agcgatctgt 9360
   ctatttcgtt catccatagt tgcctgactc cccgtcgtgt agataactac gatacgggag 9420
   ggcttaccat ctggccccag tgctgcaatg ataccgcgag acccacgctc accggctcca 9480
   gatttatcag caataaacca gccagccgga agggccgagc gcagaagtgg tcctgcaact 9540
   ttatccgcct ccatccagtc tattaattgt tgccgggaag ctagagtaag tagttcgcca 9600
   gttaatagtt tgcgcaacgt tgttgccatt gctacaggca tcgtggtgtc acgctcgtcg 9660
   tttggtatgg cttcattcag ctccggttcc caacgatcaa ggcgagttac atgatccccc 9720
   atgttgtgca aaaaagcggt tagctccttc ggtcctccga tcgttgtcag aagtaagttg 9780
   gccgcagtgt tatcactcat ggttatggca gcactgcata attctcttac tgtcatgcca 9840
   tccgtaagat gcttttctgt gactggtgag tactcaacca agtcattctg agaatagtgt 9900
   atgcggcgac cgagttgctc ttgcccggcg tcaatacggg ataataccgc gccacatagc 9960
   agaactttaa aagtgctcat cattggaaaa cgttcttcgg ggcgaaaact ctcaaggatc 10020
   ttaccgctgt tgagatccag ttcgatgtaa cccactcgtg cacccaactg atcttcagca 10080
   tcttttactt tcaccagcgt ttctgggtga gcaaaaacag gaaggcaaaa tgccgcaaaa 10140
   aagggaataa gggcgacacg gaaatgttga atactcatac tcttcctttt tcaatattat 10200
   tgaagcattt atcagggtta ttgtctcatg accaaaatcc cttaacgtga gttttcgttc 10260
   cactgagcgt cagaccccgt agaaaagatc aaaggatctt cttgagatcc tttttttctg 10320
   cgcgtaatct gctgcttgca aacaaaaaaa ccaccgctac cagcggtggt ttgtttgccg 10380
   gatcaagagc taccaactct ttttccgaag gtaactggct tcagcagagc gcagatacca 10440
   aatactgttc ttctagtgta gccgtagtta ggccaccact tcaagaactc tgtagcaccg 10500
   cctacatacc tcgctctgct aatcctgtta ccagtggctg ctgccagtgg cgataagtcg 10560
   tgtcttaccg ggttggactc aagacgatag ttaccggata aggcgcagcg gtcgggctga 10620
   acggggggtt cgtgcacaca gcccagcttg gagcgaacga cctacaccga actgagatac 10680
   ctacagcgtg agctatgaga aagcgccacg cttcccgaag ggagaaaggc ggacaggtat 10740
   ccggtaagcg gcagggtcgg aacaggagag cgcacgaggg agcttccagg gggaaacgcc 10800
   tggtatcttt atagtcctgt cgggtttcgc cacctctgac ttgagcgtcg atttttgtga 10860
   tgctcgtcag gggggcggag cctatggaaa aacgccagca acgcggcctt tttacggttc 10920
   ctggcctttt gctggccttt tgctcacatg ttctttcctg cgttatcccc tgattctgtg 10980
   gataaccgta ttaccgcctt tgagtgagct gataccgctc gccgcagccg aacgaccgag 11040
   cgcagcgagt cagtgagcga ggaagcggaa g 11071
<210> 29
   <211> 2787
   <212> DNA
   <213> Homo sapiens
<400> 29
   cctccaccaa gggcccatcg gtcttccccc tggcaccctc ctccaagagc acctctgggg      60
   gcacagcggc cctgggctgc ctggtcaagg actacttccc cgaaccggtg acggtgtcgt     120
   ggaactcagg cgccctgacc agcggcgtgc acaccttccc ggctgtccta cagtcctcag     180
   gactctactc cctcagcagc gtggtgaccg tgccctccag cagcttgggc acccagacct     240
   acatctgcaa cgtgaatcac aagcccagca acaccaaggt ggacaagaga gttgagccca     300
   aatcttgtga caaaactcac acatgcccac cgtgcccagg taagtcacta gaccagagct     360
   ccactcccgg gagaatggta agtgctgtaa acatccctgc actagaggat aagccatgta     420
   cagatccatt tccatctctc ctcatcagca cctgaactcc tggggggacc gtcagtcttc     480
   ctcttccccc caaaacccaa ggacaccctc atgatctccc ggacccctga ggtcacatgc     540
   gtggtggtgg acgtgagcca cgaagaccct gaggtcaagt tcaactggta cgtggacggc     600
   gtggaggtgc ataatgccaa gacaaagccg cgggaggagc agtacaacag cacgtaccgt     660
   gtggtcagcg tcctcaccgt cctgcaccag gactggctga atggcaagga gtacaagtgc     720
   aaggtctcca acaaagccct cccagccccc atcgagaaaa ccatctccaa agccaaaggt     780
   gagagctgca gcctgactgc atgggggctg ggatgggcat aaggataaag gtctgtgtgg     840
   acagccttct gcttcagcca tgacctttgt gtatgtttct accctcacag ggcagccccg     900
   agaaccacag gtgtacaccc tgcccccatc ccgggaggag atgaccaaga accaggtcag     960
   cctgacctgc ctggtcaaag gcttctatcc cagcgacatc gccgtggagt gggagagcaa    1020
   tgggcagccg gagaacaact acaagaccac gcctcccgtg ctggactccg acggctcctt      1080
   cttcctctat agcaagctca ccgtggacaa gagcaggtgg cagcagggga acgtcttctc      1140
   atgctccgtg atgcatgagg ctctgcacaa ccactacacg cagaagagcc tctccctgtc      1200
   cccgggtaaa tgagctcagc acccacaaaa ctctcaggtc caaagagaca cccacactca      1260
   tctccatgct tcccttgtat aaataaagca cccagcaatg cctgggacca tgtaaaactg      1320
   tcctggttct ttccaaggta tagagcatag ctcacaggct gatatttctg gccagggttg      1380
   gaggacagcc ttgtctatag gaagagaatg aggtttttgc actgcaggac tcagagctca      1440
   ttagttatcc tgccttggag tgttggggct tggctttagg cagtgccttt tccttgcctt      1500
   cctacgaacc agcagctgcc atacatagag ataatcctag gaagcctcaa atggagaagg      1560
   acacaaaccc acctccctca ggctgttcct ctatcccggc cccacttctt tacctagggg      1620
   tttctctgag tctattgtgg agttacacat ggccaggggc attccagaga cccttgtcat      1680
   ccatacactc aactcaggca gctttgcaca aacaaagtct gcacacccat acagatggct      1740
   cactcttgcc tgtgccatgt agggctgagg cacatggctc ttgctgcccc aagggaggga      1800
   ctattagata gccacactca tgctgaatcc tggcccattc aaattagcct gctgaacacc      1860
   atccagtcca tatagcacat gtatccacat gcacgtgtgc acaaaacgca tttaacacac      1920
   tgggacaaca attctgtgcc ctgcacagca cctatatcca gcaatgtatc accatacaca      1980
   cgaccaaaaa ttcaatgccc acgtttctgc catcacaaac agacacatct ttcctctctg      2040
   tggccactgc attatatgct caacacaaga cctctgaagc cagatccatc tctggcacct      2100
   cggggtcatg cttcaacccc acatgaatta tgcaaaccat agccataatg gtctgaatca      2160
   cttcacactg ggatgttccc aagttcaggc aagacgagcc acaggctctg ctgatgactg      2220
   aaggacagca aagggtcagt ccagctgtat agccactgtt gacctgggtc acaggccctg      2280
   ctgaccctcc accttctcct gtactgaagg aatgaaagat gagacaagca tagagggcac      2340
   ttgaataatc caggtcactc tgaggtccac ccaaggcatt attggactca ggtgggaagc      2400
   tgagactggt gtcccagagg gaaaggaagg aaagcaggcc ccggggaggg tctgctgtcc      2460
   cagtcaggct ggagatctct cctctgaatc catgcagaca tgtctgcctc acagggaatc      2520
   tctcccagca ccaaccatgt tgggacaaac actgactgtc ctctctgttc agagctgcaa      2580
   ctggaggaga gctgtgcgga ggcgcaggac ggggagctgg acgggctgtg gacgaccatc      2640
   accatcttca tcacactctt cctgttaagc gtgtgctaca gtgccaccgt caccttcttc      2700
   aaggtgaagt ggatcttctc ctcggtggtg gacctgaagc agaccatcat ccccgactac      2760
   aggaacatga tcggacaggg ggcctag                                         2787
<210> 30
   <211> 361
   <212> DNA
   <213> Homo sapiens
<400> 30
   gaggtgcagc tgttggagtc tgggggaggc ttggtacagc ctggggggtc cctgagactc      60
   tcctgtgcag cctctggatt cacctttagc agctatgcca tgagctgggt ccgccaggct     120
   ccagggaagg ggctggagtg ggtctcagct attagtggta gtggtggtag cacatactac     180
   gcagactccg tgaagggccg gttcaccatc tccagagaca attccaagaa cacgctgtat     240
   ctgcaaatga acagcctgag agccgaggac acggccgtat attactgtgc gaaaggatat     300
   agtggctacg attactactt tgactactgg ggccagggca ccctggtcac cgtctcctca     360
   g                                                                     361
<210> 31
   <211> 21
   <212> DNA
   <213> Artificial Sequence
<220>
   <223> Synthetic Primer
<400> 31
   ttcccgaagc gaaagccgcg t 21
<210> 32
   <211> 21
   <212> DNA
   <213> Artificial Sequence
<220>
   <223> Synthetic Primer
<400> 32
   actcaccgga ggagacgatg a 21
<210> 33
   <211> 1823
   <212> DNA
   <213> Gallus gallus
<400> 33
   ttcccgaagc gaaagccgcg tgttattttt gggtgggaaa gccccgattc gctccttttt      60
   ttttatttat tatttttgtt gtttttcgtg tctttttctt ctccgttttg cagctgcggc     120
   ctccaggtgt ccccggccgt ggtatttttg ggtcgaaaaa gccgcttttt ggctcacttg     180
   gaaacctttc gggcgtctcc aagcggagcg ttggcgggaa atggtgtttt ttttgggttg     240
   ttttcggtcc cttttttcac catttttgtg gtttttcggc ccattttcgg cctccccccc     300
   cccccccccc ttcccgtggg gctgcgctcc gtggagcgga atggggtcgt taattaaggt     360
   taattggttt aattaatggg ccgtgcaggg agaagagggc cgtgcggtgg ttgttagccc     420
   tgattaatgg cttggttgag gggagagtgt aattaagtgc ttcgttagca ctaattaatg      480
   gcttgattga gaggagagtg taattaaatg cttcataatc cctaattaat ggcttggttg      540
   agacaagagt gtaattaagt gcttcattag ccctgattaa tggcttggtt gaggggagag      600
   tgtaattaaa tgcttcgtta gcactaatta atggcttgat tgagaggaga gtgtaattaa      660
   atgcttcata atccctaatt aatggcttgg ttgagacaag agtgtaatta agtgcttcat      720
   tagccctgat taatggcttg gttgagagga gagtgtaatt aaatgcttca ttatccctaa      780
   ttagggactg aattccaagg agagtgtaat taagtgcttc attatcccta attaatggct      840
   tggttgagag gagagtgtaa ttaaatgctt tgttagccct aattaatggt ttgatggaga      900
   ggagagtgta attaagtgct tcattatccc taattaatgg cttggttgag gggagagtgt      960
   aattaaatgc ttagttagcc ctaattaatg gcttggttga gaggagagtg taattgaatg     1020
   cttcattatc cctaattagg gattgaattg agaggagagt gtaattgaat gctttgttag     1080
   ccctaattag ggattgaatt gagaggagag tgtaattaaa tgcctcatta tccctaatta     1140
   gggattgaat tgagaggaga gtttaattaa atgcctcatt atccctaatt agggactgaa     1200
   ttccgacgag agtgtaatta agtgcttcat ttagccctaa ttaatggctt gattgagagg     1260
   agagtgtaat taaatgcttc attatcccta attagggatt gaattgagag gagagtgtaa     1320
   ttaaatgctt cattatccct aattagggat tgaattgaga ggagagtgta attaaatgct     1380
   tcattatccc taattaatgg cttggttgag aggggagtgt aattaagtgc ttcgttatcc     1440
   ctaattaggg attgaattgt gaggagatgg caattaatac ttcattatcc ctaattaatt     1500
   taattagtcc ctattatccc taatttaatt catggtttgg ttgatggaag aatgtaatta     1560
   aatgctttgt tatccctaat taatggtttg attgagcgga gagtgtaatt aaatgcttca     1620
   ttatccctta ttaatggttg tgtggaatgt agtgtaatta gaagtcccta attaatggtt     1680
   taattgagag aagactgcaa ttagatgact catagcccct aataagtggt ttaattatga     1740
   gataatcaca attacatgtt tcattgtccc taattaatcg tttaattgcg agaagatcac     1800
   aattaattaa atgatcccta att                                             1823
<210> 34
   <211> 1358
   <212> DNA
   <213> Gallus gallus
<400> 34
   aatgttggcg cagaagggat tcatatcaat taattccctg tcctcaatta atgcataatt     60
   gggagtgcga tggaatcatt caataagtaa tgaggacttg gtgtcaaaat gccaattaag    120
   taattaatga cacccaatta atacacaatg gggattacag tggaataact cagtgaagtt    180
   catatctaaa ttgcaattga ataattaatt acagctaatt aatggatggg agactggcag    240
   tggtataact taatgaagga acacagtgaa ttcctgtctg aattgaaatt aattgattta    300
   ttagggctaa ttaatgaatg gtggggttaa aaatagcatt actcagcgaa ggaacatgag    360
   ttcatggcta aattgagatt gaattattaa ttacaactaa ttgattaatg actgatggga    420
   gtaagaatgg aataactcag tgaaggaaca caatggattc atggcctaat tgaaattaaa    480
   tggttaatta gagctaatta atgaatgatg gggggtggca atggaatgat acaagaaaga    540
   aacacaatgg gttcgtgtct acattgaaat taaataatta attacagcta attaataaac    600
   ggcgaggttg acaatggtat aactgtgaag agacagaatg agttcgtgac taaattggta    660
   ttaaatgatt aattagagct aattaacagg tggtggggct gagaatggca taactcaatg    720
   ggggatgatg agctgatggc ttaattggaa ttaataatta atcacaacca attaacaggt    780
   gctgttaatg gggttacagg ggcatgactt caggaagaag cacaaagtgg ccattaaata    840
   attaattgca cccaattaat tcctgatggg gattactgca gaggaactca gcgaaggatt    900
   caagcagaaa tgacacagtt aggttgctca attgcgttaa ttaattaaaa tcgctaatta    960
   ggtgagcttc attaaggcct gatggggttc atcccaacgc tgggatcaaa gaactcagcg   1020
   cagagtggat aacgataatt agaaggggac aaattaatga ggaaacacga ctttgggcct   1080
   agacggccat taaatcatta atcaacccga cgggcgccca attaatcata aagttattaa   1140
   gcaattaagt cgaggctgac gagaaaccgc aacgagagcc ctccacgctc ctttgagttg   1200
   gcgcaaacaa caaacaacgc gggctcgtta agcagcgacg acgccgagcg taacgaagca   1260
   atccccgcgc ccgacaatta acgcgctaac gaagccggag ccctccttat gcaaattagc   1320
   ccctccagag ggccataaaa gcgccggctc tccgacgg                           1358
<210> 35
   <211> 28
   <212> DNA
   <213> Artificial Sequence
<220>
   <223> Synthetic Primer
<400> 35
   ggggatcctg ggtcagtcga agggggcg 28
<210> 36
   <211> 31
   <212> DNA
   <213> Artificial Sequence
<220>
   <223> Synthetic Primer
<400> 36
   gtgcggccgc caaaagcggt aaaatccacc c 31
<210> 37
   <211> 4266
   <212> DNA
   <213> Gallus gallus
<220>
   <221> misc_feature
   <222> (688)..(688)
   <223> n is a, c, g, or t
<400> 37
   tgggtcagtc gaagggggcg gtcggtccat catttgcggc cgggtgattt ttggggccga      60
   aaagtgggaa tttggggccc aatttggggc ccaatttggg gcccaatttg ggttttcgag     120
   gggggatttt tttaggggga gatttttggg tccccggaaa atggtgawtt tgggtcaaaa     180
   atggggawtt tgggggttck ttttraggtg gggttttttg gggkagaaat ggcggtctga      240
   ggggaaaatg tcgttttggg gccattttgg gccatttgag gggaaatttg ggtcaaaaaa     300
   tgacgatttt gggtcgtttt agggaggaaa atgaatttag ggcgatttga gccaaatttg     360
   ggtcaaaaat ggcgattttg agtcaaaaaa tgattatttt gggtcatttt agggaggaaa     420
   atgaatttag ggagattgga cggcaaattt gggtcaaaaa atggtgattt tgggtcaaaa     480
   aatgattatt ttgggtcatt ttagggagaa aaatgaattt agggagattt gagggcaaat     540
   ttgggtcaaa aaatggtgat tttgggtcaa aaaatgatta ttttgggtca ttttagggag     600
   aaaaatgaat ttagggagat ttgagggcaa atttgggtcg aaaaatggcg attttgggtg     660
   aaaaatggac aattttgggt cattttangg ttaaaaatga atttagggag attggacggc     720
   aaatttgggt craaaaatgg tgatttgggg tsaaaaatgr aywattttkg gtcattttag     780
   ggataaaaaa tgaatttagg gcgatgtgag ccaattttgg gtcaaaaatg gcgattgtga     840
   gtcaaaaaat gattattttg ggtcatttta gggttaaaaa tgaatttagg gagattggac     900
   ggcaaatttg ggtcaaaaaa tggtgatttt cggtcaaaaa atgattattt tgggtcattt     960
   tagggagaaa aatgaattta gggagattgg acggcaaatt tgggtcgaaa aatggtgatt    1020
   ttgggtcaaa aatggacaat ttggggtcat tttagggata aaaatgaatt tagggagatt    1080
   tgagagcaaa tttgggtcta aaaatgtcaa tttggggtca aaaatggtga ttttgggtca    1140
   ttttagggat ataaatgaat tttgggagat ttgagggcaa atttgggtcg aaaaatggtg    1200
   atttggggtc aatttaggga ggaaaatgaa tttaaggtga tttgacggca aatttgggtt    1260
   gaaaaatgac aatttggggt cattttaaag ataaaatgaa tttagggcga tttgagggca    1320
   aatttgggtc gaagaatggt gatttgggtc aaaaaatgac aattttgggt cattttaggg    1380
   atataaatgg acttagagcg atttgagggc aaatttggtt gaaaaaatgg acaatttggg    1440
   tcattttagg gatataaatg aatttaggga gatttgatgg caaatttggg tcgaaaaatg    1500
   gtgatttggg tcaaaaatgg tgattttggt tgaaaaacag tcatttgggg tcattttagg    1560
   gattaaaatg aatttcggga gatttgaggg caaatttggg tcaaaaaatg gtgattttgg 1620
   gtcaattagt gataaaaaaa tgaatttagg gtgatttgag ggcaaatttg ggtcaaaaaa 1680
   tgacaatttg gggttttttt agggaggaga atgaatttag ggagttttga gggcaaattt 1740
   gggtcaaaaa atagtgattt gggtcaaaag tggtgatttt ggttgaaaaa cagtcatttt 1800
   gggtcatttt agggagaaaa atgaatttcg ggagatttga gggcaaattt gggtcaaaaa 1860
   atgacaattt tgggtcactt tacgaattaa aatgaattta gggagatttg agggcaaatt 1920
   tgggtcaaaa tatggtgatt ttgggtcaat ttagggagga aaatgaattt agggcgattt 1980
   gaaggcaaat ttgggtcgaa aatggtgatt ttgggtcaaa aaatgatgat tttgggtcgt 2040
   tttagggaga aaatgaattt agggagattt gagggcaaat ttgggtcaaa aaatggtgat 2100
   tttgggtcat tgtagggata aaaatgaatt tggggcgatt tgagggcaaa tttgggtgga 2160
   aaatggacaa ttttgggtca ttttagggag aaaaatgaat ttagggagat ttgagggcaa 2220
   atttgggtca aaaaatggtg atttggggtc attttcagaa gaaaaaatga ttattttccc 2280
   cactaaaaat gtatattttg gggccaaatg gtgaaaaatg gtgattttta atcaaacgtc 2340
   cccaaaattg gggaaatttc atcgatttga cccaaaattg agtttttttt ccctgttaaa 2400
   aatgtacatt ttggggtcaa tcgctgaaat gttcccactt ttcacttctt tgcccccaaa 2460
   ttttgctgtc cggtgagaaa ttaattaatt aatgatcatt aattgatcga caattaatgg 2520
   ttattaatta attaataggt cctcttttgg tgactccttc gcttttgggg ccaaaagtcc 2580
   ataaattggc cccaaaaaat taacgctgag taattggatt ccaaagtatt aatgataaac 2640
   attaaaagtg tttaattaat aatgatatta aacataattt cgtttttatt atcgatttat 2700
   caacaacgat gaacgataat actttacaac aatcgctaat aattaattaa ttatttaatt 2760
   aattaatttc taataattac ttcgcattat cggacacgag atgttgtaat tattaataat 2820
   ttaattccta ataattagaa gattcgttga aaatggtact tatctttaca aataatcact 2880
   tctaataata atgattaata atagttaata acaacaatga cgataatgac gataatatta 2940
   ataatatgtg atatatttaa tataaaattc gtattaatat attatatcta caaaatatga 3000
   tataaaatat aatattttat ttatatataa cacaatttat tattattatc attattatca 3060
   ttattaatat catcattatt aacgttatcg aaatacttat ttagaaataa taaaaacgga 3120
   tttaatgatg gcaacaaaaa tattttatta atgttaaaaa aaaataatta ataatttcca 3180
   aagattcgaa ttcggggcaa cgaacggcac tcgataattt ttaattaatt aatagtttga 3240
   attaatcggt actttttaat cctccatttt gcccgaaatc gccgtttttt gccccaaatt 3300
   ccccaccgcg gcgttaaaaa cataaagaaa ttaagcatca aaagtgccct tttttggggt 3360
   tgttttgacc caaaaaaaaa atggccgaat tgggggcggc cgttttacgg ttgggttcat 3420
   tttgggttca aaacagccaa aaatgggaac tttgggtttc gaaaacaaca acaacaaaaa 3480
   aacgggttta ttttgggctc attttgggtg tttttgggtc aggaggagaa aaaataggaa 3540
   gtttgagagc gaaacaacgg ccgcttttgg ggggaaaacg gccctttttg gtcaacggcg 3600
   ggggaaaaaa aaaagcggag tttttggggt gaaaaagagc ggttttgggt aaatttgggt 3660
   tttggggtaa aagtggagga ttttgggcaa tgggagttaa aaaatgggtg tttttatggg 3720
   ggttcggtgc agtttttcct gtttgatggg gggtttatta atccgggggg ggggattaat 3780
   gagaattaat aatgttaata gaaatatctg ggaaattaat agcaattatt aattgttaat 3840
   agttattaat agttctatat atctcacatc tacgatacaa tataatatcg ttacaatcat 3900
   atagtcgata tattacatat aattatcagt aataataata agtaacaata attagcagta 3960
   attaataata ataattaata gtattcgtta ataagattat tgataataat taagtagtag 4020
   cgattaatag agatgggatt tcgtgagaaa tggaccaaat ttgggccgtt ttgacccaaa 4080
   tttttggtgg ttttttttcc gattctttgt gaatttcggg tcggattcat cagcaattaa 4140
   ttacggttat taggggctat tagaggcttt taattgggat tattagagac ttttaagcgg 4200
   atttggggac ttttaagtgg attttatgat tcttaagtgg attttgggtg gattttaccg 4260
   cttttg 4266
<210> 38
   <211> 21
   <212> DNA
   <213> Artificial Sequence
<220>
   <223> Synthetic Primer
<400> 38
   ccaaaccacc tcctggtgtc c 21
<210> 39
   <211> 27
   <212> DNA
   <213> Artificial Sequence
<220>
   <223> Synthetic Primer
<400> 39
   caaaccaaaa ctacggattc tctgacc 27
<210> 40
   <211> 866
   <212> DNA
   <213> Gallus gallus
<400> 40
   caaaccacct cctggtgtcc cccaaccctt catagccccc caccccgtct cggtcgccca        60
   cccaaccctc cacattaacc ccttcctccc cacgctcgtc ccccgattcc cacccccctt       120
   ttcgtcacca tgcacccccc gtcccgcgag gacttcgaag gccccttccg caacgcctcc       180
   atcctctgcc agacccgcgg gcgccgccgt cccgccgagg tcacgtggta caaaaatggc       240
   agccccgtcg ccgccgccgc cgccaccacc gccaccaccg tcggccccga agtggtggcc       300
   gagagccgca tcagcgtcac cgaaagcgaa tgggacaccg gggccacctt cagctgcgtc       360
   gtggaggggg agatgaggaa caccagcaag aggatggagt gcggatgtaa gtggaggggg       420
   ggttttttgg ggggggattt ggggggaaat tgggggcttt gggggcaaaa agtccgtcat       480
   tttggtttgg aaggggtcga tgtgggggaa aatgtgccaa aaatcccaat tttgggggca       540
   ctgaagtcgt aatttgaggt ggaggaaggt agacagaaag agggtggagt gcggatgtaa       600
   gtggaggggg atttggggca gatttggggg aaattggggg tttggggggc aaataatccg       660
   tagtttcggt ttggaagggg ttgatgtggt tttgtgctga atttcggggg aaaaactgcc       720
   aaaaatccca attttggggg cattgaagtc atagtttgag gttggaagga ggtagagagc       780
   aagaggatga agtgaggatg taagtcgggg gatttttggg gcagatttgg gggaaattgg       840
   gggttttggg gtcagagaat ccgtag                                            866
<210> 41
   <211> 16786
   <212> DNA
   <213> Artificial Sequence
<220>
   <223> Targeting vector
<220>
   <221> misc_feature
   <222> (5126)..(5126)
   <223> n is a, c, g, or t
<400> 41
   gcgccctagc gcccgctcct ttcgctttct tcccttcctt tctcgccacg ttcgccggct        60
   ttccccgtca agctctaaat cgggggctcc ctttagggtt ccgatttagt gctttacggc       120
   acctcgaccc caaaaaactt gattagggtg atggttcacg tagtgggcca tcgccctgat       180
   agacggtttt tcgccctttg acgttggagt ccacgttctt taatagtgga ctcttgttcc       240
   aaactggaac aacactcaac cctatctcgg tctattcttt tgatttataa gggattttgc       300
   cgatttcggc ctattggtta aaaaatgagc tgatttaaca aaaatttaac gcgaatttta       360
   acaaaatatt aacgcttaca atttccattc gccattcagg ctgcgcaact gttgggaagg       420
   gcgatcgggt accataagaa tgcggccgct tcccgaagcg aaagccgcgt gttatttttg       480
   ggtgggaaag ccccgattcg ctcctttttt tttatttatt atttttgttg tttttcgtgt       540
   ctttttcttc tccgttttgc agctgcggcc tccaggtgtc cccggccgtg gtatttttgg       600
   gtcgaaaaag ccgctttttg gctcacttgg aaacctttcg ggcgtctcca agcggagcgt 660
   tggcgggaaa tggtgttttt tttgggttgt tttcggtccc ttttttcacc atttttgtgg 720
   tttttcggcc cattttcggc ctcccccccc ccccccccct tcccgtgggg ctgcgctccg 780
   tggagcggaa tggggtcgtt aattaaggtt aattggttta attaatgggc cgtgcaggga 840
   gaagagggcc gtgcggtggt tgttagccct gattaatggc ttggttgagg ggagagtgta 900
   attaagtgct tcgttagcac taattaatgg cttgattgag aggagagtgt aattaaatgc 960
   ttcataatcc ctaattaatg gcttggttga gacaagagtg taattaagtg cttcattagc 1020
   cctgattaat ggcttggttg aggggagagt gtaattaaat gcttcgttag cactaattaa 1080
   tggcttgatt gagaggagag tgtaattaaa tgcttcataa tccctaatta atggcttggt 1140
   tgagacaaga gtgtaattaa gtgcttcatt agccctgatt aatggcttgg ttgagaggag 1200
   agtgtaatta aatgcttcat tatccctaat tagggactga attccaagga gagtgtaatt 1260
   aagtgcttca ttatccctaa ttaatggctt ggttgagagg agagtgtaat taaatgcttt 1320
   gttagcccta attaatggtt tgatggagag gagagtgtaa ttaagtgctt cattatccct 1380
   aattaatggc ttggttgagg ggagagtgta attaaatgct tagttagccc taattaatgg 1440
   cttggttgag aggagagtgt aattgaatgc ttcattatcc ctaattaggg attgaattga 1500
   gaggagagtg taattgaatg ctttgttagc cctaattagg gattgaattg agaggagagt 1560
   gtaattaaat gcctcattat ccctaattag ggattgaatt gagaggagag tttaattaaa 1620
   tgcctcatta tccctaatta gggactgaat tccgacgaga gtgtaattaa gtgcttcatt 1680
   tagccctaat taatggcttg attgagagga gagtgtaatt aaatgcttca ttatccctaa 1740
   ttagggattg aattgagagg agagtgtaat taaatgcttc attatcccta attagggatt 1800
   gaattgagag gagagtgtaa ttaaatgctt cattatccct aattaatggc ttggttgaga 1860
   ggggagtgta attaagtgct tcgttatccc taattaggga ttgaattgtg aggagatggc 1920
   aattaatact tcattatccc taattaattt aattagtccc tattatccct aatttaattc 1980
   atggtttggt tgatggaaga atgtaattaa atgctttgtt atccctaatt aatggtttga 2040
   ttgagcggag agtgtaatta aatgcttcat tatcccttat taatggttgt gtggaatgta 2100
   gtgtaattag aagtccctaa ttaatggttt aattgagaga agactgcaat tagatgactc 2160
   atagccccta ataagtggtt taattatgag ataatcacaa ttacatgttt cattgtccct 2220
   aattaatcgt ttaattgcga gaagatcaca attaattaaa tgatccctaa tttcaatttc 2280
   tgagaagtgt ctttctcgga aattgagata tcataagaat tcgcgatcaa tttctgagaa 2340
   ctgtcattct cggaatacct gacgtcaatg ttggcgcaga agggattcat atcaattaat 2400
   tccctgtcct caattaatgc ataattggga gtgcgatgga atcattcaat aagtaatgag 2460
   gacttggtgt caaaatgcca attaagtaat taatgacacc caattaatac acaatgggga 2520
   ttacagtgga ataactcagt gaagttcata tctaaattgc aattgaataa ttaattacag 2580
   ctaattaatg gatgggagac tggcagtggt ataacttaat gaaggaacac agtgaattcc 2640
   tgtctgaatt gaaattaatt gatttattag ggctaattaa tgaatggtgg ggttaaaaat 2700
   agcattactc agcgaaggaa catgagttca tggctaaatt gagattgaat tattaattac 2760
   aactaattga ttaatgactg atgggagtaa gaatggaata actcagtgaa ggaacacaat 2820
   ggattcatgg cctaattgaa attaaatggt taattagagc taattaatga atgatggggg 2880
   gtggcaatgg aatgatacaa gaaagaaaca caatgggttc gtgtctacat tgaaattaaa 2940
   taattaatta cagctaatta ataaacggcg aggttgacaa tggtataact gtgaagagac 3000
   agaatgagtt cgtgactaaa ttggtattaa atgattaatt agagctaatt aacaggtggt 3060
   ggggctgaga atggcataac tcaatggggg atgatgagct gatggcttaa ttggaattaa 3120
   taattaatca caaccaatta acaggtgctg ttaatggggt tacaggggca tgacttcagg 3180
   aagaagcaca aagtggccat taaataatta attgcaccca attaattcct gatggggatt 3240
   actgcagagg aactcagcga aggattcaag cagaaatgac acagttaggt tgctcaattg 3300
   cgttaattaa ttaaaatcgc taattaggtg agcttcatta aggcctgatg gggttcatcc 3360
   caacgctggg atcaaagaac tcagcgcaga gtggataacg ataattagaa ggggacaaat 3420
   taatgaggaa acacgacttt gggcctagac ggccattaaa tcattaatca acccgacggg 3480
   cgcccaatta atcataaagt tattaagcaa ttaagtcgag gctgacgaga aaccgcaacg 3540
   agagccctcc acgctccttt gagttggcgc aaacaacaaa caacgcgggc tcgttaagca 3600
   gcgacgacgc cgagcgtaac gaagcaatcc ccgcgcccga caattaacgc gctaacgaag 3660
   ccggagccct ccttatgcaa attagcccct ccagagggcc ataaaagcgc cggctctccg 3720
   acggcgcggg aggagcacca gtcggctccg caaccatgag cccactcgtc tcctccctcc 3780
   tgctcctggc cgccctgcca ggtgagggcg ctgtggggct ctatggggct ctatggggtc 3840
   tcagcggggc tctgcgggct caatgggggc caaagggggg gtctgcgggc tctatggggg 3900
   ggtcaacggg gggtctcacg gggggccggc tccgcgaggc cgtgtggcgg cggctccgtc 3960
   agcgctctct gggcgcgcct ccttccccac agggctgatg gcggaggtgc agctgttgga 4020
   gtctggggga ggcttggtac agcctggggg gtccctgaga ctctcctgtg cagcctctgg 4080
   attcaccttt agcagctatg ccatgagctg ggtccgccag gctccaggga aggggctgga 4140
   gtgggtctca gctattagtg gtagtggtgg tagcacatac tacgcagact ccgtgaaggg 4200
   ccggttcacc atctccagag acaattccaa gaacacgctg tatctgcaaa tgaacagcct 4260
   gagagccgag gacacggccg tatattactg tgcgaaagga tatagtggct acgattacta 4320
   ctttgactac tggggccagg gcaccctggt caccgtctcc tcaggtgagt cttcatatga 4380
   acccccccca aaattgccgc ggcgattttg gggcaaaatc gggcgatttt atggatcctg 4440
   ggtcagtcga agggggcggt cggtccatca tttgcggccg ggtgattttt ggggccgaaa 4500
   agtgggaatt tggggcccaa tttggggccc aatttggggc ccaatttggg ttttcgaggg 4560
   gggatttttt tagggggaga tttttgggtc cccggaaaat ggtgawtttg ggtcaaaaat 4620
   ggggawtttg ggggttcktt ttraggtggg gttttttggg gkagaaatgg cggtctgagg 4680
   ggaaaatgtc gttttggggc cattttgggc catttgaggg gaaatttggg tcaaaaaatg 4740
   acgattttgg gtcgttttag ggaggaaaat gaatttaggg cgatttgagc caaatttggg 4800
   tcaaaaatgg cgattttgag tcaaaaaatg attattttgg gtcattttag ggaggaaaat 4860
   gaatttaggg agattggacg gcaaatttgg gtcaaaaaat ggtgattttg ggtcaaaaaa 4920
   tgattatttt gggtcatttt agggagaaaa atgaatttag ggagatttga gggcaaattt 4980
   gggtcaaaaa atggtgattt tgggtcaaaa aatgattatt ttgggtcatt ttagggagaa 5040
   aaatgaattt agggagattt gagggcaaat ttgggtcgaa aaatggcgat tttgggtgaa 5100
   aaatggacaa ttttgggtca ttttanggtt aaaaatgaat ttagggagat tggacggcaa 5160
   atttgggtcr aaaaatggtg atttggggts aaaaatgray wattttkggt cattttaggg 5220
   ataaaaaatg aatttagggc gatgtgagcc aattttgggt caaaaatggc gattgtgagt 5280
   caaaaaatga ttattttggg tcattttagg gttaaaaatg aatttaggga gattggacgg 5340
   caaatttggg tcaaaaaatg gtgattttcg gtcaaaaaat gattattttg ggtcatttta 5400
   gggagaaaaa tgaatttagg gagattggac ggcaaatttg ggtcgaaaaa tggtgatttt 5460
   gggtcaaaaa tggacaattt ggggtcattt tagggataaa aatgaattta gggagatttg 5520
   agagcaaatt tgggtctaaa aatgtcaatt tggggtcaaa aatggtgatt ttgggtcatt 5580
   ttagggatat aaatgaattt tgggagattt gagggcaaat ttgggtcgaa aaatggtgat 5640
   ttggggtcaa tttagggagg aaaatgaatt taaggtgatt tgacggcaaa tttgggttga 5700
   aaaatgacaa tttggggtca ttttaaagat aaaatgaatt tagggcgatt tgagggcaaa 5760
   tttgggtcga agaatggtga tttgggtcaa aaaatgacaa ttttgggtca ttttagggat 5820
   ataaatggac ttagagcgat ttgagggcaa atttggttga aaaaatggac aatttgggtc 5880
   attttaggga tataaatgaa tttagggaga tttgatggca aatttgggtc gaaaaatggt 5940
   gatttgggtc aaaaatggtg attttggttg aaaaacagtc atttggggtc attttaggga 6000
   ttaaaatgaa tttcgggaga tttgagggca aatttgggtc aaaaaatggt gattttgggt 6060
   caattagtga taaaaaaatg aatttagggt gatttgaggg caaatttggg tcaaaaaatg 6120
   acaatttggg gtttttttag ggaggagaat gaatttaggg agttttgagg gcaaatttgg 6180
   gtcaaaaaat agtgatttgg gtcaaaagtg gtgattttgg ttgaaaaaca gtcattttgg 6240
   gtcattttag ggagaaaaat gaatttcggg agatttgagg gcaaatttgg gtcaaaaaat 6300
   gacaattttg ggtcacttta cgaattaaaa tgaatttagg gagatttgag ggcaaatttg 6360
   ggtcaaaata tggtgatttt gggtcaattt agggaggaaa atgaatttag ggcgatttga 6420
   aggcaaattt gggtcgaaaa tggtgatttt gggtcaaaaa atgatgattt tgggtcgttt 6480
   tagggagaaa atgaatttag ggagatttga gggcaaattt gggtcaaaaa atggtgattt 6540
   tgggtcattg tagggataaa aatgaatttg gggcgatttg agggcaaatt tgggtggaaa 6600
   atggacaatt ttgggtcatt ttagggagaa aaatgaattt agggagattt gagggcaaat 6660
   ttgggtcaaa aaatggtgat ttggggtcat tttcagaaga aaaaatgatt attttcccca 6720
   ctaaaaatgt atattttggg gccaaatggt gaaaaatggt gatttttaat caaacgtccc 6780
   caaaattggg gaaatttcat cgatttgacc caaaattgag ttttttttcc ctgttaaaaa 6840
   tgtacatttt ggggtcaatc gctgaaatgt tcccactttt cacttctttg cccccaaatt 6900
   ttgctgtccg gtgagaaatt aattaattaa tgatcattaa ttgatcgaca attaatggtt 6960
   attaattaat taataggtcc tcttttggtg actccttcgc ttttggggcc aaaagtccat 7020
   aaattggccc caaaaaatta acgctgagta attggattcc aaagtattaa tgataaacat 7080
   taaaagtgtt taattaataa tgatattaaa cataatttcg tttttattat cgatttatca 7140
   acaacgatga acgataatac tttacaacaa tcgctaataa ttaattaatt atttaattaa 7200
   ttaatttcta ataattactt cgcattatcg gacacgagat gttgtaatta ttaataattt 7260
   aattcctaat aattagaaga ttcgttgaaa atggtactta tctttacaaa taatcacttc 7320
   taataataat gattaataat agttaataac aacaatgacg ataatgacga taatattaat 7380
   aatatgtgat atatttaata taaaattcgt attaatatat tatatctaca aaatatgata 7440
   taaaatataa tattttattt atatataaca caatttatta ttattatcat tattatcatt 7500
   attaatatca tcattattaa cgttatcgaa atacttattt agaaataata aaaacggatt 7560
   taatgatggc aacaaaaata ttttattaat gttaaaaaaa aataattaat aatttccaaa 7620
   gattcgaatt cggggcaacg aacggcactc gataattttt aattaattaa tagtttgaat 7680
   taatcggtac tttttaatcc tccattttgc ccgaaatcgc cgttttttgc cccaaattcc 7740
   ccaccgcggc gttaaaaaca taaagaaatt aagcatcaaa agtgcccttt tttggggttg 7800
   ttttgaccca aaaaaaaaat ggccgaattg ggggcggccg ttttacggtt gggttcattt 7860
   tgggttcaaa acagccaaaa atgggaactt tgggtttcga aaacaacaac aacaaaaaaa 7920
   cgggtttatt ttgggctcat tttgggtgtt tttgggtcag gaggagaaaa aataggaagt 7980
   ttgagagcga aacaacggcc gcttttgggg ggaaaacggc cctttttggt caacggcggg 8040
   ggaaaaaaaa aagcggagtt tttggggtga aaaagagcgg ttttgggtaa atttgggttt 8100
   tggggtaaaa gtggaggatt ttgggcaatg ggagttaaaa aatgggtgtt tttatggggg 8160
   ttcggtgcag tttttcctgt ttgatggggg gtttattaat ccgggggggg ggattaatga 8220
   gaattaataa tgttaataga aatatctggg aaattaatag caattattaa ttgttaatag 8280
   ttattaatag ttctatatat ctcacatcta cgatacaata taatatcgtt acaatcatat 8340
   agtcgatata ttacatataa ttatcagtaa taataataag taacaataat tagcagtaat 8400
   taataataat aattaatagt attcgttaat aagattattg ataataatta agtagtagcg 8460
   attaatagag atgggatttc gtgagaaatg gaccaaattt gggccgtttt gacccaaatt 8520
   tttggtggtt ttttttccga ttctttgtga atttcgggtc ggattcatca gcaattaatt 8580
   acggttatta ggggctatta gaggctttta attgggatta ttagagactt ttaagcggat 8640
   ttggggactt ttaagtggat tttatgattc ttaagtggat tttgggtgga ttttaccgct 8700
   tttgggccgc cgcccccctc taacccccca tctccccccc ttttctctct cccccccccc 8760
   ccccaaaccc cacagcctcc accaagggcc catcggtctt ccccctggca ccctcctcca 8820
   agagcacctc tgggggcaca gcggccctgg gctgcctggt caaggactac ttccccgaac 8880
   cggtgacggt gtcgtggaac tcaggcgccc tgaccagcgg cgtgcacacc ttcccggctg 8940
   tcctacagtc ctcaggactc tactccctca gcagcgtggt gaccgtgccc tccagcagct 9000
   tgggcaccca gacctacatc tgcaacgtga atcacaagcc cagcaacacc aaggtggaca 9060
   agagagttga gcccaaatct tgtgacaaaa ctcacacatg cccaccgtgc ccaggtaagt 9120
   cactagacca gagctccact cccgggagaa tggtaagtgc tgtaaacatc cctgcactag 9180
   aggataagcc atgtacagat ccatttccat ctctcctcat cagcacctga actcctgggg 9240
   ggaccgtcag tcttcctctt ccccccaaaa cccaaggaca ccctcatgat ctcccggacc 9300
   cctgaggtca catgcgtggt ggtggacgtg agccacgaag accctgaggt caagttcaac 9360
   tggtacgtgg acggcgtgga ggtgcataat gccaagacaa agccgcggga ggagcagtac 9420
   aacagcacgt accgtgtggt cagcgtcctc accgtcctgc accaggactg gctgaatggc 9480
   aaggagtaca agtgcaaggt ctccaacaaa gccctcccag cccccatcga gaaaaccatc 9540
   tccaaagcca aaggtgagag ctgcagcctg actgcatggg ggctgggatg ggcataagga 9600
   taaaggtctg tgtggacagc cttctgcttc agccatgacc tttgtgtatg tttctaccct 9660
   cacagggcag ccccgagaac cacaggtgta caccctgccc ccatcccggg aggagatgac 9720
   caagaaccag gtcagcctga cctgcctggt caaaggcttc tatcccagcg acatcgccgt 9780
   ggagtgggag agcaatgggc agccggagaa caactacaag accacgcctc ccgtgctgga 9840
   ctccgacggc tccttcttcc tctatagcaa gctcaccgtg gacaagagca ggtggcagca 9900
   ggggaacgtc ttctcatgct ccgtgatgca tgaggctctg cacaaccact acacgcagaa 9960
   gagcctctcc ctgtccccgg gtaaatgagc tcagcaccca caaaactctc aggtccaaag 10020
   agacacccac actcatctcc atgcttccct tgtataaata aagcacccag caatgcctgg 10080
   gaccatgtaa aactgtcctg gttctttcca aggtatagag catagctcac aggctgatat 10140
   ttctggccag ggttggagga cagccttgtc tataggaaga gaatgaggtt tttgcactgc 10200
   aggactcaga gctcattagt tatcctgcct tggagtgttg gggcttggct ttaggcagtg 10260
   ccttttcctt gccttcctac gaaccagcag ctgccataca tagagataat cctaggaagc 10320
   ctcaaatgga gaaggacaca aacccacctc cctcaggctg ttcctctatc ccggccccac 10380
   ttctttacct aggggtttct ctgagtctat tgtggagtta cacatggcca ggggcattcc 10440
   agagaccctt gtcatccata cactcaactc aggcagcttt gcacaaacaa agtctgcaca 10500
   cccatacaga tggctcactc ttgcctgtgc catgtagggc tgaggcacat ggctcttgct 10560
   gccccaaggg agggactatt agatagccac actcatgctg aatcctggcc cattcaaatt 10620
   agcctgctga acaccatcca gtccatatag cacatgtatc cacatgcacg tgtgcacaaa 10680
   acgcatttaa cacactggga caacaattct gtgccctgca cagcacctat atccagcaat 10740
   gtatcaccat acacacgacc aaaaattcaa tgcccacgtt tctgccatca caaacagaca 10800
   catctttcct ctctgtggcc actgcattat atgctcaaca caagacctct gaagccagat 10860
   ccatctctgg cacctcgggg tcatgcttca accccacatg aattatgcaa accatagcca 10920
   taatggtctg aatcacttca cactgggatg ttcccaagtt caggcaagac gagccacagg 10980
   ctctgctgat gactgaagga cagcaaaggg tcagtccagc tgtatagcca ctgttgacct 11040
   gggtcacagg ccctgctgac cctccacctt ctcctgtact gaaggaatga aagatgagac 11100
   aagcatagag ggcacttgaa taatccaggt cactctgagg tccacccaag gcattattgg 11160
   actcaggtgg gaagctgaga ctggtgtccc agagggaaag gaaggaaagc aggccccggg 11220
   gagggtctgc tgtcccagtc aggctggaga tctctcctct gaatccatgc agacatgtct 11280
   gcctcacagg gaatctctcc cagcaccaac catgttggga caaacactga ctgtcctctc 11340
   tgttcagagc tgcaactgga ggagagctgt gcggaggcgc aggacgggga gctggacggg 11400
   ctgtggacga ccatcaccat cttcatcaca ctcttcctgt taagcgtgtg ctacagtgcc 11460
   accgtcacct tcttcaaggt gaagtggatc ttctcctcgg tggtggacct gaagcagacc 11520
   atcatccccg actacaggaa catgatcgga cagggggcct aggaattaat tcgctgtctg 11580
   cgagggccag ctgttggggt gagtactccc tctcaaaagc gggcatgact tctgcgctaa 11640
   gattgtcagt ttccaaaaac gaggaggatt tgatattcac ctggcccgcg gtgatgcctt 11700
   tgagggtggc cgcgtccatc tggtcagaaa agacaatctt tttgttgtca agcttgaggt 11760
   gtggcaggct tgagatctgg ccatacactt gagtgacaat gacatccact ttgcctttct 11820
   ctccacaggt gtccactccc aggtccaact gcaggtcgag catgcatcta gggcggccaa 11880
   ttggatccta ccgttcgtat aatgtatgct atacgaagtt atcgtacgct taattaactg 11940
   atccggaatt ccgccccccc cccccccccc cctctccctc ccccccccct aacgttactg 12000
   gccgaagccg cttggaataa ggccggtgtg cgtttgtcta tatgttattt tccaccatat 12060
   tgccgtcttt tggcaatgtg agggcccgga aacctggccc tgtcttcttg acgagcattc 12120
   ctaggggtct ttcccctctc gccaaaggaa tgcaaggtct gttgaatgtc gtgaaggaag 12180
   cagttcctct ggaagcttct tgaagacaaa caacgtctgt agcgaccctt tgcaggcagc 12240
   ggaacccccc acctggcgac aggtgcctct gcggccaaaa gccacgtgta taagatacac 12300
   ctgcaaaggc ggcacaaccc cagtgccacg ttgtgagttg gatagttgtg gaaagagtca 12360
   aatggctctc ctcaagcgta ttcaacaagg ggctgaagga tgcccagaag gtaccccatt 12420
   gtatgggatc tgatctgggg cctcggtgca catgctttac atgtgtttag tcgaggttaa 12480
   aaaaacgtct aggccccccg aaccacgggg acgtggtttt cctttgaaaa acacgatgat 12540
   aagcttgcca caaccatggc tgaacaagat ggattgcacg caggttctcc ggccgcttgg 12600
   gtggagaggc tattcggcta tgactgggca caacagacaa tcggctgctc tgatgccgcc 12660
   gtgttccggc tgtcagcgca ggggcgcccg gttctttttg tcaagaccga cctgtccggt 12720
   gccctgaatg aactgcagga cgaggcagcg cggctatcgt ggctggccac gacgggcgtt 12780
   ccttgcgcag ctgtgctcga cgttgtcact gaagcgggaa gggactggct gctattgggc 12840
   gaagtgccgg ggcaggatct cctgtcatct caccttgctc ctgccgagaa agtatccatc 12900
   atggctgatg caatgcggcg gctgcatacg cttgatccgg ctacctgccc attcgaccac 12960
   caagcgaaac atcgcatcga gcgagcacgt actcggatgg aagccggtct tgtcgatcag 13020
   gatgatctgg acgaagagca tcaggggctc gcgccagccg aactgttcgc caggctcaag 13080
   gcgcgcatgc ccgacggcga ggatctcgtc gtgacccatg gcgatgcctg cttgccgaat 13140
   atcatggtgg aaaatggccg cttttctgga ttcatcgact gtggccggct gggtgtggcg 13200
   gaccgctatc aggacatagc gttggctacc cgtgatattg ctgaagagct tggcggcgaa 13260
   tgggctgacc gcttcctcgt gctttacggt atcgccgctc ccgattcgca gcgcatcgcc 13320
   ttctatcgcc ttcttgacga gttcttctga ataacttcgt ataatgtatg ctatacgaac 13380
   ggtaggatcc cgcccgcccc acgacccgca gcgcccgacc gaaaggagcg cacgacccca 13440
   tggctccgac cgaagccacc cggggcggcc ccgccgaccc cgcacccgcc cccgaggccc 13500
   accgactcta gataactgat cataatcagc cataccacat ttgtagaggt tttacttgct 13560
   ttaaaaaacc tcccacacct ccccctgaac ctgaaacata aaatgaatgc aattgttgtt 13620
   gttaacttgt ttattgcagc ttataatggt tacaaataaa gcaatagcat cacaaatttc 13680
   acaaataaag catttttttc actgtatgca aaccacctcc tggtgtcccc caacccttca 13740
   tagcccccca ccccgtctcg gtcgcccacc caaccctcca cattaacccc ttcctcccca 13800
   cgctcgtccc ccgattccca cccccctttt cgtcaccatg caccccccgt cccgcgagga 13860
   cttcgaaggc cccttccgca acgcctccat cctctgccag acccgcgggc gccgccgtcc 13920
   cgccgaggtc acgtggtaca aaaatggcag ccccgtcgcc gccgccgccg ccaccaccgc 13980
   caccaccgtc ggccccgaag tggtggccga gagccgcatc agcgtcaccg aaagcgaatg 14040
   ggacaccggg gccaccttca gctgcgtcgt ggagggggag atgaggaaca ccagcaagag 14100
   gatggagtgc ggatgtaagt ggaggggggg ttttttgggg ggggatttgg ggggaaattg 14160
   ggggctttgg gggcaaaaag tccgtcattt tggtttggaa ggggtcgatg tgggggaaaa 14220
   tgtgccaaaa atcccaattt tgggggcact gaagtcgtaa tttgaggtgg aggaaggtag 14280
   acagaaagag ggtggagtgc ggatgtaagt ggagggggat ttggggcaga tttgggggaa 14340
   attgggggtt tggggggcaa ataatccgta gtttcggttt ggaaggggtt gatgtggttt 14400
   tgtgctgaat ttcgggggaa aaactgccaa aaatcccaat tttgggggca ttgaagtcat 14460
   agtttgaggt tggaaggagg tagagagcaa gaggatgaag tgaggatgta agtcggggga 14520
   tttttggggc agatttgggg gaaattgggg gttttggggt cagagaatcc gtaggtcgac 14580
   cgcgccagct tttgttccct ttagtgaggg ttaattgcgc gcttggcgta atcatggtca 14640
   tagctgtttc ctgtgtgaaa ttgttatccg ctcacaattc cacacaacat acgagccgga 14700
   agcataaagt gtaaagcctg gggtgcctaa tgagtgagct aactcacatt aattgcgttg 14760
   cgctcactgc ccgctttcca gtcgggaaac ctgtcgtgcc agctgcatta atgaatcggc 14820
   caacgcgcgg ggagaggcgg tttgcgtatt gggcgctctt ccgcttcctc gctcactgac 14880
   tcgctgcgct cggtcgttcg gctgcggcga gcggtatcag ctcactcaaa ggcggtaata 14940
   cggttatcca cagaatcagg ggataacgca ggaaagaaca tgtgagcaaa aggccagcaa 15000
   aaggccagga accgtaaaaa ggccgcgttg ctggcgtttt tccataggct ccgcccccct 15060
   gacgagcatc acaaaaatcg acgctcaagt cagaggtggc gaaacccgac aggactataa 15120
   agataccagg cgtttccccc tggaagctcc ctcgtgcgct ctcctgttcc gaccctgccg 15180
   cttaccggat acctgtccgc ctttctccct tcgggaagcg tggcgctttc tcatagctca 15240
   cgctgtaggt atctcagttc ggtgtaggtc gttcgctcca agctgggctg tgtgcacgaa 15300
   ccccccgttc agcccgaccg ctgcgcctta tccggtaact atcgtcttga gtccaacccg 15360
   gtaagacacg acttatcgcc actggcagca gccactggta acaggattag cagagcgagg 15420
   tatgtaggcg gtgctacaga gttcttgaag tggtggccta actacggcta cactagaagg 15480
   acagtatttg gtatctgcgc tctgctgaag ccagttacct tcggaaaaag agttggtagc 15540
   tcttgatccg gcaaacaaac caccgctggt agcggtggtt tttttgtttg caagcagcag 15600
   attacgcgca gaaaaaaagg atctcaagaa gatcctttga tcttttctac ggggtctgac 15660
   gctcagtgga acgaaaactc acgttaaggg attttggtca tgagattatc aaaaaggatc 15720
   ttcacctaga tccttttaaa ttaaaaatga agttttaaat caatctaaag tatatatgag 15780
   taaacttggt ctgacagtta ccaatgctta atcagtgagg cacctatctc agcgatctgt 15840
   ctatttcgtt catccatagt tgcctgactc cccgtcgtgt agataactac gatacgggag 15900
   ggcttaccat ctggccccag tgctgcaatg ataccgcgag acccacgctc accggctcca 15960
   gatttatcag caataaacca gccagccgga agggccgagc gcagaagtgg tcctgcaact 16020
   ttatccgcct ccatccagtc tattaattgt tgccgggaag ctagagtaag tagttcgcca 16080
   gttaatagtt tgcgcaacgt tgttgccatt gctacaggca tcgtggtgtc acgctcgtcg 16140
   tttggtatgg cttcattcag ctccggttcc caacgatcaa ggcgagttac atgatccccc 16200
   atgttgtgca aaaaagcggt tagctccttc ggtcctccga tcgttgtcag aagtaagttg 16260
   gccgcagtgt tatcactcat ggttatggca gcactgcata attctcttac tgtcatgcca 16320
   tccgtaagat gcttttctgt gactggtgag tactcaacca agtcattctg agaatagtgt 16380
   atgcggcgac cgagttgctc ttgcccggcg tcaatacggg ataataccgc gccacatagc 16440
   agaactttaa aagtgctcat cattggaaaa cgttcttcgg ggcgaaaact ctcaaggatc 16500
   ttaccgctgt tgagatccag ttcgatgtaa cccactcgtg cacccaactg atcttcagca 16560
   tcttttactt tcaccagcgt ttctgggtga gcaaaaacag gaaggcaaaa tgccgcaaaa 16620
   aagggaataa gggcgacacg gaaatgttga atactcatac tcttcctttt tcaatattat 16680
   tgaagcattt atcagggtta ttgtctcatg agcggataca tatttgaatg tatttagaaa 16740
   aataaacaaa taggggttcc gcgcacattt ccccgaaaag tgccac 16786
<210> 42
   <211> 814
   <212> DNA
   <213> Artificial Sequence
<220>
   <223> Synthetic DNA
<400> 42
   tgaggagacg gtgaccaggg tgccctggcc ccagtagtca aagtagtaat cgtaagccac 60
   tatatccttt cgcacagtaa tatacggccg tgtcctcggc tctcaggctg ttcatttgca 120
   gatacagcgt gttcttggaa ttgtctctgg agatggtgaa ccggcccttc acggagtctg 180
   cgtagtatgt tgctaccacc actaccacta atagctgaga cccactccag ccccttccct 240
   ggagcctggc ggacccagct catggcatat gctgctaaag gtgaatccag aggctgcaca 300
   ggagagtctc agggaccccc caggctgtac caagcctccc ccagactcca acagctgcac 360
   ctcgtgcctg gtgactcttc cgcaatcagt cctgggacag aaacctcagg gacagccttt 420
   atgagttttg atcacatcta acagagagga tgggccagtg aggagacggt gaccagggtg 480
   ccctggcccc agtagtcaaa gtagtaatcg tgccactata tcctttcgca cagtaatata 540
   cggccgtgtc ctcggctctc aggctgttca tttgcagata cagcgtgttc ttggaattgt 600
   ctctggagat ggtgaaccgg cccttcacgg agtctgcgta gtatgtgcta ccaccaacca 660
   ctaatagctg agacccactc cagccccttc cctggagcct ggcggaccca gctcatggca 720
   tgctgctaaa ggtgaatcca gaggctgcac aggagagtct cagggacccc ccaggctgta 780
   ccaagcctcc cccagactcc aacagctgca cctc 814
<210> 43
   <211> 17606
   <212> DNA
   <213> Artificial Sequence
<220>
   <223> Targeting vector
<220>
   <221> misc_feature
   <222> (5946)..(5946)
   <223> n is a, c, g, or t
<400> 43
   gcgccctagc gcccgctcct ttcgctttct tcccttcctt tctcgccacg ttcgccggct        60
   ttccccgtca agctctaaat cgggggctcc ctttagggtt ccgatttagt gctttacggc       120
   acctcgaccc caaaaaactt gattagggtg atggttcacg tagtgggcca tcgccctgat       180
   agacggtttt tcgccctttg acgttggagt ccacgttctt taatagtgga ctcttgttcc       240
   aaactggaac aacactcaac cctatctcgg tctattcttt tgatttataa gggattttgc       300
   cgatttcggc ctattggtta aaaaatgagc tgatttaaca aaaatttaac gcgaatttta       360
   acaaaatatt aacgcttaca atttccattc gccattcagg ctgcgcaact gttgggaagg       420
   gcgatcgggt accataagaa tgcggccgct tcccgaagcg aaagccgcgt gttatttttg       480
   ggtgggaaag ccccgattcg ctcctttttt tttatttatt atttttgttg tttttcgtgt       540
   ctttttcttc tccgttttgc agctgcggcc tccaggtgtc cccggccgtg gtatttttgg       600
   gtcgaaaaag ccgctttttg gctcacttgg aaacctttcg ggcgtctcca agcggagcgt       660
   tggcgggaaa tggtgttttt tttgggttgt tttcggtccc ttttttcacc atttttgtgg       720
   tttttcggcc cattttcggc ctcccccccc ccccccccct tcccgtgggg ctgcgctccg       780
   tggagcggaa tggggtcgtt aattaaggtt aattggttta attaatgggc cgtgcaggga       840
   gaagagggcc gtgcggtggt tgttagccct gattaatggc ttggttgagg ggagagtgta       900
   attaagtgct tcgttagcac taattaatgg cttgattgag aggagagtgt aattaaatgc       960
   ttcataatcc ctaattaatg gcttggttga gacaagagtg taattaagtg cttcattagc      1020
   cctgattaat ggcttggttg aggggagagt gtaattaaat gcttcgttag cactaattaa      1080
   tggcttgatt gagaggagag tgtaattaaa tgcttcataa tccctaatta atggcttggt 1140
   tgagacaaga gtgtaattaa gtgcttcatt agccctgatt aatggcttgg ttgagaggag 1200
   agtgtaatta aatgcttcat tatccctaat tagggactga attccaagga gagtgtaatt 1260
   aagtgcttca ttatccctaa ttaatggctt ggttgagagg agagtgtaat taaatgcttt 1320
   gttagcccta attaatggtt tgatggagag gagagtgtaa ttaagtgctt cattatccct 1380
   aattaatggc ttggttgagg ggagagtgta attaaatgct tagttagccc taattaatgg 1440
   cttggttgag aggagagtgt aattgaatgc ttcattatcc ctaattaggg attgaattga 1500
   gaggagagtg taattgaatg ctttgttagc cctaattagg gattgaattg agaggagagt 1560
   gtaattaaat gcctcattat ccctaattag ggattgaatt gagaggagag tttaattaaa 1620
   tgcctcatta tccctaatta gggactgaat tccgacgaga gtgtaattaa gtgcttcatt 1680
   tagccctaat taatggcttg attgagagga gagtgtaatt aaatgcttca ttatccctaa 1740
   ttagggattg aattgagagg agagtgtaat taaatgcttc attatcccta attagggatt 1800
   gaattgagag gagagtgtaa ttaaatgctt cattatccct aattaatggc ttggttgaga 1860
   ggggagtgta attaagtgct tcgttatccc taattaggga ttgaattgtg aggagatggc 1920
   aattaatact tcattatccc taattaattt aattagtccc tattatccct aatttaattc 1980
   atggtttggt tgatggaaga atgtaattaa atgctttgtt atccctaatt aatggtttga 2040
   ttgagcggag agtgtaatta aatgcttcat tatcccttat taatggttgt gtggaatgta 2100
   gtgtaattag aagtccctaa ttaatggttt aattgagaga agactgcaat tagatgactc 2160
   atagccccta ataagtggtt taattatgag ataatcacaa ttacatgttt cattgtccct 2220
   aattaatcgt ttaattgcga gaagatcaca attaattaaa tgatccctaa tttcaatttc 2280
   tgagaagtgt ctttctcgga aattgagata tcataagaat tcgcgatcaa tttctgagaa 2340
   ctgtcattct cggaatacct gacgtctgag gagacggtga ccagggtgcc ctggccccag 2400
   tagtcaaagt agtaatcgta agccactata tcctttcgca cagtaatata cggccgtgtc 2460
   ctcggctctc aggctgttca tttgcagata cagcgtgttc ttggaattgt ctctggagat 2520
   ggtgaaccgg cccttcacgg agtctgcgta gtatgttgct accaccacta ccactaatag 2580
   ctgagaccca ctccagcccc ttccctggag cctggcggac ccagctcatg gcatatgctg 2640
   ctaaaggtga atccagaggc tgcacaggag agtctcaggg accccccagg ctgtaccaag 2700
   cctcccccag actccaacag ctgcacctcg tgcctggtga ctcttccgca atcagtcctg 2760
   ggacagaaac ctcagggaca gcctttatga gttttgatca catctaacag agaggatggg 2820
   ccagtgagga gacggtgacc agggtgccct ggccccagta gtcaaagtag taatcgtgcc 2880
   actatatcct ttcgcacagt aatatacggc cgtgtcctcg gctctcaggc tgttcatttg 2940
   cagatacagc gtgttcttgg aattgtctct ggagatggtg aaccggccct tcacggagtc 3000
   tgcgtagtat gtgctaccac caaccactaa tagctgagac ccactccagc cccttccctg 3060
   gagcctggcg gacccagctc atggcatgct gctaaaggtg aatccagagg ctgcacagga 3120
   gagtctcagg gaccccccag gctgtaccaa gcctccccca gactccaaca gctgcacctc 3180
   gacgtcaatg ttggcgcaga agggattcat atcaattaat tccctgtcct caattaatgc 3240
   ataattggga gtgcgatgga atcattcaat aagtaatgag gacttggtgt caaaatgcca 3300
   attaagtaat taatgacacc caattaatac acaatgggga ttacagtgga ataactcagt 3360
   gaagttcata tctaaattgc aattgaataa ttaattacag ctaattaatg gatgggagac 3420
   tggcagtggt ataacttaat gaaggaacac agtgaattcc tgtctgaatt gaaattaatt 3480
   gatttattag ggctaattaa tgaatggtgg ggttaaaaat agcattactc agcgaaggaa 3540
   catgagttca tggctaaatt gagattgaat tattaattac aactaattga ttaatgactg 3600
   atgggagtaa gaatggaata actcagtgaa ggaacacaat ggattcatgg cctaattgaa 3660
   attaaatggt taattagagc taattaatga atgatggggg gtggcaatgg aatgatacaa 3720
   gaaagaaaca caatgggttc gtgtctacat tgaaattaaa taattaatta cagctaatta 3780
   ataaacggcg aggttgacaa tggtataact gtgaagagac agaatgagtt cgtgactaaa 3840
   ttggtattaa atgattaatt agagctaatt aacaggtggt ggggctgaga atggcataac 3900
   tcaatggggg atgatgagct gatggcttaa ttggaattaa taattaatca caaccaatta 3960
   acaggtgctg ttaatggggt tacaggggca tgacttcagg aagaagcaca aagtggccat 4020
   taaataatta attgcaccca attaattcct gatggggatt actgcagagg aactcagcga 4080
   aggattcaag cagaaatgac acagttaggt tgctcaattg cgttaattaa ttaaaatcgc 4140
   taattaggtg agcttcatta aggcctgatg gggttcatcc caacgctggg atcaaagaac 4200
   tcagcgcaga gtggataacg ataattagaa ggggacaaat taatgaggaa acacgacttt 4260
   gggcctagac ggccattaaa tcattaatca acccgacggg cgcccaatta atcataaagt 4320
   tattaagcaa ttaagtcgag gctgacgaga aaccgcaacg agagccctcc acgctccttt 4380
   gagttggcgc aaacaacaaa caacgcgggc tcgttaagca gcgacgacgc cgagcgtaac 4440
   gaagcaatcc ccgcgcccga caattaacgc gctaacgaag ccggagccct ccttatgcaa 4500
   attagcccct ccagagggcc ataaaagcgc cggctctccg acggcgcggg aggagcacca 4560
   gtcggctccg caaccatgag cccactcgtc tcctccctcc tgctcctggc cgccctgcca 4620
   ggtgagggcg ctgtggggct ctatggggct ctatggggtc tcagcggggc tctgcgggct 4680
   caatgggggc caaagggggg gtctgcgggc tctatggggg ggtcaacggg gggtctcacg 4740
   gggggccggc tccgcgaggc cgtgtggcgg cggctccgtc agcgctctct gggcgcgcct 4800
   ccttccccac agggctgatg gcggaggtgc agctgttgga gtctggggga ggcttggtac 4860
   agcctggggg gtccctgaga ctctcctgtg cagcctctgg attcaccttt agcagctatg 4920
   ccatgagctg ggtccgccag gctccaggga aggggctgga gtgggtctca gctattagtg 4980
   gtagtggtgg tagcacatac tacgcagact ccgtgaaggg ccggttcacc atctccagag 5040
   acaattccaa gaacacgctg tatctgcaaa tgaacagcct gagagccgag gacacggccg 5100
   tatattactg tgcgaaagga tatagtggct acgattacta ctttgactac tggggccagg 5160
   gcaccctggt caccgtctcc tcaggtgagt cttcatatga acccccccca aaattgccgc 5220
   ggcgattttg gggcaaaatc gggcgatttt atggatcctg ggtcagtcga agggggcggt 5280
   cggtccatca tttgcggccg ggtgattttt ggggccgaaa agtgggaatt tggggcccaa 5340
   tttggggccc aatttggggc ccaatttggg ttttcgaggg gggatttttt tagggggaga 5400
   tttttgggtc cccggaaaat ggtgawtttg ggtcaaaaat ggggawtttg ggggttcktt 5460
   ttraggtggg gttttttggg gkagaaatgg cggtctgagg ggaaaatgtc gttttggggc 5520
   cattttgggc catttgaggg gaaatttggg tcaaaaaatg acgattttgg gtcgttttag 5580
   ggaggaaaat gaatttaggg cgatttgagc caaatttggg tcaaaaatgg cgattttgag 5640
   tcaaaaaatg attattttgg gtcattttag ggaggaaaat gaatttaggg agattggacg 5700
   gcaaatttgg gtcaaaaaat ggtgattttg ggtcaaaaaa tgattatttt gggtcatttt 5760
   agggagaaaa atgaatttag ggagatttga gggcaaattt gggtcaaaaa atggtgattt 5820
   tgggtcaaaa aatgattatt ttgggtcatt ttagggagaa aaatgaattt agggagattt 5880
   gagggcaaat ttgggtcgaa aaatggcgat tttgggtgaa aaatggacaa ttttgggtca 5940
   ttttanggtt aaaaatgaat ttagggagat tggacggcaa atttgggtcr aaaaatggtg 6000
   atttggggts aaaaatgray wattttkggt cattttaggg ataaaaaatg aatttagggc 6060
   gatgtgagcc aattttgggt caaaaatggc gattgtgagt caaaaaatga ttattttggg 6120
   tcattttagg gttaaaaatg aatttaggga gattggacgg caaatttggg tcaaaaaatg 6180
   gtgattttcg gtcaaaaaat gattattttg ggtcatttta gggagaaaaa tgaatttagg 6240
   gagattggac ggcaaatttg ggtcgaaaaa tggtgatttt gggtcaaaaa tggacaattt 6300
   ggggtcattt tagggataaa aatgaattta gggagatttg agagcaaatt tgggtctaaa 6360
   aatgtcaatt tggggtcaaa aatggtgatt ttgggtcatt ttagggatat aaatgaattt 6420
   tgggagattt gagggcaaat ttgggtcgaa aaatggtgat ttggggtcaa tttagggagg 6480
   aaaatgaatt taaggtgatt tgacggcaaa tttgggttga aaaatgacaa tttggggtca 6540
   ttttaaagat aaaatgaatt tagggcgatt tgagggcaaa tttgggtcga agaatggtga 6600
   tttgggtcaa aaaatgacaa ttttgggtca ttttagggat ataaatggac ttagagcgat 6660
   ttgagggcaa atttggttga aaaaatggac aatttgggtc attttaggga tataaatgaa 6720
   tttagggaga tttgatggca aatttgggtc gaaaaatggt gatttgggtc aaaaatggtg 6780
   attttggttg aaaaacagtc atttggggtc attttaggga ttaaaatgaa tttcgggaga 6840
   tttgagggca aatttgggtc aaaaaatggt gattttgggt caattagtga taaaaaaatg 6900
   aatttagggt gatttgaggg caaatttggg tcaaaaaatg acaatttggg gtttttttag 6960
   ggaggagaat gaatttaggg agttttgagg gcaaatttgg gtcaaaaaat agtgatttgg 7020
   gtcaaaagtg gtgattttgg ttgaaaaaca gtcattttgg gtcattttag ggagaaaaat 7080
   gaatttcggg agatttgagg gcaaatttgg gtcaaaaaat gacaattttg ggtcacttta 7140
   cgaattaaaa tgaatttagg gagatttgag ggcaaatttg ggtcaaaata tggtgatttt 7200
   gggtcaattt agggaggaaa atgaatttag ggcgatttga aggcaaattt gggtcgaaaa 7260
   tggtgatttt gggtcaaaaa atgatgattt tgggtcgttt tagggagaaa atgaatttag 7320
   ggagatttga gggcaaattt gggtcaaaaa atggtgattt tgggtcattg tagggataaa 7380
   aatgaatttg gggcgatttg agggcaaatt tgggtggaaa atggacaatt ttgggtcatt 7440
   ttagggagaa aaatgaattt agggagattt gagggcaaat ttgggtcaaa aaatggtgat 7500
   ttggggtcat tttcagaaga aaaaatgatt attttcccca ctaaaaatgt atattttggg 7560
   gccaaatggt gaaaaatggt gatttttaat caaacgtccc caaaattggg gaaatttcat 7620
   cgatttgacc caaaattgag ttttttttcc ctgttaaaaa tgtacatttt ggggtcaatc 7680
   gctgaaatgt tcccactttt cacttctttg cccccaaatt ttgctgtccg gtgagaaatt 7740
   aattaattaa tgatcattaa ttgatcgaca attaatggtt attaattaat taataggtcc 7800
   tcttttggtg actccttcgc ttttggggcc aaaagtccat aaattggccc caaaaaatta 7860
   acgctgagta attggattcc aaagtattaa tgataaacat taaaagtgtt taattaataa 7920
   tgatattaaa cataatttcg tttttattat cgatttatca acaacgatga acgataatac 7980
   tttacaacaa tcgctaataa ttaattaatt atttaattaa ttaatttcta ataattactt 8040
   cgcattatcg gacacgagat gttgtaatta ttaataattt aattcctaat aattagaaga 8100
   ttcgttgaaa atggtactta tctttacaaa taatcacttc taataataat gattaataat 8160
   agttaataac aacaatgacg ataatgacga taatattaat aatatgtgat atatttaata 8220
   taaaattcgt attaatatat tatatctaca aaatatgata taaaatataa tattttattt 8280
   atatataaca caatttatta ttattatcat tattatcatt attaatatca tcattattaa 8340
   cgttatcgaa atacttattt agaaataata aaaacggatt taatgatggc aacaaaaata 8400
   ttttattaat gttaaaaaaa aataattaat aatttccaaa gattcgaatt cggggcaacg 8460
   aacggcactc gataattttt aattaattaa tagtttgaat taatcggtac tttttaatcc 8520
   tccattttgc ccgaaatcgc cgttttttgc cccaaattcc ccaccgcggc gttaaaaaca 8580
   taaagaaatt aagcatcaaa agtgcccttt tttggggttg ttttgaccca aaaaaaaaat 8640
   ggccgaattg ggggcggccg ttttacggtt gggttcattt tgggttcaaa acagccaaaa 8700
   atgggaactt tgggtttcga aaacaacaac aacaaaaaaa cgggtttatt ttgggctcat 8760
   tttgggtgtt tttgggtcag gaggagaaaa aataggaagt ttgagagcga aacaacggcc 8820
   gcttttgggg ggaaaacggc cctttttggt caacggcggg ggaaaaaaaa aagcggagtt 8880
   tttggggtga aaaagagcgg ttttgggtaa atttgggttt tggggtaaaa gtggaggatt 8940
   ttgggcaatg ggagttaaaa aatgggtgtt tttatggggg ttcggtgcag tttttcctgt 9000
   ttgatggggg gtttattaat ccgggggggg ggattaatga gaattaataa tgttaataga 9060
   aatatctggg aaattaatag caattattaa ttgttaatag ttattaatag ttctatatat 9120
   ctcacatcta cgatacaata taatatcgtt acaatcatat agtcgatata ttacatataa 9180
   ttatcagtaa taataataag taacaataat tagcagtaat taataataat aattaatagt 9240
   attcgttaat aagattattg ataataatta agtagtagcg attaatagag atgggatttc 9300
   gtgagaaatg gaccaaattt gggccgtttt gacccaaatt tttggtggtt ttttttccga 9360
   ttctttgtga atttcgggtc ggattcatca gcaattaatt acggttatta ggggctatta 9420
   gaggctttta attgggatta ttagagactt ttaagcggat ttggggactt ttaagtggat 9480
   tttatgattc ttaagtggat tttgggtgga ttttaccgct tttgggccgc cgcccccctc 9540
   taacccccca tctccccccc ttttctctct cccccccccc ccccaaaccc cacagcctcc 9600
   accaagggcc catcggtctt ccccctggca ccctcctcca agagcacctc tgggggcaca 9660
   gcggccctgg gctgcctggt caaggactac ttccccgaac cggtgacggt gtcgtggaac 9720
   tcaggcgccc tgaccagcgg cgtgcacacc ttcccggctg tcctacagtc ctcaggactc 9780
   tactccctca gcagcgtggt gaccgtgccc tccagcagct tgggcaccca gacctacatc 9840
   tgcaacgtga atcacaagcc cagcaacacc aaggtggaca agagagttga gcccaaatct 9900
   tgtgacaaaa ctcacacatg cccaccgtgc ccaggtaagt cactagacca gagctccact 9960
   cccgggagaa tggtaagtgc tgtaaacatc cctgcactag aggataagcc atgtacagat 10020
   ccatttccat ctctcctcat cagcacctga actcctgggg ggaccgtcag tcttcctctt 10080
   ccccccaaaa cccaaggaca ccctcatgat ctcccggacc cctgaggtca catgcgtggt 10140
   ggtggacgtg agccacgaag accctgaggt caagttcaac tggtacgtgg acggcgtgga 10200
   ggtgcataat gccaagacaa agccgcggga ggagcagtac aacagcacgt accgtgtggt 10260
   cagcgtcctc accgtcctgc accaggactg gctgaatggc aaggagtaca agtgcaaggt 10320
   ctccaacaaa gccctcccag cccccatcga gaaaaccatc tccaaagcca aaggtgagag 10380
   ctgcagcctg actgcatggg ggctgggatg ggcataagga taaaggtctg tgtggacagc 10440
   cttctgcttc agccatgacc tttgtgtatg tttctaccct cacagggcag ccccgagaac 10500
   cacaggtgta caccctgccc ccatcccggg aggagatgac caagaaccag gtcagcctga 10560
   cctgcctggt caaaggcttc tatcccagcg acatcgccgt ggagtgggag agcaatgggc 10620
   agccggagaa caactacaag accacgcctc ccgtgctgga ctccgacggc tccttcttcc 10680
   tctatagcaa gctcaccgtg gacaagagca ggtggcagca ggggaacgtc ttctcatgct 10740
   ccgtgatgca tgaggctctg cacaaccact acacgcagaa gagcctctcc ctgtccccgg 10800
   gtaaatgagc tcagcaccca caaaactctc aggtccaaag agacacccac actcatctcc 10860
   atgcttccct tgtataaata aagcacccag caatgcctgg gaccatgtaa aactgtcctg 10920
   gttctttcca aggtatagag catagctcac aggctgatat ttctggccag ggttggagga 10980
   cagccttgtc tataggaaga gaatgaggtt tttgcactgc aggactcaga gctcattagt 11040
   tatcctgcct tggagtgttg gggcttggct ttaggcagtg ccttttcctt gccttcctac 11100
   gaaccagcag ctgccataca tagagataat cctaggaagc ctcaaatgga gaaggacaca 11160
   aacccacctc cctcaggctg ttcctctatc ccggccccac ttctttacct aggggtttct 11220
   ctgagtctat tgtggagtta cacatggcca ggggcattcc agagaccctt gtcatccata 11280
   cactcaactc aggcagcttt gcacaaacaa agtctgcaca cccatacaga tggctcactc 11340
   ttgcctgtgc catgtagggc tgaggcacat ggctcttgct gccccaaggg agggactatt 11400
   agatagccac actcatgctg aatcctggcc cattcaaatt agcctgctga acaccatcca 11460
   gtccatatag cacatgtatc cacatgcacg tgtgcacaaa acgcatttaa cacactggga 11520
   caacaattct gtgccctgca cagcacctat atccagcaat gtatcaccat acacacgacc 11580
   aaaaattcaa tgcccacgtt tctgccatca caaacagaca catctttcct ctctgtggcc 11640
   actgcattat atgctcaaca caagacctct gaagccagat ccatctctgg cacctcgggg 11700
   tcatgcttca accccacatg aattatgcaa accatagcca taatggtctg aatcacttca 11760
   cactgggatg ttcccaagtt caggcaagac gagccacagg ctctgctgat gactgaagga 11820
   cagcaaaggg tcagtccagc tgtatagcca ctgttgacct gggtcacagg ccctgctgac 11880
   cctccacctt ctcctgtact gaaggaatga aagatgagac aagcatagag ggcacttgaa 11940
   taatccaggt cactctgagg tccacccaag gcattattgg actcaggtgg gaagctgaga 12000
   ctggtgtccc agagggaaag gaaggaaagc aggccccggg gagggtctgc tgtcccagtc 12060
   aggctggaga tctctcctct gaatccatgc agacatgtct gcctcacagg gaatctctcc 12120
   cagcaccaac catgttggga caaacactga ctgtcctctc tgttcagagc tgcaactgga 12180
   ggagagctgt gcggaggcgc aggacgggga gctggacggg ctgtggacga ccatcaccat 12240
   cttcatcaca ctcttcctgt taagcgtgtg ctacagtgcc accgtcacct tcttcaaggt 12300
   gaagtggatc ttctcctcgg tggtggacct gaagcagacc atcatccccg actacaggaa 12360
   catgatcgga cagggggcct aggaattaat tcgctgtctg cgagggccag ctgttggggt 12420
   gagtactccc tctcaaaagc gggcatgact tctgcgctaa gattgtcagt ttccaaaaac 12480
   gaggaggatt tgatattcac ctggcccgcg gtgatgcctt tgagggtggc cgcgtccatc 12540
   tggtcagaaa agacaatctt tttgttgtca agcttgaggt gtggcaggct tgagatctgg 12600
   ccatacactt gagtgacaat gacatccact ttgcctttct ctccacaggt gtccactccc 12660
   aggtccaact gcaggtcgag catgcatcta gggcggccaa ttggatccta ccgttcgtat 12720
   aatgtatgct atacgaagtt atcgtacgct taattaactg atccggaatt ccgccccccc 12780
   cccccccccc cctctccctc ccccccccct aacgttactg gccgaagccg cttggaataa 12840
   ggccggtgtg cgtttgtcta tatgttattt tccaccatat tgccgtcttt tggcaatgtg 12900
   agggcccgga aacctggccc tgtcttcttg acgagcattc ctaggggtct ttcccctctc 12960
   gccaaaggaa tgcaaggtct gttgaatgtc gtgaaggaag cagttcctct ggaagcttct 13020
   tgaagacaaa caacgtctgt agcgaccctt tgcaggcagc ggaacccccc acctggcgac 13080
   aggtgcctct gcggccaaaa gccacgtgta taagatacac ctgcaaaggc ggcacaaccc 13140
   cagtgccacg ttgtgagttg gatagttgtg gaaagagtca aatggctctc ctcaagcgta 13200
   ttcaacaagg ggctgaagga tgcccagaag gtaccccatt gtatgggatc tgatctgggg 13260
   cctcggtgca catgctttac atgtgtttag tcgaggttaa aaaaacgtct aggccccccg 13320
   aaccacgggg acgtggtttt cctttgaaaa acacgatgat aagcttgcca caaccatggc 13380
   tgaacaagat ggattgcacg caggttctcc ggccgcttgg gtggagaggc tattcggcta 13440
   tgactgggca caacagacaa tcggctgctc tgatgccgcc gtgttccggc tgtcagcgca 13500
   ggggcgcccg gttctttttg tcaagaccga cctgtccggt gccctgaatg aactgcagga 13560
   cgaggcagcg cggctatcgt ggctggccac gacgggcgtt ccttgcgcag ctgtgctcga 13620
   cgttgtcact gaagcgggaa gggactggct gctattgggc gaagtgccgg ggcaggatct 13680
   cctgtcatct caccttgctc ctgccgagaa agtatccatc atggctgatg caatgcggcg 13740
   gctgcatacg cttgatccgg ctacctgccc attcgaccac caagcgaaac atcgcatcga 13800
   gcgagcacgt actcggatgg aagccggtct tgtcgatcag gatgatctgg acgaagagca 13860
   tcaggggctc gcgccagccg aactgttcgc caggctcaag gcgcgcatgc ccgacggcga 13920
   ggatctcgtc gtgacccatg gcgatgcctg cttgccgaat atcatggtgg aaaatggccg 13980
   cttttctgga ttcatcgact gtggccggct gggtgtggcg gaccgctatc aggacatagc 14040
   gttggctacc cgtgatattg ctgaagagct tggcggcgaa tgggctgacc gcttcctcgt 14100
   gctttacggt atcgccgctc ccgattcgca gcgcatcgcc ttctatcgcc ttcttgacga 14160
   gttcttctga ataacttcgt ataatgtatg ctatacgaac ggtaggatcc cgcccgcccc 14220
   acgacccgca gcgcccgacc gaaaggagcg cacgacccca tggctccgac cgaagccacc 14280
   cggggcggcc ccgccgaccc cgcacccgcc cccgaggccc accgactcta gataactgat 14340
   cataatcagc cataccacat ttgtagaggt tttacttgct ttaaaaaacc tcccacacct 14400
   ccccctgaac ctgaaacata aaatgaatgc aattgttgtt gttaacttgt ttattgcagc 14460
   ttataatggt tacaaataaa gcaatagcat cacaaatttc acaaataaag catttttttc 14520
   actgtatgca aaccacctcc tggtgtcccc caacccttca tagcccccca ccccgtctcg 14580
   gtcgcccacc caaccctcca cattaacccc ttcctcccca cgctcgtccc ccgattccca 14640
   cccccctttt cgtcaccatg caccccccgt cccgcgagga cttcgaaggc cccttccgca 14700
   acgcctccat cctctgccag acccgcgggc gccgccgtcc cgccgaggtc acgtggtaca 14760
   aaaatggcag ccccgtcgcc gccgccgccg ccaccaccgc caccaccgtc ggccccgaag 14820
   tggtggccga gagccgcatc agcgtcaccg aaagcgaatg ggacaccggg gccaccttca 14880
   gctgcgtcgt ggagggggag atgaggaaca ccagcaagag gatggagtgc ggatgtaagt 14940
   ggaggggggg tttttggggg ggggatttgg ggggaaattg ggggctttgg gggcaaaaag 15000
   tccgtcattt tggtttggaa ggggtcgatg tgggggaaaa tgtgccaaaa atcccaattt 15060
   tgggggcact gaagtcgtaa tttgaggtgg aggaaggtag acagaaagag ggtggagtgc 15120
   ggatgtaagt ggagggggat ttggggcaga tttgggggaa attgggggtt tggggggcaa 15180
   ataatccgta gtttcggttt ggaaggggtt gatgtggttt tgtgctgaat ttcgggggaa 15240
   aaactgccaa aaatcccaat tttgggggca ttgaagtcat agtttgaggt tggaaggagg 15300
   tagagagcaa gaggatgaag tgaggatgta agtcggggga tttttggggc agatttgggg 15360
   gaaattgggg gttttggggt cagagaatcc gtaggtcgac cgcgccagct tttgttccct 15420
   ttagtgaggg ttaattgcgc gcttggcgta atcatggtca tagctgtttc ctgtgtgaaa 15480
   ttgttatccg ctcacaattc cacacaacat acgagccgga agcataaagt gtaaagcctg 15540
   gggtgcctaa tgagtgagct aactcacatt aattgcgttg cgctcactgc ccgctttcca 15600
   gtcgggaaac ctgtcgtgcc agctgcatta atgaatcggc caacgcgcgg ggagaggcgg 15660
   tttgcgtatt gggcgctctt ccgcttcctc gctcactgac tcgctgcgct cggtcgttcg 15720
   gctgcggcga gcggtatcag ctcactcaaa ggcggtaata cggttatcca cagaatcagg 15780
   ggataacgca ggaaagaaca tgtgagcaaa aggccagcaa aaggccagga accgtaaaaa 15840
   ggccgcgttg ctggcgtttt tccataggct ccgcccccct gacgagcatc acaaaaatcg 15900
   acgctcaagt cagaggtggc gaaacccgac aggactataa agataccagg cgtttccccc 15960
   tggaagctcc ctcgtgcgct ctcctgttcc gaccctgccg cttaccggat acctgtccgc 16020
   ctttctccct tcgggaagcg tggcgctttc tcatagctca cgctgtaggt atctcagttc 16080
   ggtgtaggtc gttcgctcca agctgggctg tgtgcacgaa ccccccgttc agcccgaccg 16140
   ctgcgcctta tccggtaact atcgtcttga gtccaacccg gtaagacacg acttatcgcc 16200
   actggcagca gccactggta acaggattag cagagcgagg tatgtaggcg gtgctacaga    16260
   gttcttgaag tggtggccta actacggcta cactagaagg acagtatttg gtatctgcgc    16320
   tctgctgaag ccagttacct tcggaaaaag agttggtagc tcttgatccg gcaaacaaac    16380
   caccgctggt agcggtggtt tttttgtttg caagcagcag attacgcgca gaaaaaaagg    16440
   atctcaagaa gatcctttga tcttttctac ggggtctgac gctcagtgga acgaaaactc    16500
   acgttaaggg attttggtca tgagattatc aaaaaggatc ttcacctaga tccttttaaa    16560
   ttaaaaatga agttttaaat caatctaaag tatatatgag taaacttggt ctgacagtta    16620
   ccaatgctta atcagtgagg cacctatctc agcgatctgt ctatttcgtt catccatagt    16680
   tgcctgactc cccgtcgtgt agataactac gatacgggag ggcttaccat ctggccccag    16740
   tgctgcaatg ataccgcgag acccacgctc accggctcca gatttatcag caataaacca    16800
   gccagccgga agggccgagc gcagaagtgg tcctgcaact ttatccgcct ccatccagtc    16860
   tattaattgt tgccgggaag ctagagtaag tagttcgcca gttaatagtt tgcgcaacgt    16920
   tgttgccatt gctacaggca tcgtggtgtc acgctcgtcg tttggtatgg cttcattcag    16980
   ctccggttcc caacgatcaa ggcgagttac atgatccccc atgttgtgca aaaaagcggt    17040
   tagctccttc ggtcctccga tcgttgtcag aagtaagttg gccgcagtgt tatcactcat    17100
   ggttatggca gcactgcata attctcttac tgtcatgcca tccgtaagat gcttttctgt    17160
   gactggtgag tactcaacca agtcattctg agaatagtgt atgcggcgac cgagttgctc    17220
   ttgcccggcg tcaatacggg ataataccgc gccacatagc agaactttaa aagtgctcat    17280
   cattggaaaa cgttcttcgg ggcgaaaact ctcaaggatc ttaccgctgt tgagatccag    17340
   ttcgatgtaa cccactcgtg cacccaactg atcttcagca tcttttactt tcaccagcgt    17400
   ttctgggtga gcaaaaacag gaaggcaaaa tgccgcaaaa aagggaataa gggcgacacg    17460
   gaaatgttga atactcatac tcttcctttt tcaatattat tgaagcattt atcagggtta    17520
   ttgtctcatg agcggataca tatttgaatg tatttagaaa aataaacaaa taggggttcc    17580
   gcgcacattt ccccgaaaag tgccac                                          17606
<210> 44
   <211> 1032
   <212> DNA
   <213> Bacteriophage P1
<400> 44
   atgtccaatt tactgaccgt acaccaaaat ttgcctgcat taccggtcga tgcaacgagt 60
   gatgaggttc gcaagaacct gatggacatg ttcagggatc gccaggcgtt ttctgagcat 120
   acctggaaaa tgcttctgtc cgtttgccgg tcgtgggcgg catggtgcaa gttgaataac 180
   cggaaatggt ttcccgcaga acctgaagat gttcgcgatt atcttctata tcttcaggcg 240
   cgcggtctgg cagtaaaaac tatccagcaa catttgggcc agctaaacat gcttcatcgt 300
   cggtccgggc tgccacgacc aagtgacagc aatgctgttt cactggttat gcggcggatc 360
   cgaaaagaaa acgttgatgc cggtgaacgt gcaaaacagg ctctagcgtt cgaacgcact 420
   gatttcgacc aggttcgttc actcatggaa aatagcgatc gctgccagga tatacgtaat 480
   ctggcatttc tggggattgc ttataacacc ctgttacgta tagccgaaat tgccaggatc 540
   agggttaaag atatctcacg tactgacggt gggagaatgt taatccatat tggcagaacg 600
   aaaacgctgg ttagcaccgc aggtgtagag aaggcactta gcctgggggt aactaaactg 660
   gtcgagcgat ggatttccgt ctctggtgta gctgatgatc cgaataacta cctgttttgc 720
   cgggtcagaa aaaatggtgt tgccgcgcca tctgccacca gccagctatc aactcgcgcc 780
   ctggaaggga tttttgaagc aactcatcga ttgatttacg gcgctaagga tgactctggt 840
   cagagatacc tggcctggtc tggacacagt gcccgtgtcg gagccgcgcg agatatggcc 900
   cgcgctggag tttcaatacc ggagatcatg caagctggtg gctggaccaa tgtaaatatt 960
   gtcatgaact atatccgtaa cctggatagt gaaacagggg caatggtgcg cctgctggaa 1020
   gatggcgatt ag 1032
<210> 45
   <211> 5739
   <212> DNA
   <213> Artificial Sequence
<220>
   <223> Expression vector
<400> 45
   tagttattaa tagtaatcaa ttacggggtc attagttcat agcccatata tggagttccg        60
   cgttacataa cttacggtaa atggcccgcc tggctgaccg cccaacgacc cccgcccatt       120
   gacgtcaata atgacgtatg ttcccatagt aacgccaata gggactttcc attgacgtca       180
   atgggtggag tatttacggt aaactgccca cttggcagta catcaagtgt atcatatgcc       240
   aagtacgccc cctattgacg tcaatgacgg taaatggccc gcctggcatt atgcccagta       300
   catgacctta tgggactttc ctacttggca gtacatctac gtattagtca tcgctattac       360
   catggtgatg cggttttggc agtacatcaa tgggcgtgga tagcggtttg actcacgggg       420
   atttccaagt ctccacccca ttgacgtcaa tgggagtttg ttttggcacc aaaatcaacg       480
   ggactttcca aaatgtcgta acaactccgc cccattgacg caaatgggcg gtaggcgtgt       540
   acggtgggag gtctatataa gcagagctgg tttagtgaac cgtcagatcc gctagcgcta       600
   ccggtcgcca ccatggtgag caagggcgag gagctgttca ccggggtggt gcccatcctg       660
   gtcgagctgg acggcgacgt aaacggccac aagttcagcg tgtccggcga gggcgagggc       720
   gatgccacct acggcaagct gaccctgaag ttcatctgca ccaccggcaa gctgcccgtg 780
   ccctggccca ccctcgtgac caccctgacc tacggcgtgc agtgcttcag ccgctacccc 840
   gaccacatga agcagcacga cttcttcaag tccgccatgc ccgaaggcta cgtccaggag 900
   cgcaccatct tcttcaagga cgacggcaac tacaagaccc gcgccgaggt gaagttcgag 960
   ggcgacaccc tggtgaaccg catcgagctg aagggcatcg acttcaagga ggacggcaac 1020
   atcctggggc acaagctgga gtacaactac aacagccaca acgtctatat catggccgac 1080
   aagcagaaga acggcatcaa ggtgaacttc aagatccgcc acaacatcga ggacggcagc 1140
   gtgcagctcg ccgaccacta ccagcagaac acccccatcg gcgacggccc cgtgctgctg 1200
   cccgacaacc actacctgag cacccagtcc gccctgagca aagaccccaa cgagaagcgc 1260
   gatcacatgg tcctgctgga gttcgtgacc gccgccggga tcactctcgg catggacgag 1320
   ctgtacaagt ccggactcag atctatgtcc aatttactga ccgtacacca aaatttgcct 1380
   gcattaccgg tcgatgcaac gagtgatgag gttcgcaaga acctgatgga catgttcagg 1440
   gatcgccagg cgttttctga gcatacctgg aaaatgcttc tgtccgtttg ccggtcgtgg 1500
   gcggcatggt gcaagttgaa taaccggaaa tggtttcccg cagaacctga agatgttcgc 1560
   gattatcttc tatatcttca ggcgcgcggt ctggcagtaa aaactatcca gcaacatttg 1620
   ggccagctaa acatgcttca tcgtcggtcc gggctgccac gaccaagtga cagcaatgct 1680
   gtttcactgg ttatgcggcg gatccgaaaa gaaaacgttg atgccggtga acgtgcaaaa 1740
   caggctctag cgttcgaacg cactgatttc gaccaggttc gttcactcat ggaaaatagc 1800
   gatcgctgcc aggatatacg taatctggca tttctgggga ttgcttataa caccctgtta 1860
   cgtatagccg aaattgccag gatcagggtt aaagatatct cacgtactga cggtgggaga 1920
   atgttaatcc atattggcag aacgaaaacg ctggttagca ccgcaggtgt agagaaggca 1980
   cttagcctgg gggtaactaa actggtcgag cgatggattt ccgtctctgg tgtagctgat 2040
   gatccgaata actacctgtt ttgccgggtc agaaaaaatg gtgttgccgc gccatctgcc 2100
   accagccagc tatcaactcg cgccctggaa gggatttttg aagcaactca tcgattgatt 2160
   tacggcgcta aggatgactc tggtcagaga tacctggcct ggtctggaca cagtgcccgt 2220
   gtcggagccg cgcgagatat ggcccgcgct ggagtttcaa taccggagat catgcaagct 2280
   ggtggctgga ccaatgtaaa tattgtcatg aactatatcc gtaacctgga tagtgaaaca 2340
   ggggcaatgg tgcgcctgct ggaagatggc gattaggtcg acggtaccgc gggcccggga 2400
   tccaccggat ctagataact gatcataatc agccatacca catttgtaga ggttttactt 2460
   gctttaaaaa acctcccaca cctccccctg aacctgaaac ataaaatgaa tgcaattgtt 2520
   gttgttaact tgtttattgc agcttataat ggttacaaat aaagcaatag catcacaaat 2580
   ttcacaaata aagcattttt ttcactgcat tctagttgtg gtttgtccaa actcatcaat 2640
   gtatcttaac gcgtaaattg taagcgttaa tattttgtta aaattcgcgt taaatttttg 2700
   ttaaatcagc tcatttttta accaataggc cgaaatcggc aaaatccctt ataaatcaaa 2760
   agaatagacc gagatagggt tgagtgttgt tccagtttgg aacaagagtc cactattaaa 2820
   gaacgtggac tccaacgtca aagggcgaaa aaccgtctat cagggcgatg gcccactacg 2880
   tgaaccatca ccctaatcaa gttttttggg gtcgaggtgc cgtaaagcac taaatcggaa 2940
   ccctaaaggg agcccccgat ttagagcttg acggggaaag ccggcgaacg tggcgagaaa 3000
   ggaagggaag aaagcgaaag gagcgggcgc tagggcgctg gcaagtgtag cggtcacgct 3060
   gcgcgtaacc accacacccg ccgcgcttaa tgcgccgcta cagggcgcgt caggtggcac 3120
   ttttcgggga aatgtgcgcg gaacccctat ttgtttattt ttctaaatac attcaaatat 3180
   gtatccgctc atgagacaat aaccctgata aatgcttcaa taatattgaa aaaggaagag 3240
   tcctgaggcg gaaagaacca gctgtggaat gtgtgtcagt tagggtgtgg aaagtcccca 3300
   ggctccccag caggcagaag tatgcaaagc atgcatctca attagtcagc aaccaggtgt 3360
   ggaaagtccc caggctcccc agcaggcaga agtatgcaaa gcatgcatct caattagtca 3420
   gcaaccatag tcccgcccct aactccgccc atcccgcccc taactccgcc cagttccgcc 3480
   cattctccgc cccatggctg actaattttt tttatttatg cagaggccga ggccgcctcg 3540
   gcctctgagc tattccagaa gtagtgagga ggcttttttg gaggcctagg cttttgcaaa 3600
   gatcgatcaa gagacaggat gaggatcgtt tcgcatgatt gaacaagatg gattgcacgc 3660
   aggttctccg gccgcttggg tggagaggct attcggctat gactgggcac aacagacaat 3720
   cggctgctct gatgccgccg tgttccggct gtcagcgcag gggcgcccgg ttctttttgt 3780
   caagaccgac ctgtccggtg ccctgaatga actgcaagac gaggcagcgc ggctatcgtg 3840
   gctggccacg acgggcgttc cttgcgcagc tgtgctcgac gttgtcactg aagcgggaag 3900
   ggactggctg ctattgggcg aagtgccggg gcaggatctc ctgtcatctc accttgctcc 3960
   tgccgagaaa gtatccatca tggctgatgc aatgcggcgg ctgcatacgc ttgatccggc 4020
   tacctgccca ttcgaccacc aagcgaaaca tcgcatcgag cgagcacgta ctcggatgga 4080
   agccggtctt gtcgatcagg atgatctgga cgaagagcat caggggctcg cgccagccga 4140
   actgttcgcc aggctcaagg cgagcatgcc cgacggcgag gatctcgtcg tgacccatgg 4200
   cgatgcctgc ttgccgaata tcatggtgga aaatggccgc ttttctggat tcatcgactg 4260
   tggccggctg ggtgtggcgg accgctatca ggacatagcg ttggctaccc gtgatattgc 4320
   tgaagagctt ggcggcgaat gggctgaccg cttcctcgtg ctttacggta tcgccgctcc 4380
   cgattcgcag cgcatcgcct tctatcgcct tcttgacgag ttcttctgag cgggactctg 4440
   gggttcgaaa tgaccgacca agcgacgccc aacctgccat cacgagattt cgattccacc 4500
   gccgccttct atgaaaggtt gggcttcgga atcgttttcc gggacgccgg ctggatgatc      4560
   ctccagcgcg gggatctcat gctggagttc ttcgcccacc ctagggggag gctaactgaa      4620
   acacggaagg agacaatacc ggaaggaacc cgcgctatga cggcaataaa aagacagaat      4680
   aaaacgcacg gtgttgggtc gtttgttcat aaacgcgggg ttcggtccca gggctggcac      4740
   tctgtcgata ccccaccgag accccattgg ggccaatacg cccgcgtttc ttccttttcc      4800
   ccaccccacc ccccaagttc gggtgaaggc ccagggctcg cagccaacgt cggggcggca      4860
   ggccctgcca tagcctcagg ttactcatat atactttaga ttgatttaaa acttcatttt      4920
   taatttaaaa ggatctaggt gaagatcctt tttgataatc tcatgaccaa aatcccttaa      4980
   cgtgagtttt cgttccactg agcgtcagac cccgtagaaa agatcaaagg atcttcttga      5040
   gatccttttt ttctgcgcgt aatctgctgc ttgcaaacaa aaaaaccacc gctaccagcg      5100
   gtggtttgtt tgccggatca agagctacca actctttttc cgaaggtaac tggcttcagc      5160
   agagcgcaga taccaaatac tgtccttcta gtgtagccgt agttaggcca ccacttcaag      5220
   aactctgtag caccgcctac atacctcgct ctgctaatcc tgttaccagt ggctgctgcc      5280
   agtggcgata agtcgtgtct taccgggttg gactcaagac gatagttacc ggataaggcg      5340
   cagcggtcgg gctgaacggg gggttcgtgc acacagccca gcttggagcg aacgacctac      5400
   accgaactga gatacctaca gcgtgagcta tgagaaagcg ccacgcttcc cgaagggaga      5460
   aaggcggaca ggtatccggt aagcggcagg gtcggaacag gagagcgcac gagggagctt      5520
   ccagggggaa acgcctggta tctttatagt cctgtcgggt ttcgccacct ctgacttgag      5580
   cgtcgatttt tgtgatgctc gtcagggggg cggagcctat ggaaaaacgc cagcaacgcg      5640
   gcctttttac ggttcctggc cttttgctgg ccttttgctc acatgttctt tcctgcgtta      5700
   tcccctgatt ctgtggataa ccgtattacc gccatgcat                             5739
<210> 46
   <211> 54
   <212> DNA
   <213> Artificial Sequence
<220>
   <223> Synthetic Primer
<220>
   <221> misc_feature
   <222> (26)..(35)
   <223> n is a, g, c or t.
<400> 46
   cgtatcgcct ccctcgcgcc atcagnnnnn nnnnncaggt tccctggtgc aggc 54
<210> 47
   <211> 44
   <212> DNA
   <213> Artificial Sequence
<220>
   <223> Synthetic Primer
<400> 47
   ctatgcgcct tgccagcccg ctcaggcttg gtccctccgc cgaa 44
<210> 48
   <211> 41
   <212> DNA
   <213> Artificial Sequence
<220>
   <223> Synthetic Primer
<400> 48
   ctatgcgcct tgccagcccg ctcagtccgt cagcgctctc t 41
<210> 49
   <211> 54
   <212> DNA
   <213> Artificial Sequence
<220>
   <223> Synthetic Primer
<220>
   <221> misc_feature
   <222> (26)..(35)
   <223> n is a, g, c or t.
<400> 49
   cgtatcgcct ccctcgcgcc atcagnnnnn nnnnncggtg accagggtgc cctg 54
<210> 50
   <211> 2503
   <212> DNA
   <213> Homo sapiens
<400> 50
   tcgccccctc gacacgagtg cgtacgctcg acaagacgca ctccagtctg ccctgactca      60
   gcctgcctcc gtgtctgggt ctcctggaca gtcgatcacc atctcctgct ctggaagcag     120
   ctccaacatc ggaagtaatt atgtatactg gtaccaacag cacccaggca aagcccccaa     180
   actcatgatt tataggaata atcagcggcc ctcaggggtt tctaatcgct tctctggctc     240
   caagtctggc aacacggcct ccctgaccat ctctgggctc caggctgagg acgaggctga     300
   ttattactgc gcagcatggg atgacagcct gagtggtcat tgggtgttcg gcggagggac     360
   caagctgacc gtcctagcct gacacacaca gccctcccca gactgtggga tgaggccctg     420
   tgcccgcagt cacatgtgga atatcaagac acacacatct atgacaatca cagcactgta     480
   gatcagacac gatatcgcga gcagtctgcc ctgactcagc ctgcctccgt gtctgggtct     540
   cctggacagt cgatcaccat ctcctgctct ggaagcagct ccaacatcgg aaataatgct 600
   gtaaactggt accaacagca cccaggcaaa gcccccaaac tcatgattta ttatgatgat 660
   ctgctgccct caggggtttc taatcgcttc tctggctcca agtctggcaa cacggcctcc 720
   ctgaccatct ctgggctcca ggctgaggac gaggctgatt attactgcgg aacatgggat 780
   ggcagcctga atggtgggag gctgttcggc ggagggacca agctgaccgt cctagcctga 840
   cacacacagc cctccccaga ctgtgggatg aggccctgtg cccgcagtca catgtggaat 900
   atcaagacac acacatctat gacaatcacc gtgtctctac tcgcgtgtct ctctatgcgc 960
   tcgaggtcga cggtatcgat aagcttgata tcgaattcct gcagcccggg ggatcttacg 1020
   ctgtcttagt gtagattcga tcacgtcagt ctgccctgac tcagcctgcc tccgtgtctg 1080
   ggtctcctgg acagtcgatc accatctcct gctctggaga agcattgcca aaaaaatatg 1140
   cttattggta ccaacagcac ccaggcaaag cccccaaact catgatttat aaagacagcg 1200
   agaggccctc aggggtttct aatcgcttct ctggctccaa gtctggcaac acggcctccc 1260
   tgaccatctc tgggctccag gctgaggacg aggctgatta ttactgcaac tcttttacaa 1320
   gcaccaccaa ttatgtctta ttcggcggag ggaccaagct gaccgtccta gcctgacaca 1380
   cacagccctc cccagactgt gggatgaggc cctgtgcccg cagtcacatg tggaatatca 1440
   agacacacac atctatgaca atcactcgca ctagttctag cgacttctat actatggatc 1500
   cactagacgt acagtcacgt actcagacta cgctctacag tctgccctga ctcagcctgc 1560
   ctccgtgtct gggtctcctg gacagtcgat caccatctcc tgcaccctaa gcagtgagca 1620
   cagcacctac accatcgaat ggtaccaaca gcacccaggc aaagccccca aactcatgat 1680
   ttatgttaag agtgatggca gccacagcaa gggggacggg gtttctaatc gcttctctgg 1740
   ctccaagtct ggcaacacgg cctccctgac catctctggg ctccaggctg aggacgaggc 1800
   tgattattac tgcggagaga gccacacgat tgatggccaa gtcggtgtgg tattcggcgg 1860
   agggaccaag ctgaccgtcc tagcctgaca cacacagccc tccccagact gtgggatgag 1920
   gccctgtgcc cgcagtcaca tgtggaatat caagacacac acatctatga caatcaccta 1980
   tagcgtatac gtcatcatag tcgcataact agacagtctg ccctgactca gcctgcctcc 2040
   gtgtctgggt ctcctggaca gtcgatcacc atctcctgca ctctgagcag tgggcacagt 2100
   agctacatca tcgcatggta ccaacagcac ccaggcaaag cccccaaact catgatttat 2160
   cttgaaggta gtggaagcta caacaagggg agcggggttt ctaatcgctt ctctggctcc 2220
   aagtctggca acacggcctc cctgaccatc tctgggctcc aggctgagga cgaggctgat 2280
   tattactgcg agacctggga cagtaacact catgtggtat tcggcggagg gaccaagctg 2340
   accgtcctag cctgacacac acagccctcc ccagactgtg ggatgaggcc ctgtgcccgc 2400
   agtcacatgt ggaatatcaa gacacacaca tctatgacaa tcactatata tacatatgct 2460
   agtatcacgc gagaactagt tctagagcgg ccgccaccgc cga 2503
<210> 51
   <211> 12801
   <212> DNA
   <213> Artificial Sequence
<220>
   <223> Targeting vector
<400> 51
   agcgcccaat acgcaaaccg cctctccccg cgcgttggcc gattcattaa tgcagctggc      60
   acgacaggtt tcccgactgg aaagcgggca gtgagcgcaa cgcaattaat gtgagttagc     120
   tcactcatta ggcaccccag gctttacact ttatgcttcc ggctcgtatg ttgtgtggaa     180
   ttgtgagcgg ataacaattt cacacaggaa acagctatga ccatgattac gccaagctca     240
   gaattaaccc tcactaaagg gactagtcct gcaggtttaa acgaattcgc ccttgtcgac     300
   gggtatcacg tggggcaaat cgagagctac cattggctat ggggaggaga aaatgaggcg     360
   tgctacttcc tcgttcctgc agtgcgtgtc gaggtgccgt gttggtcgga tctcagtggc     420
   agagcaggat ggtgagcggt ggccttgggg ccgctccctc actctgctgg gctggtgcgg     480
   tggctccggg accggacttt cagtggtgtt tggtgtggtg ctcactgtcc tttggtgtca     540
   ctgtgtggtg aattccctca gctgggcggt gagagcccat gctgtgtcct ggtaggagta     600
   gtctgggctg cagtgcgggc tctttgtgcg tgttgggggc ggttccctgg aagctaattc     660
   tggaagaaat cccggctgcc tctgtaacag ttgttggcga tgtcaaacac cctggagctg     720
   ctgactggct ctgtaccatt gagtgatgca gtttaaacct ctctctttcc tcctgctgtg     780
   tgtgaagctg gtgttagtgc taggagacct gcacatccct caccgatgca gtggtttacc     840
   agcaaagttc aagagcctcc tggttccagg gaagattcag catattttgt gtacaggaaa     900
   cctctgcacc aaggagagct atgactacct caggactttg gctggagatg ttcacgttgt     960
   taagggggac tctgaggtaa tgttcttgct gctgtgaatt cctcagtcct ccatcttaca    1020
   agccgtagtt gtcccactga aagctgggaa acccttcggt gtttgcaatt cctgagcgct    1080
   aatgtgtggg ggaaggatgt aggaacaaat aaggctcaag agacaacaac ttcctaggct    1140
   cctcttattg cccttgtggt aagggaggac ttggtgtgta gtggtaatac atgcactcaa    1200
   cttattaagt tctcatagct gctgtgaagc tgctgcagtc cctcagatcc ctgcaggaag    1260
   aggggactga ctgactgttg aatagccttg aagctcctgc tctgcatgtg gttccggtca    1320
   ggctcaagat cccagttctt agctcttcag ttgctcttct gggggtatag tactagcatc    1380
   tgggtcactg cagatggaca gagactgtgc ttgggtagca aaaggcagac ttacgttgtg    1440
   aaaccttcat tgtgttggtg aggttctttg atttctacct tgtgtatctc tcttcctcag    1500
   agtctgaatt atcctgaaca gaaggttgta actgttgggc agttcagaat tgggttgatt 1560
   catggccatc aagtcattcc ctggggtgat gtggcaagct tggcattgct gcagaggcag 1620
   ctggatgtag acattcttat ctcaggacat acacacagat ttgaagcatt tgagcatgaa 1680
   aataagttct acatcaaccc aggatcagct acaggagcct acactggttt acaaacgtga 1740
   gtacaatgag ccttgtgttt gtctttaaga cagatgtctg tccttaagag tgcagcccat 1800
   gtgttgaaga gcactggtga gatgcagtgc catgcaggcc atctctccct ttcccagaga 1860
   acacggccta cctctctctg ttcataaggt cttggttaac ttatgagttg gccagcacag 1920
   cctggcactg atgtgtcatg atcataaccc tgagcccacg ctctgctatg ctgttgcttc 1980
   ttagagttgc attgccttga ggtgtattcc agaagctgtg tgctgagctc ttgttgggaa 2040
   agaggccctc agggcactgc caacatctga ggcttgcagg agctggaaca ttcagccctg 2100
   agggctgctc tggccaactg cagctctgag tctgcagtca gtgcctctga gctgtgatgc 2160
   ttctcagata actgcagccc tttgcaatgt aatgttttat aatatatttc cataggacat 2220
   cttggtaaga ggtgtaagct aatcagggtg attgacaaag tatgttttct ttccaggaac 2280
   gtcattcctt catttgtact gatggatatc caggcttcca cagttgtgac ttatgtatac 2340
   cagctaattg aggacgatgt gaaagtagaa agaattgagt tcaagaaata ctgacacgtg 2400
   tttgaatctg cctgctgact tctcactgcc tctcattctt tctctcctag ttaaagctga 2460
   atccggccac gtgggggtgc tgctgcagcg gcgtgtaact gtgagcttgg gaagttctca 2520
   actagggctt agagcaggag aaactgatta acagaaattg ccttattaaa tgtatatatt 2580
   tcctgtgtct aagaacgagg tgctgttgtt cctgacttgc ggagctgcag ctagtggtag 2640
   cttggaattt aacctctcct gtaaaatgag catagaataa aacagtattt tcacatttct 2700
   ggatcctgtt aattgtttgt taattaagag gacaggaatt cagtgtcagg gggctgctct 2760
   ttgcttccca atgtgtacca ctgaagcctc tctggattta tcagagatac tcaatggact 2820
   cttaacagca ctgtggcacc tgacctcctg ctgccttcag cctcccttcc tggctggtgt 2880
   gcttatgttg aggagctgtg ggaggctggt gcagttctgt gggatgaggt aaggccagct 2940
   gaatagcctg ggggctggtc agggggctta gtgagaggag cagggctggg gaatgttgtg 3000
   aacagcaggt ggtgccactg ctattctgag gaaggaggca gcttctgcaa atgctttgca 3060
   atgcaggatg acaaagtgct tctgctagcc ctagtcaatt tctgagaact gtcattctcg 3120
   gaatacctcg atcgataact tcgtataatg tatactatac gaagttattt tatttgtgaa 3180
   atttgtgatg ctattgcttt atttgtaacc attataagct gcaataaaca agttggggtg 3240
   ggcgaagaac tccagcatga gatccccgcg ctggaggatc atccagccgg cgtcccggaa 3300
   aacgattccg aagcccaacc tttcatagaa ggcggcggtg gaatcgaaat ctcgtgatgg 3360
   caggttgggc gtcgcttggt cggtcatttc gaaccccaga gtcccgctca gaagaactcg 3420
   tcaagaaggc gatagaaggc gatgcgctgc gaatcgggag cggcgatacc gtaaagcacg 3480
   aggaagcggt cagcccattc gccgccaagc tcttcagcaa tatcacgggt agccaacgct 3540
   atgtcctgat agcggtccgc cacacccagc cggccacagt cgatgaatcc agaaaagcgg 3600
   ccattttcca ccatgatatt cggcaagcag gcatcgccat gggtcacgac gagatcctcg 3660
   ccgtcgggca tgcgcgcctt gagcctggcg aacagttcgg ctggcgcgag cccctgatgc 3720
   tcttcgtcca gatcatcctg atcgacaaga ccggcttcca tccgagtacg tgctcgctcg 3780
   atgcgatgtt tcgcttggtg gtcgaatggg caggtagccg gatcaagcgt atgcagccgc 3840
   cgcattgcat cagccatgat ggatactttc tcggcaggag caaggtgaga tgacaggaga 3900
   tcctgccccg gcacttcgcc caatagcagc cagtcccttc ccgcttcagt gacaacgtcg 3960
   agcacagctg cgcaaggaac gcccgtcgtg gccagccacg atagccgcgc tgcctcgtcc 4020
   tgcagttcat tcagggcacc ggacaggtcg gtcttgacaa aaagaaccgg gcgcccctgc 4080
   gctgacagcc ggaacacggc ggcatcagag cagccgattg tctgttgtgc ccagtcatag 4140
   ccgaatagcc tctccaccca agcggccgga gaacctgcgt gcaatccatc ttgttcaatc 4200
   atgcgaaacg atcctcatcc tgtctcttga tcagatccga aaatggatat acaagctccc 4260
   gggagctttt tgcaaaagcc taggcctcca aaaaagcctc ctcactactt ctggaatagc 4320
   tcagaggcag aggcggcctc ggcctctgca taaataaaaa aaattagtca gccatggggc 4380
   ggagaatggg cggaactggg cggagttagg ggcgggatgg gcggagttag gggcgggact 4440
   atggttgctg actaattgag atgcatgctt tgcatacttc tgcctgctgg ggagcctggg 4500
   gactttccac acctggttgc tgactaattg agatgcatgc tttgcatact tctgcctgct 4560
   ggggagcctg gggactttcc acaccctaaa taacttcgta taatgtatac tatacgaagt 4620
   tatctcgtgt ccgataactg taattatcgg accgtcaatc gattcgcccc ctcgacacga 4680
   gtgcgtacgc tcgacaagac gcactccagt ctgccctgac tcagcctgcc tccgtgtctg 4740
   ggtctcctgg acagtcgatc accatctcct gctctggaag cagctccaac atcggaagta 4800
   attatgtata ctggtaccaa cagcacccag gcaaagcccc caaactcatg atttatagga 4860
   ataatcagcg gccctcaggg gtttctaatc gcttctctgg ctccaagtct ggcaacacgg 4920
   cctccctgac catctctggg ctccaggctg aggacgaggc tgattattac tgcgcagcat 4980
   gggatgacag cctgagtggt cattgggtgt tcggcggagg gaccaagctg accgtcctag 5040
   cctgacacac acagccctcc ccagactgtg ggatgaggcc ctgtgcccgc agtcacatgt 5100
   ggaatatcaa gacacacaca tctatgacaa tcacagcact gtagatcaga cacgatatcg 5160
   cgagcagtct gccctgactc agcctgcctc cgtgtctggg tctcctggac agtcgatcac 5220
   catctcctgc tctggaagca gctccaacat cggaaataat gctgtaaact ggtaccaaca 5280
   gcacccaggc aaagccccca aactcatgat ttattatgat gatctgctgc cctcaggggt 5340
   ttctaatcgc ttctctggct ccaagtctgg caacacggcc tccctgacca tctctgggct 5400
   ccaggctgag gacgaggctg attattactg cggaacatgg gatggcagcc tgaatggtgg 5460
   gaggctgttc ggcggaggga ccaagctgac cgtcctagcc tgacacacac agccctcccc 5520
   agactgtggg atgaggccct gtgcccgcag tcacatgtgg aatatcaaga cacacacatc 5580
   tatgacaatc accgtgtctc tactcgcgtg tctctctatg cgctcgaggt cgacggtatc 5640
   gataagcttg atatcgaatt cctgcagccc gggggatctt acgctgtctt agtgtagatt 5700
   cgatcacgtc agtctgccct gactcagcct gcctccgtgt ctgggtctcc tggacagtcg 5760
   atcaccatct cctgctctgg agaagcattg ccaaaaaaat atgcttattg gtaccaacag 5820
   cacccaggca aagcccccaa actcatgatt tataaagaca gcgagaggcc ctcaggggtt 5880
   tctaatcgct tctctggctc caagtctggc aacacggcct ccctgaccat ctctgggctc 5940
   caggctgagg acgaggctga ttattactgc aactctttta caagcaccac caattatgtc 6000
   ttattcggcg gagggaccaa gctgaccgtc ctagcctgac acacacagcc ctccccagac 6060
   tgtgggatga ggccctgtgc ccgcagtcac atgtggaata tcaagacaca cacatctatg 6120
   acaatcactc gcactagttc tagcgacttc tatactatgg atccactaga cgtacagtca 6180
   cgtactcaga ctacgctcta cagtctgccc tgactcagcc tgcctccgtg tctgggtctc 6240
   ctggacagtc gatcaccatc tcctgcaccc taagcagtga gcacagcacc tacaccatcg 6300
   aatggtacca acagcaccca ggcaaagccc ccaaactcat gatttatgtt aagagtgatg 6360
   gcagccacag caagggggac ggggtttcta atcgcttctc tggctccaag tctggcaaca 6420
   cggcctccct gaccatctct gggctccagg ctgaggacga ggctgattat tactgcggag 6480
   agagccacac gattgatggc caagtcggtg tggtattcgg cggagggacc aagctgaccg 6540
   tcctagcctg acacacacag ccctccccag actgtgggat gaggccctgt gcccgcagtc 6600
   acatgtggaa tatcaagaca cacacatcta tgacaatcac ctatagcgta tacgtcatca 6660
   tagtcgcata actagacagt ctgccctgac tcagcctgcc tccgtgtctg ggtctcctgg 6720
   acagtcgatc accatctcct gcactctgag cagtgggcac agtagctaca tcatcgcatg 6780
   gtaccaacag cacccaggca aagcccccaa actcatgatt tatcttgaag gtagtggaag 6840
   ctacaacaag gggagcgggg tttctaatcg cttctctggc tccaagtctg gcaacacggc 6900
   ctccctgacc atctctgggc tccaggctga ggacgaggct gattattact gcgagacctg 6960
   ggacagtaac actcatgtgg tattcggcgg agggaccaag ctgaccgtcc tagcctgaca 7020
   cacacagccc tccccagact gtgggatgag gccctgtgcc cgcagtcaca tgtggaatat 7080
   caagacacac acatctatga caatcactat atatacatat gctagtatca cgcgagaact 7140
   agttctagag cggccgccac cgccgaggcg cgccatattg cctccctgtg ttgctgttgc 7200
   agggttgctt ctgcctcata ctggtttctc ccttctggag gtggccaaaa gccgggccct 7260
   gtgcaaccct ggtgcataaa taccttatgg cccctaagta gggcaggtgt gggacacgct 7320
   ctggcacctg gggtgtgtgc aagtgctcag gaagacctgc aggcacaggt ggcagtgggg 7380
   gtctctggct gtgctcgagc agcagctgcc tggggtaagg gtagtactct gtgcatgaac 7440
   aatgctgcag ggctcagctc tgctcagacc acgaccctgg caccaacaga gacctgcctg 7500
   gctctgtggt catgtaaacc tttacaggag ctcaagacaa ggctgtttat tactgctctg 7560
   gcaggaaaga agcactggcc atggtcatag agagttccag caacaggaaa gtgagagccc 7620
   aagctgctga ggtaccaggg ctcctcaggt gcctgctgca gcagcttgga cacagtcgag 7680
   gaacagcaat tgtacctgtg tggtggatca ggctgtgctg cctgtgaacc tattctagca 7740
   catctgtcac ctctgtgcca ctcacaggga taccacccct gagaccccta ccccatcagc 7800
   ctctgtgtgg gatatggtgt tgggcccaag ggctctgttg cacagggaga tagaggcctg 7860
   gggaggaggg aaagcattga ggtggtgttg ataccaggga tgtgagccca agcaagagat 7920
   cagcagagca aggaggaaga attgcaggtg ttggggctgg ggaaagcccc agatggctgg 7980
   agctggtggg gccactggag atctcctcct cccatcctgc tccatgctgg ggcagctgct 8040
   gcaggctgac cagggcctgc ccgggcacgt tgtgaaggtc accaaggatg gagacttcag 8100
   agctatctgg gacacgtgtt ttaacgtttg atcagccttg cagcaaaaca gtgttttgct 8160
   ctattgaaca gtgttcaaca aaaacgatca gtggcctgga acacatctcc tacgaagaaa 8220
   ggttgcgaga gctgggtctg gagaagagaa ggctctgggg caacctccct gtggcctgtc 8280
   agtgcttaaa ggaggctcaa gagaaagatg gagagagact ttatacacgg agctgtagca 8340
   atatgacaag gagaaatggt ttaaaatgga aagagggtag atttaggtta gctatcaaga 8400
   agaaattctt cactcagagg tgatgaagca ctgtagcagg ttgcccagag aagtattgga 8460
   tgcttccttc ctggcagggc ccaaggccag actggatggg ttctgggcag cctgttctgg 8520
   tgggagatgt ccctgcctgt ggcatggctt ggaaatgggt agcctttgag gtccctctca 8580
   gcccaaatca ttctatgttc ttgtggttcc ctgcatttca gcctttgcct gttgactctg 8640
   gtgccactgt tggacaattc tactcagctg cgtttcatcc ctgccccaaa gcagtaatcc 8700
   catggataag caatgctctg aggccttcac caccctcagg gctcctgctg accctagcgg 8760
   cctccctggc agcatgagcc ctttgttgtc cgcactgctg ctgccgaggg tcctgccagc 8820
   cccccagggc cttttctgcc caggcactgc cacctgctaa aacccagcct gcccatggca 8880
   gggcatcgca tgtctgaggg atggaacggg gcaagggcag tgctgcaggc agtggttcaa 8940
   gaaacgttta gatgttgtac tgagggatgt ggtttagtgg gaaatactgg tgataggtgg 9000
   atggttggac tggaggatct tagaggtctt ttccaacctt ggtgattatg tgattctatg 9060
   aagggagcca tagcctgctc ccatcatgcc ccgccagtcc ggctctcccg agcactttgg 9120
   ttccgcagca tgcggccgca agggcgaatt cgcggccgct aaattcaatt cgccctatag 9180
   tgagtcgtat tacaattcac tggccgtcgt tttacaacgt cgtgactggg aaaaccctgg 9240
   cgttacccaa cttaatcgcc ttgcagcaca tccccctttc gccagctggc gtaatagcga 9300
   agaggcccgc accgatcgcc cttcccaaca gttgcgcagc ctatacgtac ggcagtttaa 9360
   ggtttacacc tataaaagag agagccgtta tcgtctgttt gtggatgtac agagtgatat 9420
   tattgacacg ccggggcgac ggatggtgat ccccctggcc agtgcacgtc tgctgtcaga 9480
   taaagtctcc cgtgaacttt acccggtggt gcatatcggg gatgaaagct ggcgcatgat 9540
   gaccaccgat atggccagtg tgccggtctc cgttatcggg gaagaagtgg ctgatctcag 9600
   ccaccgcgaa aatgacatca aaaacgccat taacctgatg ttctggggaa tataaatgtc 9660
   aggcatgaga ttatcaaaaa ggatcttcac ctagatcctt ttcacgtaga aagccagtcc 9720
   gcagaaacgg tgctgacccc ggatgaatgt cagctactgg gctatctgga caagggaaaa 9780
   cgcaagcgca aagagaaagc aggtagcttg cagtgggctt acatggcgat agctagactg 9840
   ggcggtttta tggacagcaa gcgaaccgga attgccagct ggggcgccct ctggtaaggt 9900
   tgggaagccc tgcaaagtaa actggatggc tttcttgccg ccaaggatct gatggcgcag 9960
   gggatcaagc tctgatcaag agacaggatg aggatcgttt cgcatgattg aacaagatgg 10020
   attgcacgca ggttctccgg ccgcttgggt ggagaggcta ttcggctatg actgggcaca 10080
   acagacaatc ggctgctctg atgccgccgt gttccggctg tcagcgcagg ggcgcccggt 10140
   tctttttgtc aagaccgacc tgtccggtgc cctgaatgaa ctgcaagacg aggcagcgcg 10200
   gctatcgtgg ctggccacga cgggcgttcc ttgcgcagct gtgctcgacg ttgtcactga 10260
   agcgggaagg gactggctgc tattgggcga agtgccgggg caggatctcc tgtcatctca 10320
   ccttgctcct gccgagaaag tatccatcat ggctgatgca atgcggcggc tgcatacgct 10380
   tgatccggct acctgcccat tcgaccacca agcgaaacat cgcatcgagc gagcacgtac 10440
   tcggatggaa gccggtcttg tcgatcagga tgatctggac gaagagcatc aggggctcgc 10500
   gccagccgaa ctgttcgcca ggctcaaggc gagcatgccc gacggcgagg atctcgtcgt 10560
   gacccatggc gatgcctgct tgccgaatat catggtggaa aatggccgct tttctggatt 10620
   catcgactgt ggccggctgg gtgtggcgga ccgctatcag gacatagcgt tggctacccg 10680
   tgatattgct gaagagcttg gcggcgaatg ggctgaccgc ttcctcgtgc tttacggtat 10740
   cgccgctccc gattcgcagc gcatcgcctt ctatcgcctt cttgacgagt tcttctgaat 10800
   tattaacgct tacaatttcc tgatgcggta ttttctcctt acgcatctgt gcggtatttc 10860
   acaccgcatc aggtggcact tttcggggaa atgtgcgcgg aacccctatt tgtttatttt 10920
   tctaaataca ttcaaatatg tatccgctca tgagattatc aaaaaggatc ttcacctaga 10980
   tccttttaaa ttaaaaatga agttttaaat caatctaaag tatatatgag taaacttggt 11040
   ctgacagtta ccaatgctta atcagtgagg cacctatctc agcgatctgt ctatttcgtt 11100
   catccatagt tgcctgactc cccgtcgtgt agataactac gatacgggag ggcttaccat 11160
   ctggccccag tgctgcaatg ataccgcgag acccacgctc accggctcca gatttatcag 11220
   caataaacca gccagccgga agggccgagc gcagaagtgg tcctgcaact ttatccgcct 11280
   ccatccagtc tattaattgt tgccgggaag ctagagtaag tagttcgcca gttaatagtt 11340
   tgcgcaacgt tgttgccatt gctacaggca tcgtggtgtc acgctcgtcg tttggtatgg 11400
   cttcattcag ctccggttcc caacgatcaa ggcgagttac atgatccccc atgttgtgca 11460
   aaaaagcggt tagctccttc ggtcctccga tcgttgtcag aagtaagttg gccgcagtgt 11520
   tatcactcat ggttatggca gcactgcata attctcttac tgtcatgcca tccgtaagat 11580
   gcttttctgt gactggtgag tactcaacca agtcattctg agaatagtgt atgcggcgac 11640
   cgagttgctc ttgcccggcg tcaatacggg ataataccgc gccacatagc agaactttaa 11700
   aagtgctcat cattggaaaa cgttcttcgg ggcgaaaact ctcaaggatc ttaccgctgt 11760
   tgagatccag ttcgatgtaa cccactcgtg cacccaactg atcttcagca tcttttactt 11820
   tcaccagcgt ttctgggtga gcaaaaacag gaaggcaaaa tgccgcaaaa aagggaataa 11880
   gggcgacacg gaaatgttga atactcatac tcttcctttt tcaatattat tgaagcattt 11940
   atcagggtta ttgtctcatg accaaaatcc cttaacgtga gttttcgttc cactgagcgt 12000
   cagaccccgt agaaaagatc aaaggatctt cttgagatcc tttttttctg cgcgtaatct 12060
   gctgcttgca aacaaaaaaa ccaccgctac cagcggtggt ttgtttgccg gatcaagagc 12120
   taccaactct ttttccgaag gtaactggct tcagcagagc gcagatacca aatactgttc 12180
   ttctagtgta gccgtagtta ggccaccact tcaagaactc tgtagcaccg cctacatacc 12240
   tcgctctgct aatcctgtta ccagtggctg ctgccagtgg cgataagtcg tgtcttaccg 12300
   ggttggactc aagacgatag ttaccggata aggcgcagcg gtcgggctga acggggggtt 12360
   cgtgcacaca gcccagcttg gagcgaacga cctacaccga actgagatac ctacagcgtg 12420
   agctatgaga aagcgccacg cttcccgaag ggagaaaggc ggacaggtat ccggtaagcg 12480
   gcagggtcgg aacaggagag cgcacgaggg agcttccagg gggaaacgcc tggtatcttt 12540
   atagtcctgt cgggtttcgc cacctctgac ttgagcgtcg atttttgtga tgctcgtcag 12600
   gggggcggag cctatggaaa aacgccagca acgcggcctt tttacggttc ctggcctttt 12660
   gctggccttt tgctcacatg ttctttcctg cgttatcccc tgattctgtg gataaccgta 12720
   ttaccgcctt tgagtgagct gataccgctc gccgcagccg aacgaccgag cgcagcgagt 12780
   cagtgagcga ggaagcggaa g 12801
<210> 52
   <211> 13120
   <212> DNA
   <213> Artificial Sequence
<220>
   <223> Targeting vector
<400> 52
   cacctgacgc gccctgtagc ggcgcattaa gcgcggcggg tgtggtggtt acgcgcagcg 60
   tgaccgctac acttgccagc gccctagcgc ccgctccttt cgctttcttc ccttcctttc 120
   tcgccacgtt cgccggcttt ccccgtcaag ctctaaatcg ggggctccct ttagggttcc 180
   gatttagtgc tttacggcac ctcgacccca aaaaacttga ttagggtgat ggttcacgta 240
   gtgggccatc gccctgatag acggtttttc gccctttgac gttggagtcc acgttcttta 300
   atagtggact cttgttccaa actggaacaa cactcaaccc tatctcggtc tattcttttg 360
   atttataagg gattttgccg atttcggcct attggttaaa aaatgagctg atttaacaaa 420
   aatttaacgc gaattttaac aaaatattaa cgcttacaat ttccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg agcgcgcgta atacgactca ctatagggcg aattggctgg 660
   gtcagtcgaa gggggcggtc ggtccatcat ttgcggccgg gtgattttgg gggccgaaaa 720
   gtgggaattt ggggcccaat ttggggccca atttggggcc caatttgggt tttcgagggg 780
   ggattttttt agggggagat tttgggtccc cggaaaatgg tgattttggg tcaaaaatgg 840
   ggattttggg gtcgttttga ggtggggttt tttggggtag aaatggcggt ctgaggggaa 900
   aatgtcgttt tggggccatt ttgggccatt tgaggggaaa tttgggtcaa aaaatgacga 960
   ttttgggtcg ttttagggag gaaaatgaat ttagggcgat ttgagccaaa tttgggtcaa 1020
   aaatggcgat tttgagtcaa aaaatgatta ttttgggtca ttttagggag gaaaatgaat 1080
   ttagggagat tggacggcaa atttgggtca aaaaatggtg attttgggtc aaaaaatgat 1140
   tattttgggt cattttaggg agaaaaatga atttagggag atttgagggc aaatttgggt 1200
   caaaaaatgg tgattttggg tcaaaaaatg attattttgg gtcattttag ggagaaaaat 1260
   gaatttaggg agatttgagg gcaaatttgg gtcgaaaaat ggcgattttg ggtgaaaaat 1320
   ggacaatttt gggtcatttt agggttaaaa atgaatttag ggagattgga cggcaaattt 1380
   gggtcaaaaa atggtgattt ggggtcaaaa aataattatt ttgggtcatt ttagggataa 1440
   aaaatgaatt tagggagatt tgagggcaaa tttgggtcga aaaatggtga tttggggtga 1500
   aaaatggaca attttgggtc attttaggga taaaaaatga atttagggcg atttgagcca 1560
   aatttgggtc aaaaatggcg attttgagtc aaaaaatgat tattttgggt cattttaggg 1620
   ttaaaaatga atttagggag attggacggc aaatttgggt caaaaaatgg tgattttcgg 1680
   tcaaaaaatg attattttgg gtcattttag ggagaaaaat gaatttaggg agattggacg 1740
   gcaaatttgg gtcgaaaaat ggtgattttg ggtcaaaaat ggacaatttg gggtcatttt 1800
   agggataaaa atgaatttag ggagatttga gagcaaattt gggtctaaaa atgtcaattt 1860
   ggggtcaaaa atggtgattt tgggtcattt tagggatata aatgaatttt gggagatttg 1920
   agggcaaatt tgggtcgaaa aatggtgatt tggggtcaat ttagggagga aaatgaattt 1980
   aaggtgattt gacggcaaat ttgggttgaa aaatgacaat ttggggtcat tttaaagata 2040
   aaatgaattt agggcgattt gagggcaaat ttgggtcgaa aaatggtgat ttgggtcaaa 2100
   aaatgacaat tttgggtcat tttagggata taaatggact tagagcgatt tgagggcaaa 2160
   tttggttgaa aaaatggaca atttgggtca ttttagggat ataaatgaat ttagggagat 2220
   ttgatggcaa atttgggtcg aaaaatggtg atttgggtca aaaatggtga ttttggttga 2280
   aaaacagtca tttggggtca ttttagggat taaaatgaat ttcgggagat ttgagggcaa 2340
   atttgggtca aaaaatggtg attttgggtc aattagtgat aaaaaaatga atttagggtg 2400
   atttgagggc aaatttgggt caaaaaatga caatttgggg tttttttagg gaggagaatg 2460
   aatttaggga gttttgaggg caaatttggg tcaaaaaata gtgatttggg tcaaaagtgg 2520
   tgattttggt tgaaaaacag tcattttggg tcattttagg gagaaaaatg aatttcggga 2580
   gatttgaggg caaatttggg tcaaaaaatg acaattttgg gtcactttac gaattaaaat 2640
   gaatttaggg agatttgagg gcaaatttgg gtcaaaatat ggtgattttg ggtcaattta 2700
   gggaggaaaa tgaatttagg gcgatttgaa ggcaaatttg ggtcgaaaat ggtgattttg 2760
   ggtcaaaaaa tgatgatttt gggtcgtttt agggagaaaa tgaatttagg gagatttgag 2820
   ggcaaatttg ggtcaaaaaa tggtgatttt gggtcattgt agggataaaa atgaatttgg 2880
   ggcgatttga gggcaaattt gggtggaaaa tggacaattt tgggtcattt tagggagaaa 2940
   aatgaattta gggagatttg agggcaaatt tgggtcaaaa aatggtgatt tggggtcatt 3000
   ttcagaagaa aaaatgatta ttttccccac taaaaatgta tattttgggg ccaaatggtg 3060
   aaaaatggtg atttttaatc aaacgtcccc aaaattgggg aaatttcatc gatttgaccc 3120
   aaaattgagt tttttttccc tgttaaaaat gtacattttg gggtcaatcg ctgaaatgtt 3180
   cccacttttc acttctttgc ccccaaattt tgctgtccgg tgagaaatta attaattaat 3240
   gatcattaat tgatcgacaa ttaatggtta ttaattaatt aataggtcct cttttggtga 3300
   ctccttcgct tttggggcca aaagtccata aattggcccc aaaaaattaa cgctgagtaa 3360
   ttggattcca aagtattaat gataaacatt aaaagtgttt aattaataat gatattaaac 3420
   ataatttcgt ttttattatc gatttatcaa caacgatgaa cgataatact ttacaacaat 3480
   cgctaataat taattaatta tttaattaat taatttctaa taattacttc gcattatcgg     3540
   acacgagatg ttgtaattat taataattta attcctaata attagaagat tcgttgaaaa     3600
   tggtacttat ctttacaaat aatcacttct aataataatg attaataata gttaataaca     3660
   acaatgacga taatgacgat aatattaata atatgtgata tatttaatat aaaattcgta     3720
   ttaatatatt atatctacaa aatatgatat aaaatataat attttattta tatataacac     3780
   aatttattat tattatcatt attatcatta ttaatatcat cattattaac gttatcgaaa     3840
   tacttattta gaaataataa aaacggattt aatgatggca acaaaaatat tttattaatg     3900
   ttaaaaaaaa ataattaata atttccaaag attcgaattc ggggcaacga acggcactcg     3960
   ataattttta attaattaat agtttgaatt aatcggtact ttttaatcct ccattttgcc     4020
   cgaaatcgcc gttttttgcc ccaaattccc caccgcggcg ttaaaaacat aaagaaatta     4080
   agcatcaaaa gtgccctttt ttggggttgt tttgacccaa aaaaaaaatg gccgaattgg     4140
   gggcggccgt tttacggttg ggttcatttt gggttcaaaa cagccaaaaa tgggaacttt     4200
   gggtttcgaa aacaacaaca acaaaaaaac gggtttattt tgggctcatt ttgggtgttt     4260
   ttgggtcagg aggagaaaaa ataggaagtt tgagagcgaa acaacggccg cttttggggg     4320
   gaaaacggcc ctttttggtc aacggcgggg gaaaaaaaaa agcggagttt ttggggtgaa     4380
   aaagagcggt tttgggtaaa tttgggtttt ggggtaaaag tggaggattt tgggcaatgg     4440
   gagttaaaaa atgggtgttt ttatgggggt tcggtgcagt ttttcctgtt tgatgggggg     4500
   tttattaatc cggggggggg gattaatgag aattaataat gttaatagaa atatctggga     4560
   aattaatagc aattattaat tgttaatagt tattaatagt tctatatatc tcacatctac     4620
   gatacaatat aatatcgtta caatcatata gtcgatatat tacatataat tatcagtaat     4680
   aataataagt aacaataatt agcagtaatt aataataata attaatagta ttcgttaata     4740
   agattattga taataattaa gtagtagcga ttaatagaga tgggatttcg tgagaaatgg     4800
   accaaatttg ggccgttttg acccaaattt ttggtggttt tttttccgat tctttgtgaa     4860
   tttcgggtcg gattcatcag caattaatta cggttattag gggctattag aggcttttaa     4920
   ttgggattat tagagacttt taagcggatt tggggacttt taagtggatt ttatgattct     4980
   taagtggatt ttgggtggat tttaccgctt ttggacgtac gatcgggtac cataagaatg     5040
   cggccgccgc ccccctctaa ccccccatct cccccccttt tctctctccc cccccccccc     5100
   caaaccccac agcctccacc aagggcccat cggtcttccc cctggcaccc tcctccaaga     5160
   gcacctctgg gggcacagcg gccctgggct gcctggtcaa ggactacttc cccgaaccgg     5220
   tgacggtgtc gtggaactca ggcgccctga ccagcggcgt gcacaccttc ccggctgtcc     5280
   tacagtcctc aggactctac tccctcagca gcgtggtgac cgtgccctcc agcagcttgg     5340
   gcacccagac ctacatctgc aacgtgaatc acaagcccag caacaccaag gtggacaaga 5400
   gagttgagcc caaatcttgt gacaaaactc acacatgccc accgtgccca ggtaagtcac 5460
   tagaccagag ctccactccc gggagaatgg taagtgctgt aaacatccct gcactagagg 5520
   ataagccatg tacagatcca tttccatctc tcctcatcag cacctgaact cctgggggga 5580
   ccgtcagtct tcctcttccc cccaaaaccc aaggacaccc tcatgatctc ccggacccct 5640
   gaggtcacat gcgtggtggt ggacgtgagc cacgaagacc ctgaggtcaa gttcaactgg 5700
   tacgtggacg gcgtggaggt gcataatgcc aagacaaagc cgcgggagga gcagtacaac 5760
   agcacgtacc gtgtggtcag cgtcctcacc gtcctgcacc aggactggct gaatggcaag 5820
   gagtacaagt gcaaggtctc caacaaagcc ctcccagccc ccatcgagaa aaccatctcc 5880
   aaagccaaag gtgagagctg cagcctgact gcatgggggc tgggatgggc ataaggataa 5940
   aggtctgtgt ggacagcctt ctgcttcagc catgaccttt gtgtatgttt ctaccctcac 6000
   agggcagccc cgagaaccac aggtgtacac cctgccccca tcccgggagg agatgaccaa 6060
   gaaccaggtc agcctgacct gcctggtcaa aggcttctat cccagcgaca tcgccgtgga 6120
   gtgggagagc aatgggcagc cggagaacaa ctacaagacc acgcctcccg tgctggactc 6180
   cgacggctcc ttcttcctct atagcaagct caccgtggac aagagcaggt ggcagcaggg 6240
   gaacgtcttc tcatgctccg tgatgcatga ggctctgcac aaccactaca cgcagaagag 6300
   cctctccctg tccccgggta aatgagctca gcacccacaa aactctcagg tccaaagaga 6360
   cacccacact catctccatg cttcccttgt ataaataaag cacccagcaa tgcctgggac 6420
   catgtaaaac tgtcctggtt ctttccaagg tatagagcat agctcacagg ctgatatttc 6480
   tggccagggt tggaggacag ccttgtctat aggaagagaa tgaggttttt gcactgcagg 6540
   actcagagct cattagttat cctgccttgg agtgttgggg cttggcttta ggcagtgcct 6600
   tttccttgcc ttcctacgaa ccagcagctg ccatacatag agataatcct aggaagcctc 6660
   aaatggagaa ggacacaaac ccacctccct caggctgttc ctctatcccg gccccacttc 6720
   tttacctagg ggtttctctg agtctattgt ggagttacac atggccaggg gcattccaga 6780
   gacccttgtc atccatacac tcaactcagg cagctttgca caaacaaagt ctgcacaccc 6840
   atacagatgg ctcactcttg cctgtgccat gtagggctga ggcacatggc tcttgctgcc 6900
   ccaagggagg gactattaga tagccacact catgctgaat cctggcccat tcaaattagc 6960
   ctgctgaaca ccatccagtc catatagcac atgtatccac atgcacgtgt gcacaaaacg 7020
   catttaacac actgggacaa caattctgtg ccctgcacag cacctatatc cagcaatgta 7080
   tcaccataca cacgaccaaa aattcaatgc ccacgtttct gccatcacaa acagacacat 7140
   ctttcctctc tgtggccact gcattatatg ctcaacacaa gacctctgaa gccagatcca 7200
   tctctggcac ctcggggtca tgcttcaacc ccacatgaat tatgcaaacc atagccataa 7260
   tggtctgaat cacttcacac tgggatgttc ccaagttcag gcaagacgag ccacaggctc 7320
   tgctgatgac tgaaggacag caaagggtca gtccagctgt atagccactg ttgacctggg 7380
   tcacaggccc tgctgaccct ccaccttctc ctgtactgaa ggaatgaaag atgagacaag 7440
   catagagggc acttgaataa tccaggtcac tctgaggtcc acccaaggca ttattggact 7500
   caggtgggaa gctgagactg gtgtcccaga gggaaaggaa ggaaagcagg ccccggggag 7560
   ggtctgctgt cccagtcagg ctggagatct ctcctctgaa tccatgcaga catgtctgcc 7620
   tcacagggaa tctctcccag caccaaccat gttgggacaa acactgactg tcctctctgt 7680
   tcagagctgc aactggagga gagctgtgcg gaggcgcagg acggggagct ggacgggctg 7740
   tggacgacca tcaccatctt catcacactc ttcctgttaa gcgtgtgcta cagtgccacc 7800
   gtcaccttct tcaaggtgaa gtggatcttc tcctcggtgg tggacctgaa gcagaccatc 7860
   atccccgact acaggaacat gatcggacag ggggcctagg aattaattcg ctgtctgcga 7920
   gggccagctg ttggggtgag tactccctct caaaagcggg catgacttct gcgctaagat 7980
   tgtcagtttc caaaaacgag gaggatttga tattcacctg gcccgcggtg atgcctttga 8040
   gggtggccgc gtccatctgg tcagaaaaga caatcttttt gttgtcaagc ttgaggtgtg 8100
   gcaggcttga gatctggcca tacacttgag tgacaatgac atccactttg cctttctctc 8160
   cacaggtgtc cactcccagg tccaactgca ggtcgagcat gcatctaggg cggccaattg 8220
   gatcctaccg ttcgtataat gtatgctata cgaagttatc gtacgcttaa ttaactgatc 8280
   cggaattccg cccccccccc ccccccccct ctccctcccc cccccctaac gttactggcc 8340
   gaagccgctt ggaataaggc cggtgtgcgt ttgtctatat gttattttcc accatattgc 8400
   cgtcttttgg caatgtgagg gcccggaaac ctggccctgt cttcttgacg agcattccta 8460
   ggggtctttc ccctctcgcc aaaggaatgc aaggtctgtt gaatgtcgtg aaggaagcag 8520
   ttcctctgga agcttcttga agacaaacaa cgtctgtagc gaccctttgc aggcagcgga 8580
   accccccacc tggcgacagg tgcctctgcg gccaaaagcc acgtgtataa gatacacctg 8640
   caaaggcggc acaaccccag tgccacgttg tgagttggat agttgtggaa agagtcaaat 8700
   ggctctcctc aagcgtattc aacaaggggc tgaaggatgc ccagaaggta ccccattgta 8760
   tgggatctga tctggggcct cggtgcacat gctttacatg tgtttagtcg aggttaaaaa 8820
   aacgtctagg ccccccgaac cacggggacg tggttttcct ttgaaaaaca cgatgataag 8880
   cttgccacaa ccatggctga acaagatgga ttgcacgcag gttctccggc cgcttgggtg 8940
   gagaggctat tcggctatga ctgggcacaa cagacaatcg gctgctctga tgccgccgtg 9000
   ttccggctgt cagcgcaggg gcgcccggtt ctttttgtca agaccgacct gtccggtgcc 9060
   ctgaatgaac tgcaggacga ggcagcgcgg ctatcgtggc tggccacgac gggcgttcct 9120
   tgcgcagctg tgctcgacgt tgtcactgaa gcgggaaggg actggctgct attgggcgaa 9180
   gtgccggggc aggatctcct gtcatctcac cttgctcctg ccgagaaagt atccatcatg 9240
   gctgatgcaa tgcggcggct gcatacgctt gatccggcta cctgcccatt cgaccaccaa 9300
   gcgaaacatc gcatcgagcg agcacgtact cggatggaag ccggtcttgt cgatcaggat 9360
   gatctggacg aagagcatca ggggctcgcg ccagccgaac tgttcgccag gctcaaggcg 9420
   cgcatgcccg acggcgagga tctcgtcgtg acccatggcg atgcctgctt gccgaatatc 9480
   atggtggaaa atggccgctt ttctggattc atcgactgtg gccggctggg tgtggcggac 9540
   cgctatcagg acatagcgtt ggctacccgt gatattgctg aagagcttgg cggcgaatgg 9600
   gctgaccgct tcctcgtgct ttacggtatc gccgctcccg attcgcagcg catcgccttc 9660
   tatcgccttc ttgacgagtt cttctgaata acttcgtata atgtatgcta tacgaacggt 9720
   aggatcccgc ccgccccacg acccgcagcg cccgaccgaa aggagcgcac gaccccatgg 9780
   ctccgaccga agccacccgg ggcggccccg ccgaccccgc acccgccccc gaggcccacc 9840
   gactctagat aactgatcat aatcagccat accacatttg tagaggtttt acttgcttta 9900
   aaaaacctcc cacacctccc cctgaacctg aaacataaaa tgaatgcaat tgttgttgtt 9960
   aacttgttta ttgcagctta taatggttac aaataaagca atagcatcac aaatttcaca 10020
   aataaagcat ttttttcact gtatgcaaac cacctcctgg tgtcccccaa cccttcatag 10080
   ccccccaccc cgtctcggtc gcccacccaa ccctccacat taaccccttc ctccccacgc 10140
   tcgtcccccg attcccaccc cccttttcgt caccatgcac cccccgtccc gcgaggactt 10200
   cgaaggcccc ttccgcaacg cctccatcct ctgccagacc cgcgggcgcc gccgtcccgc 10260
   cgaggtcacg tggtacaaaa atggcagccc cgtcgccgcc gccgccgcca ccaccgccac 10320
   caccgtcggc cccgaagtgg tggccgagag ccgcatcagc gtcaccgaaa gcgaatggga 10380
   caccggggcc accttcagct gcgtcgtgga gggggagatg aggaacacca gcaagaggat 10440
   ggagtgcgga tgtaagtgga gggggggttt tttggggggg gatttggggg gaaattgggg 10500
   gctttggggg caaaaagtcc gtcattttgg tttggaaggg gtcgatgtgg gggaaaatgt 10560
   gccaaaaatc ccaattttgg gggcactgaa gtcgtaattt gaggtggagg aaggtagaca 10620
   gaaagagggt ggagtgcgga tgtaagtgga gggggatttg gggcagattt gggggaaatt 10680
   gggggtttgg ggggcaaata atccgtagtt tcggtttgga aggggttgat gtggttttgt 10740
   gctgaatttc gggggaaaaa ctgccaaaaa tcccaatttt gggggcattg aagtcatagt 10800
   ttgaggttgg aaggaggtag agagcaagag gatgaagtga ggatgtaagt cgggggattt 10860
   ttggggcaga tttgggggaa attgggggtt ttggggtcag agaatccgta ggtcgaccgc 10920
   gccagctttt gttcccttta gtgagggtta attgcgcgct tggcgtaatc atggtcatag 10980
   ctgtttcctg tgtgaaattg ttatccgctc acaattccac acaacatacg agccggaagc 11040
   ataaagtgta aagcctgggg tgcctaatga gtgagctaac tcacattaat tgcgttgcgc 11100
   tcactgcccg ctttccagtc gggaaacctg tcgtgccagc tgcattaatg aatcggccaa 11160
   cgcgcgggga gaggcggttt gcgtattggg cgctcttccg cttcctcgct cactgactcg 11220
   ctgcgctcgg tcgttcggct gcggcgagcg gtatcagctc actcaaaggc ggtaatacgg 11280
   ttatccacag aatcagggga taacgcagga aagaacatgt gagcaaaagg ccagcaaaag 11340
   gccaggaacc gtaaaaaggc cgcgttgctg gcgtttttcc ataggctccg cccccctgac 11400
   gagcatcaca aaaatcgacg ctcaagtcag aggtggcgaa acccgacagg actataaaga 11460
   taccaggcgt ttccccctgg aagctccctc gtgcgctctc ctgttccgac cctgccgctt 11520
   accggatacc tgtccgcctt tctcccttcg ggaagcgtgg cgctttctca tagctcacgc 11580
   tgtaggtatc tcagttcggt gtaggtcgtt cgctccaagc tgggctgtgt gcacgaaccc 11640
   cccgttcagc ccgaccgctg cgccttatcc ggtaactatc gtcttgagtc caacccggta 11700
   agacacgact tatcgccact ggcagcagcc actggtaaca ggattagcag agcgaggtat 11760
   gtaggcggtg ctacagagtt cttgaagtgg tggcctaact acggctacac tagaaggaca 11820
   gtatttggta tctgcgctct gctgaagcca gttaccttcg gaaaaagagt tggtagctct 11880
   tgatccggca aacaaaccac cgctggtagc ggtggttttt ttgtttgcaa gcagcagatt 11940
   acgcgcagaa aaaaaggatc tcaagaagat cctttgatct tttctacggg gtctgacgct 12000
   cagtggaacg aaaactcacg ttaagggatt ttggtcatga gattatcaaa aaggatcttc 12060
   acctagatcc ttttaaatta aaaatgaagt tttaaatcaa tctaaagtat atatgagtaa 12120
   acttggtctg acagttacca atgcttaatc agtgaggcac ctatctcagc gatctgtcta 12180
   tttcgttcat ccatagttgc ctgactcccc gtcgtgtaga taactacgat acgggagggc 12240
   ttaccatctg gccccagtgc tgcaatgata ccgcgagacc cacgctcacc ggctccagat 12300
   ttatcagcaa taaaccagcc agccggaagg gccgagcgca gaagtggtcc tgcaacttta 12360
   tccgcctcca tccagtctat taattgttgc cgggaagcta gagtaagtag ttcgccagtt 12420
   aatagtttgc gcaacgttgt tgccattgct acaggcatcg tggtgtcacg ctcgtcgttt 12480
   ggtatggctt cattcagctc cggttcccaa cgatcaaggc gagttacatg atcccccatg 12540
   ttgtgcaaaa aagcggttag ctccttcggt cctccgatcg ttgtcagaag taagttggcc 12600
   gcagtgttat cactcatggt tatggcagca ctgcataatt ctcttactgt catgccatcc 12660
   gtaagatgct tttctgtgac tggtgagtac tcaaccaagt cattctgaga atagtgtatg 12720
   cggcgaccga gttgctcttg cccggcgtca atacgggata ataccgcgcc acatagcaga 12780
   actttaaaag tgctcatcat tggaaaacgt tcttcggggc gaaaactctc aaggatctta 12840
   ccgctgttga gatccagttc gatgtaaccc actcgtgcac ccaactgatc ttcagcatct 12900
   tttactttca ccagcgtttc tgggtgagca aaaacaggaa ggcaaaatgc cgcaaaaaag 12960
   ggaataaggg cgacacggaa atgttgaata ctcatactct tcctttttca atattattga 13020
   agcatttatc agggttattg tctcatgagc ggatacatat ttgaatgtat ttagaaaaat 13080
   aaacaaatag gggttccgcg cacatttccc cgaaaagtgc 13120
<210> 53
   <211> 2449
   <212> DNA
   <213> Homo sapiens
<400> 53
   ctggcccatc ctctctgtta gatgtgatca aaactcataa aggctgtccc tgaggtttct       60
   gtcccaggac tgattgcgga agagtcacca ggcactgagg agacggtgac cagggtgccc      120
   tggccccaga cgtccatacc gtagtagtag tagtaggttc ctttcgcaca gtaatatacg      180
   gccgtgtcct cggctctcag gctgttcatt tgcagataca gcgtgttctt ggaattgtct      240
   ctggagatgg tgaaccggcc ctggaacttc tgtgcgtaga ttgtttcacc atcttcagga      300
   tcaaaacctg agacccactc cagccccttc cctggagcct ggcggaccca gtgcatggat      360
   aattcgctaa aggtgaatcc agaggctgca caggagagtc tcagggaccc cccaggctgt      420
   accaagcctc ccccagactc caacagctgc acctcgtact agacgtagta cgtgatacgt      480
   cgtatctggc ccatcctctc tgttagatgt gatcaaaact cataaaggct gtccctgagg      540
   tttctgtccc aggactgatt gcggaagagt caccaggcac tgaggagacg gtgaccaggg      600
   tgccctggcc ccagtagtca aagtagtaat aggataacct taaatctttc gcacagtaat      660
   atacggccgt gtcctcggct ctcaggctgt tcatttgcag atacagcgtg ttcttggaat      720
   tgtctctgga gatggtgaac cggccctgga gcttctgtgc atagtttgtg ttaccattgt      780
   aagcgctgat ccatgagacc cactccagcc ccttccctgg agcctggcgg acccagctga      840
   taccatagct gctaaaggtg aatccagagg ctgcacagga gagtctcagg gaccccccag      900
   gctgtaccaa gcctccccca gactccaaca gctgcacctc gtatctctcg cactactatg      960
   agacgtatca ctggcccatc ctctctgtta gatgtgatca aaactcataa aggctgtccc     1020
   tgaggtttct gtcccaggac tgattgcgga agagtcacca ggcacgaggt gcagctgttg     1080
   gagtctgggg gaggcttggt acagcctggg gggtccctga gactctcctg tgcagcctct     1140
   ggattcacct ttagcagtta tgatatcaac tgggtccgcc aggctccagg gaaggggctg     1200
   gagtgggtct catggatgaa ccctaacagt ggtaacacag gctatgcaca gaagttccag     1260
   ggccggttca ccatctccag agacaattcc aagaacacgc tgtatctgca aatgaacagc     1320
   ctgagagccg aggacacggc cgtatattac tgtgcgaaat ccagcagctg gcactactac     1380
   tacggtatgg acgtctgggg ccagggcacc ctggtcaccg tctcctcacg catagagaga     1440
   cacgcgagta gagacacgga attcctggcc catcctctct gttagatgtg atcaaaactc     1500
   ataaaggctg tccctgaggt ttctgtccca ggactgattg cggaagagtc accaggcact     1560
   gaggagacgg tgaccagggt gccctggccc cagtagtcaa agtggacgta gtaataacca     1620
   ctactagacg ctttcgcaca gtaatatacg gccgtgtcct cggctctcag gctgttcatt     1680
   tgcagataca gcgtgttctt ggaattgtct ctggagatgg tgaaccggcc ctggaacttc     1740
   tgtgaatatt ttgtgttacc attgccagcg ttgatccatg agacccactc cagccccttc     1800
   cctggagcct ggcggaccca atgcatagca tagctgctaa aggtgaatcc agaggctgca     1860
   caggagagtc tcagggaccc cccaggctgt accaagcctc ccccagactc caacagctgc    1920
   acctcctcgc gatatcgtgt ctgatctaca gtgctctggc ccatcctctc tgttagatgt     1980
   gatcaaaact cataaaggct gtccctgagg tttctgtccc aggactgatt gcggaagagt     2040
   caccaggcac tgaggagacg gtgaccaggg tgccctggcc ccaggggtcg aaccagttga     2100
   gggtagtcac cgtatagccc caacctttcg cacagtaata tacggccgtg tcctcggctc     2160
   tcaggctgtt catttgcaga tacagcgtgt tcttggaatt gtctctggag atggtgaacc     2220
   ggccctgaaa cttctgtgca tagtttgtgc caccactgtt agggttgatc catgagaccc     2280
   actccagccc cttccctgga gcctggcgga cccagtgcat atagtagccg ctaaaggtga     2340
   atccagaggc tgcacaggag agtctcaggg accccccagg ctgtaccaag cctcccccag     2400
   actccaacag ctgcacctcg tatgtacagg agcgtagagg agtgactag                 2449
<210> 54
   <211> 2581
   <212> DNA
   <213> Gallus gallus
<400> 54 g 2581
<210> 55
   <211> 13244
   <212> DNA
   <213> Artificial Sequence
<220>
   <223> Targeting vector
<400> 55
   agcgtgaccg ctacacttgc cagcgcccta gcgcccgctc ctttcgcttt cttccttcct      60
   ttctcgccac gttcgccggc tttccccgtc aagctctaaa tcgggggctc cctttagggt     120
   tccgatttag tgctttacgg cacctcgacc ccaaaaaact tgattagggt gatggttcac     180
   gtagtgggcc atcgccctga tagacggttt ttcgcccttt gacgttggag tccacgttct     240
   ttaatagtgg actcttgttc caaactggaa caacactcaa ccctatctcg gtctattctt     300
   ttgatttata agggattttg ccgatttcgg cctattggtt aaaaaaatga gctgatttaa      360
   caaaaattta acgcgaattt taacaaaata ttaacgctta caatttccat tcgccattca     420
   ggctgcgcaa ctgttgggaa gggcgatcgg tgcgggcctc ttcgctatta cgccagctgg     480
   cgaaaggggg atgtgctgca aggcgattaa gttgggtaac gccagggttt tcccagtcac     540
   gacgttgtaa aacgacggcc agtgagcgcg cgtaatacga ctcactatag ggcgaattgg     600
   gtaccataag aatgcggccg cttcccgaag cgaaagccgc gtgttatttt tgggtgggaa     660
   agccccgatt cgctcctttt tttttattta ttatttttgt tgtttttcgt gtctttttct     720
   tctccgtttt gcagctgcgg cctccaggtg tccccggccg tggtattttt gggtcgaaaa     780
   agccgctttt tggctcactt ggaaaccttt cgggcgtctc caagcggagc gttggcggga     840
   aatggtgttt tttttgggtt gttttcggtc ccttttttca ccatttttgt ggtttttcgg     900
   cccattttcg gcctcccccc cccccccccc cttcccgtgg ggctgcgctc cgtggagcgg     960
   aatggggtcg ttaattaagg ttaattggtt taattaatgg gccgtgcagg gagaagaggg    1020
   ccgtgcggtg gttgttagcc ctgattaatg gcttggttga ggggagagtg taattaagtg    1080
   cttcgttagc actaattaat ggcttgattg agaggagagt gtaattaaat gcttcataat    1140
   ccctaattaa tggcttggtt gagacaagag tgtaattaag tgcttcatta gccctgatta    1200
   atggcttggt tgaggggaga gtgtaattaa atgcttcgtt agcactaatt aatggcttga    1260
   ttgagaggag agtgtaatta aatgcttcat aatccctaat taatggcttg gttgagacaa    1320
   gagtgtaatt aagtgcttca ttagccctga ttaatggctt ggttgagagg agagtgtaat    1380
   taaatgcttc attatcccta attagggact gaattccaag gagagtgtaa ttaagtgctt    1440
   cattatccct aattaatggc ttggttgaga ggagagtgta attaaatgct ttgttagccc    1500
   taattaatgg tttgatggag aggagagtgt aattaagtgc ttcattatcc ctaattaatg 1560
   gcttggttga ggggagagtg taattaaatg cttagttagc cctaattaat ggcttggttg 1620
   agaggagagt gtaattgaat gcttcattat ccctaattag ggattgaatt gagaggagag 1680
   tgtaattgaa tgctttgtta gccctaatta gggattgaat tgagaggaga gtgtaattaa 1740
   atgcctcatt atccctaatt agggattgaa ttgagaggag agtttaatta aatgcctcat 1800
   tatccctaat tagggactga attccgacga gagtgtaatt aagtgcttca tttagcccta 1860
   attaatggct tgattgagag gagagtgtaa ttaaatgctt cattatccct aattagggat 1920
   tgaattgaga ggagagtgta attaaatgct tcattatccc taattaggga ttgaattgag 1980
   aggagagtgt aattaaatgc ttcattatcc ctaattaatg gcttggttga gaggggagtg 2040
   taattaagtg cttcgttatc cctaattagg gattgaattg tgaggagatg gcaattaata 2100
   cttcattatc cctaattaat ttaattagtc cctattatcc ctaatttaat tcatggtttg 2160
   gttgatggaa gaatgtaatt aaatgctttg ttatccctaa ttaatggttt gattgagcgg 2220
   agagtgtaat taaatgcttc attatccctt attaatggtt gtgtggaatg tagtgtaatt 2280
   agaagtccct aattaatggt ttaattgaga gaagactgca attagatgac tcatagcccc 2340
   taataagtgg tttaattatg agataatcac aattacatgt ttcattgtcc ctaattaatc 2400
   gtttaattgc gagaagatca caattaatta aatgatccct aatttcaatt tctgagaagt 2460
   gtctttctcg gaaattgaga tatcataaga attcgcgatc aatttctgag aactgtcatt 2520
   ctcggaatac ctactagtat aagaatggtc atcatcagtc gacctactac tactcgagct 2580
   ggcccatcct ctctgttaga tgtgatcaaa actcataaag gctgtccctg aggtttctgt 2640
   cccaggactg attgcggaag agtcaccagg cactgaggag acggtgacca gggtgccctg 2700
   gccccagacg tccataccgt agtagtagta gtaggttcct ttcgcacagt aatatacggc 2760
   cgtgtcctcg gctctcaggc tgttcatttg cagatacagc gtgttcttgg aattgtctct 2820
   ggagatggtg aaccggccct ggaacttctg tgcgtagatt gtttcaccat cttcaggatc 2880
   aaaacctgag acccactcca gccccttccc tggagcctgg cggacccagt gcatggataa 2940
   ttcgctaaag gtgaatccag aggctgcaca ggagagtctc agggaccccc caggctgtac 3000
   caagcctccc ccagactcca acagctgcac ctcgtactag acgtagtacg tgatacgtcg 3060
   tatctggccc atcctctctg ttagatgtga tcaaaactca taaaggctgt ccctgaggtt 3120
   tctgtcccag gactgattgc ggaagagtca ccaggcactg aggagacggt gaccagggtg 3180
   ccctggcccc agtagtcaaa gtagtaatag gataacctta aatctttcgc acagtaatat 3240
   acggccgtgt cctcggctct caggctgttc atttgcagat acagcgtgtt cttggaattg 3300
   tctctggaga tggtgaaccg gccctggagc ttctgtgcat agtttgtgtt accattgtaa 3360
   gcgctgatcc atgagaccca ctccagcccc ttccctggag cctggcggac ccagctgata 3420
   ccatagctgc taaaggtgaa tccagaggct gcacaggaga gtctcaggga ccccccaggc 3480
   tgtaccaagc ctcccccaga ctccaacagc tgcacctcgt atctctcgca ctactatgag 3540
   acgtatcact ggcccatcct ctctgttaga tgtgatcaaa actcataaag gctgtccctg 3600
   aggtttctgt cccaggactg attgcggaag agtcaccagg cacgaggtgc agctgttgga 3660
   gtctggggga ggcttggtac agcctggggg gtccctgaga ctctcctgtg cagcctctgg 3720
   attcaccttt agcagttatg atatcaactg ggtccgccag gctccaggga aggggctgga 3780
   gtgggtctca tggatgaacc ctaacagtgg taacacaggc tatgcacaga agttccaggg 3840
   ccggttcacc atctccagag acaattccaa gaacacgctg tatctgcaaa tgaacagcct 3900
   gagagccgag gacacggccg tatattactg tgcgaaatcc agcagctggc actactacta 3960
   cggtatggac gtctggggcc agggcaccct ggtcaccgtc tcctcacgca tagagagaca 4020
   cgcgagtaga gacacggaat tcctggccca tcctctctgt tagatgtgat caaaactcat 4080
   aaaggctgtc cctgaggttt ctgtcccagg actgattgcg gaagagtcac caggcactga 4140
   ggagacggtg accagggtgc cctggcccca gtagtcaaag tggacgtagt aataaccact 4200
   actagacgct ttcgcacagt aatatacggc cgtgtcctcg gctctcaggc tgttcatttg 4260
   cagatacagc gtgttcttgg aattgtctct ggagatggtg aaccggccct ggaacttctg 4320
   tgaatatttt gtgttaccat tgccagcgtt gatccatgag acccactcca gccccttccc 4380
   tggagcctgg cggacccaat gcatagcata gctgctaaag gtgaatccag aggctgcaca 4440
   ggagagtctc agggaccccc caggctgtac caagcctccc ccagactcca acagctgcac 4500
   ctcctcgcga tatcgtgtct gatctacagt gctctggccc atcctctctg ttagatgtga 4560
   tcaaaactca taaaggctgt ccctgaggtt tctgtcccag gactgattgc ggaagagtca 4620
   ccaggcactg aggagacggt gaccagggtg ccctggcccc aggggtcgaa ccagttgagg 4680
   gtagtcaccg tatagcccca acctttcgca cagtaatata cggccgtgtc ctcggctctc 4740
   aggctgttca tttgcagata cagcgtgttc ttggaattgt ctctggagat ggtgaaccgg 4800
   ccctgaaact tctgtgcata gtttgtgcca ccactgttag ggttgatcca tgagacccac 4860
   tccagcccct tccctggagc ctggcggacc cagtgcatat agtagccgct aaaggtgaat 4920
   ccagaggctg cacaggagag tctcagggac cccccaggct gtaccaagcc tcccccagac 4980
   tccaacagct gcacctcgta tgtacaggag cgtagaggag tgactaggaa ttcctactac 5040
   tagcaatgtt ggcgcagaag ggattcatat caattaattc cctgtcctca attaatgcat 5100
   aattgggagt gcgatggaat cattcaataa gtaatgagga cttggtgtca aaatgccaat 5160
   taagtaatta atgacaccca attaatacac aatggggatt acagtggaat aactcagtga 5220
   agttcatatc taaattgcaa ttgaataatt aattacagct aattaatgga tgggagactg 5280
   gcagtggtat aacttaatga aggaacacag tgaattcctg tctgaattga aattaattga 5340
   tttattaggg ctaattaatg aatggtgggg ttaaaaatag cattactcag cgaaggaaca 5400
   tgagttcatg gctaaattga gattgaatta ttaattacaa ctaattgatt aatgactgat 5460
   gggagtaaga atggaataac tcagtgaagg aacacaatgg attcatggcc taattgaaat 5520
   taaatggtta attagagcta attaatgaat gatggggggt ggcaatggaa tgatacaaga 5580
   aagaaacaca atgggttcgt gtctacattg aaattaaata attaattaca gctaattaat 5640
   aaacggcgag gttgacaatg gtataactgt gaagagacag aatgagttcg tgactaaatt 5700
   ggtattaaat gattaattag agctaattaa caggtggtgg ggctgagaat ggcataactc 5760
   aatgggggat gatgagctga tggcttaatt ggaattaata attaatcaca accaattaac 5820
   aggtgctgtt aatggggtta caggggcatg acttcaggaa gaagcacaaa gtggccatta 5880
   aataattaat tgcacccaat taattcctga tggggattac tgcagaggaa ctcagcgaag 5940
   gattcaagca gaaatgacac agttaggttg ctcaattgcg ttaattaatt aaaatcgcta 6000
   attaggtgag cttcattaag gcctgatggg gttcatccca acgctgggat caaagaactc 6060
   agcgcagagt ggataacgat aattagaagg ggacaaatta atgaggaaac acgactttgg 6120
   gcctagacgg ccattaaatc attaatcaac ccgacgggcg cccaattaat cataaagtta 6180
   ttaagcaatt aagtcgaggc tgacgagaaa ccgcaacgag agccctccac gctcctttga 6240
   gttggcgcaa acaacaaaca acgcgggctc gttaagcagc gacgacgccg agcgtaacga 6300
   agcaatcccc gcgcccgaca attaacgcgc taacgaagcc ggagccctcc ttatgcaaat 6360
   tagcccctcc agagggccat aaaagcgccg gctctccgac ggcgcgggag gagcaccagt 6420
   cggctccgca accatgagcc cactcgtctc ctccctcctg ctcctggccg ccctgccagg 6480
   tgagggcgct gtggggctct atggggctct atggggtctc agcggggctc tgcgggctca 6540
   atgggggcca aagggggggt ctgcgggctc tatggggggg tcaacggggg gtctcacggg 6600
   gggccggctc cgcgaggccg tgtggcggcg gctccgtcag cgctctctgg gcgcgcctcc 6660
   ttccccacag ggctgatggc ggaggtgcag ctgttggagt ctgggggagg cttggtacag 6720
   cctggggggt ccctgagact ctcctgtgca gcctctggat tcacctttag cagctatgcc 6780
   atgagctggg tccgccaggc tccagggaag gggctggagt gggtctcagc tattagtggt 6840
   agtggtggta gcacatacta cgcagactcc gtgaagggcc ggttcaccat ctccagagac 6900
   aattccaaga acacgctgta tctgcaaatg aacagcctga gagccgagga cacggccgta 6960
   tattactgtg cgaaaggata tagtggctac gattactact ttgactactg gggccagggc 7020
   accctggtca ccgtctcctc aggtgagtct tcatatgaac cccccccaaa attgccgcgg 7080
   cgattttggg gcaaaatcgg gcgattttat ggatccccta ccgttcgtat aatgtatgct 7140
   atacgaagtt atagatccag acatgataag atacattgat gagtttggac aaaccacaac 7200
   tagaatgcag tgaaaaaaat gctttatttg tgaaatttgt gatgctattg ctttatttgt 7260
   aaccattata agctgcaata aacaagttaa caacaacaat tgcattcatt ttatgtttca 7320
   ggttcagggg gaggtgtggg aggtttttta aagcaagtaa aacctctaca aatgtggtat 7380
   ggctgattat gatctctagt caaggcacta tacatcaaat attccttatt aaccccttta 7440
   caaattaaaa agctaaaggt acacaatttt tgagcatagt tattaatagc agacactcta 7500
   tgcctgtgtg gagtaagaaa aaacagtatg ttatgattat aactgttatg cctacttata 7560
   aaggttacag aatatttttc cataattttc ttgtatagca gtgcagcttt ttcctttgtg 7620
   gtgtaaatag caaagcaagc aagagttcta ttactaaaca cagcatgact caaaaaactt 7680
   agcaattctg aaggaaagtc cttggggtct tctacctttc tcttcttttt tggaggagta 7740
   gaatgttgag agtcagcagt agcctcatca tcactagatg gcatttcttc tgagcaaaac 7800
   aggttttcct cattaaaggc attccaccac tgctcccatt catcagttcc ataggttgga 7860
   atctaaaata cacaaacaat tagaatcagt agtttaacac attatacact taaaaatttt 7920
   atatttacct tagagcttta aatctctgta ggtagtttgt ccaattatgt cacaccacag 7980
   aagtaaggtt ccttcacaaa gatccggacc aaagcggcca tcgtgcctcc ccactcctgc 8040
   agttcggggg catggatgcg cggatagccg ctgctggttt cctggatgcc gacggatttg 8100
   cactgccggt agaactccgc gaggtcgtcc agcctcaggc agcagccaag cttggtatgg 8160
   taaaactttt aatttcgggt atatttgagt ggaatgagtt cttcaatcgt agttttgact 8220
   aacttgccat tcatttctat taacacaaaa caatctggtg catagtctga aatcaactcc 8280
   ctacacatac cacaaggact taccactcga atacttctat ctacttcgtc agaataaggg 8340
   tgtctaacag ctacaatcgt gtcaaaatcc ttttgtccat tcgaaactgc actaccaatc 8400
   gcaatggctt ctgcacaaac agttactcgt cctatatacg cttcaatatg tactgccgaa 8460
   atgatttctc ctgttttcgt acgaattgcc gctcccacat gatgtttatt atcctcataa 8520
   agcattgtaa tcttctctgt cgctacttct actaattcta gatcttgttg agaaatgtta 8580
   aatgttttca tatattcttc ttcctccgat ttctgcaaaa atgaatccaa gcttgaatta 8640
   attctttgcc aaaatgatga gacagcacaa taaccagcac gttgcccagg agtaggaaaa 8700
   agaagaaggc atgaacatgg ttagcagagg ctctagagcc gccggtcaca cgccagaagc 8760
   cgaaccccgc cctgccccgt cccccccgaa ggcagccgtc cccccgcgga cagccccgag 8820
   gctggagagg gagaagggga cggcggcgcg gcgacgcacg aaggccctcc ccgcccattt 8880
   ccttcctgcc ggcgccgcac cgcttcgccc cgcgcccgct agagggggtg cggcggcgcc 8940
   tcccagattt cggctccgca cagatttggg acaaaggaag tccctgcgcc ctctcgcacg 9000
   attaccataa aaggcaatgg ctgcggctcg ccgcgcctcg acagccgccg gcgctccggg 9060
   ggccgccgcg cccctccccc gagccctccc cggcccgagg cggccccgcc ccgcccggca 9120
   cccccacctg ccgccacccc ccgcccggca cggcgagccc cgcgccacgc cccgtacgga 9180
   gccccgcacc cgaagccggg ccgtgctcag caactcgggg aggggggtgc acgggggggt 9240
   tgcagcccga ccgacgcgcc cacmccccct gctcaccccc ccacgcacac accccgcacg 9300
   cagcctttgt tcccctcgca gccccccccg caccgcgggg caccgccccc ggccgcgctc 9360
   cctcgcgcac actgcggagc gcacaaagcc ccgcgccgcg cccgcagcgc tcacagccgc 9420
   cgggcagcgc ggagccgcac gcggcgctcc ccacgcacac acacacgcac gcaccccccg 9480
   agccgctccc cccgcacaaa gggccctccc ggagcccctc aaggctttca cgcagccaca 9540
   gaaaagaaac aagccgtcaw waaaccaagc actrattaca gcccggaggt gaagggccgt 9600
   tyckcccgca tcaymtktgg gwktaaagcg rtcagtcaga rcckgggcgg smggcgygra 9660
   acgrcggcgg arcagggctc gggggdaagg cagsstgcaa cgaytmccgc cccccccacs 9720
   ctttgytttt taaagggccs ccgccgccgc cgcctcsccm taaaaggaaa ctttyggagc 9780
   gcgccgcttt gattggctgc cgccgcacct tttcgcctcg ccccgccccg cccctcgccc 9840
   cgccccgccc cgcctggcgc gcgccccccc cccccccccg cccccatcgc tgcacaaaat 9900
   aattaaaaaa taaataaata caaaattggg ggtggggagg ggggggagat ggggagagtg 9960
   aagcagaacg tggggctcac ctcgaggatc tgggctagcc ctgatcaata acttcgtata 10020
   atgtatgcta tacgaacggt aggggatcca ctagttgggt cagtcgaagg gggcggtcgg 10080
   tccatcattt gcggccgggt gatttttggg gccgaaaagt gggaatttgg ggcccaattt 10140
   ggggcccaat ttggggccca atttgggttt tcgagggggg atttttttag ggggagattt 10200
   tgggtccccg gaaaatggtg attttgggtc aaaaatgggg attttggggt cgttttgagg 10260
   tggggttttt tggggtagaa atggcggtct gaggggaaaa tgtcgttttg gggccatttt 10320
   gggccatttg aggggaaatt tgggtcaaaa aatgacgatt ttgggtcgtt ttagggagga 10380
   aaatgaattt agggcgattt gagccaaatt tgggtcaaaa atggcgattt tgagtcaaaa 10440
   aatgattatt ttgggtcatt ttagggagga aaatgaattt agggagattg gacggcaaat 10500
   ttgggtcaaa aaatggtgat tttgggtcaa aaaatgatta ttttgggtca ttttagggag 10560
   aaaaatgaat ttagggagat ttgagggcaa atttgggtca aaaaatggtg atttggggtc 10620
   attttcagaa gaaaaaatga ttattttccc cactaaaaat gtatattttg gggccaaatg 10680
   gtgaaaaatg gtgattttta atcaaacgtc cccaaaattg gggaaatttc atcgatttga 10740
   cccaaaattg agtttttttt ccctgttaaa aatgtacatt ttggggtcaa tcgctgaaat 10800
   gttcccactt ttcacttctt tgcccccaaa ttttgctgtc cggtgagaaa ttaattaatt 10860
   aatgatcatt aattgatcga caattaatgg ttattaatta attaataggt cctcttttgg 10920
   tgactccttc gcttttgggg ccaaaagtcc ataaattggc cccaaaaaat taacgctgag 10980
   taattggatt ccaaagtatt aatgataaac attaaaagtg tttaattaat aatgatatta 11040
   aacataattt cgtttttatt atcgatttat caacaacgat gaacgataat actttacaac 11100
   aatcgctaat aattaattaa ttatttaatt aattaatttc taataattac ttcgcattat 11160
   cggacacgag atgttgtaat tattaataat ttaattccta ataattagaa gattcgttga 11220
   aaatggtact tatctttaca aataatcact tctaataata atgattaata atagttaata 11280
   acaacaatga cgataatgac gataatatta ataatatgtg atatatttaa tataaaattc 11340
   gtattaatat attatatcta caaaatatga tataaaatat aatattttat ttatatataa 11400
   cacaatttat tattattatc attattatca ttattaatat catcattatt aacgttatcg 11460
   aaatacttat ttagaaataa taaaaacgga tttaatgatg gcaacaaaaa tattttatta 11520
   atgttaaaaa aaaataatta ataatttcca aagattcgaa ttcggggcaa cgaacggcac 11580
   tcgataattt ttaattaatt aatagtttga attaatcggt actttttaat cctccatttt 11640
   gcccgaaatc gccgtttttt gccccaaatt ccccaccgcg gcgttaaaaa cataaagaaa 11700
   ttaagcatca aaagtgccct tttttggggt tgttttgacc caaaaaaaaa atggccgaat 11760
   tgggggcggc cgttttacgg ttgggttcat tttgggttca aaacagccaa aaatgggaac 11820
   tttgggtttc gaaaacaaca acaacaaaaa aacgggttta ttttgggctc attttgggtg 11880
   tttttgggtc aggaggagaa aaaataggaa gtttgagagc gaaacaacgg ccgcttttgg 11940
   ggggaaaacg gccctttttg gtcaacggcg ggggaaaaaa aaaagcggag tttttggggt 12000
   gaaaaagagc ggttttgggt aaatttgggt tttggggtaa aagtggagga ttttgggcaa 12060
   tgggagttaa aaaatgggtg tttttatggg ggttcggtgc agtttttcct gtttgatggg 12120
   gggtttatta atccgggggg ggggattaat gagaattaat aatgttaata gaaatatctg 12180
   ggaaattaat agcaattatt aattgttaat agttattaat agttctatat atctcacatc 12240
   tacgatacaa tataatatcg ttacaatcat atagtcgata tattacatat aattatcagt 12300
   aataataata agtaacaata attagcagta attaataata ataattaata gtattcgtta 12360
   ataagattat tgataataat taagtagtag cgattaatag agatgggatt tcgtgagaaa 12420
   tggaccaaat ttgggccgtt ttgacccaaa tttttggtgg ttttttttcc gattctttgt 12480
   gaatttcggg tcggattcat cagcaattaa ttacggttat taggggctat tagaggcttt 12540
   taattgggat tattagagac ttttaagcgg atttggggac ttttaagtgg attttatgat 12600
   tcttaagtgg attttgggtg gattttaccg cttttggcgg ccgccaccgc ggtggagctc 12660
   cagcttttgt tccctttagt gagggttaat tgcgcgcttg gcgtaatcat ggtcatagct 12720
   gtttcctgtg tgaaattgtt atccgctcac aattccacac aacatacgag ccggaagcat 12780
   aaagtgtaaa gcctggggtg cctaatgagt gagctaactc acattaattg cgttgcgctc 12840
   actgcccgct ttccagtcgg gaaacctgtc gtgccagctg cattaatgaa tcggccaacg 12900
   cgcggggaga ggcggtttgc gtattgggcg ctcttccgct tcctcgctca ctgactcgct 12960
   gcgctcggtc gttcggctgc ggcgagcggt atcagctcac tcaaaggcgg taatacggtt 13020
   atccacagaa tcaggggata acgcaggaaa gaacatgtga gcaaaaggcc agcaaaaggc 13080
   caggaaccgt aaaaaggccg cgttgctggc gtttttccat aggctccgcc cccctgacga 13140
   gcatcacaaa aatcgacgct caagtcagag gtggcgaaac ccgacaggac tataaagata 13200
   ccaggcgttt ccccctgtaa gctccctcgt gcgctctcct gttc 13244
<210> 56
   <211> 41
   <212> DNA
   <213> Artificial Sequence
<220>
   <223> Synthetic Primer
<400> 56
   ctatgcgcct tgccagcccg ctcagtccgt cagcgctctc t 41
<210> 57
   <211> 54
   <212> DNA
   <213> Artificial Sequence
<220>
   <223> Synthetic Primer
<220>
   <221> misc_feature
   <222> (26)..(35)
   <223> n is a, c, g, or t
<400> 57
   cgtatcgcct ccctcgcgcc atcagnnnnn nnnnnggggg gggttcatat gaag 54
<210> 58
   <211> 10240
   <212> DNA
   <213> Artificial Sequence
<220>
   <223> Targeting vector
<400> 58
   cacctgacgc gccctgtagc ggcgcattaa gcgcggcggg tgtggtggtt acgcgcagcg 60
   tgaccgctac acttgccagc gccctagcgc ccgctccttt cgctttcttc ccttcctttc 120
   tcgccacgtt cgccggcttt ccccgtcaag ctctaaatcg ggggctccct ttagggttcc 180
   gatttagtgc tttacggcac ctcgacccca aaaaacttga ttagggtgat ggttcacgta 240
   gtgggccatc gccctgatag acggtttttc gccctttgac gttggagtcc acgttcttta 300
   atagtggact cttgttccaa actggaacaa cactcaaccc tatctcggtc tattcttttg     360
   atttataagg gattttgccg atttcggcct attggttaaa aaaatgagct gatttaacaa      420
   aaatttaacg cgaattttaa caaaatatta acgcttacaa tttccattcg ccattcaggc     480
   tgcgcaactg ttgggaaggg cgatcggtgc gggcctcttc gctattacgc cagctggcga     540
   aagggggatg tgctgcaagg cgattaagtt gggtaacgcc agggttttcc cagtcacgac     600
   gttgtaaaac gacggccagt gagcgcgcgt aatacgactc actatagggc gaattgggta     660
   ccgggccccc cctcgagtga aggtcaccaa ggatggagac ttcagagcta tctgggacac     720
   gtgttttaac gtttgatcag ccttgcagca aaacagtgtt ttgctctatt gaacagtgtt     780
   caacaaaaac gatcagtggt ctggaacaca tctcctacga agaaaggttg cgagagctgg     840
   gtctggagaa gaggaggctc tggggcaacc tccctgtggc ctgtcagtgc ttaaaggagg     900
   ctcaagagaa agatggagag agactttata cacggagctg tagcaatatg acaaggagaa     960
   atggtttaaa atggaaagag ggtagattta ggttagctat caagaagaaa ttcttcactc    1020
   agaggtgatg aagcactgta gcaggttgcc cagagaagta ttggatgctt ccttcctggc    1080
   agggcccaag gccagactgg atgggttctg ggcagcctgt tctggtggga gatgtccctg    1140
   cccgtggcac ggcttggaaa tgggtagcct ttgaggtccc tctcagccca aatcattcta    1200
   tgttcttgtg gttccctgca tttcagcctt tgcctgttga ctctggtgcc actgttggac    1260
   aattctactc agctgtgttt catccctgcc ccaaagcagt aatcccatgg ataagcaatg    1320
   ctctgaggcc ttcaccacca tcagggctcc tgctgaccct agcggcctcc cttgcagcat    1380
   gagccctttg ttgtccgcac tgctgctgct gagggtcctg ccagcccccc agggcctttt    1440
   ctgcccaggc actgccacct gctaaaaccc agcctgccca tggcagggca tcgcatgtct    1500
   gagggatgga acggggcaag ggcagtgctg caggcagtgg ttcaagaaac gtttagatgt    1560
   tgtactgagg gatgtggttt agtgggaaat actggtgata ggtggatggt tggactggag    1620
   gatcttagag gtcttttcca accttggtga ttatgtgatt ctatgaaggg agccatagcc    1680
   tgctcccatc atgccccgcc agtccggctc tcccgagcac tttggttccg cagcatccag    1740
   gcccgctctg gggccggtgt gggcctgggt gctgccacgt gtccccgctg caaggcctgg      1800
   ccaaccccgc tgtgccacaa tgcaggggct gggctaggag tgggcaggga ggtgtgggga    1860
   gaggtgtagg acgtggctgg gagcgcaggg agttatttgc ataggggggc gtgctgcgga      1920
   aggacgcggg tataaaaggg catcgaggtc cccggcacag ccccatcggc gtggggacac    1980
   acagctgctg ggattccgcc atggcctggg ctcctctcct cctggcggtg ctcgcccaca    2040
   cctcaggtac tcgttgcgcc cggtcgggga ctgtgggcac ggggctctgt cccactgctg    2100
   cgcgggcagg gctgtgcgtg cggggccgtc actgattgcc gttttctccc ctctctcctc      2160
   tccggcgcgc ctctccaggt tccctggtgc aggcacagtc tgccctgact cagcctgcct 2220
   ccgtgtctgg gtctcctgga cagtcgatca ccatctcctg cactggaacc agcagtgacg 2280
   ttggtggtta taactatgtc tcctggtacc aacaaaaccc aggcaaagcc cccaaactca 2340
   tgatttatga tgtcagtaat cggccctcag ggatttctaa tcgcttctct ggctccaagt 2400
   ctggcaacac ggcctccctg accatctctg ggctccaggc tgacgacgag gctgattatt 2460
   actgcacctc atatacaaac gacagcaatt ctcaggtatt cggcggaggg accaagctga 2520
   ccgtcctagg tgagtcgctc atatgggatc ctaccgttcg tatagcatac attatacgaa 2580
   gttatcagac atgataagat acattgatga gtttggacaa accacaacta gaatgcagtg 2640
   aaaaaaatgc tttatttgtg aaatttgtga tgctattgct ttatttgtaa ccattataag 2700
   ctgcaataaa caagttgggg tgggcgaaga actccagcat gagatccccg cgctggagga 2760
   tcatccagcc ggcgtcccgg aaaacgattc cgaagcccaa cctttcatag aaggcggcgg 2820
   tggaatcgaa atctcgtagc acgtgtcagt cctgctcctc ggccacgaag tgcttagccc 2880
   tcccacacat aaccagaggg cagcaattca cgaatcccaa ctgccgtcgg ctgtccatca 2940
   ctgtccttca ctatggcttt gatcccagga tgcagatcga gaagcacctg tcggcaccgt 3000
   ccgcaggggc tcaagatgcc cctgttctca tttccgatcg cgacgataca agtcaggttg 3060
   ccagctgccg cagcagcagc agtgcccagc accacgagtt ctgcacaagg tcccccagta 3120
   aaatgatata cattgacacc agtgaagatg cggccgtcgc tagagagagc tgcgctggcg 3180
   acgctgtagt cttcagagat ggggatgctg ttgattgtag ccgttgctct ttcaatgagg 3240
   gtggattctt cttgagacaa aggcttggcc atggtttagt tcctcacctt gtcgtattat 3300
   actatgccga tatactatgc cgatgattaa ttgtcaacac gtgctgatca gatccgaaaa 3360
   tggatataca agctcccggg agctttttgc aaaagcctag gcctccaaaa aagcctcctc 3420
   actacttctg gaatagctca gaggcagagg cggcctcggc ctctgcataa ataaaaaaaa 3480
   ttagtcagcc atggggcgga gaatgggcgg aactgggcgg agttaggggc gggatgggcg 3540
   gagttagggg cgggactatg gttgctgact aattgagatg catgctttgc atacttctgc 3600
   ctgctgggga gcctggggac tttccacacc tggttgctga ctaattgaga tgcatgcttt 3660
   gcatacttct gcctgctggg gagcctgggg actttccaca ccctaactga cacacattcc 3720
   acagataact tcgtatagca tacattatac gaaatggcgg atccactagt tctagagacc 3780
   tcgtctcggt ctttcttccc ccatcgtgaa attgtgacat tttgtcgatt tttggtgatt 3840
   tgggggtttt tcttggactt ggcggcaggc tggggtctgc caccggcgca gggccgggca 3900
   ctcagcgcga cagcctgggc tgagtcttgt ccccaccgag ccggagggct ccggtgtgcg 3960
   ccatggagga cttagggtta ttttgtcaat ggaaagttct taaaatttga ccagaaaatg 4020
   tgcccgaggt ctgtctctgc cacacgattt cagaaattgt gtctaggtcg atgagaagac 4080
   agtttttgtc tttgtcagga aattagttgt gagttgttag tccttccctc ttagtcctaa 4140
   ggactaagac ctttgtcccc ggtctggtct ctcactgggg actcttggct ccagtgccat 4200
   ggggagccca agtgtcactg acacggtgtc cttgggggtg aaattcagtt tttcagctgt 4260
   ccaggtaagt gctgtcaaaa aatggaggtt attctgctga aaaagctgag aggaatattt 4320
   tgtcattttt ttcggaaata tatatatata tatatatata aatatataaa ttatttatat 4380
   attttatata tatatatata taaaatatat gtatttctct ctttcttttt atatatatat 4440
   ataaatatat atatatttct ccctctttct ttatatatat ttatagggag agaatctata 4500
   tttttggcca atttggccaa tttctctctc tctctcttta tatatatata tatatgtata 4560
   tgtaatttat atatatatat ataaatttat atttatttat ttatttattt atatagatta 4620
   tttttatata catatatata tataaatttg gccaatttag aagaaattgg ccaaaaaaac 4680
   ccaatctgaa ccaaattact tagggcataa gtccaaaaaa aaagtgcagt tcaagaattc 4740
   ctcgctggaa agtttttaat gggggcagtt cttccccatt ttctggtgta gatatgagtg 4800
   aagatataga tatgtatgta gatatagatt tggctatagt ttgtttgatg aacagtctga 4860
   ggaagagtaa atcttgcctt cctcatggca atttttaatt ctacttcttc tgagacaagt 4920
   ctgaacatgc cttgaggtag aaagagcccg aaaattgagt gctttttctt tgaaatttta 4980
   attaaaatta aaaaatagcc ttacagcagg aaagaaaggg ggtgatggca gctaatcgga 5040
   gttccaaagc tgaaccacgt tcacccaaac acgtggttga aattttgtgt gtgtatatat 5100
   ataaatatat atatgtatac aaaagaaaat gcctatatat aaaaaaattg ttatgtatat 5160
   tatatagatt tattatagat atgtacataa gatataacac gtatacatat atatgtagtc 5220
   tgctgtatgc tttgtataaa tttttataga tatgatgtat attatatgca tgggttatat 5280
   atctataata tattttatct gtagaaggaa ctacagtata aaatgcatat atgcgtatat 5340
   atacacatat ttatatatat gtgtatatat atgtatacgc acacgtataa catccatgaa 5400
   aatagatatg gatgggtgga tgtgtttgtt ttacagaggt gcatgtgtgt ctgtacatac 5460
   acagccatac atacgcgtgt ggcgcgcctc tgcctctctc ttgcaggtca gcccaaggct 5520
   gccccctcgg tcactctgtt cccgccctcc tctgaggagc ttcaagccaa caaggccaca 5580
   ctggtgtgtc tcataagtga cttctacccg ggagccgtga cagtggcctg gaaggcagat 5640
   agcagccccg tcaaggcggg agtggagacc accacaccct ccaaacaaag caacaacaag 5700
   tacgcggcca gcagctacct gagcctgacg cctgagcagt ggaagtccca cagaagctac 5760
   agctgccagg tcacgcatga agggagcacc gtggagaaga cagtggcccc tacagaatgt 5820
   tcatagtagt cccactccat atgtcccact ggggatgcaa tgtgaggaca gtggttcctc 5880
   accctccctg tccctctggg ccgctgctgg tggcagcagc cctcacttcc cactcagatg 5940
   tcccccaccg tgcccccatc acccacctct gcctgtcgac tcctcttgcc ctcatctctc 6000
   caggtgtcac attaataaac ggatccacga cactgaacta gtgctgactc tgcatccatg 6060
   tctctgtgtc cttttgcgtg ctgtctgcat ctcacacagg ggggtcggcc ccagtatggg 6120
   gaagggctgg ggggcgcata cacacatatt ggtaatgttg ggggcggggg gggggggtgg 6180
   gggggtcaac agatcagcac tggagacact ggtgtatacc ctggcaccac caacatctaa 6240
   ggcagggtgc tttggggcaa ttttggggca gtttaaggtc tgtgctggca ctgagcacgt 6300
   ggctgtggcc gtgctgtcct catctcccac ccactacggt ctgtgcgcca ggtccctagc 6360
   agagatttgc tttatgctgg gaacaggggg agttctggct ctgttccctt gcattcagac 6420
   accctggtgc cccctgggtg ggatgtcagt gtgaatactc ctttgtgccc tgtgcctgca 6480
   gcagcctgac cctccacaca ccacacgcct tgtgtgcacc ccacccctgt cactatccct 6540
   ctccccgctc cccagggaga ttttgcagtg gcccctgtag ggcagctttt agcacagccc 6600
   ccagcagcaa gcaagcagaa agcactgctg tgcacagctt gtcagctgtg tgtgtttgct 6660
   gaggaggatc tgtcttttgc tgaggccatc agtcttgtcc tgctcaacct ccatcgatgc 6720
   tgcccacctc aacacatcta cccatctatt ccatctacac caacatctcc attcatccca 6780
   cccacccaaa catgtccatc catcacaaca cctccatcca acccgcacac tccagcacct 6840
   ccaatcattc catctacacc accatgctga tctgctacag ccactccaac gcaaccgtcc 6900
   attccatcta caccaatgtc catccatccc agccactcca gcacctccag ccatcccacc 6960
   caccctatgt ctccatccag ccactggtgg ggtgcaggac atggggccag ctctactgtc 7020
   aggactgggg tttttgcatg gccccatacc acttctgcag aagagacgca ctgaaagttt 7080
   ggctgaccat tttctccacg gtagagttgt ggcagttctg taatttaggg tcttttatcc 7140
   agtttggaga tgggctggga tctcccagct ccatggcagg cattcatgac actgggttta 7200
   gtatctgatg ggtgggatgt ggctgaactt cattttcttt ccccagtgac aaagtttttg 7260
   cagttgaata tgaattcctg ctttctgctc tatgagttgt ttttttccca ggacgtacac 7320
   agggaatcag cagtcttcat tctccctctg ccatgtgtag actctctgcc acacaggact 7380
   gtgctgtcct catgcccctg cgcccaaatt gctgccctct gcccatgcct gccaagctga 7440
   gccctgcagg ctgccatgct ggattgacat gagccctgag attggtacag aaatggtgat 7500
   tttggggttt tctttgcact caggaagctg aaggctcaat gctcagtgat ggatttacca 7560
   aactgtgccc tgaggcagct gctcatgctg gataaagtca ctggagcaca gggaaccagg 7620
   cgctgggcag ggatttctca tgggccccac ttggaaagct gcaggctgca aacctggctc 7680
   tgccttcacg cctcaccctc atgaggacaa cctcactaat tattgattaa aagattttgc 7740
   taaaccatct ccagaagcaa caacccactg aggagcatgt gctgaattat acatcacagc 7800
   tccacggccc tgccctcata gcagggctgc atggcaccca cagtggcact cagagggacc 7860
   acagggctga gacagccggg tctggtggtg gggacacagc tgagcatagg atgagccccc 7920
   cgggcagtgc tgggctttgc taatgagcag aagtatggat agaaagcaac cccagggctc 7980
   cgtacccagc tgcagctctt gctctgtcgt gtcctttggc ggccgccacc gcggtggagc 8040
   tccagctttt gttcccttta gtgagggtta attgcgcgct tggcgtaatc atggtcatag 8100
   ctgtttcctg tgtgaaattg ttatccgctc acaattccac acaacatacg agccggaagc 8160
   ataaagtgta aagcctgggg tgcctaatga gtgagctaac tcacattaat tgcgttgcgc 8220
   tcactgcccg ctttccagtc gggaaacctg tcgtgccagc tgcattaatg aatcggccaa 8280
   cgcgcgggga gaggcggttt gcgtattggg cgctcttccg cttcctcgct cactgactcg 8340
   ctgcgctcgg tcgttcggct gcggcgagcg gtatcagctc actcaaaggc ggtaatacgg 8400
   ttatccacag aatcagggga taacgcagga aagaacatgt gagcaaaagg ccagcaaaag 8460
   gccaggaacc gtaaaaaggc cgcgttgctg gcgtttttcc ataggctccg cccccctgac 8520
   gagcatcaca aaaatcgacg ctcaagtcag aggtggcgaa acccgacagg actataaaga 8580
   taccaggcgt ttccccctgg aagctccctc gtgcgctctc ctgttccgac cctgccgctt 8640
   accggatacc tgtccgcctt tctcccttcg ggaagcgtgg cgctttctca tagctcacgc 8700
   tgtaggtatc tcagttcggt gtaggtcgtt cgctccaagc tgggctgtgt gcacgaaccc 8760
   cccgttcagc ccgaccgctg cgccttatcc ggtaactatc gtcttgagtc caacccggta 8820
   agacacgact tatcgccact ggcagcagcc actggtaaca ggattagcag agcgaggtat 8880
   gtaggcggtg ctacagagtt cttgaagtgg tggcctaact acggctacac tagaaggaca 8940
   gtatttggta tctgcgctct gctgaagcca gttaccttcg gaaaaagagt tggtagctct 9000
   tgatccggca aacaaaccac cgctggtagc ggtggttttt ttgtttgcaa gcagcagatt 9060
   acgcgcagaa aaaaaggatc tcaagaagat cctttgatct tttctacggg gtctgacgct 9120
   cagtggaacg aaaactcacg ttaagggatt ttggtcatga gattatcaaa aaggatcttc 9180
   acctagatcc ttttaaatta aaaatgaagt tttaaatcaa tctaaagtat atatgagtaa 9240
   acttggtctg acagttacca atgcttaatc agtgaggcac ctatctcagc gatctgtcta 9300
   tttcgttcat ccatagttgc ctgactcccc gtcgtgtaga taactacgat acgggagggc 9360
   ttaccatctg gccccagtgc tgcaatgata ccgcgagacc cacgctcacc ggctccagat 9420
   ttatcagcaa taaaccagcc agccggaagg gccgagcgca gaagtggtcc tgcaacttta 9480
   tccgcctcca tccagtctat taattgttgc cgggaagcta gagtaagtag ttcgccagtt 9540
   aatagtttgc gcaacgttgt tgccattgct acaggcatcg tggtgtcacg ctcgtcgttt 9600
   ggtatggctt cattcagctc cggttcccaa cgatcaaggc gagttacatg atcccccatg 9660
   ttgtgcaaaa aagcggttag ctccttcggt cctccgatcg ttgtcagaag taagttggcc 9720
   gcagtgttat cactcatggt tatggcagca ctgcataatt ctcttactgt catgccatcc 9780
   gtaagatgct tttctgtgac tggtgagtac tcaaccaagt cattctgaga atagtgtatg 9840
   cggcgaccga gttgctcttg cccggcgtca atacgggata ataccgcgcc acatagcaga 9900
   actttaaaag tgctcatcat tggaaaacgt tcttcggggc gaaaactctc aaggatctta 9960
   ccgctgttga gatccagttc gatgtaaccc actcgtgcac ccaactgatc ttcagcatct 10020
   tttactttca ccagcgtttc tgggtgagca aaaacaggaa ggcaaaatgc cgcaaaaaag 10080
   ggaataaggg cgacacggaa atgttgaata ctcatactct tcctttttca atattattga 10140
   agcatttatc agggttattg tctcatgagc ggatacatat ttgaatgtat ttagaaaaat 10200
   aaacaaatag gggttccgcg cacatttccc cgaaaagtgc 10240
<210> 59
   <211> 26
   <212> DNA
   <213> Artificial Sequence
<220>
   <223> Synthetic DNA
<400> 59
   gttaaccggt atgcatcgat ttaaat 26
<210> 60
   <211> 11743
   <212> DNA
   <213> Artificial Sequence
<220>
   <223> Targeting vector
<400> 60
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgggta tcacgtgggg caaatcgaga     420
   gctaccattg gctatgggga ggagaaaatg aggcgtgcta cttcctcgtt cctgcagtgc     480
   gtgtcgaggt gccgtgttgg tcggatctca gtggcagagc aggatggtga gcggtggcct     540
   tggggccgct ccctcactct gctgggctgg tgcggtggct ccgggaccgg actttcagtg     600
   gtgtttggtg tggtgctcac tgtcctttgg tgtcactgtg tggtgaattc cctcagctgg     660
   gcggtgagag cccatgctgt gtcctggtag gagtagtctg ggctgcagtg cgggctcttt      720
   gtgcgtgttg ggggcggttc cctggaagct aattctggaa gaaatcccgg ctgcctctgt 780
   aacagttgtt ggcgatgtca aacaccctgg agctgctgac tggctctgta ccattgagtg 840
   atgcagttta aacctctctc tttcctcctg ctgtgtgtga agctggtgtt agtgctagga 900
   gacctgcaca tccctcaccg atgcagtggt ttaccagcaa agttcaagag cctcctggtt 960
   ccagggaaga ttcagcatat tttgtgtaca ggaaacctct gcaccaagga gagctatgac 1020
   tacctcagga ctttggctgg agatgttcac gttgttaagg gggactctga ggtaatgttc 1080
   ttgctgctgt gaattcctca gtcctccatc ttacaagccg tagttgtccc actgaaagct 1140
   gggaaaccct tcggtgtttg caattcctga gcgctaatgt gtgggggaag gatgtaggaa 1200
   caaataaggc tcaagagaca acaacttcct aggctcctct tattgccctt gtggtaaggg 1260
   aggacttggt gtgtagtggt aatacatgca ctcaacttat taagttctca tagctgctgt 1320
   gaagctgctg cagtccctca gatccctgca ggaagagggg actgactgac tgttgaatag 1380
   ccttgaagct cctgctctgc atgtggttcc ggtcaggctc aagatcccag ttcttagctc 1440
   ttcagttgct cttctggggg tatagtacta gcatctgggt cactgcagat ggacagagac 1500
   tgtgcttggg tagcaaaagg cagacttacg ttgtgaaacc ttcattgtgt tggtgaggtt 1560
   ctttgatttc taccttgtgt atctctcttc ctcagagtct gaattatcct gaacagaagg 1620
   ttgtaactgt tgggcagttc agaattgggt tgattcatgg ccatcaagtc attccctggg 1680
   gtgatgtggc aagcttggca ttgctgcaga ggcagctgga tgtagacatt cttatctcag 1740
   gacatacaca cagatttgaa gcatttgagc atgaaaataa gttctacatc aacccaggat 1800
   cagctacagg agcctacact ggtttacaaa cgtgagtaca atgagccttg tgtttgtctt 1860
   taagacagat gtctgtcctt aagagtgcag cccatgtgtt gaagagcact ggtgagatgc 1920
   agtgccatgc aggccatctc tccctttccc agagaacacg gcctacctct ctctgttcat 1980
   aaggtcttgg ttaacttatg agttggccag cacagcctgg cactgatgtg tcatgatcat 2040
   aaccctgagc ccacgctctg ctatgctgtt gcttcttaga gttgcattgc cttgaggtgt 2100
   attccagaag ctgtgtgctg agctcttgtt gggaaagagg ccctcagggc actgccaaca 2160
   tctgaggctt gcaggagctg gaacattcag ccctgagggc tgctctggcc aactgcagct 2220
   ctgagtctgc agtcagtgcc tctgagctgt gatgcttctc agataactgc agccctttgc 2280
   aatgtaatgt tttataatat atttccatag gacatcttgg taagaggtgt aagctaatca 2340
   gggtgattga caaagtatgt tttctttcca ggaacgtcat tccttcattt gtactgatgg 2400
   atatccaggc ttccacagtt gtgacttatg tataccagct aattgaggac gatgtgaaag 2460
   tagaaagaat tgagttcaag aaatactgac acgtgtttga atctgcctgc tgacttctca 2520
   ctgcctctca ttctttctct cctagttaaa gctgaatccg gccacgtggg ggtgctgctg 2580
   cagcggcgtg taactgtgag cttgggaagt tctcaactag ggcttagagc aggagaaact 2640
   gattaacaga aattgcctta ttaaatgtat atatttcctg tgtctaagaa cgaggtgctg 2700
   ttgttcctga cttgcggagc tgcagctagt ggtagcttgg aatttaacct ctcctgtaaa 2760
   atgagcatag aataaaacag tattttcaca tttctggatc ctgttaattg tttgttaatt 2820
   aagaggacag gaattcagtg tcagggggct gctctttgct tcccaatgtg taccactgaa 2880
   gcctctctgg atttatcaga gatactcaat ggactcttaa cagcactgtg gcacctgacc 2940
   tcctgctgcc ttcagcctcc cttcctggct ggtgtgctta tgttgaggag ctgtgggagg 3000
   ctggtgcagt tctgtgggat gaggtaaggc cagctgaata gcctgggggc tggtcagggg 3060
   gcttagtgag aggagcaggg ctggggaatg ttgtgaacag caggtggtgc cactgctatt 3120
   ctgaggaagg aggcagcttc tgcaaatgct ttgcaatgca ggatgacaaa gtgcttctgc 3180
   tagccctagg ctaccgttcg tataatgtat actatacgaa gttattttat ttgtgaaatt 3240
   tgtgatgcta ttgctttatt tgtaaccatt ataagctgca ataaacaagt tggggtgggc 3300
   gaagaactcc agcatgagat ccccgcgctg gaggatcatc cagccggcgt cccggaaaac 3360
   gattccgaag cccaaccttt catagaaggc ggcggtggaa tcgaaatctc gtgatggcag 3420
   gttgggcgtc gcttggtcgg tcatttcgaa ccccagagtc ccgctcagaa gaactcgtca 3480
   agaaggcgat agaaggcgat gcgctgcgaa tcgggagcgg cgataccgta aagcacgagg 3540
   aagcggtcag cccattcgcc gccaagctct tcagcaatat cacgggtagc caacgctatg 3600
   tcctgatagc ggtccgccac acccagccgg ccacagtcga tgaatccaga aaagcggcca 3660
   ttttccacca tgatattcgg caagcaggca tcgccatggg tcacgacgag atcctcgccg 3720
   tcgggcatgc gcgccttgag cctggcgaac agttcggctg gcgcgagccc ctgatgctct 3780
   tcgtccagat catcctgatc gacaagaccg gcttccatcc gagtacgtgc tcgctcgatg 3840
   cgatgtttcg cttggtggtc gaatgggcag gtagccggat caagcgtatg cagccgccgc 3900
   attgcatcag ccatgatgga tactttctcg gcaggagcaa ggtgagatga caggagatcc 3960
   tgccccggca cttcgcccaa tagcagccag tcccttcccg cttcagtgac aacgtcgagc 4020
   acagctgcgc aaggaacgcc cgtcgtggcc agccacgata gccgcgctgc ctcgtcctgc 4080
   agttcattca gggcaccgga caggtcggtc ttgacaaaaa gaaccgggcg cccctgcgct 4140
   gacagccgga acacggcggc atcagagcag ccgattgtct gttgtgccca gtcatagccg 4200
   aatagcctct ccacccaagc ggccggagaa cctgcgtgca atccatcttg ttcaatcatg 4260
   cgaaacgatc ctcatcctgt ctcttgatca gatccgaaaa tggatataca agctcccggg 4320
   agctttttgc aaaagcctag gcctccaaaa aagcctcctc actacttctg gaatagctca 4380
   gaggcagagg cggcctcggc ctctgcataa ataaaaaaaa ttagtcagcc atggggcgga 4440
   gaatgggcgg aactgggcgg agttaggggc gggatgggcg gagttagggg cgggactatg 4500
   gttgctgact aattgagatg catgctttgc atacttctgc ctgctgggga gcctggggac 4560
   tttccacacc tggttgctga ctaattgaga tgcatgcttt gcatacttct gcctgctggg 4620
   gagcctgggg actttccaca ccctaaataa cttcgtataa tgtatactat acgaacggta 4680
   gcgttaaccg gtatgcatcg atttaaatcg agtgaaggtc accaaggatg gagacttcag 4740
   agctatctgg gacacgtgtt ttaacgtttg atcagccttg cagcaaaaca gtgttttgct 4800
   ctattgaaca gtgttcaaca aaaacgatca gtggtctgga acacatctcc tacgaagaaa 4860
   ggttgcgaga gctgggtctg gagaagagga ggctctgggg caacctccct gtggcctgtc 4920
   agtgcttaaa ggaggctcaa gagaaagatg gagagagact ttatacacgg agctgtagca 4980
   atatgacaag gagaaatggt ttaaaatgga aagagggtag atttaggtta gctatcaaga 5040
   agaaattctt cactcagagg tgatgaagca ctgtagcagg ttgcccagag aagtattgga 5100
   tgcttccttc ctggcagggc ccaaggccag actggatggg ttctgggcag cctgttctgg 5160
   tgggagatgt ccctgcccgt ggcacggctt ggaaatgggt agcctttgag gtccctctca 5220
   gcccaaatca ttctatgttc ttgtggttcc ctgcatttca gcctttgcct gttgactctg 5280
   gtgccactgt tggacaattc tactcagctg tgtttcatcc ctgccccaaa gcagtaatcc 5340
   catggataag caatgctctg aggccttcac caccatcagg gctcctgctg accctagcgg 5400
   cctcccttgc agcatgagcc ctttgttgtc cgcactgctg ctgctgaggg tcctgccagc 5460
   cccccagggc cttttctgcc caggcactgc cacctgctaa aacccagcct gcccatggca 5520
   gggcatcgca tgtctgaggg atggaacggg gcaagggcag tgctgcaggc agtggttcaa 5580
   gaaacgttta gatgttgtac tgagggatgt ggtttagtgg gaaatactgg tgataggtgg 5640
   atggttggac tggaggatct tagaggtctt ttccaacctt ggtgattatg tgattctatg 5700
   aagggagcca tagcctgctc ccatcatgcc ccgccagtcc ggctctcccg agcactttgg 5760
   ttccgcagca tccaggcccg ctctggggcc ggtgtgggcc tgggtgctgc cacgtgtccc 5820
   cgctgcaagg cctggccaac cccgctgtgc cacaatgcag gggctgggct aggagtgggc 5880
   agggaggtgt ggggagaggt gtaggacgtg gctgggagcg cagggagtta tttgcatagg 5940
   ggggcgtgct gcggaaggac gcgggtataa aagggcatcg aggtccccgg cacagcccca 6000
   tcggcgtggg gacacacagc tgctgggatt ccgccatggc ctgggctcct ctcctcctgg 6060
   cggtgctcgc ccacacctca ggtactcgtt gcgcccggtc ggggactgtg ggcacggggc 6120
   tctgtcccac tgctgcgcgg gcagggctgt gcgtgcgggg ccgtcactga ttgccgtttt 6180
   ctcccctctc tcctctccgg cgcgcctctc caggttccct ggtgcaggca cagtctgccc 6240
   tgactcagcc tgcctccgtg tctgggtctc ctggacagtc gatcaccatc tcctgcactg 6300
   gaaccagcag tgacgttggt ggttataact atgtctcctg gtaccaacaa aacccaggca 6360
   aagcccccaa actcatgatt tatgatgtca gtaatcggcc ctcagggatt tctaatcgct 6420
   tctctggctc caagtctggc aacacggcct ccctgaccat ctctgggctc caggctgacg 6480
   acgaggctga ttattactgc acctcatata caaacgacag caattctcag gtattcggcg 6540
   gagggaccaa gctgaccgtc ctaggtgagt cgctcatatg ctaccgttcg tatagcatac 6600
   attatacgaa gttatcagac atgataagat acattgatga gtttggacaa accacaacta 6660
   gaatgcagtg aaaaaaatgc tttatttgtg aaatttgtga tgctattgct ttatttgtaa 6720
   ccattataag ctgcaataaa caagttgggg tgggcgaaga actccagcat gagatccccg 6780
   cgctggagga tcatccagcc ggcgtcccgg aaaacgattc cgaagcccaa cctttcatag 6840
   aaggcggcgg tggaatcgaa atctcgtagc acgtgtcagt cctgctcctc ggccacgaag 6900
   tgcttagccc tcccacacat aaccagaggg cagcaattca cgaatcccaa ctgccgtcgg 6960
   ctgtccatca ctgtccttca ctatggcttt gatcccagga tgcagatcga gaagcacctg 7020
   tcggcaccgt ccgcaggggc tcaagatgcc cctgttctca tttccgatcg cgacgataca 7080
   agtcaggttg ccagctgccg cagcagcagc agtgcccagc accacgagtt ctgcacaagg 7140
   tcccccagta aaatgatata cattgacacc agtgaagatg cggccgtcgc tagagagagc 7200
   tgcgctggcg acgctgtagt cttcagagat ggggatgctg ttgattgtag ccgttgctct 7260
   ttcaatgagg gtggattctt cttgagacaa aggcttggcc atggtttagt tcctcacctt 7320
   gtcgtattat actatgccga tatactatgc cgatgattaa ttgtcaacac gtgctgatca 7380
   gatccgaaaa tggatataca agctcccggg agctttttgc aaaagcctag gcctccaaaa 7440
   aagcctcctc actacttctg gaatagctca gaggcagagg cggcctcggc ctctgcataa 7500
   ataaaaaaaa ttagtcagcc atggggcgga gaatgggcgg aactgggcgg agttaggggc 7560
   gggatgggcg gagttagggg cgggactatg gttgctgact aattgagatg catgctttgc 7620
   atacttctgc ctgctgggga gcctggggac tttccacacc tggttgctga ctaattgaga 7680
   tgcatgcttt gcatacttct gcctgctggg gagcctgggg actttccaca ccctaactga 7740
   cacacattcc acagataact tcgtatagca tacattatac gaacggtagc tctagagacc 7800
   tcgtctcggt ctttcttccc ccatcgtgaa attgtgacat tttgtcgatt tttggtgatt 7860
   tgggggtttt tcttggactt ggcggcaggc tggggtctgc caccggcgca gggccgggca 7920
   ctcagcgcga cagcctgggc tgagtcttgt ccccaccgag ccggagggct ccggtgtgcg 7980
   ccatggagga cttagggtta ttttgtcaat ggaaagttct taaaatttga ccagaaaatg 8040
   tgcccgaggt ctgtctctgc cacacgattt cagaaattgt gtctaggtcg atgagaagac 8100
   agtttttgtc tttgtcagga aattagttgt gagttgttag tccttccctc ttagtcctaa 8160
   ggactaagac ctttgtcccc ggtctggtct ctcactgggg actcttggct ccagtgccat 8220
   ggggagccca agtgtcactg acacggtgtc cttgggggtg aaattcagtt tttcagctgt 8280
   ccaggtaagt gctgtcaaaa aatggaggtt attctgctga aaaagctgag aggaatattt 8340
   tgtcattttt ttcggaaata tatatatata tatatatata aatatataaa ttatttatat 8400
   attttatata tatatatata taaaatatat gtatttctct ctttcttttt atatatatat 8460
   ataaatatat atatatttct ccctctttct ttatatatat ttatagggag agaatctata 8520
   tttttggcca atttggccaa tttctctctc tctctcttta tatatatata tatatgtata 8580
   tgtaatttat atatatatat ataaatttat atttatttat ttatttattt atatagatta 8640
   tttttatata catatatata tataaatttg gccaatttag aagaaattgg ccaaaaaaac 8700
   ccaatctgaa ccaaattact tagggcataa gtccaaaaaa aaagtgcagt tcaagaattc 8760
   ctcgctggaa agtttttaat gggggcagtt cttccccatt ttctggtgta gatatgagtg 8820
   aagatataga tatgtatgta gatatagatt tggctatagt ttgtttgatg aacagtctga 8880
   ggaagagtaa atcttgcctt cctcatggca atttttaatt ctacttcttc tgagacaagt 8940
   ctgaacatgc cttgaggtag aaagagcccg aaaattgagt gctttttctt tgaaatttta 9000
   attaaaatta aaaaatagcc ttacagcagg aaagaaaggg ggtgatggca gctaatcgga 9060
   gttccaaagc tgaaccacgt tcacccaaac acgtggttga aattttgtgt gtgtatatat 9120
   ataaatatat atatgtatac aaaagaaaat gcctatatat aaaaaaattg ttatgtatat 9180
   tatatagatt tattatagat atgtacataa gatataacac gtatacatat atatgtagtc 9240
   tgctgtatgc tttgtataaa tttttataga tatgatgtat attatatgca tgggttatat 9300
   atctataata tattttatct gtagaaggaa ctacagtata aaatgcatat atgcgtatat 9360
   atacacatat ttatatatat gtgtatatat atgtatacgc acacgtataa catccatgaa 9420
   aatagatatg gatgggtgga tgtgtttgtt ttacagaggt gcatgtgtgt ctgtacatac 9480
   acagccatac atacgcgtgt gcggccgcag gcgtaatcat ggtcatagct gtttcctgtg 9540
   tgaaattgtt atccgctcac aattccacac aacatacgag ccggaagcat aaagtgtaaa 9600
   gcctggggtg cctaatgagt gagctaactc acattaattg cgttgcgctc actgcccgct 9660
   ttccagtcgg gaaacctgtc gtgccagctg cattaatgaa tcggccaacg cgcggggaga 9720
   ggcggtttgc gtattgggcg ctcttccgct tcctcgctca ctgactcgct gcgctcggtc 9780
   gttcggctgc ggcgagcggt atcagctcac tcaaaggcgg taatacggtt atccacagaa 9840
   tcaggggata acgcaggaaa gaacatgtga gcaaaaggcc agcaaaaggc caggaaccgt 9900
   aaaaaggccg cgttgctggc gtttttccat aggctccgcc cccctgacga gcatcacaaa 9960
   aatcgacgct caagtcagag gtggcgaaac ccgacaggac tataaagata ccaggcgttt 10020
   ccccctggaa gctccctcgt gcgctctcct gttccgaccc tgccgcttac cggatacctg 10080
   tccgcctttc tcccttcggg aagcgtggcg ctttctcata gctcacgctg taggtatctc 10140
   agttcggtgt aggtcgttcg ctccaagctg ggctgtgtgc acgaaccccc cgttcagccc 10200
   gaccgctgcg ccttatccgg taactatcgt cttgagtcca acccggtaag acacgactta 10260
   tcgccactgg cagcagccac tggtaacagg attagcagag cgaggtatgt aggcggtgct 10320
   acagagttct tgaagtggtg gcctaactac ggctacacta gaagaacagt atttggtatc 10380
   tgcgctctgc tgaagccagt taccttcgga aaaagagttg gtagctcttg atccggcaaa 10440
   caaaccaccg ctggtagcgg tggttttttt gtttgcaagc agcagattac gcgcagaaaa 10500
   aaaggatctc aagaagatcc tttgatcttt tctacggggt ctgacgctca gtggaacgaa 10560
   aactcacgtt aagggatttt ggtcatgaga ttatcaaaaa ggatcttcac ctagatcctt 10620
   ttaaattaaa aatgaagttt taaatcaatc taaagtatat atgagtaaac ttggtctgac 10680
   agttaccaat gcttaatcag tgaggcacct atctcagcga tctgtctatt tcgttcatcc 10740
   atagttgcct gactccccgt cgtgtagata actacgatac gggagggctt accatctggc 10800
   cccagtgctg caatgatacc gcgagaccca cgctcaccgg ctccagattt atcagcaata 10860
   aaccagccag ccggaagggc cgagcgcaga agtggtcctg caactttatc cgcctccatc 10920
   cagtctatta attgttgccg ggaagctaga gtaagtagtt cgccagttaa tagtttgcgc 10980
   aacgttgttg ccattgctac aggcatcgtg gtgtcacgct cgtcgtttgg tatggcttca 11040
   ttcagctccg gttcccaacg atcaaggcga gttacatgat cccccatgtt gtgcaaaaaa 11100
   gcggttagct ccttcggtcc tccgatcgtt gtcagaagta agttggccgc agtgttatca 11160
   ctcatggtta tggcagcact gcataattct cttactgtca tgccatccgt aagatgcttt 11220
   tctgtgactg gtgagtactc aaccaagtca ttctgagaat agtgtatgcg gcgaccgagt 11280
   tgctcttgcc cggcgtcaat acgggataat accgcgccac atagcagaac tttaaaagtg 11340
   ctcatcattg gaaaacgttc ttcggggcga aaactctcaa ggatcttacc gctgttgaga 11400
   tccagttcga tgtaacccac tcgtgcaccc aactgatctt cagcatcttt tactttcacc 11460
   agcgtttctg ggtgagcaaa aacaggaagg caaaatgccg caaaaaaggg aataagggcg 11520
   acacggaaat gttgaatact catactcttc ctttttcaat attattgaag catttatcag 11580
   ggttattgtc tcatgagcgg atacatattt gaatgtattt agaaaaataa acaaataggg 11640
   gttccgcgca catttccccg aaaagtgcca cctgacgtct aagaaaccat tattatcatg 11700
   acattaacct ataaaaatag gcgtatcacg aggccctttc gtc 11743
<210> 61
   <211> 7001
   <212> DNA
   <213> Homo sapiens
<400> 61
   accggtcgac atgcaagctt gctagcgttt aaacacgagt gcgtacgctc gacaagacgc 60
   actccagtct gccctgactc agcctgcctc cgtgtctggg tctcctggac agtcgatcac      120
   catctcctgc tctggaagca gctccaacat cggaagtaat tatgtatact ggtaccaaca      180
   gcacccaggc aaagccccca aactcatgat ttataggaat aatcagcggc cctcaggggt      240
   ttctaatcgc ttctctggct ccaagtctgg caacacggcc tccctgacca tctctgggct      300
   ccaggctgag gacgaggctg attattactg cgcagcatgg gatgacagcc tgagtggtca      360
   ttgggtgttc ggcggaggga ccaagctgac cgtcctagcc tgacacacac agccctcccc      420
   agactgtggg atgaggccct gtgcccgcag tcacatgtgg aatatcaaga cacacacatc      480
   tatgacaatc accgtgtctc tactcgcgtg tctctctatg cgcagtctgc cctgactcag      540
   cctgcctccg tgtctgggtc tcctggacag tcgatcacca tctcctgcac tgggagcagc      600
   tccaacatcg gggcaggtta tgatgtacac tggtaccaac agcacccagg caaagccccc      660
   aaactcatga tttatggtaa cagcaatcgg ccctcagggg tttctaatcg cttctctggc      720
   tccaagtctg gcaacacggc ctccctgacc atctctgggc tccaggctga ggacgaggct      780
   gattattact gccagtccta tgacagcagc ctgagtggtt ctgtggtatt cggcggaggg      840
   accaagctga ccgtcctagc ctgacacaca cagccctccc cagactgtgg gatgaggccc      900
   tgtgcccgca gtcacatgtg gaatatcaag acacacacat ctatgacaat cacatacgac      960
   gtatcacgta ctacgtctag taccagtctg ccctgactca gcctgcctcc gtgtctgggt     1020
   ctcctggaca gtcgatcacc atctcctgct ctggaagcag ctccaacatt gggaataatt     1080
   atgtatcctg gtaccaacag cacccaggca aagcccccaa actcatgatt tatgacaata     1140
   ataagcgacc ctcaggggtt tctaatcgct tctctggctc caagtctggc aacacggcct     1200
   ccctgaccat ctctgggctc caggctgagg acgaggctga ttattactgc ggaacatggg     1260
   atagcagcct gagtgctggt gctgtgttcg gcggagggac caagctgacc gtcctagcct     1320
   gacacacaca gccctcccca gactgtggga tgaggccctg tgcccgcagt cacatgtgga     1380
   atatcaagac acacacatct atgacaatca ccgtagacta gtacgagtat gtactctcgt     1440
   gcagtctgcc ctgactcagc ctgcctccgt gtctgggtct cctggacagt cgatcaccat     1500
   ctcctgcact gggagcagct ccaacattgg ggcgggttat gttgtacatt ggtaccaaca     1560
   gcacccaggc aaagccccca aactcatgat ttatgaaaat aataagcgac cctcaggggt     1620
   ttctaatcgc ttctctggct ccaagtctgg caacacggcc tccctgacca tctctgggct     1680
   ccaggctgag gacgaggctg attattactg cagctcatat acaagcagcg gcactcctgt     1740
   ggtattcggc ggagggacca agctgaccgt cctagcctga cacacacagc cctccccaga     1800
   ctgtgggatg aggccctgtg cccgcagtca catgtggaat atcaagacac acacatctat     1860
   gacaatcaca cgcgagtata ctactatgta ctgtacagtc agtctgccct gactcagcct     1920
   gcctccgtgt ctgggtctcc tggacagtcg atcaccatct cctgcactgg aaccagcagt     1980
   gatgttggga gttataacct tgtctcctgg taccaacagc acccaggcaa agcccccaaa 2040
   ctcatgattt atgagggcag taagcggccc tcaggggttt ctaatcgctt ctctggctcc 2100
   aagtctggca acacggcctc cctgaccatc tctgggctcc aggctgagga cgaggctgat 2160
   tattactgcc agtcctatga cagcagcctg agtggttgcc agtcctatga cagcagcctg 2220
   agtggtttgg tgttcggcgg agggaccaag ctgaccgtcc tagcctgaca cacacagccc 2280
   tccccagact gtgggatgag gccctgtgcc cgcagtcaca tgtggaatat caagacacac 2340
   acatctatga caatcacata gagtactcac gctacgtcag tagacgtcag tctgccctga 2400
   ctcagcctgc ctccgtgtct gggtctcctg gacagtcgat caccatctcc tgcgggggaa 2460
   acaacattgg aagtaaaaat gtgcactggt accaacagca cccaggcaaa gcccccaaac 2520
   tcatgattta tagggatagc aaccggccct ctggggtttc taatcgcttc tctggctcca 2580
   agtctggcaa cacggcctcc ctgaccatct ctgggctcca ggctgaggac gaggctgatt 2640
   attactgcca gtcctatgac aggagtggtt gggtgttcgg cggagggacc aagctgaccg 2700
   tcctagcctg acacacacag ccctccccag actgtgggat gaggccctgt gcccgcagtc 2760
   acatgtggaa tatcaagaca cacacatcta tgacaatcac tacgctgtct tagtgtagat 2820
   tcgatcacgt cagtctgccc tgactcagcc tgcctccgtg tctgggtctc ctggacagtc 2880
   gatcaccatc tcctgctctg gagaagcatt gccaaaaaaa tatgcttatt ggtaccaaca 2940
   gcacccaggc aaagccccca aactcatgat ttataaagac agcgagaggc cctcaggggt 3000
   ttctaatcgc ttctctggct ccaagtctgg caacacggcc tccctgacca tctctgggct 3060
   ccaggctgag gacgaggctg attattactg caactctttt acaagcacca ccaattatgt 3120
   cttattcggc ggagggacca agctgaccgt cctagcctga cacacacagc cctccccaga 3180
   ctgtgggatg aggccctgtg cccgcagtca catgtggaat atcaagacac acacatctat 3240
   gacaatcact gacgtatgtt gtgagtagta cagtatatac agtctgccct gactcagcct 3300
   gcctccgtgt ctgggtctcc tggacagtcg atcaccatct cctgcggggg aaacaacatt 3360
   ggaagtaaaa gtgtgcactg gtaccaacag cacccaggca aagcccccaa actcatgatt 3420
   tattatgata gcgaccggcc ctcaggggtt tctaatcgct tctctggctc caagtctggc 3480
   aacacggcct ccctgaccat ctctgggctc caggctgagg acgaggctga ttattactgc 3540
   caggtgtggg atgataatag tgatcatctt atattcggcg gagggaccaa gctgaccgtc 3600
   ctagcctgac acacacagcc ctccccagac tgtgggatga ggccctgtgc ccgcagtcac 3660
   atgtggaata tcaagacaca cacatctatg acaatcacag tatacataag tcgagagaag 3720
   tgctacgaca gtctgccctg actcagcctg cctccgtgtc tgggtctcct ggacagtcga 3780
   tcaccatctc ctgctctgga gatgcattgc caaagcaata tgcttattgg taccaacagc 3840
   acccaggcaa agcccccaaa ctcatgattt atgatgatag cgaccggccc tcaggggttt 3900
   ctaatcgctt ctctggctcc aagtctggca acacggcctc cctgaccatc tctgggctcc 3960
   aggctgagga cgaggctgat tattactgca gctcatatgc aggcagcaac aattgggtgt 4020
   tcggcggagg gaccaagctg accgtcctag cctgacacac acagccctcc ccagactgtg 4080
   ggatgaggcc ctgtgcccgc agtcacatgt ggaatatcaa gacacacaca tctatgacaa 4140
   tcaccgtaca gtcacgtact cagactacgc tctacagtct gccctgactc agcctgcctc 4200
   cgtgtctggg tctcctggac agtcgatcac catctcctgc accctaagca gtgagcacag 4260
   cacctacacc atcgaatggt accaacagca cccaggcaaa gcccccaaac tcatgattta 4320
   tgttaagagt gatggcagcc acagcaaggg ggacggggtt tctaatcgct tctctggctc 4380
   caagtctggc aacacggcct ccctgaccat ctctgggctc caggctgagg acgaggctga 4440
   ttattactgc ggagagagcc acacgattga tggccaagtc ggtgtggtat tcggcggagg 4500
   gaccaagctg accgtcctag cctgacacac acagccctcc ccagactgtg ggatgaggcc 4560
   ctgtgcccgc agtcacatgt ggaatatcaa gacacacaca tctatgacaa tcactatata 4620
   tacatatgct agtatcacgc gagacagtct gccctgactc agcctgcctc cgtgtctggg 4680
   tctcctggac agtcgatcac catctcctgc actctgagca gtgggcacag cagctacgcc 4740
   atcgcatggt accaacagca cccaggcaaa gcccccaaac tcatgattta tcttaacagt 4800
   gatggcagcc acagcaaggg ggacggggtt tctaatcgct tctctggctc caagtctggc 4860
   aacacggcct ccctgaccat ctctgggctc caggctgagg acgaggctga ttattactgc 4920
   agctcatatg caggcaacaa caatttgggg gatgtgcttt tcggcggagg gaccaagctg 4980
   accgtcctag cctgacacac acagccctcc ccagactgtg ggatgaggcc ctgtgcccgc 5040
   agtcacatgt ggaatatcaa gacacacaca tctatgacaa tcactgagtc agtatgtagt 5100
   gtgatgtcac acgacagtct gccctgactc agcctgcctc cgtgtctggg tctcctggac 5160
   agtcgatcac catctcctgc accttgcgca gtggcatcaa tgttggtacc tacaggatat 5220
   actggtacca acagcaccca ggcaaagccc ccaaactcat gatttattac aaatcagact 5280
   cagataagca gcagggctct ggggtttcta atcgcttctc tggctccaag tctggcaaca 5340
   cggcctccct gaccatctct gggctccagg ctgaggacga ggctgattat tactgcgcag 5400
   catgggatga cagcctgaat gtggtattcg gcggagggac caagctgacc gtcctagcct 5460
   gacacacaca gccctcccca gactgtggga tgaggccctg tgcccgcagt cacatgtgga 5520
   atatcaagac acacacatct atgacaatca cacatgacga cacgacagct cacgtctcat 5580
   ccagtctgcc ctgactcagc ctgcctccgt gtctgggtct cctggacagt cgatcaccat 5640
   ctcctgcacc cgcagcagtg gcagcattgc cagcaactat gtgcagtggt accaacagca 5700
   cccaggcaaa gcccccaaac tcatgattta tgaggataac caaagaccct ctggggtttc 5760
   taatcgcttc tctggctcca agtctggcaa cacggcctcc ctgaccatct ctgggctcca      5820
   ggctgaggac gaggctgatt attactgcca gtcctatgac agcagcctga ggagagtctt      5880
   cggcggaggg accaagctga ccgtcctagc ctgacacaca cagccctccc cagactgtgg      5940
   gatgaggccc tgtgcccgca gtcacatgtg gaatatcaag acacacacat ctatgacaat      6000
   cacagcgact agcagtagtg atcagtgaca caccagtctg ccctgactca gcctgcctcc      6060
   gtgtctgggt ctcctggaca gtcgatcacc atctcctgcg gctccagcac tggagctgtc      6120
   accagtggtc attatcccta ctggtaccaa cagcacccag gcaaagcccc caaactcatg      6180
   atttatgata caagcaacaa acactccggg gtttctaatc gcttctctgg ctccaagtct      6240
   ggcaacacgg cctccctgac catctctggg ctccaggctg aggacgaggc tgattattac      6300
   tgcgcaacat gggatgacag cctgaatgag ggagtgttcg gcggagggac caagctgacc      6360
   gtcctagcct gacacacaca gccctcccca gactgtggga tgaggccctg tgcccgcagt      6420
   cacatgtgga atatcaagac acacacatct atgacaatca catctactga ccacgtagat      6480
   ccacgtgtcg ccagtctgcc ctgactcagc ctgcctccgt gtctgggtct cctggacagt      6540
   cgatcaccat ctcctgcacc ctgagcagcg gctacagtaa ttataaagtg gactggtacc      6600
   aacagcaccc aggcaaagcc cccaaactca tgatttatgt gggcactggt gggattgtgg      6660
   gatccaaggg ggatggggtt tctaatcgct tctctggctc caagtctggc aacacggcct      6720
   ccctgaccat ctctgggctc caggctgagg acgaggctga ttattactgc ggggcagacc      6780
   atggcagtgg gagcaacttc gtggtggtat tcggcggagg gaccaagctg accgtcctag      6840
   cctgacacac acagccctcc ccagactgtg ggatgaggcc ctgtgcccgc agtcacatgt      6900
   ggaatatcaa gacacacaca tctatgacaa tcacgtttaa acaagcttcg aagggcccat      6960
   gcgaattccg cggatgcggc gcgcctctag aatgcatcga t                          7001
<210> 62
   <211> 18736
   <212> DNA
   <213> Artificial Sequence
<220>
   <223> Targeting vector
<400> 62
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt 360
   tttcccagtc acgacgttgt aaaacgacgg ccagtgcggc cgcgggtatc acgtggggca 420
   aatcgagagc taccattggc tatggggagg agaaaatgag gcgtgctact tcctcgttcc 480
   tgcagtgcgt gtcgaggtgc cgtgttggtc ggatctcagt ggcagagcag gatggtgagc 540
   ggtggccttg gggccgctcc ctcactctgc tgggctggtg cggtggctcc gggaccggac 600
   tttcagtggt gtttggtgtg gtgctcactg tcctttggtg tcactgtgtg gtgaattccc 660
   tcagctgggc ggtgagagcc catgctgtgt cctggtagga gtagtctggg ctgcagtgcg 720
   ggctctttgt gcgtgttggg ggcggttccc tggaagctaa ttctggaaga aatcccggct 780
   gcctctgtaa cagttgttgg cgatgtcaaa caccctggag ctgctgactg gctctgtacc 840
   attgagtgat gcagtttaaa cctctctctt tcctcctgct gtgtgtgaag ctggtgttag 900
   tgctaggaga cctgcacatc cctcaccgat gcagtggttt accagcaaag ttcaagagcc 960
   tcctggttcc agggaagatt cagcatattt tgtgtacagg aaacctctgc accaaggaga 1020
   gctatgacta cctcaggact ttggctggag atgttcacgt tgttaagggg gactctgagg 1080
   taatgttctt gctgctgtga attcctcagt cctccatctt acaagccgta gttgtcccac 1140
   tgaaagctgg gaaacccttc ggtgtttgca attcctgagc gctaatgtgt gggggaagga 1200
   tgtaggaaca aataaggctc aagagacaac aacttcctag gctcctctta ttgcccttgt 1260
   ggtaagggag gacttggtgt gtagtggtaa tacatgcact caacttatta agttctcata 1320
   gctgctgtga agctgctgca gtccctcaga tccctgcagg aagaggggac tgactgactg 1380
   ttgaatagcc ttgaagctcc tgctctgcat gtggttccgg tcaggctcaa gatcccagtt 1440
   cttagctctt cagttgctct tctgggggta tagtactagc atctgggtca ctgcagatgg 1500
   acagagactg tgcttgggta gcaaaaggca gacttacgtt gtgaaacctt cattgtgttg 1560
   gtgaggttct ttgatttcta ccttgtgtat ctctcttcct cagagtctga attatcctga 1620
   acagaaggtt gtaactgttg ggcagttcag aattgggttg attcatggcc atcaagtcat 1680
   tccctggggt gatgtggcaa gcttggcatt gctgcagagg cagctggatg tagacattct 1740
   tatctcagga catacacaca gatttgaagc atttgagcat gaaaataagt tctacatcaa 1800
   cccaggatca gctacaggag cctacactgg tttacaaacg tgagtacaat gagccttgtg 1860
   tttgtcttta agacagatgt ctgtccttaa gagtgcagcc catgtgttga agagcactgg 1920
   tgagatgcag tgccatgcag gccatctctc cctttcccag agaacacggc ctacctctct 1980
   ctgttcataa ggtcttggtt aacttatgag ttggccagca cagcctggca ctgatgtgtc 2040
   atgatcataa ccctgagccc acgctctgct atgctgttgc ttcttagagt tgcattgcct 2100
   tgaggtgtat tccagaagct gtgtgctgag ctcttgttgg gaaagaggcc ctcagggcac 2160
   tgccaacatc tgaggcttgc aggagctgga acattcagcc ctgagggctg ctctggccaa 2220
   ctgcagctct gagtctgcag tcagtgcctc tgagctgtga tgcttctcag ataactgcag 2280
   ccctttgcaa tgtaatgttt tataatatat ttccatagga catcttggta agaggtgtaa 2340
   gctaatcagg gtgattgaca aagtatgttt tctttccagg aacgtcattc cttcatttgt 2400
   actgatggat atccaggctt ccacagttgt gacttatgta taccagctaa ttgaggacga 2460
   tgtgaaagta gaaagaattg agttcaagaa atactgacac gtgtttgaat ctgcctgctg 2520
   acttctcact gcctctcatt ctttctctcc tagttaaagc tgaatccggc cacgtggggg 2580
   tgctgctgca gcggcgtgta actgtgagct tgggaagttc tcaactaggg cttagagcag 2640
   gagaaactga ttaacagaaa ttgccttatt aaatgtatat atttcctgtg tctaagaacg 2700
   aggtgctgtt gttcctgact tgcggagctg cagctagtgg tagcttggaa tttaacctct 2760
   cctgtaaaat gagcatagaa taaaacagta ttttcacatt tctggatcct gttaattgtt 2820
   tgttaattaa gaggacagga attcagtgtc agggggctgc tctttgcttc ccaatgtgta 2880
   ccactgaagc ctctctggat ttatcagaga tactcaatgg actcttaaca gcactgtggc 2940
   acctgacctc ctgctgcctt cagcctccct tcctggctgg tgtgcttatg ttgaggagct 3000
   gtgggaggct ggtgcagttc tgtgggatga ggtaaggcca gctgaatagc ctgggggctg 3060
   gtcagggggc ttagtgagag gagcagggct ggggaatgtt gtgaacagca ggtggtgcca 3120
   ctgctattct gaggaaggag gcagcttctg caaatgcttt gcaatgcagg atgacaaagt 3180
   gcttctgcta gccctaggct accgttcgta taatgtatac tatacgaagt tattttattt 3240
   gtgaaatttg tgatgctatt gctttatttg taaccattat aagctgcaat aaacaagttg 3300
   gggtgggcga agaactccag catgagatcc ccgcgctgga ggatcatcca gccggcgtcc 3360
   cggaaaacga ttccgaagcc caacctttca tagaaggcgg cggtggaatc gaaatctcgt 3420
   gatggcaggt tgggcgtcgc ttggtcggtc atttcgaacc ccagagtccc gctcagaaga 3480
   actcgtcaag aaggcgatag aaggcgatgc gctgcgaatc gggagcggcg ataccgtaaa 3540
   gcacgaggaa gcggtcagcc cattcgccgc caagctcttc agcaatatca cgggtagcca 3600
   acgctatgtc ctgatagcgg tccgccacac ccagccggcc acagtcgatg aatccagaaa 3660
   agcggccatt ttccaccatg atattcggca agcaggcatc gccatgggtc acgacgagat 3720
   cctcgccgtc gggcatgcgc gccttgagcc tggcgaacag ttcggctggc gcgagcccct 3780
   gatgctcttc gtccagatca tcctgatcga caagaccggc ttccatccga gtacgtgctc 3840
   gctcgatgcg atgtttcgct tggtggtcga atgggcaggt agccggatca agcgtatgca 3900
   gccgccgcat tgcatcagcc atgatggata ctttctcggc aggagcaagg tgagatgaca 3960
   ggagatcctg ccccggcact tcgcccaata gcagccagtc ccttcccgct tcagtgacaa 4020
   cgtcgagcac agctgcgcaa ggaacgcccg tcgtggccag ccacgatagc cgcgctgcct 4080
   cgtcctgcag ttcattcagg gcaccggaca ggtcggtctt gacaaaaaga accgggcgcc 4140
   cctgcgctga cagccggaac acggcggcat cagagcagcc gattgtctgt tgtgcccagt 4200
   catagccgaa tagcctctcc acccaagcgg ccggagaacc tgcgtgcaat ccatcttgtt 4260
   caatcatgcg aaacgatcct catcctgtct cttgatcaga tccgaaaatg gatatacaag 4320
   ctcccgggag ctttttgcaa aagcctaggc ctccaaaaaa gcctcctcac tacttctgga 4380
   atagctcaga ggcagaggcg gcctcggcct ctgcataaat aaaaaaaatt agtcagccat 4440
   ggggcggaga atgggcggaa ctgggcggag ttaggggcgg gatgggcgga gttaggggcg 4500
   ggactatggt tgctgactaa ttgagatgca tgctttgcat acttctgcct gctggggagc 4560
   ctggggactt tccacacctg gttgctgact aattgagatg catgctttgc atacttctgc 4620
   ctgctgggga gcctggggac tttccacacc ctaaataact tcgtataatg tatactatac 4680
   gaacggtagc gttaaccggt cgacatgcaa gcttgctagc gtttaaacac gagtgcgtac 4740
   gctcgacaag acgcactcca gtctgccctg actcagcctg cctccgtgtc tgggtctcct 4800
   ggacagtcga tcaccatctc ctgctctgga agcagctcca acatcggaag taattatgta 4860
   tactggtacc aacagcaccc aggcaaagcc cccaaactca tgatttatag gaataatcag 4920
   cggccctcag gggtttctaa tcgcttctct ggctccaagt ctggcaacac ggcctccctg 4980
   accatctctg ggctccaggc tgaggacgag gctgattatt actgcgcagc atgggatgac 5040
   agcctgagtg gtcattgggt gttcggcgga gggaccaagc tgaccgtcct agcctgacac 5100
   acacagccct ccccagactg tgggatgagg ccctgtgccc gcagtcacat gtggaatatc 5160
   aagacacaca catctatgac aatcaccgtg tctctactcg cgtgtctctc tatgcgcagt 5220
   ctgccctgac tcagcctgcc tccgtgtctg ggtctcctgg acagtcgatc accatctcct 5280
   gcactgggag cagctccaac atcggggcag gttatgatgt acactggtac caacagcacc 5340
   caggcaaagc ccccaaactc atgatttatg gtaacagcaa tcggccctca ggggtttcta 5400
   atcgcttctc tggctccaag tctggcaaca cggcctccct gaccatctct gggctccagg 5460
   ctgaggacga ggctgattat tactgccagt cctatgacag cagcctgagt ggttctgtgg 5520
   tattcggcgg agggaccaag ctgaccgtcc tagcctgaca cacacagccc tccccagact 5580
   gtgggatgag gccctgtgcc cgcagtcaca tgtggaatat caagacacac acatctatga 5640
   caatcacata cgacgtatca cgtactacgt ctagtaccag tctgccctga ctcagcctgc 5700
   ctccgtgtct gggtctcctg gacagtcgat caccatctcc tgctctggaa gcagctccaa 5760
   cattgggaat aattatgtat cctggtacca acagcaccca ggcaaagccc ccaaactcat 5820
   gatttatgac aataataagc gaccctcagg ggtttctaat cgcttctctg gctccaagtc 5880
   tggcaacacg gcctccctga ccatctctgg gctccaggct gaggacgagg ctgattatta 5940
   ctgcggaaca tgggatagca gcctgagtgc tggtgctgtg ttcggcggag ggaccaagct 6000
   gaccgtccta gcctgacaca cacagccctc cccagactgt gggatgaggc cctgtgcccg 6060
   cagtcacatg tggaatatca agacacacac atctatgaca atcaccgtag actagtacga 6120
   gtatgtactc tcgtgcagtc tgccctgact cagcctgcct ccgtgtctgg gtctcctgga 6180
   cagtcgatca ccatctcctg cactgggagc agctccaaca ttggggcggg ttatgttgta 6240
   cattggtacc aacagcaccc aggcaaagcc cccaaactca tgatttatga aaataataag 6300
   cgaccctcag gggtttctaa tcgcttctct ggctccaagt ctggcaacac ggcctccctg 6360
   accatctctg ggctccaggc tgaggacgag gctgattatt actgcagctc atatacaagc 6420
   agcggcactc ctgtggtatt cggcggaggg accaagctga ccgtcctagc ctgacacaca 6480
   cagccctccc cagactgtgg gatgaggccc tgtgcccgca gtcacatgtg gaatatcaag 6540
   acacacacat ctatgacaat cacacgcgag tatactacta tgtactgtac agtcagtctg 6600
   ccctgactca gcctgcctcc gtgtctgggt ctcctggaca gtcgatcacc atctcctgca 6660
   ctggaaccag cagtgatgtt gggagttata accttgtctc ctggtaccaa cagcacccag 6720
   gcaaagcccc caaactcatg atttatgagg gcagtaagcg gccctcaggg gtttctaatc 6780
   gcttctctgg ctccaagtct ggcaacacgg cctccctgac catctctggg ctccaggctg 6840
   aggacgaggc tgattattac tgccagtcct atgacagcag cctgagtggt tgccagtcct 6900
   atgacagcag cctgagtggt ttggtgttcg gcggagggac caagctgacc gtcctagcct 6960
   gacacacaca gccctcccca gactgtggga tgaggccctg tgcccgcagt cacatgtgga 7020
   atatcaagac acacacatct atgacaatca catagagtac tcacgctacg tcagtagacg 7080
   tcagtctgcc ctgactcagc ctgcctccgt gtctgggtct cctggacagt cgatcaccat 7140
   ctcctgcggg ggaaacaaca ttggaagtaa aaatgtgcac tggtaccaac agcacccagg 7200
   caaagccccc aaactcatga tttataggga tagcaaccgg ccctctgggg tttctaatcg 7260
   cttctctggc tccaagtctg gcaacacggc ctccctgacc atctctgggc tccaggctga 7320
   ggacgaggct gattattact gccagtccta tgacaggagt ggttgggtgt tcggcggagg 7380
   gaccaagctg accgtcctag cctgacacac acagccctcc ccagactgtg ggatgaggcc 7440
   ctgtgcccgc agtcacatgt ggaatatcaa gacacacaca tctatgacaa tcactacgct 7500
   gtcttagtgt agattcgatc acgtcagtct gccctgactc agcctgcctc cgtgtctggg 7560
   tctcctggac agtcgatcac catctcctgc tctggagaag cattgccaaa aaaatatgct 7620
   tattggtacc aacagcaccc aggcaaagcc cccaaactca tgatttataa agacagcgag 7680
   aggccctcag gggtttctaa tcgcttctct ggctccaagt ctggcaacac ggcctccctg 7740
   accatctctg ggctccaggc tgaggacgag gctgattatt actgcaactc ttttacaagc 7800
   accaccaatt atgtcttatt cggcggaggg accaagctga ccgtcctagc ctgacacaca 7860
   cagccctccc cagactgtgg gatgaggccc tgtgcccgca gtcacatgtg gaatatcaag 7920
   acacacacat ctatgacaat cactgacgta tgttgtgagt agtacagtat atacagtctg 7980
   ccctgactca gcctgcctcc gtgtctgggt ctcctggaca gtcgatcacc atctcctgcg 8040
   ggggaaacaa cattggaagt aaaagtgtgc actggtacca acagcaccca ggcaaagccc 8100
   ccaaactcat gatttattat gatagcgacc ggccctcagg ggtttctaat cgcttctctg 8160
   gctccaagtc tggcaacacg gcctccctga ccatctctgg gctccaggct gaggacgagg 8220
   ctgattatta ctgccaggtg tgggatgata atagtgatca tcttatattc ggcggaggga 8280
   ccaagctgac cgtcctagcc tgacacacac agccctcccc agactgtggg atgaggccct 8340
   gtgcccgcag tcacatgtgg aatatcaaga cacacacatc tatgacaatc acagtataca 8400
   taagtcgaga gaagtgctac gacagtctgc cctgactcag cctgcctccg tgtctgggtc 8460
   tcctggacag tcgatcacca tctcctgctc tggagatgca ttgccaaagc aatatgctta 8520
   ttggtaccaa cagcacccag gcaaagcccc caaactcatg atttatgatg atagcgaccg 8580
   gccctcaggg gtttctaatc gcttctctgg ctccaagtct ggcaacacgg cctccctgac 8640
   catctctggg ctccaggctg aggacgaggc tgattattac tgcagctcat atgcaggcag 8700
   caacaattgg gtgttcggcg gagggaccaa gctgaccgtc ctagcctgac acacacagcc 8760
   ctccccagac tgtgggatga ggccctgtgc ccgcagtcac atgtggaata tcaagacaca 8820
   cacatctatg acaatcaccg tacagtcacg tactcagact acgctctaca gtctgccctg 8880
   actcagcctg cctccgtgtc tgggtctcct ggacagtcga tcaccatctc ctgcacccta 8940
   agcagtgagc acagcaccta caccatcgaa tggtaccaac agcacccagg caaagccccc 9000
   aaactcatga tttatgttaa gagtgatggc agccacagca agggggacgg ggtttctaat 9060
   cgcttctctg gctccaagtc tggcaacacg gcctccctga ccatctctgg gctccaggct 9120
   gaggacgagg ctgattatta ctgcggagag agccacacga ttgatggcca agtcggtgtg 9180
   gtattcggcg gagggaccaa gctgaccgtc ctagcctgac acacacagcc ctccccagac 9240
   tgtgggatga ggccctgtgc ccgcagtcac atgtggaata tcaagacaca cacatctatg 9300
   acaatcacta tatatacata tgctagtatc acgcgagaca gtctgccctg actcagcctg 9360
   cctccgtgtc tgggtctcct ggacagtcga tcaccatctc ctgcactctg agcagtgggc 9420
   acagcagcta cgccatcgca tggtaccaac agcacccagg caaagccccc aaactcatga 9480
   tttatcttaa cagtgatggc agccacagca agggggacgg ggtttctaat cgcttctctg 9540
   gctccaagtc tggcaacacg gcctccctga ccatctctgg gctccaggct gaggacgagg 9600
   ctgattatta ctgcagctca tatgcaggca acaacaattt gggggatgtg cttttcggcg 9660
   gagggaccaa gctgaccgtc ctagcctgac acacacagcc ctccccagac tgtgggatga 9720
   ggccctgtgc ccgcagtcac atgtggaata tcaagacaca cacatctatg acaatcactg 9780
   agtcagtatg tagtgtgatg tcacacgaca gtctgccctg actcagcctg cctccgtgtc 9840
   tgggtctcct ggacagtcga tcaccatctc ctgcaccttg cgcagtggca tcaatgttgg 9900
   tacctacagg atatactggt accaacagca cccaggcaaa gcccccaaac tcatgattta 9960
   ttacaaatca gactcagata agcagcaggg ctctggggtt tctaatcgct tctctggctc 10020
   caagtctggc aacacggcct ccctgaccat ctctgggctc caggctgagg acgaggctga 10080
   ttattactgc gcagcatggg atgacagcct gaatgtggta ttcggcggag ggaccaagct 10140
   gaccgtccta gcctgacaca cacagccctc cccagactgt gggatgaggc cctgtgcccg 10200
   cagtcacatg tggaatatca agacacacac atctatgaca atcacacatg acgacacgac 10260
   agctcacgtc tcatccagtc tgccctgact cagcctgcct ccgtgtctgg gtctcctgga 10320
   cagtcgatca ccatctcctg cacccgcagc agtggcagca ttgccagcaa ctatgtgcag 10380
   tggtaccaac agcacccagg caaagccccc aaactcatga tttatgagga taaccaaaga 10440
   ccctctgggg tttctaatcg cttctctggc tccaagtctg gcaacacggc ctccctgacc 10500
   atctctgggc tccaggctga ggacgaggct gattattact gccagtccta tgacagcagc 10560
   ctgaggagag tcttcggcgg agggaccaag ctgaccgtcc tagcctgaca cacacagccc 10620
   tccccagact gtgggatgag gccctgtgcc cgcagtcaca tgtggaatat caagacacac 10680
   acatctatga caatcacagc gactagcagt agtgatcagt gacacaccag tctgccctga 10740
   ctcagcctgc ctccgtgtct gggtctcctg gacagtcgat caccatctcc tgcggctcca 10800
   gcactggagc tgtcaccagt ggtcattatc cctactggta ccaacagcac ccaggcaaag 10860
   cccccaaact catgatttat gatacaagca acaaacactc cggggtttct aatcgcttct 10920
   ctggctccaa gtctggcaac acggcctccc tgaccatctc tgggctccag gctgaggacg 10980
   aggctgatta ttactgcgca acatgggatg acagcctgaa tgagggagtg ttcggcggag 11040
   ggaccaagct gaccgtccta gcctgacaca cacagccctc cccagactgt gggatgaggc 11100
   cctgtgcccg cagtcacatg tggaatatca agacacacac atctatgaca atcacatcta 11160
   ctgaccacgt agatccacgt gtcgccagtc tgccctgact cagcctgcct ccgtgtctgg 11220
   gtctcctgga cagtcgatca ccatctcctg caccctgagc agcggctaca gtaattataa 11280
   agtggactgg taccaacagc acccaggcaa agcccccaaa ctcatgattt atgtgggcac 11340
   tggtgggatt gtgggatcca agggggatgg ggtttctaat cgcttctctg gctccaagtc 11400
   tggcaacacg gcctccctga ccatctctgg gctccaggct gaggacgagg ctgattatta 11460
   ctgcggggca gaccatggca gtgggagcaa cttcgtggtg gtattcggcg gagggaccaa 11520
   gctgaccgtc ctagcctgac acacacagcc ctccccagac tgtgggatga ggccctgtgc 11580
   ccgcagtcac atgtggaata tcaagacaca cacatctatg acaatcacgt ttaaacaagc 11640
   ttcgaagggc ccatgcgaat tccgcggatg cggcgcgcct ctagaatgca tcgatttaaa 11700
   tcgagtgaag gtcaccaagg atggagactt cagagctatc tgggacacgt gttttaacgt 11760
   ttgatcagcc ttgcagcaaa acagtgtttt gctctattga acagtgttca acaaaaacga 11820
   tcagtggtct ggaacacatc tcctacgaag aaaggttgcg agagctgggt ctggagaaga 11880
   ggaggctctg gggcaacctc cctgtggcct gtcagtgctt aaaggaggct caagagaaag 11940
   atggagagag actttataca cggagctgta gcaatatgac aaggagaaat ggtttaaaat 12000
   ggaaagaggg tagatttagg ttagctatca agaagaaatt cttcactcag aggtgatgaa 12060
   gcactgtagc aggttgccca gagaagtatt ggatgcttcc ttcctggcag ggcccaaggc 12120
   cagactggat gggttctggg cagcctgttc tggtgggaga tgtccctgcc cgtggcacgg 12180
   cttggaaatg ggtagccttt gaggtccctc tcagcccaaa tcattctatg ttcttgtggt 12240
   tccctgcatt tcagcctttg cctgttgact ctggtgccac tgttggacaa ttctactcag 12300
   ctgtgtttca tccctgcccc aaagcagtaa tcccatggat aagcaatgct ctgaggcctt 12360
   caccaccatc agggctcctg ctgaccctag cggcctccct tgcagcatga gccctttgtt 12420
   gtccgcactg ctgctgctga gggtcctgcc agccccccag ggccttttct gcccaggcac 12480
   tgccacctgc taaaacccag cctgcccatg gcagggcatc gcatgtctga gggatggaac 12540
   ggggcaaggg cagtgctgca ggcagtggtt caagaaacgt ttagatgttg tactgaggga 12600
   tgtggtttag tgggaaatac tggtgatagg tggatggttg gactggagga tcttagaggt 12660
   cttttccaac cttggtgatt atgtgattct atgaagggag ccatagcctg ctcccatcat 12720
   gccccgccag tccggctctc ccgagcactt tggttccgca gcatccaggc ccgctctggg 12780
   gccggtgtgg gcctgggtgc tgccacgtgt ccccgctgca aggcctggcc aaccccgctg 12840
   tgccacaatg caggggctgg gctaggagtg ggcagggagg tgtggggaga ggtgtaggac 12900
   gtggctggga gcgcagggag ttatttgcat aggggggcgt gctgcggaag gacgcgggta 12960
   taaaagggca tcgaggtccc cggcacagcc ccatcggcgt ggggacacac agctgctggg 13020
   attccgccat ggcctgggct cctctcctcc tggcggtgct cgcccacacc tcaggtactc 13080
   gttgcgcccg gtcggggact gtgggcacgg ggctctgtcc cactgctgcg cgggcagggc 13140
   tgtgcgtgcg gggccgtcac tgattgccgt tttctcccct ctctcctctc cggcgcgcct 13200
   ctccaggttc cctggtgcag gcacagtctg ccctgactca gcctgcctcc gtgtctgggt 13260
   ctcctggaca gtcgatcacc atctcctgca ctggaaccag cagtgacgtt ggtggttata 13320
   actatgtctc ctggtaccaa caaaacccag gcaaagcccc caaactcatg atttatgatg 13380
   tcagtaatcg gccctcaggg atttctaatc gcttctctgg ctccaagtct ggcaacacgg 13440
   cctccctgac catctctggg ctccaggctg acgacgaggc tgattattac tgcacctcat 13500
   atacaaacga cagcaattct caggtattcg gcggagggac caagctgacc gtcctaggtg 13560
   agtcgctcat atgctaccgt tcgtatagca tacattatac gaagttatca gacatgataa 13620
   gatacattga tgagtttgga caaaccacaa ctagaatgca gtgaaaaaaa tgctttattt 13680
   gtgaaatttg tgatgctatt gctttatttg taaccattat aagctgcaat aaacaagttg 13740
   gggtgggcga agaactccag catgagatcc ccgcgctgga ggatcatcca gccggcgtcc 13800
   cggaaaacga ttccgaagcc caacctttca tagaaggcgg cggtggaatc gaaatctcgt 13860
   agcacgtgtc agtcctgctc ctcggccacg aagtgcttag ccctcccaca cataaccaga 13920
   gggcagcaat tcacgaatcc caactgccgt cggctgtcca tcactgtcct tcactatggc 13980
   tttgatccca ggatgcagat cgagaagcac ctgtcggcac cgtccgcagg ggctcaagat 14040
   gcccctgttc tcatttccga tcgcgacgat acaagtcagg ttgccagctg ccgcagcagc 14100
   agcagtgccc agcaccacga gttctgcaca aggtccccca gtaaaatgat atacattgac 14160
   accagtgaag atgcggccgt cgctagagag agctgcgctg gcgacgctgt agtcttcaga 14220
   gatggggatg ctgttgattg tagccgttgc tctttcaatg agggtggatt cttcttgaga 14280
   caaaggcttg gccatggttt agttcctcac cttgtcgtat tatactatgc cgatatacta 14340
   tgccgatgat taattgtcaa cacgtgctga tcagatccga aaatggatat acaagctccc 14400
   gggagctttt tgcaaaagcc taggcctcca aaaaagcctc ctcactactt ctggaatagc 14460
   tcagaggcag aggcggcctc ggcctctgca taaataaaaa aaattagtca gccatggggc 14520
   ggagaatggg cggaactggg cggagttagg ggcgggatgg gcggagttag gggcgggact 14580
   atggttgctg actaattgag atgcatgctt tgcatacttc tgcctgctgg ggagcctggg 14640
   gactttccac acctggttgc tgactaattg agatgcatgc tttgcatact tctgcctgct 14700
   ggggagcctg gggactttcc acaccctaac tgacacacat tccacagata acttcgtata 14760
   gcatacatta tacgaacggt agctctagag acctcgtctc ggtctttctt cccccatcgt 14820
   gaaattgtga cattttgtcg atttttggtg atttgggggt ttttcttgga cttggcggca 14880
   ggctggggtc tgccaccggc gcagggccgg gcactcagcg cgacagcctg ggctgagtct 14940
   tgtccccacc gagccggagg gctccggtgt gcgccatgga ggacttaggg ttattttgtc 15000
   aatggaaagt tcttaaaatt tgaccagaaa atgtgcccga ggtctgtctc tgccacacga 15060
   tttcagaaat tgtgtctagg tcgatgagaa gacagttttt gtctttgtca ggaaattagt 15120
   tgtgagttgt tagtccttcc ctcttagtcc taaggactaa gacctttgtc cccggtctgg 15180
   tctctcactg gggactcttg gctccagtgc catggggagc ccaagtgtca ctgacacggt 15240
   gtccttgggg gtgaaattca gtttttcagc tgtccaggta agtgctgtca aaaaatggag 15300
   gttattctgc tgaaaaagct gagaggaata ttttgtcatt tttttcggaa atatatatat 15360
   atatatatat ataaatatat aaattattta tatattttat atatatatat atataaaata 15420
   tatgtatttc tctctttctt tttatatata tatataaata tatatatatt tctccctctt 15480
   tctttatata tatttatagg gagagaatct atatttttgg ccaatttggc caatttctct 15540
   ctctctctct ttatatatat atatatatgt atatgtaatt tatatatata tatataaatt 15600
   tatatttatt tatttattta tttatataga ttatttttat atacatatat atatataaat 15660
   ttggccaatt tagaagaaat tggccaaaaa aacccaatct gaaccaaatt acttagggca 15720
   taagtccaaa aaaaaagtgc agttcaagaa ttcctcgctg gaaagttttt aatgggggca 15780
   gttcttcccc attttctggt gtagatatga gtgaagatat agatatgtat gtagatatag 15840
   atttggctat agtttgtttg atgaacagtc tgaggaagag taaatcttgc cttcctcatg 15900
   gcaattttta attctacttc ttctgagaca agtctgaaca tgccttgagg tagaaagagc 15960
   ccgaaaattg agtgcttttt ctttgaaatt ttaattaaaa ttaaaaaata gccttacagc 16020
   aggaaagaaa gggggtgatg gcagctaatc ggagttccaa agctgaacca cgttcaccca 16080
   aacacgtggt tgaaattttg tgtgtgtata tatataaata tatatatgta tacaaaagaa 16140
   aatgcctata tataaaaaaa ttgttatgta tattatatag atttattata gatatgtaca 16200
   taagatataa cacgtataca tatatatgta gtctgctgta tgctttgtat aaatttttat 16260
   agatatgatg tatattatat gcatgggtta tatatctata atatatttta tctgtagaag 16320
   gaactacagt ataaaatgca tatatgcgta tatatacaca tatttatata tatgtgtata 16380
   tatatgtata cgcacacgta taacatccat gaaaatagat atggatgggt ggatgtgttt 16440
   gttttacaga ggtgcatgtg tgtctgtaca tacacagcca tacatacgcg tgtgcggccg 16500
   caggcgtaat catggtcata gctgtttcct gtgtgaaatt gttatccgct cacaattcca 16560
   cacaacatac gagccggaag cataaagtgt aaagcctggg gtgcctaatg agtgagctaa 16620
   ctcacattaa ttgcgttgcg ctcactgccc gctttccagt cgggaaacct gtcgtgccag 16680
   ctgcattaat gaatcggcca acgcgcgggg agaggcggtt tgcgtattgg gcgctcttcc 16740
   gcttcctcgc tcactgactc gctgcgctcg gtcgttcggc tgcggcgagc ggtatcagct 16800
   cactcaaagg cggtaatacg gttatccaca gaatcagggg ataacgcagg aaagaacatg 16860
   tgagcaaaag gccagcaaaa ggccaggaac cgtaaaaagg ccgcgttgct ggcgtttttc 16920
   cataggctcc gcccccctga cgagcatcac aaaaatcgac gctcaagtca gaggtggcga 16980
   aacccgacag gactataaag ataccaggcg tttccccctg gaagctccct cgtgcgctct 17040
   cctgttccga ccctgccgct taccggatac ctgtccgcct ttctcccttc gggaagcgtg 17100
   gcgctttctc atagctcacg ctgtaggtat ctcagttcgg tgtaggtcgt tcgctccaag 17160
   ctgggctgtg tgcacgaacc ccccgttcag cccgaccgct gcgccttatc cggtaactat 17220
   cgtcttgagt ccaacccggt aagacacgac ttatcgccac tggcagcagc cactggtaac 17280
   aggattagca gagcgaggta tgtaggcggt gctacagagt tcttgaagtg gtggcctaac 17340
   tacggctaca ctagaagaac agtatttggt atctgcgctc tgctgaagcc agttaccttc 17400
   ggaaaaagag ttggtagctc ttgatccggc aaacaaacca ccgctggtag cggtggtttt 17460
   tttgtttgca agcagcagat tacgcgcaga aaaaaaggat ctcaagaaga tcctttgatc 17520
   ttttctacgg ggtctgacgc tcagtggaac gaaaactcac gttaagggat tttggtcatg 17580
   agattatcaa aaaggatctt cacctagatc cttttaaatt aaaaatgaag ttttaaatca 17640
   atctaaagta tatatgagta aacttggtct gacagttacc aatgcttaat cagtgaggca 17700
   cctatctcag cgatctgtct atttcgttca tccatagttg cctgactccc cgtcgtgtag 17760
   ataactacga tacgggaggg cttaccatct ggccccagtg ctgcaatgat accgcgagac 17820
   ccacgctcac cggctccaga tttatcagca ataaaccagc cagccggaag ggccgagcgc 17880
   agaagtggtc ctgcaacttt atccgcctcc atccagtcta ttaattgttg ccgggaagct 17940
   agagtaagta gttcgccagt taatagtttg cgcaacgttg ttgccattgc tacaggcatc 18000
   gtggtgtcac gctcgtcgtt tggtatggct tcattcagct ccggttccca acgatcaagg 18060
   cgagttacat gatcccccat gttgtgcaaa aaagcggtta gctccttcgg tcctccgatc 18120
   gttgtcagaa gtaagttggc cgcagtgtta tcactcatgg ttatggcagc actgcataat 18180
   tctcttactg tcatgccatc cgtaagatgc ttttctgtga ctggtgagta ctcaaccaag 18240
   tcattctgag aatagtgtat gcggcgaccg agttgctctt gcccggcgtc aatacgggat 18300
   aataccgcgc cacatagcag aactttaaaa gtgctcatca ttggaaaacg ttcttcgggg 18360
   cgaaaactct caaggatctt accgctgttg agatccagtt cgatgtaacc cactcgtgca 18420
   cccaactgat cttcagcatc ttttactttc accagcgttt ctgggtgagc aaaaacagga 18480
   aggcaaaatg ccgcaaaaaa gggaataagg gcgacacgga aatgttgaat actcatactc 18540
   ttcctttttc aatattattg aagcatttat cagggttatt gtctcatgag cggatacata 18600
   tttgaatgta tttagaaaaa taaacaaata ggggttccgc gcacatttcc ccgaaaagtg 18660
   ccacctgacg tctaagaaac cattattatc atgacattaa cctataaaaa taggcgtatc 18720
   acgaggccct ttcgtc 18736
<210> 63
   <211> 10494
   <212> DNA
   <213> Artificial Sequence
<220>
   <223> Targeting vector
<400> 63
   cacctgacgc gccctgtagc ggcgcattaa gcgcggcggg tgtggtggtt acgcgcagcg 60
   tgaccgctac acttgccagc gccctagcgc ccgctccttt cgctttcttc ccttcctttc 120
   tcgccacgtt cgccggcttt ccccgtcaag ctctaaatcg ggggctccct ttagggttcc 180
   gatttagtgc tttacggcac ctcgacccca aaaaacttga ttagggtgat ggttcacgta 240
   gtgggccatc gccctgatag acggtttttc gccctttgac gttggagtcc acgttcttta 300
   atagtggact cttgttccaa actggaacaa cactcaaccc tatctcggtc tattcttttg 360
   atttataagg gattttgccg atttcggcct attggttaaa aaatgagctg atttaacaaa 420
   aatttaacgc gaattttaac aaaatattaa cgcttacaat ttccattcgc cattcaggct 480
   gcgcaactgt tgggaagggc gatcggtgcg ggcctcttcg ctattacgcc agctggcgaa 540
   agggggatgt gctgcaaggc gattaagttg ggtaacgcca gggttttccc agtcacgacg 600
   ttgtaaaacg acggccagtg aattgtaata cgactcacta tagggcgaat tggaatgttg 660
   gcgcagaagg gattcatatc aattaattcc ctgtcctcaa ttaatgcata attgggagtg 720
   cgatggaatc attcaataag taatgaggac ttggtgtcaa aatgccaatt aagtaattaa 780
   tgacacccaa ttaatacaca atggggatta cagtggaata actcagtgaa gttcatatct 840
   aaattgcaat tgaataatta attacagcta attaatggat gggagactgg cagtggtata 900
   acttaatgaa ggaacacagt gaattcctgt ctgaattgaa attaattgat ttattagggc 960
   taattaatga atggtggggt taaaaatagc attactcagc gaaggaacat gagttcatgg 1020
   ctaaattgag attgaattat taattacaac taattgatta atgactgatg ggagtaagaa 1080
   tggaataact cagtgaagga acacaatgga ttcatggcct aattgaaatt aaatggttaa 1140
   ttagagctaa ttaatgaatg atggggggtg gcaatggaat gatacaagaa agaaacacaa 1200
   tgggttcgtg tctacattga aattaaataa ttaattacag ctaattaata aacggcgagg 1260
   ttgacaatgg tataactgtg aagagacaga atgagttcgt gactaaattg gtattaaatg 1320
   attaattaga gctaattaac aggtggtggg gctgagaatg gcataactca atgggggatg 1380
   atgagctgat ggcttaattg gaattaataa ttaatcacaa ccaattaaca ggtgctgtta 1440
   atggggttac aggggcatga cttcaggaag aagcacaaag tggccattaa ataattaatt 1500
   gcacccaatt aattcctgat ggggattact gcagaggaac tcagcgaagg attcaagcag 1560
   aaatgacaca gttaggttgc tcaattgcgt taattaatta aaatcgctaa ttaggtgagc 1620
   ttcattaagg cctgatgggg ttcatcccaa cgctgggatc aaagaactca gcgcagagtg 1680
   gataacgata attagaaggg gacaaattaa tgaggaaaca cgactttggg cctagacggc 1740
   cattaaatca ttaatcaacc cgacgggcgc ccaattaatc ataaagttat taagcaatta 1800
   agtcgaggct gacgagaaac cgcaacgaga gccctccacg ctcctttgag ttggcgcaaa 1860
   caacaaacaa cgcgggctcg ttaagcagcg acgacgccga gcgtaacgaa gcaatccccg 1920
   cgcccgacaa ttaacgcgct aacgaagccg gagccctcct tatgcaaatt agcccctcca 1980
   gagggccata aaagcgccgg ctctccgacg gcgcgggagg agcaccagtc ggctccgcaa 2040
   ccatgagccc actcgtctcc tccctcctgc tcctggccgc cctgccaggt gagggcgctg 2100
   tggggctcta tggggctcta tggggtctca gcggggctct gcgggctcaa tgggggccaa 2160
   agggggggtc tgcgggctct atgggggggt caacgggggg tctcacgggg ggccggctcc 2220
   gcgaggccgt gtggcggcgg ctccgtcagc gctctctggg cgcgcctcct tccccacagg 2280
   gctgatggcg gaggtgcagc tgttggagtc tgggggaggc ttggtacagc ctggggggtc 2340
   cctgagactc tcctgtgcag cctctggatt cacctttagc agctatgcca tgagctgggt 2400
   ccgccaggct ccagggaagg ggctggagtg ggtctcagct attagtggta gtggtggtag 2460
   cacatactac gcagactccg tgaagggccg gttcaccatc tccagagaca attccaagaa 2520
   cacgctgtat ctgcaaatga acagcctgag agccgaggac acggccgtat attactgtgc 2580
   gaaaggatat agtggctacg attactactt tgactactgg ggccagggca ccctggtcac 2640
   cgtctcctca ggtgagtctt catatgaacc ccccccaaaa ttgccgcggc gattttgggg 2700
   caaaatcggg cgattttatg gatccccgct accgttcgta tagcatacat tatacgaagt 2760
   tatcagacat gataagatac attgatgagt ttggacaaac cacaactaga atgcagtgaa 2820
   aaaaatgctt tatttgtgaa atttgtgatg ctattgcttt atttgtaacc attataagct 2880
   gcaataaaca agttggggtg ggcgaagaac tccagcatga gatccccgcg ctggaggatc 2940
   atccagccgg cgtcccggaa aacgattccg aagcccaacc tttcatagaa ggcggcggtg 3000
   gaatcgaaat ctcgtagcac gtgtcagtcc tgctcctcgg ccacgaagtg cttagccctc 3060
   ccacacataa ccagagggca gcaattcacg aatcccaact gccgtcggct gtccatcact 3120
   gtccttcact atggctttga tcccaggatg cagatcgaga agcacctgtc ggcaccgtcc 3180
   gcaggggctc aagatgcccc tgttctcatt tccgatcgcg acgatacaag tcaggttgcc 3240
   agctgccgca gcagcagcag tgcccagcac cacgagttct gcacaaggtc ccccagtaaa 3300
   atgatataca ttgacaccag tgaagatgcg gccgtcgcta gagagagctg cgctggcgac 3360
   gctgtagtct tcagagatgg ggatgctgtt gattgtagcc gttgctcttt caatgagggt 3420
   ggattcttct tgagacaaag gcttggccat ggtttagttc ctcaccttgt cgtattatac 3480
   tatgccgata tactatgccg atgattaatt gtcaacacgt gctgatcaga tccgaaaatg 3540
   gatatacaag ctcccgggag ctttttgcaa aagcctaggc ctccaaaaaa gcctcctcac 3600
   tacttctgga atagctcaga ggcagaggcg gcctcggcct ctgcataaat aaaaaaaatt 3660
   agtcagccat ggggcggaga atgggcggaa ctgggcggag ttaggggcgg gatgggcgga 3720
   gttaggggcg ggactatggt tgctgactaa ttgagatgca tgctttgcat acttctgcct 3780
   gctggggagc ctggggactt tccacacctg gttgctgact aattgagatg catgctttgc 3840
   atacttctgc ctgctgggga gcctggggac tttccacacc ctaactgaca cacattccac 3900
   agataacttc gtatagcata cattatacga acggtagcct gggtcagtcg aagggggcgg 3960
   tcggtccatc atttgcggcc gggtgatttt tggggccgaa aagtgggaat ttggggccca 4020
   atttggggcc caatttgggg cccaatttgg gttttcgagg ggggattttt ttagggggag 4080
   attttgggtc cccggaaaat ggtgattttg ggtcaaaaat ggggattttg gggtcgtttt 4140
   gaggtggggt tttttggggt agaaatggcg gtctgagggg aaaatgtcgt tttggggcca 4200
   ttttgggcca tttgagggga aatttgggtc aaaaaatgac gattttgggt cgttttaggg 4260
   aggaaaatga atttagggcg atttgagcca aatttgggtc aaaaatggcg attttgagtc 4320
   aaaaaatgat tattttgggt cattttaggg aggaaaatga atttagggag attggacggc 4380
   aaatttgggt caaaaaatgg tgattttggg tcaaaaaatg attattttgg gtcattttag 4440
   ggagaaaaat gaatttaggg agatttgagg gcaaatttgg gtcaaaaaat ggtgattttg 4500
   ggtcaaaaaa tgattatttt gggtcatttt agggagaaaa atgaatttag ggagatttga 4560
   gggcaaattt gggtcgaaaa atggcgattt tgggtgaaaa atggacaatt ttgggtcatt 4620
   ttagggttaa aaatgaattt agggagattg gacggcaaat ttgggtcaaa aaatggtgat 4680
   ttggggtcaa aaaataatta ttttgggtca ttttagggat aaaaaatgaa tttagggaga 4740
   tttgagggca aatttgggtc gaaaaatggt gatttggggt gaaaaatgga caattttggg 4800
   tcattttagg gataaaaaat gaatttaggg cgatttgagc caaatttggg tcaaaaatgg 4860
   cgattttgag tcaaaaaatg attattttgg gtcattttag ggttaaaaat gaatttaggg 4920
   agattggacg gcaaatttgg gtcaaaaaat ggtgattttc ggtcaaaaaa tgattatttt 4980
   gggtcatttt agggagaaaa atgaatttag ggagattgga cggcaaattt gggtcgaaaa 5040
   atggtgattt tgggtcaaaa atggacaatt tggggtcatt ttagggataa aaatgaattt 5100
   agggagattt gagagcaaat ttgggtctaa aaatgtcaat ttggggtcaa aaatggtgat 5160
   tttgggtcat tttagggata taaatgaatt ttgggagatt tgagggcaaa tttgggtcga 5220
   aaaatggtga tttggggtca atttagggag gaaaatgaat ttaaggtgat ttgacggcaa 5280
   atttgggttg aaaaatgaca atttggggtc attttaaaga taaaatgaat ttagggcgat 5340
   ttgagggcaa atttgggtcg aaaaatggtg atttgggtca aaaaatgaca attttgggtc 5400
   attttaggga tataaatgga cttagagcga tttgagggca aatttggttg aaaaaatgga 5460
   caatttgggt cattttaggg atataaatga atttagggag atttgatggc aaatttgggt 5520
   cgaaaaatgg tgatttgggt caaaaatggt gattttggtt gaaaaacagt catttggggt 5580
   cattttaggg attaaaatga atttcgggag atttgagggc aaatttgggt caaaaaatgg 5640
   tgattttggg tcaattagtg ataaaaaaat gaatttaggg tgatttgagg gcaaatttgg 5700
   gtcaaaaaat gacaatttgg ggttttttta gggaggagaa tgaatttagg gagttttgag 5760
   ggcaaatttg ggtcaaaaaa tagtgatttg ggtcaaaagt ggtgattttg gttgaaaaac 5820
   agtcattttg ggtcatttta gggagaaaaa tgaatttcgg gagatttgag ggcaaatttg 5880
   ggtcaaaaaa tgacaatttt gggtcacttt acgaattaaa atgaatttag ggagatttga 5940
   gggcaaattt gggtcaaaat atggtgattt tgggtcaatt tagggaggaa aatgaattta 6000
   gggcgatttg aaggcaaatt tgggtcgaaa atggtgattt tgggtcaaaa aatgatgatt 6060
   ttgggtcgtt ttagggagaa aatgaattta gggagatttg agggcaaatt tgggtcaaaa 6120
   aatggtgatt ttgggtcatt gtagggataa aaatgaattt ggggcgattt gagggcaaat 6180
   ttgggtggaa aatggacaat tttgggtcat tttagggaga aaaatgaatt tagggagatt 6240
   tgagggcaaa tttgggtcaa aaaatggtga tttggggtca ttttcagaag aaaaaatgat 6300
   tattttcccc actaaaaatg tatattttgg ggccaaatgg tgaaaaatgg tgatttttaa 6360
   tcaaacgtcc ccaaaattgg ggaaatttca tcgatttgac ccaaaattga gttttttttc 6420
   cctgttaaaa atgtacattt tggggtcaat cgctgaaatg ttcccacttt tcacttcttt 6480
   gcccccaaat tttgctgtcc ggtgagaaat taattaatta atgatcatta attgatcgac 6540
   aattaatggt tattaattaa ttaataggtc ctcttttggt gactccttcg cttttggggc 6600
   caaaagtcca taaattggcc ccaaaaaatt aacgctgagt aattggattc caaagtatta 6660
   atgataaaca ttaaaagtgt ttaattaata atgatattaa acataatttc gtttttatta 6720
   tcgatttatc aacaacgatg aacgataata ctttacaaca atcgctaata attaattaat 6780
   tatttaatta attaatttct aataattact tcgcattatc ggacacgaga tgttgtaatt 6840
   attaataatt taattcctaa taattagaag attcgttgaa aatggtactt atctttacaa 6900
   ataatcactt ctaataataa tgattaataa tagttaataa caacaatgac gataatgacg 6960
   ataatattaa taatatgtga tatatttaat ataaaattcg tattaatata ttatatctac 7020
   aaaatatgat ataaaatata atattttatt tatatataac acaatttatt attattatca 7080
   ttattatcat tattaatatc atcattatta acgttatcga aatacttatt tagaaataat 7140
   aaaaacggat ttaatgatgg caacaaaaat attttattaa tgttaaaaaa aaataattaa 7200
   taatttccaa agattcgaat tcggggcaac gaacggcact cgataatttt taattaatta 7260
   atagtttgaa ttaatcggta ctttttaatc ctccattttg cccgaaatcg ccgttttttg 7320
   ccccaaattc cccaccgcgg cgttaaaaac ataaagaaat taagcatcaa aagtgccctt 7380
   ttttggggtt gttttgaccc aaaaaaaaaa tggccgaatt gggggcggcc gttttacggt 7440
   tgggttcatt ttgggttcaa aacagccaaa aatgggaact ttgggtttcg aaaacaacaa 7500
   caacaaaaaa acgggtttat tttgggctca ttttgggtgt ttttgggtca ggaggagaaa 7560
   aaataggaag tttgagagcg aaacaacggc cgcttttggg gggaaaacgg ccctttttgg 7620
   tcaacggcgg gggaaaaaaa aaagcggagt ttttggggtg aaaaagagcg gttttgggta 7680
   aatttgggtt ttggggtaaa agtggaggat tttgggcaat gggagttaaa aaatgggtgt 7740
   ttttatgggg gttcggtgca gtttttcctg tttgatgggg ggtttattaa tccggggggg 7800
   gggattaatg agaattaata atgttaatag aaatatctgg gaaattaata gcaattatta 7860
   attgttaata gttattaata gttctatata tctcacatct acgatacaat ataatatcgt 7920
   tacaatcata tagtcgatat attacatata attatcagta ataataataa gtaacaataa 7980
   ttagcagtaa ttaataataa taattaatag tattcgttaa taagattatt gataataatt 8040
   aagtagtagc gattaataga gatgggattt cgtgagaaat ggaccaaatt tgggccgttt 8100
   tgacccaaat ttttggtggt tttttttccg attctttgtg aatttcgggt cggattcatc 8160
   agcaattaat tacggttatt aggggctatt agaggctttt aattgggatt attagagact 8220
   tttaagcgga tttggggact tttaagtgga ttttatgatt cttaagtgga ttttgggtgg 8280
   attttaccgc ttttgccagc ttttgttccc tttagtgagg gttaatttcg agcttggcgt 8340
   aatcatggtc atagctgttt cctgtgtgaa attgttatcc gctcacaatt ccacacaaca 8400
   tacgagccgg aagcataaag tgtaaagcct ggggtgccta atgagtgagc taactcacat 8460
   taattgcgtt gcgctcactg cccgctttcc agtcgggaaa cctgtcgtgc cagctgcatt 8520
   aatgaatcgg ccaacgcgcg gggagaggcg gtttgcgtat tgggcgctct tccgcttcct 8580
   cgctcactga ctcgctgcgc tcggtcgttc ggctgcggcg agcggtatca gctcactcaa 8640
   aggcggtaat acggttatcc acagaatcag gggataacgc aggaaagaac atgtgagcaa 8700
   aaggccagca aaaggccagg aaccgtaaaa aggccgcgtt gctggcgttt ttccataggc 8760
   tccgcccccc tgacgagcat cacaaaaatc gacgctcaag tcagaggtgg cgaaacccga 8820
   caggactata aagataccag gcgtttcccc ctggaagctc cctcgtgcgc tctcctgttc 8880
   cgaccctgcc gcttaccgga tacctgtccg cctttctccc ttcgggaagc gtggcgcttt 8940
   ctcatagctc acgctgtagg tatctcagtt cggtgtaggt cgttcgctcc aagctgggct 9000
   gtgtgcacga accccccgtt cagcccgacc gctgcgcctt atccggtaac tatcgtcttg 9060
   agtccaaccc ggtaagacac gacttatcgc cactggcagc agccactggt aacaggatta 9120
   gcagagcgag gtatgtaggc ggtgctacag agttcttgaa gtggtggcct aactacggct 9180
   acactagaag gacagtattt ggtatctgcg ctctgctgaa gccagttacc ttcggaaaaa 9240
   gagttggtag ctcttgatcc ggcaaacaaa ccaccgctgg tagcggtggt ttttttgttt 9300
   gcaagcagca gattacgcgc agaaaaaaag gatctcaaga agatcctttg atcttttcta 9360
   cggggtctga cgctcagtgg aacgaaaact cacgttaagg gattttggtc atgagattat 9420
   caaaaaggat cttcacctag atccttttaa attaaaaatg aagttttaaa tcaatctaaa 9480
   gtatatatga gtaaacttgg tctgacagtt accaatgctt aatcagtgag gcacctatct 9540
   cagcgatctg tctatttcgt tcatccatag ttgcctgact ccccgtcgtg tagataacta 9600
   cgatacggga gggcttacca tctggcccca gtgctgcaat gataccgcga gacccacgct     9660
   caccggctcc agatttatca gcaataaacc agccagccgg aagggccgag cgcagaagtg     9720
   gtcctgcaac tttatccgcc tccatccagt ctattaattg ttgccgggaa gctagagtaa     9780
   gtagttcgcc agttaatagt ttgcgcaacg ttgttgccat tgctacaggc atcgtggtgt     9840
   cacgctcgtc gtttggtatg gcttcattca gctccggttc ccaacgatca aggcgagtta     9900
   catgatcccc catgttgtgc aaaaaagcgg ttagctcctt cggtcctccg atcgttgtca     9960
   gaagtaagtt ggccgcagtg ttatcactca tggttatggc agcactgcat aattctctta    10020
   ctgtcatgcc atccgtaaga tgcttttctg tgactggtga gtactcaacc aagtcattct    10080
   gagaatagtg tatgcggcga ccgagttgct cttgcccggc gtcaatacgg gataataccg    10140
   cgccacatag cagaacttta aaagtgctca tcattggaaa acgttcttcg gggcgaaaac    10200
   tctcaaggat cttaccgctg ttgagatcca gttcgatgta acccactcgt gcacccaact    10260
   gatcttcagc atcttttact ttcaccagcg tttctgggtg agcaaaaaca ggaaggcaaa    10320
   atgccgcaaa aaagggaata agggcgacac ggaaatgttg aatactcata ctcttccttt    10380
   ttcaatatta ttgaagcatt tatcagggtt attgtctcat gagcggatac atatttgaat    10440
   gtatttagaa aaataaacaa ataggggttc cgcgcacatt tccccgaaaa gtgc          10494
<210> 64
   <211> 7433
   <212> DNA
   <213> Homo sapiens
<400> 64
   ctcgagagct catgcggtac cgctagcgtt taaacctagt cactcctcta cgctcctgta      60
   catacgaggt gcagctgttg gagtctgggg gaggcttggt acagcctggg gggtccctga     120
   gactctcctg tgcagcctct ggattcacct ttagcggcta ctatatgcac tgggtccgcc     180
   aggctccagg gaaggggctg gagtgggtct catggatcaa ccctaacagt ggtggcacaa     240
   actatgcaca gaagtttcag ggccggttca ccatctccag agacaattcc aagaacacgc     300
   tgtatctgca aatgaacagc ctgagagccg aggacacggc cgtatattac tgtgcgaaag     360
   gttggggcta tacggtgact accctcaact ggttcgaccc ctggggccag ggcaccctgg     420
   tcaccgtctc ctcagtgcct ggtgactctt ccgcaatcag tcctgggaca gaaacctcag     480
   ggacagcctt tatgagtttt gatcacatct aacagagagg atgggccagc gtgtctctac     540
   tcgcgtgtct ctctatgcgg aggtgcagct gttggagtct gggggaggct tggtacagcc     600
   tggggggtcc ctgagactct cctgtgcagc ctctggattc acctttagca gttatgatat      660
   caactgggtc cgccaggctc cagggaaggg gctggagtgg gtctcatgga tgaaccctaa     720
   cagtggtaac acaggctatg cacagaagtt ccagggccgg ttcaccatct ccagagacaa     780
   ttccaagaac acgctgtatc tgcaaatgaa cagcctgaga gccgaggaca cggccgtata 840
   ttactgtgcg aaatccagca gctggcacta ctactacggt atggacgtct ggggccaggg 900
   caccctggtc accgtctcct cagtgcctgg tgactcttcc gcaatcagtc ctgggacaga 960
   aacctcaggg acagccttta tgagttttga tcacatctaa cagagaggat gggccagata 1020
   cgacgtatca cgtactacgt ctagtacgag gtgcagctgt tggagtctgg gggaggcttg 1080
   gtacagcctg gggggtccct gagactctcc tgtgcagcct ctggattcac ctttagcgaa 1140
   ttatccatgc actgggtccg ccaggctcca gggaaggggc tggagtgggt ctcaggtttt 1200
   gatcctgaag atggtgaaac aatctacgca cagaagttcc agggccggtt caccatctcc 1260
   agagacaatt ccaagaacac gctgtatctg caaatgaaca gcctgagagc cgaggacacg 1320
   gccgtatatt actgtgcgaa aggaacctac tactactact acggtatgga cgtctggggc 1380
   cagggcaccc tggtcaccgt ctcctcagtg cctggtgact cttccgcaat cagtcctggg 1440
   acagaaacct cagggacagc ctttatgagt tttgatcaca tctaacagag aggatgggcc 1500
   agcgtagact agtacgagta tgtactctcg tggaggtgca gctgttggag tctgggggag 1560
   gcttggtaca gcctgggggg tccctgagac tctcctgtgc agcctctgga ttcaccttta 1620
   gcagctacta tatgcactgg gtccgccagg ctccagggaa ggggctggag tgggtctcaa 1680
   taatcaaccc tagtggtggt agcacaagct acgcacagaa gttccagggc cggttcacca 1740
   tctccagaga caattccaag aacacgctgt atctgcaaat gaacagcctg agagccgagg 1800
   acacggccgt atattactgt gcgaaaagtt tcagcagcag ctggtacaac tggttcgacc 1860
   cctggggcca gggcaccctg gtcaccgtct cctcagtgcc tggtgactct tccgcaatca 1920
   gtcctgggac agaaacctca gggacagcct ttatgagttt tgatcacatc taacagagag 1980
   gatgggccag acgcgagtat actactatgt actgtacagt gaggtgcagc tgttggagtc 2040
   tgggggaggc ttggtacagc ctggggggtc cctgagactc tcctgtgcag cctctggatt 2100
   cacctttagc agctatgcta tcagctgggt ccgccaggct ccagggaagg ggctggagtg 2160
   ggtctcaggg atcatcccta tctttggtac agcaaactac gcacagaagt tccagggccg 2220
   gttcaccatc tccagagaca attccaagaa cacgctgtat ctgcaaatga acagcctgag 2280
   agccgaggac acggccgtat attactgtgc gaaagggtcc gtcctcggtg atgcttttga 2340
   tatctggggc cagggcaccc tggtcaccgt ctcctcagtg cctggtgact cttccgcaat 2400
   cagtcctggg acagaaacct cagggacagc ctttatgagt tttgatcaca tctaacagag 2460
   aggatgggcc agatagagta ctcacgctac gtcagtagac gtgaggtgca gctgttggag 2520
   tctgggggag gcttggtaca gcctgggggg tccctgagac tctcctgtgc agcctctgga 2580
   ttcaccttta gcaatgctag aatgggtgtg agctgggtcc gccaggctcc agggaagggg 2640
   ctggagtggg tctcacacat tttttcgaat gacgaaaaat cctacagcac atctctgaag 2700
   agccggttca ccatctccag agacaattcc aagaacacgc tgtatctgca aatgaacagc 2760
   ctgagagccg aggacacggc cgtatattac tgtgcgaaaa tcccgtatga agcagtggct 2820
   ggctactttg actactgggg ccagggcacc ctggtcaccg tctcctcagt gcctggtgac 2880
   tcttccgcaa tcagtcctgg gacagaaacc tcagggacag cctttatgag ttttgatcac 2940
   atctaacaga gaggatgggc cagtacgctg tcttagtgta gattcgatca cgtgaggtgc 3000
   agctgttgga gtctggggga ggcttggtac agcctggggg gtccctgaga ctctcctgtg 3060
   cagcctctgg attcaccttt agcagctatt ggatgagctg ggtccgccag gctccaggga 3120
   aggggctgga gtgggtctca aacataaagc aagatggaag tgagaaatac tatgtggact 3180
   ctgtgaaggg ccggttcacc atctccagag acaattccaa gaacacgctg tatctgcaaa 3240
   tgaacagcct gagagccgag gacacggccg tatattactg tgcgaaagtt acggtgaccg 3300
   ggacctggta cttcgatctc tggggccagg gcaccctggt caccgtctcc tcagtgcctg 3360
   gtgactcttc cgcaatcagt cctgggacag aaacctcagg gacagccttt atgagttttg 3420
   atcacatcta acagagagga tgggccagtg acgtatgttg tgagtagtac agtatataga 3480
   ggtgcagctg ttggagtctg gggaggcttt ggtacagcct ggggggtccc tgagactctc 3540
   ctgtgcagcc tctggattca cctttagcag ctacgacatg cactgggtcc gccaggctcc 3600
   agggaagggg ctggagtggg tctcagctat tggtactgct ggtgacacat actatccagg 3660
   ctccgtgaag ggccggttca ccatctccag agacaattcc aagaacacgc tgtatctgca 3720
   aatgaacagc ctgagagccg aggacacggc cgtatattac tgtgcgaaag cggatacagc 3780
   tatggtcatt tactactact actacggtat ggacgtctgg ggccagggca ccctggtcac 3840
   cgtctcctca gtgcctggtg actcttccgc aatcagtcct gggacagaaa cctcagggac 3900
   agcctttatg agttttgatc acatctaaca gagaggatgg gccagagtat acataagtcg 3960
   agagaagtgc tacgagaggt gcagctgttg gagtctgggg gaggcttggt acagcctggg 4020
   gggtccctga gactctcctg tgcagcctct ggattcacct ttagcagcta tagcatgaac 4080
   tgggtccgcc aggctccagg gaaggggctg gagtgggtct catccattag tagtagtagt 4140
   agttacatat actacgcaga ctcagtgaag ggccggttca ccatctccag agacaattcc 4200
   aagaacacgc tgtatctgca aatgaacagc ctgagagccg aggacacggc cgtatattac 4260
   tgtgcgaaag agaatagtgg ctatgattac tttgactact ggggccaggg caccctggtc 4320
   accgtctcct cagtgcctgg tgactcttcc gcaatcagtc ctgggacaga aacctcaggg 4380
   acagccttta tgagttttga tcacatctaa cagagaggat gggccagcgt acagtcacgt 4440
   actcagacta cgctctagag gtgcagctgt tggagtctgg gggaggcttg gtacagcctg 4500
   gggggtccct gagactctcc tgtgcagcct ctggattcac ctttagcgat tatgctatga 4560
   gctgggtccg ccaggctcca gggaaggggc tggagtgggt ctcattcatt agaagcaaag 4620
   cttatggtgg gacaacagaa tacaccgcgt ctgtgaaagg ccggttcacc atctccagag 4680
   acaattccaa gaacacgctg tatctgcaaa tgaacagcct gagagccgag gacacggccg 4740
   tatattactg tgcgaaagat ctaggtagtg gctttgacta ctggggccag ggcaccctgg 4800
   tcaccgtctc ctcagtgcct ggtgactctt ccgcaatcag tcctgggaca gaaacctcag 4860
   ggacagcctt tatgagtttt gatcacatct aacagagagg atgggccagt atatatacat 4920
   atgctagtat cacgcgagag aggtgcagct gttggagtct gggggaggct tggtacagcc 4980
   tggggggtcc ctgagactct cctgtgcagc ctctggattc acctttagcg accactacat 5040
   ggactgggtc cgccaggctc cagggaaggg gctggagtgg gtctcacgta ctagaaacaa 5100
   agctaacagt tacaccacag aatacgccgc gtctgtgaaa ggccggttca ccatctccag 5160
   agacaattcc aagaacacgc tgtatctgca aatgaacagc ctgagagccg aggacacggc 5220
   cgtatattac tgtgcgaaag gaggccaggg gagccgatac tactactact acggtatgga 5280
   cgtctggggc cagggcaccc tggtcaccgt ctcctcagtg cctggtgact cttccgcaat 5340
   cagtcctggg acagaaacct cagggacagc ctttatgagt tttgatcaca tctaacagag 5400
   aggatgggcc agtgagtcag tatgtagtgt gatgtcacac gagaggtgca gctgttggag 5460
   tctgggggag gcttggtaca gcctgggggg tccctgagac tctcctgtgc agcctctgga 5520
   ttcaccttta gcagcaatga gatgagctgg gtccgccagg ctccagggaa ggggctggag 5580
   tgggtctcat ccattagtgg tggtagcaca tactacgcag actccaggaa gggccggttc 5640
   accatctcca gagacaattc caagaacacg ctgtatctgc aaatgaacag cctgagagcc 5700
   gaggacacgg ccgtatatta ctgtgcgaaa gactcatatg atgcttttga tatctggggc 5760
   cagggcaccc tggtcaccgt ctcctcagtg cctggtgact cttccgcaat cagtcctggg 5820
   acagaaacct cagggacagc ctttatgagt tttgatcaca tctaacagag aggatgggcc 5880
   agacatgacg acacgacagc tcacgtctca tcgaggtgca gctgttggag tctgggggag 5940
   gcttggtaca gcctgggggg tccctgagac tctcctgtgc agcctctgga ttcaccttta 6000
   gcagtggtgg ttactcctgg agctgggtcc gccaggctcc agggaagggg ctggagtggg 6060
   tctcatacat ctatcatagt gggagcacct actacaaccc gtccctcaag agtcggttca 6120
   ccatctccag agacaattcc aagaacacgc tgtatctgca aatgaacagc ctgagagccg 6180
   aggacacggc cgtatattac tgtgcgaaag atgcgagtaa ccatttcttt gactactggg 6240
   gccagggcac cctggtcacc gtctcctcag tgcctggtga ctcttccgca atcagtcctg 6300
   ggacagaaac ctcagggaca gcctttatga gttttgatca catctaacag agaggatggg 6360
   ccagagcgac tagcagtagt gatcagtgac acacgaggtg cagctgttgg agtctggggg 6420
   aggcttggta cagcctgggg ggtccctgag actctcctgt gcagcctctg gattcacctt      6480
   tagcagtagt agttactact ggggctgggt ccgccaggct ccagggaagg ggctggagtg      6540
   ggtctcaagt atctattata gtgggagcac ctactacaac ccgtccctca agagtcggtt      6600
   caccatctcc agagacaatt ccaagaacac gctgtatctg caaatgaaca gcctgagagc      6660
   cgaggacacg gccgtatatt actgtgcgaa aaaggactgg aacgacaact ggttcgaccc      6720
   ctggggccag ggcaccctgg tcaccgtctc ctcagtgcct ggtgactctt ccgcaatcag      6780
   tcctgggaca gaaacctcag ggacagcctt tatgagtttt gatcacatct aacagagagg      6840
   atgggccaga tctactgacc acgtagatcc acgtgtcgcg aggtgcagct gttggagtct      6900
   gggggaggct tggtacagcc tggggggtcc ctgagactct cctgtgcagc ctctggattc      6960
   acctttagca gcaacagtgc tgcttggaac tgggtccgcc aggctccagg gaaggggctg      7020
   gagtgggtct caaggacata ctacaggtcc aagtggtata atgattatgc agtatctgtg      7080
   aaaagtcggt tcaccatctc cagagacaat tccaagaaca cgctgtatct gcaaatgaac      7140
   agcctgagag ccgaggacac ggccgtatat tactgtgcga aagatattgt agtagtacca      7200
   gctgctatat actggtactt cgatctctgg ggccagggca ccctggtcac cgtctcctca      7260
   gtgcctggtg actcttccgc aatcagtcct gggacagaaa cctcagggac agcctttatg      7320
   agttttgatc acatctaaca gagaggatgg gccaggttta aacggtacct ggccaggatc      7380
   catgcccatg gccgcggatg cggcgcgcca tcgatatgcg tcgacattta aat             7433
<210> 65
   <211> 29
   <212> DNA
   <213> Artificial Sequence
<220>
   <223> Synthetic DNA
<400> 65
   gttaactcga gatgcgtcga catttaaat 29
<210> 66
   <211> 16247
   <212> DNA
   <213> Artificial Sequence
<220>
   <223> Targeting vector
<400> 66
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtttccc gaagcgaaag ccgcgtgtta 420
   tttttgggtg ggaaagcccc gattcgctcc ttttttttta tttattattt ttgttgtttt 480
   tcgtgtcttt ttcttctccg ttttgcagct gcggcctcca ggtgtccccg gccgtggtat 540
   ttttgggtcg aaaaagccgc tttttggctc acttggaaac ctttcgggcg tctccaagcg 600
   gagcgttggc gggaaatggt gttttttttg ggttgttttc ggtccctttt ttcaccattt 660
   ttgtggtttt tcggcccatt ttcggcctcc cccccccccc cccccttccc gtggggctgc 720
   gctccgtgga gcggaatggg gtcgttaatt aaggttaatt ggtttaatta atgggccgtg 780
   cagggagaag agggccgtgc ggtggttgtt agccctgatt aatggcttgg ttgaggggag 840
   agtgtaatta agtgcttcgt tagcactaat taatggcttg attgagagga gagtgtaatt 900
   aaatgcttca taatccctaa ttaatggctt ggttgagaca agagtgtaat taagtgcttc 960
   attagccctg attaatggct tggttgaggg gagagtgtaa ttaaatgctt cgttagcact 1020
   aattaatggc ttgattgaga ggagagtgta attaaatgct tcataatccc taattaatgg 1080
   cttggttgag acaagagtgt aattaagtgc ttcattagcc ctgattaatg gcttggttga 1140
   gaggagagtg taattaaatg cttcattatc cctaattagg gactgaattc caaggagagt 1200
   gtaattaagt gcttcattat ccctaattaa tggcttggtt gagaggagag tgtaattaaa 1260
   tgctttgtta gccctaatta atggtttgat ggagaggaga gtgtaattaa gtgcttcatt 1320
   atccctaatt aatggcttgg ttgaggggag agtgtaatta aatgcttagt tagccctaat 1380
   taatggcttg gttgagagga gagtgtaatt gaatgcttca ttatccctaa ttagggattg 1440
   aattgagagg agagtgtaat tgaatgcttt gttagcccta attagggatt gaattgagag 1500
   gagagtgtaa ttaaatgcct cattatccct aattagggat tgaattgaga ggagagttta 1560
   attaaatgcc tcattatccc taattaggga ctgaattccg acgagagtgt aattaagtgc 1620
   ttcatttagc cctaattaat ggcttgattg agaggagagt gtaattaaat gcttcattat 1680
   ccctaattag ggattgaatt gagaggagag tgtaattaaa tgcttcatta tccctaatta 1740
   gggattgaat tgagaggaga gtgtaattaa atgcttcatt atccctaatt aatggcttgg 1800
   ttgagagggg agtgtaatta agtgcttcgt tatccctaat tagggattga attgtgagga 1860
   gatggcaatt aatacttcat tatccctaat taatttaatt agtccctatt atccctaatt 1920
   taattcatgg tttggttgat ggaagaatgt aattaaatgc tttgttatcc ctaattaatg 1980
   gtttgattga gcggagagtg taattaaatg cttcattatc ccttattaat ggttgtgtgg 2040
   aatgtagtgt aattagaagt ccctaattaa tggtttaatt gagagaagac tgcaattaga 2100
   tgactcatag cccctaataa gtggtttaat tatgagataa tcacaattac atgtttcatt 2160
   gtccctaatt aatcgtttaa ttgcgagaag atcacaatta attaaatgat ccctaattgc 2220
   taccgttcgt ataatgtata ctatacgaag ttattttatt tgtgaaattt gtgatgctat 2280
   tgctttattt gtaaccatta taagctgcaa taaacaagtt ggggtgggcg aagaactcca 2340
   gcatgagatc cccgcgctgg aggatcatcc agccggcgtc ccggaaaacg attccgaagc 2400
   ccaacctttc atagaaggcg gcggtggaat cgaaatctcg tgatggcagg ttgggcgtcg 2460
   cttggtcggt catttcgaac cccagagtcc cgctcagaag aactcgtcaa gaaggcgata 2520
   gaaggcgatg cgctgcgaat cgggagcggc gataccgtaa agcacgagga agcggtcagc 2580
   ccattcgccg ccaagctctt cagcaatatc acgggtagcc aacgctatgt cctgatagcg 2640
   gtccgccaca cccagccggc cacagtcgat gaatccagaa aagcggccat tttccaccat 2700
   gatattcggc aagcaggcat cgccatgggt cacgacgaga tcctcgccgt cgggcatgcg 2760
   cgccttgagc ctggcgaaca gttcggctgg cgcgagcccc tgatgctctt cgtccagatc 2820
   atcctgatcg acaagaccgg cttccatccg agtacgtgct cgctcgatgc gatgtttcgc 2880
   ttggtggtcg aatgggcagg tagccggatc aagcgtatgc agccgccgca ttgcatcagc 2940
   catgatggat actttctcgg caggagcaag gtgagatgac aggagatcct gccccggcac 3000
   ttcgcccaat agcagccagt cccttcccgc ttcagtgaca acgtcgagca cagctgcgca 3060
   aggaacgccc gtcgtggcca gccacgatag ccgcgctgcc tcgtcctgca gttcattcag 3120
   ggcaccggac aggtcggtct tgacaaaaag aaccgggcgc ccctgcgctg acagccggaa 3180
   cacggcggca tcagagcagc cgattgtctg ttgtgcccag tcatagccga atagcctctc 3240
   cacccaagcg gccggagaac ctgcgtgcaa tccatcttgt tcaatcatgc gaaacgatcc 3300
   tcatcctgtc tcttgatcag atccgaaaat ggatatacaa gctcccggga gctttttgca 3360
   aaagcctagg cctccaaaaa agcctcctca ctacttctgg aatagctcag aggcagaggc 3420
   ggcctcggcc tctgcataaa taaaaaaaat tagtcagcca tggggcggag aatgggcgga 3480
   actgggcgga gttaggggcg ggatgggcgg agttaggggc gggactatgg ttgctgacta 3540
   attgagatgc atgctttgca tacttctgcc tgctggggag cctggggact ttccacacct 3600
   ggttgctgac taattgagat gcatgctttg catacttctg cctgctgggg agcctgggga 3660
   ctttccacac cctaaataac ttcgtataat gtatactata cgaacggtag cgttaacaag 3720
   cttctgtgga atgtgtgtca gttagggtgt ggaaagtccc caggctcccc aggcaggcag 3780
   aagtatgcaa agcatgcatc tcaattagtc agcaaccagg tgtggaaagt ccccaggctc 3840
   cccagcaggc agaagtatgc aaagcatgca tctcaattag tcagcaacca tagtcccgcc 3900
   cctaactccg cccatcccgc ccctaactcc gcccagttcc gcccattctc cgccccatgg 3960
   ctgactaatt ttttttattt atgcagaggc cgaggccgcc tctgcctctg agctattcca 4020
   gaagtagtga ggaggctttt ttggaggcct aggcttttgc aaaaagctat aacttcgtat 4080
   atggtattat atacgaagtt atcccacccc aacttgttta ttgcagctta taatggttac 4140
   aaataaagca atagcatcac aaatttcaca aataaagcat ttttttcact gcattctagt 4200
   tgtggtttgt ccaaactcat caatgtatct tatcatgtct gtataccgtc gacctctagc 4260
   tagagcttgg cgtaatcatg gtcatagctg tttcctgtgt gcccacccca acttgtttat 4320
   tgcagcttat aatggttaca aataaagcaa tagcatcaca aatttcacaa ataaagcatt 4380
   tttttcactg cattctagtt gtggtttgtc caaactcatc aatgtatctt atcatgtctg 4440
   gagctcgcta gcgtttaaac ggtaccataa cttcgtatat tctatcttat acgaacggta 4500
   ctcgagagct catgcggtac cgctagcgtt taaacctagt cactcctcta cgctcctgta 4560
   catacgaggt gcagctgttg gagtctgggg gaggcttggt acagcctggg gggtccctga 4620
   gactctcctg tgcagcctct ggattcacct ttagcggcta ctatatgcac tgggtccgcc 4680
   aggctccagg gaaggggctg gagtgggtct catggatcaa ccctaacagt ggtggcacaa 4740
   actatgcaca gaagtttcag ggccggttca ccatctccag agacaattcc aagaacacgc 4800
   tgtatctgca aatgaacagc ctgagagccg aggacacggc cgtatattac tgtgcgaaag 4860
   gttggggcta tacggtgact accctcaact ggttcgaccc ctggggccag ggcaccctgg 4920
   tcaccgtctc ctcagtgcct ggtgactctt ccgcaatcag tcctgggaca gaaacctcag 4980
   ggacagcctt tatgagtttt gatcacatct aacagagagg atgggccagc gtgtctctac 5040
   tcgcgtgtct ctctatgcgg aggtgcagct gttggagtct gggggaggct tggtacagcc 5100
   tggggggtcc ctgagactct cctgtgcagc ctctggattc acctttagca gttatgatat 5160
   caactgggtc cgccaggctc cagggaaggg gctggagtgg gtctcatgga tgaaccctaa 5220
   cagtggtaac acaggctatg cacagaagtt ccagggccgg ttcaccatct ccagagacaa 5280
   ttccaagaac acgctgtatc tgcaaatgaa cagcctgaga gccgaggaca cggccgtata 5340
   ttactgtgcg aaatccagca gctggcacta ctactacggt atggacgtct ggggccaggg 5400
   caccctggtc accgtctcct cagtgcctgg tgactcttcc gcaatcagtc ctgggacaga 5460
   aacctcaggg acagccttta tgagttttga tcacatctaa cagagaggat gggccagata 5520
   cgacgtatca cgtactacgt ctagtacgag gtgcagctgt tggagtctgg gggaggcttg 5580
   gtacagcctg gggggtccct gagactctcc tgtgcagcct ctggattcac ctttagcgaa 5640
   ttatccatgc actgggtccg ccaggctcca gggaaggggc tggagtgggt ctcaggtttt 5700
   gatcctgaag atggtgaaac aatctacgca cagaagttcc agggccggtt caccatctcc 5760
   agagacaatt ccaagaacac gctgtatctg caaatgaaca gcctgagagc cgaggacacg 5820
   gccgtatatt actgtgcgaa aggaacctac tactactact acggtatgga cgtctggggc 5880
   cagggcaccc tggtcaccgt ctcctcagtg cctggtgact cttccgcaat cagtcctggg 5940
   acagaaacct cagggacagc ctttatgagt tttgatcaca tctaacagag aggatgggcc 6000
   agcgtagact agtacgagta tgtactctcg tggaggtgca gctgttggag tctgggggag 6060
   gcttggtaca gcctgggggg tccctgagac tctcctgtgc agcctctgga ttcaccttta 6120
   gcagctacta tatgcactgg gtccgccagg ctccagggaa ggggctggag tgggtctcaa 6180
   taatcaaccc tagtggtggt agcacaagct acgcacagaa gttccagggc cggttcacca 6240
   tctccagaga caattccaag aacacgctgt atctgcaaat gaacagcctg agagccgagg 6300
   acacggccgt atattactgt gcgaaaagtt tcagcagcag ctggtacaac tggttcgacc 6360
   cctggggcca gggcaccctg gtcaccgtct cctcagtgcc tggtgactct tccgcaatca 6420
   gtcctgggac agaaacctca gggacagcct ttatgagttt tgatcacatc taacagagag 6480
   gatgggccag acgcgagtat actactatgt actgtacagt gaggtgcagc tgttggagtc 6540
   tgggggaggc ttggtacagc ctggggggtc cctgagactc tcctgtgcag cctctggatt 6600
   cacctttagc agctatgcta tcagctgggt ccgccaggct ccagggaagg ggctggagtg 6660
   ggtctcaggg atcatcccta tctttggtac agcaaactac gcacagaagt tccagggccg 6720
   gttcaccatc tccagagaca attccaagaa cacgctgtat ctgcaaatga acagcctgag 6780
   agccgaggac acggccgtat attactgtgc gaaagggtcc gtcctcggtg atgcttttga 6840
   tatctggggc cagggcaccc tggtcaccgt ctcctcagtg cctggtgact cttccgcaat 6900
   cagtcctggg acagaaacct cagggacagc ctttatgagt tttgatcaca tctaacagag 6960
   aggatgggcc agatagagta ctcacgctac gtcagtagac gtgaggtgca gctgttggag 7020
   tctgggggag gcttggtaca gcctgggggg tccctgagac tctcctgtgc agcctctgga 7080
   ttcaccttta gcaatgctag aatgggtgtg agctgggtcc gccaggctcc agggaagggg 7140
   ctggagtggg tctcacacat tttttcgaat gacgaaaaat cctacagcac atctctgaag 7200
   agccggttca ccatctccag agacaattcc aagaacacgc tgtatctgca aatgaacagc 7260
   ctgagagccg aggacacggc cgtatattac tgtgcgaaaa tcccgtatga agcagtggct 7320
   ggctactttg actactgggg ccagggcacc ctggtcaccg tctcctcagt gcctggtgac 7380
   tcttccgcaa tcagtcctgg gacagaaacc tcagggacag cctttatgag ttttgatcac 7440
   atctaacaga gaggatgggc cagtacgctg tcttagtgta gattcgatca cgtgaggtgc 7500
   agctgttgga gtctggggga ggcttggtac agcctggggg gtccctgaga ctctcctgtg 7560
   cagcctctgg attcaccttt agcagctatt ggatgagctg ggtccgccag gctccaggga 7620
   aggggctgga gtgggtctca aacataaagc aagatggaag tgagaaatac tatgtggact 7680
   ctgtgaaggg ccggttcacc atctccagag acaattccaa gaacacgctg tatctgcaaa 7740
   tgaacagcct gagagccgag gacacggccg tatattactg tgcgaaagtt acggtgaccg 7800
   ggacctggta cttcgatctc tggggccagg gcaccctggt caccgtctcc tcagtgcctg 7860
   gtgactcttc cgcaatcagt cctgggacag aaacctcagg gacagccttt atgagttttg 7920
   atcacatcta acagagagga tgggccagtg acgtatgttg tgagtagtac agtatataga 7980
   ggtgcagctg ttggagtctg gggaggcttg gtacagcctg gggggtcccc tgagactctc 8040
   ctgtgcagcc tctggattca cctttagcag ctacgacatg cactgggtcc gccaggctcc 8100
   agggaagggg ctggagtggg tctcagctat tggtactgct ggtgacacat actatccagg 8160
   ctccgtgaag ggccggttca ccatctccag agacaattcc aagaacacgc tgtatctgca 8220
   aatgaacagc ctgagagccg aggacacggc cgtatattac tgtgcgaaag cggatacagc 8280
   tatggtcatt tactactact actacggtat ggacgtctgg ggccagggca ccctggtcac 8340
   cgtctcctca gtgcctggtg actcttccgc aatcagtcct gggacagaaa cctcagggac 8400
   agcctttatg agttttgatc acatctaaca gagaggatgg gccagagtat acataagtcg 8460
   agagaagtgc tacgagaggt gcagctgttg gagtctgggg gaggcttggt acagcctggg 8520
   gggtccctga gactctcctg tgcagcctct ggattcacct ttagcagcta tagcatgaac 8580
   tgggtccgcc aggctccagg gaaggggctg gagtgggtct catccattag tagtagtagt 8640
   agttacatat actacgcaga ctcagtgaag ggccggttca ccatctccag agacaattcc 8700
   aagaacacgc tgtatctgca aatgaacagc ctgagagccg aggacacggc cgtatattac 8760
   tgtgcgaaag agaatagtgg ctatgattac tttgactact ggggccaggg caccctggtc 8820
   accgtctcct cagtgcctgg tgactcttcc gcaatcagtc ctgggacaga aacctcaggg 8880
   acagccttta tgagttttga tcacatctaa cagagaggat gggccagcgt acagtcacgt 8940
   actcagacta cgctctagag gtgcagctgt tggagtctgg gggaggcttg gtacagcctg 9000
   gggggtccct gagactctcc tgtgcagcct ctggattcac ctttagcgat tatgctatga 9060
   gctgggtccg ccaggctcca gggaaggggc tggagtgggt ctcattcatt agaagcaaag 9120
   cttatggtgg gacaacagaa tacaccgcgt ctgtgaaagg ccggttcacc atctccagag 9180
   acaattccaa gaacacgctg tatctgcaaa tgaacagcct gagagccgag gacacggccg 9240
   tatattactg tgcgaaagat ctaggtagtg gctttgacta ctggggccag ggcaccctgg 9300
   tcaccgtctc ctcagtgcct ggtgactctt ccgcaatcag tcctgggaca gaaacctcag 9360
   ggacagcctt tatgagtttt gatcacatct aacagagagg atgggccagt atatatacat 9420
   atgctagtat cacgcgagag aggtgcagct gttggagtct gggggaggct tggtacagcc 9480
   tggggggtcc ctgagactct cctgtgcagc ctctggattc acctttagcg accactacat 9540
   ggactgggtc cgccaggctc cagggaaggg gctggagtgg gtctcacgta ctagaaacaa 9600
   agctaacagt tacaccacag aatacgccgc gtctgtgaaa ggccggttca ccatctccag 9660
   agacaattcc aagaacacgc tgtatctgca aatgaacagc ctgagagccg aggacacggc 9720
   cgtatattac tgtgcgaaag gaggccaggg gagccgatac tactactact acggtatgga 9780
   cgtctggggc cagggcaccc tggtcaccgt ctcctcagtg cctggtgact cttccgcaat 9840
   cagtcctggg acagaaacct cagggacagc ctttatgagt tttgatcaca tctaacagag 9900
   aggatgggcc agtgagtcag tatgtagtgt gatgtcacac gagaggtgca gctgttggag 9960
   tctgggggag gcttggtaca gcctgggggg tccctgagac tctcctgtgc agcctctgga 10020
   ttcaccttta gcagcaatga gatgagctgg gtccgccagg ctccagggaa ggggctggag 10080
   tgggtctcat ccattagtgg tggtagcaca tactacgcag actccaggaa gggccggttc 10140
   accatctcca gagacaattc caagaacacg ctgtatctgc aaatgaacag cctgagagcc 10200
   gaggacacgg ccgtatatta ctgtgcgaaa gactcatatg atgcttttga tatctggggc 10260
   cagggcaccc tggtcaccgt ctcctcagtg cctggtgact cttccgcaat cagtcctggg 10320
   acagaaacct cagggacagc ctttatgagt tttgatcaca tctaacagag aggatgggcc 10380
   agacatgacg acacgacagc tcacgtctca tcgaggtgca gctgttggag tctgggggag 10440
   gcttggtaca gcctgggggg tccctgagac tctcctgtgc agcctctgga ttcaccttta 10500
   gcagtggtgg ttactcctgg agctgggtcc gccaggctcc agggaagggg ctggagtggg 10560
   tctcatacat ctatcatagt gggagcacct actacaaccc gtccctcaag agtcggttca 10620
   ccatctccag agacaattcc aagaacacgc tgtatctgca aatgaacagc ctgagagccg 10680
   aggacacggc cgtatattac tgtgcgaaag atgcgagtaa ccatttcttt gactactggg 10740
   gccagggcac cctggtcacc gtctcctcag tgcctggtga ctcttccgca atcagtcctg 10800
   ggacagaaac ctcagggaca gcctttatga gttttgatca catctaacag agaggatggg 10860
   ccagagcgac tagcagtagt gatcagtgac acacgaggtg cagctgttgg agtctggggg 10920
   aggcttggta cagcctgggg ggtccctgag actctcctgt gcagcctctg gattcacctt 10980
   tagcagtagt agttactact ggggctgggt ccgccaggct ccagggaagg ggctggagtg 11040
   ggtctcaagt atctattata gtgggagcac ctactacaac ccgtccctca agagtcggtt 11100
   caccatctcc agagacaatt ccaagaacac gctgtatctg caaatgaaca gcctgagagc 11160
   cgaggacacg gccgtatatt actgtgcgaa aaaggactgg aacgacaact ggttcgaccc 11220
   ctggggccag ggcaccctgg tcaccgtctc ctcagtgcct ggtgactctt ccgcaatcag 11280
   tcctgggaca gaaacctcag ggacagcctt tatgagtttt gatcacatct aacagagagg 11340
   atgggccaga tctactgacc acgtagatcc acgtgtcgcg aggtgcagct gttggagtct 11400
   gggggaggct tggtacagcc tggggggtcc ctgagactct cctgtgcagc ctctggattc 11460
   acctttagca gcaacagtgc tgcttggaac tgggtccgcc aggctccagg gaaggggctg 11520
   gagtgggtct caaggacata ctacaggtcc aagtggtata atgattatgc agtatctgtg 11580
   aaaagtcggt tcaccatctc cagagacaat tccaagaaca cgctgtatct gcaaatgaac 11640
   agcctgagag ccgaggacac ggccgtatat tactgtgcga aagatattgt agtagtacca 11700
   gctgctatat actggtactt cgatctctgg ggccagggca ccctggtcac cgtctcctca 11760
   gtgcctggtg actcttccgc aatcagtcct gggacagaaa cctcagggac agcctttatg 11820
   agttttgatc acatctaaca gagaggatgg gccaggttta aacggtacct ggccaggatc 11880
   catgcccatg gccgcggatg cggcgcgcca tcgatatgcg tcgacattta aatgttggcg 11940
   cagaagggat tcatatcaat taattccctg tcctcaatta atgcataatt gggagtgcga 12000
   tggaatcatt caataagtaa tgaggacttg gtgtcaaaat gccaattaag taattaatga 12060
   cacccaatta atacacaatg gggattacag tggaataact cagtgaagtt catatctaaa 12120
   ttgcaattga ataattaatt acagctaatt aatggatggg agactggcag tggtataact 12180
   taatgaagga acacagtgaa ttcctgtctg aattgaaatt aattgattta ttagggctaa 12240
   ttaatgaatg gtggggttaa aaatagcatt actcagcgaa ggaacatgag ttcatggcta 12300
   aattgagatt gaattattaa ttacaactaa ttgattaatg actgatggga gtaagaatgg 12360
   aataactcag tgaaggaaca caatggattc atggcctaat tgaaattaaa tggttaatta 12420
   gagctaatta atgaatgatg gggggtggca atggaatgat acaagaaaga aacacaatgg 12480
   gttcgtgtct acattgaaat taaataatta attacagcta attaataaac ggcgaggttg 12540
   acaatggtat aactgtgaag agacagaatg agttcgtgac taaattggta ttaaatgatt 12600
   aattagagct aattaacagg tggtggggct gagaatggca taactcaatg ggggatgatg 12660
   agctgatggc ttaattggaa ttaataatta atcacaacca attaacaggt gctgttaatg 12720
   gggttacagg ggcatgactt caggaagaag cacaaagtgg ccattaaata attaattgca 12780
   cccaattaat tcctgatggg gattactgca gaggaactca gcgaaggatt caagcagaaa 12840
   tgacacagtt aggttgctca attgcgttaa ttaattaaaa tcgctaatta ggtgagcttc 12900
   attaaggcct gatggggttc atcccaacgc tgggatcaaa gaactcagcg cagagtggat 12960
   aacgataatt agaaggggac aaattaatga ggaaacacga ctttgggcct agacggccat 13020
   taaatcatta atcaacccga cgggcgccca attaatcata aagttattaa gcaattaagt 13080
   cgaggctgac gagaaaccgc aacgagagcc ctccacgctc ctttgagttg gcgcaaacaa 13140
   caaacaacgc gggctcgtta agcagcgacg acgccgagcg taacgaagca atccccgcgc 13200
   ccgacaatta acgcgctaac gaagccggag ccctccttat gcaaattagc ccctccagag 13260
   ggccataaaa gcgccggctc tccgacggcg cgggaggagc accagtcggc tccgcaacca 13320
   tgagcccact cgtctcctcc ctcctgctcc tggccgccct gccaggtgag ggcgctgtgg 13380
   ggctctatgg ggctctatgg ggtctcagcg gggctctgcg ggctcaatgg gggccaaagg 13440
   gggggtctgc gggctctatg ggggggtcaa cggggggtct cacggggggc cggctccgcg 13500
   aggccgtgtg gcggcggctc cgtcagcgct ctctgggcgc gcctccttcc ccacagggct 13560
   gatggcggag gtgcagctgt tggagtctgg gggaggcttg gtacagcctg gggggtccct 13620
   gagactctcc tgtgcagcct ctggattcac ctttagcagc tatgccatga gctgggtccg 13680
   ccaggctcca gggaaggggc tggagtgggt ctcagctatt agtggtagtg gtggtagcac 13740
   atactacgca gactccgtga agggccggtt caccatctcc agagacaatt ccaagaacac 13800
   gctgtatctg caaatgaaca gcctgagagc cgaggacacg gccgtatatt actgtgcgaa 13860
   aggatatagt ggctacgatt actactttga ctactggggc cagggcaccc tggtcaccgt 13920
   ctcctcaggt gagtcttcat atgaaccccc cccaaaattg ccgcggcgat tttggggcaa 13980
   aatcgggcga ttttatggat ccccgcggcc gcaggcgtaa tcatggtcat agctgtttcc 14040
   tgtgtgaaat tgttatccgc tcacaattcc acacaacata cgagccggaa gcataaagtg 14100
   taaagcctgg ggtgcctaat gagtgagcta actcacatta attgcgttgc gctcactgcc 14160
   cgctttccag tcgggaaacc tgtcgtgcca gctgcattaa tgaatcggcc aacgcgcggg 14220
   gagaggcggt ttgcgtattg ggcgctcttc cgcttcctcg ctcactgact cgctgcgctc 14280
   ggtcgttcgg ctgcggcgag cggtatcagc tcactcaaag gcggtaatac ggttatccac 14340
   agaatcaggg gataacgcag gaaagaacat gtgagcaaaa ggccagcaaa aggccaggaa 14400
   ccgtaaaaag gccgcgttgc tggcgttttt ccataggctc cgcccccctg acgagcatca 14460
   caaaaatcga cgctcaagtc agaggtggcg aaacccgaca ggactataaa gataccaggc 14520
   gtttccccct ggaagctccc tcgtgcgctc tcctgttccg accctgccgc ttaccggata 14580
   cctgtccgcc tttctccctt cgggaagcgt ggcgctttct catagctcac gctgtaggta 14640
   tctcagttcg gtgtaggtcg ttcgctccaa gctgggctgt gtgcacgaac cccccgttca 14700
   gcccgaccgc tgcgccttat ccggtaacta tcgtcttgag tccaacccgg taagacacga 14760
   cttatcgcca ctggcagcag ccactggtaa caggattagc agagcgaggt atgtaggcgg 14820
   tgctacagag ttcttgaagt ggtggcctaa ctacggctac actagaagaa cagtatttgg 14880
   tatctgcgct ctgctgaagc cagttacctt cggaaaaaga gttggtagct cttgatccgg 14940
   caaacaaacc accgctggta gcggtggttt ttttgtttgc aagcagcaga ttacgcgcag 15000
   aaaaaaagga tctcaagaag atcctttgat cttttctacg gggtctgacg ctcagtggaa 15060
   cgaaaactca cgttaaggga ttttggtcat gagattatca aaaaggatct tcacctagat 15120
   ccttttaaat taaaaatgaa gttttaaatc aatctaaagt atatatgagt aaacttggtc 15180
   tgacagttac caatgcttaa tcagtgaggc acctatctca gcgatctgtc tatttcgttc 15240
   atccatagtt gcctgactcc ccgtcgtgta gataactacg atacgggagg gcttaccatc 15300
   tggccccagt gctgcaatga taccgcgaga cccacgctca ccggctccag atttatcagc 15360
   aataaaccag ccagccggaa gggccgagcg cagaagtggt cctgcaactt tatccgcctc    15420
   catccagtct attaattgtt gccgggaagc tagagtaagt agttcgccag ttaatagttt    15480
   gcgcaacgtt gttgccattg ctacaggcat cgtggtgtca cgctcgtcgt ttggtatggc    15540
   ttcattcagc tccggttccc aacgatcaag gcgagttaca tgatccccca tgttgtgcaa    15600
   aaaagcggtt agctccttcg gtcctccgat cgttgtcaga agtaagttgg ccgcagtgtt    15660
   atcactcatg gttatggcag cactgcataa ttctcttact gtcatgccat ccgtaagatg    15720
   cttttctgtg actggtgagt actcaaccaa gtcattctga gaatagtgta tgcggcgacc    15780
   gagttgctct tgcccggcgt caatacggga taataccgcg ccacatagca gaactttaaa    15840
   agtgctcatc attggaaaac gttcttcggg gcgaaaactc tcaaggatct taccgctgtt    15900
   gagatccagt tcgatgtaac ccactcgtgc acccaactga tcttcagcat cttttacttt    15960
   caccagcgtt tctgggtgag caaaaacagg aaggcaaaat gccgcaaaaa agggaataag    16020
   ggcgacacgg aaatgttgaa tactcatact cttccttttt caatattatt gaagcattta    16080
   tcagggttat tgtctcatga gcggatacat atttgaatgt atttagaaaa ataaacaaat    16140
   aggggttccg cgcacatttc cccgaaaagt gccacctgac gtctaagaaa ccattattat    16200
   catgacatta acctataaaa ataggcgtat cacgaggccc tttcgtc                  16247
<210> 67
   <211> 1674
   <212> DNA
   <213> Homo sapiens
<400> 67
   atgaaagcca tgccctggaa ctggacctgc cttctctccc acctcctcat ggtgggcatg        60
   ggctcctcca ctttgctcac ccggcagcca gccccgctgt cccagaagca gcggtcattt       120
   gtcacattcc gaggagagcc cgccgagggt ttcaatcacc tggtggtgga tgagaggaca       180
   ggacacattt acttgggggc cgtcaatcgg atttacaagc tctccagcga cctgaaggtc       240
   ttggtgacgc atgagacagg gccggacgag gacaacccca agtgttaccc accccgcatc       300
   gtccagacct gcaatgagcc cctgaccacc accaacaatg tcaacaagat gctcctcata       360
   gactacaagg agaacaggct gattgcctgt gggagcctgt accaaggcat ctgcaagctg       420
   ctgaggctgg aggacctctt caagctgggg gagccttatc ataagaagga gcactatctg       480
   tcaggtgtca acgagagcgg ctcagtcttt ggagtgatcg tctcctacag caacctggat       540
   gacaagctgt tcattgccac ggcagtggat gggaagcccg agtattttcc caccatctcc       600
   agccggaaac tgaccaagaa ctctgaggcg gatggcatgt tcgcgtacgt cttccatgat       660
   gagttcgtgg cctcgatgat taagatccct tcggacacct tcaccatcat ccctgacttt       720
   gatatctact atgtctatgg ttttagcagt ggcaactttg tctacttttt gaccctccaa       780
   cctgagatgg tgtctccacc aggctccacc accaaggagc aggtgtatac atccaagctc       840
   gtgaggcttt gcaaggagga cacagccttc aactcctatg tagaggtgcc cattggctgt       900
   gagcgcagtg gggtggagta ccgcctgctg caggctgcct acctgtccaa agcgggggcc        960
   gtgcttggca ggacccttgg agtccatcca gatgatgacc tgctcttcac cgtcttctcc      1020
   aagggccaga agcggaaaat gaaatccctg gatgagtcgg ccctgtgcat cttcatcttg      1080
   aagcagataa atgaccgcat taaggagcgg ctgcagtctt gttaccgggg cgagggcacg      1140
   ctggacctgg cctggctcaa ggtgaaggac atcccctgca gcagtgcgct cttaaccatt      1200
   gacgataact tctgtggcct ggacatgaat gctcccctgg gagtgtccga catggtgcgt      1260
   ggaattcccg tcttcacgga ggacagggac cgcatgacgt ctgtcatcgc atatgtctac      1320
   aagaaccact ctctggcctt tgtgggcacc aaaagtggca agctgaagaa gatccgggtg      1380
   gatggaccca ggggcaacgc cctccagtat gagacggtgc aggtggtgga ccccggccca      1440
   gtcctccggg atatggcctt ctccaaggac cacgagcaac tctacatcat gtcagagagg      1500
   cagctcacca gagtccctgt ggagtcctgt ggtcagtatc agagctgcgg cgagtgcctt      1560
   ggctcaggcg acccccactg tggctggtgt gtgctgcaca acacttgcac ccggaaggag      1620
   cggtgtgagc ggtccaagga gcccgactac aaggacgacg atgacaagtg atga            1674
<210> 68
   <211> 556
   <212> PRT
   <213> Homo sapiens
<400> 68
   Met Lys Ala Met Pro Trp Asn Trp Thr Cys Leu Leu Ser His Leu Leu
   1               5                   10                  15
   Met Val Gly Met Gly Ser Ser Thr Leu Leu Thr Arg Gln Pro Ala Pro
               20                  25                  30
   Leu Ser Gln Lys Gln Arg Ser Phe Val Thr Phe Arg Gly Glu Pro Ala
           35                  40                  45
   Glu Gly Phe Asn His Leu Val Val Asp Glu Arg Thr Gly His Ile Tyr
       50                  55                  60
   Leu Gly Ala Val Asn Arg Ile Tyr Lys Leu Ser Ser Asp Leu Lys Val
   65                  70                  75                  80
   Leu Val Thr His Glu Thr Gly Pro Asp Glu Asp Asn Pro Lys Cys Tyr
                   85                  90                  95
   Pro Pro Arg Ile Val Gln Thr Cys Asn Glu Pro Leu Thr Thr Thr Asn
               100                 105                 110
   Asn Val Asn Lys Met Leu Leu Ile Asp Tyr Lys Glu Asn Arg Leu Ile
           115                 120                 125
   Ala Cys Gly Ser Leu Tyr Gln Gly Ile Cys Lys Leu Leu Arg Leu Glu
       130                 135                 140
   Asp Leu Phe Lys Leu Gly Glu Pro Tyr His Lys Lys Glu His Tyr Leu
   145                 150                 155                 160
   Ser Gly Val Asn Glu Ser Gly Ser Val Phe Gly Val Ile Val Ser Tyr
                   165                 170                 175
   Ser Asn Leu Asp Asp Lys Leu Phe Ile Ala Thr Ala Val Asp Gly Lys
               180                 185                 190
   Pro Glu Tyr Phe Pro Thr Ile Ser Ser Arg Lys Leu Thr Lys Asn Ser
           195                 200                 205
   Glu Ala Asp Gly Met Phe Ala Tyr Val Phe His Asp Glu Phe Val Ala
       210                 215                 220
   Ser Met Ile Lys Ile Pro Ser Asp Thr Phe Thr Ile Ile Pro Asp Phe
   225                 230                 235                 240
   Asp Ile Tyr Tyr Val Tyr Gly Phe Ser Ser Gly Asn Phe Val Tyr Phe
                   245                 250                 255
   Leu Thr Leu Gln Pro Glu Met Val Ser Pro Pro Gly Ser Thr Thr Lys
               260                 265                 270
   Glu Gln Val Tyr Thr Ser Lys Leu Val Arg Leu Cys Lys Glu Asp Thr
           275                 280                 285
   Ala Phe Asn Ser Tyr Val Glu Val Pro Ile Gly Cys Glu Arg Ser Gly
       290                 295                 300
   Val Glu Tyr Arg Leu Leu Gln Ala Ala Tyr Leu Ser Lys Ala Gly Ala
   305                 310                 315                 320
   Val Leu Gly Arg Thr Leu Gly Val His Pro Asp Asp Asp Leu Leu Phe
                   325                 330                 335
   Thr Val Phe Ser Lys Gly Gln Lys Arg Lys Met Lys Ser Leu Asp Glu
               340                 345                 350
   Ser Ala Leu Cys Ile Phe Ile Leu Lys Gln Ile Asn Asp Arg Ile Lys
   355 360 365
   Glu Arg Leu Gln Ser Cys Tyr Arg Gly Glu Gly Thr Leu Asp Leu Ala
   370 375 380
   Trp Leu Lys Val Lys Asp Ile Pro Cys Ser Ser Ala Leu Leu Thr Ile
   385 390 395 400
   Asp Asp Asn Phe Cys Gly Leu Asp Met Asn Ala Pro Leu Gly Val Ser
   405 410 415
   Asp Met Val Arg Gly Ile Pro Val Phe Thr Glu Asp Arg Asp Arg Met
   420 425 430
   Thr Ser Val Ile Ala Tyr Val Tyr Lys Asn His Ser Leu Ala Phe Val
   435 440 445
   Gly Thr Lys Ser Gly Lys Leu Lys Lys Ile Arg Val Asp Gly Pro Arg
   450 455 460
   Gly Asn Ala Leu Gln Tyr Glu Thr Val Gln Val Val Asp Pro Gly Pro
   465 470 475 480
   Val Leu Arg Asp Met Ala Phe Ser Lys Asp His Glu Gln Leu Tyr Ile
   485 490 495
   Met Ser Glu Arg Gln Leu Thr Arg Val Pro Val Glu Ser Cys Gly Gln
   500 505 510
   Tyr Gln Ser Cys Gly Glu Cys Leu Gly Ser Gly Asp Pro His Cys Gly
   515 520 525
   Trp Cys Val Leu His Asn Thr Cys Thr Arg Lys Glu Arg Cys Glu Arg
   530 535 540
   Ser Lys Glu Pro Asp Tyr Lys Asp Asp Asp Asp Lys
   545 550 555
<210> 69
   <211> 333
   <212> DNA
   <213> Artificial Sequence
<220>
   <223> Synthetic DNA
<400> 69
   cagtctgccc tgactcagcc tgcctccgtg tctgggtctc ctggacagtc gatcaccatc 60
   tcctgctctg gaagcagctc caacatcgga agtaattatg tatactggta ccaacagcac 120
   ccaggcaaag cccccaaact catgatttat aggaataatc agcggccctc aggggtttct 180
   aatcgcttct ctggctccaa gtctggcaac acggcctccc tgaccatctc tgggctccag 240
   gctgaggacg aggctgatta ttactgcgca gcatgggatg acagcctgag tggtcattgg 300
   gtgttcggcg gagggaccaa gctgaccgtc cta 333
<210> 70
   <211> 333
   <212> DNA
   <213> Artificial Sequence
<220>
   <223> Synthetic DNA
<400> 70
   cagtctgccc tgactcagcc tgcctccgtg tctgggtctc ctggacagtc gatcaccatc 60
   tcctgctctg gaagcagctc caacatcgga aataatgctg taaactggta ccaacagcac 120
   ccaggcaaag cccccaaact catgatttat tatgatgatc tgctgccctc aggggtttct 180
   aatcgcttct ctggctccaa gtctggcaac acggcctccc tgaccatctc tgggctccag 240
   gctgaggacg aggctgatta ttactgcgga acatgggatg gcagcctgaa tggtgggagg 300
   ctgttcggcg gagggaccaa gctgaccgtc cta 333
<210> 71
   <211> 324
   <212> DNA
   <213> Artificial Sequence
<220>
   <223> Synthetic DNA
<400> 71
   cagtctgccc tgactcagcc tgcctccgtg tctgggtctc ctggacagtc gatcaccatc 60
   tcctgctctg gagaagcatt gccaaaaaaa tatgcttatt ggtaccaaca gcacccaggc 120
   aaagccccca aactcatgat ttataaagac agcgagaggc cctcaggggt ttctaatcgc 180
   ttctctggct ccaagtctgg caacacggcc tccctgacca tctctgggct ccaggctgag 240
   gacgaggctg attattactg caactctttt acaagcacca ccaattatgt cttattcggc 300
   ggagggacca agctgaccgt ccta 324
<210> 72
   <211> 345
   <212> DNA
   <213> Artificial Sequence
<220>
   <223> Synthetic DNA
<210> 73
   <211> 336
   <212> DNA
   <213> Artificial Sequence
<220>
   <223> Synthetic DNA
<400> 73
   cagtctgccc tgactcagcc tgcctccgtg tctgggtctc ctggacagtc gatcaccatc      60
   tcctgcactc tgagcagtgg gcacagtagc tacatcatcg catggtacca acagcaccca     120
   ggcaaagccc ccaaactcat gatttatctt gaaggtagtg gaagctacaa caaggggagc     180
   ggggtttcta atcgcttctc tggctccaag tctggcaaca cggcctccct gaccatctct     240
   gggctccagg ctgaggacga ggctgattat tactgcgaga cctgggacag taacactcat     300
   gtggtattcg gcggagggac caagctgacc gtccta                                336
<210> 74
   <211> 333
   <212> DNA
   <213> Artificial Sequence
<220>
   <223> Synthetic DNA
<400> 74
   cagtctgccc tgactcagcc tgcctccgtg tctgggtctc ctggacagtc gatcaccatc 60
   tcctgctctg gaagcagctc caacatcgga agtaattatg tatactggta ccaacagcac 120
   ccaggcaaag cccccaaact catgatttat aggaataatc agcggccctc aggggtttct 180
   aatcgcttct ctggctccaa gtctggcaac acggcctccc tgaccatctc tgggctccag 240
   gctgaggacg aggctgatta ttactgcgca gcatgggatg acagcctgag tggtcattgg 300
   gtgttcggcg gagggaccaa gctgaccgtc cta 333
<210> 75
   <211> 336
   <212> DNA
   <213> Artificial Sequence
<220>
   <223> Synthetic DNA
<400> 75
   cagtctgccc tgactcagcc tgcctccgtg tctgggtctc ctggacagtc gatcaccatc 60
   tcctgcactg ggagcagctc caacatcggg gcaggttatg atgtacactg gtaccaacag 120
   cacccaggca aagcccccaa actcatgatt tatggtaaca gcaatcggcc ctcaggggtt 180
   tctaatcgct tctctggctc caagtctggc aacacggcct ccctgaccat ctctgggctc 240
   caggctgagg acgaggctga ttattactgc cagtcctatg acagcagcct gagtggttct 300
   gtggtattcg gcggagggac caagctgacc gtccta 336
<210> 76
   <211> 333
   <212> DNA
   <213> Artificial Sequence
<220>
   <223> Synthetic DNA
<400> 76
   cagtctgccc tgactcagcc tgcctccgtg tctgggtctc ctggacagtc gatcaccatc 60
   tcctgctctg gaagcagctc caacattggg aataattatg tatcctggta ccaacagcac 120
   ccaggcaaag cccccaaact catgatttat gacaataata agcgaccctc aggggtttct 180
   aatcgcttct ctggctccaa gtctggcaac acggcctccc tgaccatctc tgggctccag 240
   gctgaggacg aggctgatta ttactgcgga acatgggata gcagcctgag tgctggtgct 300
   gtgttcggcg gagggaccaa gctgaccgtc cta 333
<210> 77
   <211> 333
   <212> DNA
   <213> Artificial Sequence
<220>
   <223> Synthetic DNA
<400> 77
   cagtctgccc tgactcagcc tgcctccgtg tctgggtctc ctggacagtc gatcaccatc      60
   tcctgcactg ggagcagctc caacattggg gcgggttatg ttgtacattg gtaccaacag     120
   cacccaggca aagcccccaa actcatgatt tatgaaaata ataagcgacc ctcaggggtt     180
   tctaatcgct tctctggctc caagtctggc aacacggcct ccctgaccat ctctgggctc     240
   caggctgagg acgaggctga ttattactgc agctcatata caagcagcgg cactcctgtg     300
   gtattcggcg gagggaccaa gctgaccgtc cta                                  333
<210> 78
   <211> 363
   <212> DNA
   <213> Artificial Sequence
<220>
   <223> Synthetic DNA
<400> 78
   cagtctgccc tgactcagcc tgcctccgtg tctgggtctc ctggacagtc gatcaccatc      60
   tcctgcactg gaaccagcag tgatgttggg agttataacc ttgtctcctg gtaccaacag     120
   cacccaggca aagcccccaa actcatgatt tatgagggca gtaagcggcc ctcaggggtt     180
   tctaatcgct tctctggctc caagtctggc aacacggcct ccctgaccat ctctgggctc     240
   caggctgagg acgaggctga ttattactgc cagtcctatg acagcagcct gagtggttgc     300
   cagtcctatg acagcagcct gagtggtttg gtgttcggcg gagggaccaa gctgaccgtc     360
   cta                                                                   363
<210> 79
   <211> 318
   <212> DNA
   <213> Artificial Sequence
<220>
   <223> Synthetic DNA
<400> 79
   cagtctgccc tgactcagcc tgcctccgtg tctgggtctc ctggacagtc gatcaccatc 60
   tcctgcgggg gaaacaacat tggaagtaaa aatgtgcact ggtaccaaca gcacccaggc 120
   aaagccccca aactcatgat ttatagggat agcaaccggc cctctggggt ttctaatcgc 180
   ttctctggct ccaagtctgg caacacggcc tccctgacca tctctgggct ccaggctgag 240
   gacgaggctg attattactg ccagtcctat gacaggagtg gttgggtgtt cggcggaggg 300
   accaagctga ccgtccta 318
<210> 80
   <211> 324
   <212> DNA
   <213> Artificial Sequence
<220>
   <223> Synthetic DNA
<400> 80
   cagtctgccc tgactcagcc tgcctccgtg tctgggtctc ctggacagtc gatcaccatc 60
   tcctgctctg gagaagcatt gccaaaaaaa tatgcttatt ggtaccaaca gcacccaggc 120
   aaagccccca aactcatgat ttataaagac agcgagaggc cctcaggggt ttctaatcgc 180
   ttctctggct ccaagtctgg caacacggcc tccctgacca tctctgggct ccaggctgag 240
   gacgaggctg attattactg caactctttt acaagcacca ccaattatgt cttattcggc 300
   ggagggacca agctgaccgt ccta 324
<210> 81
   <211> 324
   <212> DNA
   <213> Artificial Sequence
<220>
   <223> Synthetic DNA
<400> 81
   cagtctgccc tgactcagcc tgcctccgtg tctgggtctc ctggacagtc gatcaccatc 60
   tcctgcgggg gaaacaacat tggaagtaaa agtgtgcact ggtaccaaca gcacccaggc 120
   aaagccccca aactcatgat ttattatgat agcgaccggc cctcaggggt ttctaatcgc 180
   ttctctggct ccaagtctgg caacacggcc tccctgacca tctctgggct ccaggctgag 240
   gacgaggctg attattactg ccaggtgtgg gatgataata gtgatcatct tatattcggc 300
   ggagggacca agctgaccgt ccta 324
<210> 82
   <211> 321
   <212> DNA
   <213> Artificial Sequence
<220>
   <223> Synthetic DNA
<400> 82
   cagtctgccc tgactcagcc tgcctccgtg tctgggtctc ctggacagtc gatcaccatc 60
   tcctgctctg gagatgcatt gccaaagcaa tatgcttatt ggtaccaaca gcacccaggc     120
   aaagccccca aactcatgat ttatgatgat agcgaccggc cctcaggggt ttctaatcgc     180
   ttctctggct ccaagtctgg caacacggcc tccctgacca tctctgggct ccaggctgag 240
   gacgaggctg attattactg cagctcatat gcaggcagca acaattgggt gttcggcgga 300
   gggaccaagc tgaccgtcct a 321
<210> 83
   <211> 345
   <212> DNA
   <213> Artificial Sequence
<220>
   <223> Synthetic DNA
<400> 83
   cagtctgccc tgactcagcc tgcctccgtg tctgggtctc ctggacagtc gatcaccatc 60
   tcctgcaccc taagcagtga gcacagcacc tacaccatcg aatggtacca acagcaccca 120
   ggcaaagccc ccaaactcat gatttatgtt aagagtgatg gcagccacag caagggggac 180
   ggggtttcta atcgcttctc tggctccaag tctggcaaca cggcctccct gaccatctct 240
   gggctccagg ctgaggacga ggctgattat tactgcggag agagccacac gattgatggc 300
   caagtcggtg tggtattcgg cggagggacc aagctgaccg tccta 345
<210> 84
   <211> 345
   <212> DNA
   <213> Artificial Sequence
<220>
   <223> Synthetic DNA
<400> 84
   cagtctgccc tgactcagcc tgcctccgtg tctgggtctc ctggacagtc gatcaccatc 60
   tcctgcactc tgagcagtgg gcacagcagc tacgccatcg catggtacca acagcaccca 120
   ggcaaagccc ccaaactcat gatttatctt aacagtgatg gcagccacag caagggggac 180
   ggggtttcta atcgcttctc tggctccaag tctggcaaca cggcctccct gaccatctct 240
   gggctccagg ctgaggacga ggctgattat tactgcagct catatgcagg caacaacaat 300
   ttgggggatg tgcttttcgg cggagggacc aagctgaccg tccta 345
<210> 85
   <211> 342
   <212> DNA
   <213> Artificial Sequence
<220>
   <223> Synthetic DNA
<400> 85
   cagtctgccc tgactcagcc tgcctccgtg tctgggtctc ctggacagtc gatcaccatc 60
   tcctgcacct tgcgcagtgg catcaatgtt ggtacctaca ggatatactg gtaccaacag 120
   cacccaggca aagcccccaa actcatgatt tattacaaat cagactcaga taagcagcag 180
   ggctctgggg tttctaatcg cttctctggc tccaagtctg gcaacacggc ctccctgacc 240
   atctctgggc tccaggctga ggacgaggct gattattact gcgcagcatg ggatgacagc 300
   ctgaatgtgg tattcggcgg agggaccaag ctgaccgtcc ta 342
<210> 86
   <211> 327
   <212> DNA
   <213> Artificial Sequence
<220>
   <223> Synthetic DNA
<400> 86
   cagtctgccc tgactcagcc tgcctccgtg tctgggtctc ctggacagtc gatcaccatc 60
   tcctgcaccc gcagcagtgg cagcattgcc agcaactatg tgcagtggta ccaacagcac 120
   ccaggcaaag cccccaaact catgatttat gaggataacc aaagaccctc tggggtttct 180
   aatcgcttct ctggctccaa gtctggcaac acggcctccc tgaccatctc tgggctccag 240
   gctgaggacg aggctgatta ttactgccag tcctatgaca gcagcctgag gagagtcttc 300
   ggcggaggga ccaagctgac cgtccta 327
<210> 87
   <211> 333
   <212> DNA
   <213> Artificial Sequence
<220>
   <223> Synthetic DNA
<400> 87
   cagtctgccc tgactcagcc tgcctccgtg tctgggtctc ctggacagtc gatcaccatc      60
   tcctgcggct ccagcactgg agctgtcacc agtggtcatt atccctactg gtaccaacag     120
   cacccaggca aagcccccaa actcatgatt tatgatacaa gcaacaaaca ctccggggtt     180
   tctaatcgct tctctggctc caagtctggc aacacggcct ccctgaccat ctctgggctc     240
   caggctgagg acgaggctga ttattactgc gcaacatggg atgacagcct gaatgaggga     300
   gtgttcggcg gagggaccaa gctgaccgtc cta                                  333
<210> 88
   <211> 348
   <212> DNA
   <213> Artificial Sequence
<220>
   <223> Synthetic DNA
<400> 88
   cagtctgccc tgactcagcc tgcctccgtg tctgggtctc ctggacagtc gatcaccatc      60
   tcctgcaccc tgagcagcgg ctacagtaat tataaagtgg actggtacca acagcaccca     120
   ggcaaagccc ccaaactcat gatttatgtg ggcactggtg ggattgtggg atccaagggg     180
   gatggggttt ctaatcgctt ctctggctcc aagtctggca acacggcctc cctgaccatc     240
   tctgggctcc aggctgagga cgaggctgat tattactgcg gggcagacca tggcagtggg     300
   agcaacttcg tggtggtatt cggcggaggg accaagctga ccgtccta                  348
<210> 89
   <211> 369
   <212> DNA
   <213> Artificial Sequence
<220>
   <223> Synthetic DNA
<400> 89
   gaggtgcagc tgttggagtc tgggggaggc ttggtacagc ctggggggtc cctgagactc 60
   tcctgtgcag cctctggatt cacctttagc ggctactata tgcactgggt ccgccaggct 120
   ccagggaagg ggctggagtg ggtctcatgg atcaacccta acagtggtgg cacaaactat 180
   gcacagaagt ttcagggccg gttcaccatc tccagagaca attccaagaa cacgctgtat 240
   ctgcaaatga acagcctgag agccgaggac acggccgtat attactgtgc gaaaggttgg 300
   ggctatacgg tgactaccct caactggttc gacccctggg gccagggcac cctggtcacc 360
   gtctcctca 369
<210> 90
   <211> 366
   <212> DNA
   <213> Artificial Sequence
<220>
   <223> Synthetic DNA
<400> 90
   gaggtgcagc tgttggagtc tgggggaggc ttggtacagc ctggggggtc cctgagactc 60
   tcctgtgcag cctctggatt cacctttagc agctatgcta tgcattgggt ccgccaggct 120
   ccagggaagg ggctggagtg ggtctcatgg atcaacgctg gcaatggtaa cacaaaatat 180
   tcacagaagt tccagggccg gttcaccatc tccagagaca attccaagaa cacgctgtat 240
   ctgcaaatga acagcctgag agccgaggac acggccgtat attactgtgc gaaagcgtct 300
   agtagtggtt attactacgt ccactttgac tactggggcc agggcaccct ggtcaccgtc 360
   tcctca 366
<210> 91
   <211> 363
   <212> DNA
   <213> Artificial Sequence
<220>
   <223> Synthetic DNA
<400> 91
   gaggtgcagc tgttggagtc tgggggaggc ttggtacagc ctggggggtc cctgagactc 60
   tcctgtgcag cctctggatt cacctttagc agttatgata tcaactgggt ccgccaggct 120
   ccagggaagg ggctggagtg ggtctcatgg atgaacccta acagtggtaa cacaggctat 180
   gcacagaagt tccagggccg gttcaccatc tccagagaca attccaagaa cacgctgtat 240
   ctgcaaatga acagcctgag agccgaggac acggccgtat attactgtgc gaaatccagc 300
   agctggcact actactacgg tatggacgtc tggggccagg gcaccctggt caccgtctcc 360
   tca 363
<210> 92
   <211> 360
   <212> DNA
   <213> Artificial Sequence
<220>
   <223> Synthetic DNA
<400> 92
   gaggtgcagc tgttggagtc tgggggaggc ttggtacagc ctggggggtc cctgagactc 60
   tcctgtgcag cctctggatt cacctttagc agctatggta tcagctgggt ccgccaggct 120
   ccagggaagg ggctggagtg ggtctcatgg atcagcgctt acaatggtaa cacaaactat 180
   gcacagaagc tccagggccg gttcaccatc tccagagaca attccaagaa cacgctgtat 240
   ctgcaaatga acagcctgag agccgaggac acggccgtat attactgtgc gaaagattta 300
   aggttatcct attactactt tgactactgg ggccagggca ccctggtcac cgtctcctca 360
<210> 93
   <211> 360
   <212> DNA
   <213> Artificial Sequence
<220>
   <223> Synthetic DNA
<400> 93
   gaggtgcagc tgttggagtc tgggggaggc ttggtacagc ctggggggtc cctgagactc 60
   tcctgtgcag cctctggatt cacctttagc gaattatcca tgcactgggt ccgccaggct 120
   ccagggaagg ggctggagtg ggtctcaggt tttgatcctg aagatggtga aacaatctac 180
   gcacagaagt tccagggccg gttcaccatc tccagagaca attccaagaa cacgctgtat 240
   ctgcaaatga acagcctgag agccgaggac acggccgtat attactgtgc gaaaggaacc 300
   tactactact actacggtat ggacgtctgg ggccagggca ccctggtcac cgtctcctca 360
<210> 94
   <211> 369
   <212> DNA
   <213> Artificial Sequence
<220>
   <223> Synthetic DNA
<400> 94 ggctatacgg tgactaccct caactggttc gacccctggg gccagggcac cctggtcacc 360
gtctcctca 369
<210> 96
   <211> 360
   <212> DNA
   <213> Artificial Sequence
<220>
   <223> Synthetic DNA
<400> 96
   gaggtgcagc tgttggagtc tgggggaggc ttggtacagc ctggggggtc cctgagactc 60
   tcctgtgcag cctctggatt cacctttagc gaattatcca tgcactgggt ccgccaggct 120
   ccagggaagg ggctggagtg ggtctcaggt tttgatcctg aagatggtga aacaatctac 180
   gcacagaagt tccagggccg gttcaccatc tccagagaca attccaagaa cacgctgtat 240
   ctgcaaatga acagcctgag agccgaggac acggccgtat attactgtgc gaaaggaacc 300
   tactactact actacggtat ggacgtctgg ggccagggca ccctggtcac cgtctcctca 360
<210> 97
   <211> 363
   <212> DNA
   <213> Artificial Sequence
<220>
   <223> Synthetic DNA
<400> 97
   gaggtgcagc tgttggagtc tgggggaggc ttggtacagc ctggggggtc cctgagactc 60
   tcctgtgcag cctctggatt cacctttagc agctactata tgcactgggt ccgccaggct 120
   ccagggaagg ggctggagtg ggtctcaata atcaacccta gtggtggtag cacaagctac 180
   gcacagaagt tccagggccg gttcaccatc tccagagaca attccaagaa cacgctgtat 240
   ctgcaaatga acagcctgag agccgaggac acggccgtat attactgtgc gaaaagtttc 300
   agcagcagct ggtacaactg gttcgacccc tggggccagg gcaccctggt caccgtctcc 360
   tca 363
<210> 98
   <211> 357
   <212> DNA
   <213> Artificial Sequence
<220>
   <223> Synthetic DNA
<400> 98
   gaggtgcagc tgttggagtc tgggggaggc ttggtacagc ctggggggtc cctgagactc 60
   tcctgtgcag cctctggatt cacctttagc agctatgcta tcagctgggt ccgccaggct 120
   ccagggaagg ggctggagtg ggtctcaggg atcatcccta tctttggtac agcaaactac 180
   gcacagaagt tccagggccg gttcaccatc tccagagaca attccaagaa cacgctgtat 240
   ctgcaaatga acagcctgag agccgaggac acggccgtat attactgtgc gaaagggtcc 300
   gtcctcggtg atgcttttga tatctggggc cagggcaccc tggtcaccgt ctcctca 357
<210> 99
   <211> 366
   <212> DNA
   <213> Artificial Sequence
<220>
   <223> Synthetic DNA
<400> 99
   gaggtgcagc tgttggagtc tgggggaggc ttggtacagc ctggggggtc cctgagactc        60
   tcctgtgcag cctctggatt cacctttagc aatgctagaa tgggtgtgag ctgggtccgc       120
   caggctccag ggaaggggct ggagtgggtc tcacacattt tttcgaatga cgaaaaatcc       180
   tacagcacat ctctgaagag ccggttcacc atctccagag acaattccaa gaacacgctg       240
   tatctgcaaa tgaacagcct gagagccgag gacacggccg tatattactg tgcgaaaatc       300
   ccgtatgaag cagtggctgg ctactttgac tactggggcc agggcaccct ggtcaccgtc       360
   tcctca                                                                  366
<210> 100
   <211> 360
   <212> DNA
   <213> Artificial Sequence
<220>
   <223> Synthetic DNA
<400> 100
   gaggtgcagc tgttggagtc tgggggaggc ttggtacagc ctggggggtc cctgagactc        60
   tcctgtgcag cctctggatt cacctttagc agctattgga tgagctgggt ccgccaggct       120
   ccagggaagg ggctggagtg ggtctcaaac ataaagcaag atggaagtga gaaatactat       180
   gtggactctg tgaagggccg gttcaccatc tccagagaca attccaagaa cacgctgtat       240
   ctgcaaatga acagcctgag agccgaggac acggccgtat attactgtgc gaaagttacg       300
   gtgaccggga cctggtactt cgatctctgg ggccagggca ccctggtcac cgtctcctca       360
<210> 101
   <211> 372
   <212> DNA
   <213> Artificial Sequence
<220>
   <223> Synthetic DNA
<400> 101
   gaggtgcagc tgttggagtc tgggggaggc ttggtacagc ctggggggtc cctgagactc        60
   tcctgtgcag cctctggatt cacctttagc agctacgaca tgcactgggt ccgccaggct       120
   ccagggaagg ggctggagtg ggtctcagct attggtactg ctggtgacac atactatcca       180
   ggctccgtga agggccggtt caccatctcc agagacaatt ccaagaacac gctgtatctg       240
   caaatgaaca gcctgagagc cgaggacacg gccgtatatt actgtgcgaa agcggataca       300
   gctatggtca tttactacta ctactacggt atggacgtct ggggccaggg caccctggtc       360
   accgtctcct ca                                                           372
<210> 102
   <211> 357
   <212> DNA
   <213> Artificial Sequence
<220>
   <223> Synthetic DNA
<400> 102
   gaggtgcagc tgttggagtc tgggggaggc ttggtacagc ctggggggtc cctgagactc        60
   tcctgtgcag cctctggatt cacctttagc agctatagca tgaactgggt ccgccaggct       120
   ccagggaagg ggctggagtg ggtctcatcc attagtagta gtagtagtta catatactac       180
   gcagactcag tgaagggccg gttcaccatc tccagagaca attccaagaa cacgctgtat       240
   ctgcaaatga acagcctgag agccgaggac acggccgtat attactgtgc gaaagagaat       300
   agtggctatg attactttga ctactggggc cagggcaccc tggtcaccgt ctcctca          357
<210> 103
   <211> 357
   <212> DNA
   <213> Artificial Sequence
<220>
   <223> Synthetic DNA
<400> 103
   gaggtgcagc tgttggagtc tgggggaggc ttggtacagc ctggggggtc cctgagactc        60
   tcctgtgcag cctctggatt cacctttagc gattatgcta tgagctgggt ccgccaggct       120
   ccagggaagg ggctggagtg ggtctcattc attagaagca aagcttatgg tgggacaaca       180
   gaatacaccg cgtctgtgaa aggccggttc accatctcca gagacaattc caagaacacg       240
   ctgtatctgc aaatgaacag cctgagagcc gaggacacgg ccgtatatta ctgtgcgaaa       300
   gatctaggta gtggctttga ctactggggc cagggcaccc tggtcaccgt ctcctca          357
<210> 104
   <211> 378
   <212> DNA
   <213> Artificial Sequence
<220>
   <223> Synthetic DNA
<400> 104
   gaggtgcagc tgttggagtc tgggggaggc ttggtacagc ctggggggtc cctgagactc        60
   tcctgtgcag cctctggatt cacctttagc gaccactaca tggactgggt ccgccaggct       120
   ccagggaagg ggctggagtg ggtctcacgt actagaaaca aagctaacag ttacaccaca       180
   gaatacgccg cgtctgtgaa aggccggttc accatctcca gagacaattc caagaacacg       240
   ctgtatctgc aaatgaacag cctgagagcc gaggacacgg ccgtatatta ctgtgcgaaa       300
   ggaggccagg ggagccgata ctactactac tacggtatgg acgtctgggg ccagggcacc       360
   ctggtcaccg tctcctca                                                     378
<210> 105
   <211> 345
   <212> DNA
   <213> Artificial Sequence
<220>
   <223> Synthetic DNA
<400> 105
   gaggtgcagc tgttggagtc tgggggaggc ttggtacagc ctggggggtc cctgagactc        60
   tcctgtgcag cctctggatt cacctttagc agcaatgaga tgagctgggt ccgccaggct       120
   ccagggaagg ggctggagtg ggtctcatcc attagtggtg gtagcacata ctacgcagac       180
   tccaggaagg gccggttcac catctccaga gacaattcca agaacacgct gtatctgcaa       240
   atgaacagcc tgagagccga ggacacggcc gtatattact gtgcgaaaga ctcatatgat       300
   gcttttgata tctggggcca gggcaccctg gtcaccgtct cctca                       345
<210> 106
   <211> 357
   <212> DNA
   <213> Artificial Sequence
<220>
   <223> Synthetic DNA
<400> 106
   gaggtgcagc tgttggagtc tgggggaggc ttggtacagc ctggggggtc cctgagactc        60
   tcctgtgcag cctctggatt cacctttagc agtggtggtt actcctggag ctgggtccgc       120
   caggctccag ggaaggggct ggagtgggtc tcatacatct atcatagtgg gagcacctac       180
   tacaacccgt ccctcaagag tcggttcacc atctccagag acaattccaa gaacacgctg       240
   tatctgcaaa tgaacagcct gagagccgag gacacggccg tatattactg tgcgaaagat       300
   gcgagtaacc atttctttga ctactggggc cagggcaccc tggtcaccgt ctcctca          357
<210> 107
   <211> 360
   <212> DNA
   <213> Artificial Sequence
<220>
   <223> Synthetic DNA
<400> 107
   gaggtgcagc tgttggagtc tgggggaggc ttggtacagc ctggggggtc cctgagactc        60
   tcctgtgcag cctctggatt cacctttagc agtagtagtt actactgggg ctgggtccgc       120
   caggctccag ggaaggggct ggagtgggtc tcaagtatct attatagtgg gagcacctac       180
   tacaacccgt ccctcaagag tcggttcacc atctccagag acaattccaa gaacacgctg       240
   tatctgcaaa tgaacagcct gagagccgag gacacggccg tatattactg tgcgaaaaag       300
   gactggaacg acaactggtt cgacccctgg ggccagggca ccctggtcac cgtctcctca       360
<210> 108
   <211> 381
   <212> DNA
   <213> Artificial Sequence
<220>
   <223> Synthetic DNA
<400> 108
   gaggtgcagc tgttggagtc tgggggaggc ttggtacagc ctggggggtc cctgagactc        60
   tcctgtgcag cctctggatt cacctttagc agcaacagtg ctgcttggaa ctgggtccgc       120
   caggctccag ggaaggggct ggagtgggtc tcaaggacat actacaggtc caagtggtat       180
   aatgattatg cagtatctgt gaaaagtcgg ttcaccatct ccagagacaa ttccaagaac       240
   acgctgtatc tgcaaatgaa cagcctgaga gccgaggaca cggccgtata ttactgtgcg       300
   aaagatattg tagtagtacc agctgctata tactggtact tcgatctctg gggccagggc       360
   accctggtca ccgtctcctc a                                                 381
<210> 109
   <211> 14405
   <212> DNA
   <213> Artificial Sequence
<220>
   <223> Synthetic DNA
<400> 109
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtttccc gaagcgaaag ccgcgtgtta     420
   tttttgggtg ggaaagcccc gattcgctcc ttttttttta tttattattt ttgttgtttt     480
   tcgtgtcttt ttcttctccg ttttgcagct gcggcctcca ggtgtccccg gccgtggtat     540
   ttttgggtcg aaaaagccgc tttttggctc acttggaaac ctttcgggcg tctccaagcg     600
   gagcgttggc gggaaatggt gttttttttg ggttgttttc ggtccctttt ttcaccattt     660
   ttgtggtttt tcggcccatt ttcggcctcc cccccccccc cccccttccc gtggggctgc     720
   gctccgtgga gcggaatggg gtcgttaatt aaggttaatt ggtttaatta atgggccgtg     780
   cagggagaag agggccgtgc ggtggttgtt agccctgatt aatggcttgg ttgaggggag     840
   agtgtaatta agtgcttcgt tagcactaat taatggcttg attgagagga gagtgtaatt     900
   aaatgcttca taatccctaa ttaatggctt ggttgagaca agagtgtaat taagtgcttc     960
   attagccctg attaatggct tggttgaggg gagagtgtaa ttaaatgctt cgttagcact    1020
   aattaatggc ttgattgaga ggagagtgta attaaatgct tcataatccc taattaatgg    1080
   cttggttgag acaagagtgt aattaagtgc ttcattagcc ctgattaatg gcttggttga    1140
   gaggagagtg taattaaatg cttcattatc cctaattagg gactgaattc caaggagagt    1200
   gtaattaagt gcttcattat ccctaattaa tggcttggtt gagaggagag tgtaattaaa    1260
   tgctttgtta gccctaatta atggtttgat ggagaggaga gtgtaattaa gtgcttcatt    1320
   atccctaatt aatggcttgg ttgaggggag agtgtaatta aatgcttagt tagccctaat 1380
   taatggcttg gttgagagga gagtgtaatt gaatgcttca ttatccctaa ttagggattg 1440
   aattgagagg agagtgtaat tgaatgcttt gttagcccta attagggatt gaattgagag 1500
   gagagtgtaa ttaaatgcct cattatccct aattagggat tgaattgaga ggagagttta 1560
   attaaatgcc tcattatccc taattaggga ctgaattccg acgagagtgt aattaagtgc 1620
   ttcatttagc cctaattaat ggcttgattg agaggagagt gtaattaaat gcttcattat 1680
   ccctaattag ggattgaatt gagaggagag tgtaattaaa tgcttcatta tccctaatta 1740
   gggattgaat tgagaggaga gtgtaattaa atgcttcatt atccctaatt aatggcttgg 1800
   ttgagagggg agtgtaatta agtgcttcgt tatccctaat tagggattga attgtgagga 1860
   gatggcaatt aatacttcat tatccctaat taatttaatt agtccctatt atccctaatt 1920
   taattcatgg tttggttgat ggaagaatgt aattaaatgc tttgttatcc ctaattaatg 1980
   gtttgattga gcggagagtg taattaaatg cttcattatc ccttattaat ggttgtgtgg 2040
   aatgtagtgt aattagaagt ccctaattaa tggtttaatt gagagaagac tgcaattaga 2100
   tgactcatag cccctaataa gtggtttaat tatgagataa tcacaattac atgtttcatt 2160
   gtccctaatt aatcgtttaa ttgcgagaag atcacaatta attaaatgat ccctaattgc 2220
   taccgttcgt ataatgtata ctatacgaag ttattttatt tgtgaaattt gtgatgctat 2280
   tgctttattt gtaaccatta taagctgcaa taaacaagtt ggggtgggcg aagaactcca 2340
   gcatgagatc cccgcgctgg aggatcatcc agccggcgtc ccggaaaacg attccgaagc 2400
   ccaacctttc atagaaggcg gcggtggaat cgaaatctcg tgatggcagg ttgggcgtcg 2460
   cttggtcggt catttcgaac cccagagtcc cgctcagaag aactcgtcaa gaaggcgata 2520
   gaaggcgatg cgctgcgaat cgggagcggc gataccgtaa agcacgagga agcggtcagc 2580
   ccattcgccg ccaagctctt cagcaatatc acgggtagcc aacgctatgt cctgatagcg 2640
   gtccgccaca cccagccggc cacagtcgat gaatccagaa aagcggccat tttccaccat 2700
   gatattcggc aagcaggcat cgccatgggt cacgacgaga tcctcgccgt cgggcatgcg 2760
   cgccttgagc ctggcgaaca gttcggctgg cgcgagcccc tgatgctctt cgtccagatc 2820
   atcctgatcg acaagaccgg cttccatccg agtacgtgct cgctcgatgc gatgtttcgc 2880
   ttggtggtcg aatgggcagg tagccggatc aagcgtatgc agccgccgca ttgcatcagc 2940
   catgatggat actttctcgg caggagcaag gtgagatgac aggagatcct gccccggcac 3000
   ttcgcccaat agcagccagt cccttcccgc ttcagtgaca acgtcgagca cagctgcgca 3060
   aggaacgccc gtcgtggcca gccacgatag ccgcgctgcc tcgtcctgca gttcattcag 3120
   ggcaccggac aggtcggtct tgacaaaaag aaccgggcgc ccctgcgctg acagccggaa 3180
   cacggcggca tcagagcagc cgattgtctg ttgtgcccag tcatagccga atagcctctc 3240
   cacccaagcg gccggagaac ctgcgtgcaa tccatcttgt tcaatcatgc gaaacgatcc 3300
   tcatcctgtc tcttgatcag atccgaaaat ggatatacaa gctcccggga gctttttgca 3360
   aaagcctagg cctccaaaaa agcctcctca ctacttctgg aatagctcag aggcagaggc 3420
   ggcctcggcc tctgcataaa taaaaaaaat tagtcagcca tggggcggag aatgggcgga 3480
   actgggcgga gttaggggcg ggatgggcgg agttaggggc gggactatgg ttgctgacta 3540
   attgagatgc atgctttgca tacttctgcc tgctggggag cctggggact ttccacacct 3600
   ggttgctgac taattgagat gcatgctttg catacttctg cctgctgggg agcctgggga 3660
   ctttccacac cctaaataac ttcgtataat gtatactata cgaacggtag cgttaacaag 3720
   cttctgtgga atgtgtgtca gttagggtgt ggaaagtccc caggctcccc aggcaggcag 3780
   aagtatgcaa agcatgcatc tcaattagtc agcaaccagg tgtggaaagt ccccaggctc 3840
   cccagcaggc agaagtatgc aaagcatgca tctcaattag tcagcaacca tagtcccgcc 3900
   cctaactccg cccatcccgc ccctaactcc gcccagttcc gcccattctc cgccccatgg 3960
   ctgactaatt ttttttattt atgcagaggc cgaggccgcc tctgcctctg agctattcca 4020
   gaagtagtga ggaggctttt ttggaggcct aggcttttgc aaaaagctat aacttcgtat 4080
   atggtattat atacgaagtt atcccacccc aacttgttta ttgcagctta taatggttac 4140
   aaataaagca atagcatcac aaatttcaca aataaagcat ttttttcact gcattctagt 4200
   tgtggtttgt ccaaactcat caatgtatct tatcatgtct gtataccgtc gacctctagc 4260
   tagagcttgg cgtaatcatg gtcatagctg tttcctgtgt gcccacccca acttgtttat 4320
   tgcagcttat aatggttaca aataaagcaa tagcatcaca aatttcacaa ataaagcatt 4380
   tttttcactg cattctagtt gtggtttgtc caaactcatc aatgtatctt atcatgtctg 4440
   gagctcgcta gcgtttaaac ggtaccataa cttcgtatat tctatcttat acgaacggta 4500
   ctcgagatgc gtcgacattt aaatgttggc gcagaaggga ttcatatcaa ttaattccct 4560
   gtcctcaatt aatgcataat tgggagtgcg atggaatcat tcaataagta atgaggactt 4620
   ggtgtcaaaa tgccaattaa gtaattaatg acacccaatt aatacacaat ggggattaca 4680
   gtggaataac tcagtgaagt tcatatctaa attgcaattg aataattaat tacagctaat 4740
   taatggatgg gagactggca gtggtataac ttaatgaagg aacacagtga attcctgtct 4800
   gaattgaaat taattgattt attagggcta attaatgaat ggtggggtta aaaatagcat 4860
   tactcagcga aggaacatga gttcatggct aaattgagat tgaattatta attacaacta 4920
   attgattaat gactgatggg agtaagaatg gaataactca gtgaaggaac acaatggatt 4980
   catggcctaa ttgaaattaa atggttaatt agagctaatt aatgaatgat ggggggtggc 5040
   aatggaatga tacaagaaag aaacacaatg ggttcgtgtc tacattgaaa ttaaataatt 5100
   aattacagct aattaataaa cggcgaggtt gacaatggta taactgtgaa gagacagaat 5160
   gagttcgtga ctaaattggt attaaatgat taattagagc taattaacag gtggtggggc 5220
   tgagaatggc ataactcaat gggggatgat gagctgatgg cttaattgga attaataatt 5280
   aatcacaacc aattaacagg tgctgttaat ggggttacag gggcatgact tcaggaagaa 5340
   gcacaaagtg gccattaaat aattaattgc acccaattaa ttcctgatgg ggattactgc 5400
   agaggaactc agcgaaggat tcaagcagaa atgacacagt taggttgctc aattgcgtta 5460
   attaattaaa atcgctaatt aggtgagctt cattaaggcc tgatggggtt catcccaacg 5520
   ctgggatcaa agaactcagc gcagagtgga taacgataat tagaagggga caaattaatg 5580
   aggaaacacg actttgggcc tagacggcca ttaaatcatt aatcaacccg acgggcgccc 5640
   aattaatcat aaagttatta agcaattaag tcgaggctga cgagaaaccg caacgagagc 5700
   cctccacgct cctttgagtt ggcgcaaaca acaaacaacg cgggctcgtt aagcagcgac 5760
   gacgccgagc gtaacgaagc aatccccgcg cccgacaatt aacgcgctaa cgaagccgga 5820
   gccctcctta tgcaaattag cccctccaga gggccataaa agcgccggct ctccgacggc 5880
   gcgggaggag caccagtcgg ctccgcaacc atgagcccac tcgtctcctc cctcctgctc 5940
   ctggccgccc tgccaggtga gggcgctgtg gggctctatg gggctctatg gggtctcagc 6000
   ggggctctgc gggctcaatg ggggccaaag ggggggtctg cgggctctat gggggggtca 6060
   acggggggtc tcacgggggg ccggctccgc gaggccgtgt ggcggcggct ccgtcagcgc 6120
   tctctgggcg cgcctccttc cccacagggc tgatggcgga ggtgcagctg ttggagtctg 6180
   ggggaggctt ggtacagcct ggggggtccc tgagactctc ctgtgcagcc tctggattca 6240
   cctttagcag ctatgccatg agctgggtcc gccaggctcc agggaagggg ctggagtggg 6300
   tctcagctat tagtggtagt ggtggtagca catactacgc agactccgtg aagggccggt 6360
   tcaccatctc cagagacaat tccaagaaca cgctgtatct gcaaatgaac agcctgagag 6420
   ccgaggacac ggccgtatat tactgtgcga aaggatatag tggctacgat tactactttg 6480
   actactgggg ccagggcacc ctggtcaccg tctcctcagg tgagtcttca tatgaacccc 6540
   ccccaaaatt gccgcggcga ttttggggca aaatcgggcg attttatgga tccccgctac 6600
   cgttcgtata gcatacatta tacgaagtta tcagacatga taagatacat tgatgagttt 6660
   ggacaaacca caactagaat gcagtgaaaa aaatgcttta tttgtgaaat ttgtgatgct 6720
   attgctttat ttgtaaccat tataagctgc aataaacaag ttggggtggg cgaagaactc 6780
   cagcatgaga tccccgcgct ggaggatcat ccagccggcg tcccggaaaa cgattccgaa 6840
   gcccaacctt tcatagaagg cggcggtgga atcgaaatct cgtagcacgt gtcagtcctg 6900
   ctcctcggcc acgaagtgct tagccctccc acacataacc agagggcagc aattcacgaa 6960
   tcccaactgc cgtcggctgt ccatcactgt ccttcactat ggctttgatc ccaggatgca 7020
   gatcgagaag cacctgtcgg caccgtccgc aggggctcaa gatgcccctg ttctcatttc 7080
   cgatcgcgac gatacaagtc aggttgccag ctgccgcagc agcagcagtg cccagcacca 7140
   cgagttctgc acaaggtccc ccagtaaaat gatatacatt gacaccagtg aagatgcggc 7200
   cgtcgctaga gagagctgcg ctggcgacgc tgtagtcttc agagatgggg atgctgttga 7260
   ttgtagccgt tgctctttca atgagggtgg attcttcttg agacaaaggc ttggccatgg 7320
   tttagttcct caccttgtcg tattatacta tgccgatata ctatgccgat gattaattgt 7380
   caacacgtgc tgatcagatc cgaaaatgga tatacaagct cccgggagct ttttgcaaaa 7440
   gcctaggcct ccaaaaaagc ctcctcacta cttctggaat agctcagagg cagaggcggc 7500
   ctcggcctct gcataaataa aaaaaattag tcagccatgg ggcggagaat gggcggaact 7560
   gggcggagtt aggggcggga tgggcggagt taggggcggg actatggttg ctgactaatt 7620
   gagatgcatg ctttgcatac ttctgcctgc tggggagcct ggggactttc cacacctggt 7680
   tgctgactaa ttgagatgca tgctttgcat acttctgcct gctggggagc ctggggactt 7740
   tccacaccct aactgacaca cattccacag ataacttcgt atagcataca ttatacgaac 7800
   ggtagcctgg gtcagtcgaa gggggcggtc ggtccatcat ttgcggccgg gtgatttttg 7860
   gggccgaaaa gtgggaattt ggggcccaat ttggggccca atttggggcc caatttgggt 7920
   tttcgagggg ggattttttt agggggagat tttgggtccc cggaaaatgg tgattttggg 7980
   tcaaaaatgg ggattttggg gtcgttttga ggtggggttt tttggggtag aaatggcggt 8040
   ctgaggggaa aatgtcgttt tggggccatt ttgggccatt tgaggggaaa tttgggtcaa 8100
   aaaatgacga ttttgggtcg ttttagggag gaaaatgaat ttagggcgat ttgagccaaa 8160
   tttgggtcaa aaatggcgat tttgagtcaa aaaatgatta ttttgggtca ttttagggag 8220
   gaaaatgaat ttagggagat tggacggcaa atttgggtca aaaaatggtg attttgggtc 8280
   aaaaaatgat tattttgggt cattttaggg agaaaaatga atttagggag atttgagggc 8340
   aaatttgggt caaaaaatgg tgattttggg tcaaaaaatg attattttgg gtcattttag 8400
   ggagaaaaat gaatttaggg agatttgagg gcaaatttgg gtcgaaaaat ggcgattttg 8460
   ggtgaaaaat ggacaatttt gggtcatttt agggttaaaa atgaatttag ggagattgga 8520
   cggcaaattt gggtcaaaaa atggtgattt ggggtcaaaa aataattatt ttgggtcatt 8580
   ttagggataa aaaatgaatt tagggagatt tgagggcaaa tttgggtcga aaaatggtga 8640
   tttggggtga aaaatggaca attttgggtc attttaggga taaaaaatga atttagggcg 8700
   atttgagcca aatttgggtc aaaaatggcg attttgagtc aaaaaatgat tattttgggt 8760
   cattttaggg ttaaaaatga atttagggag attggacggc aaatttgggt caaaaaatgg 8820
   tgattttcgg tcaaaaaatg attattttgg gtcattttag ggagaaaaat gaatttaggg 8880
   agattggacg gcaaatttgg gtcgaaaaat ggtgattttg ggtcaaaaat ggacaatttg 8940
   gggtcatttt agggataaaa atgaatttag ggagatttga gagcaaattt gggtctaaaa 9000
   atgtcaattt ggggtcaaaa atggtgattt tgggtcattt tagggatata aatgaatttt 9060
   gggagatttg agggcaaatt tgggtcgaaa aatggtgatt tggggtcaat ttagggagga 9120
   aaatgaattt aaggtgattt gacggcaaat ttgggttgaa aaatgacaat ttggggtcat 9180
   tttaaagata aaatgaattt agggcgattt gagggcaaat ttgggtcgaa aaatggtgat 9240
   ttgggtcaaa aaatgacaat tttgggtcat tttagggata taaatggact tagagcgatt 9300
   tgagggcaaa tttggttgaa aaaatggaca atttgggtca ttttagggat ataaatgaat 9360
   ttagggagat ttgatggcaa atttgggtcg aaaaatggtg atttgggtca aaaatggtga 9420
   ttttggttga aaaacagtca tttggggtca ttttagggat taaaatgaat ttcgggagat 9480
   ttgagggcaa atttgggtca aaaaatggtg attttgggtc aattagtgat aaaaaaatga 9540
   atttagggtg atttgagggc aaatttgggt caaaaaatga caatttgggg tttttttagg 9600
   gaggagaatg aatttaggga gttttgaggg caaatttggg tcaaaaaata gtgatttggg 9660
   tcaaaagtgg tgattttggt tgaaaaacag tcattttggg tcattttagg gagaaaaatg 9720
   aatttcggga gatttgaggg caaatttggg tcaaaaaatg acaattttgg gtcactttac 9780
   gaattaaaat gaatttaggg agatttgagg gcaaatttgg gtcaaaatat ggtgattttg 9840
   ggtcaattta gggaggaaaa tgaatttagg gcgatttgaa ggcaaatttg ggtcgaaaat 9900
   ggtgattttg ggtcaaaaaa tgatgatttt gggtcgtttt agggagaaaa tgaatttagg 9960
   gagatttgag ggcaaatttg ggtcaaaaaa tggtgatttt gggtcattgt agggataaaa 10020
   atgaatttgg ggcgatttga gggcaaattt gggtggaaaa tggacaattt tgggtcattt 10080
   tagggagaaa aatgaattta gggagatttg agggcaaatt tgggtcaaaa aatggtgatt 10140
   tggggtcatt ttcagaagaa aaaatgatta ttttccccac taaaaatgta tattttgggg 10200
   ccaaatggtg aaaaatggtg atttttaatc aaacgtcccc aaaattgggg aaatttcatc 10260
   gatttgaccc aaaattgagt tttttttccc tgttaaaaat gtacattttg gggtcaatcg 10320
   ctgaaatgtt cccacttttc acttctttgc cccaaatttt tgctgtccgg tgagaaatta 10380
   attaattaat gatcattaat tgatcgacaa ttaatggtta ttaattaatt aataggtcct 10440
   cttttggtga ctccttcgct tttggggcca aaagtccata aattggcccc aaaaaattaa 10500
   cgctgagtaa ttggattcca aagtattaat gataaacatt aaaagtgttt aattaataat 10560
   gatattaaac ataatttcgt ttttattatc gatttatcaa caacgatgaa cgataatact 10620
   ttacaacaat cgctaataat taattaatta tttaattaat taatttctaa taattacttc 10680
   gcattatcgg acacgagatg ttgtaattat taataattta attcctaata attagaagat 10740
   tcgttgaaaa tggtacttat ctttacaaat aatcacttct aataataatg attaataata 10800
   gttaataaca acaatgacga taatgacgat aatattaata atatgtgata tatttaatat 10860
   aaaattcgta ttaatatatt atatctacaa aatatgatat aaaatataat attttattta 10920
   tatataacac aatttattat tattatcatt attatcatta ttaatatcat cattattaac 10980
   gttatcgaaa tacttattta gaaataataa aaacggattt aatgatggca acaaaaatat 11040
   tttattaatg ttaaaaaaaa ataattaata atttccaaag attcgaattc ggggcaacga 11100
   acggcactcg ataattttta attaattaat agtttgaatt aatcggtact ttttaatcct 11160
   ccattttgcc cgaaatcgcc gttttttgcc ccaaattccc caccgcggcg ttaaaaacat 11220
   aaagaaatta agcatcaaaa gtgccctttt ttggggttgt tttgacccaa aaaaaaaatg 11280
   gccgaattgg gggcggccgt tttacggttg ggttcatttt gggttcaaaa cagccaaaaa 11340
   tgggaacttt gggtttcgaa aacaacaaca acaaaaaaac gggtttattt tgggctcatt 11400
   ttgggtgttt ttgggtcagg aggagaaaaa ataggaagtt tgagagcgaa acaacggccg 11460
   cttttggggg gaaaacggcc ctttttggtc aacggcgggg gaaaaaaaaa agcggagttt 11520
   ttggggtgaa aaagagcggt tttgggtaaa tttgggtttt ggggtaaaag tggaggattt 11580
   tgggcaatgg gagttaaaaa atgggtgttt ttatgggggt tcggtgcagt ttttcctgtt 11640
   tgatgggggg tttattaatc cggggggggg gattaatgag aattaataat gttaatagaa 11700
   atatctggga aattaatagc aattattaat tgttaatagt tattaatagt tctatatatc 11760
   tcacatctac gatacaatat aatatcgtta caatcatata gtcgatatat tacatataat 11820
   tatcagtaat aataataagt aacaataatt agcagtaatt aataataata attaatagta 11880
   ttcgttaata agattattga taataattaa gtagtagcga ttaatagaga tgggatttcg 11940
   tgagaaatgg accaaatttg ggccgttttg acccaaattt ttggtggttt tttttccgat 12000
   tctttgtgaa tttcgggtcg gattcatcag caattaatta cggttattag gggctattag 12060
   aggcttttaa ttgggattat tagagacttt taagcggatt tggggacttt taagtggatt 12120
   ttatgattct taagtggatt ttgggtggat tttaccgctt ttgcggccgc aggcgtaatc 12180
   atggtcatag ctgtttcctg tgtgaaattg ttatccgctc acaattccac acaacatacg 12240
   agccggaagc ataaagtgta aagcctgggg tgcctaatga gtgagctaac tcacattaat 12300
   tgcgttgcgc tcactgcccg ctttccagtc gggaaacctg tcgtgccagc tgcattaatg 12360
   aatcggccaa cgcgcgggga gaggcggttt gcgtattggg cgctcttccg cttcctcgct 12420
   cactgactcg ctgcgctcgg tcgttcggct gcggcgagcg gtatcagctc actcaaaggc 12480
   ggtaatacgg ttatccacag aatcagggga taacgcagga aagaacatgt gagcaaaagg 12540
   ccagcaaaag gccaggaacc gtaaaaaggc cgcgttgctg gcgtttttcc ataggctccg 12600
   cccccctgac gagcatcaca aaaatcgacg ctcaagtcag aggtggcgaa acccgacagg 12660
   actataaaga taccaggcgt ttccccctgg aagctccctc gtgcgctctc ctgttccgac 12720
   cctgccgctt accggatacc tgtccgcctt tctcccttcg ggaagcgtgg cgctttctca 12780
   tagctcacgc tgtaggtatc tcagttcggt gtaggtcgtt cgctccaagc tgggctgtgt 12840
   gcacgaaccc cccgttcagc ccgaccgctg cgccttatcc ggtaactatc gtcttgagtc 12900
   caacccggta agacacgact tatcgccact ggcagcagcc actggtaaca ggattagcag 12960
   agcgaggtat gtaggcggtg ctacagagtt cttgaagtgg tggcctaact acggctacac 13020
   tagaagaaca gtatttggta tctgcgctct gctgaagcca gttaccttcg gaaaaagagt 13080
   tggtagctct tgatccggca aacaaaccac cgctggtagc ggtggttttt ttgtttgcaa 13140
   gcagcagatt acgcgcagaa aaaaaggatc tcaagaagat cctttgatct tttctacggg 13200
   gtctgacgct cagtggaacg aaaactcacg ttaagggatt ttggtcatga gattatcaaa 13260
   aaggatcttc acctagatcc ttttaaatta aaaatgaagt tttaaatcaa tctaaagtat 13320
   atatgagtaa acttggtctg acagttacca atgcttaatc agtgaggcac ctatctcagc 13380
   gatctgtcta tttcgttcat ccatagttgc ctgactcccc gtcgtgtaga taactacgat 13440
   acgggagggc ttaccatctg gccccagtgc tgcaatgata ccgcgagacc cacgctcacc 13500
   ggctccagat ttatcagcaa taaaccagcc agccggaagg gccgagcgca gaagtggtcc 13560
   tgcaacttta tccgcctcca tccagtctat taattgttgc cgggaagcta gagtaagtag 13620
   ttcgccagtt aatagtttgc gcaacgttgt tgccattgct acaggcatcg tggtgtcacg 13680
   ctcgtcgttt ggtatggctt cattcagctc cggttcccaa cgatcaaggc gagttacatg 13740
   atcccccatg ttgtgcaaaa aagcggttag ctccttcggt cctccgatcg ttgtcagaag 13800
   taagttggcc gcagtgttat cactcatggt tatggcagca ctgcataatt ctcttactgt 13860
   catgccatcc gtaagatgct tttctgtgac tggtgagtac tcaaccaagt cattctgaga 13920
   atagtgtatg cggcgaccga gttgctcttg cccggcgtca atacgggata ataccgcgcc 13980
   acatagcaga actttaaaag tgctcatcat tggaaaacgt tcttcggggc gaaaactctc 14040
   aaggatctta ccgctgttga gatccagttc gatgtaaccc actcgtgcac ccaactgatc 14100
   ttcagcatct tttactttca ccagcgtttc tgggtgagca aaaacaggaa ggcaaaatgc 14160
   cgcaaaaaag ggaataaggg cgacacggaa atgttgaata ctcatactct tcctttttca 14220
   atattattga agcatttatc agggttattg tctcatgagc ggatacatat ttgaatgtat 14280
   ttagaaaaat aaacaaatag gggttccgcg cacatttccc cgaaaagtgc cacctgacgt 14340
   ctaagaaacc attattatca tgacattaac ctataaaaat aggcgtatca cgaggccctt 14400
   tcgtc 14405
<210> 110
   <211> 366
   <212> DNA
   <213> Artificial Sequence
<220>
   <223> Synthetic DNA
<400> 110
   gaggtgcagc tgttggagtc tgggggaggc ttggtacagc ctggggggtc cctgagactc      60
   tcctgtgcag cctctggatt cacctttagc agctatgcta tgcattgggt ccgccaggct     120
   ccagggaagg ggctggagtg ggtctcatgg atcaacgctg gcaatggtaa cacaaaatat     180
   tcacagaagt tccagggccg gttcaccatc tccagagaca attccaagaa cacgctgtat     240
   ctgcaaatga acagcctgag agccgaggac acggccgtat attactgtgc gaaagcgtct     300
   agtagtggtt attactacgt ccactttgac tactggggcc agggcaccct ggtcaccgtc     360
   tcctca                                                                366
<210> 111
   <211> 360
   <212> DNA
   <213> Artificial Sequence
<220>
   <223> Synthetic DNA
<400> 111
   gaggtgcagc tgttggagtc tgggggaggc ttggtacagc ctggggggtc cctgagactc      60
   tcctgtgcag cctctggatt cacctttagc agctatggta tcagctgggt ccgccaggct     120
   ccagggaagg ggctggagtg ggtctcatgg atcagcgctt acaatggtaa cacaaactat     180
   gcacagaagc tccagggccg gttcaccatc tccagagaca attccaagaa cacgctgtat     240
   ctgcaaatga acagcctgag agccgaggac acggccgtat attactgtgc gaaagattta     300
   aggttatcct attactactt tgactactgg ggccagggca ccctggtcac cgtctcctca     360
<210> 112
   <211> 375
   <212> DNA
   <213> Artificial Sequence
<220>
   <223> Synthetic DNA
<400> 112
   gaggtgcagc tgttggagtc tgggggaggc ttggtacagc ctggggggtc cctgagactc 60
   tcctgtgcag cctctggatt cacctttagc ggctttgcca tgaactgggt ccgccaggct 120
   ccagggaagg ggctggagtg ggtctcaact attagtaata gtgctggtag cacatattac 180
   gcagactccg tgaagggccg gttcaccatc tccagagaca attccaagaa cacgctgtat 240
   ctgcaaatga acagcctgag agccgaggac acggccgtat attactgtgc gaaatatggg 300
   gaggatattg tcgttgtgcc agctcgctac ggtgtggacg tctggggcca gggcaccctg 360
   gtcaccgtct cctca 375
<210> 113
   <211> 378
   <212> DNA
   <213> Artificial Sequence
<220>
   <223> Synthetic DNA
<400> 113
   gaggtgcagc tgttggagtc tgggggaggc ttggtacagc ctggggggtc cctgagactc 60
   tcctgtgcag cctctggatt cacctttagc agctctgctg tgcagtgggt ccgccaggct 120
   ccagggaagg ggctggagtg ggtctcatgg atcgtcgttg gcagtggtaa cacaaactac 180
   gcacagaagt tccaggaacg gttcaccatc tccagagaca attccaagaa cacgctgtat 240
   ctgcaaatga acagcctgag agccgaggac acggccgtat attactgtgc gaaagacgtc 300
   cttgtagtac cagctgcccc ctactactac tacggtatgg acgtctgggg ccagggcacc 360
   ctggtcaccg tctcctca 378
<210> 114
   <211> 378
   <212> DNA
   <213> Artificial Sequence
<220>
   <223> Synthetic DNA
<400> 114
   gaggtgcagc tgttggagtc tgggggaggc ttggtacagc ctggggggtc cctgagactc 60
   tcctgtgcag cctctggatt cacctttagc actagtggag tgggtgtggg ctgggtccgc 120
   caggctccag ggaaggggct ggagtgggtc tcactcattt attggaatga tgataagcgc 180
   tacagcccat ctctgaagag ccggttcacc atctccagag acaattccaa gaacacgctg 240
   tatctgcaaa tgaacagcct gagagccgag gacacggccg tatattactg tgcgaaagac 300
   gatggggcgt atgggccgta tagcagcgcc tggacctttg actactgggg ccagggcacc 360
   ctggtcaccg tctcctca 378
<210> 115
   <211> 369
   <212> DNA
   <213> Artificial Sequence
<220>
   <223> Synthetic DNA
<400> 115
   gaggtgcagc tgttggagtc tgggggaggc ttggtacagc ctggggggtc cctgagactc 60
   tcctgtgcag cctctggatt cacctttagc actagtggaa tgcgtgtgag ctgggtccgc 120
   caggctccag ggaaggggct ggagtgggtc tcacgcattg attgggatga tgataaattc 180
   tacagcacat ctctgaagac ccggttcacc atctccagag acaattccaa gaacacgctg 240
   tatctgcaaa tgaacagcct gagagccgag gacacggccg tatattactg tgcgaaagat 300
   gacggtggca ccccgcccgg ggaattcttt gactactggg gccagggcac cctggtcacc 360
   gtctcctca 369
<210> 116
   <211> 360
   <212> DNA
   <213> Artificial Sequence
<220>
   <223> Synthetic DNA
<400> 116
   gaggtgcagc tgttggagtc tgggggaggc ttggtacagc ctggggggtc cctgagactc 60
   tcctgtgcag cctctggatt cacctttagc gattatgcca tgcactgggt ccgccaggct 120
   ccagggaagg ggctggagtg ggtctcaggt attagttgga atagtggtag cataggctat 180
   gcggactctg tgaagggccg gttcaccatc tccagagaca attccaagaa cacgctgtat 240
   ctgcaaatga acagcctgag agccgaggac acggccgtat attactgtgc gaaaatagtg 300
   ggagctactt acaactggtt cgacccctgg ggccagggca ccctggtcac cgtctcctca 360
<210> 117
   <211> 363
   <212> DNA
   <213> Artificial Sequence
<220>
   <223> Synthetic DNA
<400> 117
   gaggtgcagc tgttggagtc tgggggaggc ttggtacagc ctggggggtc cctgagactc 60
   tcctgtgcag cctctggatt cacctttagc aacgcctgga tgagctgggt ccgccaggct 120
   ccagggaagg ggctggagtg ggtctcacgt attaaaagca aaactgatgg tgggacaaca 180
   gactacgctg cacccgtgaa aggccggttc accatctcca gagacaattc caagaacacg 240
   ctgtatctgc aaatgaacag cctgagagcc gaggacacgg ccgtatatta ctgtgcgaaa 300
   gaaaggggta gcagctcgtc ctttgactac tggggccagg gcaccctggt caccgtctcc 360
   tca 363
<210> 118
   <211> 366
   <212> DNA
   <213> Artificial Sequence
<220>
   <223> Synthetic DNA
<400> 118
   gaggtgcagc tgttggagtc tgggggaggc ttggtacagc ctggggggtc cctgagactc 60
   tcctgtgcag cctctggatt cacctttagc agctatggca tgcactgggt ccgccaggct 120
   ccagggaagg ggctggagtg ggtctcagtt atatggtatg atggaagtaa taaatactat 180
   gcagactccg tgaagggccg gttcaccatc tccagagaca attccaagaa cacgctgtat 240
   ctgcaaatga acagcctgag agccgaggac acggccgtat attactgtgc gaaagatctt 300
   gtcttcgggc cagctgctac tccttttgac tactggggcc agggcaccct ggtcaccgtc 360
   tcctca 366
<210> 119
   <211> 366
   <212> DNA
   <213> Artificial Sequence
<220>
   <223> Synthetic DNA
<400> 119
   gaggtgcagc tgttggagtc tgggggaggc ttggtacagc ctggggggtc cctgagactc 60
   tcctgtgcag cctctggatt cacctttagc agcaactaca tgagctgggt ccgccaggct 120
   ccagggaagg ggctggagtg ggtctcagtt atttatagcg gtggtagcac atactacgca 180
   gactccgtga agggccggtt caccatctcc agagacaatt ccaagaacac gctgtatctg 240
   caaatgaaca gcctgagagc cgaggacacg gccgtatatt actgtgcgaa agcgaagttc 300
   tcctatggtt cagggagtta ttactttgac tactggggcc agggcaccct ggtcaccgtc 360
   tcctca 366
<210> 120
   <211> 372
   <212> DNA
   <213> Artificial Sequence
<220>
   <223> Synthetic DNA
<400> 120
   gaggtgcagc tgttggagtc tgggggaggc ttggtacagc ctggggggtc cctgagactc 60
   tcctgtgcag cctctggatt cacctttagc agctactgga tgcactgggt ccgccaggct 120
   ccagggaagg ggctggagtg ggtctcacgt attaatagtg atgggagtag cacaagctac 180
   gcggactccg tgaagggccg gttcaccatc tccagagaca attccaagaa cacgctgtat 240
   ctgcaaatga acagcctgag agccgaggac acggccgtat attactgtgc gaaaggatat 300
   tgtagtagta ccagctgcta tgctgatgct tttgatatct ggggccaggg caccctggtc 360
   accgtctcct ca 372
<210> 121
   <211> 354
   <212> DNA
   <213> Artificial Sequence
<220>
   <223> Synthetic DNA
<400> 121
   gaggtgcagc tgttggagtc tgggggaggc ttggtacagc ctggggggtc cctgagactc 60
   tcctgtgcag cctctggatt cacctttagc agtagtaact ggtggggctg ggtccgccag 120
   gctccaggga aggggctgga gtgggtctca tacatctatt atagtgggag cacctactac 180
   aacccgtccc tcaagagtcg gttcaccatc tccagagaca attccaagaa cacgctgtat 240
   ctgcaaatga acagcctgag agccgaggac acggccgtat attactgtgc gaaagatcga 300
   agtagagggg gttttgacta ctggggccag ggcaccctgg tcaccgtctc ctca 354
<210> 122
   <211> 363
   <212> DNA
   <213> Artificial Sequence
<220>
   <223> Synthetic DNA
<400> 122
   gaggtgcagc tgttggagtc tgggggaggc ttggtacagc ctggggggtc cctgagactc 60
   tcctgtgcag cctctggatt cacctttagc ggttactact ggagctgggt ccgccaggct 120
   ccagggaagg ggctggagtg ggtctcagaa atcaatcata gtggaagcac caactacaac 180
   ccgtccctca agagtcggtt caccatctcc agagacaatt ccaagaacac gctgtatctg 240
   caaatgaaca gcctgagagc cgaggacacg gccgtatatt actgtgcgaa agcccgtacc 300
   agcgcgggcc cgggtggtgc ttttgatttc tggggccagg gcaccctggt caccgtctcc 360
   tca 363
<210> 123
   <211> 384
   <212> DNA
   <213> Artificial Sequence
<220>
   <223> Synthetic DNA
<400> 123
   gaggtgcagc tgttggagtc tgggggaggc ttggtacagc ctggggggtc cctgagactc 60
   tcctgtgcag cctctggatt cacctttagc agctactgga tcggctgggt ccgccaggct 120
   ccagggaagg ggctggagtg ggtctcaatc atctatcctg gtgactctga taccagatac 180
   agcccgtcct tccaaggccg gttcaccatc tccagagaca attccaagaa cacgctgtat 240
   ctgcaaatga acagcctgag agccgaggac acggccgtat attactgtgc gaaaggggtt 300
   tgtagtacca gctcgtacgc cttttactac tactactacg gtatggacgt ctggggccag 360
   ggcaccctgg tcaccgtctc ctca 384
<210> 124
   <211> 363
   <212> DNA
   <213> Artificial Sequence
<220>
   <223> Synthetic DNA
<400> 124
   gaggtgcagc tgttggagtc tgggggaggc ttggtacagc ctggggggtc cctgagactc 60
   tcctgtgcag cctctggatt cacctttagc agctatgcta tgaattgggt ccgccaggct 120
   ccagggaagg ggctggagtg ggtctcatgg atcaacacca acactgggaa cccaacgtat 180
   gcccagggct tcacaggacg gttcaccatc tccagagaca attccaagaa cacgctgtat 240
   ctgcaaatga acagcctgag agccgaggac acggccgtat attactgtgc gaaagactac 300
   ggtggtaact cctactggta cttcgatctc tggggccagg gcaccctggt caccgtctcc 360
   tca 363
<210> 125
   <211> 14861
   <212> DNA
   <213> Artificial Sequence
<220>
   <223> Synthetic DNA
<400> 125
<210> 126
   <211> 22891
   <212> DNA
   <213> Artificial Sequence
<220>
   <223> Synthetic DNA
<400> 126
<210> 127
   <211> 333
   <212> DNA
   <213> Artificial Sequence
<220>
   <223> Synthetic DNA
<400> 127
   cagtctgccc tgactcagcc tgcctccgtg tctgggtctc ctggacagtc gatcaccatc       60
   tcctgctctg gaagcagctc caacatcgga aataatgctg taaactggta ccaacagcac      120
   ccaggcaaag cccccaaact catgatttat tatgatgatc tgctgccctc aggggtttct      180
   aatcgcttct ctggctccaa gtctggcaac acggcctccc tgaccatctc tgggctccag      240
   gctgaggacg aggctgatta ttactgcgga acatgggatg gcagcctgaa tggtgggagg      300
   ctgttcggcg gagggaccaa gctgaccgtc cta                                   333
<210> 128
   <211> 330
   <212> DNA
   <213> Artificial Sequence
<220>
   <223> Synthetic DNA
<400> 128
   cagtctgccc tgactcagcc tgcctccgtg tctgggtctc ctggacagtc gatcaccatc       60
   tcctgctctg gaagcagctc caacatcgga agtaatactg taaactggta ccaacagcac      120
   ccaggcaaag cccccaaact catgatttat agtaataatc agcggccctc aggggtttct      180
   aatcgcttct ctggctccaa gtctggcaac acggcctccc tgaccatctc tgggctccag      240
   gctgaggacg aggctgatta ttactgcgca gcatgggatg acagcctgag tggtgtggtt      300
   ttcggcggag ggaccaagct gaccgtccta                                       330
<210> 129
   <211> 330
   <212> DNA
   <213> Artificial Sequence
<220>
   <223> Synthetic DNA
<400> 129
   cagtctgccc tgactcagcc tgcctccgtg tctgggtctc ctggacagtc gatcaccatc 60
   tcctgctctg gaagcagctc cgacatgggg aattatgcgg tatcctggta ccaacagcac 120
   ccaggcaaag cccccaaact catgatttat gatgtcagta agcggccctc aggggtttct 180
   aatcgcttct ctggctccaa gtctggcaac acggcctccc tgaccatctc tgggctccag 240
   gctgaggacg aggctgatta ttactgcagc tcatatgaag acagcaacaa tttgggggta 300
   ttcggcggag ggaccaagct gaccgtccta 330
<210> 130
   <211> 342
   <212> DNA
   <213> Artificial Sequence
<220>
   <223> Synthetic DNA
<400> 130
   cagtctgccc tgactcagcc tgcctccgtg tctgggtctc ctggacagtc gatcaccatc 60
   tcctgcactg gaaccagcag tgacgttggt agttataacc gtgtctcctg gtaccaacag 120
   cacccaggca aagcccccaa actcatgatt tatgaggtca gtaatcggcc ctcaggggtt 180
   tctaatcgct tctctggctc caagtctggc aacacggcct ccctgaccat ctctgggctc 240
   caggctgagg acgaggctga ttattactgc gcagcatggg atggcagccc ggccccgagt 300
   gacttttatg tcttcggcgg agggaccaag ctgaccgtcc ta 342
<210> 131
   <211> 348
   <212> DNA
   <213> Artificial Sequence
<220>
   <223> Synthetic DNA
<400> 131
   cagtctgccc tgactcagcc tgcctccgtg tctgggtctc ctggacagtc gatcaccatc 60
   tcctgctctg gagataaatt gggggataaa tatgcttgct ggtaccaaca gcacccaggc 120
   aaagccccca aactcatgat ttatcaagat agcaagcggc cctcaggggt ttctaatcgc 180
   ttctctggct ccaagtctgg caacacggcc tccctgacca tctctgggct ccaggctgag 240
   gacgaggctg attattactg ccagtccttt gacaacagcc tgtgccagtc ctttgacaac 300
   agcctgaatt atgtgctttt cggcggaggg accaagctga ccgtccta 348
<210> 132
   <211> 330
   <212> DNA
   <213> Artificial Sequence
<220>
   <223> Synthetic DNA
<400> 132
   cagtctgccc tgactcagcc tgcctccgtg tctgggtctc ctggacagtc gatcaccatc      60
   tcctgctctg gagatgcatt gccaaaaaaa tatgcttatt ggtaccaaca gcacccaggc     120
   aaagccccca aactcatgat ttatgaggac agcaaacgac cctccggggt ttctaatcgc     180
   ttctctggct ccaagtctgg caacacggcc tccctgacca tctctgggct ccaggctgag     240
   gacgaggctg attattactg cgcagcatgg gatgacagcc tgaatggtcc cttttatgtc     300
   ttcggcggag ggaccaagct gaccgtccta                                      330
<210> 133
   <211> 324
   <212> DNA
   <213> Artificial Sequence
<220>
   <223> Synthetic DNA
<400> 133
   cagtctgccc tgactcagcc tgcctccgtg tctgggtctc ctggacagtc gatcaccatc      60
   tcctgccaag gagacagcct cagaagctat tatgcaagct ggtaccaaca gcacccaggc     120
   aaagccccca aactcatgat ttatggtaaa aacaaccggc cctcaggggt ttctaatcgc     180
   ttctctggct ccaagtctgg caacacggcc tccctgacca tctctgggct ccaggctgag     240
   gacgaggctg attattactg ccagtcctat gacagcagcc tgagtggtgt ggtattcggc     300
   ggagggacca agctgaccgt ccta                                            324
<210> 134
   <211> 324
   <212> DNA
   <213> Artificial Sequence
<220>
   <223> Synthetic DNA
<400> 134
   cagtctgccc tgactcagcc tgcctccgtg tctgggtctc ctggacagtc gatcaccatc 60
   tcctgctctg gagatgtact gggggaaaat tatgctgact ggtaccaaca gcacccaggc 120
   aaagccccca aactcatgat ttatgaagat agtgagcggt accctggggt ttctaatcgc 180
   ttctctggct ccaagtctgg caacacggcc tccctgacca tctctgggct ccaggctgag 240
   gacgaggctg attattactg cggaacatgg gatagcagcc tgagtggtgt ggtattcggc 300
   ggagggacca agctgaccgt ccta 324
<210> 135
   <211> 321
   <212> DNA
   <213> Artificial Sequence
<220>
   <223> Synthetic DNA
<400> 135
   cagtctgccc tgactcagcc tgcctccgtg tctgggtctc ctggacagtc gatcaccatc 60
   tcctgctcag gagatgtact ggcaaaaaaa tatgctcggt ggtaccaaca gcacccaggc 120
   aaagccccca aactcatgat ttataggaaa aacagccaac tctcaggggt ttctaatcgc 180
   ttctctggct ccaagtctgg caacacggcc tccctgacca tctctgggct ccaggctgag 240
   gacgaggctg attattactg ctactctgcg gctgacaaca atcttgtggt attcggcgga 300
   gggaccaagc tgaccgtcct a 321
<210> 136
   <211> 336
   <212> DNA
   <213> Artificial Sequence
<220>
   <223> Synthetic DNA
<400> 136
   cagtctgccc tgactcagcc tgcctccgtg tctgggtctc ctggacagtc gatcaccatc 60
   tcctgcactc tgagcagtgg gcacagtagc tacatcatcg catggtacca acagcaccca 120
   ggcaaagccc ccaaactcat gatttatctt gaaggtagtg gaagctacaa caaggggagc 180
   ggggtttcta atcgcttctc tggctccaag tctggcaaca cggcctccct gaccatctct 240
   gggctccagg ctgaggacga ggctgattat tactgcgaga cctgggacag taacactcat 300
   gtggtattcg gcggagggac caagctgacc gtccta 336
<210> 137
   <211> 348
   <212> DNA
   <213> Artificial Sequence
<220>
   <223> Synthetic DNA
<400> 137
   cagtctgccc tgactcagcc tgcctccgtg tctgggtctc ctggacagtc gatcaccatc      60
   tcctgcacct tgcccagtga catcaatgtt ggtagctaca acatatactg gtaccaacag     120
   cacccaggca aagcccccaa actcatgatt tattactact cagactcaga taagggccag     180
   ggctctgggg tttctaatcg cttctctggc tccaagtctg gcaacacggc ctccctgacc     240
   atctctgggc tccaggctga ggacgaggct gattattact gccactcata tgcaggcggc     300
   gacactttcg atgtggtgtt cggcggaggg accaagctga ccgtccta                  348
<210> 138
   <211> 348
   <212> DNA
   <213> Artificial Sequence
<220>
   <223> Synthetic DNA
<400> 138
   cagtctgccc tgactcagcc tgcctccgtg tctgggtctc ctggacagtc gatcaccatc      60
   tcctgcatgc tgagcagtgg cttcagtgtt ggggacttct ggataaggtg gtaccaacag     120
   cacccaggca aagcccccaa actcatgatt tattaccact cagactccaa taagggccaa     180
   ggctctgggg tttctaatcg cttctctggc tccaagtctg gcaacacggc ctccctgacc     240
   atctctgggc tccaggctga ggacgaggct gattattact gcggtacatg gcacagcaac     300
   tctaagactc atgtggtatt cggcggaggg accaagctga ccgtccta                  348
<210> 139
   <211> 330
   <212> DNA
   <213> Artificial Sequence
<220>
   <223> Synthetic DNA
<400> 139
   cagtctgccc tgactcagcc tgcctccgtg tctgggtctc ctggacagtc gatcaccatc 60
   tcctgcgctt ccagcactgg agcagtcacc agtggttact atccaaactg gtaccaacag 120
   cacccaggca aagcccccaa actcatgatt tatagtacaa gcaacaaaca ctccggggtt 180
   tctaatcgct tctctggctc caagtctggc aacacggcct ccctgaccat ctctgggctc 240
   caggctgagg acgaggctga ttattactgc agctcatata caatcaccga catcgtggtc 300
   ttcggcggag ggaccaagct gaccgtccta 330
<210> 140
   <211> 333
   <212> DNA
   <213> Artificial Sequence
<220>
   <223> Synthetic DNA
<400> 140
   cagtctgccc tgactcagcc tgcctccgtg tctgggtctc ctggacagtc gatcaccatc 60
   tcctgcggct tgagctctgg ctcagtctct actagttact accccagctg gtaccaacag 120
   cacccaggca aagcccccaa actcatgatt tatagcacaa acactcgctc ttctggggtt 180
   tctaatcgct tctctggctc caagtctggc aacacggcct ccctgaccat ctctgggctc 240
   caggctgagg acgaggctga ttattactgc gtgctgtata tgggtagtgg catttctgtg 300
   gtattcggcg gagggaccaa gctgaccgtc cta 333
<210> 141
   <211> 333
   <212> DNA
   <213> Artificial Sequence
<220>
   <223> Synthetic DNA
<400> 141
   cagtctgccc tgactcagcc tgcctccgtg tctgggtctc ctggacagtc gatcaccatc 60
   tcctgcactg ggaacagcaa caatgttggc aaccaaggag cagcttggta ccaacagcac 120
   ccaggcaaag cccccaaact catgatttat aggaataaca accggccctc aggggtttct 180
   aatcgcttct ctggctccaa gtctggcaac acggcctccc tgaccatctc tgggctccag 240
   gctgaggacg aggctgatta ttactgcgca gcatgggatg acagcctgag tgcccatgtg 300
   gtattcggcg gagggaccaa gctgaccgtc cta 333
<210> 142
   <211> 13829
   <212> DNA
   <213> Artificial Sequence
<220>
   <223> Synthetic DNA
<400> 142
   acgagtgcgt acgctcgaca agacgcactc cagtctgccc tgactcagcc tgcctccgtg      60
   tctgggtctc ctggacagtc gatcaccatc tcctgctctg gaagcagctc caacatcgga     120
   agtaattatg tatactggta ccaacagcac ccaggcaaag cccccaaact catgatttat     180
   aggaataatc agcggccctc aggggtttct aatcgcttct ctggctccaa gtctggcaac     240
   acggcctccc tgaccatctc tgggctccag gctgaggacg aggctgatta ttactgcgca     300
   gcatgggatg acagcctgag tggtcattgg gtgttcggcg gagggaccaa gctgaccgtc     360
   ctagcctgac acacacagcc ctccccagac tgtgggatga ggccctgtgc ccgcagtcac     420
   atgtggaata tcaagacaca cacatctatg acaatcaccg tgtctctact cgcgtgtctc     480
   tctatgcgca gtctgccctg actcagcctg cctccgtgtc tgggtctcct ggacagtcga     540
   tcaccatctc ctgcactggg agcagctcca acatcggggc aggttatgat gtacactggt     600
   accaacagca cccaggcaaa gcccccaaac tcatgattta tggtaacagc aatcggccct     660
   caggggtttc taatcgcttc tctggctcca agtctggcaa cacggcctcc ctgaccatct     720
   ctgggctcca ggctgaggac gaggctgatt attactgcca gtcctatgac agcagcctga     780
   gtggttctgt ggtattcggc ggagggacca agctgaccgt cctagcctga cacacacagc     840
   cctccccaga ctgtgggatg aggccctgtg cccgcagtca catgtggaat atcaagacac     900
   acacatctat gacaatcaca tacgacgtat cacgtactac gtctagtacc agtctgccct     960
   gactcagcct gcctccgtgt ctgggtctcc tggacagtcg atcaccatct cctgctctgg    1020
   aagcagctcc aacattggga ataattatgt atcctggtac caacagcacc caggcaaagc    1080
   ccccaaactc atgatttatg acaataataa gcgaccctca ggggtttcta atcgcttctc    1140
   tggctccaag tctggcaaca cggcctccct gaccatctct gggctccagg ctgaggacga    1200
   ggctgattat tactgcggaa catgggatag cagcctgagt gctggtgctg tgttcggcgg    1260
   agggaccaag ctgaccgtcc tagcctgaca cacacagccc tccccagact gtgggatgag    1320
   gccctgtgcc cgcagtcaca tgtggaatat caagacacac acatctatga caatcaccgt    1380
   agactagtac gagtatgtac tctcgtgcag tctgccctga ctcagcctgc ctccgtgtct 1440
   gggtctcctg gacagtcgat caccatctcc tgcactggga gcagctccaa cattggggcg 1500
   ggttatgttg tacattggta ccaacagcac ccaggcaaag cccccaaact catgatttat 1560
   gaaaataata agcgaccctc aggggtttct aatcgcttct ctggctccaa gtctggcaac 1620
   acggcctccc tgaccatctc tgggctccag gctgaggacg aggctgatta ttactgcagc 1680
   tcatatacaa gcagcggcac tcctgtggta ttcggcggag ggaccaagct gaccgtccta 1740
   gcctgacaca cacagccctc cccagactgt gggatgaggc cctgtgcccg cagtcacatg 1800
   tggaatatca agacacacac atctatgaca atcacacgcg agtatactac tatgtactgt 1860
   acagtcagtc tgccctgact cagcctgcct ccgtgtctgg gtctcctgga cagtcgatca 1920
   ccatctcctg cactggaacc agcagtgatg ttgggagtta taaccttgtc tcctggtacc 1980
   aacagcaccc aggcaaagcc cccaaactca tgatttatga gggcagtaag cggccctcag 2040
   gggtttctaa tcgcttctct ggctccaagt ctggcaacac ggcctccctg accatctctg 2100
   ggctccaggc tgaggacgag gctgattatt actgccagtc ctatgacagc agcctgagtg 2160
   gttgccagtc ctatgacagc agcctgagtg gtttggtgtt cggcggaggg accaagctga 2220
   ccgtcctagc ctgacacaca cagccctccc cagactgtgg gatgaggccc tgtgcccgca 2280
   gtcacatgtg gaatatcaag acacacacat ctatgacaat cacatagagt actcacgcta 2340
   cgtcagtaga cgtcagtctg ccctgactca gcctgcctcc gtgtctgggt ctcctggaca 2400
   gtcgatcacc atctcctgcg ggggaaacaa cattggaagt aaaaatgtgc actggtacca 2460
   acagcaccca ggcaaagccc ccaaactcat gatttatagg gatagcaacc ggccctctgg 2520
   ggtttctaat cgcttctctg gctccaagtc tggcaacacg gcctccctga ccatctctgg 2580
   gctccaggct gaggacgagg ctgattatta ctgccagtcc tatgacagga gtggttgggt 2640
   gttcggcgga gggaccaagc tgaccgtcct agcctgacac acacagccct ccccagactg 2700
   tgggatgagg ccctgtgccc gcagtcacat gtggaatatc aagacacaca catctatgac 2760
   aatcactacg ctgtcttagt gtagattcga tcacgtcagt ctgccctgac tcagcctgcc 2820
   tccgtgtctg ggtctcctgg acagtcgatc accatctcct gctctggaga agcattgcca 2880
   aaaaaatatg cttattggta ccaacagcac ccaggcaaag cccccaaact catgatttat 2940
   aaagacagcg agaggccctc aggggtttct aatcgcttct ctggctccaa gtctggcaac 3000
   acggcctccc tgaccatctc tgggctccag gctgaggacg aggctgatta ttactgcaac 3060
   tcttttacaa gcaccaccaa ttatgtctta ttcggcggag ggaccaagct gaccgtccta 3120
   gcctgacaca cacagccctc cccagactgt gggatgaggc cctgtgcccg cagtcacatg 3180
   tggaatatca agacacacac atctatgaca atcactgacg tatgttgtga gtagtacagt 3240
   atatacagtc tgccctgact cagcctgcct ccgtgtctgg gtctcctgga cagtcgatca 3300
   ccatctcctg cgggggaaac aacattggaa gtaaaagtgt gcactggtac caacagcacc 3360
   caggcaaagc ccccaaactc atgatttatt atgatagcga ccggccctca ggggtttcta 3420
   atcgcttctc tggctccaag tctggcaaca cggcctccct gaccatctct gggctccagg 3480
   ctgaggacga ggctgattat tactgccagg tgtgggatga taatagtgat catcttatat 3540
   tcggcggagg gaccaagctg accgtcctag cctgacacac acagccctcc ccagactgtg 3600
   ggatgaggcc ctgtgcccgc agtcacatgt ggaatatcaa gacacacaca tctatgacaa 3660
   tcacagtata cataagtcga gagaagtgct acgacagtct gccctgactc agcctgcctc 3720
   cgtgtctggg tctcctggac agtcgatcac catctcctgc tctggagatg cattgccaaa 3780
   gcaatatgct tattggtacc aacagcaccc aggcaaagcc cccaaactca tgatttatga 3840
   tgatagcgac cggccctcag gggtttctaa tcgcttctct ggctccaagt ctggcaacac 3900
   ggcctccctg accatctctg ggctccaggc tgaggacgag gctgattatt actgcagctc 3960
   atatgcaggc agcaacaatt gggtgttcgg cggagggacc aagctgaccg tcctagcctg 4020
   acacacacag ccctccccag actgtgggat gaggccctgt gcccgcagtc acatgtggaa 4080
   tatcaagaca cacacatcta tgacaatcac cgtacagtca cgtactcaga ctacgctcta 4140
   cagtctgccc tgactcagcc tgcctccgtg tctgggtctc ctggacagtc gatcaccatc 4200
   tcctgcaccc taagcagtga gcacagcacc tacaccatcg aatggtacca acagcaccca 4260
   ggcaaagccc ccaaactcat gatttatgtt aagagtgatg gcagccacag caagggggac 4320
   ggggtttcta atcgcttctc tggctccaag tctggcaaca cggcctccct gaccatctct 4380
   gggctccagg ctgaggacga ggctgattat tactgcggag agagccacac gattgatggc 4440
   caagtcggtg tggtattcgg cggagggacc aagctgaccg tcctagcctg acacacacag 4500
   ccctccccag actgtgggat gaggccctgt gcccgcagtc acatgtggaa tatcaagaca 4560
   cacacatcta tgacaatcac tatatataca tatgctagta tcacgcgaga cagtctgccc 4620
   tgactcagcc tgcctccgtg tctgggtctc ctggacagtc gatcaccatc tcctgcactc 4680
   tgagcagtgg gcacagcagc tacgccatcg catggtacca acagcaccca ggcaaagccc 4740
   ccaaactcat gatttatctt aacagtgatg gcagccacag caagggggac ggggtttcta 4800
   atcgcttctc tggctccaag tctggcaaca cggcctccct gaccatctct gggctccagg 4860
   ctgaggacga ggctgattat tactgcagct catatgcagg caacaacaat ttgggggatg 4920
   tgcttttcgg cggagggacc aagctgaccg tcctagcctg acacacacag ccctccccag 4980
   actgtgggat gaggccctgt gcccgcagtc acatgtggaa tatcaagaca cacacatcta 5040
   tgacaatcac tgagtcagta tgtagtgtga tgtcacacga cagtctgccc tgactcagcc 5100
   tgcctccgtg tctgggtctc ctggacagtc gatcaccatc tcctgcacct tgcgcagtgg 5160
   catcaatgtt ggtacctaca ggatatactg gtaccaacag cacccaggca aagcccccaa 5220
   actcatgatt tattacaaat cagactcaga taagcagcag ggctctgggg tttctaatcg 5280
   cttctctggc tccaagtctg gcaacacggc ctccctgacc atctctgggc tccaggctga 5340
   ggacgaggct gattattact gcgcagcatg ggatgacagc ctgaatgtgg tattcggcgg 5400
   agggaccaag ctgaccgtcc tagcctgaca cacacagccc tccccagact gtgggatgag 5460
   gccctgtgcc cgcagtcaca tgtggaatat caagacacac acatctatga caatcacaca 5520
   tgacgacacg acagctcacg tctcatccag tctgccctga ctcagcctgc ctccgtgtct 5580
   gggtctcctg gacagtcgat caccatctcc tgcacccgca gcagtggcag cattgccagc 5640
   aactatgtgc agtggtacca acagcaccca ggcaaagccc ccaaactcat gatttatgag 5700
   gataaccaaa gaccctctgg ggtttctaat cgcttctctg gctccaagtc tggcaacacg 5760
   gcctccctga ccatctctgg gctccaggct gaggacgagg ctgattatta ctgccagtcc 5820
   tatgacagca gcctgaggag agtcttcggc ggagggacca agctgaccgt cctagcctga 5880
   cacacacagc cctccccaga ctgtgggatg aggccctgtg cccgcagtca catgtggaat 5940
   atcaagacac acacatctat gacaatcaca gcgactagca gtagtgatca gtgacacacc 6000
   agtctgccct gactcagcct gcctccgtgt ctgggtctcc tggacagtcg atcaccatct 6060
   cctgcggctc cagcactgga gctgtcacca gtggtcatta tccctactgg taccaacagc 6120
   acccaggcaa agcccccaaa ctcatgattt atgatacaag caacaaacac tccggggttt 6180
   ctaatcgctt ctctggctcc aagtctggca acacggcctc cctgaccatc tctgggctcc 6240
   aggctgagga cgaggctgat tattactgcg caacatggga tgacagcctg aatgagggag 6300
   tgttcggcgg agggaccaag ctgaccgtcc tagcctgaca cacacagccc tccccagact 6360
   gtgggatgag gccctgtgcc cgcagtcaca tgtggaatat caagacacac acatctatga 6420
   caatcacatc tactgaccac gtagatccac gtgtcgccag tctgccctga ctcagcctgc 6480
   ctccgtgtct gggtctcctg gacagtcgat caccatctcc tgcaccctga gcagcggcta 6540
   cagtaattat aaagtggact ggtaccaaca gcacccaggc aaagccccca aactcatgat 6600
   ttatgtgggc actggtggga ttgtgggatc caagggggat ggggtttcta atcgcttctc 6660
   tggctccaag tctggcaaca cggcctccct gaccatctct gggctccagg ctgaggacga 6720
   ggctgattat tactgcgggg cagaccatgg cagtgggagc aacttcgtgg tggtattcgg 6780
   cggagggacc aagctgaccg tcctagcctg acacacacag ccctccccag actgtgggat 6840
   gaggccctgt gcccgcagtc acatgtggaa tatcaagaca cacacatcta tgacaatcac 6900
   gtttaaacaa gcttcgaagg gcccgctagc gtcgacgttt aaacagcact gtagatcaga 6960
   cacgatatcg cgagcagtct gccctgactc agcctgcctc cgtgtctggg tctcctggac 7020
   agtcgatcac catctcctgc tctggaagca gctccaacat cggaaataat gctgtaaact 7080
   ggtaccaaca gcacccaggc aaagccccca aactcatgat ttattatgat gatctgctgc 7140
   cctcaggggt ttctaatcgc ttctctggct ccaagtctgg caacacggcc tccctgacca 7200
   tctctgggct ccaggctgag gacgaggctg attattactg cggaacatgg gatggcagcc 7260
   tgaatggtgg gaggctgttc ggcggaggga ccaagctgac cgtcctagcc tgacacacac 7320
   agccctcccc agactgtggg atgaggccct gtgcccgcag tcacatgtgg aatatcaaga 7380
   cacacacatc tatgacaatc actgatacgt ctcatagtag tgcgagagat accagtctgc 7440
   cctgactcag cctgcctccg tgtctgggtc tcctggacag tcgatcacca tctcctgctc 7500
   tggaagcagc tccaacatcg gaagtaatac tgtaaactgg taccaacagc acccaggcaa 7560
   agcccccaaa ctcatgattt atagtaataa tcagcggccc tcaggggttt ctaatcgctt 7620
   ctctggctcc aagtctggca acacggcctc cctgaccatc tctgggctcc aggctgagga 7680
   cgaggctgat tattactgcg cagcatggga tgacagcctg agtggtgtgg ttttcggcgg 7740
   agggaccaag ctgaccgtcc tagcctgaca cacacagccc tccccagact gtgggatgag 7800
   gccctgtgcc cgcagtcaca tgtggaatat caagacacac acatctatga caatcactct 7860
   acgtagctgt actactcacg actacagcag tctgccctga ctcagcctgc ctccgtgtct 7920
   gggtctcctg gacagtcgat caccatctcc tgctctggaa gcagctccga catggggaat 7980
   tatgcggtat cctggtacca acagcaccca ggcaaagccc ccaaactcat gatttatgat 8040
   gtcagtaagc ggccctcagg ggtttctaat cgcttctctg gctccaagtc tggcaacacg 8100
   gcctccctga ccatctctgg gctccaggct gaggacgagg ctgattatta ctgcagctca 8160
   tatgaagaca gcaacaattt gggggtattc ggcggaggga ccaagctgac cgtcctagcc 8220
   tgacacacac agccctcccc agactgtggg atgaggccct gtgcccgcag tcacatgtgg 8280
   aatatcaaga cacacacatc tatgacaatc actagagacg agtcgtcgct cgacatacgc 8340
   gtcagtctgc cctgactcag cctgcctccg tgtctgggtc tcctggacag tcgatcacca 8400
   tctcctgcac tggaaccagc agtgacgttg gtagttataa ccgtgtctcc tggtaccaac 8460
   agcacccagg caaagccccc aaactcatga tttatgaggt cagtaatcgg ccctcagggg 8520
   tttctaatcg cttctctggc tccaagtctg gcaacacggc ctccctgacc atctctgggc 8580
   tccaggctga ggacgaggct gattattact gcgcagcatg ggatggcagc ccggccccga 8640
   gtgactttta tgtcttcggc ggagggacca agctgaccgt cctagcctga cacacacagc 8700
   cctccccaga ctgtgggatg aggccctgtg cccgcagtca catgtggaat atcaagacac 8760
   acacatctat gacaatcaca gactatacta gcgtcgtcta gtacgctatc agtctgccct 8820
   gactcagcct gcctccgtgt ctgggtctcc tggacagtcg atcaccatct cctgctctgg 8880
   agataaattg ggggataaat atgcttgctg gtaccaacag cacccaggca aagcccccaa 8940
   actcatgatt tatcaagata gcaagcggcc ctcaggggtt tctaatcgct tctctggctc    9000
   caagtctggc aacacggcct ccctgaccat ctctgggctc caggctgagg acgaggctga    9060
   ttattactgc cagtcctttg acaacagcct gtgccagtcc tttgacaaca gcctgaatta    9120
   tgtgcttttc ggcggaggga ccaagctgac cgtcctagcc tgacacacac agccctcccc    9180
   agactgtggg atgaggccct gtgcccgcag tcacatgtgg aatatcaaga cacacacatc    9240
   tatgacaatc accgacgtga cttacacaca cttacagatc gtcagtctgc cctgactcag    9300
   cctgcctccg tgtctgggtc tcctggacag tcgatcacca tctcctgctc tggagatgca    9360
   ttgccaaaaa aatatgctta ttggtaccaa cagcacccag gcaaagcccc caaactcatg    9420
   atttatgagg acagcaaacg accctccggg gtttctaatc gcttctctgg ctccaagtct    9480
   ggcaacacgg cctccctgac catctctggg ctccaggctg aggacgaggc tgattattac    9540
   tgcgcagcat gggatgacag cctgaatggt cccttttatg tcttcggcgg agggaccaag    9600
   ctgaccgtcc tagcctgaca cacacagccc tccccagact gtgggatgag gccctgtgcc    9660
   cgcagtcaca tgtggaatat caagacacac acatctatga caatcactcg cactagttct    9720
   agcgacttct atactatcag tctgccctga ctcagcctgc ctccgtgtct gggtctcctg    9780
   gacagtcgat caccatctcc tgccaaggag acagcctcag aagctattat gcaagctggt    9840
   accaacagca cccaggcaaa gcccccaaac tcatgattta tggtaaaaac aaccggccct    9900
   caggggtttc taatcgcttc tctggctcca agtctggcaa cacggcctcc ctgaccatct    9960
   ctgggctcca ggctgaggac gaggctgatt attactgcca gtcctatgac agcagcctga   10020
   gtggtgtggt attcggcgga gggaccaagc tgaccgtcct agcctgacac acacagccct   10080
   ccccagactg tgggatgagg ccctgtgccc gcagtcacat gtggaatatc aagacacaca   10140
   catctatgac aatcacacgc gatcgaacta gcagtaagct cacgtacagt ctgccctgac   10200
   tcagcctgcc tccgtgtctg ggtctcctgg acagtcgatc accatctcct gctctggaga   10260
   tgtactgggg gaaaattatg ctgactggta ccaacagcac ccaggcaaag cccccaaact   10320
   catgatttat gaagatagtg agcggtaccc tggggtttct aatcgcttct ctggctccaa   10380
   gtctggcaac acggcctccc tgaccatctc tgggctccag gctgaggacg aggctgatta   10440
   ttactgcgga acatgggata gcagcctgag tggtgtggta ttcggcggag ggaccaagct   10500
   gaccgtccta gcctgacaca cacagccctc cccagactgt gggatgaggc cctgtgcccg   10560
   cagtcacatg tggaatatca agacacacac atctatgaca atcaccgatc gtatacgcag   10620
   tacgacgcgt atacacagtc tgccctgact cagcctgcct ccgtgtctgg gtctcctgga   10680
   cagtcgatca ccatctcctg ctcaggagat gtactggcaa aaaaatatgc tcggtggtac   10740
   caacagcacc caggcaaagc cccaaaactc atgatttata ggaaaaacag ccaactctca    10800
   ggggtttcta atcgcttctc tggctccaag tctggcaaca cggcctccct gaccatctct 10860
   gggctccagg ctgaggacga ggctgattat tactgctact ctgcggctga caacaatctt 10920
   gtggtattcg gcggagggac caagctgacc gtcctagcct gacacacaca gccctcccca 10980
   gactgtggga tgaggccctg tgcccgcagt cacatgtgga atatcaagac acacacatct 11040
   atgacaatca cctatagcgt atacgtcatc atagtcgcat acagtctgcc ctgactcagc 11100
   ctgcctccgt gtctgggtct cctggacagt cgatcaccat ctcctgcact ctgagcagtg 11160
   ggcacagtag ctacatcatc gcatggtacc aacagcaccc aggcaaagcc cccaaactca 11220
   tgatttatct tgaaggtagt ggaagctaca acaaggggag cggggtttct aatcgcttct 11280
   ctggctccaa gtctggcaac acggcctccc tgaccatctc tgggctccag gctgaggacg 11340
   aggctgatta ttactgcgag acctgggaca gtaacactca tgtggtattc ggcggaggga 11400
   ccaagctgac cgtcctagcc tgacacacac agccctcccc agactgtggg atgaggccct 11460
   gtgcccgcag tcacatgtgg aatatcaaga cacacacatc tatgacaatc actcgatagt 11520
   gatcgctgcg tatctgacgt cacagtctgc cctgactcag cctgcctccg tgtctgggtc 11580
   tcctggacag tcgatcacca tctcctgcac cttgcccagt gacatcaatg ttggtagcta 11640
   caacatatac tggtaccaac agcacccagg caaagccccc aaactcatga tttattacta 11700
   ctcagactca gataagggcc agggctctgg ggtttctaat cgcttctctg gctccaagtc 11760
   tggcaacacg gcctccctga ccatctctgg gctccaggct gaggacgagg ctgattatta 11820
   ctgccactca tatgcaggcg gcgacacttt cgatgtggtg ttcggcggag ggaccaagct 11880
   gaccgtccta gcctgacaca cacagccctc cccagactgt gggatgaggc cctgtgcccg 11940
   cagtcacatg tggaatatca agacacacac atctatgaca atcactgtcg tcgcaacaca 12000
   tacgcacagt cgtgccagtc tgccctgact cagcctgcct ccgtgtctgg gtctcctgga 12060
   cagtcgatca ccatctcctg catgctgagc agtggcttca gtgttgggga cttctggata 12120
   aggtggtacc aacagcaccc aggcaaagcc cccaaactca tgatttatta ccactcagac 12180
   tccaataagg gccaaggctc tggggtttct aatcgcttct ctggctccaa gtctggcaac 12240
   acggcctccc tgaccatctc tgggctccag gctgaggacg aggctgatta ttactgcggt 12300
   acatggcaca gcaactctaa gactcatgtg gtattcggcg gagggaccaa gctgaccgtc 12360
   ctagcctgac acacacagcc ctccccagac tgtgggatga ggccctgtgc ccgcagtcac 12420
   atgtggaata tcaagacaca cacatctatg acaatcacac tcatctacac tcgcgcacag 12480
   agcgtcacca gtctgccctg actcagcctg cctccgtgtc tgggtctcct ggacagtcga 12540
   tcaccatctc ctgcgcttcc agcactggag cagtcaccag tggttactat ccaaactggt 12600
   accaacagca cccaggcaaa gcccccaaac tcatgattta tagtacaagc aacaaacact 12660
   ccggggtttc taatcgcttc tctggctcca agtctggcaa cacggcctcc ctgaccatct 12720
   ctgggctcca ggctgaggac gaggctgatt attactgcag ctcatataca atcaccgaca 12780
   tcgtggtctt cggcggaggg accaagctga ccgtcctagc ctgacacaca cagccctccc 12840
   cagactgtgg gatgaggccc tgtgcccgca gtcacatgtg gaatatcaag acacacacat 12900
   ctatgacaat cacagtgtat gtcatagata gacatatagt cgccagtctg ccctgactca 12960
   gcctgcctcc gtgtctgggt ctcctggaca gtcgatcacc atctcctgcg gcttgagctc 13020
   tggctcagtc tctactagtt actaccccag ctggtaccaa cagcacccag gcaaagcccc 13080
   caaactcatg atttatagca caaacactcg ctcttctggg gtttctaatc gcttctctgg 13140
   ctccaagtct ggcaacacgg cctccctgac catctctggg ctccaggctg aggacgaggc 13200
   tgattattac tgcgtgctgt atatgggtag tggcatttct gtggtattcg gcggagggac 13260
   caagctgacc gtcctagcct gacacacaca gccctcccca gactgtggga tgaggccctg 13320
   tgcccgcagt cacatgtgga atatcaagac acacacatct atgacaatca ccatactcta 13380
   ccgacactat ccgagacgcg ccagtctgcc ctgactcagc ctgcctccgt gtctgggtct 13440
   cctggacagt cgatcaccat ctcctgcact gggaacagca acaatgttgg caaccaagga 13500
   gcagcttggt accaacagca cccaggcaaa gcccccaaac tcatgattta taggaataac 13560
   aaccggccct caggggtttc taatcgcttc tctggctcca agtctggcaa cacggcctcc 13620
   ctgaccatct ctgggctcca ggctgaggac gaggctgatt attactgcgc agcatgggat 13680
   gacagcctga gtgcccatgt ggtattcggc ggagggacca agctgaccgt cctagcctga 13740
   cacacacagc cctccccaga ctgtgggatg aggccctgtg cccgcagtca catgtggaat 13800
   atcaagacac acacatctat gacaatcac 13829
<210> 143
   <211> 25655
   <212> DNA
   <213> Artificial Sequence
<220>
   <223> Synthetic DNA
<400> 143
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgcggc cgcgggtatc acgtggggca     420
   aatcgagagc taccattggc tatggggagg agaaaatgag gcgtgctact tcctcgttcc 480
   tgcagtgcgt gtcgaggtgc cgtgttggtc ggatctcagt ggcagagcag gatggtgagc 540
   ggtggccttg gggccgctcc ctcactctgc tgggctggtg cggtggctcc gggaccggac 600
   tttcagtggt gtttggtgtg gtgctcactg tcctttggtg tcactgtgtg gtgaattccc 660
   tcagctgggc ggtgagagcc catgctgtgt cctggtagga gtagtctggg ctgcagtgcg 720
   ggctctttgt gcgtgttggg ggcggttccc tggaagctaa ttctggaaga aatcccggct 780
   gcctctgtaa cagttgttgg cgatgtcaaa caccctggag ctgctgactg gctctgtacc 840
   attgagtgat gcagtttaaa cctctctctt tcctcctgct gtgtgtgaag ctggtgttag 900
   tgctaggaga cctgcacatc cctcaccgat gcagtggttt accagcaaag ttcaagagcc 960
   tcctggttcc agggaagatt cagcatattt tgtgtacagg aaacctctgc accaaggaga 1020
   gctatgacta cctcaggact ttggctggag atgttcacgt tgttaagggg gactctgagg 1080
   taatgttctt gctgctgtga attcctcagt cctccatctt acaagccgta gttgtcccac 1140
   tgaaagctgg gaaacccttc ggtgtttgca attcctgagc gctaatgtgt gggggaagga 1200
   tgtaggaaca aataaggctc aagagacaac aacttcctag gctcctctta ttgcccttgt 1260
   ggtaagggag gacttggtgt gtagtggtaa tacatgcact caacttatta agttctcata 1320
   gctgctgtga agctgctgca gtccctcaga tccctgcagg aagaggggac tgactgactg 1380
   ttgaatagcc ttgaagctcc tgctctgcat gtggttccgg tcaggctcaa gatcccagtt 1440
   cttagctctt cagttgctct tctgggggta tagtactagc atctgggtca ctgcagatgg 1500
   acagagactg tgcttgggta gcaaaaggca gacttacgtt gtgaaacctt cattgtgttg 1560
   gtgaggttct ttgatttcta ccttgtgtat ctctcttcct cagagtctga attatcctga 1620
   acagaaggtt gtaactgttg ggcagttcag aattgggttg attcatggcc atcaagtcat 1680
   tccctggggt gatgtggcaa gcttggcatt gctgcagagg cagctggatg tagacattct 1740
   tatctcagga catacacaca gatttgaagc atttgagcat gaaaataagt tctacatcaa 1800
   cccaggatca gctacaggag cctacactgg tttacaaacg tgagtacaat gagccttgtg 1860
   tttgtcttta agacagatgt ctgtccttaa gagtgcagcc catgtgttga agagcactgg 1920
   tgagatgcag tgccatgcag gccatctctc cctttcccag agaacacggc ctacctctct 1980
   ctgttcataa ggtcttggtt aacttatgag ttggccagca cagcctggca ctgatgtgtc 2040
   atgatcataa ccctgagccc acgctctgct atgctgttgc ttcttagagt tgcattgcct 2100
   tgaggtgtat tccagaagct gtgtgctgag ctcttgttgg gaaagaggcc ctcagggcac 2160
   tgccaacatc tgaggcttgc aggagctgga acattcagcc ctgagggctg ctctggccaa 2220
   ctgcagctct gagtctgcag tcagtgcctc tgagctgtga tgcttctcag ataactgcag 2280
   ccctttgcaa tgtaatgttt tataatatat ttccatagga catcttggta agaggtgtaa 2340
   gctaatcagg gtgattgaca aagtatgttt tctttccagg aacgtcattc cttcatttgt 2400
   actgatggat atccaggctt ccacagttgt gacttatgta taccagctaa ttgaggacga 2460
   tgtgaaagta gaaagaattg agttcaagaa atactgacac gtgtttgaat ctgcctgctg 2520
   acttctcact gcctctcatt ctttctctcc tagttaaagc tgaatccggc cacgtggggg 2580
   tgctgctgca gcggcgtgta actgtgagct tgggaagttc tcaactaggg cttagagcag 2640
   gagaaactga ttaacagaaa ttgccttatt aaatgtatat atttcctgtg tctaagaacg 2700
   aggtgctgtt gttcctgact tgcggagctg cagctagtgg tagcttggaa tttaacctct 2760
   cctgtaaaat gagcatagaa taaaacagta ttttcacatt tctggatcct gttaattgtt 2820
   tgttaattaa gaggacagga attcagtgtc agggggctgc tctttgcttc ccaatgtgta 2880
   ccactgaagc ctctctggat ttatcagaga tactcaatgg actcttaaca gcactgtggc 2940
   acctgacctc ctgctgcctt cagcctccct tcctggctgg tgtgcttatg ttgaggagct 3000
   gtgggaggct ggtgcagttc tgtgggatga ggtaaggcca gctgaatagc ctgggggctg 3060
   gtcagggggc ttagtgagag gagcagggct ggggaatgtt gtgaacagca ggtggtgcca 3120
   ctgctattct gaggaaggag gcagcttctg caaatgcttt gcaatgcagg atgacaaagt 3180
   gcttctgcta gccctaggct accgttcgta taatgtatac tatacgaagt tattttattt 3240
   gtgaaatttg tgatgctatt gctttatttg taaccattat aagctgcaat aaacaagttg 3300
   gggtgggcga agaactccag catgagatcc ccgcgctgga ggatcatcca gccggcgtcc 3360
   cggaaaacga ttccgaagcc caacctttca tagaaggcgg cggtggaatc gaaatctcgt 3420
   gatggcaggt tgggcgtcgc ttggtcggtc atttcgaacc ccagagtccc gctcagaaga 3480
   actcgtcaag aaggcgatag aaggcgatgc gctgcgaatc gggagcggcg ataccgtaaa 3540
   gcacgaggaa gcggtcagcc cattcgccgc caagctcttc agcaatatca cgggtagcca 3600
   acgctatgtc ctgatagcgg tccgccacac ccagccggcc acagtcgatg aatccagaaa 3660
   agcggccatt ttccaccatg atattcggca agcaggcatc gccatgggtc acgacgagat 3720
   cctcgccgtc gggcatgcgc gccttgagcc tggcgaacag ttcggctggc gcgagcccct 3780
   gatgctcttc gtccagatca tcctgatcga caagaccggc ttccatccga gtacgtgctc 3840
   gctcgatgcg atgtttcgct tggtggtcga atgggcaggt agccggatca agcgtatgca 3900
   gccgccgcat tgcatcagcc atgatggata ctttctcggc aggagcaagg tgagatgaca 3960
   ggagatcctg ccccggcact tcgcccaata gcagccagtc ccttcccgct tcagtgacaa 4020
   cgtcgagcac agctgcgcaa ggaacgcccg tcgtggccag ccacgatagc cgcgctgcct 4080
   cgtcctgcag ttcattcagg gcaccggaca ggtcggtctt gacaaaaaga accgggcgcc 4140
   cctgcgctga cagccggaac acggcggcat cagagcagcc gattgtctgt tgtgcccagt 4200
   catagccgaa tagcctctcc acccaagcgg ccggagaacc tgcgtgcaat ccatcttgtt 4260
   caatcatgcg aaacgatcct catcctgtct cttgatcaga tccgaaaatg gatatacaag 4320
   ctcccgggag ctttttgcaa aagcctaggc ctccaaaaaa gcctcctcac tacttctgga 4380
   atagctcaga ggcagaggcg gcctcggcct ctgcataaat aaaaaaaatt agtcagccat 4440
   ggggcggaga atgggcggaa ctgggcggag ttaggggcgg gatgggcgga gttaggggcg 4500
   ggactatggt tgctgactaa ttgagatgca tgctttgcat acttctgcct gctggggagc 4560
   ctggggactt tccacacctg gttgctgact aattgagatg catgctttgc atacttctgc 4620
   ctgctgggga gcctggggac tttccacacc ctaaataact tcgtataatg tatactatac 4680
   gaacggtagc gttaaccggt cgacatgcaa gcttgctagc gtttaaacac gagtgcgtac 4740
   gctcgacaag acgcactcca gtctgccctg actcagcctg cctccgtgtc tgggtctcct 4800
   ggacagtcga tcaccatctc ctgctctgga agcagctcca acatcggaag taattatgta 4860
   tactggtacc aacagcaccc aggcaaagcc cccaaactca tgatttatag gaataatcag 4920
   cggccctcag gggtttctaa tcgcttctct ggctccaagt ctggcaacac ggcctccctg 4980
   accatctctg ggctccaggc tgaggacgag gctgattatt actgcgcagc atgggatgac 5040
   agcctgagtg gtcattgggt gttcggcgga gggaccaagc tgaccgtcct agcctgacac 5100
   acacagccct ccccagactg tgggatgagg ccctgtgccc gcagtcacat gtggaatatc 5160
   aagacacaca catctatgac aatcaccgtg tctctactcg cgtgtctctc tatgcgcagt 5220
   ctgccctgac tcagcctgcc tccgtgtctg ggtctcctgg acagtcgatc accatctcct 5280
   gcactgggag cagctccaac atcggggcag gttatgatgt acactggtac caacagcacc 5340
   caggcaaagc ccccaaactc atgatttatg gtaacagcaa tcggccctca ggggtttcta 5400
   atcgcttctc tggctccaag tctggcaaca cggcctccct gaccatctct gggctccagg 5460
   ctgaggacga ggctgattat tactgccagt cctatgacag cagcctgagt ggttctgtgg 5520
   tattcggcgg agggaccaag ctgaccgtcc tagcctgaca cacacagccc tccccagact 5580
   gtgggatgag gccctgtgcc cgcagtcaca tgtggaatat caagacacac acatctatga 5640
   caatcacata cgacgtatca cgtactacgt ctagtaccag tctgccctga ctcagcctgc 5700
   ctccgtgtct gggtctcctg gacagtcgat caccatctcc tgctctggaa gcagctccaa 5760
   cattgggaat aattatgtat cctggtacca acagcaccca ggcaaagccc ccaaactcat 5820
   gatttatgac aataataagc gaccctcagg ggtttctaat cgcttctctg gctccaagtc 5880
   tggcaacacg gcctccctga ccatctctgg gctccaggct gaggacgagg ctgattatta 5940
   ctgcggaaca tgggatagca gcctgagtgc tggtgctgtg ttcggcggag ggaccaagct 6000
   gaccgtccta gcctgacaca cacagccctc cccagactgt gggatgaggc cctgtgcccg 6060
   cagtcacatg tggaatatca agacacacac atctatgaca atcaccgtag actagtacga 6120
   gtatgtactc tcgtgcagtc tgccctgact cagcctgcct ccgtgtctgg gtctcctgga 6180
   cagtcgatca ccatctcctg cactgggagc agctccaaca ttggggcggg ttatgttgta 6240
   cattggtacc aacagcaccc aggcaaagcc cccaaactca tgatttatga aaataataag 6300
   cgaccctcag gggtttctaa tcgcttctct ggctccaagt ctggcaacac ggcctccctg 6360
   accatctctg ggctccaggc tgaggacgag gctgattatt actgcagctc atatacaagc 6420
   agcggcactc ctgtggtatt cggcggaggg accaagctga ccgtcctagc ctgacacaca 6480
   cagccctccc cagactgtgg gatgaggccc tgtgcccgca gtcacatgtg gaatatcaag 6540
   acacacacat ctatgacaat cacacgcgag tatactacta tgtactgtac agtcagtctg 6600
   ccctgactca gcctgcctcc gtgtctgggt ctcctggaca gtcgatcacc atctcctgca 6660
   ctggaaccag cagtgatgtt gggagttata accttgtctc ctggtaccaa cagcacccag 6720
   gcaaagcccc caaactcatg atttatgagg gcagtaagcg gccctcaggg gtttctaatc 6780
   gcttctctgg ctccaagtct ggcaacacgg cctccctgac catctctggg ctccaggctg 6840
   aggacgaggc tgattattac tgccagtcct atgacagcag cctgagtggt tgccagtcct 6900
   atgacagcag cctgagtggt ttggtgttcg gcggagggac caagctgacc gtcctagcct 6960
   gacacacaca gccctcccca gactgtggga tgaggccctg tgcccgcagt cacatgtgga 7020
   atatcaagac acacacatct atgacaatca catagagtac tcacgctacg tcagtagacg 7080
   tcagtctgcc ctgactcagc ctgcctccgt gtctgggtct cctggacagt cgatcaccat 7140
   ctcctgcggg ggaaacaaca ttggaagtaa aaatgtgcac tggtaccaac agcacccagg 7200
   caaagccccc aaactcatga tttataggga tagcaaccgg ccctctgggg tttctaatcg 7260
   cttctctggc tccaagtctg gcaacacggc ctccctgacc atctctgggc tccaggctga 7320
   ggacgaggct gattattact gccagtccta tgacaggagt ggttgggtgt tcggcggagg 7380
   gaccaagctg accgtcctag cctgacacac acagccctcc ccagactgtg ggatgaggcc 7440
   ctgtgcccgc agtcacatgt ggaatatcaa gacacacaca tctatgacaa tcactacgct 7500
   gtcttagtgt agattcgatc acgtcagtct gccctgactc agcctgcctc cgtgtctggg 7560
   tctcctggac agtcgatcac catctcctgc tctggagaag cattgccaaa aaaatatgct 7620
   tattggtacc aacagcaccc aggcaaagcc cccaaactca tgatttataa agacagcgag 7680
   aggccctcag gggtttctaa tcgcttctct ggctccaagt ctggcaacac ggcctccctg 7740
   accatctctg ggctccaggc tgaggacgag gctgattatt actgcaactc ttttacaagc 7800
   accaccaatt atgtcttatt cggcggaggg accaagctga ccgtcctagc ctgacacaca 7860
   cagccctccc cagactgtgg gatgaggccc tgtgcccgca gtcacatgtg gaatatcaag 7920
   acacacacat ctatgacaat cactgacgta tgttgtgagt agtacagtat atacagtctg 7980
   ccctgactca gcctgcctcc gtgtctgggt ctcctggaca gtcgatcacc atctcctgcg 8040
   ggggaaacaa cattggaagt aaaagtgtgc actggtacca acagcaccca ggcaaagccc 8100
   ccaaactcat gatttattat gatagcgacc ggccctcagg ggtttctaat cgcttctctg 8160
   gctccaagtc tggcaacacg gcctccctga ccatctctgg gctccaggct gaggacgagg 8220
   ctgattatta ctgccaggtg tgggatgata atagtgatca tcttatattc ggcggaggga 8280
   ccaagctgac cgtcctagcc tgacacacac agccctcccc agactgtggg atgaggccct 8340
   gtgcccgcag tcacatgtgg aatatcaaga cacacacatc tatgacaatc acagtataca 8400
   taagtcgaga gaagtgctac gacagtctgc cctgactcag cctgcctccg tgtctgggtc 8460
   tcctggacag tcgatcacca tctcctgctc tggagatgca ttgccaaagc aatatgctta 8520
   ttggtaccaa cagcacccag gcaaagcccc caaactcatg atttatgatg atagcgaccg 8580
   gccctcaggg gtttctaatc gcttctctgg ctccaagtct ggcaacacgg cctccctgac 8640
   catctctggg ctccaggctg aggacgaggc tgattattac tgcagctcat atgcaggcag 8700
   caacaattgg gtgttcggcg gagggaccaa gctgaccgtc ctagcctgac acacacagcc 8760
   ctccccagac tgtgggatga ggccctgtgc ccgcagtcac atgtggaata tcaagacaca 8820
   cacatctatg acaatcaccg tacagtcacg tactcagact acgctctaca gtctgccctg 8880
   actcagcctg cctccgtgtc tgggtctcct ggacagtcga tcaccatctc ctgcacccta 8940
   agcagtgagc acagcaccta caccatcgaa tggtaccaac agcacccagg caaagccccc 9000
   aaactcatga tttatgttaa gagtgatggc agccacagca agggggacgg ggtttctaat 9060
   cgcttctctg gctccaagtc tggcaacacg gcctccctga ccatctctgg gctccaggct 9120
   gaggacgagg ctgattatta ctgcggagag agccacacga ttgatggcca agtcggtgtg 9180
   gtattcggcg gagggaccaa gctgaccgtc ctagcctgac acacacagcc ctccccagac 9240
   tgtgggatga ggccctgtgc ccgcagtcac atgtggaata tcaagacaca cacatctatg 9300
   acaatcacta tatatacata tgctagtatc acgcgagaca gtctgccctg actcagcctg 9360
   cctccgtgtc tgggtctcct ggacagtcga tcaccatctc ctgcactctg agcagtgggc 9420
   acagcagcta cgccatcgca tggtaccaac agcacccagg caaagccccc aaactcatga 9480
   tttatcttaa cagtgatggc agccacagca agggggacgg ggtttctaat cgcttctctg 9540
   gctccaagtc tggcaacacg gcctccctga ccatctctgg gctccaggct gaggacgagg 9600
   ctgattatta ctgcagctca tatgcaggca acaacaattt gggggatgtg cttttcggcg 9660
   gagggaccaa gctgaccgtc ctagcctgac acacacagcc ctccccagac tgtgggatga 9720
   ggccctgtgc ccgcagtcac atgtggaata tcaagacaca cacatctatg acaatcactg 9780
   agtcagtatg tagtgtgatg tcacacgaca gtctgccctg actcagcctg cctccgtgtc 9840
   tgggtctcct ggacagtcga tcaccatctc ctgcaccttg cgcagtggca tcaatgttgg 9900
   tacctacagg atatactggt accaacagca cccaggcaaa gcccccaaac tcatgattta 9960
   ttacaaatca gactcagata agcagcaggg ctctggggtt tctaatcgct tctctggctc 10020
   caagtctggc aacacggcct ccctgaccat ctctgggctc caggctgagg acgaggctga 10080
   ttattactgc gcagcatggg atgacagcct gaatgtggta ttcggcggag ggaccaagct 10140
   gaccgtccta gcctgacaca cacagccctc cccagactgt gggatgaggc cctgtgcccg 10200
   cagtcacatg tggaatatca agacacacac atctatgaca atcacacatg acgacacgac 10260
   agctcacgtc tcatccagtc tgccctgact cagcctgcct ccgtgtctgg gtctcctgga 10320
   cagtcgatca ccatctcctg cacccgcagc agtggcagca ttgccagcaa ctatgtgcag 10380
   tggtaccaac agcacccagg caaagccccc aaactcatga tttatgagga taaccaaaga 10440
   ccctctgggg tttctaatcg cttctctggc tccaagtctg gcaacacggc ctccctgacc 10500
   atctctgggc tccaggctga ggacgaggct gattattact gccagtccta tgacagcagc 10560
   ctgaggagag tcttcggcgg agggaccaag ctgaccgtcc tagcctgaca cacacagccc 10620
   tccccagact gtgggatgag gccctgtgcc cgcagtcaca tgtggaatat caagacacac 10680
   acatctatga caatcacagc gactagcagt agtgatcagt gacacaccag tctgccctga 10740
   ctcagcctgc ctccgtgtct gggtctcctg gacagtcgat caccatctcc tgcggctcca 10800
   gcactggagc tgtcaccagt ggtcattatc cctactggta ccaacagcac ccaggcaaag 10860
   cccccaaact catgatttat gatacaagca acaaacactc cggggtttct aatcgcttct 10920
   ctggctccaa gtctggcaac acggcctccc tgaccatctc tgggctccag gctgaggacg 10980
   aggctgatta ttactgcgca acatgggatg acagcctgaa tgagggagtg ttcggcggag 11040
   ggaccaagct gaccgtccta gcctgacaca cacagccctc cccagactgt gggatgaggc 11100
   cctgtgcccg cagtcacatg tggaatatca agacacacac atctatgaca atcacatcta 11160
   ctgaccacgt agatccacgt gtcgccagtc tgccctgact cagcctgcct ccgtgtctgg 11220
   gtctcctgga cagtcgatca ccatctcctg caccctgagc agcggctaca gtaattataa 11280
   agtggactgg taccaacagc acccaggcaa agcccccaaa ctcatgattt atgtgggcac 11340
   tggtgggatt gtgggatcca agggggatgg ggtttctaat cgcttctctg gctccaagtc 11400
   tggcaacacg gcctccctga ccatctctgg gctccaggct gaggacgagg ctgattatta 11460
   ctgcggggca gaccatggca gtgggagcaa cttcgtggtg gtattcggcg gagggaccaa 11520
   gctgaccgtc ctagcctgac acacacagcc ctccccagac tgtgggatga ggccctgtgc 11580
   ccgcagtcac atgtggaata tcaagacaca cacatctatg acaatcacgt ttaaacaagc 11640
   ttcgaagggc ccgctagcgt cgacgtttaa acagcactgt agatcagaca cgatatcgcg 11700
   agcagtctgc cctgactcag cctgcctccg tgtctgggtc tcctggacag tcgatcacca 11760
   tctcctgctc tggaagcagc tccaacatcg gaaataatgc tgtaaactgg taccaacagc 11820
   acccaggcaa agcccccaaa ctcatgattt attatgatga tctgctgccc tcaggggttt 11880
   ctaatcgctt ctctggctcc aagtctggca acacggcctc cctgaccatc tctgggctcc 11940
   aggctgagga cgaggctgat tattactgcg gaacatggga tggcagcctg aatggtggga 12000
   ggctgttcgg cggagggacc aagctgaccg tcctagcctg acacacacag ccctccccag 12060
   actgtgggat gaggccctgt gcccgcagtc acatgtggaa tatcaagaca cacacatcta 12120
   tgacaatcac tgatacgtct catagtagtg cgagagatac cagtctgccc tgactcagcc 12180
   tgcctccgtg tctgggtctc ctggacagtc gatcaccatc tcctgctctg gaagcagctc 12240
   caacatcgga agtaatactg taaactggta ccaacagcac ccaggcaaag cccccaaact 12300
   catgatttat agtaataatc agcggccctc aggggtttct aatcgcttct ctggctccaa 12360
   gtctggcaac acggcctccc tgaccatctc tgggctccag gctgaggacg aggctgatta 12420
   ttactgcgca gcatgggatg acagcctgag tggtgtggtt ttcggcggag ggaccaagct 12480
   gaccgtccta gcctgacaca cacagccctc cccagactgt gggatgaggc cctgtgcccg 12540
   cagtcacatg tggaatatca agacacacac atctatgaca atcactctac gtagctgtac 12600
   tactcacgac tacagcagtc tgccctgact cagcctgcct ccgtgtctgg gtctcctgga 12660
   cagtcgatca ccatctcctg ctctggaagc agctccgaca tggggaatta tgcggtatcc 12720
   tggtaccaac agcacccagg caaagccccc aaactcatga tttatgatgt cagtaagcgg 12780
   ccctcagggg tttctaatcg cttctctggc tccaagtctg gcaacacggc ctccctgacc 12840
   atctctgggc tccaggctga ggacgaggct gattattact gcagctcata tgaagacagc 12900
   aacaatttgg gggtattcgg cggagggacc aagctgaccg tcctagcctg acacacacag 12960
   ccctccccag actgtgggat gaggccctgt gcccgcagtc acatgtggaa tatcaagaca 13020
   cacacatcta tgacaatcac tagagacgag tcgtcgctcg acatacgcgt cagtctgccc 13080
   tgactcagcc tgcctccgtg tctgggtctc ctggacagtc gatcaccatc tcctgcactg 13140
   gaaccagcag tgacgttggt agttataacc gtgtctcctg gtaccaacag cacccaggca 13200
   aagcccccaa actcatgatt tatgaggtca gtaatcggcc ctcaggggtt tctaatcgct 13260
   tctctggctc caagtctggc aacacggcct ccctgaccat ctctgggctc caggctgagg 13320
   acgaggctga ttattactgc gcagcatggg atggcagccc ggccccgagt gacttttatg 13380
   tcttcggcgg agggaccaag ctgaccgtcc tagcctgaca cacacagccc tccccagact 13440
   gtgggatgag gccctgtgcc cgcagtcaca tgtggaatat caagacacac acatctatga 13500
   caatcacaga ctatactagc gtcgtctagt acgctatcag tctgccctga ctcagcctgc 13560
   ctccgtgtct gggtctcctg gacagtcgat caccatctcc tgctctggag ataaattggg 13620
   ggataaatat gcttgctggt accaacagca cccaggcaaa gcccccaaac tcatgattta 13680
   tcaagatagc aagcggccct caggggtttc taatcgcttc tctggctcca agtctggcaa 13740
   cacggcctcc ctgaccatct ctgggctcca ggctgaggac gaggctgatt attactgcca 13800
   gtcctttgac aacagcctgt gccagtcctt tgacaacagc ctgaattatg tgcttttcgg 13860
   cggagggacc aagctgaccg tcctagcctg acacacacag ccctccccag actgtgggat 13920
   gaggccctgt gcccgcagtc acatgtggaa tatcaagaca cacacatcta tgacaatcac 13980
   cgacgtgact tacacacact tacagatcgt cagtctgccc tgactcagcc tgcctccgtg 14040
   tctgggtctc ctggacagtc gatcaccatc tcctgctctg gagatgcatt gccaaaaaaa 14100
   tatgcttatt ggtaccaaca gcacccaggc aaagccccca aactcatgat ttatgaggac 14160
   agcaaacgac cctccggggt ttctaatcgc ttctctggct ccaagtctgg caacacggcc 14220
   tccctgacca tctctgggct ccaggctgag gacgaggctg attattactg cgcagcatgg 14280
   gatgacagcc tgaatggtcc cttttatgtc ttcggcggag ggaccaagct gaccgtccta 14340
   gcctgacaca cacagccctc cccagactgt gggatgaggc cctgtgcccg cagtcacatg 14400
   tggaatatca agacacacac atctatgaca atcactcgca ctagttctag cgacttctat 14460
   actatcagtc tgccctgact cagcctgcct ccgtgtctgg gtctcctgga cagtcgatca 14520
   ccatctcctg ccaaggagac agcctcagaa gctattatgc aagctggtac caacagcacc 14580
   caggcaaagc ccccaaactc atgatttatg gtaaaaacaa ccggccctca ggggtttcta 14640
   atcgcttctc tggctccaag tctggcaaca cggcctccct gaccatctct gggctccagg 14700
   ctgaggacga ggctgattat tactgccagt cctatgacag cagcctgagt ggtgtggtat 14760
   tcggcggagg gaccaagctg accgtcctag cctgacacac acagccctcc ccagactgtg 14820
   ggatgaggcc ctgtgcccgc agtcacatgt ggaatatcaa gacacacaca tctatgacaa 14880
   tcacacgcga tcgaactagc agtaagctca cgtacagtct gccctgactc agcctgcctc 14940
   cgtgtctggg tctcctggac agtcgatcac catctcctgc tctggagatg tactggggga 15000
   aaattatgct gactggtacc aacagcaccc aggcaaagcc cccaaactca tgatttatga 15060
   agatagtgag cggtaccctg gggtttctaa tcgcttctct ggctccaagt ctggcaacac 15120
   ggcctccctg accatctctg ggctccaggc tgaggacgag gctgattatt actgcggaac 15180
   atgggatagc agcctgagtg gtgtggtatt cggcggaggg accaagctga ccgtcctagc 15240
   ctgacacaca cagccctccc cagactgtgg gatgaggccc tgtgcccgca gtcacatgtg 15300
   gaatatcaag acacacacat ctatgacaat caccgatcgt atacgcagta cgacgcgtat 15360
   acacagtctg ccctgactca gcctgcctcc gtgtctgggt ctcctggaca gtcgatcacc 15420
   atctcctgct caggagatgt actggcaaaa aaatatgctc ggtggtacca acagcaccca 15480
   ggcaaagccc ccaaactcat gatttatagg aaaaacagcc aactctcagg ggtttctaat 15540
   cgcttctctg gctccaagtc tggcaacacg gcctccctga ccatctctgg gctccaggct 15600
   gaggacgagg ctgattatta ctgctactct gcggctgaca acaatcttgt ggtattcggc 15660
   ggagggacca agctgaccgt cctagcctga cacacacagc cctccccaga ctgtgggatg 15720
   aggccctgtg cccgcagtca catgtggaat atcaagacac acacatctat gacaatcacc 15780
   tatagcgtat acgtcatcat agtcgcatac agtctgccct gactcagcct gcctccgtgt 15840
   ctgggtctcc tggacagtcg atcaccatct cctgcactct gagcagtggg cacagtagct 15900
   acatcatcgc atggtaccaa cagcacccag gcaaagcccc caaactcatg atttatcttg 15960
   aaggtagtgg aagctacaac aaggggagcg gggtttctaa tcgcttctct ggctccaagt 16020
   ctggcaacac ggcctccctg accatctctg ggctccaggc tgaggacgag gctgattatt 16080
   actgcgagac ctgggacagt aacactcatg tggtattcgg cggagggacc aagctgaccg 16140
   tcctagcctg acacacacag ccctccccag actgtgggat gaggccctgt gcccgcagtc 16200
   acatgtggaa tatcaagaca cacacatcta tgacaatcac tcgatagtga tcgctgcgta 16260
   tctgacgtca cagtctgccc tgactcagcc tgcctccgtg tctgggtctc ctggacagtc 16320
   gatcaccatc tcctgcacct tgcccagtga catcaatgtt ggtagctaca acatatactg 16380
   gtaccaacag cacccaggca aagcccccaa actcatgatt tattactact cagactcaga 16440
   taagggccag ggctctgggg tttctaatcg cttctctggc tccaagtctg gcaacacggc 16500
   ctccctgacc atctctgggc tccaggctga ggacgaggct gattattact gccactcata 16560
   tgcaggcggc gacactttcg atgtggtgtt cggcggaggg accaagctga ccgtcctagc 16620
   ctgacacaca cagccctccc cagactgtgg gatgaggccc tgtgcccgca gtcacatgtg 16680
   gaatatcaag acacacacat ctatgacaat cactgtcgtc gcaacacata cgcacagtcg 16740
   tgccagtctg ccctgactca gcctgcctcc gtgtctgggt ctcctggaca gtcgatcacc 16800
   atctcctgca tgctgagcag tggcttcagt gttggggact tctggataag gtggtaccaa 16860
   cagcacccag gcaaagcccc caaactcatg atttattacc actcagactc caataagggc 16920
   caaggctctg gggtttctaa tcgcttctct ggctccaagt ctggcaacac ggcctccctg 16980
   accatctctg ggctccaggc tgaggacgag gctgattatt actgcggtac atggcacagc 17040
   aactctaaga ctcatgtggt attcggcgga gggaccaagc tgaccgtcct agcctgacac 17100
   acacagccct ccccagactg tgggatgagg ccctgtgccc gcagtcacat gtggaatatc 17160
   aagacacaca catctatgac aatcacactc atctacactc gcgcacagag cgtcaccagt 17220
   ctgccctgac tcagcctgcc tccgtgtctg ggtctcctgg acagtcgatc accatctcct 17280
   gcgcttccag cactggagca gtcaccagtg gttactatcc aaactggtac caacagcacc 17340
   caggcaaagc ccccaaactc atgatttata gtacaagcaa caaacactcc ggggtttcta 17400
   atcgcttctc tggctccaag tctggcaaca cggcctccct gaccatctct gggctccagg 17460
   ctgaggacga ggctgattat tactgcagct catatacaat caccgacatc gtggtcttcg 17520
   gcggagggac caagctgacc gtcctagcct gacacacaca gccctcccca gactgtggga 17580
   tgaggccctg tgcccgcagt cacatgtgga atatcaagac acacacatct atgacaatca 17640
   cagtgtatgt catagataga catatagtcg ccagtctgcc ctgactcagc ctgcctccgt 17700
   gtctgggtct cctggacagt cgatcaccat ctcctgcggc ttgagctctg gctcagtctc 17760
   tactagttac taccccagct ggtaccaaca gcacccaggc aaagccccca aactcatgat 17820
   ttatagcaca aacactcgct cttctggggt ttctaatcgc ttctctggct ccaagtctgg 17880
   caacacggcc tccctgacca tctctgggct ccaggctgag gacgaggctg attattactg 17940
   cgtgctgtat atgggtagtg gcatttctgt ggtattcggc ggagggacca agctgaccgt 18000
   cctagcctga cacacacagc cctccccaga ctgtgggatg aggccctgtg cccgcagtca 18060
   catgtggaat atcaagacac acacatctat gacaatcacc atactctacc gacactatcc 18120
   gagacgcgcc agtctgccct gactcagcct gcctccgtgt ctgggtctcc tggacagtcg 18180
   atcaccatct cctgcactgg gaacagcaac aatgttggca accaaggagc agcttggtac 18240
   caacagcacc caggcaaagc ccccaaactc atgatttata ggaataacaa ccggccctca 18300
   ggggtttcta atcgcttctc tggctccaag tctggcaaca cggcctccct gaccatctct 18360
   gggctccagg ctgaggacga ggctgattat tactgcgcag catgggatga cagcctgagt 18420
   gcccatgtgg tattcggcgg agggaccaag ctgaccgtcc tagcctgaca cacacagccc 18480
   tccccagact gtgggatgag gccctgtgcc cgcagtcaca tgtggaatat caagacacac 18540
   acatctatga caatcacgtt taaacaagct tcgaagaatt ccgcggatgc ggcgcgcctc 18600
   tagaatgcat cgatttaaat cgagtgaagg tcaccaagga tggagacttc agagctatct 18660
   gggacacgtg ttttaacgtt tgatcagcct tgcagcaaaa cagtgttttg ctctattgaa 18720
   cagtgttcaa caaaaacgat cagtggtctg gaacacatct cctacgaaga aaggttgcga 18780
   gagctgggtc tggagaagag gaggctctgg ggcaacctcc ctgtggcctg tcagtgctta 18840
   aaggaggctc aagagaaaga tggagagaga ctttatacac ggagctgtag caatatgaca 18900
   aggagaaatg gtttaaaatg gaaagagggt agatttaggt tagctatcaa gaagaaattc 18960
   ttcactcaga ggtgatgaag cactgtagca ggttgcccag agaagtattg gatgcttcct 19020
   tcctggcagg gcccaaggcc agactggatg ggttctgggc agcctgttct ggtgggagat 19080
   gtccctgccc gtggcacggc ttggaaatgg gtagcctttg aggtccctct cagcccaaat 19140
   cattctatgt tcttgtggtt ccctgcattt cagcctttgc ctgttgactc tggtgccact 19200
   gttggacaat tctactcagc tgtgtttcat ccctgcccca aagcagtaat cccatggata 19260
   agcaatgctc tgaggccttc accaccatca gggctcctgc tgaccctagc ggcctccctt 19320
   gcagcatgag ccctttgttg tccgcactgc tgctgctgag ggtcctgcca gccccccagg 19380
   gccttttctg cccaggcact gccacctgct aaaacccagc ctgcccatgg cagggcatcg 19440
   catgtctgag ggatggaacg gggcaagggc agtgctgcag gcagtggttc aagaaacgtt 19500
   tagatgttgt actgagggat gtggtttagt gggaaatact ggtgataggt ggatggttgg 19560
   actggaggat cttagaggtc ttttccaacc ttggtgatta tgtgattcta tgaagggagc 19620
   catagcctgc tcccatcatg ccccgccagt ccggctctcc cgagcacttt ggttccgcag 19680
   catccaggcc cgctctgggg ccggtgtggg cctgggtgct gccacgtgtc cccgctgcaa 19740
   ggcctggcca accccgctgt gccacaatgc aggggctggg ctaggagtgg gcagggaggt 19800
   gtggggagag gtgtaggacg tggctgggag cgcagggagt tatttgcata ggggggcgtg 19860
   ctgcggaagg acgcgggtat aaaagggcat cgaggtcccc ggcacagccc catcggcgtg 19920
   gggacacaca gctgctggga ttccgccatg gcctgggctc ctctcctcct ggcggtgctc 19980
   gcccacacct caggtactcg ttgcgcccgg tcggggactg tgggcacggg gctctgtccc 20040
   actgctgcgc gggcagggct gtgcgtgcgg ggccgtcact gattgccgtt ttctcccctc 20100
   tctcctctcc ggcgcgcctc tccaggttcc ctggtgcagg cacagtctgc cctgactcag 20160
   cctgcctccg tgtctgggtc tcctggacag tcgatcacca tctcctgcac tggaaccagc 20220
   agtgacgttg gtggttataa ctatgtctcc tggtaccaac aaaacccagg caaagccccc 20280
   aaactcatga tttatgatgt cagtaatcgg ccctcaggga tttctaatcg cttctctggc 20340
   tccaagtctg gcaacacggc ctccctgacc atctctgggc tccaggctga cgacgaggct 20400
   gattattact gcacctcata tacaaacgac agcaattctc aggtattcgg cggagggacc 20460
   aagctgaccg tcctaggtga gtcgctcata tgctaccgtt cgtatagcat acattatacg 20520
   aagttatcag acatgataag atacattgat gagtttggac aaaccacaac tagaatgcag 20580
   tgaaaaaaat gctttatttg tgaaatttgt gatgctattg ctttatttgt aaccattata 20640
   agctgcaata aacaagttgg ggtgggcgaa gaactccagc atgagatccc cgcgctggag 20700
   gatcatccag ccggcgtccc ggaaaacgat tccgaagccc aacctttcat agaaggcggc 20760
   ggtggaatcg aaatctcgta gcacgtgtca gtcctgctcc tcggccacga agtgcttagc 20820
   cctcccacac ataaccagag ggcagcaatt cacgaatccc aactgccgtc ggctgtccat 20880
   cactgtcctt cactatggct ttgatcccag gatgcagatc gagaagcacc tgtcggcacc 20940
   gtccgcaggg gctcaagatg cccctgttct catttccgat cgcgacgata caagtcaggt 21000
   tgccagctgc cgcagcagca gcagtgccca gcaccacgag ttctgcacaa ggtcccccag 21060
   taaaatgata tacattgaca ccagtgaaga tgcggccgtc gctagagaga gctgcgctgg 21120
   cgacgctgta gtcttcagag atggggatgc tgttgattgt agccgttgct ctttcaatga 21180
   gggtggattc ttcttgagac aaaggcttgg ccatggttta gttcctcacc ttgtcgtatt 21240
   atactatgcc gatatactat gccgatgatt aattgtcaac acgtgctgat cagatccgaa 21300
   aatggatata caagctcccg ggagcttttt gcaaaagcct aggcctccaa aaaagcctcc 21360
   tcactacttc tggaatagct cagaggcaga ggcggcctcg gcctctgcat aaataaaaaa 21420
   aattagtcag ccatggggcg gagaatgggc ggaactgggc ggagttaggg gcgggatggg 21480
   cggagttagg ggcgggacta tggttgctga ctaattgaga tgcatgcttt gcatacttct 21540
   gcctgctggg gagcctgggg actttccaca cctggttgct gactaattga gatgcatgct 21600
   ttgcatactt ctgcctgctg gggagcctgg ggactttcca caccctaact gacacacatt 21660
   ccacagataa cttcgtatag catacattat acgaacggta gctctagaga cctcgtctcg 21720
   gtctttcttc ccccatcgtg aaattgtgac attttgtcga tttttggtga tttgggggtt 21780
   tttcttggac ttggcggcag gctggggtct gccaccggcg cagggccggg cactcagcgc 21840
   gacagcctgg gctgagtctt gtccccaccg agccggaggg ctccggtgtg cgccatggag 21900
   gacttagggt tattttgtca atggaaagtt cttaaaattt gaccagaaaa tgtgcccgag 21960
   gtctgtctct gccacacgat ttcagaaatt gtgtctaggt cgatgagaag acagtttttg 22020
   tctttgtcag gaaattagtt gtgagttgtt agtccttccc tcttagtcct aaggactaag 22080
   acctttgtcc ccggtctggt ctctcactgg ggactcttgg ctccagtgcc atggggagcc 22140
   caagtgtcac tgacacggtg tccttggggg tgaaattcag tttttcagct gtccaggtaa 22200
   gtgctgtcaa aaaatggagg ttattctgct gaaaaagctg agaggaatat tttgtcattt 22260
   ttttcggaaa tatatatata tatatatata taaatatata aattatttat atattttata 22320
   tatatatata tataaaatat atgtatttct ctctttcttt ttatatatat atataaatat 22380
   atatatattt ctccctcttt ctttatatat atttataggg agagaatcta tatttttggc 22440
   caatttggcc aatttctctc tctctctctt tatatatata tatatatgta tatgtaattt 22500
   atatatatat atataaattt atatttattt atttatttat ttatatagat tatttttata 22560
   tacatatata tatataaatt tggccaattt agaagaaatt ggccaaaaaa acccaatctg 22620
   aaccaaatta cttagggcat aagtccaaaa aaaaagtgca gttcaagaat tcctcgctgg 22680
   aaagttttta atgggggcag ttcttcccca ttttctggtg tagatatgag tgaagatata 22740
   gatatgtatg tagatataga tttggctata gtttgtttga tgaacagtct gaggaagagt 22800
   aaatcttgcc ttcctcatgg caatttttaa ttctacttct tctgagacaa gtctgaacat 22860
   gccttgaggt agaaagagcc cgaaaattga gtgctttttc tttgaaattt taattaaaat 22920
   taaaaaatag ccttacagca ggaaagaaag ggggtgatgg cagctaatcg gagttccaaa 22980
   gctgaaccac gttcacccaa acacgtggtt gaaattttgt gtgtgtatat atataaatat 23040
   atatatgtat acaaaagaaa atgcctatat ataaaaaaat tgttatgtat attatataga 23100
   tttattatag atatgtacat aagatataac acgtatacat atatatgtag tctgctgtat 23160
   gctttgtata aatttttata gatatgatgt atattatatg catgggttat atatctataa 23220
   tatattttat ctgtagaagg aactacagta taaaatgcat atatgcgtat atatacacat 23280
   atttatatat atgtgtatat atatgtatac gcacacgtat aacatccatg aaaatagata 23340
   tggatgggtg gatgtgtttg ttttacagag gtgcatgtgt gtctgtacat acacagccat 23400
   acatacgcgt gtgcggccgc aggcgtaatc atggtcatag ctgtttcctg tgtgaaattg 23460
   ttatccgctc acaattccac acaacatacg agccggaagc ataaagtgta aagcctgggg 23520
   tgcctaatga gtgagctaac tcacattaat tgcgttgcgc tcactgcccg ctttccagtc 23580
   gggaaacctg tcgtgccagc tgcattaatg aatcggccaa cgcgcgggga gaggcggttt 23640
   gcgtattggg cgctcttccg cttcctcgct cactgactcg ctgcgctcgg tcgttcggct 23700
   gcggcgagcg gtatcagctc actcaaaggc ggtaatacgg ttatccacag aatcagggga 23760
   taacgcagga aagaacatgt gagcaaaagg ccagcaaaag gccaggaacc gtaaaaaggc 23820
   cgcgttgctg gcgtttttcc ataggctccg cccccctgac gagcatcaca aaaatcgacg 23880
   ctcaagtcag aggtggcgaa acccgacagg actataaaga taccaggcgt ttccccctgg 23940
   aagctccctc gtgcgctctc ctgttccgac cctgccgctt accggatacc tgtccgcctt 24000
   tctcccttcg ggaagcgtgg cgctttctca tagctcacgc tgtaggtatc tcagttcggt 24060
   gtaggtcgtt cgctccaagc tgggctgtgt gcacgaaccc cccgttcagc ccgaccgctg 24120
   cgccttatcc ggtaactatc gtcttgagtc caacccggta agacacgact tatcgccact 24180
   ggcagcagcc actggtaaca ggattagcag agcgaggtat gtaggcggtg ctacagagtt 24240
   cttgaagtgg tggcctaact acggctacac tagaagaaca gtatttggta tctgcgctct 24300
   gctgaagcca gttaccttcg gaaaaagagt tggtagctct tgatccggca aacaaaccac 24360
   cgctggtagc ggtggttttt ttgtttgcaa gcagcagatt acgcgcagaa aaaaaggatc 24420
   tcaagaagat cctttgatct tttctacggg gtctgacgct cagtggaacg aaaactcacg 24480
   ttaagggatt ttggtcatga gattatcaaa aaggatcttc acctagatcc ttttaaatta 24540
   aaaatgaagt tttaaatcaa tctaaagtat atatgagtaa acttggtctg acagttacca 24600
   atgcttaatc agtgaggcac ctatctcagc gatctgtcta tttcgttcat ccatagttgc 24660
   ctgactcccc gtcgtgtaga taactacgat acgggagggc ttaccatctg gccccagtgc 24720
   tgcaatgata ccgcgagacc cacgctcacc ggctccagat ttatcagcaa taaaccagcc 24780
   agccggaagg gccgagcgca gaagtggtcc tgcaacttta tccgcctcca tccagtctat 24840
   taattgttgc cgggaagcta gagtaagtag ttcgccagtt aatagtttgc gcaacgttgt 24900
   tgccattgct acaggcatcg tggtgtcacg ctcgtcgttt ggtatggctt cattcagctc 24960
   cggttcccaa cgatcaaggc gagttacatg atcccccatg ttgtgcaaaa aagcggttag 25020
   ctccttcggt cctccgatcg ttgtcagaag taagttggcc gcagtgttat cactcatggt 25080
   tatggcagca ctgcataatt ctcttactgt catgccatcc gtaagatgct tttctgtgac 25140
   tggtgagtac tcaaccaagt cattctgaga atagtgtatg cggcgaccga gttgctcttg 25200
   cccggcgtca atacgggata ataccgcgcc acatagcaga actttaaaag tgctcatcat 25260
   tggaaaacgt tcttcggggc gaaaactctc aaggatctta ccgctgttga gatccagttc 25320
   gatgtaaccc actcgtgcac ccaactgatc ttcagcatct tttactttca ccagcgtttc 25380
   tgggtgagca aaaacaggaa ggcaaaatgc cgcaaaaaag ggaataaggg cgacacggaa 25440
   atgttgaata ctcatactct tcctttttca atattattga agcatttatc agggttattg 25500
   tctcatgagc ggatacatat ttgaatgtat ttagaaaaat aaacaaatag gggttccgcg 25560
   cacatttccc cgaaaagtgc cacctgacgt ctaagaaacc attattatca tgacattaac 25620
   ctataaaaat aggcgtatca cgaggccctt tcgtc 25655
<210> 144
   <211> 7420
   <212> DNA
   <213> Artificial Sequence
<220>
   <223> Synthetic DNA
<400> 144
<210> 145
   <211> 11635
   <212> DNA
   <213> Artificial Sequence
<220>
   <223> Synthetic DNA
<400> 145
   tcgcgcgttt cggtgatgac ggtgaaaacc tctgacacat gcagctcccg gagacggtca        60
   cagcttgtct gtaagcggat gccgggagca gacaagcccg tcagggcgcg tcagcgggtg       120
   ttggcgggtg tcggggctgg cttaactatg cggcatcaga gcagattgta ctgagagtgc       180
   accatatgcg gtgtgaaata ccgcacagat gcgtaaggag aaaataccgc atcaggcgcc       240
   attcgccatt caggctgcgc aactgttggg aagggcgatc ggtgcgggcc tcttcgctat       300
   tacgccagct ggcgaaaggg ggatgtgctg caaggcgatt aagttgggta acgccagggt       360
   tttcccagtc acgacgttgt aaaacgacgg ccagtgaatt catatgtagc ttacgacact       420
   gataacttcg tatatggtat tatatacgaa gttataagct tcaccatgat tgaacaagat       480
   ggattgcacg caggttctcc ggccgcttgg gtggagaggc tattcggcta tgactgggca       540
   caacagacaa tcggctgctc tgatgccgcc gtgttccggc tgtcagcgca ggggcgcccg       600
   gttctttttg tcaagaccga cctgtccggt gccctgaatg aactgcagga cgaggcagcg       660
   cggctatcgt ggctggccac gacgggcgtt ccttgcgcag ctgtgctcga cgttgtcact       720
   gaagcgggaa gggactggct gctattgggc gaagtgccgg ggcaggatct cctgtcatct 780
   caccttgctc ctgccgagaa agtatccatc atggctgatg caatgcggcg gctgcatacg 840
   cttgatccgg ctacctgccc attcgaccac caagcgaaac atcgcatcga gcgagcacgt 900
   actcggatgg aagccggtct tgtcgatcag gatgatctgg acgaagagca tcaggggctc 960
   gcgccagccg aactgttcgc caggctcaag gcgcgcatgc ccgacggcga ggatctcgtc 1020
   gtgacccatg gcgatgcctg cttgccgaat atcatggtgg aaaatggccg cttttctgga 1080
   ttcatcgact gtggccggct gggtgtggcg gaccgctatc aggacatagc gttggctacc 1140
   cgtgatattg ctgaagagct tggcggcgaa tgggctgacc gcttcctcgt gctttacggt 1200
   atcgccgctc ccgattcgca gcgcatcgcc ttctatcgcc ttcttgacga gttcttctga 1260
   actagtgcgg gactctgggg ttcgaaatga ccgaccaagc gacgcccaac ctgccatcac 1320
   gagatttcga ttccaccgcc gccttctatg aaaggttggg cttcggaatc gttttccggg 1380
   acgccggctg gatgatcctc cagcgcgggg atctcatgct ggagttcttc gcccacccca 1440
   acttgtttat tgcagcttat aatggttaca aataaagcaa tagcatcaca aatttcacaa 1500
   ataaagcatt tttttcactg cattctagtt gtggtttgtc caaactcatc aatgtatctt 1560
   atcatgtctg ttataccgtc gatctctagc tagagcttgg cgtaatcatg gtcatagctg 1620
   tttcctgtgt gcccacccca acttgtttat tgcagcttat aatggttaca aataaagcaa 1680
   tagcatcaca aatttcacaa ataaagcatt tttttcactg cattctagtt gtggtttgtc 1740
   caaactcatc aatgtatctt atcatgtctg ataacttcgt ataatgtatg ctatacgaac 1800
   ggtactcgag agctcgttta aacagcactg tagatcagac acgatatcgc gaggaggtgc 1860
   agctgttgga gtctggggga ggcttggtac agcctggggg gtccctgaga ctctcctgtg 1920
   cagcctctgg attcaccttt agcagctatg ctatgcattg ggtccgccag gctccaggga 1980
   aggggctgga gtgggtctca tggatcaacg ctggcaatgg taacacaaaa tattcacaga 2040
   agttccaggg ccggttcacc atctccagag acaattccaa gaacacgctg tatctgcaaa 2100
   tgaacagcct gagagccgag gacacggccg tatattactg tgcgaaagcg tctagtagtg 2160
   gttattacta cgtccacttt gactactggg gccagggcac cctggtcacc gtctcctcag 2220
   tgcctggtga ctcttccgca atcagtcctg ggacagaaac ctcagggaca gcctttatga 2280
   gttttgatca catctaacag agaggatggg ccagtgatac gtctcatagt agtgcgagag 2340
   atacgaggtg cagctgttgg agtctggggg aggcttggta cagcctgggg ggtccctgag 2400
   actctcctgt gcagcctctg gattcacctt tagcagctat ggtatcagct gggtccgcca 2460
   ggctccaggg aaggggctgg agtgggtctc atggatcagc gcttacaatg gtaacacaaa 2520
   ctatgcacag aagctccagg gccggttcac catctccaga gacaattcca agaacacgct 2580
   gtatctgcaa atgaacagcc tgagagccga ggacacggcc gtatattact gtgcgaaaga 2640
   tttaaggtta tcctattact actttgacta ctggggccag ggcaccctgg tcaccgtctc 2700
   ctcagtgcct ggtgactctt ccgcaatcag tcctgggaca gaaacctcag ggacagcctt 2760
   tatgagtttt gatcacatct aacagagagg atgggccagt ctacgtagct gtactactca 2820
   cgactacagg aggtgcagct gttggagtct gggggaggct tggtacagcc tggggggtcc 2880
   ctgagactct cctgtgcagc ctctggattc acctttagcg gctttgccat gaactgggtc 2940
   cgccaggctc cagggaaggg gctggagtgg gtctcaacta ttagtaatag tgctggtagc 3000
   acatattacg cagactccgt gaagggccgg ttcaccatct ccagagacaa ttccaagaac 3060
   acgctgtatc tgcaaatgaa cagcctgaga gccgaggaca cggccgtata ttactgtgcg 3120
   aaatatgggg aggatattgt cgttgtgcca gctcgctacg gtgtggacgt ctggggccag 3180
   ggcaccctgg tcaccgtctc ctcagtgcct ggtgactctt ccgcaatcag tcctgggaca 3240
   gaaacctcag ggacagcctt tatgagtttt gatcacatct aacagagagg atgggccagt 3300
   agagacgagt cgtcgctcga catacgcgtg aggtgcagct gttggagtct gggggaggct 3360
   tggtacagcc tggggggtcc ctgagactct cctgtgcagc ctctggattc acctttagca 3420
   gctctgctgt gcagtgggtc cgccaggctc cagggaaggg gctggagtgg gtctcatgga 3480
   tcgtcgttgg cagtggtaac acaaactacg cacagaagtt ccaggaacgg ttcaccatct 3540
   ccagagacaa ttccaagaac acgctgtatc tgcaaatgaa cagcctgaga gccgaggaca 3600
   cggccgtata ttactgtgcg aaagacgtcc ttgtagtacc agctgccccc tactactact 3660
   acggtatgga cgtctggggc cagggcaccc tggtcaccgt ctcctcagtg cctggtgact 3720
   cttccgcaat cagtcctggg acagaaacct cagggacagc ctttatgagt tttgatcaca 3780
   tctaacagag aggatgggcc agagactata ctagcgtcgt ctagtacgct atgaggtgca 3840
   gctgttggag tctgggggag gcttggtaca gcctgggggg tccctgagac tctcctgtgc 3900
   agcctctgga ttcaccttta gcactagtgg agtgggtgtg ggctgggtcc gccaggctcc 3960
   agggaagggg ctggagtggg tctcactcat ttattggaat gatgataagc gctacagccc 4020
   atctctgaag agccggttca ccatctccag agacaattcc aagaacacgc tgtatctgca 4080
   aatgaacagc ctgagagccg aggacacggc cgtatattac tgtgcgaaag acgatggggc 4140
   gtatgggccg tatagcagcg cctggacctt tgactactgg ggccagggca ccctggtcac 4200
   cgtctcctca gtgcctggtg actcttccgc aatcagtcct gggacagaaa cctcagggac 4260
   agcctttatg agttttgatc acatctaaca gagaggatgg gccagcgacg tgacttacac 4320
   acacttacag atcgtgaggt gcagctgttg gagtctgggg gaggcttggt acagcctggg 4380
   gggtccctga gactctcctg tgcagcctct ggattcacct ttagcactag tggaatgcgt 4440
   gtgagctggg tccgccaggc tccagggaag gggctggagt gggtctcacg cattgattgg 4500
   gatgatgata aattctacag cacatctctg aagacccggt tcaccatctc cagagacaat 4560
   tccaagaaca cgctgtatct gcaaatgaac agcctgagag ccgaggacac ggccgtatat 4620
   tactgtgcga aagatgacgg tggcaccccg cccggggaat tctttgacta ctggggccag 4680
   ggcaccctgg tcaccgtctc ctcagtgcct ggtgactctt ccgcaatcag tcctgggaca 4740
   gaaacctcag ggacagcctt tatgagtttt gatcacatct aacagagagg atgggccagt 4800
   cgcactagtt ctagcgactt ctatactatg aggtgcagct gttggagtct gggggaggct 4860
   tggtacagcc tggggggtcc ctgagactct cctgtgcagc ctctggattc acctttagcg 4920
   attatgccat gcactgggtc cgccaggctc cagggaaggg gctggagtgg gtctcaggta 4980
   ttagttggaa tagtggtagc ataggctatg cggactctgt gaagggccgg ttcaccatct 5040
   ccagagacaa ttccaagaac acgctgtatc tgcaaatgaa cagcctgaga gccgaggaca 5100
   cggccgtata ttactgtgcg aaaatagtgg gagctactta caactggttc gacccctggg 5160
   gccagggcac cctggtcacc gtctcctcag tgcctggtga ctcttccgca atcagtcctg 5220
   ggacagaaac ctcagggaca gcctttatga gttttgatca catctaacag agaggatggg 5280
   ccagacgcga tcgaactagc agtaagctca cgtagaggtg cagctgttgg agtctggggg 5340
   aggcttggta cagcctgggg ggtccctgag actctcctgt gcagcctctg gattcacctt 5400
   tagcaacgcc tggatgagct gggtccgcca ggctccaggg aaggggctgg agtgggtctc 5460
   acgtattaaa agcaaaactg atggtgggac aacagactac gctgcacccg tgaaaggccg 5520
   gttcaccatc tccagagaca attccaagaa cacgctgtat ctgcaaatga acagcctgag 5580
   agccgaggac acggccgtat attactgtgc gaaagaaagg ggtagcagct cgtcctttga 5640
   ctactggggc cagggcaccc tggtcaccgt ctcctcagtg cctggtgact cttccgcaat 5700
   cagtcctggg acagaaacct cagggacagc ctttatgagt tttgatcaca tctaacagag 5760
   aggatgggcc agcgatcgta tacgcagtac gacgcgtata cagaggtgca gctgttggag 5820
   tctgggggag gcttggtaca gcctgggggg tccctgagac tctcctgtgc agcctctgga 5880
   ttcaccttta gcagctatgg catgcactgg gtccgccagg ctccagggaa ggggctggag 5940
   tgggtctcag ttatatggta tgatggaagt aataaatact atgcagactc cgtgaagggc 6000
   cggttcacca tctccagaga caattccaag aacacgctgt atctgcaaat gaacagcctg 6060
   agagccgagg acacggccgt atattactgt gcgaaagatc ttgtcttcgg gccagctgct 6120
   actccttttg actactgggg ccagggcacc ctggtcaccg tctcctcagt gcctggtgac 6180
   tcttccgcaa tcagtcctgg gacagaaacc tcagggacag cctttatgag ttttgatcac 6240
   atctaacaga gaggatgggc cagctatagc gtatacgtca tcatagtcgc atagaggtgc 6300
   agctgttgga gtctggggga ggcttggtac agcctggggg gtccctgaga ctctcctgtg 6360
   cagcctctgg attcaccttt agcagcaact acatgagctg ggtccgccag gctccaggga 6420
   aggggctgga gtgggtctca gttatttata gcggtggtag cacatactac gcagactccg 6480
   tgaagggccg gttcaccatc tccagagaca attccaagaa cacgctgtat ctgcaaatga 6540
   acagcctgag agccgaggac acggccgtat attactgtgc gaaagcgaag ttctcctatg 6600
   gttcagggag ttattacttt gactactggg gccagggcac cctggtcacc gtctcctcag 6660
   tgcctggtga ctcttccgca atcagtcctg ggacagaaac ctcagggaca gcctttatga 6720
   gttttgatca catctaacag agaggatggg ccagtcgata gtgatcgctg cgtatctgac 6780
   gtcagaggtg cagctgttgg agtctggggg aggcttggta cagcctgggg ggtccctgag 6840
   actctcctgt gcagcctctg gattcacctt tagcagctac tggatgcact gggtccgcca 6900
   ggctccaggg aaggggctgg agtgggtctc acgtattaat agtgatggga gtagcacaag 6960
   ctacgcggac tccgtgaagg gccggttcac catctccaga gacaattcca agaacacgct 7020
   gtatctgcaa atgaacagcc tgagagccga ggacacggcc gtatattact gtgcgaaagg 7080
   atattgtagt agtaccagct gctatgctga tgcttttgat atctggggcc agggcaccct 7140
   ggtcaccgtc tcctcagtgc ctggtgactc ttccgcaatc agtcctggga cagaaacctc 7200
   agggacagcc tttatgagtt ttgatcacat ctaacagaga ggatgggcca gtgtcgtcgc 7260
   aacacatacg cacagtcgtg cgaggtgcag ctgttggagt ctgggggagg cttggtacag 7320
   cctggggggt ccctgagact ctcctgtgca gcctctggat tcacctttag cagtagtaac 7380
   tggtggggct gggtccgcca ggctccaggg aaggggctgg agtgggtctc atacatctat 7440
   tatagtggga gcacctacta caacccgtcc ctcaagagtc ggttcaccat ctccagagac 7500
   aattccaaga acacgctgta tctgcaaatg aacagcctga gagccgagga cacggccgta 7560
   tattactgtg cgaaagatcg aagtagaggg ggttttgact actggggcca gggcaccctg 7620
   gtcaccgtct cctcagtgcc tggtgactct tccgcaatca gtcctgggac agaaacctca 7680
   gggacagcct ttatgagttt tgatcacatc taacagagag gatgggccag actcatctac 7740
   actcgcgcac agagcgtcac gaggtgcagc tgttggagtc tgggggaggc ttggtacagc 7800
   ctggggggtc cctgagactc tcctgtgcag cctctggatt cacctttagc ggttactact 7860
   ggagctgggt ccgccaggct ccagggaagg ggctggagtg ggtctcagaa atcaatcata 7920
   gtggaagcac caactacaac ccgtccctca agagtcggtt caccatctcc agagacaatt 7980
   ccaagaacac gctgtatctg caaatgaaca gcctgagagc cgaggacacg gccgtatatt 8040
   actgtgcgaa agcccgtacc agcgcgggcc cgggtggtgc ttttgatttc tggggccagg 8100
   gcaccctggt caccgtctcc tcagtgcctg gtgactcttc cgcaatcagt cctgggacag 8160
   aaacctcagg gacagccttt atgagttttg atcacatcta acagagagga tgggccagag 8220
   tgtatgtcat agatagacat atagtcgcga ggtgcagctg ttggagtctg ggggaggctt 8280
   ggtacagcct ggggggtccc tgagactctc ctgtgcagcc tctggattca cctttagcag 8340
   ctactggatc ggctgggtcc gccaggctcc agggaagggg ctggagtggg tctcaatcat 8400
   ctatcctggt gactctgata ccagatacag cccgtccttc caaggccggt tcaccatctc 8460
   cagagacaat tccaagaaca cgctgtatct gcaaatgaac agcctgagag ccgaggacac 8520
   ggccgtatat tactgtgcga aaggggtttg tagtaccagc tcgtacgcct tttactacta 8580
   ctactacggt atggacgtct ggggccaggg caccctggtc accgtctcct cagtgcctgg 8640
   tgactcttcc gcaatcagtc ctgggacaga aacctcaggg acagccttta tgagttttga 8700
   tcacatctaa cagagaggat gggccagcat actctaccga cactatccga gacgcgcgag 8760
   gtgcagctgt tggagtctgg gggaggcttg gtacagcctg gggggtccct gagactctcc 8820
   tgtgcagcct ctggattcac ctttagcagc tatgctatga attgggtccg ccaggctcca 8880
   gggaaggggc tggagtgggt ctcatggatc aacaccaaca ctgggaaccc aacgtatgcc 8940
   cagggcttca caggacggtt caccatctcc agagacaatt ccaagaacac gctgtatctg 9000
   caaatgaaca gcctgagagc cgaggacacg gccgtatatt actgtgcgaa agactacggt 9060
   ggtaactcct actggtactt cgatctctgg ggccagggca ccctggtcac cgtctcctca 9120
   gtgcctggtg actcttccgc aatcagtcct gggacagaaa cctcagggac agcctttatg 9180
   agttttgatc acatctaaca gagaggatgg gccaggttta aacggtaccg ctagcatgct 9240
   ggccaggatc catgcccatg gccgcggatg cggcgcgcca tcgatatgcg tcgacattta 9300
   aatgggcccc ccggtgatat ccccgggtct agagcggccg ctaccgttcg tatattctat 9360
   cttatacgaa gttatatcga attcctgcag agatctagct tggcgtaatc atggtcatag 9420
   ctgtttcctg tgtgaaattg ttatccgctc acaattccac acaacatacg agccggaagc 9480
   ataaagtgta aagcctgggg tgcctaatga gtgagctaac tcacattaat tgcgttgcgc 9540
   tcactgcccg ctttccagtc gggaaacctg tcgtgccagc tgcattaatg aatcggccaa 9600
   cgcgcgggga gaggcggttt gcgtattggg cgctcttccg cttcctcgct cactgactcg 9660
   ctgcgctcgg tcgttcggct gcggcgagcg gtatcagctc actcaaaggc ggtaatacgg 9720
   ttatccacag aatcagggga taacgcagga aagaacatgt gagcaaaagg ccagcaaaag 9780
   gccaggaacc gtaaaaaggc cgcgttgctg gcgtttttcc ataggctccg cccccctgac 9840
   gagcatcaca aaaatcgacg ctcaagtcag aggtggcgaa acccgacagg actataaaga 9900
   taccaggcgt ttccccctgg aagctccctc gtgcgctctc ctgttccgac cctgccgctt 9960
   accggatacc tgtccgcctt tctcccttcg ggaagcgtgg cgctttctca tagctcacgc 10020
   tgtaggtatc tcagttcggt gtaggtcgtt cgctccaagc tgggctgtgt gcacgaaccc 10080
   cccgttcagc ccgaccgctg cgccttatcc ggtaactatc gtcttgagtc caacccggta 10140
   agacacgact tatcgccact ggcagcagcc actggtaaca ggattagcag agcgaggtat 10200
   gtaggcggtg ctacagagtt cttgaagtgg tggcctaact acggctacac tagaagaaca 10260
   gtatttggta tctgcgctct gctgaagcca gttaccttcg gaaaaagagt tggtagctct 10320
   tgatccggca aacaaaccac cgctggtagc ggtggttttt ttgtttgcaa gcagcagatt 10380
   acgcgcagaa aaaaaggatc tcaagaagat cctttgatct tttctacggg gtctgacgct 10440
   cagtggaacg aaaactcacg ttaagggatt ttggtcatga gattatcaaa aaggatcttc 10500
   acctagatcc ttttaaatta aaaatgaagt tttaaatcaa tctaaagtat atatgagtaa 10560
   acttggtctg acagttacca atgcttaatc agtgaggcac ctatctcagc gatctgtcta 10620
   tttcgttcat ccatagttgc ctgactcccc gtcgtgtaga taactacgat acgggagggc 10680
   ttaccatctg gccccagtgc tgcaatgata ccgcgagacc cacgctcacc ggctccagat 10740
   ttatcagcaa taaaccagcc agccggaagg gccgagcgca gaagtggtcc tgcaacttta 10800
   tccgcctcca tccagtctat taattgttgc cgggaagcta gagtaagtag ttcgccagtt 10860
   aatagtttgc gcaacgttgt tgccattgct acaggcatcg tggtgtcacg ctcgtcgttt 10920
   ggtatggctt cattcagctc cggttcccaa cgatcaaggc gagttacatg atcccccatg 10980
   ttgtgcaaaa aagcggttag ctccttcggt cctccgatcg ttgtcagaag taagttggcc 11040
   gcagtgttat cactcatggt tatggcagca ctgcataatt ctcttactgt catgccatcc 11100
   gtaagatgct tttctgtgac tggtgagtac tcaaccaagt cattctgaga atagtgtatg 11160
   cggcgaccga gttgctcttg cccggcgtca atacgggata ataccgcgcc acatagcaga 11220
   actttaaaag tgctcatcat tggaaaacgt tcttcggggc gaaaactctc aaggatctta 11280
   ccgctgttga gatccagttc gatgtaaccc actcgtgcac ccaactgatc ttcagcatct 11340
   tttactttca ccagcgtttc tgggtgagca aaaacaggaa ggcaaaatgc cgcaaaaaag 11400
   ggaataaggg cgacacggaa atgttgaata ctcatactct tcctttttca atattattga 11460
   agcatttatc agggttattg tctcatgagc ggatacatat ttgaatgtat ttagaaaaat 11520
   aaacaaatag gggttccgcg cacatttccc cgaaaagtgc cacctgacgt ctaagaaacc 11580
   attattatca tgacattaac ctataaaaat aggcgtatca cgaggccctt tcgtc 11635
<210> 146
   <211> 11624
   <212> DNA
   <213> Artificial Sequence
<220>
   <223> Synthetic DNA
<400> 146
   tcgcgcgttt cggtgatgac ggtgaaaacc tctgacacat gcagctcccg gagacggtca       60
   cagcttgtct gtaagcggat gccgggagca gacaagcccg tcagggcgcg tcagcgggtg      120
   ttggcgggtg tcggggctgg cttaactatg cggcatcaga gcagattgta ctgagagtgc      180
   accatatgcg gtgtgaaata ccgcacagat gcgtaaggag aaaataccgc atcaggcgcc      240
   attcgccatt caggctgcgc aactgttggg aagggcgatc ggtgcgggcc tcttcgctat      300
   tacgccagct ggcgaaaggg ggatgtgctg caaggcgatt aagttgggta acgccagggt      360
   tttcccagtc acgacgttgt aaaacgacgg ccagtgaatt catatgtagc ttacgacact      420
   gataacttcg tatatggtat tatatacgaa gttataagct tcaccatgat tgaacaagat      480
   ggattgcacg caggttctcc ggccgcttgg gtggagaggc tattcggcta tgactgggca      540
   caacagacaa tcggctgctc tgatgccgcc gtgttccggc tgtcagcgca ggggcgcccg      600
   gttctttttg tcaagaccga cctgtccggt gccctgaatg aactgcagga cgaggcagcg      660
   cggctatcgt ggctggccac gacgggcgtt ccttgcgcag ctgtgctcga cgttgtcact      720
   gaagcgggaa gggactggct gctattgggc gaagtgccgg ggcaggatct cctgtcatct      780
   caccttgctc ctgccgagaa agtatccatc atggctgatg caatgcggcg gctgcatacg      840
   cttgatccgg ctacctgccc attcgaccac caagcgaaac atcgcatcga gcgagcacgt      900
   actcggatgg aagccggtct tgtcgatcag gatgatctgg acgaagagca tcaggggctc      960
   gcgccagccg aactgttcgc caggctcaag gcgcgcatgc ccgacggcga ggatctcgtc     1020
   gtgacccatg gcgatgcctg cttgccgaat atcatggtgg aaaatggccg cttttctgga     1080
   ttcatcgact gtggccggct gggtgtggcg gaccgctatc aggacatagc gttggctacc     1140
   cgtgatattg ctgaagagct tggcggcgaa tgggctgacc gcttcctcgt gctttacggt     1200
   atcgccgctc ccgattcgca gcgcatcgcc ttctatcgcc ttcttgacga gttcttctga     1260
   actagtgcgg gactctgggg ttcgaaatga ccgaccaagc gacgcccaac ctgccatcac     1320
   gagatttcga ttccaccgcc gccttctatg aaaggttggg cttcggaatc gttttccggg     1380
   acgccggctg gatgatcctc cagcgcgggg atctcatgct ggagttcttc gcccacccca     1440
   acttgtttat tgcagcttat aatggttaca aataaagcaa tagcatcaca aatttcacaa     1500
   ataaagcatt tttttcactg cattctagtt gtggtttgtc caaactcatc aatgtatctt     1560
   atcatgtctg ttataccgtc gatctctagc tagagcttgg cgtaatcatg gtcatagctg     1620
   tttcctgtgt gcccacccca acttgtttat tgcagcttat aatggttaca aataaagcaa     1680
   tagcatcaca aatttcacaa ataaagcatt tttttcactg cattctagtt gtggtttgtc     1740
   caaactcatc aatgtatctt atcatgtctg ataacttcgt ataatgtatg ctatacgaac     1800
   ggtactcgag agctcatgcg gtaccgttta aacctggccc atcctctctg ttagatgtga     1860
   tcaaaactca taaaggctgt ccctgaggtt tctgtcccag gactgattgc ggaagagtca     1920
   ccaggcactg aggagacggt gaccagggtg ccctggcccc agagatcgaa gtaccagtag     1980
   gagttaccac cgtagtcttt cgcacagtaa tatacggccg tgtcctcggc tctcaggctg     2040
   ttcatttgca gatacagcgt gttcttggaa ttgtctctgg agatggtgaa ccgtcctgtg 2100
   aagccctggg catacgttgg gttcccagtg ttggtgttga tccatgagac ccactccagc 2160
   cccttccctg gagcctggcg gacccaattc atagcatagc tgctaaaggt gaatccagag 2220
   gctgcacagg agagtctcag ggacccccca ggctgtacca agcctccccc agactccaac 2280
   agctgcacct cgcgcgtctc ggatagtgtc ggtagagtat gctggcccat cctctctgtt 2340
   agatgtgatc aaaactcata aaggctgtcc ctgaggtttc tgtcccagga ctgattgcgg 2400
   aagagtcacc aggcactgag gagacggtga ccagggtgcc ctggccccag acgtccatac 2460
   cgtagtagta gtagtaaaag gcgtacgagc tggtactaca aacccctttc gcacagtaat 2520
   atacggccgt gtcctcggct ctcaggctgt tcatttgcag atacagcgtg ttcttggaat 2580
   tgtctctgga gatggtgaac cggccttgga aggacgggct gtatctggta tcagagtcac 2640
   caggatagat gattgagacc cactccagcc ccttccctgg agcctggcgg acccagccga 2700
   tccagtagct gctaaaggtg aatccagagg ctgcacagga gagtctcagg gaccccccag 2760
   gctgtaccaa gcctccccca gactccaaca gctgcacctc gcgactatat gtctatctat 2820
   gacatacact ctggcccatc ctctctgtta gatgtgatca aaactcataa aggctgtccc 2880
   tgaggtttct gtcccaggac tgattgcgga agagtcacca ggcactgagg agacggtgac 2940
   cagggtgccc tggccccaga aatcaaaagc accacccggg cccgcgctgg tacgggcttt 3000
   cgcacagtaa tatacggccg tgtcctcggc tctcaggctg ttcatttgca gatacagcgt 3060
   gttcttggaa ttgtctctgg agatggtgaa ccgactcttg agggacgggt tgtagttggt 3120
   gcttccacta tgattgattt ctgagaccca ctccagcccc ttccctggag cctggcggac 3180
   ccagctccag tagtaaccgc taaaggtgaa tccagaggct gcacaggaga gtctcaggga 3240
   ccccccaggc tgtaccaagc ctcccccaga ctccaacagc tgcacctcgt gacgctctgt 3300
   gcgcgagtgt agatgagtct ggcccatcct ctctgttaga tgtgatcaaa actcataaag 3360
   gctgtccctg aggtttctgt cccaggactg attgcggaag agtcaccagg cactgaggag 3420
   acggtgacca gggtgccctg gccccagtag tcaaaacccc ctctacttcg atctttcgca 3480
   cagtaatata cggccgtgtc ctcggctctc aggctgttca tttgcagata cagcgtgttc 3540
   ttggaattgt ctctggagat ggtgaaccga ctcttgaggg acgggttgta gtaggtgctc 3600
   ccactataat agatgtatga gacccactcc agccccttcc ctggagcctg gcggacccag 3660
   ccccaccagt tactactgct aaaggtgaat ccagaggctg cacaggagag tctcagggac 3720
   cccccaggct gtaccaagcc tcccccagac tccaacagct gcacctcgca cgactgtgcg 3780
   tatgtgttgc gacgacactg gcccatcctc tctgttagat gtgatcaaaa ctcataaagg 3840
   ctgtccctga ggtttctgtc ccaggactga ttgcggaaga gtcaccaggc actgaggaga 3900
   cggtgaccag ggtgccctgg ccccagatat caaaagcatc agcatagcag ctggtactac 3960
   tacaatatcc tttcgcacag taatatacgg ccgtgtcctc ggctctcagg ctgttcattt 4020
   gcagatacag cgtgttcttg gaattgtctc tggagatggt gaaccggccc ttcacggagt 4080
   ccgcgtagct tgtgctactc ccatcactat taatacgtga gacccactcc agccccttcc 4140
   ctggagcctg gcggacccag tgcatccagt agctgctaaa ggtgaatcca gaggctgcac 4200
   aggagagtct cagggacccc ccaggctgta ccaagcctcc cccagactcc aacagctgca 4260
   cctctgacgt cagatacgca gcgatcacta tcgactggcc catcctctct gttagatgtg 4320
   atcaaaactc ataaaggctg tccctgaggt ttctgtccca ggactgattg cggaagagtc 4380
   accaggcact gaggagacgg tgaccagggt gccctggccc cagtagtcaa agtaataact 4440
   ccctgaacca taggagaact tcgctttcgc acagtaatat acggccgtgt cctcggctct 4500
   caggctgttc atttgcagat acagcgtgtt cttggaattg tctctggaga tggtgaaccg 4560
   gcccttcacg gagtctgcgt agtatgtgct accaccgcta taaataactg agacccactc 4620
   cagccccttc cctggagcct ggcggaccca gctcatgtag ttgctgctaa aggtgaatcc 4680
   agaggctgca caggagagtc tcagggaccc cccaggctgt accaagcctc ccccagactc 4740
   caacagctgc acctctatgc gactatgatg acgtatacgc tatagctggc ccatcctctc 4800
   tgttagatgt gatcaaaact cataaaggct gtccctgagg tttctgtccc aggactgatt 4860
   gcggaagagt caccaggcac tgaggagacg gtgaccaggg tgccctggcc ccagtagtca 4920
   aaaggagtag cagctggccc gaagacaaga tctttcgcac agtaatatac ggccgtgtcc 4980
   tcggctctca ggctgttcat ttgcagatac agcgtgttct tggaattgtc tctggagatg 5040
   gtgaaccggc ccttcacgga gtctgcatag tatttattac ttccatcata ccatataact 5100
   gagacccact ccagcccctt ccctggagcc tggcggaccc agtgcatgcc atagctgcta 5160
   aaggtgaatc cagaggctgc acaggagagt ctcagggacc ccccaggctg taccaagcct 5220
   cccccagact ccaacagctg cacctctgta tacgcgtcgt actgcgtata cgatcgctgg 5280
   cccatcctct ctgttagatg tgatcaaaac tcataaaggc tgtccctgag gtttctgtcc 5340
   caggactgat tgcggaagag tcaccaggca ctgaggagac ggtgaccagg gtgccctggc 5400
   cccagtagtc aaaggacgag ctgctacccc tttctttcgc acagtaatat acggccgtgt 5460
   cctcggctct caggctgttc atttgcagat acagcgtgtt cttggaattg tctctggaga 5520
   tggtgaaccg gcctttcacg ggtgcagcgt agtctgttgt cccaccatca gttttgcttt 5580
   taatacgtga gacccactcc agccccttcc ctggagcctg gcggacccag ctcatccagg 5640
   cgttgctaaa ggtgaatcca gaggctgcac aggagagtct cagggacccc ccaggctgta 5700
   ccaagcctcc cccagactcc aacagctgca cctctacgtg agcttactgc tagttcgatc 5760
   gcgtctggcc catcctctct gttagatgtg atcaaaactc ataaaggctg tccctgaggt 5820
   ttctgtccca ggactgattg cggaagagtc accaggcact gaggagacgg tgaccagggt 5880
   gccctggccc caggggtcga accagttgta agtagctccc actattttcg cacagtaata 5940
   tacggccgtg tcctcggctc tcaggctgtt catttgcaga tacagcgtgt tcttggaatt 6000
   gtctctggag atggtgaacc ggcccttcac agagtccgca tagcctatgc taccactatt 6060
   ccaactaata cctgagaccc actccagccc cttccctgga gcctggcgga cccagtgcat 6120
   ggcataatcg ctaaaggtga atccagaggc tgcacaggag agtctcaggg accccccagg 6180
   ctgtaccaag cctcccccag actccaacag ctgcacctca tagtatagaa gtcgctagaa 6240
   ctagtgcgac tggcccatcc tctctgttag atgtgatcaa aactcataaa ggctgtccct 6300
   gaggtttctg tcccaggact gattgcggaa gagtcaccag gcactgagga gacggtgacc 6360
   agggtgccct ggccccagta gtcaaagaat tccccgggcg gggtgccacc gtcatctttc 6420
   gcacagtaat atacggccgt gtcctcggct ctcaggctgt tcatttgcag atacagcgtg 6480
   ttcttggaat tgtctctgga gatggtgaac cgggtcttca gagatgtgct gtagaattta 6540
   tcatcatccc aatcaatgcg tgagacccac tccagcccct tccctggagc ctggcggacc 6600
   cagctcacac gcattccact agtgctaaag gtgaatccag aggctgcaca ggagagtctc 6660
   agggaccccc caggctgtac caagcctccc ccagactcca acagctgcac ctcacgatct 6720
   gtaagtgtgt gtaagtcacg tcgctggccc atcctctctg ttagatgtga tcaaaactca 6780
   taaaggctgt ccctgaggtt tctgtcccag gactgattgc ggaagagtca ccaggcactg 6840
   aggagacggt gaccagggtg ccctggcccc agtagtcaaa ggtccaggcg ctgctatacg 6900
   gcccatacgc cccatcgtct ttcgcacagt aatatacggc cgtgtcctcg gctctcaggc 6960
   tgttcatttg cagatacagc gtgttcttgg aattgtctct ggagatggtg aaccggctct 7020
   tcagagatgg gctgtagcgc ttatcatcat tccaataaat gagtgagacc cactccagcc 7080
   ccttccctgg agcctggcgg acccagccca cacccactcc actagtgcta aaggtgaatc 7140
   cagaggctgc acaggagagt ctcagggacc ccccaggctg taccaagcct cccccagact 7200
   ccaacagctg cacctcatag cgtactagac gacgctagta tagtctctgg cccatcctct 7260
   ctgttagatg tgatcaaaac tcataaaggc tgtccctgag gtttctgtcc caggactgat 7320
   tgcggaagag tcaccaggca ctgaggagac ggtgaccagg gtgccctggc cccagacgtc 7380
   cataccgtag tagtagtagg gggcagctgg tactacaagg acgtctttcg cacagtaata 7440
   tacggccgtg tcctcggctc tcaggctgtt catttgcaga tacagcgtgt tcttggaatt 7500
   gtctctggag atggtgaacc gttcctggaa cttctgtgcg tagtttgtgt taccactgcc 7560
   aacgacgatc catgagaccc actccagccc cttccctgga gcctggcgga cccactgcac 7620
   agcagagctg ctaaaggtga atccagaggc tgcacaggag agtctcaggg accccccagg 7680
   ctgtaccaag cctcccccag actccaacag ctgcacctca cgcgtatgtc gagcgacgac 7740
   tcgtctctac tggcccatcc tctctgttag atgtgatcaa aactcataaa ggctgtccct 7800
   gaggtttctg tcccaggact gattgcggaa gagtcaccag gcactgagga gacggtgacc 7860
   agggtgccct ggccccagac gtccacaccg tagcgagctg gcacaacgac aatatcctcc 7920
   ccatatttcg cacagtaata tacggccgtg tcctcggctc tcaggctgtt catttgcaga 7980
   tacagcgtgt tcttggaatt gtctctggag atggtgaacc ggcccttcac ggagtctgcg 8040
   taatatgtgc taccagcact attactaata gttgagaccc actccagccc cttccctgga 8100
   gcctggcgga cccagttcat ggcaaagccg ctaaaggtga atccagaggc tgcacaggag 8160
   agtctcaggg accccccagg ctgtaccaag cctcccccag actccaacag ctgcacctcc 8220
   tgtagtcgtg agtagtacag ctacgtagac tggcccatcc tctctgttag atgtgatcaa 8280
   aactcataaa ggctgtccct gaggtttctg tcccaggact gattgcggaa gagtcaccag 8340
   gcactgagga gacggtgacc agggtgccct ggccccagta gtcaaagtag taataggata 8400
   accttaaatc tttcgcacag taatatacgg ccgtgtcctc ggctctcagg ctgttcattt 8460
   gcagatacag cgtgttcttg gaattgtctc tggagatggt gaaccggccc tggagcttct 8520
   gtgcatagtt tgtgttacca ttgtaagcgc tgatccatga gacccactcc agccccttcc 8580
   ctggagcctg gcggacccag ctgataccat agctgctaaa ggtgaatcca gaggctgcac 8640
   aggagagtct cagggacccc ccaggctgta ccaagcctcc cccagactcc aacagctgca 8700
   cctcgtatct ctcgcactac tatgagacgt atcactggcc catcctctct gttagatgtg 8760
   atcaaaactc ataaaggctg tccctgaggt ttctgtccca ggactgattg cggaagagtc 8820
   accaggcact gaggagacgg tgaccagggt gccctggccc cagtagtcaa agtggacgta 8880
   gtaataacca ctactagacg ctttcgcaca gtaatatacg gccgtgtcct cggctctcag 8940
   gctgttcatt tgcagataca gcgtgttctt ggaattgtct ctggagatgg tgaaccggcc 9000
   ctggaacttc tgtgaatatt ttgtgttacc attgccagcg ttgatccatg agacccactc 9060
   cagccccttc cctggagcct ggcggaccca atgcatagca tagctgctaa aggtgaatcc 9120
   agaggctgca caggagagtc tcagggaccc cccaggctgt accaagcctc ccccagactc 9180
   caacagctgc acctcctcgc gatatcgtgt ctgatctaca gtgctgttta aacgagctcg 9240
   ctagcggatc ccgcggatgc ggcgcgccat cgatatgcgt cgacatttaa atgggccccc 9300
   cggtgatatc ccgggtctta gagcggccgc taccgttcgt atattctatc ttatacgaag 9360
   ttatatcgaa ttcctgcaga gatctagctt ggcgtaatca tggtcatagc tgtttcctgt 9420
   gtgaaattgt tatccgctca caattccaca caacatacga gccggaagca taaagtgtaa 9480
   agcctggggt gcctaatgag tgagctaact cacattaatt gcgttgcgct cactgcccgc 9540
   tttccagtcg ggaaacctgt cgtgccagct gcattaatga atcggccaac gcgcggggag 9600
   aggcggtttg cgtattgggc gctcttccgc ttcctcgctc actgactcgc tgcgctcggt 9660
   cgttcggctg cggcgagcgg tatcagctca ctcaaaggcg gtaatacggt tatccacaga 9720
   atcaggggat aacgcaggaa agaacatgtg agcaaaaggc cagcaaaagg ccaggaaccg 9780
   taaaaaggcc gcgttgctgg cgtttttcca taggctccgc ccccctgacg agcatcacaa 9840
   aaatcgacgc tcaagtcaga ggtggcgaaa cccgacagga ctataaagat accaggcgtt 9900
   tccccctgga agctccctcg tgcgctctcc tgttccgacc ctgccgctta ccggatacct 9960
   gtccgccttt ctcccttcgg gaagcgtggc gctttctcat agctcacgct gtaggtatct 10020
   cagttcggtg taggtcgttc gctccaagct gggctgtgtg cacgaacccc ccgttcagcc 10080
   cgaccgctgc gccttatccg gtaactatcg tcttgagtcc aacccggtaa gacacgactt 10140
   atcgccactg gcagcagcca ctggtaacag gattagcaga gcgaggtatg taggcggtgc 10200
   tacagagttc ttgaagtggt ggcctaacta cggctacact agaagaacag tatttggtat 10260
   ctgcgctctg ctgaagccag ttaccttcgg aaaaagagtt ggtagctctt gatccggcaa 10320
   acaaaccacc gctggtagcg gtggtttttt tgtttgcaag cagcagatta cgcgcagaaa 10380
   aaaaggatct caagaagatc ctttgatctt ttctacgggg tctgacgctc agtggaacga 10440
   aaactcacgt taagggattt tggtcatgag attatcaaaa aggatcttca cctagatcct 10500
   tttaaattaa aaatgaagtt ttaaatcaat ctaaagtata tatgagtaaa cttggtctga 10560
   cagttaccaa tgcttaatca gtgaggcacc tatctcagcg atctgtctat ttcgttcatc 10620
   catagttgcc tgactccccg tcgtgtagat aactacgata cgggagggct taccatctgg 10680
   ccccagtgct gcaatgatac cgcgagaccc acgctcaccg gctccagatt tatcagcaat 10740
   aaaccagcca gccggaaggg ccgagcgcag aagtggtcct gcaactttat ccgcctccat 10800
   ccagtctatt aattgttgcc gggaagctag agtaagtagt tcgccagtta atagtttgcg 10860
   caacgttgtt gccattgcta caggcatcgt ggtgtcacgc tcgtcgtttg gtatggcttc 10920
   attcagctcc ggttcccaac gatcaaggcg agttacatga tcccccatgt tgtgcaaaaa 10980
   agcggttagc tccttcggtc ctccgatcgt tgtcagaagt aagttggccg cagtgttatc 11040
   actcatggtt atggcagcac tgcataattc tcttactgtc atgccatccg taagatgctt 11100
   ttctgtgact ggtgagtact caaccaagtc attctgagaa tagtgtatgc ggcgaccgag 11160
   ttgctcttgc ccggcgtcaa tacgggataa taccgcgcca catagcagaa ctttaaaagt 11220
   gctcatcatt ggaaaacgtt cttcggggcg aaaactctca aggatcttac cgctgttgag 11280
   atccagttcg atgtaaccca ctcgtgcacc caactgatct tcagcatctt ttactttcac 11340
   cagcgtttct gggtgagcaa aaacaggaag gcaaaatgcc gcaaaaaagg gaataagggc 11400
   gacacggaaa tgttgaatac tcatactctt cctttttcaa tattattgaa gcatttatca 11460
   gggttattgt ctcatgagcg gatacatatt tgaatgtatt tagaaaaata aacaaatagg 11520
   ggttccgcgc acatttcccc gaaaagtgcc acctgacgtc taagaaacca ttattatcat 11580
   gacattaacc tataaaaata ggcgtatcac gaggcccttt cgtc 11624
<210> 147
   <211> 339
   <212> DNA
   <213> Artificial Sequence
<220>
   <223> Synthetic DNA
<400> 147
   gaggtgcagc tgttggagtc tgggggaggc ttggtacagc ctggggggtc cctgagactc 60
   tcctgtgcag cctctggatt cacctttagc atctatgtca tgagctgggt ccgccaggct 120
   ccagggaagg ggctggagtg ggtctcagct attagtgcta gtggtgataa cacatactac 180
   gcagactccg tgaagggccg gttcaccatc tccagagaca attccaagaa cacgctgtat 240
   ctgcaaatga acagcctgag agccgaggac acggccgtat attactgtgc gaaaacccct 300
   acttactggg gccagggcac cctggtcacc gtctcctca 339
<210> 148
   <211> 354
   <212> DNA
   <213> Artificial Sequence
<220>
   <223> Synthetic DNA
<400> 148
   gaggtgcagc tgttggagtc tgggggaggc ttggtacagc ctggggggtc cctgagactc 60
   tcctgtgcag cctctggatt cacctttagc agctttttca tgaactgggt ccgccaggct 120
   ccagggaagg ggctggagtg ggtctcaact attagtgcca gtgatggtag gacacactac 180
   gcagactccg tgaagggccg gttcaccatc tccagagaca attccaagaa cacgctgtat 240
   ctgcaaatga acagcctgag agccgaggac acggccgtat attactgtgc gaaagatctc 300
   cactggtacg gtatggacgc ctggggccag ggcaccctgg tcaccgtctc ctca 354
<210> 149
   <211> 375
   <212> DNA
   <213> Artificial Sequence
<220>
   <223> Synthetic DNA
<400> 149
   gaggtgcagc tgttggagtc tgggggaggc ttggtacagc ctggggggtc cctgagactc 60
   tcctgtgcag cctctggatt cacctttagc agctttgaca tgagctgggt ccgccaggct 120
   ccagggaagg ggctggagtg ggtctcaagt attagtggta gtggtattag aacatattac 180
   gcagactccg tgaagggccg gttcaccatc tccagagaca attccaagaa cacgctgtat 240
   ctgcaaatga acagcctgag agccgaggac acggccgtat attactgtgc gaaagatggg 300
   gtgtatagca gtggccagta ctaccattac ggtatggacg tctggggcca gggcaccctg 360
   gtcaccgtct cctca 375
<210> 150
   <211> 387
   <212> DNA
   <213> Artificial Sequence
<220>
   <223> Synthetic DNA
<400> 150
   gaggtgcagc tgttggagtc tgggggaggc ttggtacagc ctggggggtc cctgagactc 60
   tcctgtgcag cctctggatt cacctttagc acctttgtca tgacgtgggt ccgccaggct 120
   ccagggaagg ggctggagtg ggtctcagct attactggta gtgggggtga cacatactac 180
   gcagactccg tgaagggccg gttcaccatc tccagagaca attccaagaa cacgctgtat 240
   ctgcaaatga acagcctgag agccgaggac acggccgtat attactgtgc gaaagatctc 300
   cacgtagaag atgttttggc tggagaagat gttttggctg ggggtctgga cgtctggggc 360
   cagggcaccc tggtcaccgt ctcctca 387
<210> 151
   <211> 363
   <212> DNA
   <213> Artificial Sequence
<220>
   <223> Synthetic DNA
<400> 151
   gaggtgcagc tgttggagtc tgggggaggc ttggtacagc ctggggggtc cctgagactc        60
   tcctgtgcag cctctggatt cacctttagc aagtacctta tgaggtgggt ccgccaggct       120
   ccagggaagg ggctggagtg ggtctcaagt attagtgaca gtggtgaagg cccaacctat       180
   tcagactccg tgaagggccg gttcaccatc tccagagaca attccaagaa cacgctgtat       240
   ctgcaaatga acagcctgag agccgaggac acggccgtat attactgtgc gaaatgcgat       300
   ggttcggggc gtgactgcgg tatggacgtc tggggccagg gcaccctggt caccgtctcc       360
   tca                                                                     363
<210> 152
   <211> 339
   <212> DNA
   <213> Artificial Sequence
<220>
   <223> Synthetic DNA
<400> 152
   gaggtgcagc tgttggagtc tgggggaggc ttggtacagc ctggggggtc cctgagactc        60
   tcctgtgcag cctctggatt cacctttagc aactatgcca tgacctgggt ccgccaggct       120
   ccagggaagg ggctggagtg ggtctcagct attagtaatt atggtggtag cacatactac       180
   tcagactccg tgaagggccg gttcaccatc tccagagaca attccaagaa cacgctgtat       240
   ctgcaaatga acagcctgag agccgaggac acggccgtat attactgtgc gaaatacttt       300
   gacttctggg gccagggcac cctggtcacc gtctcctca                              339
<210> 153
   <211> 381
   <212> DNA
   <213> Artificial Sequence
<220>
   <223> Synthetic DNA
<400> 153
   gaggtgcagc tgttggagtc tgggggaggc ttggtacagc ctggggggtc cctgagactc      60
   tcctgtgcag cctctggatt cacctttagc acctatgtca tgggctgggt ccgccaggct     120
   ccagggaagg ggctggagtg ggtctcaggt attagtggtc gtggtggtag cacatactac     180
   gcagactccg tgaagggccg gttcaccatc tccagagaca attccaagaa cacgctgtat     240
   ctgcaaatga acagcctgag agccgaggac acggccgtat attactgtgc gaaagacaac     300
   tcttacgatt tttggagagg ttactactac tactacggta tggacgtctg gggccagggc      360
   accctggtca ccgtctcctc a                                               381
<210> 154
   <211> 369
   <212> DNA
   <213> Artificial Sequence
<220>
   <223> Synthetic DNA
<400> 154
   gaggtgcagc tgttggagtc tgggggaggc ttggtacagc ctggggggtc cctgagactc      60
   tcctgtgcag cctctggatt cacctttagc agctattcca tgagctgggt ccgccaggct     120
   ccagggaagg ggctggagtg ggtctcagct attagtggca ctggtggtgc catatactac     180
   gcagactccg tgaagggccg gttcaccatc tccagagaca attccaagaa cacgctgtat     240
   ctgcaaatga acagcctgag agccgaggac acggccgtat attactgtgc gaaagggggt     300
   ctcaccaaat attgtagtgg tgattactgt cactattggg gccagggcac cctggtcacc     360
   gtctcctca                                                             369
<210> 155
   <211> 351
   <212> DNA
   <213> Artificial Sequence
<220>
   <223> Synthetic DNA
<400> 155
   gaggtgcagc tgttggagtc tgggggaggc ttggtacagc ctggggggtc cctgagactc 60
   tcctgtgcag cctctggatt cacctttagc aactttgcca tgacctgggt ccgccaggct 120
   ccagggaagg ggctggagtg ggtctcaact gttactagta gtggtgctgg cacatactac 180
   gcagactccg tgaagggccg gttcaccatc tccagagaca attccaagaa cacgctgtat 240
   ctgcaaatga acagcctgag agccgaggac acggccgtat attactgtgc gaaatctctt 300
   ggcagcagcc ttgactactg gggccagggc accctggtca ccgtctcctc a 351
<210> 156
   <211> 360
   <212> DNA
   <213> Artificial Sequence
<220>
   <223> Synthetic DNA
<400> 156
   gaggtgcagc tgttggagtc tgggggaggc ttggtacagc ctggggggtc cctgagactc 60
   tcctgtgcag cctctggatt cacctttagc aactttgacc tcagctgggt ccgccaggct 120
   ccagggaagg ggctggagtg ggtctcagct attcgtggta gcggtagtag cgctttctac 180
   gcagactccg tgcagggccg gttcaccatc tccagagaca attccaagaa cacgctgtat 240
   ctgcaaatga acagcctgag agccgaggac acggccgtat attactgtgc gaaattatcc 300
   tggcattact ctggttgggg gaatccttgg ggccagggca ccctggtcac cgtctcctca 360
<210> 157
   <211> 357
   <212> DNA
   <213> Artificial Sequence
<220>
   <223> Synthetic DNA
<400> 157
   gaggtgcagc tgttggagtc tgggggaggc ttggtacagc ctggggggtc cctgagactc 60
   tcctgtgcag cctctggatt cacctttagc aactttggca tggactgggt ccgccaggct 120
   ccagggaagg ggctggagtg ggtctcaact attactgtca gtggtgaaaa cacatattac 180
   gcagactccg tgaagggccg gttcaccatc tccagagaca attccaagaa cacgctgtat 240
   ctgcaaatga acagcctgag agccgaggac acggccgtat attactgtgc gaaaggcttt 300
   agcagtggct ggtcatttga ctactggggc cagggcaccc tggtcaccgt ctcctca 357
<210> 158
   <211> 363
   <212> DNA
   <213> Artificial Sequence
<220>
   <223> Synthetic DNA
<400> 158
   gaggtgcagc tgttggagtc tgggggaggc ttggtacagc ctggggggtc cctgagactc 60
   tcctgtgcag cctctggatt cacctttagc agttatgccc tgagctgggt ccgccaggct 120
   ccagggaagg ggctggagtg ggtctcactt gttagtggta gtgctattag tacatactac 180
   gcagactccg tgaagggccg gttcaccatc tccagagaca attccaagaa cacgctgtat 240
   ctgcaaatga acagcctgag agccgaggac acggccgtat attactgtgc gaaaggggct 300
   cgagcccacc tctactacga tatggacgtc tggggccagg gcaccctggt caccgtctcc 360
   tca 363
<210> 159
   <211> 360
   <212> DNA
   <213> Artificial Sequence
<220>
   <223> Synthetic DNA
<400> 159
   gaggtgcagc tgttggagtc tgggggaggc ttggtacagc ctggggggtc cctgagactc 60
   tcctgtgcag cctctggatt cacctttagc acctttttca tgagctgggt ccgccaggct 120
   ccagggaagg ggctggagtg ggtctcaact aatactggtg atggtggtag tacatactac 180
   tcagactccg tgaagggccg gttcaccatc tccagagaca attccaagaa cacgctgtat 240
   ctgcaaatga acagcctgag agccgaggac acggccgtat attactgtgc gaaaactact 300
   atggttcggg gagttatgct tgactactgg ggccagggca ccctggtcac cgtctcctca 360
<210> 160
   <211> 375
   <212> DNA
   <213> Artificial Sequence
<220>
   <223> Synthetic DNA
<400> 160
   gaggtgcagc tgttggagtc tgggggaggc ttggtacagc ctggggggtc cctgagactc 60
   tcctgtgcag cctctggatt cacctttagc agatatgcca tgggctgggt ccgccaggct 120
   ccagggaagg ggctggagtg ggtctcaagt atgagtgcta gtgctggcag cacatactac 180
   gcagactccg tgaggggccg gttcaccatc tccagagaca attccaagaa cacgctgtat 240
   ctgcaaatga acagcctgag agccgaggac acggccgtat attactgtgc gaaagatctg 300
   gttcacgcgg atatagtggc cctggggtgt ggccctgact actggggcca gggcaccctg 360
   gtcaccgtct cctca 375
<210> 161
   <211> 381
   <212> DNA
   <213> Artificial Sequence
<220>
   <223> Synthetic DNA
<400> 161
   gaggtgcagc tgttggagtc tgggggaggc ttggtacagc ctggggggtc cctgagactc 60
   tcctgtgcag cctctggatt cacctttagc aactttgcca taagctgggt ccgccaggct 120
   ccagggaagg ggctggagtg ggtctcaggt atcagtggta ctggtggtag gacatactac 180
   gcagactccg tgaagggccg gttcaccatc tccagagaca attccaagaa cacgctgtat 240
   ctgcaaatga acagcctgag agccgaggac acggccgtat attactgtgc gaaaggggtc 300
   gatgctacat atagtgccac ctggtacgtg gggggctact atgagtattg gggccagggc 360
   accctggtca ccgtctcctc a 381
<210> 162
   <211> 6974
   <212> DNA
   <213> Artificial Sequence
<220>
   <223> Synthetic DNA
<400> 162
   ccgcggggcg cgccgaggtg cagctgttgg agtctggggg aggcttggta cagcctgggg 60
   ggtccctgag actctcctgt gcagcctctg gattcacctt tagcatctat gtcatgagct 120
   gggtccgcca ggctccaggg aaggggctgg agtgggtctc agctattagt gctagtggtg 180
   ataacacata ctacgcagac tccgtgaagg gccggttcac catctccaga gacaattcca 240
   agaacacgct gtatctgcaa atgaacagcc tgagagccga ggacacggcc gtatattact 300
   gtgcgaaaac ccctacttac tggggccagg gcaccctggt caccgtctcc tcagatgaga 360
   tttgatagaa acctcattga gaccccgctg atgaccccat gagaccacat agagacccta 420
   cagagaccca gagaggtcag agcctgcctg gaggaggtgc agctgttgga gtctggggga 480
   ggcttggtac agcctggggg gtccctgaga ctctcctgtg cagcctctgg attcaccttt 540
   agcagctttt tcatgaactg ggtccgccag gctccaggga aggggctgga gtgggtctca 600
   actattagtg ccagtgatgg taggacacac tacgcagact ccgtgaaggg ccggttcacc 660
   atctccagag acaattccaa gaacacgctg tatctgcaaa tgaacagcct gagagccgag 720
   gacacggccg tatattactg tgcgaaagat ctccactggt acggtatgga cgcctggggc 780
   cagggcaccc tggtcaccgt ctcctcatgg gctctgtcac cgtcatcagc accactgcca 840
   tagcctgaga tgtcatagag agccatagag acaccactga aaccccataa aggccctgta 900
   gggacccgag gtgcagctgt tggagtctgg gggaggcttg gtacagcctg gggggtccct 960
   gagactctcc tgtgcagcct ctggattcac ctttagcagc tttgacatga gctgggtccg 1020
   ccaggctcca gggaaggggc tggagtgggt ctcaagtatt agtggtagtg gtattagaac 1080
   atattacgca gactccgtga agggccggtt caccatctcc agagacaatt ccaagaacac 1140
   gctgtatctg caaatgaaca gcctgagagc cgaggacacg gccgtatatt actgtgcgaa 1200
   agatggggtg tatagcagtg gccagtacta ccattacggt atggacgtct ggggccaggg 1260
   caccctggtc accgtctcct cacgccatca gccctgtggg gaaggacaga gagcgctgac 1320
   ggagccgccg ccacacaacc tcgtggagct gggcctcata aaacccccag agacccccag 1380
   aggaggtgca gctgttggag tctgggggag gcttggtaca gcctgggggg tccctgagac 1440
   tctcctgtgc agcctctgga ttcaccttta gcacctttgt catgacgtgg gtccgccagg 1500
   ctccagggaa ggggctggag tgggtctcag ctattactgg tagtgggggt gacacatact 1560
   acgcagactc cgtgaagggc cggttcacca tctccagaga caattccaag aacacgctgt 1620
   atctgcaaat gaacagcctg agagccgagg acacggccgt atattactgt gcgaaagatc 1680
   tccacgtaga agatgttttg gctggagaag atgttttggc tgggggtctg gacgtctggg 1740
   gccagggcac cctggtcacc gtctcctcag cggggttcgg acacgaccga aaggactcgt 1800
   ggtttcctgc cccgaaatag ctctgatgtg gggacagctg gaggtgttct gcagctgggc 1860
   tgtggtcgcg aggtgcagct gttggagtct gggggaggct tggtacagcc tggggggtcc 1920
   ctgagactct cctgtgcagc ctctggattc acctttagca agtaccttat gaggtgggtc 1980
   cgccaggctc cagggaaggg gctggagtgg gtctcaagta ttagtgacag tggtgaaggc 2040
   ccaacctatt cagactccgt gaagggccgg ttcaccatct ccagagacaa ttccaagaac 2100
   acgctgtatc tgcaaatgaa cagcctgaga gccgaggaca cggccgtata ttactgtgcg 2160
   aaatgcgatg gttcggggcg tgactgcggt atggacgtct ggggccaggg caccctggtc 2220
   accgtctcct cagagcacga aaataggatg gaaaggggag aaaaaaataa aacatcgact 2280
   gaaaggggcc aaagagagca tcggccgcag ctgctccatc tgctggcacg gcgaggtgca 2340
   gctgttggag tctgggggag gcttggtaca gcctgggggg tccctgagac tctcctgtgc 2400
   agcctctgga ttcaccttta gcaactatgc catgacctgg gtccgccagg ctccagggaa 2460
   ggggctggag tgggtctcag ctattagtaa ttatggtggt agcacatact actcagactc 2520
   cgtgaagggc cggttcacca tctccagaga caattccaag aacacgctgt atctgcaaat 2580
   gaacagcctg agagccgagg acacggccgt atattactgt gcgaaatact ttgacttctg 2640
   gggccagggc accctggtca ccgtctcctc aggggtctgc acgtctcaga agaggcacaa 2700
   cccgctccac agctcgctgc aggggtggtg gcatcccctg ctgaccccac gcagctgcac 2760
   tggctgcgaa agaggtgcag ctgttggagt ctgggggagg cttggtacag cctggggggt 2820
   ccctgagact ctcctgtgca gcctctggat tcacctttag cacctatgtc atgggctggg 2880
   tccgccaggc tccagggaag gggctggagt gggtctcagg tattagtggt cgtggtggta 2940
   gcacatacta cgcagactcc gtgaagggcc ggttcaccat ctccagagac aattccaaga 3000
   acacgctgta tctgcaaatg aacagcctga gagccgagga cacggccgta tattactgtg 3060
   cgaaagacaa ctcttacgat ttttggagag gttactacta ctactacggt atggacgtct 3120
   ggggccaggg caccctggtc accgtctcct caggtctctc ttatgaaccg ggcctgaaga 3180
   ccacccatag gacgcacatg ctgtgatggt cacgtgtagg aatgctgccc cgcaccaaca 3240
   gggtgtcctt atgaggtgca gctgttggag tctgggggag gcttggtaca gcctgggggg 3300
   tccctgagac tctcctgtgc agcctctgga ttcaccttta gcagctattc catgagctgg 3360
   gtccgccagg ctccagggaa ggggctggag tgggtctcag ctattagtgg cactggtggt 3420
   gccatatact acgcagactc cgtgaagggc cggttcacca tctccagaga caattccaag 3480
   aacacgctgt atctgcaaat gaacagcctg agagccgagg acacggccgt atattactgt 3540
   gcgaaagggg gtctcaccaa atattgtagt ggtgattact gtcactattg gggccagggc 3600
   accctggtca ccgtctcctc atctaaatgg tctttacgtg gtttcagtca catctctatg 3660
   gctctctatg acatctcagt ggggtctctt tgtggtcgga gtgtggtccg gctccgcgag 3720
   ggaggtgcag ctgttggagt ctgggggagg cttggtacag cctggggggt ccctgagact 3780
   ctcctgtgca gcctctggat tcacctttag caactttgcc atgacctggg tccgccaggc 3840
   tccagggaag gggctggagt gggtctcaac tgttactagt agtggtgctg gcacatacta 3900
   cgcagactcc gtgaagggcc ggttcaccat ctccagagac aattccaaga acacgctgta 3960
   tctgcaaatg aacagcctga gagccgagga cacggccgta tattactgtg cgaaatctct 4020
   tggcagcagc cttgactact ggggccaggg caccctggtc accgtctcct cactctatga 4080
   gatctcagtg atgtctcgaa gggaattcaa cgcaatctat atgagatctc ttcggagtcg 4140
   caatgaggtc tctattagaa ctctatgggg tcgaggtgca gctgttggag tctgggggag 4200
   gcttggtaca gcctgggggg tccctgagac tctcctgtgc agcctctgga ttcaccttta 4260
   gcaactttga cctcagctgg gtccgccagg ctccagggaa ggggctggag tgggtctcag 4320
   ctattcgtgg tagcggtagt agcgctttct acgcagactc cgtgcagggc cggttcacca 4380
   tctccagaga caattccaag aacacgctgt atctgcaaat gaacagcctg agagccgagg 4440
   acacggccgt atattactgt gcgaaattat cctggcatta ctctggttgg gggaatcctt 4500
   ggggccaggg caccctggtc accgtctcct caggtgggct tcggcccttt ccggggtctt 4560
   tagggcatct acggggtctc actgtgttct cagtggggtc tctatgaggt gttgactgaa 4620
   cctcatcggg atgaggtgca gctgttggag tctgggggag gcttggtaca gcctgggggg 4680
   tccctgagac tctcctgtgc agcctctgga ttcaccttta gcaactttgg catggactgg 4740
   gtccgccagg ctccagggaa ggggctggag tgggtctcaa ctattactgt cagtggtgaa 4800
   aacacatatt acgcagactc cgtgaagggc cggttcacca tctccagaga caattccaag 4860
   aacacgctgt atctgcaaat gaacagcctg agagccgagg acacggccgt atattactgt 4920
   gcgaaaggct ttagcagtgg ctggtcattt gactactggg gccagggcac cctggtcacc 4980
   gtctcctcag ctctcctaag aacagaacaa gccctgatct ccttcccccc gtgatttcgc 5040
   caccatcagc cctcatagct cccatctgct cctcttggct gccccgccag aggtgcagct 5100
   gttggagtct gggggaggct tggtacagcc tggggggtcc ctgagactct cctgtgcagc 5160
   ctctggattc acctttagca gttatgccct gagctgggtc cgccaggctc cagggaaggg 5220
   gctggagtgg gtctcacttg ttagtggtag tgctattagt acatactacg cagactccgt 5280
   gaagggccgg ttcaccatct ccagagacaa ttccaagaac acgctgtatc tgcaaatgaa 5340
   cagcctgaga gccgaggaca cggccgtata ttactgtgcg aaaggggctc gagcccacct 5400
   ctactacgat atggacgtct ggggccaggg caccctggtc accgtctcct cagctgagag 5460
   aggggatggg ctccgtgccc acattggggc tcagctccac gctgctgctg tatggagccg 5520
   tgcgatgtgg ggctgtgctg ggcacggaca cggaggtgca gctgttggag tctgggggag 5580
   gcttggtaca gcctgggggg tccctgagac tctcctgtgc agcctctgga ttcaccttta 5640
   gcaccttttt catgagctgg gtccgccagg ctccagggaa ggggctggag tgggtctcaa 5700
   ctaatactgg tgatggtggt agtacatact actcagactc cgtgaagggc cggttcacca 5760
   tctccagaga caattccaag aacacgctgt atctgcaaat gaacagcctg agagccgagg 5820
   acacggccgt atattactgt gcgaaaacta ctatggttcg gggagttatg cttgactact 5880
   ggggccaggg caccctggtc accgtctcct caatgcagag ctgtgtgctg ggacgaagaa 5940
   gtggagctgt gcgtggaagc acacaacggc tgagttctga agagaccctc tgtcctctcg 6000
   tccccccgct gtgaggtgca gctgttggag tctgggggag gcttggtaca gcctgggggg 6060
   tccctgagac tctcctgtgc agcctctgga ttcaccttta gcagatatgc catgggctgg 6120
   gtccgccagg ctccagggaa ggggctggag tgggtctcaa gtatgagtgc tagtgctggc 6180
   agcacatact acgcagactc cgtgaggggc cggttcacca tctccagaga caattccaag      6240
   aacacgctgt atctgcaaat gaacagcctg agagccgagg acacggccgt atattactgt      6300
   gcgaaagatc tggttcacgc ggatatagtg gccctggggt gtggccctga ctactggggc      6360
   cagggcaccc tggtcaccgt ctcctcagac cccattgatg tcccattgat accccagacg      6420
   gactccactg aagctcaagc gaggccgggg cgcccctgga ggggcagcca agatctcctt      6480
   ccctcccgag gtgcagctgt tggagtctgg gggaggcttg gtacagcctg gggggtccct      6540
   gagactctcc tgtgcagcct ctggattcac ctttagcaac tttgccataa gctgggtccg      6600
   ccaggctcca gggaaggggc tggagtgggt ctcaggtatc agtggtactg gtggtaggac      6660
   atactacgca gactccgtga agggccggtt caccatctcc agagacaatt ccaagaacac      6720
   gctgtatctg caaatgaaca gcctgagagc cgaggacacg gccgtatatt actgtgcgaa      6780
   aggggtcgat gctacatata gtgccacctg gtacgtgggg ggctactatg agtattgggg      6840
   ccagggcacc ctggtcaccg tctcctcaac accctttact tacatcggtc agatctaata      6900
   aagaccacat tcagatccca tgcaaagcgc attgatgccc ctcagagacc ccacagagtc      6960
   ccctcagaat cgat                                                        6974
<210> 163
   <211> 10804
   <212> DNA
   <213> Artificial Sequence
<220>
   <223> Synthetic DNA
<400> 163
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt gacgcgtatt gggatggaac 420
   catatgtagc ttacgacact gataacttcg tatatggtat tatatacgaa gttataagct 480
   tcaccatggc caagcctttg tctcaagaag aatccaccct cattgaaaga gcaacggcta 540
   caatcaacag catccccatc tctgaagact acagcgtcgc cagcgcagct ctctctagcg 600
   acggccgcat cttcactggt gtcaatgtat atcattttac tgggggacct tgtgcagaac 660
   tcgtggtgct gggcactgct gctgctgcgg cagctggcaa cctgacttgt atcgtcgcga 720
   tcggaaatga gaacaggggc atcttgagcc cctgcggacg gtgtcgacag gtgcttctcg 780
   atctgcatcc tgggatcaaa gcgatagtga aggacagtga tggacagccg acggcagttg 840
   ggattcgtga attgctgccc tctggttatg tgtgggaggg ctaaactagt gcacttcgtg 900
   gccgaggagc aggactgaca cgtgctacga gatttcgatt ccaccgccgc cttctatgaa 960
   aggttgggct tcggaatcgt tttccgggac gccggctgga tgatcctcca gcgcggggat 1020
   ctcatgctgg agttcttcgc ccaccccaac ttgtttattg cagcttataa tggttacaaa 1080
   taaagcaata gcatcacaaa tttcacaaat aaagcatttt tttcactgca ttctagttgt 1140
   ggtttgtcca aactcatcaa tgtatcttat catgtctgta taccgtcgac ctctagctag 1200
   agcttggcgt aatcatggtc atagctgttt cctgtgtgcc caccccaact tgtttattgc 1260
   agcttataat ggttacaaat aaagcaatag catcacaaat ttcacaaata aagcattttt 1320
   ttcactgcat tctagttgtg gtttgtccaa actcatcaat gtatcttatc atgtctgata 1380
   acttcgtata ttctatctta tacgaacggt actcgagagc tcatgcggta ccgctagcat 1440
   gctggccagg atccatgccc atggccgcgg ggcgcgccga ggtgcagctg ttggagtctg 1500
   ggggaggctt ggtacagcct ggggggtccc tgagactctc ctgtgcagcc tctggattca 1560
   cctttagcat ctatgtcatg agctgggtcc gccaggctcc agggaagggg ctggagtggg 1620
   tctcagctat tagtgctagt ggtgataaca catactacgc agactccgtg aagggccggt 1680
   tcaccatctc cagagacaat tccaagaaca cgctgtatct gcaaatgaac agcctgagag 1740
   ccgaggacac ggccgtatat tactgtgcga aaacccctac ttactggggc cagggcaccc 1800
   tggtcaccgt ctcctcagat gagatttgat agaaacctca ttgagacccc gctgatgacc 1860
   ccatgagacc acatagagac cctacagaga cccagagagg tcagagcctg cctggaggag 1920
   gtgcagctgt tggagtctgg gggaggcttg gtacagcctg gggggtccct gagactctcc 1980
   tgtgcagcct ctggattcac ctttagcagc tttttcatga actgggtccg ccaggctcca 2040
   gggaaggggc tggagtgggt ctcaactatt agtgccagtg atggtaggac acactacgca 2100
   gactccgtga agggccggtt caccatctcc agagacaatt ccaagaacac gctgtatctg 2160
   caaatgaaca gcctgagagc cgaggacacg gccgtatatt actgtgcgaa agatctccac 2220
   tggtacggta tggacgcctg gggccagggc accctggtca ccgtctcctc atgggctctg 2280
   tcaccgtcat cagcaccact gccatagcct gagatgtcat agagagccat agagacacca 2340
   ctgaaacccc ataaaggccc tgtagggacc cgaggtgcag ctgttggagt ctgggggagg 2400
   cttggtacag cctggggggt ccctgagact ctcctgtgca gcctctggat tcacctttag 2460
   cagctttgac atgagctggg tccgccaggc tccagggaag gggctggagt gggtctcaag 2520
   tattagtggt agtggtatta gaacatatta cgcagactcc gtgaagggcc ggttcaccat 2580
   ctccagagac aattccaaga acacgctgta tctgcaaatg aacagcctga gagccgagga 2640
   cacggccgta tattactgtg cgaaagatgg ggtgtatagc agtggccagt actaccatta 2700
   cggtatggac gtctggggcc agggcaccct ggtcaccgtc tcctcacgcc atcagccctg 2760
   tggggaagga cagagagcgc tgacggagcc gccgccacac aacctcgtgg agctgggcct 2820
   cataaaaccc ccagagaccc ccagaggagg tgcagctgtt ggagtctggg ggaggcttgg 2880
   tacagcctgg ggggtccctg agactctcct gtgcagcctc tggattcacc tttagcacct 2940
   ttgtcatgac gtgggtccgc caggctccag ggaaggggct ggagtgggtc tcagctatta 3000
   ctggtagtgg gggtgacaca tactacgcag actccgtgaa gggccggttc accatctcca 3060
   gagacaattc caagaacacg ctgtatctgc aaatgaacag cctgagagcc gaggacacgg 3120
   ccgtatatta ctgtgcgaaa gatctccacg tagaagatgt tttggctgga gaagatgttt 3180
   tggctggggg tctggacgtc tggggccagg gcaccctggt caccgtctcc tcagcggggt 3240
   tcggacacga ccgaaaggac tcgtggtttc ctgccccgaa atagctctga tgtggggaca 3300
   gctggaggtg ttctgcagct gggctgtggt cgcgaggtgc agctgttgga gtctggggga 3360
   ggcttggtac agcctggggg gtccctgaga ctctcctgtg cagcctctgg attcaccttt 3420
   agcaagtacc ttatgaggtg ggtccgccag gctccaggga aggggctgga gtgggtctca 3480
   agtattagtg acagtggtga aggcccaacc tattcagact ccgtgaaggg ccggttcacc 3540
   atctccagag acaattccaa gaacacgctg tatctgcaaa tgaacagcct gagagccgag 3600
   gacacggccg tatattactg tgcgaaatgc gatggttcgg ggcgtgactg cggtatggac 3660
   gtctggggcc agggcaccct ggtcaccgtc tcctcagagc acgaaaatag gatggaaagg 3720
   ggagaaaaaa ataaaacatc gactgaaagg ggccaaagag agcatcggcc gcagctgctc 3780
   catctgctgg cacggcgagg tgcagctgtt ggagtctggg ggaggcttgg tacagcctgg 3840
   ggggtccctg agactctcct gtgcagcctc tggattcacc tttagcaact atgccatgac 3900
   ctgggtccgc caggctccag ggaaggggct ggagtgggtc tcagctatta gtaattatgg 3960
   tggtagcaca tactactcag actccgtgaa gggccggttc accatctcca gagacaattc 4020
   caagaacacg ctgtatctgc aaatgaacag cctgagagcc gaggacacgg ccgtatatta 4080
   ctgtgcgaaa tactttgact tctggggcca gggcaccctg gtcaccgtct cctcaggggt 4140
   ctgcacgtct cagaagaggc acaacccgct ccacagctcg ctgcaggggt ggtggcatcc 4200
   cctgctgacc ccacgcagct gcactggctg cgaaagaggt gcagctgttg gagtctgggg 4260
   gaggcttggt acagcctggg gggtccctga gactctcctg tgcagcctct ggattcacct 4320
   ttagcaccta tgtcatgggc tgggtccgcc aggctccagg gaaggggctg gagtgggtct 4380
   caggtattag tggtcgtggt ggtagcacat actacgcaga ctccgtgaag ggccggttca 4440
   ccatctccag agacaattcc aagaacacgc tgtatctgca aatgaacagc ctgagagccg 4500
   aggacacggc cgtatattac tgtgcgaaag acaactctta cgatttttgg agaggttact 4560
   actactacta cggtatggac gtctggggcc agggcaccct ggtcaccgtc tcctcaggtc 4620
   tctcttatga accgggcctg aagaccaccc ataggacgca catgctgtga tggtcacgtg 4680
   taggaatgct gccccgcacc aacagggtgt ccttatgagg tgcagctgtt ggagtctggg 4740
   ggaggcttgg tacagcctgg ggggtccctg agactctcct gtgcagcctc tggattcacc 4800
   tttagcagct attccatgag ctgggtccgc caggctccag ggaaggggct ggagtgggtc 4860
   tcagctatta gtggcactgg tggtgccata tactacgcag actccgtgaa gggccggttc 4920
   accatctcca gagacaattc caagaacacg ctgtatctgc aaatgaacag cctgagagcc 4980
   gaggacacgg ccgtatatta ctgtgcgaaa gggggtctca ccaaatattg tagtggtgat 5040
   tactgtcact attggggcca gggcaccctg gtcaccgtct cctcatctaa atggtcttta 5100
   cgtggtttca gtcacatctc tatggctctc tatgacatct cagtggggtc tctttgtggt 5160
   cggagtgtgg tccggctccg cgagggaggt gcagctgttg gagtctgggg gaggcttggt 5220
   acagcctggg gggtccctga gactctcctg tgcagcctct ggattcacct ttagcaactt 5280
   tgccatgacc tgggtccgcc aggctccagg gaaggggctg gagtgggtct caactgttac 5340
   tagtagtggt gctggcacat actacgcaga ctccgtgaag ggccggttca ccatctccag 5400
   agacaattcc aagaacacgc tgtatctgca aatgaacagc ctgagagccg aggacacggc 5460
   cgtatattac tgtgcgaaat ctcttggcag cagccttgac tactggggcc agggcaccct 5520
   ggtcaccgtc tcctcactct atgagatctc agtgatgtct cgaagggaat tcaacgcaat 5580
   ctatatgaga tctcttcgga gtcgcaatga ggtctctatt agaactctat ggggtcgagg 5640
   tgcagctgtt ggagtctggg ggaggcttgg tacagcctgg ggggtccctg agactctcct 5700
   gtgcagcctc tggattcacc tttagcaact ttgacctcag ctgggtccgc caggctccag 5760
   ggaaggggct ggagtgggtc tcagctattc gtggtagcgg tagtagcgct ttctacgcag 5820
   actccgtgca gggccggttc accatctcca gagacaattc caagaacacg ctgtatctgc 5880
   aaatgaacag cctgagagcc gaggacacgg ccgtatatta ctgtgcgaaa ttatcctggc 5940
   attactctgg ttgggggaat ccttggggcc agggcaccct ggtcaccgtc tcctcaggtg 6000
   ggcttcggcc ctttccgggg tctttagggc atctacgggg tctcactgtg ttctcagtgg 6060
   ggtctctatg aggtgttgac tgaacctcat cgggatgagg tgcagctgtt ggagtctggg 6120
   ggaggcttgg tacagcctgg ggggtccctg agactctcct gtgcagcctc tggattcacc 6180
   tttagcaact ttggcatgga ctgggtccgc caggctccag ggaaggggct ggagtgggtc 6240
   tcaactatta ctgtcagtgg tgaaaacaca tattacgcag actccgtgaa gggccggttc 6300
   accatctcca gagacaattc caagaacacg ctgtatctgc aaatgaacag cctgagagcc 6360
   gaggacacgg ccgtatatta ctgtgcgaaa ggctttagca gtggctggtc atttgactac 6420
   tggggccagg gcaccctggt caccgtctcc tcagctctcc taagaacaga acaagccctg 6480
   atctccttcc ccccgtgatt tcgccaccat cagccctcat agctcccatc tgctcctctt 6540
   ggctgccccg ccagaggtgc agctgttgga gtctggggga ggcttggtac agcctggggg 6600
   gtccctgaga ctctcctgtg cagcctctgg attcaccttt agcagttatg ccctgagctg 6660
   ggtccgccag gctccaggga aggggctgga gtgggtctca cttgttagtg gtagtgctat 6720
   tagtacatac tacgcagact ccgtgaaggg ccggttcacc atctccagag acaattccaa 6780
   gaacacgctg tatctgcaaa tgaacagcct gagagccgag gacacggccg tatattactg 6840
   tgcgaaaggg gctcgagccc acctctacta cgatatggac gtctggggcc agggcaccct 6900
   ggtcaccgtc tcctcagctg agagagggga tgggctccgt gcccacattg gggctcagct 6960
   ccacgctgct gctgtatgga gccgtgcgat gtggggctgt gctgggcacg gacacggagg 7020
   tgcagctgtt ggagtctggg ggaggcttgg tacagcctgg ggggtccctg agactctcct 7080
   gtgcagcctc tggattcacc tttagcacct ttttcatgag ctgggtccgc caggctccag 7140
   ggaaggggct ggagtgggtc tcaactaata ctggtgatgg tggtagtaca tactactcag 7200
   actccgtgaa gggccggttc accatctcca gagacaattc caagaacacg ctgtatctgc 7260
   aaatgaacag cctgagagcc gaggacacgg ccgtatatta ctgtgcgaaa actactatgg 7320
   ttcggggagt tatgcttgac tactggggcc agggcaccct ggtcaccgtc tcctcaatgc 7380
   agagctgtgt gctgggacga agaagtggag ctgtgcgtgg aagcacacaa cggctgagtt 7440
   ctgaagagac cctctgtcct ctcgtccccc cgctgtgagg tgcagctgtt ggagtctggg 7500
   ggaggcttgg tacagcctgg ggggtccctg agactctcct gtgcagcctc tggattcacc 7560
   tttagcagat atgccatggg ctgggtccgc caggctccag ggaaggggct ggagtgggtc 7620
   tcaagtatga gtgctagtgc tggcagcaca tactacgcag actccgtgag gggccggttc 7680
   accatctcca gagacaattc caagaacacg ctgtatctgc aaatgaacag cctgagagcc 7740
   gaggacacgg ccgtatatta ctgtgcgaaa gatctggttc acgcggatat agtggccctg 7800
   gggtgtggcc ctgactactg gggccagggc accctggtca ccgtctcctc agaccccatt 7860
   gatgtcccat tgatacccca gacggactcc actgaagctc aagcgaggcc ggggcgcccc 7920
   tggaggggca gccaagatct ccttccctcc cgaggtgcag ctgttggagt ctgggggagg 7980
   cttggtacag cctggggggt ccctgagact ctcctgtgca gcctctggat tcacctttag 8040
   caactttgcc ataagctggg tccgccaggc tccagggaag gggctggagt gggtctcagg 8100
   tatcagtggt actggtggta ggacatacta cgcagactcc gtgaagggcc ggttcaccat 8160
   ctccagagac aattccaaga acacgctgta tctgcaaatg aacagcctga gagccgagga 8220
   cacggccgta tattactgtg cgaaaggggt cgatgctaca tatagtgcca cctggtacgt 8280
   ggggggctac tatgagtatt ggggccaggg caccctggtc accgtctcct caacaccctt 8340
   tacttacatc ggtcagatct aataaagacc acattcagat cccatgcaaa gcgcattgat 8400
   gcccctcaga gaccccacag agtcccctca gaatcgatat gcatttaaat gggccccccg 8460
   gtgatatccc cgggtctaga gcggccgcta ccgttcgtat aatgtatgct atacgaagtt 8520
   atatcgaatt cctgcagaga tctgttccat cccaatggcg cgccgagctt ggcgtaatca 8580
   tggtcatagc tgtttcctgt gtgaaattgt tatccgctca caattccaca caacatacga 8640
   gccggaagca taaagtgtaa agcctggggt gcctaatgag tgagctaact cacattaatt 8700
   gcgttgcgct cactgcccgc tttccagtcg ggaaacctgt cgtgccagct gcattaatga 8760
   atcggccaac gcgcggggag aggcggtttg cgtattgggc gctcttccgc ttcctcgctc 8820
   actgactcgc tgcgctcggt cgttcggctg cggcgagcgg tatcagctca ctcaaaggcg 8880
   gtaatacggt tatccacaga atcaggggat aacgcaggaa agaacatgtg agcaaaaggc 8940
   cagcaaaagg ccaggaaccg taaaaaggcc gcgttgctgg cgtttttcca taggctccgc 9000
   ccccctgacg agcatcacaa aaatcgacgc tcaagtcaga ggtggcgaaa cccgacagga 9060
   ctataaagat accaggcgtt tccccctgga agctccctcg tgcgctctcc tgttccgacc 9120
   ctgccgctta ccggatacct gtccgccttt ctcccttcgg gaagcgtggc gctttctcat 9180
   agctcacgct gtaggtatct cagttcggtg taggtcgttc gctccaagct gggctgtgtg 9240
   cacgaacccc ccgttcagcc cgaccgctgc gccttatccg gtaactatcg tcttgagtcc 9300
   aacccggtaa gacacgactt atcgccactg gcagcagcca ctggtaacag gattagcaga 9360
   gcgaggtatg taggcggtgc tacagagttc ttgaagtggt ggcctaacta cggctacact 9420
   agaagaacag tatttggtat ctgcgctctg ctgaagccag ttaccttcgg aaaaagagtt 9480
   ggtagctctt gatccggcaa acaaaccacc gctggtagcg gtggtttttt tgtttgcaag 9540
   cagcagatta cgcgcagaaa aaaaggatct caagaagatc ctttgatctt ttctacgggg 9600
   tctgacgctc agtggaacga aaactcacgt taagggattt tggtcatgag attatcaaaa 9660
   aggatcttca cctagatcct tttaaattaa aaatgaagtt ttaaatcaat ctaaagtata 9720
   tatgagtaaa cttggtctga cagttaccaa tgcttaatca gtgaggcacc tatctcagcg 9780
   atctgtctat ttcgttcatc catagttgcc tgactccccg tcgtgtagat aactacgata 9840
   cgggagggct taccatctgg ccccagtgct gcaatgatac cgcgagaccc acgctcaccg 9900
   gctccagatt tatcagcaat aaaccagcca gccggaaggg ccgagcgcag aagtggtcct 9960
   gcaactttat ccgcctccat ccagtctatt aattgttgcc gggaagctag agtaagtagt 10020
   tcgccagtta atagtttgcg caacgttgtt gccattgcta caggcatcgt ggtgtcacgc 10080
   tcgtcgtttg gtatggcttc attcagctcc ggttcccaac gatcaaggcg agttacatga 10140
   tcccccatgt tgtgcaaaaa agcggttagc tccttcggtc ctccgatcgt tgtcagaagt 10200
   aagttggccg cagtgttatc actcatggtt atggcagcac tgcataattc tcttactgtc 10260
   atgccatccg taagatgctt ttctgtgact ggtgagtact caaccaagtc attctgagaa 10320
   tagtgtatgc ggcgaccgag ttgctcttgc ccggcgtcaa tacgggataa taccgcgcca 10380
   catagcagaa ctttaaaagt gctcatcatt ggaaaacgtt cttcggggcg aaaactctca 10440
   aggatcttac cgctgttgag atccagttcg atgtaaccca ctcgtgcacc caactgatct 10500
   tcagcatctt ttactttcac cagcgtttct gggtgagcaa aaacaggaag gcaaaatgcc 10560
   gcaaaaaagg gaataagggc gacacggaaa tgttgaatac tcatactctt cctttttcaa 10620
   tattattgaa gcatttatca gggttattgt ctcatgagcg gatacatatt tgaatgtatt 10680
   tagaaaaata aacaaatagg ggttccgcgc acatttcccc gaaaagtgcc acctgacgtc 10740
   taagaaacca ttattatcat gacattaacc tataaaaata ggcgtatcac gaggcccttt 10800
   cgtc 10804
<210> 164
   <211> 10827
   <212> DNA
   <213> Artificial Sequence
<220>
   <223> Synthetic DNA
<400> 164
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt gacgcgtatt gggatggaac 420
   catatgtagc ttacgacact gataacttcg tatatggtat tatatacgaa gttataagct 480
   tcaccatggc caagcctttg tctcaagaag aatccaccct cattgaaaga gcaacggcta 540
   caatcaacag catccccatc tctgaagact acagcgtcgc cagcgcagct ctctctagcg 600
   acggccgcat cttcactggt gtcaatgtat atcattttac tgggggacct tgtgcagaac 660
   tcgtggtgct gggcactgct gctgctgcgg cagctggcaa cctgacttgt atcgtcgcga 720
   tcggaaatga gaacaggggc atcttgagcc cctgcggacg gtgtcgacag gtgcttctcg 780
   atctgcatcc tgggatcaaa gcgatagtga aggacagtga tggacagccg acggcagttg 840
   ggattcgtga attgctgccc tctggttatg tgtgggaggg ctaaactagt gcacttcgtg 900
   gccgaggagc aggactgaca cgtgctacga gatttcgatt ccaccgccgc cttctatgaa 960
   aggttgggct tcggaatcgt tttccgggac gccggctgga tgatcctcca gcgcggggat 1020
   ctcatgctgg agttcttcgc ccaccccaac ttgtttattg cagcttataa tggttacaaa 1080
   taaagcaata gcatcacaaa tttcacaaat aaagcatttt tttcactgca ttctagttgt 1140
   ggtttgtcca aactcatcaa tgtatcttat catgtctgta taccgtcgac ctctagctag 1200
   agcttggcgt aatcatggtc atagctgttt cctgtgtgcc caccccaact tgtttattgc 1260
   agcttataat ggttacaaat aaagcaatag catcacaaat ttcacaaata aagcattttt 1320
   ttcactgcat tctagttgtg gtttgtccaa actcatcaat gtatcttatc atgtctgata 1380
   acttcgtata ttctatctta tacgaacggt actcgagagc tcatgcggta ccgctagcat 1440
   gctggccagg atccatgccc atggccgcgg atgcggcgcg ccatcgattc tgaggggact 1500
   ctgtggggtc tctgaggggc atcaatgcgc tttgcatggg atctgaatgt ggtctttatt 1560
   agatctgacc gatgtaagta aagggtgttg aggagacggt gaccagggtg ccctggcccc 1620
   aatactcata gtagcccccc acgtaccagg tggcactata tgtagcatcg acccctttcg 1680
   cacagtaata tacggccgtg tcctcggctc tcaggctgtt catttgcaga tacagcgtgt 1740
   tcttggaatt gtctctggag atggtgaacc ggcccttcac ggagtctgcg tagtatgtcc 1800
   taccaccagt accactgata cctgagaccc actccagccc cttccctgga gcctggcgga 1860
   cccagcttat ggcaaagttg ctaaaggtga atccagaggc tgcacaggag agtctcaggg 1920
   accccccagg ctgtaccaag cctcccccag actccaacag ctgcacctcg ggagggaagg 1980
   agatcttggc tgcccctcca ggggcgcccc ggcctcgctt gagcttcagt ggagtccgtc 2040
   tggggtatca atgggacatc aatggggtct gaggagacgg tgaccagggt gccctggccc 2100
   cagtagtcag ggccacaccc cagggccact atatccgcgt gaaccagatc tttcgcacag 2160
   taatatacgg ccgtgtcctc ggctctcagg ctgttcattt gcagatacag cgtgttcttg 2220
   gaattgtctc tggagatggt gaaccggccc ctcacggagt ctgcgtagta tgtgctgcca 2280
   gcactagcac tcatacttga gacccactcc agccccttcc ctggagcctg gcggacccag 2340
   cccatggcat atctgctaaa ggtgaatcca gaggctgcac aggagagtct cagggacccc 2400
   ccaggctgta ccaagcctcc cccagactcc aacagctgca cctcacagcg gggggacgag 2460
   aggacagagg gtctcttcag aactcagccg ttgtgtgctt ccacgcacag ctccacttct 2520
   tcgtcccagc acacagctct gcattgagga gacggtgacc agggtgccct ggccccagta 2580
   gtcaagcata actccccgaa ccatagtagt tttcgcacag taatatacgg ccgtgtcctc 2640
   ggctctcagg ctgttcattt gcagatacag cgtgttcttg gaattgtctc tggagatggt 2700
   gaaccggccc ttcacggagt ctgagtagta tgtactacca ccatcaccag tattagttga 2760
   gacccactcc agccccttcc ctggagcctg gcggacccag ctcatgaaaa aggtgctaaa 2820
   ggtgaatcca gaggctgcac aggagagtct cagggacccc ccaggctgta ccaagcctcc 2880
   cccagactcc aacagctgca cctccgtgtc cgtgcccagc acagccccac atcgcacggc 2940
   tccatacagc agcagcgtgg agctgagccc caatgtgggc acggagccca tcccctctct 3000
   cagctgagga gacggtgacc agggtgccct ggccccagac gtccatatcg tagtagaggt 3060
   gggctcgagc ccctttcgca cagtaatata cggccgtgtc ctcggctctc aggctgttca 3120
   tttgcagata cagcgtgttc ttggaattgt ctctggagat ggtgaaccgg cccttcacgg 3180
   agtctgcgta gtatgtacta atagcactac cactaacaag tgagacccac tccagcccct 3240
   tccctggagc ctggcggacc cagctcaggg cataactgct aaaggtgaat ccagaggctg 3300
   cacaggagag tctcagggac cccccaggct gtaccaagcc tcccccagac tccaacagct 3360
   gcacctctgg cggggcagcc aagaggagca gatgggagct atgagggctg atggtggcga 3420
   aatcacgggg ggaaggagat cagggcttgt tctgttctta ggagagctga ggagacggtg 3480
   accagggtgc cctggcccca gtagtcaaat gaccagccac tgctaaagcc tttcgcacag 3540
   taatatacgg ccgtgtcctc ggctctcagg ctgttcattt gcagatacag cgtgttcttg 3600
   gaattgtctc tggagatggt gaaccggccc ttcacggagt ctgcgtaata tgtgttttca 3660
   ccactgacag taatagttga gacccactcc agccccttcc ctggagcctg gcggacccag 3720
   tccatgccaa agttgctaaa ggtgaatcca gaggctgcac aggagagtct cagggacccc 3780
   ccaggctgta ccaagcctcc cccagactcc aacagctgca cctcatcccg atgaggttca 3840
   gtcaacacct catagagacc ccactgagaa cacagtgaga ccccgtagat gccctaaaga 3900
   ccccggaaag ggccgaagcc cacctgagga gacggtgacc agggtgccct ggccccaagg 3960
   attcccccaa ccagagtaat gccaggataa tttcgcacag taatatacgg ccgtgtcctc 4020
   ggctctcagg ctgttcattt gcagatacag cgtgttcttg gaattgtctc tggagatggt 4080
   gaaccggccc tgcacggagt ctgcgtagaa agcgctacta ccgctaccac gaatagctga 4140
   gacccactcc agccccttcc ctggagcctg gcggacccag ctgaggtcaa agttgctaaa 4200
   ggtgaatcca gaggctgcac aggagagtct cagggacccc ccaggctgta ccaagcctcc 4260
   cccagactcc aacagctgca cctcgacccc atagagttct aatagagacc tcattgcgac 4320
   tccgaagaga tctcatatag attgcgttga attcccttcg agacatcact gagatctcat 4380
   agagtgagga gacggtgacc agggtgccct ggccccagta gtcaaggctg ctgccaagag 4440
   atttcgcaca gtaatatacg gccgtgtcct cggctctcag gctgttcatt tgcagataca 4500
   gcgtgttctt ggaattgtct ctggagatgg tgaaccggcc cttcacggag tctgcgtagt 4560
   atgtgccagc accactacta gtaacagttg agacccactc cagccccttc cctggagcct 4620
   ggcggaccca ggtcatggca aagttgctaa aggtgaatcc agaggctgca caggagagtc 4680
   tcagggaccc cccaggctgt accaagcctc ccccagactc caacagctgc acctccctcg 4740
   cggagccgga ccacactccg accacaaaga gaccccactg agatgtcata gagagccata 4800
   gagatgtgac tgaaaccacg taaagaccat ttagatgagg agacggtgac cagggtgccc 4860
   tggccccaat agtgacagta atcaccacta caatatttgg tgagaccccc tttcgcacag 4920
   taatatacgg ccgtgtcctc ggctctcagg ctgttcattt gcagatacag cgtgttcttg 4980
   gaattgtctc tggagatggt gaaccggccc ttcacggagt ctgcgtagta tatggcacca 5040
   ccagtgccac taatagctga gacccactcc agccccttcc ctggagcctg gcggacccag 5100
   ctcatggaat agctgctaaa ggtgaatcca gaggctgcac aggagagtct cagggacccc 5160
   ccaggctgta ccaagcctcc cccagactcc aacagctgca cctcataagg acaccctgtt 5220
   ggtgcggggc agcattccta cacgtgacca tcacagcatg tgcgtcctat gggtggtctt 5280
   caggcccggt tcataagaga gacctgagga gacggtgacc agggtgccct ggccccagac 5340
   gtccataccg tagtagtagt agtaacctct ccaaaaatcg taagagttgt ctttcgcaca 5400
   gtaatatacg gccgtgtcct cggctctcag gctgttcatt tgcagataca gcgtgttctt 5460
   ggaattgtct ctggagatgg tgaaccggcc cttcacggag tctgcgtagt atgtgctacc 5520
   accacgacca ctaatacctg agacccactc cagccccttc cctggagcct ggcggaccca 5580
   gcccatgaca taggtgctaa aggtgaatcc agaggctgca caggagagtc tcagggaccc 5640
   cccaggctgt accaagcctc ccccagactc caacagctgc acctctttcg cagccagtgc 5700
   agctgcgtgg ggtcagcagg ggatgccacc acccctgcag cgagctgtgg agcgggttgt 5760
   gcctcttctg agacgtgcag acccctgagg agacggtgac cagggtgccc tggccccaga 5820
   agtcaaagta tttcgcacag taatatacgg ccgtgtcctc ggctctcagg ctgttcattt 5880
   gcagatacag cgtgttcttg gaattgtctc tggagatggt gaaccggccc ttcacggagt 5940
   ctgagtagta tgtgctacca ccataattac taatagctga gacccactcc agccccttcc 6000
   ctggagcctg gcggacccag gtcatggcat agttgctaaa ggtgaatcca gaggctgcac 6060
   aggagagtct cagggacccc ccaggctgta ccaagcctcc cccagactcc aacagctgca 6120
   cctcgccgtg ccagcagatg gagcagctgc ggccgatgct ctctttggcc cctttcagtc 6180
   gatgttttat ttttttctcc cctttccatc ctattttcgt gctctgagga gacggtgacc 6240
   agggtgccct ggccccagac gtccataccg cagtcacgcc ccgaaccatc gcatttcgca 6300
   cagtaatata cggccgtgtc ctcggctctc aggctgttca tttgcagata cagcgtgttc 6360
   ttggaattgt ctctggagat ggtgaaccgg cccttcacgg agtctgaata ggttgggcct 6420
   tcaccactgt cactaatact tgagacccac tccagcccct tccctggagc ctggcggacc 6480
   cacctcataa ggtacttgct aaaggtgaat ccagaggctg cacaggagag tctcagggac 6540
   cccccaggct gtaccaagcc tcccccagac tccaacagct gcacctcgcg accacagccc 6600
   agctgcagaa cacctccagc tgtccccaca tcagagctat ttcggggcag gaaaccacga 6660
   gtcctttcgg tcgtgtccga accccgctga ggagacggtg accagggtgc cctggcccca 6720
   gacgtccaga cccccagcca aaacatcttc tccagccaaa acatcttcta cgtggagatc 6780
   tttcgcacag taatatacgg ccgtgtcctc ggctctcagg ctgttcattt gcagatacag 6840
   cgtgttcttg gaattgtctc tggagatggt gaaccggccc ttcacggagt ctgcgtagta 6900
   tgtgtcaccc ccactaccag taatagctga gacccactcc agccccttcc ctggagcctg 6960
   gcggacccac gtcatgacaa aggtgctaaa ggtgaatcca gaggctgcac aggagagtct 7020
   cagggacccc ccaggctgta ccaagcctcc cccagactcc aacagctgca cctcctctgg 7080
   gggtctctgg gggttttatg aggcccagct ccacgaggtt gtgtggcggc ggctccgtca 7140
   gcgctctctg tccttcccca cagggctgat ggcgtgagga gacggtgacc agggtgccct 7200
   ggccccagac gtccataccg taatggtagt actggccact gctatacacc ccatctttcg 7260
   cacagtaata tacggccgtg tcctcggctc tcaggctgtt catttgcaga tacagcgtgt 7320
   tcttggaatt gtctctggag atggtgaacc ggcccttcac ggagtctgcg taatatgttc 7380
   taataccact accactaata cttgagaccc actccagccc cttccctgga gcctggcgga 7440
   cccagctcat gtcaaagctg ctaaaggtga atccagaggc tgcacaggag agtctcaggg 7500
   accccccagg ctgtaccaag cctcccccag actccaacag ctgcacctcg ggtccctaca 7560
   gggcctttat ggggtttcag tggtgtctct atggctctct atgacatctc aggctatggc 7620
   agtggtgctg atgacggtga cagagcccat gaggagacgg tgaccagggt gccctggccc 7680
   caggcgtcca taccgtacca gtggagatct ttcgcacagt aatatacggc cgtgtcctcg 7740
   gctctcaggc tgttcatttg cagatacagc gtgttcttgg aattgtctct ggagatggtg 7800
   aaccggccct tcacggagtc tgcgtagtgt gtcctaccat cactggcact aatagttgag 7860
   acccactcca gccccttccc tggagcctgg cggacccagt tcatgaaaaa gctgctaaag 7920
   gtgaatccag aggctgcaca ggagagtctc agggaccccc caggctgtac caagcctccc 7980
   ccagactcca acagctgcac ctcctccagg caggctctga cctctctggg tctctgtagg 8040
   gtctctatgt ggtctcatgg ggtcatcagc ggggtctcaa tgaggtttct atcaaatctc 8100
   atctgaggag acggtgacca gggtgccctg gccccagtaa gtaggggttt tcgcacagta 8160
   atatacggcc gtgtcctcgg ctctcaggct gttcatttgc agatacagcg tgttcttgga 8220
   attgtctctg gagatggtga accggccctt cacggagtct gcgtagtatg tgttatcacc 8280
   actagcacta atagctgaga cccactccag ccccttccct ggagcctggc ggacccagct 8340
   catgacatag atgctaaagg tgaatccaga ggctgcacag gagagtctca gggacccccc 8400
   aggctgtacc aagcctcccc cagactccaa cagctgcacc tcggcgcgcc ccgcggatgt 8460
   cgacgattta aatgggcccc ccggtgatat ccccgggtct agagcggccg ctaccgttcg 8520
   tataatgtat gctatacgaa gttatatcga attcctgcag agatctgttc catcccaatg 8580
   gcgcgccgag cttggcgtaa tcatggtcat agctgtttcc tgtgtgaaat tgttatccgc 8640
   tcacaattcc acacaacata cgagccggaa gcataaagtg taaagcctgg ggtgcctaat 8700
   gagtgagcta actcacatta attgcgttgc gctcactgcc cgctttccag tcgggaaacc 8760
   tgtcgtgcca gctgcattaa tgaatcggcc aacgcgcggg gagaggcggt ttgcgtattg 8820
   ggcgctcttc cgcttcctcg ctcactgact cgctgcgctc ggtcgttcgg ctgcggcgag 8880
   cggtatcagc tcactcaaag gcggtaatac ggttatccac agaatcaggg gataacgcag 8940
   gaaagaacat gtgagcaaaa ggccagcaaa aggccaggaa ccgtaaaaag gccgcgttgc 9000
   tggcgttttt ccataggctc cgcccccctg acgagcatca caaaaatcga cgctcaagtc 9060
   agaggtggcg aaacccgaca ggactataaa gataccaggc gtttccccct ggaagctccc 9120
   tcgtgcgctc tcctgttccg accctgccgc ttaccggata cctgtccgcc tttctccctt 9180
   cgggaagcgt ggcgctttct catagctcac gctgtaggta tctcagttcg gtgtaggtcg 9240
   ttcgctccaa gctgggctgt gtgcacgaac cccccgttca gcccgaccgc tgcgccttat 9300
   ccggtaacta tcgtcttgag tccaacccgg taagacacga cttatcgcca ctggcagcag 9360
   ccactggtaa caggattagc agagcgaggt atgtaggcgg tgctacagag ttcttgaagt 9420
   ggtggcctaa ctacggctac actagaagaa cagtatttgg tatctgcgct ctgctgaagc 9480
   cagttacctt cggaaaaaga gttggtagct cttgatccgg caaacaaacc accgctggta 9540
   gcggtggttt ttttgtttgc aagcagcaga ttacgcgcag aaaaaaagga tctcaagaag 9600
   atcctttgat cttttctacg gggtctgacg ctcagtggaa cgaaaactca cgttaaggga 9660
   ttttggtcat gagattatca aaaaggatct tcacctagat ccttttaaat taaaaatgaa 9720
   gttttaaatc aatctaaagt atatatgagt aaacttggtc tgacagttac caatgcttaa 9780
   tcagtgaggc acctatctca gcgatctgtc tatttcgttc atccatagtt gcctgactcc 9840
   ccgtcgtgta gataactacg atacgggagg gcttaccatc tggccccagt gctgcaatga 9900
   taccgcgaga cccacgctca ccggctccag atttatcagc aataaaccag ccagccggaa 9960
   gggccgagcg cagaagtggt cctgcaactt tatccgcctc catccagtct attaattgtt 10020
   gccgggaagc tagagtaagt agttcgccag ttaatagttt gcgcaacgtt gttgccattg 10080
   ctacaggcat cgtggtgtca cgctcgtcgt ttggtatggc ttcattcagc tccggttccc 10140
   aacgatcaag gcgagttaca tgatccccca tgttgtgcaa aaaagcggtt agctccttcg 10200
   gtcctccgat cgttgtcaga agtaagttgg ccgcagtgtt atcactcatg gttatggcag 10260
   cactgcataa ttctcttact gtcatgccat ccgtaagatg cttttctgtg actggtgagt 10320
   actcaaccaa gtcattctga gaatagtgta tgcggcgacc gagttgctct tgcccggcgt 10380
   caatacggga taataccgcg ccacatagca gaactttaaa agtgctcatc attggaaaac 10440
   gttcttcggg gcgaaaactc tcaaggatct taccgctgtt gagatccagt tcgatgtaac 10500
   ccactcgtgc acccaactga tcttcagcat cttttacttt caccagcgtt tctgggtgag 10560
   caaaaacagg aaggcaaaat gccgcaaaaa agggaataag ggcgacacgg aaatgttgaa 10620
   tactcatact cttccttttt caatattatt gaagcattta tcagggttat tgtctcatga 10680
   gcggatacat atttgaatgt atttagaaaa ataaacaaat aggggttccg cgcacatttc 10740
   cccgaaaagt gccacctgac gtctaagaaa ccattattat catgacatta acctataaaa 10800
   ataggcgtat cacgaggccc tttcgtc 10827
<210> 165
   <211> 375
   <212> DNA
   <213> Artificial Sequence
<220>
   <223> Synthetic DNA
<400> 165
   gaggtgcagc tgttggagtc tgggggaggc ttggtacagc ctggggggtc cctgagactc 60
   tcctgtgcag cctctggatt cacctttagc ggctttgcca tgaactgggt ccgccaggct 120
   ccagggaagg ggctggagtg ggtctcaact attagtaata gtgctggtag cacatattac 180
   gcagactccg tgaagggccg gttcaccatc tccagagaca attccaagaa cacgctgtat 240
   ctgcaaatga acagcctgag agccgaggac acggccgtat attactgtgc gaaatatggg 300
   gaggatattg tcgttgtgcc agctcgctac ggtgtggacg tctggggcca gggcaccctg 360
   gtcaccgtct cctca 375
<210> 166
   <211> 363
   <212> DNA
   <213> Artificial Sequence
<220>
   <223> Synthetic DNA
<400> 166
   gaggtgcagc tgttggagtc tgggggaggc ttggtacagc ctggggggtc cctgagactc 60
   tcctgtgcag cctctggatt cacctttagc agttttgccc tgaattgggt ccgccaggct 120
   ccagggaagg ggctggagtg ggtctcaact attactggta gtgctagtaa cacatactac 180
   gcagactccg tgaagggccg gttcaccatc tccagagaca attccaagaa cacgctgtat 240
   ctgcaaatga acagcctgag agccgaggac acggccgtat attactgtgc gaaaggggca 300
   catagtcgca gcctaacttg gttcggcccc tggggccagg gcaccctggt caccgtctcc 360
   tca 363
<210> 167
   <211> 372
   <212> DNA
   <213> Artificial Sequence
<220>
   <223> Synthetic DNA
<400> 167
   gaggtgcagc tgttggagtc tgggggaggc ttggtacagc ctggggggtc cctgagactc 60
   tcctgtgcag cctctggatt cacctttagc aactatgcca tgagctgggt ccgccaggct 120
   ccagggaagg ggctggagtg ggtctcaact tttagtaaga ctggtgatag aacattttat 180
   gcagactccg tgaagggccg gttcaccatc tccagagaca attccaagaa cacgctgtat 240
   ctgcaaatga acagcctgag agccgaggac acggccgtat attactgtgc gaaaaatggg 300
   ccctttggtt gtggtggtga ctgcgcagac tttgactact ggggccaggg caccctggtc 360
   accgtctcct ca 372
<210> 168
   <211> 369
   <212> DNA
   <213> Artificial Sequence
<220>
   <223> Synthetic DNA
<400> 168
   gaggtgcagc tgttggagtc tgggggaggc ttggtacagc ctggggggtc cctgagactc 60
   tcctgtgcag cctctggatt cacctttagc aactttccca tgagctgggt ccgccaggct 120
   ccagggaagg ggctggagtg ggtctcagtt attagtggta gtggtggaag cacactctac 180
   acagactccg tgaagggccg gttcaccatc tccagagaca attccaagaa cacgctgtat 240
   ctgcaaatga acagcctgag agccgaggac acggccgtat attactgtgc gaaagatcag 300
   gacgattttt ggagtgtttc aaattggttc gacccctggg gccagggcac cctggtcacc 360
   gtctcctca 369
<210> 169
   <211> 357
   <212> DNA
   <213> Artificial Sequence
<220>
   <223> Synthetic DNA
<400> 169
   gaggtgcagc tgttggagtc tgggggaggc ttggtacagc ctggggggtc cctgagactc 60
   tcctgtgcag cctctggatt cacctttagc agctttgcca tgacctgggt ccgccaggct 120
   ccagggaagg ggctggagtg ggtctcagct attagtggta gtgttggtag cacatactac 180
   gcagactccg tgcagggccg gttcaccatc tccagagaca attccaagaa cacgctgtat 240
   ctgcaaatga acagcctgag agccgaggac acggccgtat attactgtgc gaaagacggt 300
   ggggcctact acggaacggt caactggggc cagggcaccc tggtcaccgt ctcctca 357
<210> 170
   <211> 378
   <212> DNA
   <213> Artificial Sequence
<220>
   <223> Synthetic DNA
<400> 170
   gaggtgcagc tgttggagtc tgggggaggc ttggtacagc ctggggggtc cctgagactc        60
   tcctgtgcag cctctggatt cacctttagc atctttgccg tgagctgggt ccgccaggct       120
   ccagggaagg ggctggagtg ggtctcagct attagtggtc atggtcttag cacatactac       180
   ggagactccg tgaagggccg gttcaccatc tccagagaca attccaagaa cacgctgtat       240
   ctgcaaatga acagcctgag agccgaggac acggccgtat attactgtgc gaaagccgga       300
   agtgctggta gcagctaccc gttctactac tacggtatgg acgtctgggg ccagggcacc       360
   ctggtcaccg tctcctca                                                     378
<210> 171
   <211> 387
   <212> DNA
   <213> Artificial Sequence
<220>
   <223> Synthetic DNA
<400> 171
   gaggtgcagc tgttggagtc tgggggaggc ttggtacagc ctggggggtc cctgagactc        60
   tcctgtgcag cctctggatt cacctttagc agctatgcca tgatctgggt ccgccaggct       120
   ccagggaagg ggctggagtg ggtctcaggt attactgtta gtggcggtag cacagactac       180
   gcagactccg tgaagggccg gttcaccatc tccagagaca attccaagaa cacgctgtat       240
   ctgcaaatga acagcctgag agccgaggac acggccgtat attactgtgc gaaagggggg       300
   ccccaactta tagcagcagc tgggacttat tatcactact acggtatgga cgtctggggc       360
   cagggcaccc tggtcaccgt ctcctca                                           387
<210> 172
   <211> 360
   <212> DNA
   <213> Artificial Sequence
<220>
   <223> Synthetic DNA
<400> 172
   gaggtgcagc tgttggagtc tgggggaggc ttggtacagc ctggggggtc cctgagactc 60
   tcctgtgcag cctctggatt cacctttagc agctatacca tgaactgggt ccgccaggct 120
   ccagggaagg ggctggagtg ggtctcatac attagtagtg gtagtggtag catatactac 180
   gcagactctg tgaagggccg gttcaccatc tccagagaca attccaagaa cacgctgtat 240
   ctgcaaatga acagcctgag agccgaggac acggccgtat attactgtgc gaaacgaggt 300
   gatagtagtg catacgccct tgactactgg ggccagggca ccctggtcac cgtctcctca 360
<210> 173
   <211> 372
   <212> DNA
   <213> Artificial Sequence
<220>
   <223> Synthetic DNA
<400> 173
   gaggtgcagc tgttggagtc tgggggaggc ttggtacagc ctggggggtc cctgagactc 60
   tcctgtgcag cctctggatt cacctttagc agccatagca tgaactgggt ccgccaggct 120
   ccagggaagg ggctggagtg ggtctcatac attagaagta gtagtagtac catatactac 180
   gcagactctg tgaagggtcg gttcaccatc tccagagaca attccaagaa cacgctgtat 240
   ctgcaaatga acagcctgag agccgaggac acggccgtat attactgtgc gaaagtcccc 300
   aattactatg gttcatttcc tcgagatggt atggacgtct ggggccaggg caccctggtc 360
   accgtctcct ca 372
<210> 174
   <211> 369
   <212> DNA
   <213> Artificial Sequence
<220>
   <223> Synthetic DNA
<400> 174
   gaggtgcagc tgttggagtc tgggggaggc ttggtacagc ctggggggtc cctgagactc 60
   tcctgtgcag cctctggatt cacctttagc agctataaca tgaactgggt ccgccaggct 120
   ccagggaagg ggctggagtg ggtctcatac gttagtagtg attctagtac catatactac 180
   gcagactttg tggagggccg gttcaccatc tccagagaca attccaagaa cacgctgtat 240
   ctgcaaatga acagcctgag agccgaggac acggccgtat attactgtgc gaaaggcctg 300
   gcgttctgcg ccagctgcta tggatacttc cagctctggg gccagggcac cctggtcacc 360
   gtctcctca 369
<210> 175
   <211> 357
   <212> DNA
   <213> Artificial Sequence
<220>
   <223> Synthetic DNA
<400> 175
   gaggtgcagc tgttggagtc tgggggaggc ttggtacagc ctggggggtc cctgagactc 60
   tcctgtgcag cctctggatt cacctttagc agcaactcca tgagctgggt ccgccaggct 120
   ccagggaagg ggctggagtg ggtctcactt atttatagca gtggtggcac atactacgca 180
   gactccgtga agggccggtt caccatctcc agagacaatt ccaagaacac gctgtatctg 240
   caaatgaaca gcctgagagc cgaggacacg gccgtatatt actgtgcgaa agcgtgggtg 300
   tatgatagta gtgtttttga ctattggggc cagggcaccc tggtcaccgt ctcctca 357
<210> 176
   <211> 360
   <212> DNA
   <213> Artificial Sequence
<220>
   <223> Synthetic DNA
<400> 176
   gaggtgcagc tgttggagtc tgggggaggc ttggtacagc ctggggggtc cctgagactc 60
   tcctgtgcag cctctggatt cacctttagc tatgccataa gctgggtccg ccaggctcca 120
   gggaaggggc tggagtgggt ctcaggtatt agtggcggcg gtggtagcac attctacgct 180
   gactccgtgc agggccggtt caccatctcc agagacaatt ccaagaacac gctgtatctg 240
   caaatgaaca gcctgagagc cgaggacacg gccgtatatt actgtgcgaa acccgagaga 300
   gggatagtgg gggctgcatt tgactcctgg ggccagggca ccctggtcac cgtctcctca 360
<210> 177
   <211> 354
   <212> DNA
   <213> Artificial Sequence
<220>
   <223> Synthetic DNA
<400> 177
   gaggtgcagc tgttggagtc tgggggaggc ttggtacagc ctggggggtc cctgagactc 60
   tcctgtgcag cctctggatt cacctttagc agccattgga tgaggtgggt ccgccaggct 120
   ccagggaagg ggctggagtg ggtctcaaac ataaagcaag gtggaagtga agaatattat 180
   gtggactctg tgaagggccg gttcaccatc tccagagaca attccaagaa cacgctgtat 240
   ctgcaaatga acagcctgag agccgaggac acggccgtat attactgtgc gaaaggaaat 300
   tataaggcag gttgtgacta ctggggccag ggcaccctgg tcaccgtctc ctca 354
<210> 178
   <211> 348
   <212> DNA
   <213> Artificial Sequence
<220>
   <223> Synthetic DNA
<400> 178
   gaggtgcagc tgttggagtc tgggggaggc ttggtacagc ctggggggtc cctgagactc 60
   tcctgtgcag cctctggatt cacctttagc acctatagca tgaactgggt ccgccaggct 120
   ccagggaagg ggctggagtg ggtctcatcc attactagta gtggtagtta catatactgc 180
   gcagactcag tgaagggccg gttcaccatc tccagagaca attccaagaa cacgctgtat 240
   ctgcaaatga acagcctgag agccgaggac acggccgtat attactgtgc gaaagatggc 300
   tccggtatgg acgtctgggg ccagggcacc ctggtcaccg tctcctca 348
<210> 179
   <211> 357
   <212> DNA
   <213> Artificial Sequence
<220>
   <223> Synthetic DNA
<400> 179
   gaggtgcagc tgttggagtc tgggggaggc ttggtacagc ctggggggtc cctgagactc 60
   tcctgtgcag cctctggatt cacctttagc tcctatagca tgacctgggt ccgccaggct 120
   ccagggaagg ggctggagtg ggtctcaaac attagcagta gtagtaatac cgcattctac 180
   gcagactccg tgaagggccg gttcaccatc tccagagaca attccaagaa cacgctgtat 240
   ctgcaaatga acagcctgag agccgaggac acggccgtat attactgtgc gaaagatcga 300
   aatggctaca atttctttga ctactggggc cagggcaccc tggtcaccgt ctcctca 357
<210> 180
   <211> 7155
   <212> DNA
   <213> Artificial Sequence
<220>
   <223> Synthetic DNA
<400> 180
   ggtaccgagg tgcagctgtt ggagtctggg ggaggcttgg tacagcctgg ggggtccctg 60
   agactctcct gtgcagcctc tggattcacc tttagcagtt ttgccctgaa ttgggtccgc 120
   caggctccag ggaaggggct ggagtgggtc tcaactatta ctggtagtgc tagtaacaca 180
   tactacgcag actccgtgaa gggccggttc accatctcca gagacaattc caagaacacg 240
   ctgtatctgc aaatgaacag cctgagagcc gaggacacgg ccgtatatta ctgtgcgaaa 300
   ggggcacata gtcgcagcct aacttggttc ggcccctggg gccagggcac cctggtcacc 360
   gtctcctcag tgcctggtga ctcttccgca atcagtcctg ggacagaaac ctcagggaca 420
   gcctttatga gttttgatca catctaacag agaggatggg ccagtgagct agagtgatag 480
   agcgtgcgtg tgcggaggtg cagctgttgg agtctggggg aggcttggta cagcctgggg 540
   ggtccctgag actctcctgt gcagcctctg gattcacctt tagcaactat gccatgagct 600
   gggtccgcca ggctccaggg aaggggctgg agtgggtctc aacttttagt aagactggtg 660
   atagaacatt ttatgcagac tccgtgaagg gccggttcac catctccaga gacaattcca 720
   agaacacgct gtatctgcaa atgaacagcc tgagagccga ggacacggcc gtatattact 780
   gtgcgaaaaa tgggcccttt ggttgtggtg gtgactgcgc agactttgac tactggggcc 840
   agggcaccct ggtcaccgtc tcctcagtgc ctggtgactc ttccgcaatc agtcctggga 900
   cagaaacctc agggacagcc tttatgagtt ttgatcacat ctaacagaga ggatgggcca 960
   ggaggtgcag ctgttggagt ctgggggagg cttggtacag cctggggggt ccctgagact 1020
   ctcctgtgca gcctctggat tcacctttag caactttccc atgagctggg tccgccaggc 1080
   tccagggaag gggctggagt gggtctcagt tattagtggt agtggtggaa gcacactcta 1140
   cacagactcc gtgaagggcc ggttcaccat ctccagagac aattccaaga acacgctgta 1200
   tctgcaaatg aacagcctga gagccgagga cacggccgta tattactgtg cgaaagatca 1260
   ggacgatttt tggagtgttt caaattggtt cgacccctgg ggccagggca ccctggtcac 1320
   cgtctcctca gtgcctggtg actcttccgc aatcagtcct gggacagaaa cctcagggac 1380
   agcctttatg agttttgatc acatctaaca gagaggatgg gccagtatag acatctatga 1440
   tacgctcact catacgaggt gcagctgttg gagtctgggg gaggcttggt acagcctggg 1500
   gggtccctga gactctcctg tgcagcctct ggattcacct ttagcagctt tgccatgacc 1560
   tgggtccgcc aggctccagg gaaggggctg gagtgggtct cagctattag tggtagtgtt 1620
   ggtagcacat actacgcaga ctccgtgcag ggccggttca ccatctccag agacaattcc 1680
   aagaacacgc tgtatctgca aatgaacagc ctgagagccg aggacacggc cgtatattac 1740
   tgtgcgaaag acggtggggc ctactacgga acggtcaact ggggccaggg caccctggtc 1800
   accgtctcct cagtgcctgg tgactcttcc gcaatcagtc ctgggacaga aacctcaggg 1860
   acagccttta tgagttttga tcacatctaa cagagaggat gggccaggag gtgcagctgt 1920
   tggagtctgg gggaggcttg gtacagcctg gggggtccct gagactctcc tgtgcagcct 1980
   ctggattcac ctttagcatc tttgccgtga gctgggtccg ccaggctcca gggaaggggc 2040
   tggagtgggt ctcagctatt agtggtcatg gtcttagcac atactacgga gactccgtga 2100
   agggccggtt caccatctcc agagacaatt ccaagaacac gctgtatctg caaatgaaca 2160
   gcctgagagc cgaggacacg gccgtatatt actgtgcgaa agccggaagt gctggtagca 2220
   gctacccgtt ctactactac ggtatggacg tctggggcca gggcaccctg gtcaccgtct 2280
   cctcagtgcc tggtgactct tccgcaatca gtcctgggac agaaacctca gggacagcct 2340
   ttatgagttt tgatcacatc taacagagag gatgggccag tcatcgagtc tcgagctctc 2400
   tcgcagacac gaggtgcagc tgttggagtc tgggggaggc ttggtacagc ctggggggtc 2460
   cctgagactc tcctgtgcag cctctggatt cacctttagc agctatgcca tgatctgggt 2520
   ccgccaggct ccagggaagg ggctggagtg ggtctcaggt attactgtta gtggcggtag 2580
   cacagactac gcagactccg tgaagggccg gttcaccatc tccagagaca attccaagaa 2640
   cacgctgtat ctgcaaatga acagcctgag agccgaggac acggccgtat attactgtgc 2700
   gaaagggggg ccccaactta tagcagcagc tgggacttat tatcactact acggtatgga 2760
   cgtctggggc cagggcaccc tggtcaccgt ctcctcagtg cctggtgact cttccgcaat 2820
   cagtcctggg acagaaacct cagggacagc ctttatgagt tttgatcaca tctaacagag 2880
   aggatgggcc aggaggtgca gctgttggag tctgggggag gcttggtaca gcctgggggg 2940
   tccctgagac tctcctgtgc agcctctgga ttcaccttta gcagctatac catgaactgg 3000
   gtccgccagg ctccagggaa ggggctggag tgggtctcat acattagtag tggtagtggt 3060
   agcatatact acgcagactc tgtgaagggc cggttcacca tctccagaga caattccaag 3120
   aacacgctgt atctgcaaat gaacagcctg agagccgagg acacggccgt atattactgt 3180
   gcgaaacgag gtgatagtag tgcatacgcc cttgactact ggggccaggg caccctggtc 3240
   accgtctcct cagtgcctgg tgactcttcc gcaatcagtc ctgggacaga aacctcaggg 3300
   acagccttta tgagttttga tcacatctaa cagagaggat gggccagtct gtctcgctga 3360
   gtgacgctga tgtgtacgag gtgcagctgt tggagtctgg gggaggcttg gtacagcctg 3420
   gggggtccct gagactctcc tgtgcagcct ctggattcac ctttagcagc catagcatga 3480
   actgggtccg ccaggctcca gggaaggggc tggagtgggt ctcatacatt agaagtagta 3540
   gtagtaccat atactacgca gactctgtga agggtcggtt caccatctcc agagacaatt 3600
   ccaagaacac gctgtatctg caaatgaaca gcctgagagc cgaggacacg gccgtatatt 3660
   actgtgcgaa agtccccaat tactatggtt catttcctcg agatggtatg gacgtctggg 3720
   gccagggcac cctggtcacc gtctcctcag tgcctggtga ctcttccgca atcagtcctg 3780
   ggacagaaac ctcagggaca gcctttatga gttttgatca catctaacag agaggatggg 3840
   ccagtgctat agactgctcg ctacacgtgc agcggaggtg cagctgttgg agtctggggg 3900
   aggcttggta cagcctgggg ggtccctgag actctcctgt gcagcctctg gattcacctt 3960
   tagcagctat aacatgaact gggtccgcca ggctccaggg aaggggctgg agtgggtctc 4020
   atacgttagt agtgattcta gtaccatata ctacgcagac tttgtggagg gccggttcac 4080
   catctccaga gacaattcca agaacacgct gtatctgcaa atgaacagcc tgagagccga 4140
   ggacacggcc gtatattact gtgcgaaagg cctggcgttc tgcgccagct gctatggata 4200
   cttccagctc tggggccagg gcaccctggt caccgtctcc tcagtgcctg gtgactcttc 4260
   cgcaatcagt cctgggacag aaacctcagg gacagccttt atgagttttg atcacatcta 4320
   acagagagga tgggccagga ggtgcagctg ttggagtctg ggggaggctt ggtacagcct 4380
   ggggggtccc tgagactctc ctgtgcagcc tctggattca cctttagcag caactccatg 4440
   agctgggtcc gccaggctcc agggaagggg ctggagtggg tctcacttat ttatagcagt 4500
   ggtggcacat actacgcaga ctccgtgaag ggccggttca ccatctccag agacaattcc 4560
   aagaacacgc tgtatctgca aatgaacagc ctgagagccg aggacacggc cgtatattac 4620
   tgtgcgaaag cgtgggtgta tgatagtagt gtttttgact attggggcca gggcaccctg 4680
   gtcaccgtct cctcagtgcc tggtgactct tccgcaatca gtcctgggac agaaacctca 4740
   gggacagcct ttatgagttt tgatcacatc taacagagag gatgggccag actcacagag 4800
   agactcagcg agagagtgtg gaggtgcagc tgttggagtc tgggggaggc ttggtacagc 4860
   ctggggggtc cctgagactc tcctgtgcag cctctggatt cacctttagc tatgccataa 4920
   gctgggtccg ccaggctcca gggaaggggc tggagtgggt ctcaggtatt agtggcggcg 4980
   gtggtagcac attctacgct gactccgtgc agggccggtt caccatctcc agagacaatt 5040
   ccaagaacac gctgtatctg caaatgaaca gcctgagagc cgaggacacg gccgtatatt 5100
   actgtgcgaa acccgagaga gggatagtgg gggctgcatt tgactcctgg ggccagggca 5160
   ccctggtcac cgtctcctca gtgcctggtg actcttccgc aatcagtcct gggacagaaa 5220
   cctcagggac agcctttatg agttttgatc acatctaaca gagaggatgg gccaggaggt 5280
   gcagctgttg gagtctgggg gaggcttggt acagcctggg gggtccctga gactctcctg 5340
   tgcagcctct ggattcacct ttagcagcca ttggatgagg tgggtccgcc aggctccagg 5400
   gaaggggctg gagtgggtct caaacataaa gcaaggtgga agtgaagaat attatgtgga 5460
   ctctgtgaag ggccggttca ccatctccag agacaattcc aagaacacgc tgtatctgca 5520
   aatgaacagc ctgagagccg aggacacggc cgtatattac tgtgcgaaag gaaattataa 5580
   ggcaggttgt gactactggg gccagggcac cctggtcacc gtctcctcag tgcctggtga 5640
   ctcttccgca atcagtcctg ggacagaaac ctcagggaca gcctttatga gttttgatca 5700
   catctaacag agaggatggg ccagagctat cgcgagtctg actgagtgag ctcggaggtg 5760
   cagctgttgg agtctggggg aggcttggta cagcctgggg ggtccctgag actctcctgt 5820
   gcagcctctg gattcacctt tagcacctat agcatgaact gggtccgcca ggctccaggg 5880
   aaggggctgg agtgggtctc atccattact agtagtggta gttacatata ctgcgcagac 5940
   tcagtgaagg gccggttcac catctccaga gacaattcca agaacacgct gtatctgcaa 6000
   atgaacagcc tgagagccga ggacacggcc gtatattact gtgcgaaaga tggctccggt 6060
   atggacgtct ggggccaggg caccctggtc accgtctcct cagtgcctgg tgactcttcc 6120
   gcaatcagtc ctgggacaga aacctcaggg acagccttta tgagttttga tcacatctaa 6180
   cagagaggat gggccaggag gtgcagctgt tggagtctgg gggaggcttg gtacagcctg 6240
   gggggtccct gagactctcc tgtgcagcct ctggattcac ctttagctcc tatagcatga 6300
   cctgggtccg ccaggctcca gggaaggggc tggagtgggt ctcaaacatt agcagtagta 6360
   gtaataccgc attctacgca gactccgtga agggccggtt caccatctcc agagacaatt 6420
   ccaagaacac gctgtatctg caaatgaaca gcctgagagc cgaggacacg gccgtatatt 6480
   actgtgcgaa agatcgaaat ggctacaatt tctttgacta ctggggccag ggcaccctgg 6540
   tcaccgtctc ctcagtgcct ggtgactctt ccgcaatcag tcctgggaca gaaacctcag 6600
   ggacagcctt tatgagtttt gatcacatct aacagagagg atgggccaga tagctctcga 6660
   tcacgtgcga tcgtagcagg aggtgcagct gttggagtct gggggaggct tggtacagcc 6720
   tggggggtcc ctgagactct cctgtgcagc ctctggattc acctttagcg gctttgccat 6780
   gaactgggtc cgccaggctc cagggaaggg gctggagtgg gtctcaacta ttagtaatag 6840
   tgctggtagc acatattacg cagactccgt gaagggccgg ttcaccatct ccagagacaa 6900
   ttccaagaac acgctgtatc tgcaaatgaa cagcctgaga gccgaggaca cggccgtata 6960
   ttactgtgcg aaatatgggg aggatattgt cgttgtgcca gctcgctacg gtgtggacgt 7020
   ctggggccag ggcaccctgg tcaccgtctc ctcagtgcct ggtgactctt ccgcaatcag 7080
   tcctgggaca gaaacctcag ggacagcctt tatgagtttt gatcacatct aacagagagg 7140
   atgggccagg ctagc 7155
<210> 181
   <211> 11374
   <212> DNA
   <213> Artificial Sequence
<220>
   <223> Synthetic DNA
<400> 181
<210> 182
   <211> 11392
   <212> DNA
   <213> Artificial Sequence
<220>
   <223> Synthetic DNA
<400> 182
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt catatgtagc ttacgacact 420
   gataacttcg tatatggtat tatatacgaa gttataagct tcaccatgat tgaacaagat 480
   ggattgcacg caggttctcc ggccgcttgg gtggagaggc tattcggcta tgactgggca 540
   caacagacaa tcggctgctc tgatgccgcc gtgttccggc tgtcagcgca ggggcgcccg 600
   gttctttttg tcaagaccga cctgtccggt gccctgaatg aactgcagga cgaggcagcg 660
   cggctatcgt ggctggccac gacgggcgtt ccttgcgcag ctgtgctcga cgttgtcact 720
   gaagcgggaa gggactggct gctattgggc gaagtgccgg ggcaggatct cctgtcatct 780
   caccttgctc ctgccgagaa agtatccatc atggctgatg caatgcggcg gctgcatacg 840
   cttgatccgg ctacctgccc attcgaccac caagcgaaac atcgcatcga gcgagcacgt 900
   actcggatgg aagccggtct tgtcgatcag gatgatctgg acgaagagca tcaggggctc 960
   gcgccagccg aactgttcgc caggctcaag gcgcgcatgc ccgacggcga ggatctcgtc 1020
   gtgacccatg gcgatgcctg cttgccgaat atcatggtgg aaaatggccg cttttctgga 1080
   ttcatcgact gtggccggct gggtgtggcg gaccgctatc aggacatagc gttggctacc 1140
   cgtgatattg ctgaagagct tggcggcgaa tgggctgacc gcttcctcgt gctttacggt 1200
   atcgccgctc ccgattcgca gcgcatcgcc ttctatcgcc ttcttgacga gttcttctga 1260
   actagtgcgg gactctgggg ttcgaaatga ccgaccaagc gacgcccaac ctgccatcac 1320
   gagatttcga ttccaccgcc gccttctatg aaaggttggg cttcggaatc gttttccggg 1380
   acgccggctg gatgatcctc cagcgcgggg atctcatgct ggagttcttc gcccacccca 1440
   acttgtttat tgcagcttat aatggttaca aataaagcaa tagcatcaca aatttcacaa 1500
   ataaagcatt tttttcactg cattctagtt gtggtttgtc caaactcatc aatgtatctt 1560
   atcatgtctg ttataccgtc gatctctagc tagagcttgg cgtaatcatg gtcatagctg 1620
   tttcctgtgt gcccacccca acttgtttat tgcagcttat aatggttaca aataaagcaa 1680
   tagcatcaca aatttcacaa ataaagcatt tttttcactg cattctagtt gtggtttgtc 1740
   caaactcatc aatgtatctt atcatgtctg ataacttcgt ataatgtatg ctatacgaac 1800
   ggtactcgag agctcatgcg gtaccgctag catgctggcc aggatccatg cccatggccg 1860
   cggatgcggc gcgccatcga tatgcgtcga catttaaatg ggcccccggg tgatatcccc 1920
   ggggctagcc tggcccatcc tctctgttag atgtgatcaa aactcataaa ggctgtccct 1980
   gaggtttctg tcccaggact gattgcggaa gagtcaccag gcactgagga gacggtgacc 2040
   agggtgccct ggccccagac gtccacaccg tagcgagctg gcacaacgac aatatcctcc 2100
   ccatatttcg cacagtaata tacggccgtg tcctcggctc tcaggctgtt catttgcaga 2160
   tacagcgtgt tcttggaatt gtctctggag atggtgaacc ggcccttcac ggagtctgcg 2220
   taatatgtgc taccagcact attactaata gttgagaccc actccagccc cttccctgga 2280
   gcctggcgga cccagttcat ggcaaagccg ctaaaggtga atccagaggc tgcacaggag 2340
   agtctcaggg acccccaggg ctgtaccaag cctcccccag actccaacag ctgcacctcc 2400
   tgctacgatc gcacgtgatc gagagctatc tggcccatcc tctctgttag atgtgatcaa 2460
   aactcataaa ggctgtccct gaggtttctg tcccaggact gattgcggaa gagtcaccag 2520
   gcactgagga gacggtgacc agggtgccct ggccccagta gtcaaagaaa ttgtagccat 2580
   ttcgatcttt cgcacagtaa tatacggccg tgtcctcggc tctcaggctg ttcatttgca 2640
   gatacagcgt gttcttggaa ttgtctctgg agatggtgaa ccggcccttc acggagtctg 2700
   cgtagaatgc ggtattacta ctactgctaa tgtttgagac ccactccagc cccttccctg 2760
   gagcctggcg gacccaggtc atgctatagg agctaaaggt gaatccagag gctgcacagg 2820
   agagtctcag ggacccccca ggctgtacca agcctccccc agactccaac agctgcacct 2880
   cctggcccat cctctctgtt agatgtgatc aaaactcata aaggctgtcc ctgaggtttc 2940
   tgtcccagga ctgattgcgg aagagtcacc aggcactgag gagacggtga ccagggtgcc 3000
   ctggccccag acgtccatac cggagccatc tttcgcacag taatatacgg ccgtgtcctc 3060
   ggctctcagg ctgttcattt gcagatacag cgtgttcttg gaattgtctc tggagatggt 3120
   gaaccggccc ttcactgagt ctgcgcagta tatgtaacta ccactactag taatggatga 3180
   gacccactcc agccccttcc ctggagcctg gcggacccag ttcatgctat aggtgctaaa 3240
   ggtgaatcca gaggctgcac aggagagtct cagggacccc ccaggctgta ccaagcctcc 3300
   cccagactcc aacagctgca cctccgagct cactcagtca gactcgcgat agctctggcc 3360
   catcctctct gttagatgtg atcaaaactc ataaaggctg tccctgaggt ttctgtccca 3420
   ggactgattg cggaagagtc accaggcact gaggagacgg tgaccagggt gccctggccc 3480
   cagtagtcac aacctgcctt ataatttcct ttcgcacagt aatatacggc cgtgtcctcg 3540
   gctctcaggc tgttcatttg cagatacagc gtgttcttgg aattgtctct ggagatggtg 3600
   aaccggccct tcacagagtc cacataatat tcttcacttc caccttgctt tatgtttgag 3660
   acccactcca gccccttccc tggagcctgg cggacccacc tcatccaatg gctgctaaag     3720
   gtgaatccag aggctgcaca ggagagtctc agggaccccc caggctgtac caagcctccc     3780
   ccagactcca acagctgcac ctcctggccc atcctctctg ttagatgtga tcaaaactca     3840
   taaaggctgt ccctgaggtt tctgtcccag gactgattgc ggaagagtca ccaggcactg     3900
   aggagacggt gaccagggtg ccctggcccc aggagtcaaa tgcagccccc actatccctc     3960
   tctcgggttt cgcacagtaa tatacggccg tgtcctcggc tctcaggctg ttcatttgca     4020
   gatacagcgt gttcttggaa ttgtctctgg agatggtgaa ccggccctgc acggagtcag     4080
   cgtagaatgt gctaccaccg ccgccactaa tacctgagac ccactccagc cccttccctg     4140
   gagcctggcg gacccagctt atggcatagc taaaggtgaa tccagaggct gcacaggaga     4200
   gtctcaggga ccccccaggc tgtaccaagc ctcccccaga ctccaacagc tgcacctcca     4260
   cactctctcg ctgagtctct ctgtgagtct ggcccatcct ctctgttaga tgtgatcaaa     4320
   actcataaag gctgtccctg aggtttctgt cccaggactg attgcggaag agtcaccagg     4380
   cactgaggag acggtgacca gggtgccctg gccccaatag tcaaaaacac tactatcata     4440
   cacccacgct ttcgcacagt aatatacggc cgtgtcctcg gctctcaggc tgttcatttg     4500
   cagatacagc gtgttcttgg aattgtctct ggagatggtg aaccggccct tcacggagtc     4560
   tgcgtagtat gtgccaccac tgctataaat aagtgagacc cactccagcc ccttccctgg     4620
   agcctggcgg acccagctca tggagttgct gctaaaggtg aatccagagg ctgcacagga     4680
   gagtctcagg gaccccccag gctgtaccaa gcctccccca gactccaaca gctgcacctc     4740
   ctggcccatc ctctctgtta gatgtgatca aaactcataa aggctgtccc tgaggtttct     4800
   gtcccaggac tgattgcgga agagtcacca ggcactgagg agacggtgac cagggtgccc     4860
   tggccccaga gctggaagta tccatagcag ctggcgcaga acgccaggcc tttcgcacag     4920
   taatatacgg ccgtgtcctc ggctctcagg ctgttcattt gcagatacag cgtgttcttg     4980
   gaattgtctc tggagatggt gaaccggccc tccacaaagt ctgcgtagta tatggtacta     5040
   gaatcactac taacgtatga gacccactcc agccccttcc ctggagcctg gcggacccag     5100
   ttcatgttat agctgctaaa ggtgaatcca gaggctgcac aggagagtct cagggacccc     5160
   ccaggctgta ccaagcctcc cccagactcc aacagctgca cctccgctgc acgtgtagcg     5220
   agcagtctat agcactggcc catcctctct gttagatgtg atcaaaactc ataaaggctg     5280
   tccctgaggt ttctgtccca ggactgattg cggaagagtc accaggcact gaggagacgg     5340
   tgaccagggt gccctggccc cagacgtcca taccatctcg aggaaatgaa ccatagtaat     5400
   tggggacttt cgcacagtaa tatacggccg tgtcctcggc tctcaggctg ttcatttgca     5460
   gatacagcgt gttcttggaa ttgtctctgg agatggtgaa ccgacccttc acagagtctg     5520
   cgtagtatat ggtactacta ctacttctaa tgtatgagac ccactccagc cccttccctg 5580
   gagcctggcg gacccagttc atgctatggc tgctaaaggt gaatccagag gctgcacagg 5640
   agagtctcag ggacccccca ggctgtacca agcctccccc agactccaac agctgcacct 5700
   cgtacacatc agcgtcactc agcgagacag actggcccat cctctctgtt agatgtgatc 5760
   aaaactcata aaggctgtcc ctgaggtttc tgtcccagga ctgattgcgg aagagtcacc 5820
   aggcactgag gagacggtga ccagggtgcc ctggccccag tagtcaaggg cgtatgcact 5880
   actatcacct cgtttcgcac agtaatatac ggccgtgtcc tcggctctca ggctgttcat 5940
   ttgcagatac agcgtgttct tggaattgtc tctggagatg gtgaaccggc ccttcacaga 6000
   gtctgcgtag tatatgctac cactaccact actaatgtat gagacccact ccagcccctt 6060
   ccctggagcc tggcggaccc agttcatggt atagctgcta aaggtgaatc cagaggctgc 6120
   acaggagagt ctcagggacc ccccaggctg taccaagcct cccccagact ccaacagctg 6180
   cacctcctgg cccatcctct ctgttagatg tgatcaaaac tcataaaggc tgtccctgag 6240
   gtttctgtcc caggactgat tgcggaagag tcaccaggca ctgaggagac ggtgaccagg 6300
   gtgccctggc cccagacgtc cataccgtag tagtgataat aagtcccagc tgctgctata 6360
   agttggggcc cccctttcgc acagtaatat acggccgtgt cctcggctct caggctgttc 6420
   atttgcagat acagcgtgtt cttggaattg tctctggaga tggtgaaccg gcccttcacg 6480
   gagtctgcgt agtctgtgct accgccacta acagtaatac ctgagaccca ctccagcccc 6540
   ttccctggag cctggcggac ccagatcatg gcatagctgc taaaggtgaa tccagaggct 6600
   gcacaggaga gtctcaggga ccccccaggc tgtaccaagc ctcccccaga ctccaacagc 6660
   tgcacctcgt gtctgcgaga gagctcgaga ctcgatgact ggcccatcct ctctgttaga 6720
   tgtgatcaaa actcataaag gctgtccctg aggtttctgt cccaggactg attgcggaag 6780
   agtcaccagg cactgaggag acggtgacca gggtgccctg gccccagacg tccataccgt 6840
   agtagtagaa cgggtagctg ctaccagcac ttccggcttt cgcacagtaa tatacggccg 6900
   tgtcctcggc tctcaggctg ttcatttgca gatacagcgt gttcttggaa ttgtctctgg 6960
   agatggtgaa ccggcccttc acggagtctc cgtagtatgt gctaagacca tgaccactaa 7020
   tagctgagac ccactccagc cccttccctg gagcctggcg gacccagctc acggcaaaga 7080
   tgctaaaggt gaatccagag gctgcacagg agagtctcag ggacccccca ggctgtacca 7140
   agcctccccc agactccaac agctgcacct cctggcccat cctctctgtt agatgtgatc 7200
   aaaactcata aaggctgtcc ctgaggtttc tgtcccagga ctgattgcgg aagagtcacc 7260
   aggcactgag gagacggtga ccagggtgcc ctggccccag ttgaccgttc cgtagtaggc 7320
   cccaccgtct ttcgcacagt aatatacggc cgtgtcctcg gctctcaggc tgttcatttg 7380
   cagatacagc gtgttcttgg aattgtctct ggagatggtg aaccggccct gcacggagtc     7440
   tgcgtagtat gtgctaccaa cactaccact aatagctgag acccactcca gccccttccc     7500
   tggagcctgg cggacccagg tcatggcaaa gctgctaaag gtgaatccag aggctgcaca 7560
   ggagagtctc agggaccccc caggctgtac caagcctccc ccagactcca acagctgcac 7620
   ctcgtatgag tgagcgtatc atagatgtct atactggccc atcctctctg ttagatgtga 7680
   tcaaaactca taaaggctgt ccctgaggtt tctgtcccag gactgattgc ggaagagtca 7740
   ccaggcactg aggagacggt gaccagggtg ccctggcccc aggggtcgaa ccaatttgaa 7800
   acactccaaa aatcgtcctg atctttcgca cagtaatata cggccgtgtc ctcggctctc 7860
   aggctgttca tttgcagata cagcgtgttc ttggaattgt ctctggagat ggtgaaccgg 7920
   cccttcacgg agtctgtgta gagtgtgctt ccaccactac cactaataac tgagacccac 7980
   tccagcccct tccctggagc ctggcggacc cagctcatgg gaaagttgct aaaggtgaat 8040
   ccagaggctg cacaggagag tctcagggac cccccaggct gtaccaagcc tcccccagac 8100
   tccaacagct gcacctcctg gcccatcctc tctgttagat gtgatcaaaa ctcataaagg 8160
   ctgtccctga ggtttctgtc ccaggactga ttgcggaaga gtcaccaggc actgaggaga 8220
   cggtgaccag ggtgccctgg ccccagtagt caaagtctgc gcagtcacca ccacaaccaa 8280
   agggcccatt tttcgcacag taatatacgg ccgtgtcctc ggctctcagg ctgttcattt 8340
   gcagatacag cgtgttcttg gaattgtctc tggagatggt gaaccggccc ttcacggagt 8400
   ctgcataaaa tgttctatca ccagtcttac taaaagttga gacccactcc agccccttcc 8460
   ctggagcctg gcggacccag ctcatggcat agttgctaaa ggtgaatcca gaggctgcac 8520
   aggagagtct cagggacccc ccaggctgta ccaagcctcc cccagactcc aacagctgca 8580
   cctccgcaca cgcacgctct atcactctag ctcactggcc catcctctct gttagatgtg 8640
   atcaaaactc ataaaggctg tccctgaggt ttctgtccca ggactgattg cggaagagtc 8700
   accaggcact gaggagacgg tgaccagggt gccctggccc caggggccga accaagttag 8760
   gctgcgacta tgtgcccctt tcgcacagta atatacggcc gtgtcctcgg ctctcaggct 8820
   gttcatttgc agatacagcg tgttcttgga attgtctctg gagatggtga accggccctt 8880
   cacggagtct gcgtagtatg tgttactagc actaccagta atagttgaga cccactccag 8940
   ccccttccct ggagcctggc ggacccaatt cagggcaaaa ctgctaaagg tgaatccaga 9000
   ggctgcacag gagagtctca gggacccccc aggctgtacc aagcctcccc cagactccaa 9060
   cagctgcacc tcggtaccgg atcctctaga gcggccgcta ccgttcgtat attctatctt 9120
   atacgaagtt atatcgaatt cctgcagaga tctagcttgg cgtaatcatg gtcatagctg 9180
   tttcctgtgt gaaattgtta tccgctcaca attccacaca acatacgagc cggaagcata 9240
   aagtgtaaag cctggggtgc ctaatgagtg agctaactca cattaattgc gttgcgctca 9300
   ctgcccgctt tccagtcggg aaacctgtcg tgccagctgc attaatgaat cggccaacgc 9360
   gcggggagag gcggtttgcg tattgggcgc tcttccgctt cctcgctcac tgactcgctg 9420
   cgctcggtcg ttcggctgcg gcgagcggta tcagctcact caaaggcggt aatacggtta 9480
   tccacagaat caggggataa cgcaggaaag aacatgtgag caaaaggcca gcaaaaggcc 9540
   aggaaccgta aaaaggccgc gttgctggcg tttttccata ggctccgccc ccctgacgag 9600
   catcacaaaa atcgacgctc aagtcagagg tggcgaaacc cgacaggact ataaagatac 9660
   caggcgtttc cccctggaag ctccctcgtg cgctctcctg ttccgaccct gccgcttacc 9720
   ggatacctgt ccgcctttct cccttcggga agcgtggcgc tttctcatag ctcacgctgt 9780
   aggtatctca gttcggtgta ggtcgttcgc tccaagctgg gctgtgtgca cgaacccccc 9840
   gttcagcccg accgctgcgc cttatccggt aactatcgtc ttgagtccaa cccggtaaga 9900
   cacgacttat cgccactggc agcagccact ggtaacagga ttagcagagc gaggtatgta 9960
   ggcggtgcta cagagttctt gaagtggtgg cctaactacg gctacactag aagaacagta 10020
   tttggtatct gcgctctgct gaagccagtt accttcggaa aaagagttgg tagctcttga 10080
   tccggcaaac aaaccaccgc tggtagcggt ggtttttttg tttgcaagca gcagattacg 10140
   cgcagaaaaa aaggatctca agaagatcct ttgatctttt ctacggggtc tgacgctcag 10200
   tggaacgaaa actcacgtta agggattttg gtcatgagat tatcaaaaag gatcttcacc 10260
   tagatccttt taaattaaaa atgaagtttt aaatcaatct aaagtatata tgagtaaact 10320
   tggtctgaca gttaccaatg cttaatcagt gaggcaccta tctcagcgat ctgtctattt 10380
   cgttcatcca tagttgcctg actccccgtc gtgtagataa ctacgatacg ggagggctta 10440
   ccatctggcc ccagtgctgc aatgataccg cgagacccac gctcaccggc tccagattta 10500
   tcagcaataa accagccagc cggaagggcc gagcgcagaa gtggtcctgc aactttatcc 10560
   gcctccatcc agtctattaa ttgttgccgg gaagctagag taagtagttc gccagttaat 10620
   agtttgcgca acgttgttgc cattgctaca ggcatcgtgg tgtcacgctc gtcgtttggt 10680
   atggcttcat tcagctccgg ttcccaacga tcaaggcgag ttacatgatc ccccatgttg 10740
   tgcaaaaaag cggttagctc cttcggtcct ccgatcgttg tcagaagtaa gttggccgca 10800
   gtgttatcac tcatggttat ggcagcactg cataattctc ttactgtcat gccatccgta 10860
   agatgctttt ctgtgactgg tgagtactca accaagtcat tctgagaata gtgtatgcgg 10920
   cgaccgagtt gctcttgccc ggcgtcaata cgggataata ccgcgccaca tagcagaact 10980
   ttaaaagtgc tcatcattgg aaaacgttct tcggggcgaa aactctcaag gatcttaccg 11040
   ctgttgagat ccagttcgat gtaacccact cgtgcaccca actgatcttc agcatctttt 11100
   actttcacca gcgtttctgg gtgagcaaaa acaggaaggc aaaatgccgc aaaaaaggga 11160
   ataagggcga cacggaaatg ttgaatactc atactcttcc tttttcaata ttattgaagc 11220
   atttatcagg gttattgtct catgagcgga tacatatttg aatgtattta gaaaaataaa 11280
   caaatagggg ttccgcgcac atttccccga aaagtgccac ctgacgtcta agaaaccatt 11340
   attatcatga cattaaccta taaaaatagg cgtatcacga ggccctttcg tc 11392
<210> 183
   <211> 1251
   <212> PRT
   <213> Homo sapiens
<400> 183
   Ile Ile Pro Val Asn His His His His His His Glu Glu Glu Asn Pro
   1 5 10 15
   Asp Phe Trp Asn Arg Glu Ala Ala Glu Ala Leu Gly Ala Ala Lys Lys
   20 25 30
   Leu Gln Pro Ala Gln Thr Ala Ala Lys Asn Leu Ile Ile Phe Leu Gly
   35 40 45
   Asp Gly Met Gly Val Ser Thr Val Thr Ala Ala Arg Ile Leu Lys Gly
   50 55 60
   Gln Lys Lys Asp Lys Leu Gly Pro Glu Ile Pro Leu Ala Met Asp Arg
   65 70 75 80
   Phe Pro Tyr Val Ala Leu Ser Lys Thr Tyr Asn Val Asp Lys His Val
   85 90 95
   Pro Asp Ser Gly Ala Thr Ala Thr Ala Tyr Leu Cys Gly Val Lys Gly
   100 105 110
   Asn Phe Gln Thr Ile Gly Leu Ser Ala Ala Ala Arg Phe Asn Gln Cys
   115 120 125
   Asn Thr Thr Arg Gly Asn Glu Val Ile Ser Val Met Asn Arg Ala Lys
   130 135 140
   Lys Ala Gly Lys Ser Val Gly Val Val Thr Thr Thr Arg Val Gln His
   145 150 155 160
   Ala Ser Pro Ala Gly Thr Tyr Ala His Thr Val Asn Arg Asn Trp Tyr
   165 170 175
   Ser Asp Ala Asp Val Pro Ala Ser Ala Arg Gln Glu Gly Cys Gln Asp
   180 185 190
   Ile Ala Thr Gln Leu Ile Ser Asn Met Asp Ile Asp Val Ile Leu Gly
   195 200 205
   Gly Gly Arg Lys Tyr Met Phe Pro Met Gly Thr Pro Asp Pro Glu Tyr
   210 215 220
   Pro Asp Asp Tyr Ser Gln Gly Gly Thr Arg Leu Asp Gly Lys Asn Leu
   225 230 235 240
   Val Gln Glu Trp Leu Ala Lys Arg Gln Gly Ala Arg Tyr Val Trp Asn
   245 250 255
   Arg Thr Glu Leu Met Gln Ala Ser Leu Asp Pro Ser Val Thr His Leu
   260 265 270
   Met Gly Leu Phe Glu Pro Gly Asp Met Lys Tyr Glu Ile His Arg Asp
   275 280 285
   Ser Thr Leu Asp Pro Ser Leu Met Glu Met Thr Glu Ala Ala Leu Arg
   290 295 300
   Leu Leu Ser Arg Asn Pro Arg Gly Phe Phe Leu Phe Val Glu Gly Gly
   305 310 315 320
   Arg Ile Asp His Gly His His Glu Ser Arg Ala Tyr Arg Ala Leu Thr
   325 330 335
   Glu Thr Ile Met Phe Asp Asp Ala Ile Glu Arg Ala Gly Gln Leu Thr
   340 345 350
   Ser Glu Glu Asp Thr Leu Ser Leu Val Thr Ala Asp His Ser His Val
   355 360 365
   Phe Ser Phe Gly Gly Tyr Pro Leu Arg Gly Ser Ser Ile Phe Gly Leu
   370 375 380
   Ala Pro Gly Lys Ala Arg Asp Arg Lys Ala Tyr Thr Val Leu Leu Tyr
   385 390 395 400
   Gly Asn Gly Pro Gly Tyr Val Leu Lys Asp Gly Ala Arg Pro Asp Val
   405 410 415
   Thr Glu Ser Glu Ser Gly Ser Pro Glu Tyr Arg Gln Gln Ser Ala Val
   420 425 430
   Pro Leu Asp Glu Glu Thr His Ala Gly Glu Asp Val Ala Val Phe Ala
   435 440 445
   Arg Gly Pro Gln Ala His Leu Val His Gly Val Gln Glu Gln Thr Phe
       450                 455                 460
   Ile Ala His Val Met Ala Phe Ala Ala Cys Leu Glu Pro Tyr Thr Ala
   465                 470                 475                 480
   Cys Asp Leu Ala Pro Pro Ala Gly Thr Thr Asp Ala Ala His Pro Gly
                   485                 490                 495
   Glu Phe Arg Ala Asn Tyr Gln Asn Gly Lys Asn Asn Val Pro Arg Leu
               500                 505                 510
   Lys Leu Ser Tyr Lys Glu Met Leu Glu Ser Asn Asn Val Ile Thr Phe
           515                 520                 525
   Asn Gly Leu Ala Asn Ser Ser Ser Tyr His Thr Phe Leu Leu Asp Glu
       530                 535                 540
   Glu Arg Ser Arg Leu Tyr Val Gly Ala Lys Asp His Ile Phe Ser Phe
   545                 550                 555                 560
   Asp Leu Val Asn Ile Lys Asp Phe Gln Lys Ile Val Trp Pro Val Ser
                   565                 570                 575
   Tyr Thr Arg Arg Asp Glu Cys Lys Trp Ala Gly Lys Asp Ile Leu Lys
               580                 585                 590
   Glu Cys Ala Asn Phe Ile Lys Val Leu Lys Ala Tyr Asn Gln Thr His
           595                 600                 605
   Leu Tyr Ala Cys Gly Thr Gly Ala Phe His Pro Ile Cys Thr Tyr Ile
       610                 615                 620
   Glu Ile Gly His His Pro Glu Asp Asn Ile Phe Lys Leu Glu Asn Ser
   625                 630                 635                 640
   His Phe Glu Asn Gly Arg Gly Lys Ser Pro Tyr Asp Pro Lys Leu Leu
                   645                 650                 655
   Thr Ala Ser Leu Leu Ile Asp Gly Glu Leu Tyr Ser Gly Thr Ala Ala
               660                 665                 670
   Asp Phe Met Gly Arg Asp Phe Ala Ile Phe Arg Thr Leu Gly His His
           675                 680                 685
   His Pro Ile Arg Thr Glu Gln His Asp Ser Arg Trp Leu Asn Asp Pro
       690                 695                 700
   Lys Phe Ile Ser Ala His Leu Ile Ser Glu Ser Asp Asn Pro Glu Asp
   705                 710                 715                 720
   Asp Lys Val Tyr Phe Phe Phe Arg Glu Asn Ala Ile Asp Gly Glu His
                   725                 730                 735
   Ser Gly Lys Ala Thr His Ala Arg Ile Gly Gln Ile Cys Lys Asn Asp
               740                 745                 750
   Phe Gly Gly His Arg Ser Leu Val Asn Lys Trp Thr Thr Phe Leu Lys
           755                 760                 765
   Ala Arg Leu Ile Cys Ser Val Pro Gly Pro Asn Gly Ile Asp Thr His
       770                 775                 780
   Phe Asp Glu Leu Gln Asp Val Phe Leu Met Asn Phe Lys Asp Pro Lys
   785                 790                 795                 800
   Asn Pro Val Val Tyr Gly Val Phe Thr Thr Ser Ser Asn Ile Phe Lys
                   805                 810                 815
   Gly Ser Ala Val Cys Met Tyr Ser Met Ser Asp Val Arg Arg Val Phe
               820                 825                 830
   Leu Gly Pro Tyr Ala His Arg Asp Gly Pro Asn Tyr Gln Trp Val Pro
           835                 840                 845
   Tyr Gln Gly Arg Val Pro Tyr Pro Arg Pro Gly Thr Cys Pro Ser Lys
       850                 855                 860
   Thr Phe Gly Gly Phe Asp Ser Thr Lys Asp Leu Pro Asp Asp Val Ile
   865                 870                 875                 880
   Thr Phe Ala Arg Ser His Pro Ala Met Tyr Asn Pro Val Phe Pro Met
                   885                 890                 895
   Asn Asn Arg Pro Ile Val Ile Lys Thr Asp Val Asn Tyr Gln Phe Thr
               900                 905                 910
   Gln Ile Val Val Asp Arg Val Asp Ala Glu Asp Gly Gln Tyr Asp Val
           915                 920                 925
   Met Phe Ile Gly Thr Asp Val Gly Thr Val Leu Lys Val Val Ser Ile
       930                 935                 940
   Pro Lys Glu Thr Trp Tyr Asp Leu Glu Glu Val Leu Leu Glu Glu Met
   945 950 955 960
   Thr Val Phe Arg Glu Pro Thr Ala Ile Ser Ala Met Glu Leu Ser Thr
   965 970 975
   Lys Gln Gln Gln Leu Tyr Ile Gly Ser Thr Ala Gly Val Ala Gln Leu
   980 985 990
   Pro Leu His Arg Cys Asp Ile Tyr Gly Lys Ala Cys Ala Glu Cys Cys
   995 1000 1005
   Leu Ala Arg Asp Pro Tyr Cys Ala Trp Asp Gly Ser Ala Cys Ser
   1010 1015 1020
   Arg Tyr Phe Pro Thr Ala Lys Arg Arg Thr Arg Arg Gln Asp Ile
   1025 1030 1035
   Arg Asn Gly Asp Pro Leu Thr His Cys Ser Asp Leu His His Asp
   1040 1045 1050
   Asn His His Gly His Ser Pro Glu Glu Arg Ile Ile Tyr Gly Val
   1055 1060 1065
   Glu Asn Ser Ser Thr Phe Leu Glu Cys Ser Pro Lys Ser Gln Arg
   1070 1075 1080
   Ala Leu Val Tyr Trp Gln Phe Gln Arg Arg Asn Glu Glu Arg Lys
   1085 1090 1095
   Glu Glu Ile Arg Val Asp Asp His Ile Ile Arg Thr Asp Gln Gly
   1100 1105 1110
   Leu Leu Leu Arg Ser Leu Gln Gln Lys Asp Ser Gly Asn Tyr Leu
   1115 1120 1125
   Cys His Ala Val Glu His Gly Phe Ile Gln Thr Leu Leu Lys Val
   1130 1135 1140
   Thr Leu Glu Val Ile Asp Thr Glu His Leu Glu Glu Leu Leu His
   1145 1150 1155
   Lys Asp Asp Asp Gly Asp Gly Ser Lys Thr Lys Glu Met Ser Asn
   1160 1165 1170
   Ser Met Thr Pro Ser Gln Lys Val Trp Tyr Arg Asp Phe Met Gln
   1175 1180 1185
   Leu Ile  Asn His Pro Asn Leu  Asn Thr Met Asp Glu  Phe Cys Glu
       1190                 1195                 1200
   Gln Val  Trp Lys Arg Asp Arg  Lys Gln Arg Arg Gln  Arg Pro Gly
       1205                 1210                 1215
   His Thr  Pro Gly Asn Ser Asn  Lys Trp Lys His Leu  Gln Glu Asn
       1220                 1225                 1230
   Lys Lys  Gly Arg Asn Arg Arg  Thr His Glu Phe Glu  Arg Ala Pro
       1235                 1240                 1245
   Arg Ser  Val
       1250